# EUROPEAN PATENT APPLICATION

(11) **EP 1 336 602 A1**
(43) Date of publication of application: **20.08.2003**
(21) Application number: 02425075.5
(22) Date of filing: 13.02.2002
(51) Int. Cl.: C07C 205/00, A61K 31/00

(54) **Nitrate prodrugs able to release nitric oxide in a controlled and selective way and their use for prevention and treatment of inflammatory, ischemic and proliferative diseases**

(71) Applicant: Scaramuzzino, Giovanni, 20052 Monza (Milano) (IT)
(72) Inventor: Scaramuzzino, Giovanni, 20052 Monza (Milano) (IT)

(57) **Abstract**

New pharmaceutical compounds of general formula (I): F-(X)_{q} where q is an integer from 1 to 5, preferably 1; -F is chosen among drugs described in the text, -X is chosen among 4 groups -M, -T, -V and -Y as described in the text.

The compounds of general formula (I) are nitrate prodrugs which can release nitric oxide in vivo in a controlled and selective way and without hypotensive side effects and for this reason they are useful for the preparation of medicines for prevention and treatment of inflammatory, ischemic, degenerative and proliferative diseases of musculoskeletal, tegumental, respiratory, gastrointestinal, genito-urinary and central nervous systems.

## Description

The present invention relates to new nitrate prodrugs which can release nitric oxide in vivo in a controlled and selective way and without the side effects typical of nitrate vasodilators drugs.

In fact, contrary to any expectation, the prodrugs of this invention can release nitric oxide in a controlled way thanks to their slow absorption in vivo.

Furthermore the prodrugs of the invention release nitric oxide in a specific way because they can exploit the specific carrier of erythrocytes.

The prodrugs of the invention are devoid of the side effects typical of nitrate vasodilators drugs (e.g. nitroglycerine, isosorbide mononitrate) used for the treatment of angina pectoris. These effects, like orthostatic hypotension, migraine and dizziness, are linked to the vasorelaxant properties of nitric oxide which, besides the benefits for the treatment of angina, causes this side effects if released in an uncontrolled way.

Surprisingly the prodrugs of the invention can exploit the advantages of a controlled and selective release of nitric oxide without side effects for several diseases and they have a better activity and/or fewer side effects than the original drugs.

For all these reasons the prodrugs of this invention are useful for a chronic, controlled and selective administration of nitric oxide and therefore for the preparation of medicines for prevention and treatment of inflammatory, ischemic, degenerative and proliferative diseases of musculoskeletal, tegumental, respiratory, gastrointestinal, genito-urinary and central nervous systems.

One has been trying to exploit the therapeutical properties of nitric oxide for many years but the problems to solve are many and, at this time, not yet solved.

The main problems are the very short half-life of nitric oxide that is 3-5 seconds, its aspecificity of action and most of all the necessity to control strictly the quantity of nitric oxide released. This last problem is particularly important because nitric oxide has often effects which are even opposite, according to the quantity released in the time.

In fact nitric oxide is produced in our body by three isoenzymes called neuronal (nNOS), endothelial (eNOS) and inducible (iNOS) nitric oxide synthetases.

While the nitric oxide produced by eNOS has antiinflammatory and antiapoptotic activity, the nitric oxide produced by iNOS has opposite effects because it is produced in a great quantity and in a short time, favouring the formation of peroxynitrites and other compounds which are very cytotoxic for tissues («The dual personality of NO», Colasanti M. et al, Trends in Pharmaceutical Sciences, 21, 2000, 249-253).

Furthermore, for the same reason and on the contrary of eNOS, iNOS enzyme causes a marked hypotension.

Also an uncontrolled administration of a nitric oxide-donor (NO-donor) can cause several side effects due to the vasorelaxant activity of nitric oxide, which causes orthostatic hypotension, migraine and dizziness if released excessively (see for example Goodman & Gilman "The Pharmaceutical Basis of Terapeutics", 9^{th} Ed., 1996).

Recently some researchers (Patel et al, J. Biol. Chem., 274,22;15487-15492 ; Woltzt et al, J. Biol. Chem., 274,41,28983-28990) have discovered how nitric oxide could be carried in the body despite its very short half-life.

It can bind itself to hemoglobin of erythrocytes giving two products (Hb(Fe(II))NO and SNO-Hb). Only SNO-Hb is active that is it can nitrosylate important cysteine groups of many proteins modifying so their activity.

Furthermore it has been suggested that, thanks to this interaction, nitric oxide could have some specificity of action because hemoglobin releases nitric oxide only in vascular districts having very low partial pressure of oxygen that is in hypoxic, ischemic and inflamed districts.

Furthermore nitric oxide can be transferred into every cell because hemoglobin can donate it to glutathione producing nitrosoglutathione (GSNO) which is stable (Spencer et al, J. Biol. Chem., 275,47,36562-36567).

The beneficial activities of the nitric oxide released by eNOS enzyme are several and known (see for example "Nitric Oxide", Handbook of Experimental Pharmacology, Vol. 143, 2000, Ed. Springer-Verlag, pag. 1-655). Therefore controlled doses or controlled concentrations of nitric oxide, like those released by eNOS enzyme, can carry out some pharmacological activities which are potentially useful for prevention and treatment of inflammatory, ischemic, degenerative and proliferative diseases of musculoskeletal, tegumental, respiratory, gastrointestinal, genito-urinary and central nervous systems.

The release of nitric oxide from NO-donors can occur both in an enzymatic and a non-enzymatic way. As for nitrate esters this release mainly occurs by some p450 reductase enzymes and by glutathione S-transferase (Minamiyama K. Et al, FEBS Letters. Vol 452(3) (pp 165-169), 1999).

As for the possibility to exploit therapeutically the properties of nitric oxide for the prevention and the treatment of the above-mentioned diseases, at the moment the problems to solve are still many among which the most important are:
- to obtain a controlled release of nitric oxide to avoid the serious above-mentioned side effects, caused by an uncontrolled release of it
- to exploit the specific nitric oxide carrier system of erythrocytes designing molecules which can produce nitrosohemoglobin (SNO-Hb)

With great surprise we have synthesized compounds which can have both these requirements. In particular, the unexpected advantages of the compounds of this invention for prevention and treatment of inflammatory, ischemic, degenerative and proliferative diseases of musculoskeletal, tegumental, respiratory, gastrointestinal, genito-urinary and central nervous systems are the following.
1) As for controlled release, we have unexpectedly found that the best mode to control nitric oxide release is to slow the absorption of prodrugs. In fact the prodrugs of the invention are adsorbed more slowly than the original drugs and most of all than all known nitrate vasodilators.
2) In fact for this reason the compounds of the invention are unexpectedly devoid of the marked hypotensive activity and of the side effects related to it, typical of nitrate vasodilators drugs and therefore they are useful for a chronic administration of low doses of nitric oxide.
3) The compound of the invention can unexpectedly exploit the specific nitric oxide carrier of erythrocytes because they can produce nitrosohemoglobin (SNO-Hb).
4) The compound of the invention, being esters and salts and therefore easily hydrolyzable, can release the original drugs active for prevention and treatment of inflammatory, ischemic, degenerative and proliferative diseases, of which they can improve the efficacy and diminish the side effects thanks to the synergic properties of nitric oxide.

The compounds of this invention are more effective than the original drugs. For example derivatives of proton pump inhibitors or of their metabolites are more effective for prevention and treatment of peptic ulcer thanks to the properties of nitric oxide. Furthermore derivatives of selective estrogen receptor modulators drugs are more effective for prevention of osteoporosis thanks to the properties of nitric oxide.

The compounds of the invention have not necessarily the same therapeutic application of the original drugs (e.g. derivatives of antiinflammatory drugs have also carcinogenesis inhibitor activity, thanks to the properties of nitric oxide).

The compounds of the invention can have more synergic activities for the same therapeutic application (e.g. derivatives of 5α-testosterone reductase inhibitor drugs for prevention of benign prostatic hypertrophy can also, thanks to nitric oxide, inhibit the proliferation of smooth muscle cells and they have also antiinflammatory and muscolorelaxant properties useful for the concomitant urinary incontinence).

The compounds of this invention can also be useful to overcome the serious side effects of the original drugs. For example derivatives of cyclooxigenase 2 (COX-2) inhibitors, thanks to the antithrombotic properties of nitric oxide, can solve the serious problems of cardiovascular toxicity due to the selective inhibition of COX-2 recently shown in VIGOR clinic study with more than 8000 patients treated with rofecoxib and whose results have raised serious doubts about the safety of these drugs.

The compound of this invention can also be at the same time more effective and with fewer side effects than the original drugs. For example the paranitroxymethylbenzoic acid derivatives of COX-2 inhibitors (in particular celecoxib and rofecoxib) have better antiinflammatory and analgesic activities than the original drugs, in particular for prevention of familial polyposis, for prevention of Alzheimer disesase and for treatment of urinary incontinence.

Besides, as said before, they can solve the serious problems of cardiovascular toxicity due to the selective inhibition of COX-2. Furthermore, in another example, the paranitroxymethylbenzoic acid derivatives of glucocorticoid (e.g. prednisolone, hydrocortisone, budesonide) are more effective than the original drugs for treatment of chronic inflammatory diseases (e.g. ulcerous colitis, psoriasis, asthma) and there are devoid of typical side effects of glucocorticoid (e.g. ulcer, osteoporosis).

The prodrugs of the invention are useful for a chronic, controlled and selective administration of nitric oxide and therefore for prevention and treatment of inflammatory, ischemic, degenerative and proliferative diseases of muscoloskeletal system (e.g. rheumatoid arthritis, osteoarthritis, lupus, osteoporosis, Paget disease), of tegumental system (e.g. ulcers, aphtha, psoriasis, dermatitis, skin tumors), of respiratory system (e.g. asthma, chronic obstructive pulmonary disease, allergy, emphysema, bronchitis, ARDS, rhinitis, cystic fibrosis, lung tumors), of gastrointestinal system (e.g. peptic ulcer, duodenal ulcer, ulcerative colitis, Crohn disease, hepatic cirrhosis, pancreatitis, esophagitis, gastritis, colon familial polyposis, colon-rectum tumors, liver tumors, oesophasus tumors, pancreas tumors, stomach tumors), of genital system (e.g. benign prostatic hypertrophy, prostatitis, sexual disorders, prostate tumors, breast tumors, uterus tumors, ovary tumors), of urinary system (urinary incontinence, bladder tumors) and of central nervous system (e.g, multiple sclerosis, Alzheimer disease, brain tumours).

An object of the present invention are compounds of general formula (I), their pharmaceutically acceptable salts, their in vivo hydrolyzable prodrugs, their tautomers and their diastereomers,

F-(X)_{q} (I)

where:
- q is an integer from 1 to 5, preferably 1;
- F is a molecule or a drug for prevention and treatment of inflammatory, ischemic, degenerative and proliferative diseases of musculoskeletal, tegumental, respiratory, gastrointestinal, genito-urinary and central nervous systems;
- X is chosen among 3 groups of substituents, -M, -T and -V where:
   - M is chosen among the following groups which can release nitric oxide in vivo: nitrate ester (Al) -ONO₂; nitrate salt (All); nitrite ester (AIII) -ONO; thionitrite (AIV) -SNO; NONOate (AV) -NO(NO)

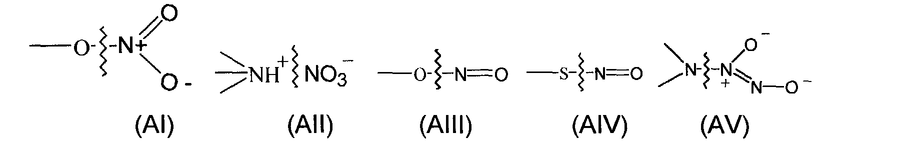

The undulated line shows where and how is the bond which is necessary to link M (on the right of the line) and the drug F (on the left of the line);
- T is chosen among the following radicals, optionally salified preferably with nitric acid:
- O-R₁-M; -O-R₁-O-R₁-M;-O-R₁-S-R₁-M;-O-R₁-NR₂-R₁-M;
- S-R₁-M; -S-R₁-O-R₁-M; -S-R₁-S-R₁-M; -S-R₁-NR₂-R₁-M;
- NR₂-R₁-M; -NR₂-R₁-O-R₁-M; -NR₂-R₁-S-R₁-M; -NR₂-R₁-NR₂-R₁-M;
- (CO)-R₁-M; -(CO)-R₁-O-R₁-M; -(CO)-R₁-S-R₁-M;-(CO)-R₁-NR₂-R₁-M;
- O-(CO)-R₁-M;
- R₁-O-R₁-M; -R₁-S-R₁-M; -R₁-NR₂-R₁-M;
- PO(O-)O-R₁-M;
where M is described as above;
- R₁- is a saturated or unsaturated, linear or branched alkylene, having from 1 to 21 carbon atoms or a saturated or unsaturated, optionally heterosubstituted or branched cycloalkylene, having from 3 to 7 carbon atoms or an optionally heterosubstituted arylene having from 3 to 7 carbon atoms;
- R₂ is H or a saturated or unsaturated, linear or branched alkyl, having from 1 to 21 carbon atoms or a saturated or unsaturated, optionally heterosubstituted or branched cycloalkyl, having from 3 to 7 carbon atoms or an optionally heterosubstituted aryl having from 3 to 7 carbon atoms;
- R₁- and -R₂ may be substituted with -OH, -SH, -F, -Cl, -Br, -OPO₃H₂, -COOH, NR₂ or with a saturated or unsaturated, linear or branched alkyl, having from 1 to 10 carbon atoms or with a saturated or unsaturated, optionally heterosubstituted or branched cycloalkyl, having from 3 to 7 carbon atoms
- the bond between -F and -T is hydrolyzable in vivo by metabolic or enzymatic activity and in in particular is a carboxylic ester, a carboxylic amide, a glycoside, an acetal, a ketal, an azo compound, an hydrazide, a carbammate, a thioester, a thioamide, a phosphoric ester, a phosphonic ester, a sulphuric ester, a sulphonic amide, a sulphonic ester.

The above cited pharmaceutically acceptable salts are salts from inorganic acid (e.g. nitrate, nitrite, chlorhydrate, bromhydrate, sulphate, phosphate), salts from organic acids (e.g. citrate tartrate, acetate, maleate, fumarate, ossalate, p-toluensulphonate, methanesulphonate, ethansulphonate, benzenesulphonate), from inorganic bases (e.g. ammonium, sodium, litium, potassium, magnesium, calcium and zinc salts) or from organic bases (e.g. ammonium salts of organic amines). Preferably they are nitrate salts, chlorhydrate, sodium salts and potassium salts. More preferably they are nitrate salts.

The bond between -R₁ and -M is described by the above cited formulae from (Al) to (AV) that is the undulated line shows here where and how is the bond which is necessary to link -M (on the right of the line) and -R₁ (on the left of the line);
- V is chosen among -Z-M₂, -O-Z-M₂, -N(R₂)-Z-M₂, -R₁-Z-M₂, -O-R₁-M₂, -O-R₁-Z-M₂ where:
- M₂ is chosen among -M, -R₁-M, -O-R₁-M, -S-R₁-M, -N(R₂)-R₁-M
where R₁, R₂ and M are described as above;
- Z-M₂ is chosen among the following radicals, optionally salified preferably with nitric acid: 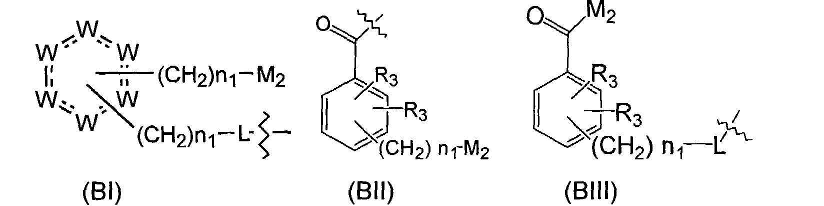 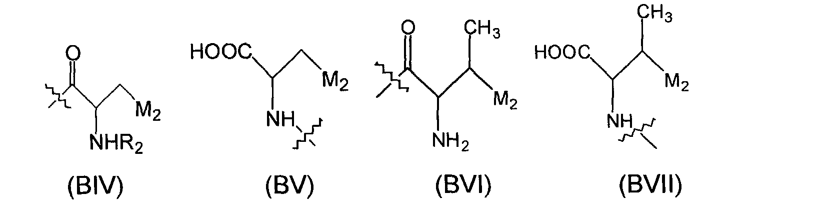 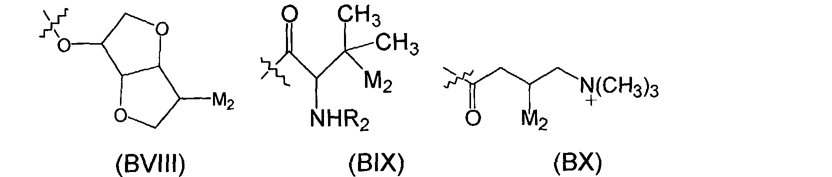 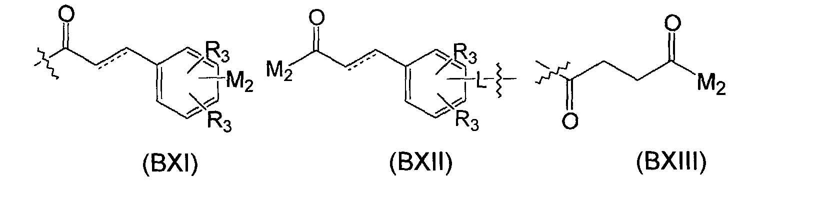 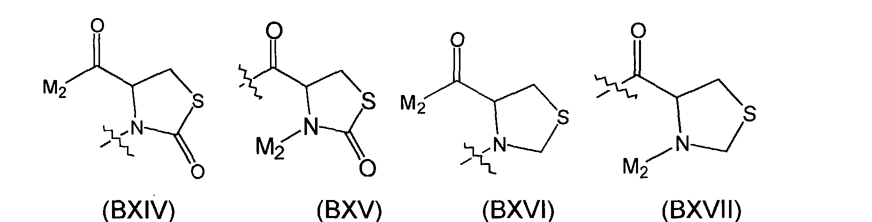 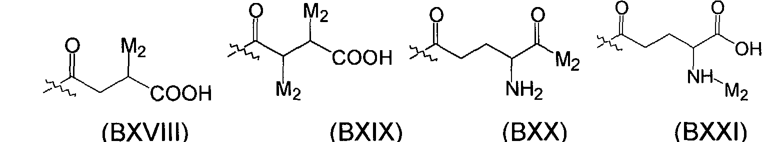 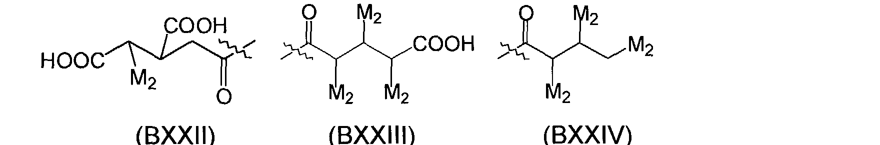 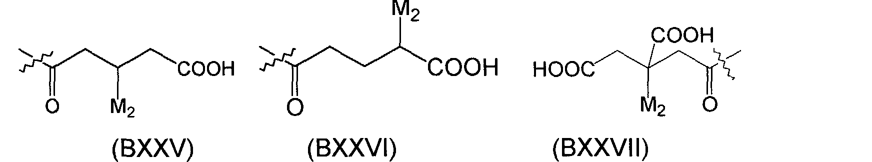 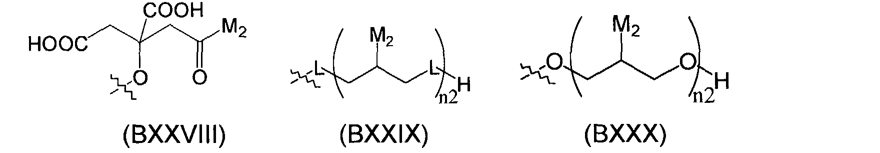 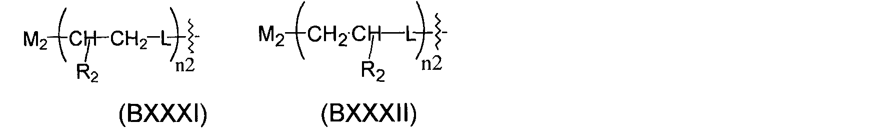 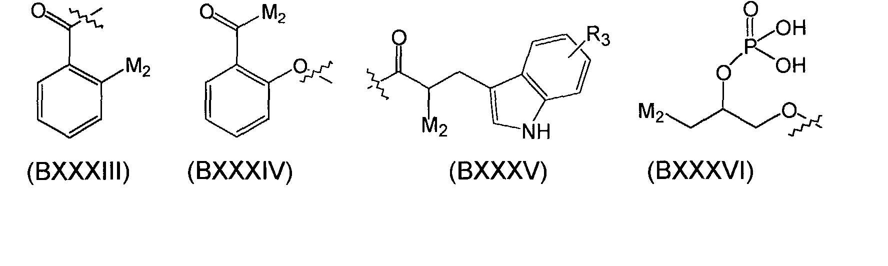 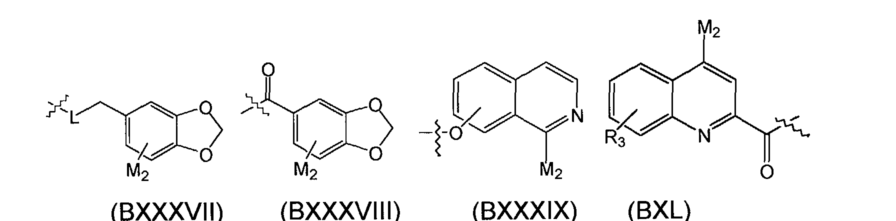 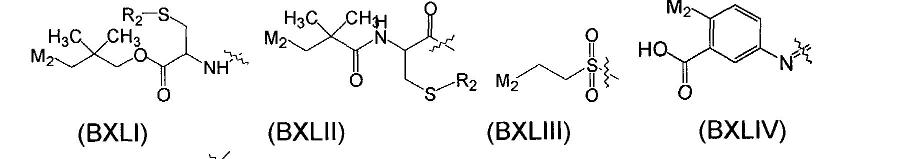 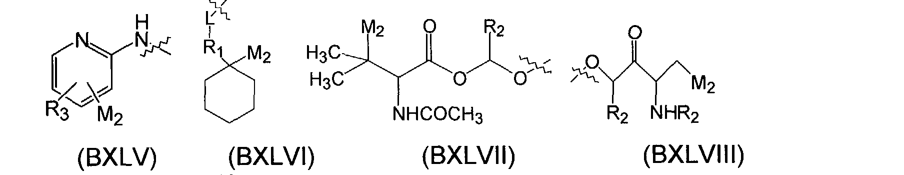 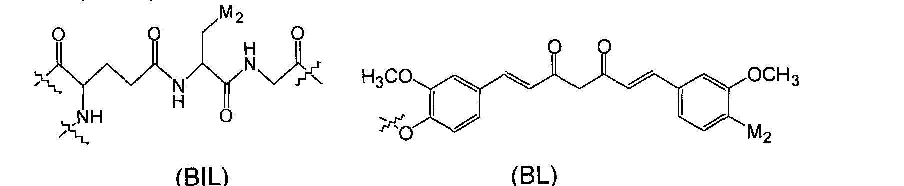 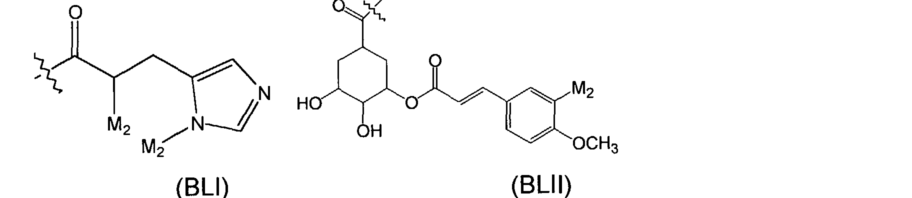 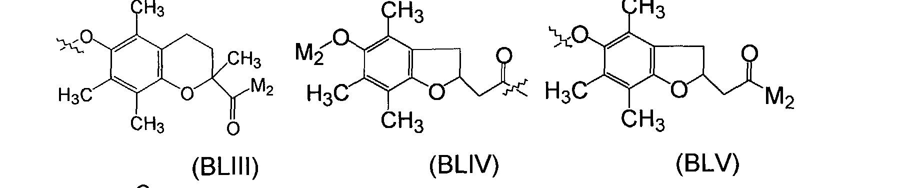 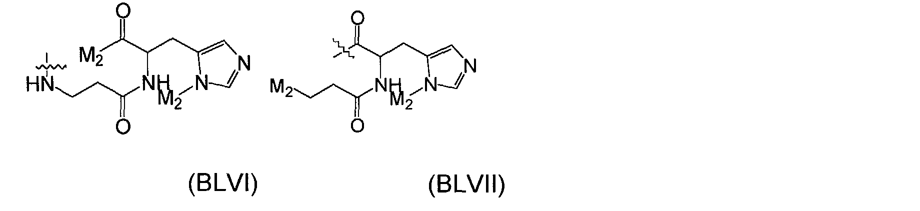 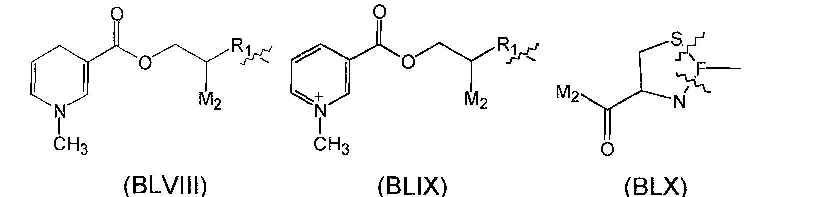 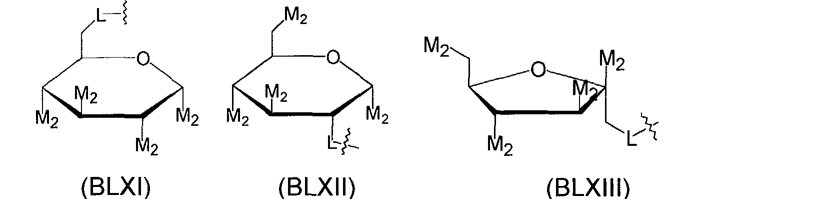 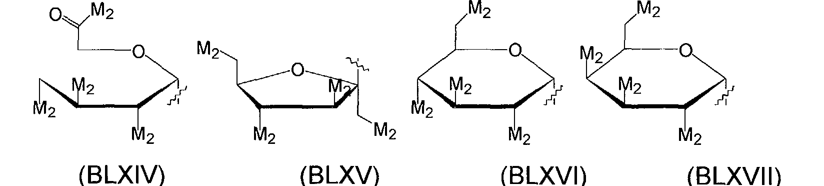 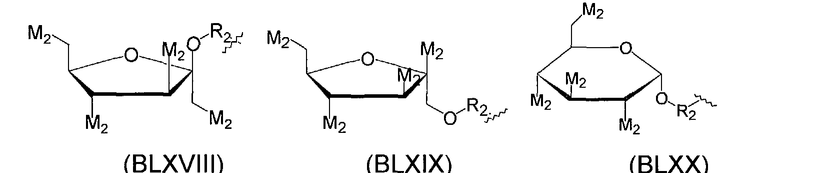 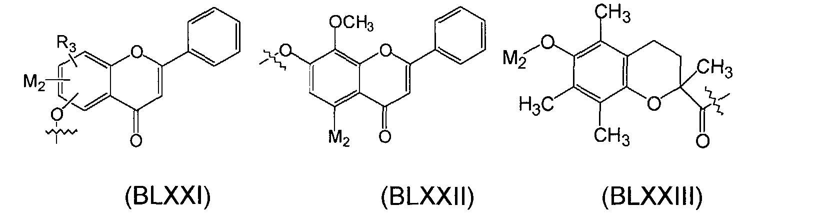
where:
- R₃ is chosen among -H, -OH, -NH₂ -NO₂, -CH₃, -CN, -OCH₃ , -CH₂OH, -COOH, -CHO, -F, -Cl, -Br, -CF₃;
- "L" is an oxygen atom -O-, a sulphur atom -S-, a nitrogen atom -NR₂-, a selenium atom -Se- o a carbon atom -CR₂R₂-;
- W is chosen among -CH=, =N-, -N+(O-)=; n₁ is an integer from 0 to 3; n₂ is an integer from 1 to 6, preferably from 1 to 4; -R₁, R₂, are described as above:
- the dotted line is a simple bond or a double bond; -Ar₁- is an optionally heterosubstituted arylene having from 3 to 7 carbon atoms;
- the undulated line shows where is the bond between -V and the drug -F;
- the bond between -F and -V is hydrolyzable in vivo by metabolic or enzymatic activity and in particular is a carboxylic ester, a carboxylic amide, a glycoside, an acetal, a ketal, an azo compound, an hydrazide, a carbammate, a thioester, a thioamide, a phosphoric ester, a phosphonic ester, a sulphonic amide, a sulphonic ester. Preferably it is a carboxylic ester or a carboxylic amide.

In -T and -V groups, the preferred meanings of -X are the following monovalent radicals -Y_{1,} optionally substituted preferably with nitric acid: 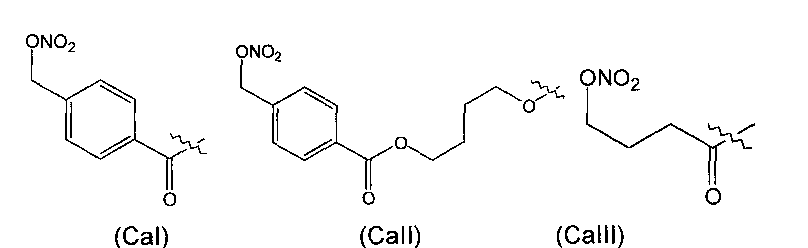 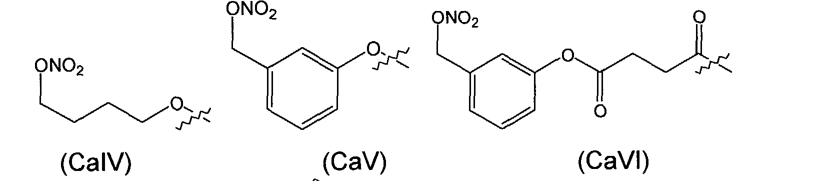 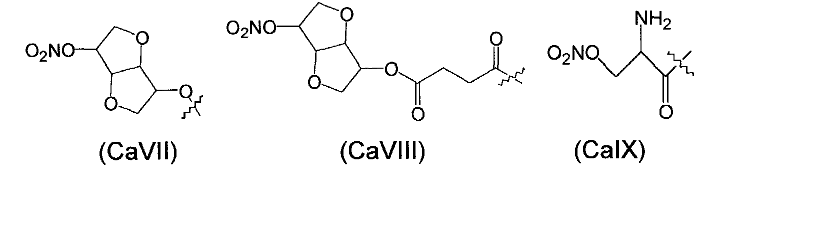 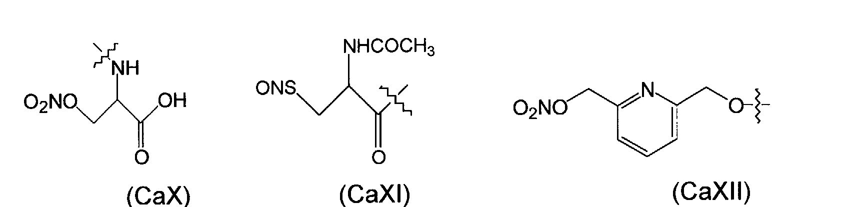 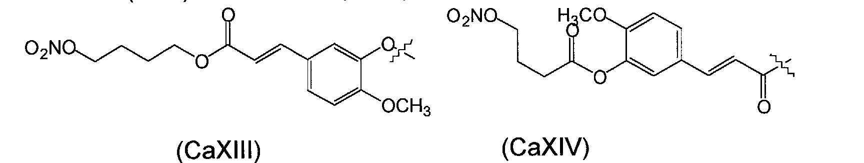 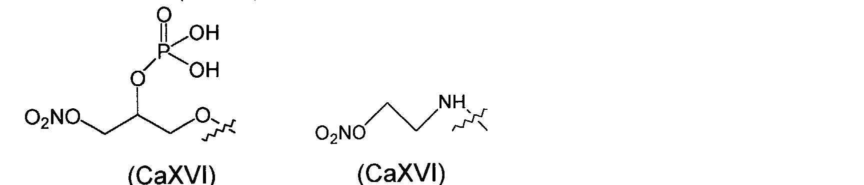 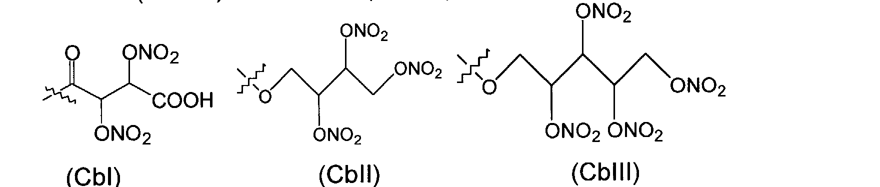 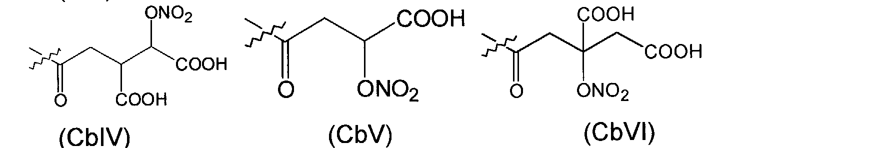 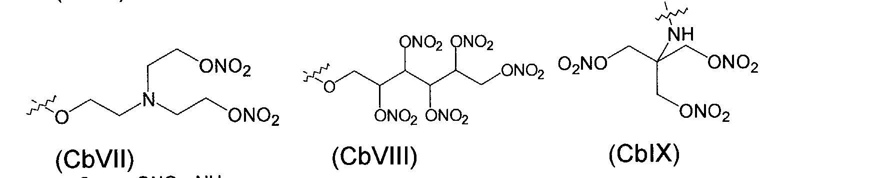 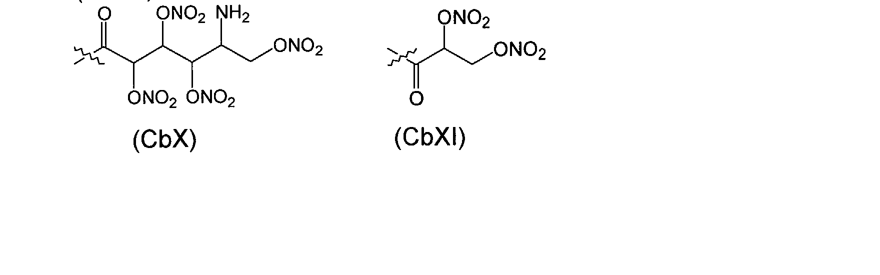 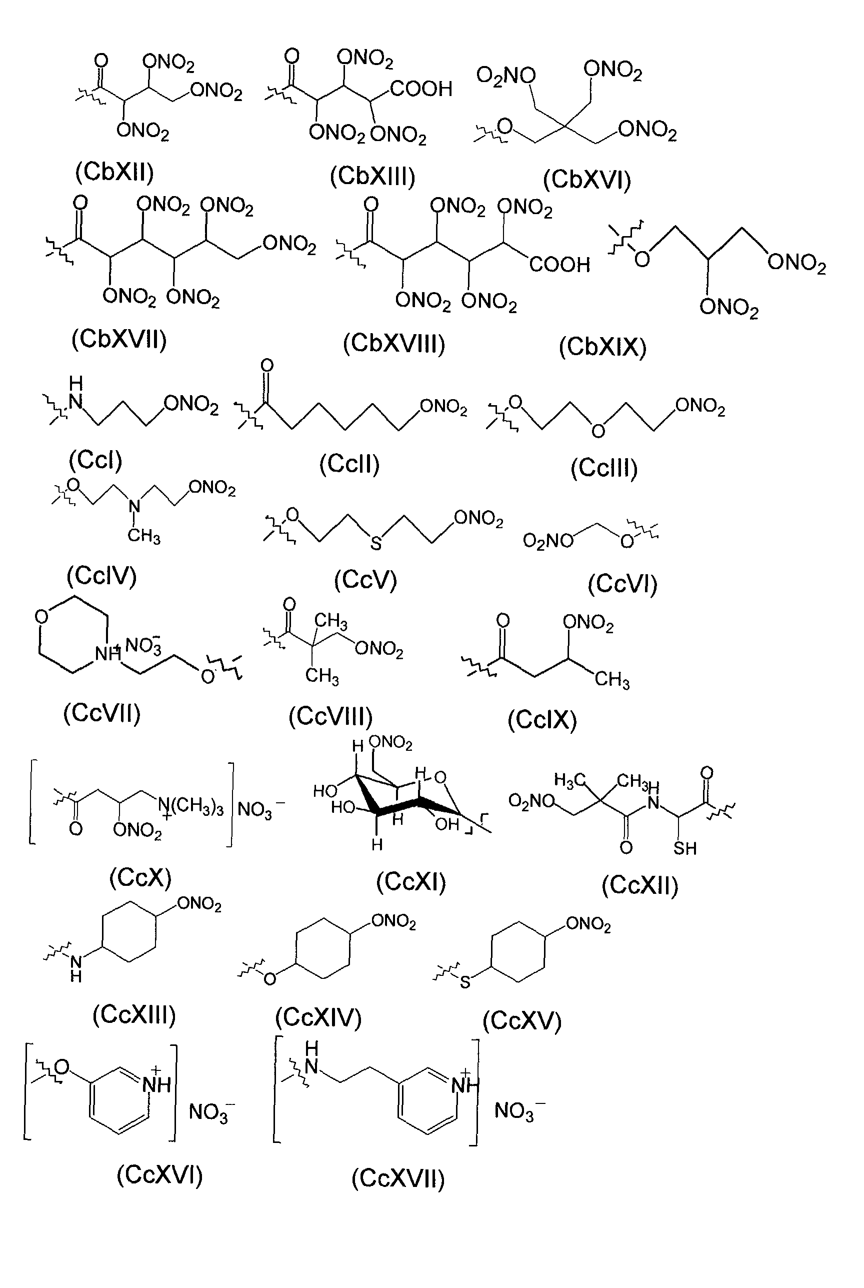 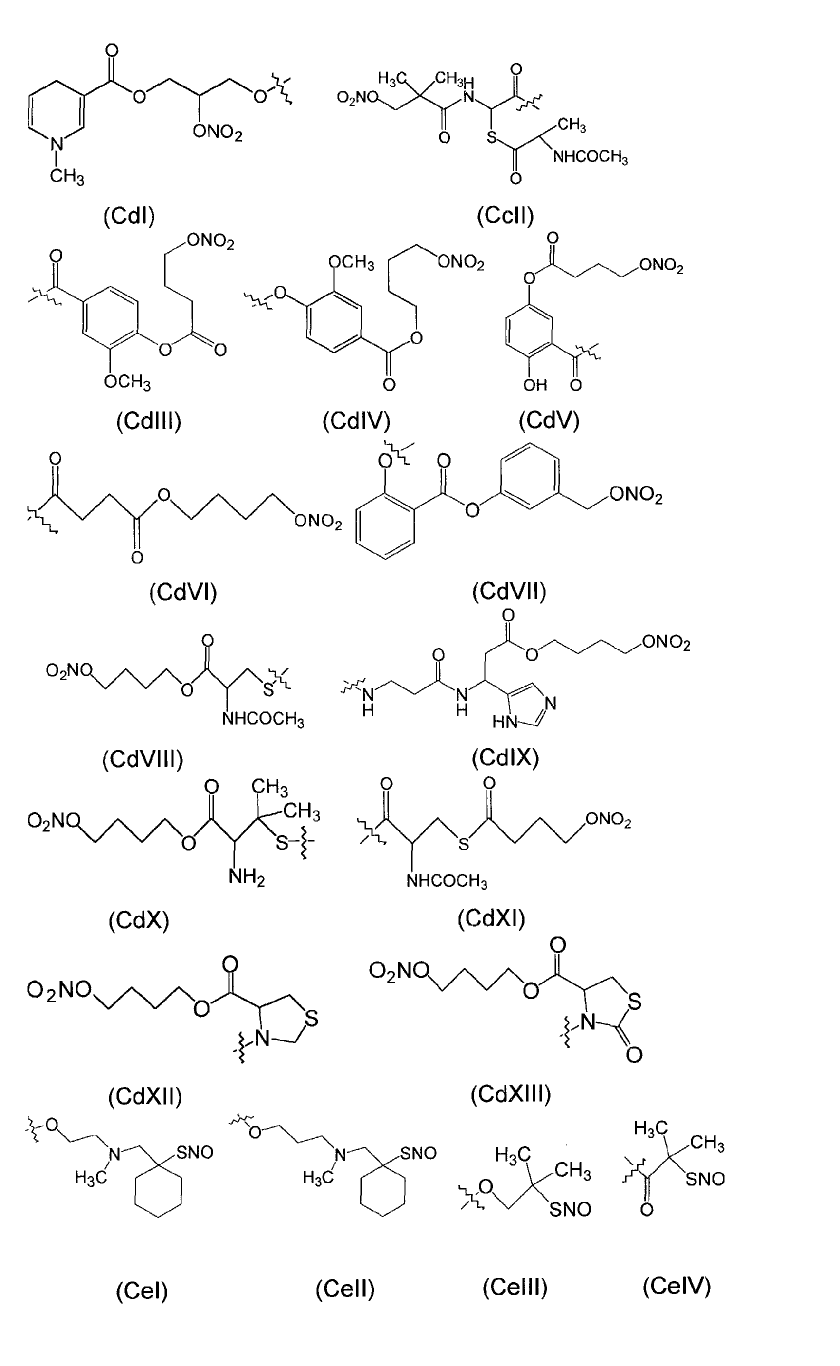 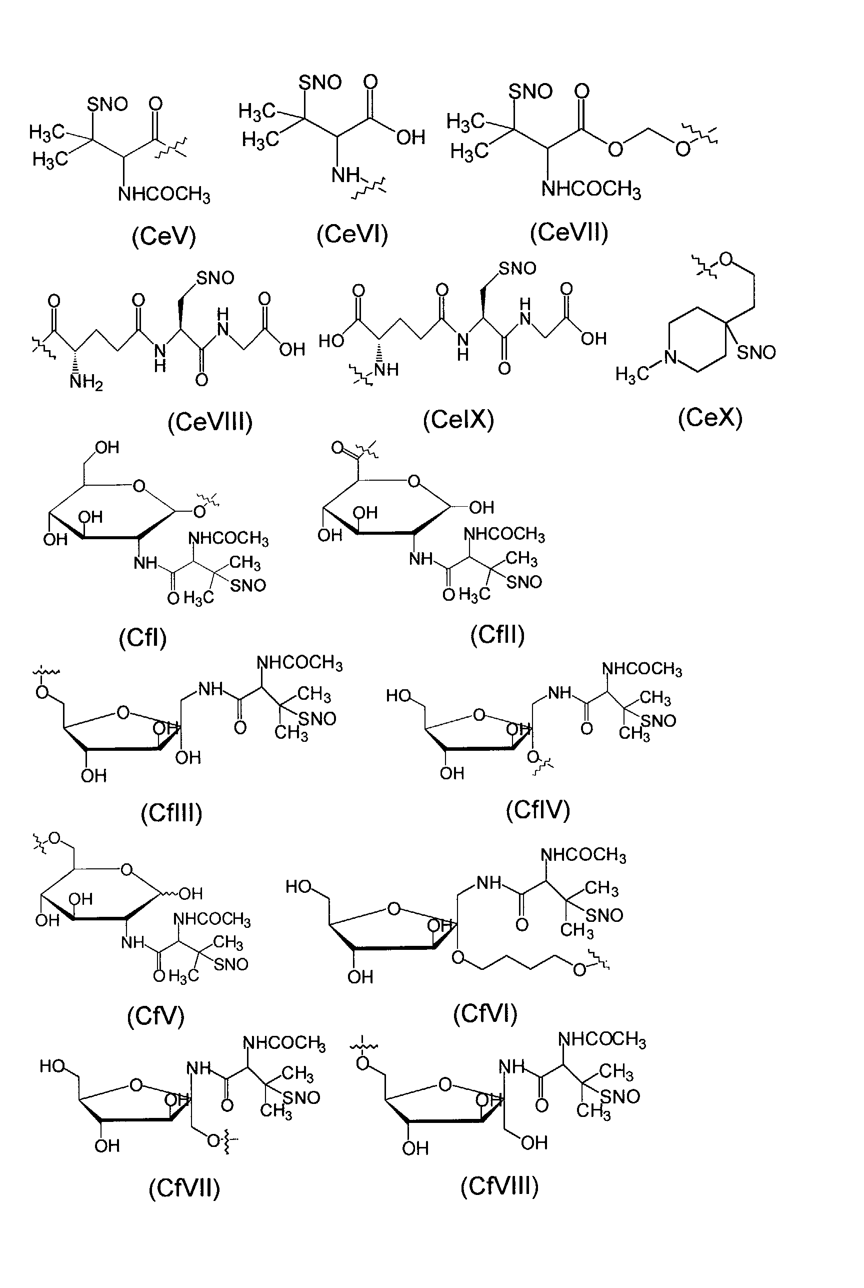 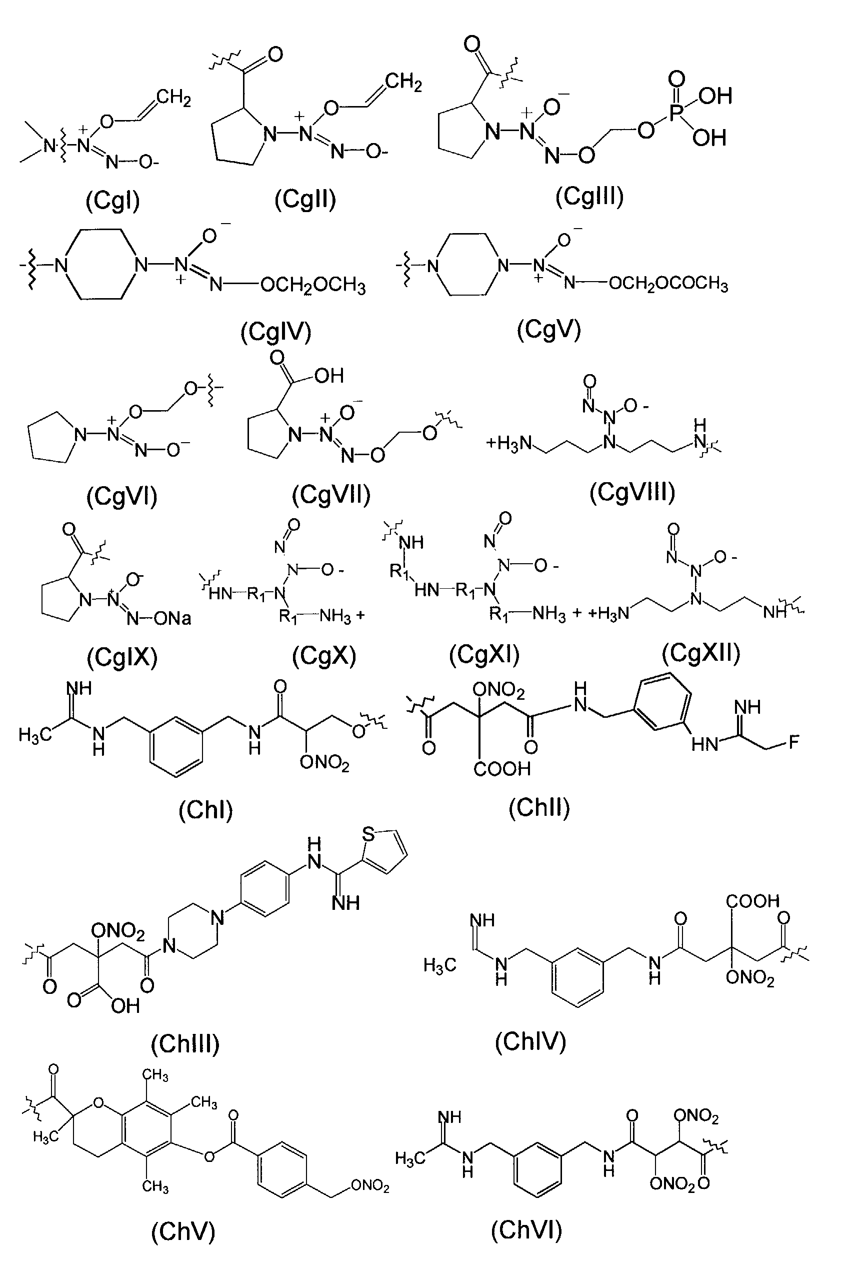 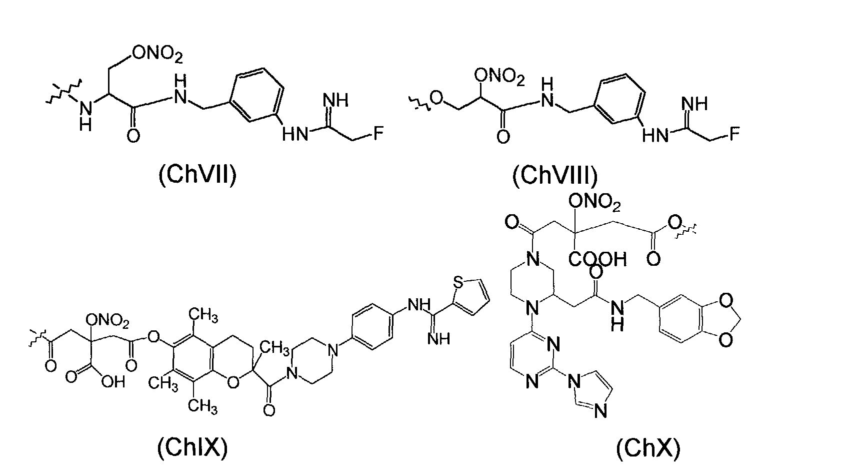 where:
- the dotted line is a simple bond or a double bond;
- the undulated line shows where is the bond between -Y₁ and the drug -F;
- the bond between -F and -Y₁ is preferably hydrolyzable in vivo by metabolic or enzymatic activity and in particular is a carboxylic ester, a carboxylic amide, a glycoside, an acetal, a ketal, an azo compound, an hydrazide, a carbammate, a thioester, a thioamide, a phosphoric ester, a phosphonic ester, a sulphonic amide, a sulphonic ester. Preferably it is a carboxylic ester or a carboxylic amide.

Preferably -X is a nitrate salt (All) in a stoichiometric ratio with F of at least 1:1 and preferably -X is chosen among the following monovalent radicals -Y₂ optionally salified preferably with nitric acid: 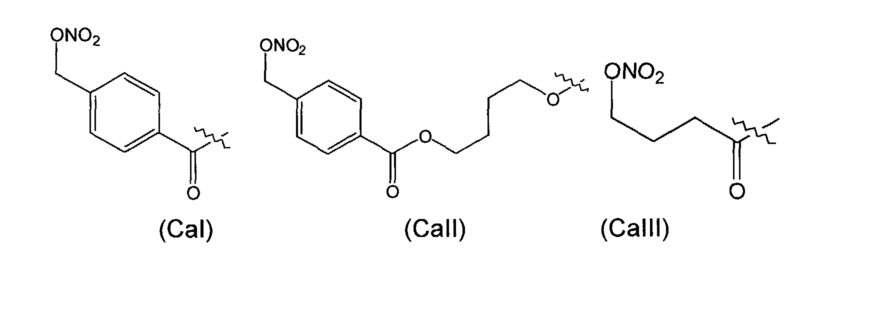 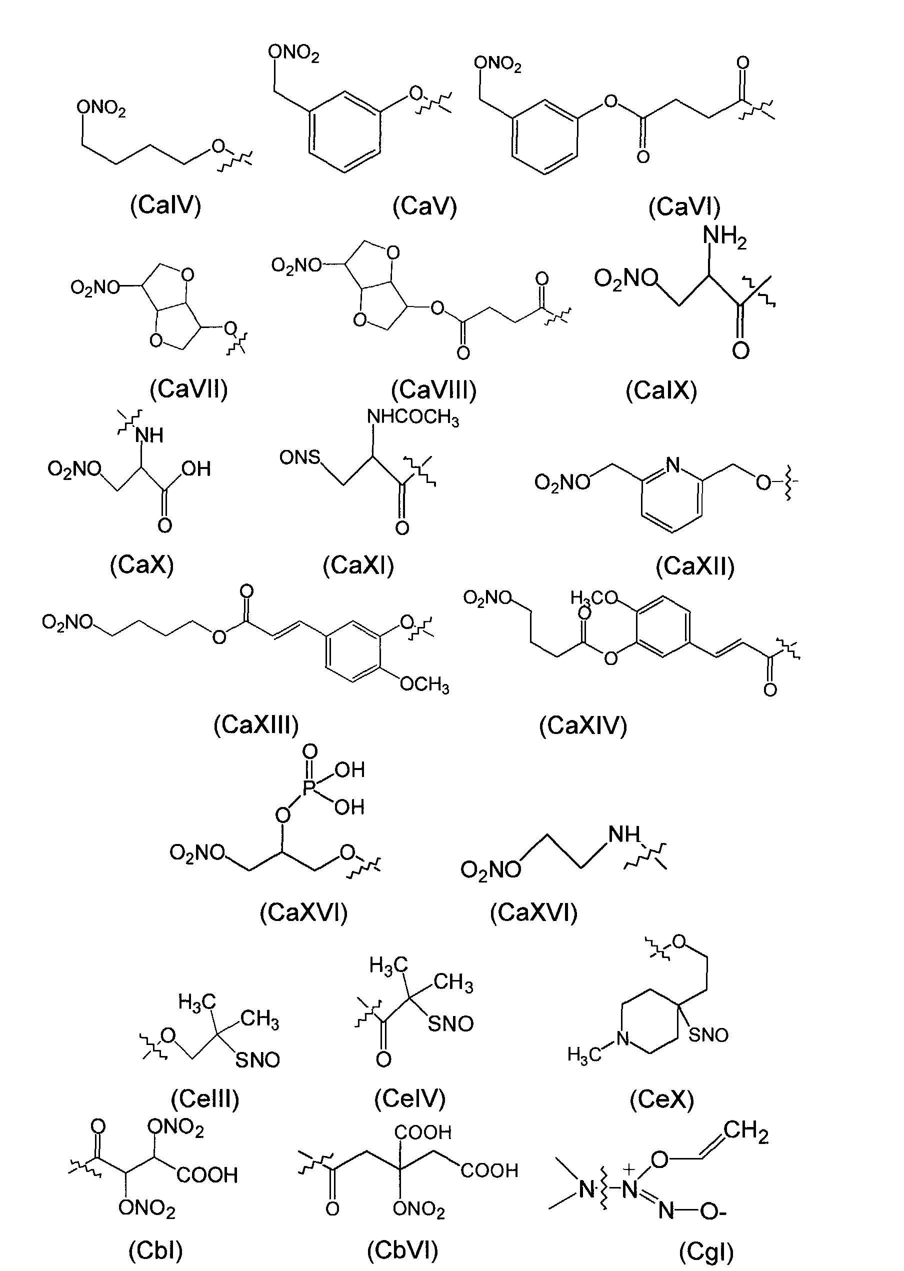 where the bond between -Y₂ and the drug -F, in the point shown by the undulated line, is a carboxylic ester or a carboxylic amide.

More preferably -X is the following monovalent radical -Y₃ (paranitroxymethylbenzoyl): 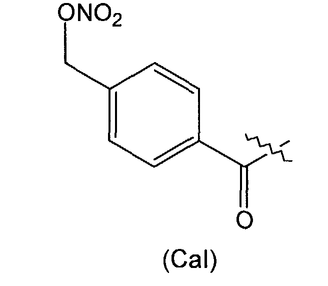 where the bond between -Y₃ and the drug -F, in the point shown by the undulated line, is a carboxylic ester or a carboxylic amide.

F may be bound to M as described above in formulae from (Al) to (AV), or may be bound to T as described above, or may be bound to V as described above in formulae from (BI) to (BLXXIII); more preferably F is bound to -Y₁ as described in formulae from (Cal) to (ChX) and even more preferably F is bound to a -Y₂ e -Y₃ as described in formulae from (Cal) to (CaXVI).

F is chosen among the following 8 groups of molecules (from 1A to 8A) which are identified by their own INN ("International Nonproprietary Name", from "World Health Organization" database) or by their own company code or by their own chemical name.

### GROUP 1A:

ζ₁-tocopherol, ζ₂-tocopherol, δ-tocopherol, ε-tocopherol, η-tocopherol, β-tocopherol, γ-tocopherol, α-tocopherol, 17β-estradiol, 21-acetoxypregnenolone, 6-mercaptopurine, 8α-hydroxy-riboflavin, abetimus, aceclofenac, acemetacin, acetaminosalol, acetoxolone, acexamic acid, aconitine, 3-acetylconitine, actarit, adapalene, adxanthromycin, ajulemic acid, alclofenac, alclometasone, aldesleukin, algestone, alonacic, allopurinol, alminoprofen, amcinafal, amcinonide, amebucort, amelometasone, amfenac, amiprilose, amixitrene, ampiroxicam, amrinone, amtolmetin guacil, anakinra, anirolac, anisperimus, anitrazafen, apafant, apazone, aprepitant, araprofen, artecanin, asimadoline, asobamast, aspirin, ascomycin, ataquimast, atiprimod, atizoram, atliprofen, atrasentan, atreleuton, aurothioglucose, aurothiomalat, aurothiomalic acid, azapropazone, azaspirane, azastene, azathioprine, azimexon, azure A, azure B, azure C, bakeprofen, bamaquimast, balsalazide, basiliximab, batimastat, beclometasone, bendazac, benorylate, benoxaprofen, benzbromarone, benzpiperylone, benzydamine, bepafant, berberine, bermoprofen, betamethasone, betamethasone acibutate, beiwutine, bilirubin, biliverdin, bifeprofen, bimosiamose, bindarit (ELXVII), bosentan, boswellic acids, brasilicardin a, brequinar, bromfenac, bromhexine, bromisoval, bromosaligenin, broperamole, bucillamine, bucloxic acid, bucolome, budesonide, bufexamac, bufezolac, bulleyaconitine, bumadizone, busulfan, butanixin, butibufen, butixirate, butixocort, carbenoxolone, canin, carprofen, celecoxib, chenodeoxycholic acid, chloroprednisone, chloroquine, ciamexon, ciclesonide, cicloprofen, cinaproxen, cinfenoac, cinmetacin, cipemastat, ciproquazone, clamidoxic acid, clidanac, cliprofen, clobetasol, clobetasone, clobuzarit, chlorin e6, chlorophyll, clocortolone, clofenamic acid, clofexamide, clofurac, clometacin, clonixin, clopirac, cloticasone, cloprednol, cloximate, cobanamide, colchicine, colfenamate, coproporphyrin (I, II, III), cormetasone, cortisone, cortivazol, cortodoxone, cridanimod, cyclophosphamide, cyclosporin (A,B,C,D,G), 10-deazaaminopterin, dacopafant, daltroban, dapitant, darbufelone, darusentan, deflazacort, defoslimod, dehydrocholic acid, delmetacin, deloxolone, deltibant, deracoxib, descinolone, desonide, desoximetasone, dexamethasone, dexamethasone acefurate, dexamethasone-21-phosphate, dexbudesonide, dexibuprofen, dexindoprofen, dexketoprofen, dexpemedolac, dextiopronin, deuteroporphyrin IX, diacerein, diclofenac, diclonixin, difenamizole, difenpiramide, diflorasone, diflucortolone, diflumidone, diflunisal, difluprednate, diftalone, diethylhomospermine, dimepranol, dipyrocetyl, dipyrone, diphenyleneiodonium, ditazole, domoprednate, drocinonide, dronabinol, droxicam, duometacin, edatrexate, eltenac, emorfazone, emoctakin, endrysone, enfenamic acid, enolicam, enoxolone, enrasentan, epirizole, eremophyllene a, esculamine, esculentin A, esflurbiprofen, esonarimod, esonarimod, estriol, etersalate, etodolac, 12-epi-scalaradial, etofenamate, etoricoxib, everolimus, ezlopitant, falcinonide, famprofazone, felbinac, fenamole, fenbufen, fenclofenac, fenclorac, fenclozic acid, fendosal, fenflumizole, fenleuton, fenoprofen, fenpipalone, fentiazac, fepradinol, feprazone, figopitant, flavine-adenine dinucleotide, flavonone hydrazone, flazalone, flosulide, flobufen, fluarandrenolide, fluazacort, flucloronide, fludrocortisone, flufenamic acid, flumetasone, flumizole, flunisolide, flunixin, flunoxaprofen, fluocinolone acetonide, fluocinonide, fluocortin, fluocortolone, fluorometholone, fluperolone, fluprednidene, fluprednisolone, fluprofen, fluproquazone, fluquazone, flurbiprofen, fluretofen, flutiazin, fluticasone, folinic acid, forfenimex, formocortal, foropafant, frabuprofen, furaprofen, furcloprofen, furobufen, furofenac, ganaxolone, gentisic acid, gestodene, glaspimod, glatiramer, glucametacin, glycol salicylate, glucosamine, gold thiomalic acid, guaiazulene, gusperimus, halcinonide, halobetasol, halometasone, halopredone, hepatyrix, hemin, hematoporphyrin IX, hematoporphyrin D, hemipyocyanine, hexaprofen, higenamine, hydrocortamate, hydrocortisone, hydroxychloroquine, 3-hydroxyparthenolide, kutkoside, ibufenac, ibuprofen, ibuproxam, icariin, icatibant, ilomastat, ilonidap, imiquimod, indobufen, indometacin, indoprofen, inosine, intrazole, iotroxic acid, iodinin, isatoribine, isofezolac, isoflupredone, isonixin, isoprednidene, isoprofen, isoxepac, isoxicam, israpafant, ivarimod, ketoprofen, ketorolac, ketorolac tromethamine, ketotrexate, kobiin, laflunimus, labradimil, lanepitant, lappaconitine, leflunomide, levamisole, lexipafant, lexofenac, limazocic, licovek, livax, lisofylline, lobenzarit, lobuprofen, lofemizole, lonaprofen, lonazolac, lornoxicam, losmiprofen, loteprednol etabonate, lotifazole, loxoprofen, lubeluzole, mabuprofen, mafosfamide, marimastat, mazipredone, meclofenamic acid, meclorisone, medrysone, mefenamic acid, melittin, meloxicam, meprednisone, mesalazine, meseclazone, mesoporphyrin IX, metacetamol, metanixin, metbufen, methotrexate, methocarbamol, methylprednisolone, methylprednisolone suleptanate, methylcobalamin, methimazole, metiazinic acid, metronidazole, mexoprofen, minocycline, minopafant, mipragoside, miroprofen, misoprostol, mitoxantrone, mizoribine, modafinil, modipafant, mobenakin, mofebutazone, mofetil, mofezolac, mometasone, morazone, morniflumate, moxilubant, mycophenolate mofetil, myoral, N-deacetylranaconitine, N-deacetylfinaconitine, N-deacetyllappaconitine, N-methyl-protoporphyrin IX, nabumetone, naflocort , nagarine, napirimus, naproxen, nemorobucin, nepadutant, nepafenac, nicortonide, niflumic acid, nimesulide, niometacin, nitraquazone, N-desmetylbenzydamine ,nivacortol, nolpitantium, nupafant, octisalate, odalprofen, olsalazine, oleanolic acid, onapristone, oprelvekin, orpanoxin, orazipone, osanetant, oxaceprol, oxametacin, oxaprozin, oxepinac, oxindanac, oxipurinol, oxolamine, oxyfenamate, oxyphenbutazone, paclitaxel, paeony, paracetamol, paramethasone, paranyline, parecoxib, parthenolide, parsalmide, pelubiprofen, pemedolac, penicillamine, pentostatin, pentoxifylline, perisoxal, perizoxal, petrosaspongiolide M, phenylbutazone, piclamilast, picroside I, pidolacetamol, pidotimod, pidotimod, piketoprofen, pilocarpine, pimecrolimus, pimetacin, pipebuzone, piperylone, pirazolac, pirfenidone, piroxicam, pirprofen, pitonakin, pralnacasan, pranoprofen, prasterone, prednazate, prednazoline, prednicarbate, prednisolone, prednisolone 25-diethylaminoacetate, prednisolone sodium phosphate, prednisone, prednival, prednazoline, prednylidene, prefenamate, prifelone, prinomastat, prinomide, probenecid, procodazole, proglumetacin, propacetamol, propyphenazone, proquazone, prospidium, protizinic acid, protoporphirin IX, quercetin, rameswaralide, ramifenazone, ranaconitine, rapamycin, resiquimod, resiquimod, resocortol, rhein, ribavirin , riboflavin, riboflavin monophosphate, riboflavin tetrabutyrate, riboflavin triacetate, triptolide, ridogrel, rimexolone, rimonabant, rocepafant, rofecoxib, romazarit, romurtide, roquinimex, s-adenosylmethionine, salacetamide, salicylamide, salicylamide o-acetic acid, salicylsulfuric acid, salaspermic acid, salnacedin, salsalate, saredutant, scyphostatin, seclazone, secotanaparthenolide, seprilose, sermetacin, setipafant, sibenadet, sirolimus, sitaxentan, sodium hycluronate, sonermin, solimastat, stepronin, stercobilin, sudoxicam, sufosfamide, sulfaloxic acid, sulfasalazine, sulfinpyrazone, sulindac, sumacetamol, suprofen, susalimod, susalimod, suxibuzone, tacrolimus, talmetacin, talnetant, talniflumate, talosalate, tanetin, tanomastat, tarasentan, tauroselcholic acid, tasonermin, tazeprofen, tazofelone, tebufelone, temarotene, tenidap, tenoxicam, teceleukin, tepoxalin, teriflunomide, terofenamate, terprenin, tesicam, tesimide, tetrandrine, tetriprofen, tezosentan, thalidomide, therafectin, thielocin B3, thiazolinobutazone, thiethazole, thymocartin, thymopentin, thymotrinan, tianafac, tiaprofenic acid, tiaramide, ticolubant, tifacogin, tiflamizole, tifurac, tilmacoxib, tilnoprofen arbamel, timegadine, tinoridine, tiopinac, tiopronin, tioxaprofen, tiprotimod, tixocortol, tobramycin , tolfenamic acid, tolmetin, tomoxiprole, tresperimus, triamcinolone, 1-(2-trifluoromethylphenyl) imidazole, 1H-[1,2,4]Oxadiazolo[4,3-a]quinoxalin-1-one, tribuzone, triclonide, trifezolac, triflumidate, trimetrexate, triptolide, tripedane, triptochlorolide, tripdiolide, triptonide, triptolidenol, triptriolide, 16-hydroxytriptolide, tripterine, triptotriterpenic acid B, 12-methoxy triptonoterpene, tropesin, tucaresol, tulopafant, ubenimex, ufenamate, ularitide, ursodeoxycholic acid, ursulcholic acid, urobilin, uroporphyrin (I, III), valacyclovir, valdecoxib, valganciclovir, vedaprofen, vitamin E acid succinate, vitamin E nicotinate, vofopitant, xanoxic acid, xenbucin, ximoprofen, zaltoprofen, zidometacin, zileuton, zoliprofen, zomepirac, α-bisabolol, yunaconitine, wilforlide A, wilforlide B, A-176120, A-197574, A-119435, A-205804, A-241548, A-252444.0, A-293507, A-771726, A-77951, ABS-103, ABT-281, ABT-839, AGIX-4207, AGT-1, ALX-0600, AM-6898A, AM-6927, AM-724, AM-80, AP-1903, APC-2059, APO-501, AR-C73346XX, ARM-434, ATM-027, ATM-027, ATL-2502, BASB024, BASB033, BASB034, BAY-12-9566, BAY-U-3405, BB-2275, BBIC, BB-2827, BB-76163, BCX-1777, BCX-34, BF-389, BMS-184000, BMS-188667, BMS-193884, BMS-214662, BMS-453, Bio-1211, Bio-1272, BX-471, BXT-51072, CARN-1000, C-220, CBF-BS2, CBP-1011, CC-1069, CC-1088, CC-3052, CDB, CDC-501, CDC-801, CDF-571, CGP-28238, CGS-33090A, CHI 621, CK-17, CK-101A, CK-102, CK-103A, CK-112, CK-113, CK-114, CK-115, CK-116, CK-119, CK-120, CK-122, CK-111, CK-117, CK-118, CK-121, CK-123, CK-124, CK-125, CK-126, CK-127, CK-128, CK-130, CK-131, CK-132, CK-133, CK-134, CK-135, CK-136, CK-137, CK-138, CK-139, CK-140, CK-141, CK-142, CK-143, CK-144, CK-145, CK-146, CK-147, CK-148, CK-149, CK-150, CK-151, CK-152, CK-153, CK-154, CK-156, CK-160, CK-163, CK-CLASP-1, COX-189, CPH-82, CP-123369, CPI-1714, CR-3294, CS-670, CSIMM-1, CT-12441, CT-5219, CT-757, CT-767, CT-5269, CY-9701, DA-9601, D-1927, D-609, D-9120, DMP777, DPC-789, DU-6712, DUP-697, DX-88, E-5110, E-5531, E-5564, E-6080, ER-34122, ERL-080, FK-506, FO-5904, FR-167344, FR-153657, FR-228352, FTY-720, G009, GI-245402, GL-701, GPI-1046, GPI-6150, GR-205171, GR-253035, GW-406381, GW-468816, GW-273629, GW-274150, HCL-113, HE-2200, HEP-689, HMR-3408A, HS-378, IC-14, IC-202A, IC-202B, IC-485, ICN-17261, IM-46068, IR-208, IR-208, IR-501, ISA(TX)247, ISIS-104838, ISIS-13280, ISIS-13989, ISIS-16374, ISIS-17044, ISIS-17470, ISIS-18155, ISIS-18669, ISIS-19493, ISIS-2302, ISIS-3082, ISIS-25302, ISIS-27104, IVL-745, J-104132, J-113397, J-113863, JJ-319, JTE-522, K-1115A, KE-298, KE-748, KE-758, KF-20444, KME-4, KT-651, L-167307, L-3002, L-685818, L-732531, L-733725, L-745337, L-761000, L-766112, L-776967, L-778123, L-778736, L-784506, L-784512, L-788123, L-791456, L-818571, LDP-02, LF-173657, LF-160335, LF-160687, LJP-394, UP-712, LK-413, LU-135252, LY-262691, LY-242040, LY-311727, LY-733725, M-40403, MC-1R, MCP-3, MDL-108180, MDL-201112, MK-591, MK-0663, ML-3000, ML-3116, ML-3176, ML-3177, MNA-715, MSP-771, MSP-771 , MX-32, MX-56, MX-68, NBI-5788, NMI-377, NS-398, OB-101, OB-186, OM-89, OM-294DP, ONO-NT-126, ONO-4817, ONO-AE-1329, OP-229-648, OP-2000, PCM-4, PD-057081, PD-098059, PD-159879, PD-56840, PD-980589, PEP-1261, PGE-2946979, PGV-20229, PIXY-321, PMX 622, PNU-168727, PNU-190192, PP1D-1, PS-508, PTI-601, R-115777, R-125224, R-173262, RAD-001, RO-320-1195, RO-32-3555, RPR-130401, RPR-200765A, RS-130830, RS-57607, RVC-588, RVC-589, RVC-593, RVC-595, RVC-596, RVC-597, RVC-598, RVC-599, RVC-600, RVC-601, RWJ-57504, RWJ-67657, RWJ-68354, S-19812, S-2474, S-27609, S-33516, SA-6541, SA-9499, SB-202190, SB-203580, SB-203580, SB-206718, SB-209670, SB-210313, SB-217242, SB-217969, SB-220025, SB-226882, SB-242235, SB-262105, SB-273005, SB-683698, SC-236, SC-26196, SC-77537, SC-299, SC-558, SC-560, SC-57461A, SC-57666, SC-58125, SC-58236, SC-58451, SCH-218157, SCH-23863, SCH-66336, SCIO-469, SCV-07, SDZ-RAD, SDZ MDL-987, SDZ-281240, SDZ ASM-981, SEK-1005, SH-636, SI-3501, SIT-115, SKF-105809, SKF-86002, SP-057, SP-4-1, SP-600125, SPA-1761B, SR-31747, SR-140333A, SU-101, SU-20, T-614, TAK-044, TAK-603, TAN-2474A, TBP-1, TBC-3486, TBC-3342, TMC-2A, TMC-2B, TMC-2C, TMC-95A, TMX-67, TR-9109, TP-10, TRK-530, TS-932, TSL-225, U-19451A, VK-19911, VML-295, VRT-37742, VX-148, VX-702, VX-740 (HMR-3480), VX-765, VX-944, VX-954, WA-8242B, WF-27082B, WIN-64338, WY-14643, Y-39041, WY-48489, Y-700, Z-53, ZD-2315, ZD-6416, ZD-7349, ZD-7349A, ZK-809984, ZK-810000, 1400W

### GROUP 2A:

1-α-hydroxy-vitamin D₂, 17β-estradiol, 22-oxy-calcitriol, 24,28-methylene-1-α-hydroxy-vitamin D₂, 3-allylfarnesol, 4B4-6-21, 4-hydroxy-tamoxifen, A-176120, A-197574, abiraterone, ABT-839, AGIX-4207, AGT-1, alendronic acid, altrenogest, AP-21946, AP-22188, AP-22408, AP-22409, AP-22650, APC-3328, arginylglycylaspartic acid, arzoxifene, avicatonin, bafilomycin A1, BB-2275, BF-389, BMP-17, BMS-214662, butedronic acid, calcifediol, calcipotriol, calcitonin, calcitriol, celecoxib, CGP-77675, CHF-3142, CHF-3150, CHF-3316.01, CLIK-071, CLIK-164, CLIK-166, clodronic acid, clomifene, colecalciferol, concanamycin A, COX-189-, CP-336156, CP-336156, CP-424391, cystatin C, D-609, DCR5, deaminohydroxy-toremifene, deracoxib, desogestrel, DF-1601A, dienogest, dihydroraloxifene, dihydrotachysterol, 21,25-dihydroxycholecalciferol, doxercalciferol, droloxifene, E2-BP, E-5110, ED-71, elcatonin, EMD-84444, enclomifene, ergocalciferol, esomeprazole, esonarimod, estradiol, etidronic acid, etonogestrel, examorelin, 775B, FA-70D, falecalcitriol, FC-1271, flocalcitriol, formebolone, FR-167356, FR-177995, GGTI-2166, GGTI-298, HEP-187, HEP-689, HMR-3339A, HMR-3408A, ibandronic acid, idoxifene, IGF-I/BPII, IM-46068, incadronic acid, inocoterone, ipriflavone, ISIS-104838, ISIS-17044, ISIS-27104, KE-298, KE-748, KE-758, KME-4, KW-8232, L-167307, L-778123, L-788123, lansoprazole, lasofoxifene, leminoprazole, levormeloxifene, levosimendan, lidadronic acid, liposomal isa-13-1, MDL-201000, MDL-201003, MDL-201053, MDL-201112, medronic acid, medroxyprogesterone , mesabolone, minodronic acid, miproxifene, NDE-1003, 4-hydroxy-tamoxifen, N-desmethyl-tamoxifen, neridronic acid, nitromifene, NNC-45-0095, nomegestrol, norelgestromin, norethindrone , norethisterone, NPS-2143, NPS-2143, NSL-1406, NVP-AAK980, NVP-AAK-980, olpadronic acid, omeprazole, OPG, ORG-30659, ormeloxifene, osaterone, oxidronic acid, pamidronic acid, panomifene, pantoprazole, PCM-4, PD-098059, PD-098059, PD-980589, PGE-336646, pipendoxifene, piridronic acid, plicamycin, pralnacasan, progesterone, promegestone, R-115777, rabeprazole, raloxifene, ranelic acid, risedronic acid, RO-26-9228, roxibolone, RPR-130401, RPR-200765A, RPR-200765A, RWJ-67657, RWJ-68354, S-2474, SB-202190, SB-203580, SB-206718, SB-210313, SB-220025, SB-223245, SB-242235, SB-242784, SB-242784, SB-265123, SB-267268, SB-273005, SC-56631, SC-68448, SCH-60663, SCH-66336, SCIO-469, SCRC-2941-18, secalciferol, secalciferol, semparatide, SH-636, simendan, SKF-105809, SKF-86002, solimastat, SR-31747, SUN-E3001, T-614, TAK-778, TAN-2483A, TBP-1, tamoxifen, tenatoprazole, teriflunomide, teriparatide, teriparatide, tibolone, tilmacoxib, tiludronic acid, TJN-135, toremifene, trenbolone, trimegestone, trimgesterone, trioxifene, TSE-424, valdecoxib, vinigrol, vitamin D₁, vitamin D₂, vitamin D₃, vitamin D₃-1-retinate, vitamin D₄, vitamin D₅, vitamin K₂, VK-19911, VX-702, VX-740, VX-745, VX-765, VX-954, WY-47766, XT-238, XT-44, XT-533, XW-630, YM-175, zanoterone, ZG-2807A, zindoxifene, zoledronic acid, zuclomifene

### GROUP 3A:

ABT-594, ADL-10-0101, ALX-0646, 5-bromosalicylic acid acetate, acetaminophen, acetylsalicylsalicylacid, 2-acetylsalicylamido-4-picoline, alendronic acid, alfentanil, alimadol, alletorphine, allylprodine, almotriptan, alniditan, aloxiprin, alphaprodine, alpiropride, ALX-0388, ALX-0646, AM-374, AM-404, aminochlorthenoxazin, aminopropylon, aminopyrine, anandamide, anileridine, anilopam, antipyrine, antrafenine, apadoline, aprepitant, asimadoline, aspirin, avitriptan, benzydamine, benzylmorphine, bezitramide, BCH-2963, BIBN-4096BS, botulinum toxin type A, bremazocine, brifentanil, bromadoline, bucetin, buprenorphine, butacetin, bupivacaine, butinazocine, butorphanol, calcium acetylsalicylate, capsavanil, carbamazepine, carbiphene, carfentanil, carsalam, chlorthenoxazine, cinchophen, ciprefadol, ciprofloxacin, ciramadol, cizolirtine, clodronic acid, clonitazene, clonixeril, cloracetadol, codeine, cogazocine, CP-96345, CP-99994, CP-55940, cropropamide, crotethamide, dapitant, delmopinol, desomorphine, dexoxadrol, dextromoramide, dexmedetomidine, dezocine, diampromide, dibusadol, didemnin B, difenamizole, dihydrocodeine, dihydroergotamine, dihydromorphine, dimenoxadol, dimepheptadol, dimethylthiambutene, dioxaphetyl, dipipanone, diproxadol, dipyrocetyl, dipyrone, divalproex, dolasetron, donitriptan, dotarizine, doxpicomine, E-2078, eletriptan, emorfazone, enadoline, epirizole, eptazocine, epibatidine, ergocornine, ergocorninine, ergocryptine, ergotamine, eseridine, esketamine, ET-142, ethenzamide, ethoheptazine, ethoxazene, ethylmethylthiambutene, ethylmorphine, etodolac, etofenamate, etonitazene, ezlopitant, F-11356, fampridine, fenoprofen, fentanyl, figopitant, filenadol, FK-888, floctafenine, flufenamic acid, flumedroxone, flumexadol, fluoresone, fluoxetine, flupirtine, fluradoline, flurbiprofen, fonazine, fosfosal, frakefamide, frovatriptan, gaboxadol, ganaxolone, GI-87084B, glafenine, GR-73632, GR-82334, GR-203040, GV-150526, GV-228869, GV-213237, GV-196771, GV-217828, GW419458, hydrocodone, hydroxypethidine, HU-210, ibandronic acid, ibazocine, icatibant, ICI-174864, incadronic acid, indomethacin, iprazochrome, isoladol, IS-159, isomethadone, JTE-522, JWH-051, JWH-015, ketazocine, ketobemidone, ketoprofen, ketorfanol, ketorfanol, ketorolac, L-365260, lanepitant, lappaconitine, lefetamine, letimide, levonantradol, levorphanol, levobupivacaine, lidadronic acid, lisuride, lofentanil, lomerizine, lorcinadol, L-732138, L-758298, L-759633, L-759656, L-759787, L-768242, L-775606, LY-235959, LY-274614, LY-303870, LY-334370, LY-344864, LY-334370, MBT, MDL-747216, meperidine, meptazinol, metacetamol, metazocine, metergotamine, methadone, methotrimeprazine, methyldihydromorphine, methysergide, metkephamid, metoclopramide, metofoline, metopon, mexiletine, mimbane, minodronic acid, mirfentanil, MK-801, molinazone, morphine, moxazocine, MR-2266, MT-500, myfadol, myrophine, nabitan, nafoxadol, nalbuphine, nalpitantium, nalmexone, nantradol, naproxen, naproxol, naratriptan, narceine, nefopam, nefopam, nepadutant, nerbacadol, nexeridine, nicocodine, nicodicodine, nicomorphine, nifenazone, nonivamide, norcodeine, norlevorphanol, normethadone, normorphine, norpipanone, NS-398, ocfentanil, OHM3295, olvanil, osanetant, oxapadol, oxetorone, oxitriptan, oxycodone, oxymorphone, pamidronic acid, paracetamol, parsalmide, p-bromoacetanilide, PD-117302, PD-154075, pentazocine, perisoxal, phenacetin, phenadoxone, phenazocine, phenazopyridine, pheniramidol, phenocoll, phenoperidine, picenadol, pidolacetamol, piminodine, pinadoline, pipradimadol, pipramadol, piritramide, pizotyline, PL-017, p-lactophenetide, PNU-109291, pravadoline, pravadoline, prodilidine, prodolic acid, profadol, proheptazine, promedol, propacetamol, propiram, propoxyphene, propofol, proxorphan, quadazocine, R-84760, remifentanil, rimazolium, rimonabant, risedronic acid, rizatriptan, ropivacaine, RP-60180, RP-67580, RP-68651, RPR-100893, RS-127445, S-19014, salethamide, salicin, sameridine, salverine, saredutant, SB-209509, SB-218842, SB-220453, SB-235863, SCH-23390, SCH-50971, SDZ-NKT-343, sergolexole, simetride, SNF-8702, SNF-8814, SNF-8820, SNF-9007, spiradoline, SR-140333, SR-141716A, SR-142801, SR-144528, SR-48965, SR-48968, sufentanil, sumacetamol, sumatriptan, suprofen, talnetant, tetrandrine, thiambutene, tidembersat, tifluadom, tilidine, tiludronic acid, tinoridine, tolpadol, tonaberstat, tonazocine, topiramate, tramadol, trefentanil, triclacetamol, tropoxin, U-50488H, U-62066, U-69593, veradoline, verilopam, viminol, vofopitant, volazocine, xorphanol, WIN-55212-2, zatosetron, zenazocine, zoledronic acid, zolmitriptan, zucapsaicin, ziconotide

### GROUP 4A:

ζ₁-tocopherol , ζ₂-tocopherol, α-tocopherol , δ-tocopherol, ε-tocopherol, η-tocopherol, β-tocopherol, γ-tocopherol, atizoram, 6-azauridine, 8α-hydroxy-riboflavin, acitretin, adapalene, algestone, γ-aminolevulinic acid, amlexanox, anthralin, azaribine, azelaic acid, APC-2059, ASM-981, becaplermin, bergaptene, bexarotene, BMS-185411, BMS-297208, bucladesine, butantrone, calcipotriene, cedefingol, chrysarobin, cioteronel, CMI-392, CP-80633, CPR-1152, cycloheximide, cyproterone, cytoxazone , dapsone, dexamethasone, FR-900520, domoprednate, enazadrem, ER-38930, ER-41666, etretinate, FK-506, H-1305, HU1124, hyaluronic acid, hydrocortisone, IR-502, ISIS-12854, ISIS-18268, L-762943, lactic acid, lexacalcitol, lobophorin A, lonapalene, maxacalcitol, momordin Ic, motretinide, MRE-0094, NAB2, NIP-530, pantoprazole, pauciflorine a , peldesine, pexiganan, prednisolone, prednisone, pyrogallol, R-115866, repifermin, resorcinol, retinoic acid, riboflavin, riboflavin monophosphate, riboflavin tetrabutyrate, riboflavin triacetate, seocalcitol, paricalcitol, RO-201724, SDZ-ASM-981, safingol, SB-216754, SB-235699, SB-332235, SCH-47112, sulfapyridine, tacalcitol, TAK-427, tazarotene, temarotene, tepoxalin, terbinafine , tetroquinone, thalidomide, ticolubant, tioxolone, TP-508, TRK-820, vitamin E acid succinate, VX-497

### GROUP 5A:

4-phenylbutyrate, ablukast, acefylline clofibrol, acetylcysteine, acitazanolast, acreozast, acrivastine, adamexine, adapalene, adibendan, albifylline, albuterol, alclometasone, alifedrine, alinastine, alloclamide, almeterol xinafoate, almitrine, alprogen , altapizone, ambicromil, ambroxol, amebucort, amelometasone, amicibone, amipizone, amlexanox, amphotericin B, andolast, antazoline, apafant, apaxifylline, argatroban, argimesna, arofylline, asmanex, asobamast, astemizole, ataquimast, atizoram, atreleuton, azatadine, azelastine, baclofen, baicalein, bamaquimast, bambuterol, bamipine, barmastine, batebulast, beclometasone, becotide, beloxamide, benafentrine, bencisteine, benproperine, benzonatate, bepafant, bepotastine, betamethasone, bietanautine, bilastine, bimosiamose, binizolast, bitolterol, bromhexine, bromodiphenhydramine, brompheniramine, brovanexine, broxaterol, budesonide, bufenadrine, bufrolin, bunaprolast, butamirate, butaprost, butethamate, butixocort, cabastine, caffeine, calfactant, calcitrol, camonagrel, caramiphen, carbapenem, carbetapentane, carbinoxamine, carbocisteine, carebastine, carmantadine, cartasteine, cefaclor, cefpimizole, cetirizine, cetoxime, chlophedianol, chlorcyclizine, chloropyramine, chlorothen, chlorpheniramine, ciclesonide, ciclotropium, cilomilast, cilutazoline, cimaterol, cimetropium, cinalukast, cinnarizine, cipamfylline, ciproquazone, cirazoline, cistinexine, clemastine, clemizole, climbazole, clobenzepam;, clobenztropine, clobutinol, clocinizine, cloperastine, clopidogrel, closiramine, cloticasone, cloxacepride, codeine, codeine N-oxide, codeine phosphate, colterol, cormetasone, cromakalim, cromoglicate lisetil, cromoglicic acid, cromolyn sodium , cyclexanone, cycliramine, cyproheptadine, cysteine, dacemazine, dacisteine, dacopafant, dalbraminol, daltroban, dametralast, danosteine, dapitant, darodipine, dazmegrel, dazoquinast, decominol, deflazacort, deltibant, dembrexine, denbufylline, deptropine, descarboethoxyloratadine, descinolone, desloratadine, desonide, desoximetasone, dexamethasone, dexamethasone-21-phosphate, dexbrompheniramine, dexbudesonide, dexchlorpheniramine, dexetozoline, dexsecoverine, dextiopronin, dextromethorphan, dibenzonium, difeterol, dihydrocodeine, dihydrocodeinone enol acetate, dimemorfan, dimesna, dimethindene, dimethoxanate, dimetorfan, dioxethedrin, diphenhydramine, diphenylpyraline, diproteverine, divabuterol, docebenone, domipizone, domitroban, doqualast, dorastine, doxaminol, doxaprost, doxepin, doxofylline, doxylamine, dropopizine, drotebanol, duramycin, ebastine, eclazolast, efletirizine, eflumast, elziverine, embramine, emedastine, enefexine, enazadrem phoshate, enofelast, enoxamast, enprofylline, ensulizole, epinastine, epinephrine, eprazinone, erdosteine, esculetin, espatropate, etacepride, etanterol, ethylmorphine, etolotifen, etymemazine, ezlopitant, fenethazine, fenleuton, fenoterol, fenprinast, fexofenadine, figopitant, filaminast, flerobuterol, flezelastine, flixotide, fluazacort, flixoride, flufylline, flumetasone, flunisolide, fluprofylline, fluproquazone, fluticasone, flutropium, fominoben, formoterol, foropafant, fudosteine, furafylline, furegrelate, furoate, gamfexine, genleuton, genistein, glimepiride, guaiactamine, guaiapate, guaietolin, guaifenesin, guaifylline, guaimesal, guaisteine, halometasone, hexopyrronium, heterocodeine, histamine, histapyrrodine, hydrocodone, ibudilast, hydroxychloroquine, hydroxyzine, icatibant, idaverine, idenast, ifetroban, ilonidap, imitrodast, imoxiterol, indanazoline, ipratropium , iproheptine, iralukast, isalsteine, isamoxole, isbogrel, isbufylline, isoaminile, isocromil, isoetharine, isopromethazine, isoproterenol, isothipendyl, israpafant, itazigrel, ketotifen, lanepitant, laprafylline, letosteine, levalbuterol, levcromakalim, levmetamfetamine, levocabastine, levocetirizine, levodropropizine, levopropoxyphene, levopropylhexedrine, levosulpiride, lexipafant, lifarizine, linazolast, linetastine, linotroban, lisofylline, lodoxamide, lofemizole, lombazole, lomerizine, lonapalene, loratadine, loxanast, luteolin, mabuterol, mapinastine, masoprocol, mebhydrolin, mecysteine, medrylamine, melquinast, menthol, mepixanox, mequitamium, mequitazine, meribendan, meropenem, mesna, metaproterenol, metron s, metaterol, methafurylene, methaphenilene, methapyrilene, methotrexate, methylprednisolone, mexafylline, midaxifylline, midazogrel, midesteine, mifentidine, milrinone, milverine, minocromil, minopafant, mipitroban, mizolastine, mizolastine , mizolastine, modipafant, moguisteine, mometasone, mometasone, montelukast, morclofone, morphine sulphate, motapizone, moxaverine, moxifloxacin, moxilubant, nafagrel, naflocort, naminterol, napactadine, naphazoline, narceine, nardeterol, nedocromil, neltenexine, nemazoline, nepadutant, neraminol, nesosteine, nestifylline, nicogrelate, nisbuterol, nitraquazone, nivimedone, noberastine, norastemizole, norgestrel , normethadone, noscapine, nuclomedone, nupafant, nuvenzepine, olopatadine, olpimedone, olprinone, omonasteine, ontazolast, orphenadrine, osanetant, oxabrexine, oxagrelate, oxalinast, oxarbazole, oxatomide, oxeladin, oxolamine, oxomemazine, ozagrel., pamicogrel, paramethasone, pargeverine, pemirolast , pentoxifylline,, perbufylline, phenindamine, pheniramine, phenylephrine , phenylpropanolamine, phenyltoloxamine, phenylbutyrate, pholcodine, piclamilast, picoperine, picumast, picumeterol, pimobendan, pinacidil, pipazethate, pirbuterol, pirmagrel, pirodomast, pirolate, pobilukast, pranlukast, prednisolone, prednisolone farnesylate, prednisone, prenalterol, prenisteine, prenoverine, prenoxdiazine, procaterol, promethazine, propentofylline, propiverine, proxicromil, pseudoephedrine, pumafentrine, pyribenzamine, pyrilamine, pyroxamine, pyrrobutamine, quazolast, quifenadine, quiflapon, quillaic acid, quinotolast, racemethorphan, racephedrine, ramatroban, radolmidine, raxofelast, repirinast, resiquimod, revatropate, revenast, ridogrel, rimiterol, rilopirox, rimonabant, rispenzepine, ritolukast, rocastine, rocepafant, rociverine, roflumilast, rolafagrel, rolipram, rotoxamine, rupatadine, salbutamol, salmeterol, salmeterol xinafoate, salmisteine, samixogrel, saredutant, sarpogrelate, satigrel, scopinast, secoverine, seratrodast, seretide, setipafant, sevitropium mesilate, siguazodan, siltenzepine, sintropium, sivelestat , somantadine, sophocarpine , spirofylline, stacofylline, stepronin, sudexanox, sulotroban, sulukast, sunagrel, suplatast, tagorizine, talnetant, tasuldine, taurosteine, tazanolast, tazifylline, taziprinone, telmesteine, tematropium , temelastine, temiverine, tenidap, tepoxalin, terbogrel, terbucromil, terbutaline, tetrazolast, texacromil, thenaldine, theophylline, thonzylamine, tiacrilast, tibenelast, ticolubant, tiflamizole, tilbroquinol, tiliquinol, tinazoline, tiotropium, tioxamast, tipepidine, tiprinast, tixanox, tobramycin , tobuterol, tolafentrine, tolazoline, tolpropamine, tomelukast, torbafylline, tranilast, tranilast, traxanox, triamcinolone, triamcinolone acetonide, trifenagrel, tripolidine, tritoqualine, troleandomycin, tulobuterol, tulopafant, tyloxaphol, ularitide, uridine 5'-triphosphate, urushiol, verlukast, verofylline, vinpocetine, vofopitant, xamoterol, xanoxic acid, zafirlukast, zanamivir, zaprinast, zardaverine, zileuton, zilpaterol, zindotrine, zinterol, zipeprol, zolamine, A-277249, A-64077, A-76745, A-78773, A-78773, A-79175, A-85761, AA-861, ABT-761, AH-21132, AMG-126737, APC-366, AR-C68397, AR-C89855, AR-D111421, AWD-12-281, AWD-12-343, AY-0068, BA-679-BR, BAY-19-8004, BAY-19-9996, BAY-K-8644, BAY-U-3405, BAY-U-9773, BAY-X-1005, BAY-X-7195, BAY-X-9773, BBR-2221, BBR-2222, BIIC-1996, BIIL-284, BIIX-1, BIO-1211, BI-RM-270, BIRZ-227, BM-113, BM-16.2115, BOM-1006, BRL-52974, BPC-15, BW-755C, BWA-78U, BY-217, CDP-840, CDS-90910, CE-2072, CE-1037, CGH-2466, CGH-2466, CGP-45715A, CGP-49823, CGP-69669A, CGP-73400, CGS-21680, CGS-25019C, CH-13584, Cl-1018, CI-1044, CMI-392, CMI-977, CP-105696, CP-195494, CP-195543, CP-199330, CP-199331, CP-220629, CP-288886, CP-293121, CP-80633, CP-99994, CPX, CS-560, CS-615, CT-5219, CT-747, DAX, D-22888, D-4396, D-4418, D-58, DCF-987, DF-1681, DMP-777, DPC657, E-25, E-3040, EF-40, EPI-2010, ER-27319, ETH-615, F-1322, FK-224, FK-355, FK-888, FPL-55712, FR-134043, FR-184280, FUB-593, GR-213487, GT-261163X, GW-215864, GW-250495, GW-311616A, GW-328267, HMR-4011, HMR-4011A, HP-3, ICI-198615, ICI-204219, ICI-211965, ICI-D2138, ICI-200355, IDEC-152, IL-4-DM, INS-365, INS-37217, IPL-4088, IPL-576092, IVL-745, J-104129, K-5993, KAA-276, L-615919, L-648051, L-649923, L-651392, L-689065, L-658758, L-691816, L-697198, L-699333, L-708780, L-733321, L-739010, L-746530, L-743986, L-826141, LAS-31025, LDP-977, LM-1484, LM-1507.NA, LY-170680, LY-171883, LY-213024, LY-210073, LY-223982, LY-233469, LY-255283, LY-264086, LY-292728, LY-293111, LY-303870, MAFP, MDL-105212A, MDL-105172A, MDL-108207DA, MEN-10627, MEN-11467, MJ-451, MK-0476, MK-0591, MK-0679, MK-571, MK-679, MK-886, MKS-492, ML-3000, ML-3176, MOL-294, MPB-07, MPB-07, MRS-1523, NA-00226A, NA-00226B, NCS-613, NCS-630, NCS-631, NCS-632, NIP-520, NKP-608C, NPC-18915, OC-229-648, ONO-LB457, ONO-LB448, ONO-1078, ONO-4057, ONO-5046, ONO-6818, OR-1958, ORG-20241, ORG-30029, PA-1806, PF-5901, PF-10042, PNU-142731A, PR-001337, R-113281, R-133663, R-146225, R-840, RK-0202, REV-5901, RG-12,525, RG-12525, RO-20-1724, RP-23618, RP-64966, RP-66153, RP-73401, RPR-106541, RPR-106541, RPR-114597, RPR-122818, RPR-132294, RPR-132703, RS-601, RS-635, RU-486, RWJ-2048, RWJ-63556, S-1319, S-1674, S-2474, SB 683698 , SB-201146, SB-209247, SB-205312, SB-207499, SB-209247, SB-223412, SB-227122, SB-244525, SB-332235, S-36496, SC-41930, SC-53228, SC-50605, SC-51146, SC-53228, SCH-205528, SCH-217048, SCH-351591, SCH-55700, SCH-60059, SIL-13RA2-FC, SKF-104353, SHF-104493, SKF-106203, SKF-94836, SKF-96231, SM-15178, SR-140333, SR-144190, SR-48212, SR-48968, SYN-1390, SYN-1396, T-0757, T-2585, TA-270, TAK-661, TASP-V, TBC-1269, TBC-3342, TBC-3586, TEI-8362, TEI-9874, TMC-120B, TP10, TMK-688, TRK-851, TR-14531, TR-9109, U-50488-H, U-62066E, U-875302, V-11294, VB-5122, VML-530, VUF-5087, VUFB-19363, VUF-K-8707, VUF-K-8788, WAY-121006, WHI-P180, WHI-P97, Y-27632, YM-38336, YM-44778, YM-49244, YM-976, ZD-0892, ZD-2138, ZD-4407, ZD-6021, ZD-8321, ZM-230487, ZM-274773, YM-976

### GROUP 6A:

4-amino-salicylic acid, 5-hydroxy-omeprazole, aceglutamide, acetorphan, acetoxolone, aldioxa, alemcinal, alizapride, alkofanone, alosetron, amiglumide, amoxicillin, arbaprostil, asperlicin, azacitidine, azasetron, azithromycin, bemesetron, benexate, benzotript, bergenin, bisfentidine, bromcresol green, bromcresol purple, budesonide, buzepide, carbenoxolone, casofekamide, catechin, cetraxate, cilansetron, cimetidine, cinitapride, cisapride, clarithromycin, dalcotidine, darenzepine, darifenacin, deboxamet, deprostil, devazepide, devazepide, dexloxiglumide, didanosine, difenoxin, dimeticone, diphenoxylate, dolasetron, donetidine, dosmalfate, ebrotidine, ecabapide, ecabapide, ecabet, egualen, enprostil, esaprazole, esomeprazole, etintidine, exisulind, fabesetron, famotidine, famotidine, fedotozine, fenoctimin, galdansetron, gefarnate, goodyeroside A, granisetron, idremcinal, indisetron, irsogladine, irsogladine, isotiquimide, itasetron, itopride, itriglumide, lafutidine, lagatide , lamivudine, lamtidine, lansoprazole, lavoltidine, leminoprazole, lerisetron, letrazurile, lidamidine, lintitript, lirexapride, loperamide, lorglumide, loxiglumide, lozilurea, lupitidine, lurosetron, mebiquine, meciadanol, 6-mercaptopurine, mesalamine, mesalazine, mesalazine., methylnaltrexone, metoclopramide, metronidazole, mexiprostil, mifentidine, mirisetron, mosapride, mosapride, mosapride, nepaprazole, niperotidine, nizatidine, nolinium, norcisapride , norcisapride, nuvenzepine, octeotride, olsalazine, olsalazine, omeprazole, omeprazole sulphone, ondansetron, ornoprostil, osutidine, oxmetidine, palonosetron, pantoprazole, phenolphthalein, piboserod, pibutidine, picoprazole, pifarnine, pipratecol, pirenzepine, pirenzepine, plaunotol, porfimer, pranazepide, proglumide, prucalopride, pumaprazole, rabeprazole, racecadotril, ramixotidine, ramosetron, ranitidine, rebamipide, remiprostol, renzapride, ricasetron, ridogrel, rifaximin, rioprostil, rispenzepine, rofleponide, rosaprostol, rotraxate, roxatidine, saviprazole, siltenzepine, simeticone, sofalcone, somatostatin, sopromidine, spiriprostil, spiroglumide, spizofurone, sucralfate, sucrosofate, sufotidine, sulamserod, sulfasalazine, sulisatin, tarazepide, tazofelone, tegaserod, tegaserod, telenzepine, telenzepine, tenatoprazole, teprenone, thalidomide, ticalopride, timoprazole, tinidazole, tiotidine, tizanidine, tolimidone, tomoglumide, tranexamic acid, trapencaine, triletide, triletide, trimoprostil, tritiozine, tropisetron, troxipide, tuvatidine, ufiprazole, uzarin, venritidine, zaldaride, zaltidine, zatosetron, zolenzepine, zolenzepine, zolimidine, ε-acetamidocaproic acid, 5-ASA-CYS, A-71378, A-71623, ABT-229, AD-2698, ADL-8-2698, APC-2059, AR-R15849, AT-61, ATL-2502, AU-006, AU-130, AU-224, AU-413, AU-461, BIMU-8, BILN-303-SE, BXT-51072, BXT-51108, BY-112, CA-1028, CAM-CAM-1028, 1481, CDC-801, CDP-571, CHF-17454, CJ-11974, CI-988, CP-212454, CP-122721, CR-1392, CR-1409, CR-1505, CR-2345, CR-2622, CR-2945, CR-2194, DA-3934, DA-6034, DA-9601, DA-9601, DZ-3514, E-3620, EB-3810, EM-574, FK-480, FK-1052, FR-127519, FR-145715, FR-180102, FR-182024, FR-193879, FR-208419, G-009, GR-205171, GM-611, GS-332, GW-5823, GW-7178, H199, H-335/25, HC-62, HC-70II, HC-74, HP-0310, ICM-3, IQM-95333, ISIS-2302, ISIS-14803, ISIS-22023, IT-9302, IY-81149, J-104129, J-106366, JB-9315, JK-1085, KC-11458, L-364718, L-365260, L-368730, L-368935, L-740093, L-754030, LDP-02, LY-206890, LY-219057, LY-246736, LY-315535, LY-316108, MK-329, MKC-733, NABI-3700001, NE-0080, OP-2000, OTC SB-300, P371A1, PA53, PD-134308, PD-135158, PD-138450, PD-140548, PTR-3046, Q-33160A, R-116712, RO-32-6167, RO-48-5695, RP-69758, RP-73870, S-15176, SB-207266, SC-50998, SCH-28080, SCH-33405, SDZ-HTF-919, SHANVAC-B, SK-896, SK-951, SKK-47029, SKF-96067, SKF-97574, SL-65.0102, SLV-305, SPI-447, SR-27897, SR-27897B, SR-58611, T-0632, T-0970, TA-510, TP-1202, TP-680, TR-14035, TS-951, TT-S24, VRT-21493, XYZ-033MP, Y-34867, Y-34959, Y-36912, YH-1885, YJA-20379, YJA-20379-5, YJA-20379-8, YM-022, YM-114, YM-53389, YNS-15P, Z-338, ZNC-2381, Y-905, YNS-15P,

### GROUP 7A:

17β-estradiol, 1-docosanol, 3-allylfarnesol, 7-morpholinomethyltheophylline, abarelix, acefylline, acetazolamide, alfuzosin, alprostadil, altizide, alverine, amanozine, ambuside, amikacin, amiloride, amitriptyline, apomorphine, arbutin, atosiban, atropine, azosemide, baclofen, bendroflumethiazide, benzthiazide, bethanechol, bexlosteride, botulinum toxin A, bumetanide, butizide, candesartan cilexetil, canrenoic acid, cetrorelix, chloraminophenamide, chlorazanil, chlorothiazide, ciprofloxacin, clenbuterol, clinafloxacin, clindamycin, clofenamide, clopamide, clorexolone, cyclopenthiazide, cyclothiazide, dantrolene, darifenacin, deacetylmoxisylyte, desacetylspironolactone, 6β-idrossi-7a-tiometil spironolactone, desipramine, desmopressin, desogestrel, dicycloverine, dicyclomine, dienestrol, dihydrotestosterone , disulfamide, doxazosin, doxepin, doxercalciferol, doxiverin, drospirenone, duloxetine, dutasteride, eflornithine, emepronium, ephedrine, epitizide, eplerenone, epristeride, estradiol, estriol, ethacrynic acid, ethiazide, ethinylestradiol, ethoxzolamide, etonogestrel, etozolin, fenquizone, fesoterodine, fiduxosin, finasteride, flavoxate, flumethiazide, fluoxetine, flurbiprofen, fosfomycin, furosemide, ganirelix acetate, gatifloxacin, gestonorone, hydracarbazine, hydrochlorothiazide, hydroflumethiazide, hyoscyamine, hyoscine, imidapril, imipramine, inaperisone, indapamide, indometacin, ipratropium, isosorbide, izonsteride, lanperisone, loperamide, losartan, mannitol, mebeverine, mefruside, mercaptamine, mepartricin, mespirenone, mesterolone, methazolamide, methocalcone, methyclothiazide, methyltestosterone, meticrane, metolazone, midodrine, midodrine, moxisylyte, muzolimine, naftopidil, naftopidil, nepadutant, nifedipine, niguldipine, nitrofurantoin, norethindrone , norfloxacin, nortriptyline, ofloxacin, oleandrin, osanetant, osaterone, oxendolone, oxybutynin, oxybutynin, papaverine, paroxetine, perhexiline, phenoxybenzamine, phentolamine, phenylephrine, phenylpropanolamine, pimagedine, pinacidil, piretanide, piretanide, polythiazide, prasterone, prazosin, propantheline, propiverine, prostaglandin E₁, prostaglandin F_{2α}, prostavasin, protheobromine, pseudoephedrine, quinethazone, resiniferatoxin, roxithromycin, salbutamol, saredutant, sevelamer, sildenafil, sirolimus, suplatast, tacrolimus, tacrolimus, talnetant, tamsulosin, teclothiazide, temiverine, terazosin, terbutaline, terodiline, testosterone, theobromine, tibolone, ticrynafen, tolterodine, torsemide, trans-retinoic acid, trazodone, tretinoin, triamterene, trichlormethiazide, trimegestone, tripamide, triptorelin, troglitazone, trospium, turosteride, upidosin, vamicamide , vardenafil, vardenafil, xenoxin-1, xipamide, yohimbine, A-176120, A-197574, ABT-232, ABT-839, ABT-866, ABT-980, AH-9700, AIO-8507L, ALRT-4204, ALRT-4326, AZD0947, AZD5106, BAY-38-9456, BMS-214662, BMS-284756/T-3811ME, BMS-341400, BMY-7378, CDB-4124, Chi-MrIA, CL-385004, CS-891, CV787, DA-8159, DecbSM, DV-182, DWH-146e, E-4010, E-8010, EMD-221829, ESR-1150, FK-176, FK-453, FR-119680, FR-149175, FR-229934, GI-198745, GS-332, GW-419458, GYKI-16084, HP-4, HP-7, HY-770U, IC-351, IM-46068, ISIS-24195, J-811, JTP-20993, K-4610178, K-4610422, KCO-616, KRP-197, KW-3902, KW-7158, L-372662, L-374943, L-757464, L-771688, L-778123, L-780945, L-788123, LG-120746, LG-121071, LG-121104, LU25-109, LU-25-109, LU-302872, MEDI-491, MEDI-501, MEDI-503, MEDI-504, melanotan II, MEN-10627, MK-826-, MX-6, NC-1800, NK-433, NMI-187, NMI-221, NMI-678-11, NNC-45-0781, NS-21, NS-49, NS-8, NZ-419, ONO-5816,ONO-8815, OPC-41061, OPC-51803, Org-30659, PNU-157706, PNU-156765, PNU-156804, PNU-200577, PT-14, R-115777, RCC-36, RCC-36, Rec-15/2615, Rho-TIA, RO-60-0332, RO-70-0004, RPR-100579, RPR-130401, RS-100329, RSD-992, RU-58642, RWJ-38063-, SB-223412, Sch-66336, SGN-00101, SL-5770499, SL-251039, SNA-4606-1, SNAP-5089, SNAP-5399, SNAP-6201, SNAP-6383, SNAP-5540, SNAP-6543, SNAP-6552, SNAP-6991, SNAP-7242, SNAP-7915, SPM-007, SR-144190, SR-48968, SR-49059, SR-59026A, T-1032, TA-1790, TA-1790, TAK-637, TSE-424, UK-343664, UK-357903, VIPGC-NO, VLM-588, VNA-932, VP-365, WAY-133537, WAY-141608, WAY-151616, YM-31758, YM-32906, YM-36117, YM-58790, YM-905, YM-934, Z-15, Z-350, ZD-0947, ZD-6169, MUDE, EDEX, TOPIGLAN, ALPROX-TD, INVICORP

### GROUP 8A:

β sitosterol, α tocopherol, β tocopherol, γ tocopherol, δ tocopherol, ε tocopherol, η tocopherol, α1 sitosterol, ζ₁ tocopherol, ζ₂ tocopherol, α-carotene, β-carotene, γ-carotene, δ-carotene, β-citraurin, γ-sitosterol, β-sitosterol, α-tocopherol, 10-bromopaullone, 10-desacetylbaccatin III, 13-cis-retinoic acid, 16-Methyloxazolomycin, 17-(Allylamino)-17-demethoxygeldanamycin, 2-(2-amino-3-metoxyphenyl)-chromone, 2,2,5,7,8-pentamethyl-6-hydroxy-chromane, 2-hydroxychalcone, 2-methoxyestradiol, 3-allylfarnesol, 3-Aminopyridine-2-carboxaldehyde, 3-deoxysilychristin, 3-hydroxyflavone, 2-phthalimidino glutaric acid, 3,4-didehydroretinoic acid, 3-indolemethanol, 4'-bromoflavone, 4-aminothalidomide, 4-bromothenylguanine, 4-dedimethylaminosancycline, 4-Demethylpenclomedine, 4-hydroxybutirroxymethyl-retinate, 5,6-dimethylxanthenone-4-acetic acid, 5'-hydroxythalidomide , 5-aza-2-deoxycytidine, 5-azacytidine, 5-fluorouracil, 5-hydroxytryptophan, 5-methoxytriptamine, 6-azauridine, 6-gingerol , 6-hydroxymethyl-acylfulvene, 6-mercaptopurine, 6-shogaol , 6-thioguanine, 7-Hydroxycoumarin, 7-hydroxystaurosporine, 9-cis retinoic acid, 9-aminocamptothecin, 9-aminocamptothecin, 9-Hydroxycrisamicin A, 9-nitrobromopaullone, 14-hydroxy-4,14-retroretinol, abarelix, acacetin, aceglatone, acetylharpagide, acetyldinaline, acitretin, acrimarin F, acyfulvene, adefovir, adenine, adozelesin, aeruginosamide, alanine, alanosine, albendazole, aldesleukin, alitretinoin, allantoin, allcis-5,8,11,14,17-eicosapentaenoic acid, allicin, allin, allixin, allopurinol, all-trans retinoic acid, all-trans-4-hydroxyretinol, all-trans-4-hydroxyretinoic acid, all-trans-4-oxoretinol, all-trans-4-oxo-retinoic acid, almuheptolide A, alpha-amyrin, alpha-curcumene, alpha-linolenic-acid, alpha-pinene, alpha-terpinene, altretamine, alsterpaullone, altretamine, amamistatin A, amentoflavone, amifostine, aminoglutethimide, aminopterin, anguillosporal, ampelopsin, amphidinolide T, amrubicin, amsacrine, amygdalin, anastrozole, ancestim, ancitabine, anecortave, anethole, angiopoietins, angiostatin, anhydrovinblastine, annamycin, annomuricin D, annopentocins, anserine, anthocyanins, anthocyanosides, anthranilic acid, antimycin A1, antimycin A3, antineoplaston A10, anvirzel, apigenin, apigetrin, apiin, apiole, aplidine, apocyanin (A, B, C, D), apomine, arabinogalactan, arbutin, arcyriacyanin A, arginine, arglabin, ariculatin, asimin, asimicin, asiminocin, asmarine B, asperuloside, astacin, astaxanthin, astragalin, avrainvillamide, axillarin, azacitidine, azafrin, azaserine, azidodideoxyguanosine, baicalein, baptigenina, batimastat, bengamide, bergapten, beta-alethine, betaine, berberine, beta-ionone, betulin, bexarotene, bicalutamide, bifendati, bilobalide, bilobols, biochanin A, biotin, biricodar, bisantrene, bisnafide, bixin, bizelesin, bohemine, borneol, bornyl-acetate, brassicolene, brassinin, brevicollin, bromelain, bromotaxane, bropirimine, bryostatin 1, buserelin, busulfan, busurelin, bullanin, buthionine, butylidene-phthalide, butyric acid, caffeic acid, caffeic acid, caloporoside, calicheamycin, calusterone, calphostin S, camphene, camphor, camptothecin, canavaninelupeol, canpagin, canstatin, cantharidin, canthaxanthin, canventol, capecitabine, capsaicin, capsanthin, carboplatin, carboxyamidotriazole, cardanols, carmofur, carmustine, carnosic acid, carnosine, carnosol, carnosolic acid, carvacrol, carolin (A, B, C), carvone, caryophyllene, caryophyllene-oxide, carzelesin, carzinophilin, carboquone, catechin, catechin, ceftiofur, cemadotin, centaurein, cercosporin, cetrorelix, chavicol, chalcones, cherimoline, chloptosin, chlormadinone, chlorogenic acid, chlorophyll, chloroquinoxaline sulfonamide, chromocarb, chrysanthemaxanthin, chrysin, chrysoeriol, chlorambucil, chlornaphazine, chlorozotocin, cichoric acid, cilengitide, cineole, cinnamic acid, cipemastat, cirsimaritrin, cirsineol, cis-inochiresinol, cisplatin, citral, citric acid, citrusine, cladribine, clofarabine, cnicin, cnidilide, cobalamin, combretastatin, combretastatin A4, combretastatin A4 phosphate, conophylline, contortrostatin, conyferyl alcol, coporang, cordycepin, cortivazol, cotara, coumarin, coumestrol, crellastatin A, CRM-51005, crocetin, cryptophycin, cryptophycin-1, cryptophycin-52, cryptoxanthin (α, β, γ, δ), curacin A, cuscenol B, curcumin, cyanidin, cyanidin 3 galactoside, cyanidin 3 glucoside, cyanidin 3,5 diglucoside, cyanidin 3-rhamnoglucoside, cyanidin 3-soforoside, cyasterone, cycloartenol, cyclophosphamide, cymene, cystemustine, cytarabine, cytotrienin IV, daidzein, dacarbazine, daidzin, damnacanthal, daphnezomine G, daphnodorin A, datiscetin, daunorubicin, decitabine, declopramide, defosfamide, dehydrodidemnin B, delphinidin, delphinidin 3,5 diglucoside, delta-3-carene, delta-7-sterols, 5,7-dichloroemodin, demecolcine, denileukin diftitox, denopterin, depsipeptide, desmethyleleutherobin, desmopressin, desoxyepothilone a, desoxyepothilone B, cis-2'-deoxy-2'-fluoromehtylene-cytidine, dexamethasone, dexormaplatin, dexrazoxane, detirelix, diazaphilonic acid, diaziquone, dibromodulcitol , diethylnorspermine, diethylstilbestrol monophosphate, diflomotecan, dihydroxycalcitriol, dimefline, dimesna, diosmetin, diosmin, discodermolide, discorhabdin P, diphyllins, docetaxel, dolastatin-10, dolastatin-15, dolostatin, doxifluridine, doxil, doxorubicin, dromostanolone, echinacoside, echinenone, ecteinascidin-757, ecteinascidin-743, edatrexate, edelfosine, edeine, edotreotide, eflornithine, elacridar, elemene, eleutherobin, elinafide, ellagic acid, emfilermin, emitefur, emtricitabine, emodin, endostatin, eniluracil, enloplatin, enocitabine, epicatechin, epigallocatechin, epigallocatechin-3-O-gallate, epipachysamine E, epirubicin, epitiostanol, epothilone (A, B, C, N-oxide, E, D), eptaplatin, equol, erbstastin, eriodictyol, eruboside-b, esculetin, estragole, espelicin, estramustine, ethanidazole, etoglucid, etoposide, etoposide phosphate, etretinate, eugenol, eupatorin, eudistomin H, exatecan, exemestane, exisulind, fadrozole, falcarindiol, fagopyrin, falnidamol, farnesylthiosalicylic acid, fenretinide, ferulic-acid, feruloyl-4-hydroxycinnamoylmethane, fialuridine, finrozole, fisetin, flavin-adeninin-8a-hydroxy-riboflavin, flavine-adenine dinucleotide, flavone-8-acetic acid, flavopiridol, flavoxanthin, floxuridine, fluasterone, fludarabine, fludarabine phosphate, fluoropyrimidine, fluorouracil, flutamide, fluvirucin B2, folic acid, folinic acid, formamicin, formestane, formononetin, fosfestrol, fraxinol, fucoxanthin, fulvestrant, fumagillol, fumagillol chloroacetylcarbamate, fustin, galangin, galarubicin, gallic acid, gallocatechin, galocitabine, gamma-terpinene, ganirelix, gardenin (A, B, C, D, E), geldanamycin, gemcitabine, gemfibrozil, genistein, genistein-7-phosphate, genistin, genkwanin, geldanamycin, gentisic acid, geraniol, gimeracil, goniodonin, ginkgolide A, ginkgolide B, glufosfamide, glutathione, glutethimide, glucose, 1-glucosamine , 2-glucosamine , 6-glucosamine, 2-galattosamine, 1-fructosamine, 2-fructosamine, glyceollin, glyceollin-II, glycine, glycitein, glycitin, gnidimacrin, goserelin, gossypol, griseolic acid, halimide, harmine, harmaline, harmalol, harman, halofuginone, heliquinomycin, herbimycin A, hemiasterlin, hepaxanthin, hesperetin, hesperidin, heteroxylan, hexestrol, hispidulin, histamine, histidinol, homoharringtonine, hycamtin, hypericin, 7,7'-dichlorohypericin, hydrocaffeic acid, α-hydroxyfarnesylphosphonic acid, hydroxymethylacylfulvene, hydroxyurea, 5-hydroxynoracronycine, hycamtin, hyperin, hyperoside, hypocrellin (A, B), hispidospermidine, human endostatin 6-49, idarubicin, ilatreotide, ilomastat, imatinib, imexon, imidacrine, improsulfan, indanocine, indirubin, indole-3-acetic-acid, inosine, inositol, intoplicine, ipriflavone, iproplatin, irigenin, irinotecan, irisquinone, irofulven, iseganan, isatoribine, isochlorogenic acid, isoeugenol, isofraxidin, isoliquiritogenin, isopimpinellin isoquercetin, isoquercitrin, isorhamnetin, isorosmanol, isovitexin, iturelix, justicidin A, kaempferol, kahalalide F, karenitecin, ketotrexate, krestin, kynurenic acid, kushenol (B, H, E, K, N, M), kuraninone, kosamol A, kuraridin, labiatic-acid, lactacystin, ladirubicin, lapachol, lavendustin, I-carnosine, ledoxantrone, lentinan, leridistim, letrozole, leucocyanidin, leucotropin, leucovorin, leuprolide, leuprorelin, leurubicin, liarozole, licocoumarone, licoflavanone, licochalcone A, lignin, limonene, linalol, linarin, linoleic-acid, liquiritigenin, lisofylline, lobaplatin, lobradimil, lometrexol, lonidamine, losoxantrone, luminacin D, lupeol, lurtotecan, lutein, luteolin, luteolin-7-glucoside, luzindole, lycopene, lycobetaine, lycophyll, lycoxanthin, macrocarpin A, malvidine 3 glucoside, malvidine 3,5 diglucoside, mammastatin, mannomustine, marimastat, maslinic-acid, maspin, mechlorethamine, megestrol, melatonin, melengestrol, melphalan, menadiol, menogaril, menthone, mepitiostane, mesna, meshima-ex, methotrexate, 1-methoxyagroclavine, 3-methoxyquercetin, miboplatin, midostaurin, mifepristone, migrastatin, miltefosine, mirostipen, mispyric acid, mitalactol, mitobronitol, mitoguazone, mitolactol, mitomycin C, mitotane, mitoxantrone, mivobulin, mofarotene, mopidamol, morin, motretinide, myo-inositol, myrcene ,myroridin K, myricetin, myristicin, 14-methylpentadecanoic acid, 16.methyllinoleic acid methyl ester, N-(2 hydroxyethyl)-retinamide, N-(2 hydroxyphenyl)-retinamide, N-(2-carboxyphenyl)retinamide, N-(4-carboxyphenyl)-retinamide, N-(4-carboxyphenyl)-retinamide, N-(ethyl)retinamide, N-(4-etoxycarbonylphenyl)-retinamide, N-(4-hydroxyphenyl)-retinamide, N-(4-hydroxyphenyl)-retinamide-O-glucoronide, naamidine A, N-acetyl-5-hydroxytriptamine, naringenin, naringin, nedaplatin, nemorubicin, neohesperidin, neplanocin A, neparensinol B, nepetrin, N-hydroxymethyl-thalidomide, nicardipine, N-hydroxyphthalimide, nicotinamide, nilutamide, nimustine, nitracrine, nobiletin, nolatrexed, nonataxel, nordihydroguaiaretic acid, noscapine, novembicin, O-6-benzylguanine, octreotide, oleanolic-acid, oliverine, olomoucine, oltipraz, onapristone, oncocidin A1, oprelvekin, orientin, ormaplatin, orotic acid, oroxylin, oteracil, oxaliplatin, oxostephanine, ozogamicin, paclitaxel, pachystaudine, pancratistatin phosphate, paeciloquinone A, pantothenic acid, pantothenic acid, p-coumaric acid, pavetannin B1, p-cymene, panorex, pectolinarigenin, pelargonidin, pelargonidin 3 glucoside , pelargonidin 3,5 diglucoside, peloruside a, peltatins, pemetrexed, Penicillamine, Pentacea, pentamidine, pentetreotide, pentostatin, peonidin, perifosine, perillyl alcohol, petunidin, phenamet, phenesterine, phenylahistin, phenylbutyrate, phloretin, phloroglucinol, phosphatidyl-choline, phthalascidin, p-hydroxybenzoic-acid, phytic-acid, pibrozelesin, picibanil, piceatannol, pifithrin-A, pinitol, pirarubicin, piritrexim, pironetin, piroxantrone, podophyllinic acid, podophyllotoxin, porfimer, pratensein, prednimustine, prinomastat, proanthocyanidins, probenecid, procarbazine, progenipoietin G, propionic acid, protocatechuic acid, prunetin, prunellin, psoralen, pteropterin, purpurogallin, pyrazoloacridine , pyridoxine, pyrisulfoxin a, pyroxamide, Q-12713, quercetagetin, quercetin, quercetin -O-β-D-galactoside, quercetin-3-galactoside, quercetin-3-rhamnoglycoside, quercimeritrin, quercitrin, raltitrexed, ranimustine, ranpirnase, razoxane, radicicol, rebeccamycin, reblastatin, repifermin, resveratrol, retinal, retinilhydroxymethylether, retinol, rhamnetin, rhodomycin, riboflavin, riboflavin monophosphate, riboflavin tetrabutyrate , riboflavin triacetate, rhinacanthin (C, D), robinetin, robinin, rohitukine, roquinimex, rosmarinic acid, roscovitine, rosmadial, rosmanol, rosmaridiphenol, rosmarinic acid, rosmariquinone, rubitecan , rubixanthin, rutin, safingol, safrole, sakuranetin, salicylic-acid saponin, salicylihalamide A, sanguinarine, s-allyl-I-cysteine, saponaretin, saponins , sarcodictyin A, sarcophytol A, sardomozide, sargramostim, saskatchewan, satraplatin, scoparin, scopoletin, scopafungin, scutellarein, schizophyllan, scytonemin, sebriplatin, secobatzelline A, sedoxantrone, selenocysteine, selenomethionine, sensamide, serotonin, shermilamine D, sigmastanol, silandrin, silybin, silychristin, silydianin, silymarin, silymonin, sinapic acid, sitosterol, sizofiran, sobuzoxane, soblidotin, solasodine, solimastat, somatostatin, sophoricoside, sophoraflavone G, spiroplatin, sparfosic acid, spisulosine, spongistatin, spinencin, squalamine, β-tethymustine, staurosporine, stigmasterol, streptonigrin, streptothricin B, streptozocin, suberanilohydroxamic acid, sulfuretin , sulidac solfone, supidimide, suradista, suramin, syringaldehyde, syringic acid, taglutimide, talaporfin, taiwanhomoflavone A, tamandarin A, tannic acid, tannin, tanomastat, tasonermin, taurine, taurolidine, tautomycin, taxifolin, taxol, tazarotene, tecogalan, tectorigenin, tegafur, teloxantrone, temozolomide, tengeretin, teniposide, tenuazonic acid, terpinen-4-ol, tesmilifene, testolactone, tetrahydrocortisol, tetramethylpyrazine, tetrandrine, tetrocarcin A, tezacitabine, thalidomide, theaflavin, theaflavine, thielavin B, thiamine, thiamiprine, thionine, thyrsiferyl 23-acetate, thioctic acid, thioguanine, thymalfasin, thymol , tipifarnib, tiazofurin, timcodar, tirapazamine, tirazone, tirilazad, tocladesine, tocol, tonkinecin, tokaramide A, tomudex, topostatin, topotecan, torularhodin, trans ferulic acid, trapoxin b, triaziquone, tributyrin, trichostatin (A, B, C, D), tridolgosir, triethylenemelamine, trigonelline , trilostane, trimetrexate, trimidox, triptorelin, triptofordin C2, trolox C, troponin I, troxacitabine, troxerutin, trunkamide A, tryptamine, tryptophan, tubercidin, tubulysin A, tumerone, tumstatin, tyrphostin, ubenimex, umbelliferone, uncarinic acid A, uracil mustard, urethan, ursolic acid, valereinic acid , valeric acid, valrubicin, valspodar, vanillic acid, vanillin, vapreotide, vatalanib, verapamil, verteporfin, vesnarinone, vinblastine, vincristine, vindesine, vinflunine, vinorelbine, violaxanthin, vitamin a2, vitamin E acid succinate, vitexin , vitilevuamide, virulizin, vorozole, wogonin, xanthophyll, xanthotoxin, xanthotoxol, xenomin 1, zacopride, zalcitabine, zeaxanthin, zeniplatin, ziconotide, zingerone, zingibain , zinostatin, zorubicin, 506U78, 5F-203, 6-MCDF, 6-MPG, 776C85, A-007, A-105972, A-176120, A-125800, A-177430, A-177775, A-197574, A-198401, A-228839, A-316023, ABT-518, ABT-627, ABT-839, AC-7700, AC-9301, ACRP30R2, AD-198, AE-941, AG-99, AG-12275, AG-12286, AG-1288, AG-1478, AG-18, AG-2034, AG-2037, AG-3340, AG-3433, AG-494, AG-555, AG-7352, AG-825, AG-879, AGN-190168, AGN-193109, AGM-1883, AGR-129, AH-12, AH-27, AH-30, AH-7, AL-245, AL-6, AM-580, AMD-473, AMD-3100, AMP-404, AN-207, AN-238, APC-8015, APC-8024, AR54-, AR5LAC20, ARH460--23, AS5-2H, AT-5015, AVI-4126, AZD-3409, AZD-6474, B-317, B-429, B-581, B-956, B-1086, BAL-9602, BAM-002, BAY-12-9166, BAY-12-9566, BAY-38-3441, BAY-43-9006, BB-1277, BB-21295, BB-67357, BB-76163, BB-2516, BB-3644, BB-94, BBL22, BBR-2778, BBR-3438, BBR-3464, BBR-3576, BBR-3610, BE-41956A, BE-43547A1, BE-45985X, BE-54238A, BE-55051, BE-56384, BE-56980, BE-60828, BE-67251, BEC2, BGP-15, BIBH-1, BIBX-1382, BIM-46068, BIWA4, BLP-25, BLS-0597, BMD-188, BMP-6, BMS-27291, BMS-181174, BMS-182751, BMS-184476, BMS-184477, BMS-186511, BMS-188797, BMS-200659, BMS-214662, BMS-247550, BMS-247615, BMS-247616, BMS-265246, BMS-275291, BN-80245, BN-80915, BN-80927, BNP-1100, BNP-1350, BNP-7787, BP-04, BP-1, BPHA, BR-630, BSU-1075, BSU-9057, BZA-2B, BZA-5B, C-1027, C-1311, C-75, CB-96, CB-300907, CB-300919, CB-30865, CB-7646, CC-3052, CC-4047, CC-5013, CC-7085, CCI-779, CD-1599, CD-437, CD-2314, CDC-501, CDC-801, CEP-2563, CEP-701, CGP-41251, CGP-48664, CGP-52608, CGP-74514, CGP-79787, CGP-79807, CGS20267, CGS-24592, CGS-27023A, CHAP-31, CHAP-81, CHS-828, CHT-22, CI-1006, CI-1021, CI-1027, CI-1031, CI-1033, CI-1042, CI-958, CI-980, CI-994, CJ-11974, CKD-602, CKD-731, CL-387785, CM101, CM-101, CMA-676, CMB401, CN-706, CN-787, COL-3, CP-1, CP-248, CP-263114, CP-358744, CP-358774, CP-461, CP-609754, CPC-405, CPD-5, CPR-2003, CPR-2005, CPR-3005, CPT-111, CRL-1005, CRL-8246, CRL-8256, CRM-51005, CRM-646-A, CSA, CS-682, CT-052923, CT-17, CT-2103, CT-2584, CT-2584, CTP-37, CV706, CV-787, CWJ-A-5, CYA-246, CYS-3-AMBA-Met, D-2163, D-24241, D-24851, D-609, DAB389IL-7, DB-67, DB-81, DC-TA-46, DDE-131, DDE-261, DDE-313, DDE-380, DE-310, DJS-811, DOX-GA3, DP-242/3-, DP-71, DR-70, DT388-GM-CSF, DTI-015, DTPA-BRE-3, DW-2143, DW-2282, DX-8951F, DZ-3358, E21R, E-350, E-3620, E6-BP, E-7070, E-7869, EB-1089, ED-501, EDP-137, EG009, EKB-569, ELF3, EM-12, EM-800, EMD-1211974, EO-9, EPO-906, ERA-923, ER-34617, ER-35794, ER-38925, ER-68203, ER-68203-00, ERA-923, ES-936, ET-743, ETM-775, F-11782, FB-642, FC-2.15, FCE-28948, FCE-29381, FCE-29771, FD-594, FDUMP, FDU-NOAC, FE-200486, FE-200486,, FE-399, FJ-5002, FK317, FPP-33, FR-143430, FR-182877, FR-186054, FR-901228, FRI-20, FT-ALZ, FTI-2148, FTI-2153, FTI-276, FTI-277, FY21-AA09, GI-198745, G-207, G-3139, GBC-590, GF-120918, GD0039, GEM-231, GEX1Q5, GF-109203X, GFB-111, GFMO-1, GGTI-2166, GGTI-298, GI-129471, GL-331, GLI-328, GM2-KLH/QS-21, GM-95, GMK, GnRH-III, GPI-0100, GR-891, GR-128107, GR-135533, GRN-5384, GSDI-126, GTI-2040, GTI-2501, GTX-006, GV-1301, GVH-313, GW-1843, GW-2059, GW-395058, GW-5181, GW572016, GW-8510, GW-9499, GW-974, H-10, HA-1, HA-14-1, HI-368, HPKCI, HSCS-1, HSPE-7, HSPPC-96, HWL-12, HX-600, HYB-0432, HYB-165, IB-367, IIK7, IB-96212, ICI-164384, ICI-245991, IDEC-Y2B8, IDN-5005, IDN-5109, ILX23-7553, ILX-295501, ILX-651, IM-46068, IM862, IMC-C225, INGN-201, INGN-241, INGN-251, INX-3001, INX-3280, INXC-6295, IRX-2, ISIS-10639, ISIS-12539, ISIS-12884, ISIS-14864, ISIS-15999, ISIS-16009, ISIS-16518, ISIS-16601, ISIS-16609, ISIS-16834, ISIS-17509, ISIS-19387, ISIS-20772, ISIS-21028, ISIS-22783, ISIS-23722, ISIS-23905, ISIS-2503, ISIS-25513, ISIS-27067, ISIS-28313, ISIS-28949, ISIS-3521, ISIS-4189, ISIS-5132, ISIS-5132, ISIS-7597, J-104134, J-104871, J-107088, J-109404, JBT-3002, JF-05-59, JK-1624S02, JKC-362, JM-216, JMV-1802, JTE-522, K-135, K-252a, K-22387, KAN-108308, KAN-104141, KF-22678, KF-25706, KF-41399, KF-58333, KHPC-10, KI-6783, KM-043, KNK-437, KOCH-5, KR-30035, KRN-5500, KRN-7000, KRN-8602, KS-505a, KT-5720, KT-6149, KT-6352, KW-2170, L-377202, L-39, L-452958, L-651582, L-731734, L-731735, L-739749, L-739750, L-744832, L-745631, L-778123, L-779450, L-779575, L-783277, LB-42908, LCS-640, LDI-200, LDP-341, LFM-A12, LFM-A13, LG-120744, LG-120753, LG-190119, LGD-1069, LGD-1550, LM-4108, LMB-2, LMB-7, LMS-83177, L-NDDP, LU-103793, LU-223651, LY-231514, LY-294002, LY-309887, LY-320236, LY-329146, LY-333531, LY-335979, LY-355703, MAG-283, MCC, MC-1473, MDL-101731, MDL-101986, MDL-73811, MDL-73811, MDX-11, MDX-210, MDX-220, MDX-447, MDX-H210, MEDI-503, MEDI-507, MEDI-517, MEN-10710, MEN-11066, MEN-11951, MER-1020DA, MER-WF1726, MG-132, MG98, MGI-114, MGV, MIBG, MK-4, MK571, MK-869, MK-886, MKT-077, MI-1544, MI-1892, MM-36, MMI-270, MPC-7869, M-PGA, MPI-5020, MPV-2213AD, MR-20492, MS-209, MS-247, MS-27-275, MS-275, MS-325, MSI-1857, MTP-PE, MUP-98176, MV9411, MX6, MT965-A, MT965-B, N276-15, NA-22598B1, NAMI-A, NB-506, NB-1011, NBI-3001, NC100150, NCA-0424, NCO-700, NF-062, NF-06307, NG-60, NK-611, NIK-250, NHTS, NLCQ-1, NM-3, NS-398, NSC-314622, NSC-330507, NSC-354258, NSC-614491, NSC-614491, NSC-649488, NSC-655649, NSC-658875, NSC-674495, NSC-676632, NSC-678515, NSC-686288, NSC-693569, NSC-693632, NSC-7103505, NSC-D699127, NU/ICRF-505, NU:UB-31, NU:UB-73, NU-2058, NU-3076, NU-3121, NV1020, NX-1838, NX-211, OC-1012, OC-144-093, ODN-2009, ODN-698, ODN-83, OGT-719, ON-5543, ONO-4007, ONO-8711, ONYX-015, ONYX-838, OPB-3206, ORI-1313, OSI-774, P-4055, PADP, PAM4, PAK-104, PAK-104P, PD-089828, PD-145709, PD-0174073, PD-0183812, PD-090560, PD-098059, PD-128763, PD-153035, PD-158780, PD-160678, PD-166285, PD-166866, PD-168393, PD-173074, PD-173955, PD-173956, PD-180970, PD-183805, PD-184352, PI-88, PI-CA91, PKC-412, PKI-166, PLQ-22, PNU 145156E, PNU-144113, PNU 153429, PNU-157914, PNU-157977, PNU-159548, PNU-166196, PNU-177496, PP-33, PR-39, PR-69, PRO-533, PS-341, PS-724, PS-990, PSC-833, PSP-94, PT-523, PT-624, PTK-787, PX12, P-X-1544, Q-85, QM16-A, QS-21, QW-1624F2-2, R-101933, R-109339, R115777, R-116010, R123942, RB94, RK-0202, RL-0903, RMP-7, RO-09-4889, RO-25-4020, RO-25-6760, RO-25-9716, RO-28-2653, RO-31-7453, RO-31-7453, RO-41-5253, RO-50-4258, RO-65-7674, RP-527, RPR-108518, RPR-108514A, RPR-109881, RPR-115781, RPR-116258A, RPR-130401, RSR13, S-1127, S-16020-2, S179D-PRL, S-20928, S-20929, S-23906-1, S-30372, S-30972-1, S-3304, S-8184, S-836, SAHA, SAM-484, SB 249553, SB-203580, SB-209763, SB-238039, SB-247464, SB-251353, SB-408075, SB 596168, SBAS2, SBR-11-2897, SBR-11-3702, SBR-170-, SB-RA-131012, SB-T-1213, SB-T-1250, SB-T-101187, SC-1, SC-68448, SC-72115, SC-AAD9, SCH-207758, SCH-56396, SCH-58500, SCH-66336, SCHL-705, SD-7, SGI-101, SGN-10, SGN-15, SH-207, SJG-136, SL11047, SL-11093, SN-38, SMT-487, SN-38, SNF-4435C,SQ-67454, SP-1053C, SPC8490, SPD-424, SPIKET-P, SR 2789, SR 27897, SR-271425, SR-271425-, SR29142, SR-29142, SR-45023A, SR-4895, SR-49059, SRL-172, SS-555, ST-1481, STA-BX909, STI-412, STI-481, STI-571, SU-0879, SU-101, SU-1433, SU-1498, SU4312, SU-4793, SU-4942, SU-4984, SU-5204, SU-5212, SU-5402, SU-5406, SU-5416, SU-5477, SU-5614, SU-6668, SU-9516, SU-9902, SW-163E, T12, T-138067, T-2591, T-3782, T-607, T-64, T-67, T-900607, T-904064, T-98475, TA-2516, TA-2620-, TAS-103, TAS106, TER-117, TER-199, TER-286, TG-1031, TIP-19.40, TJN-151, TLC-144, TLC-D-99, TLC-ELL-12, TLK-199, TLK286, TMC-169, TMC-96, TNP-470, TOP-53, TRK-710, TTNPB, TT-2, TT-232, TX-1123, TX-1920, TXD-258, TZT-1027, U-0126, UCN-01, UCS-1025A-, UCT-1072M1, V-93, VGA1102, VLTS-587, VN/85-1, VN/87-1, VNP-20009, VNP40101M, VP-16, VUF-5000, VUF-5434, VX-710, VX-853, WAY-170523, WHI-D11, WHI-D12, WHI-P154, WHI-P273, WIN-61651, WMC-26, WP-631, WP-745, WP-769, WR-1065, WT-126-34, WX-293T, XK-469, XR-11576, XR-3054, XR5000, XR-5944, XR-9576, YH-137, YH-138, YM294, YM511, YM529, YMB-6H9, ZD-0473, ZD-1626, ZD-1839, ZD-2767, ZD-4190, ZD-6126, ZD-6474, ZD-9063P, ZD-9331, ZK-119010, ZK-112993, ZK-191703, ZK-222584, ZK-229561, ZK-230211, ZM-445526, ZNPCF64, IRESSA, NEOQUIN
- F is preferably chosen among the following 8 groups of molecules (from 1 B to 8B) which are identified by their own structural formula or by their own INN ("International Nonproprietary Name" from "World Health Organization" database) or by their own company code or by their own chemical name.

### GROUP 1B:

ABT-963, α-tocopherol, 8α-hydroxy-riboflavin, aceclofenac, acemetacin, actarit, alclofenac, alclometasone, AGIX-4207, ajulemic acid (EL), amebucort, amelometasone, amiprilose, ampiroxicam, amtolmetin guacil, anakinra, anirolac, anisperimus, aprepitant, aspirin (ECI), AR-C73346XX (ECX), ataquimast (ECLX), atiprimod, atizoram, atreleuton, balsalazide (ECXVIII), bendazac, benorylate, benoxaprofen, benzydamine (ELX), bermoprofen, beclomethasone, betamethasone, betamethasone acibutate, bindarit, brequinar, BF-389 (EXLVIII), bromfenac, bucillamine, bucillamine, budesonide, bufexamac, bumadizone, butixocort, CB-2431, CC-1069, CC-1088, CEP-1347, celecoxib (El) and 2 metabolites of it ((EII), (EIII)), chenodeoxycholic acid, chloroquine, ciclesonide, cinaproxen, cinmetacin, clidanac, clobuzarit, clofenamic acid, CGS-33090A, CLX-0921, CLX-120500, CK-135, COX-189, CS-502, cloticasone, CR-3558, cridanimod, cyclosporin A, darbufelone (EXLV), deflazacort, dehydrocholic acid, deltibant, deracoxib (EXIII), descinolone, desonide, dexamethasone (ECXC), dexketoprofen, dexamethasone acefurate, dexamethasone-21-phosphate, dexbudesonide, dexpemedolac, dextiopronin, diacerein, diclofenac (ECXIII), diflunisal, dipyrone, DPC-333, droxicam, erlotinib, esonarimod (XXXIV), etodolac (ECXVI), etofenamate, etoricoxib (EXI), E-5110 (EXLVI), everolimus, ezlopitant, fampridine (ELXI), felbinac, fenbufen, fenleuton, fentiazac, figopitant, flavine-adenine dinucleotide, flobufen (ECXVII), flosulide (EXXII), fluazacort, flufenamic acid, flunisolide, flurbiprofen (ECIV), fluticasone, fluocortin, FR-228352 (EVI), FTY-720 (ECL), ganaxolone, glucosamine (EXXX), ginkgolide B, GR-205171, GW-4459, halometasone, hydroxychloroquine, hydrocortisone (ECVIII), hyperin, ibufenac, ibuprofen (ECXIV), icatibant, ilonidap, indometacin (ECIX), indoprofen, ISIS-104838, isoxicam, ketoprofen (ECXII), ketorolac (EXC), KME-4 (EXLVII), KE-758 (EXXXV), KE-748 (EXXXVI), LL-Z-1640-2, laquinimod, lanepitant, leflunomide (EXXV), limazocic, lobenzarit, L-745337 (EXX), lornoxicam, loteprednol, J-113863 (ELXXX), meclofenamic acid, mesalazine (ECII), meloxicam (ECXV), methotrexate (ECC), 6-methoxy-2-naphthylacetic acid (ELXIV), mofetil, ML-3000 (EVIII), mizoribine, mivotilate, mofezolac, mometasone, moxilubant, mycophenolate mofetil (EXXVIII), mycophenolic acid (EXXIX), MX-68 (EC), nabumetone, naflocort, naproxen (ECV), N-desmethylbenzydamine (ELXIII), nepadutant, nepafenac, niflumic acid, nimesulide (EXV), NS-398 (EXXXIII), olsalazine, oxaprozin, oxolamine, oxisopred, paracetamol (ECVI), paramethasone, parecoxib (EIV), pemedolac, penicillamine, pentostatin, pimecrolimus, piroxicam, PNU-156804, pralnacasan (EXXXVII), prasterone (ECLXII), prifelone, prednisone, prednisolone (ECVII), prednazoline (ECCI), propacetamol, proglumetacin, quercetin, reminertant, resiquimod, rhein, riboflavin, riboflavin monophosphate, riboflavin tetrabutyrate, riboflavin triacetate, ridogrel (ECXL), rimonabant, rofecoxib (EIX), romazarit, rofleponide, roquinimex (ECLXI), RWJ-67657, RWJ-68354, salicylic acid (ECXCI), S-19812 (ECXXX), SB-202190, SB-203580, SB-220025, SCIO-469, sinomenine, sirolimus, sivelestat, solimastat, SP-600125, spiradolene, sulfasalazine, S-2474 (EXXVII), SU-5271 (ELXII), sulindac (ECXI), suprofen, suxibuzone, tacrolimus, talnetant, tanomastat, tauroselcholic acid, tazofelone, tazadolene, tebufelone (ECXXXI), tenidap (ECIII), tenoxicam, tepoxalin (EVII), teriflunomide (EXXIV), thalidomide, tiaramide, ticolubant, tifurac, tilmacoxib (EIV), tilnoprofen arbamel, tiopronin, tomoxiprole, tolfenamic acid, tresperimus, T-614 (EXXI), TBC-1269, TSL-225, TTL-3, triamcinolone, triptolide, ursulcholic acid, UR-8813, ursodiol (ELXV) (EgXII), valdecoxib (EV), vofopitant, VX-745, WHI-P131 (ECLI), Y-39041, zileuton (ECXX), zomepirac, (EX), (EXII), (EXVI), (EXVII) (EXVIII), (EXIX), (EXXI-b), (EXXXI), (EXXXII), (EXXXIII), (ELXX), (EXXXVIII), (EXXXIX), (EXL),(EXLI), (EXLII) (ECLXIII), (ECLXX) (ECLXXI), (ECLXXII), (ECLXXX), (ECLXXXI), (ECLXXXII), (ECLXXXIII), (ECLXXXIV), (ECLXXXV), (ECLXXXVI)

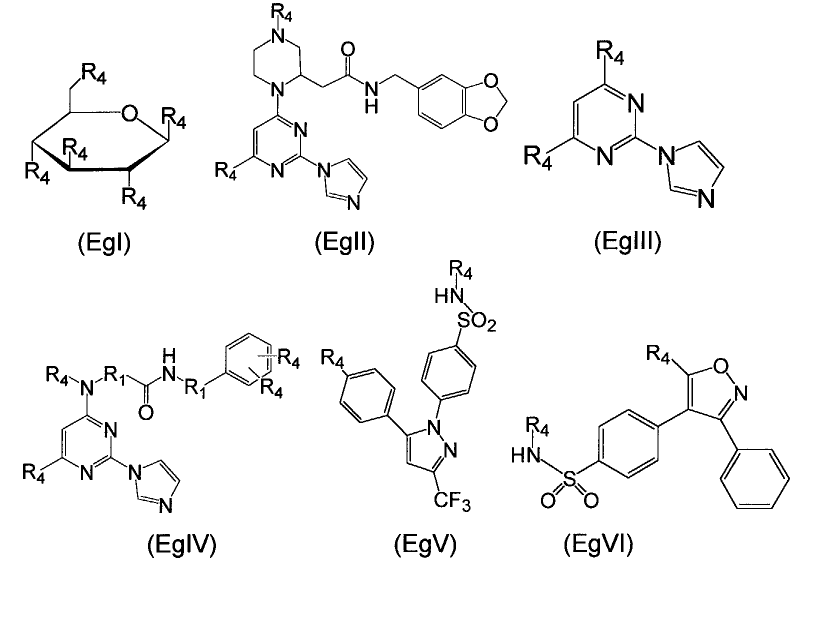

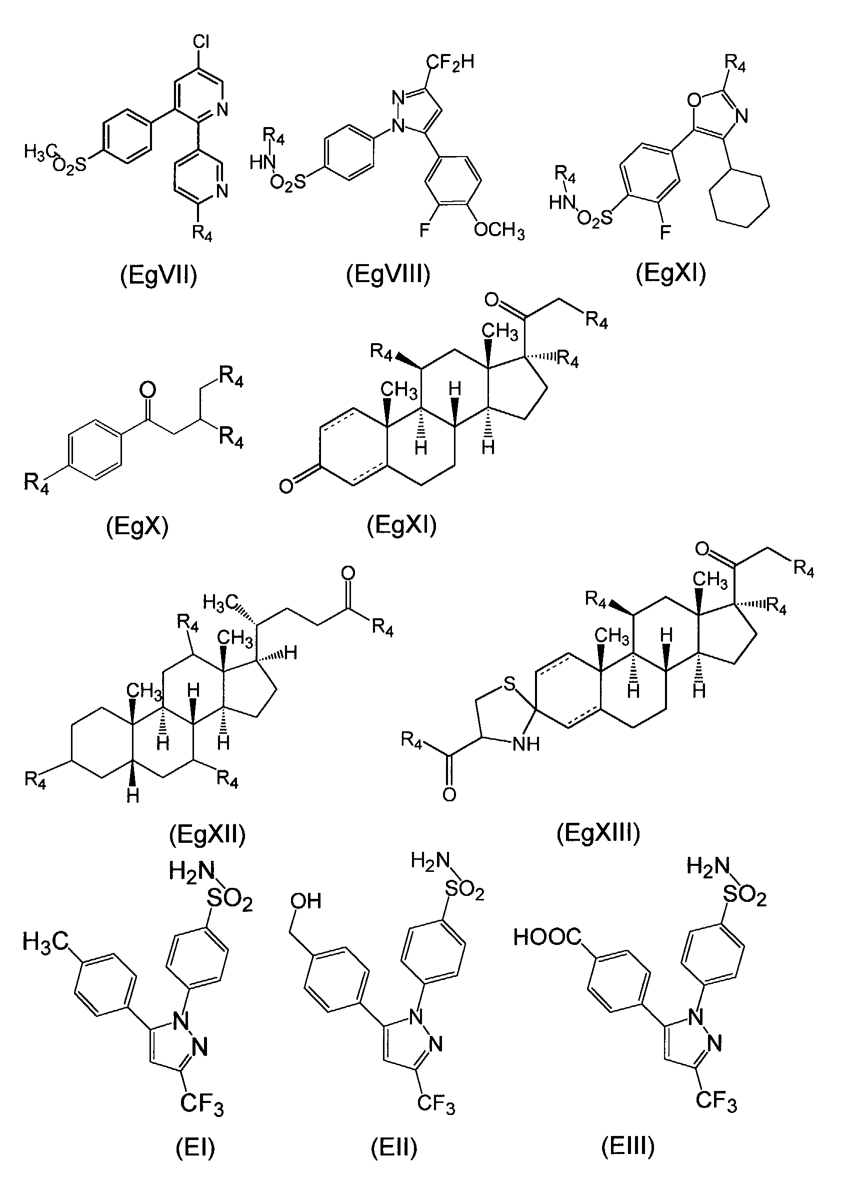

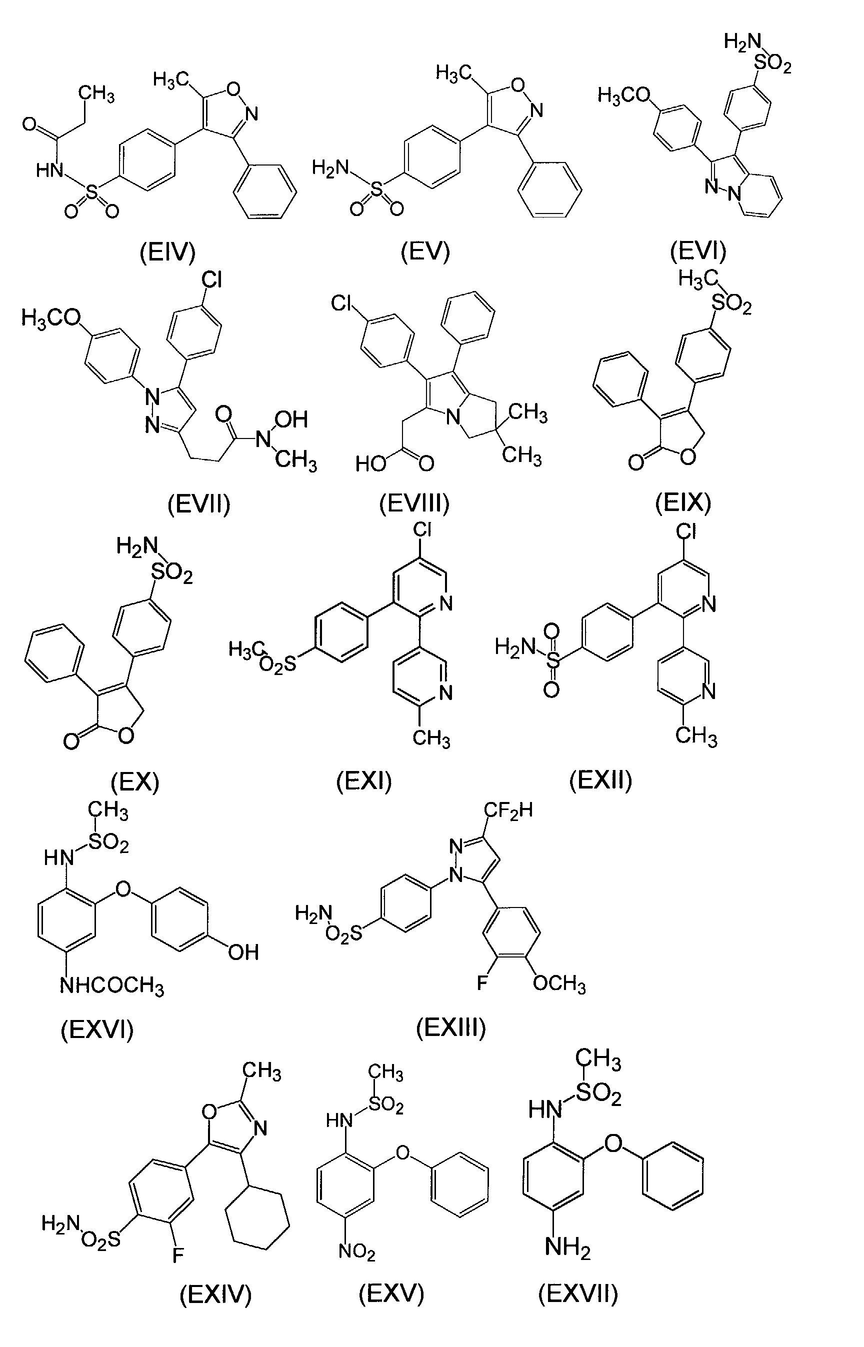

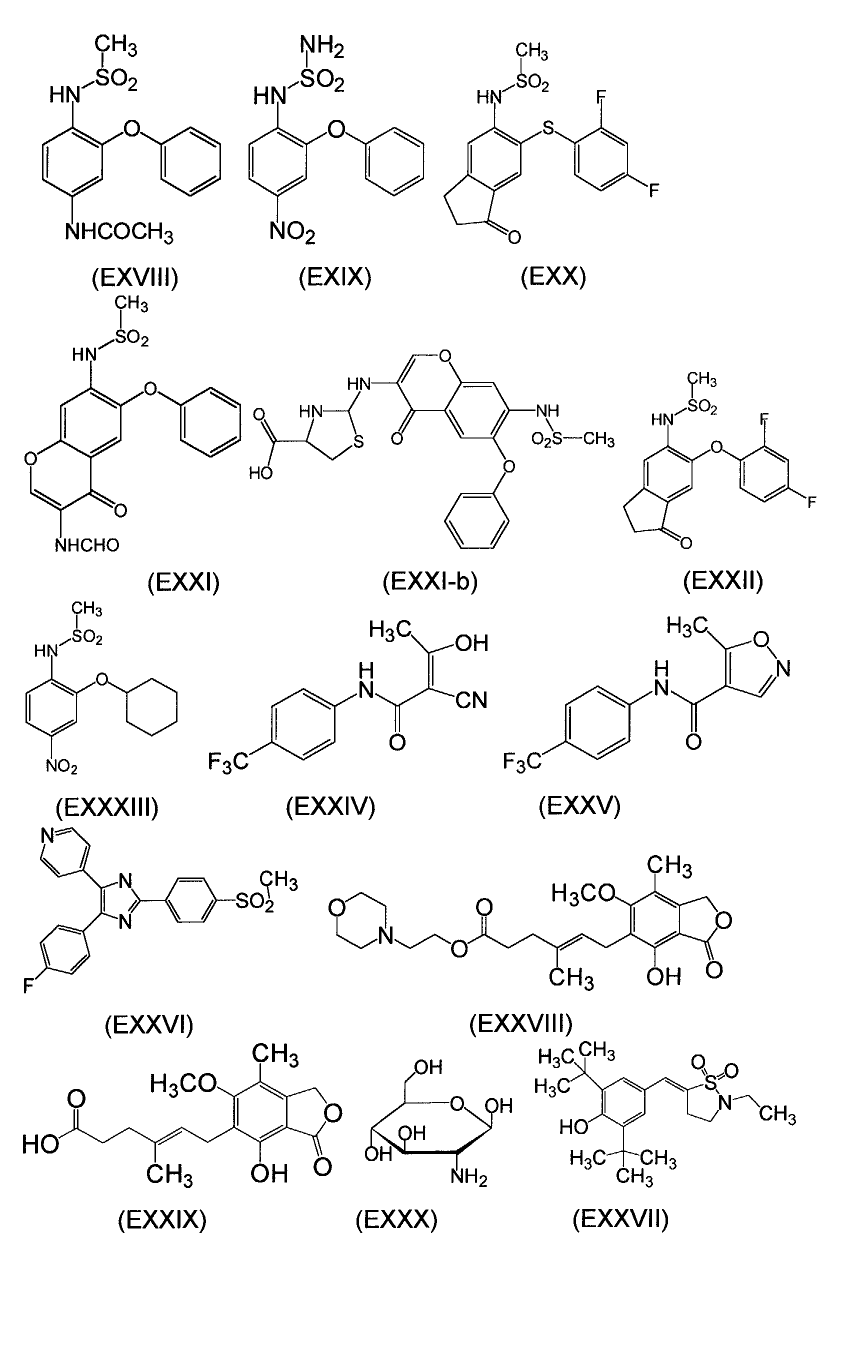

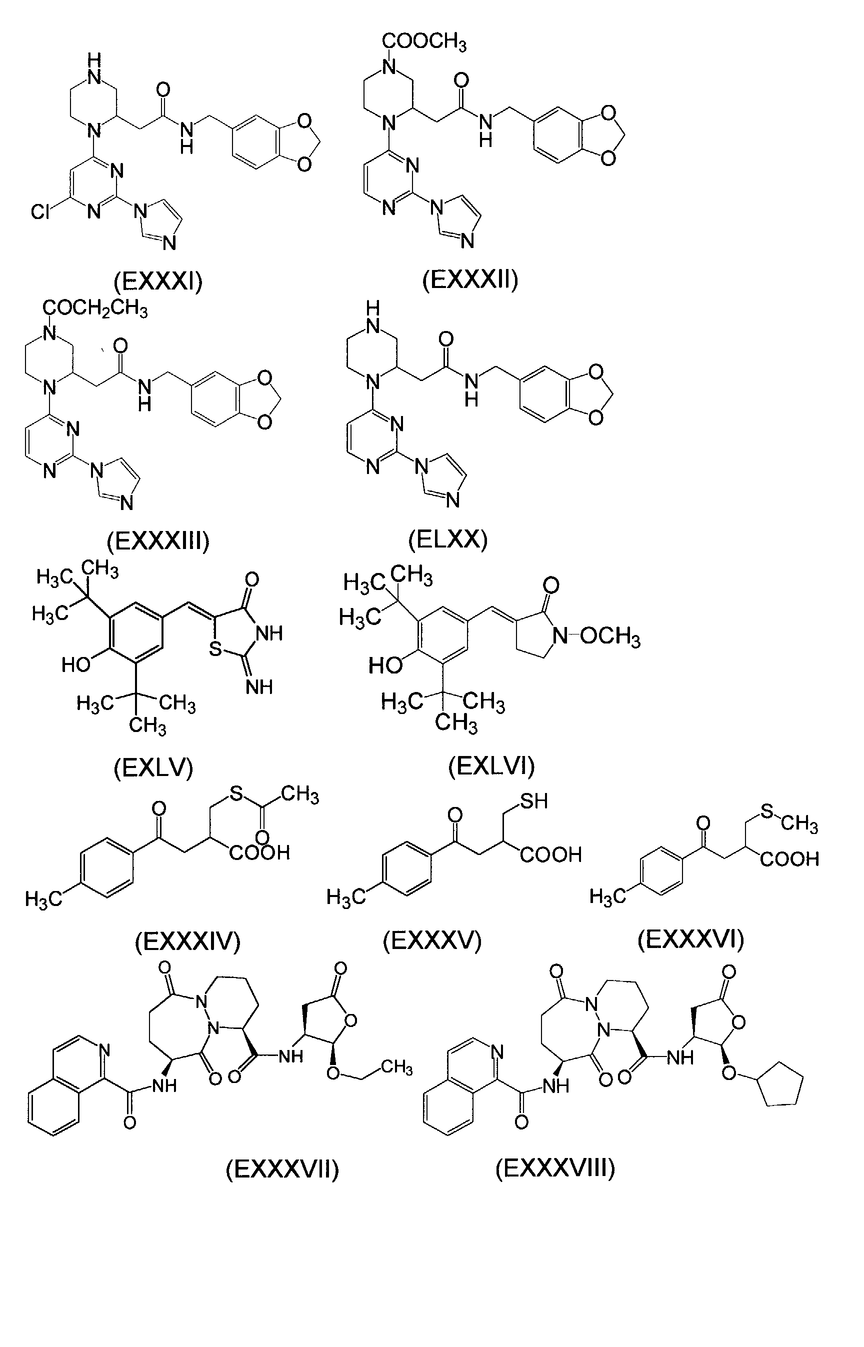

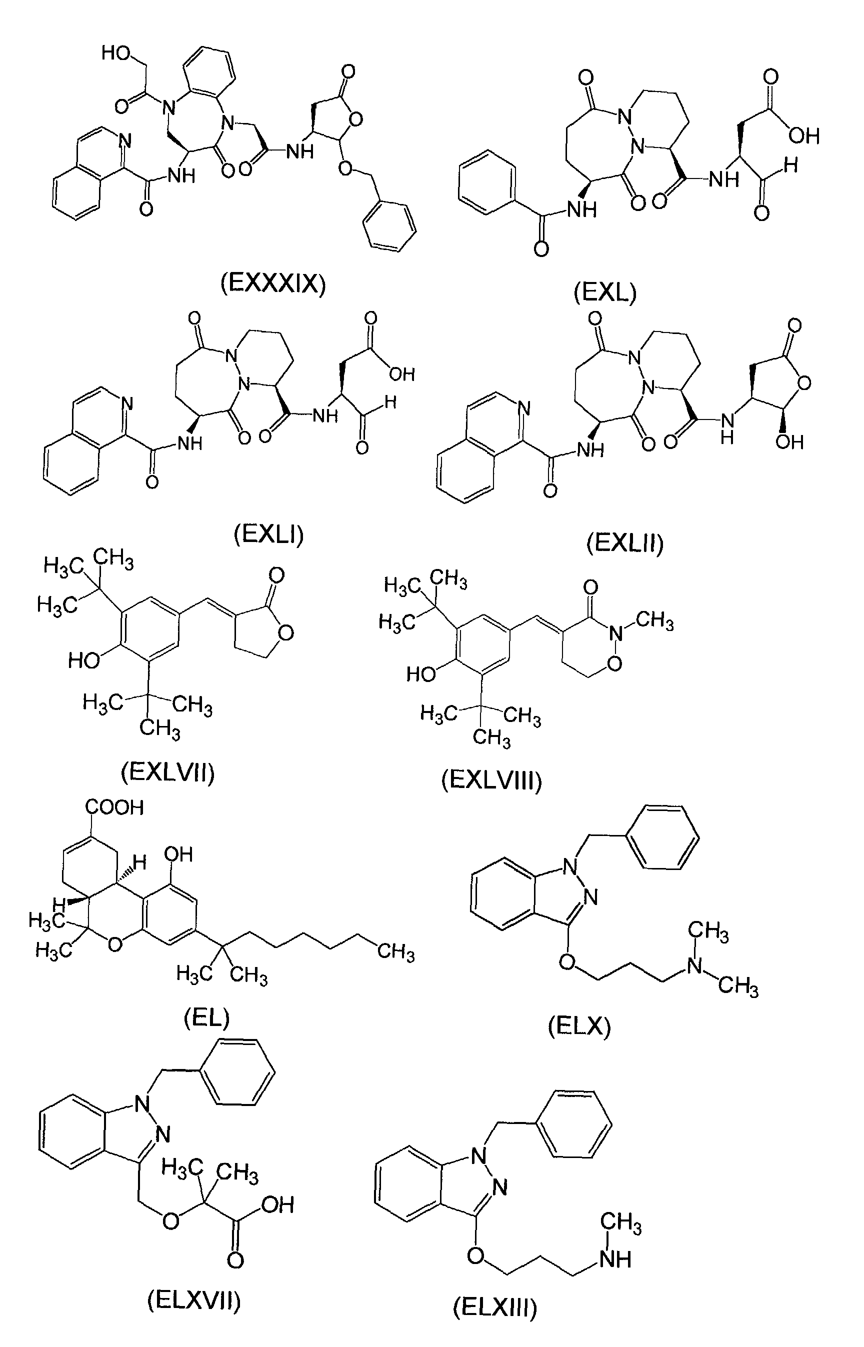

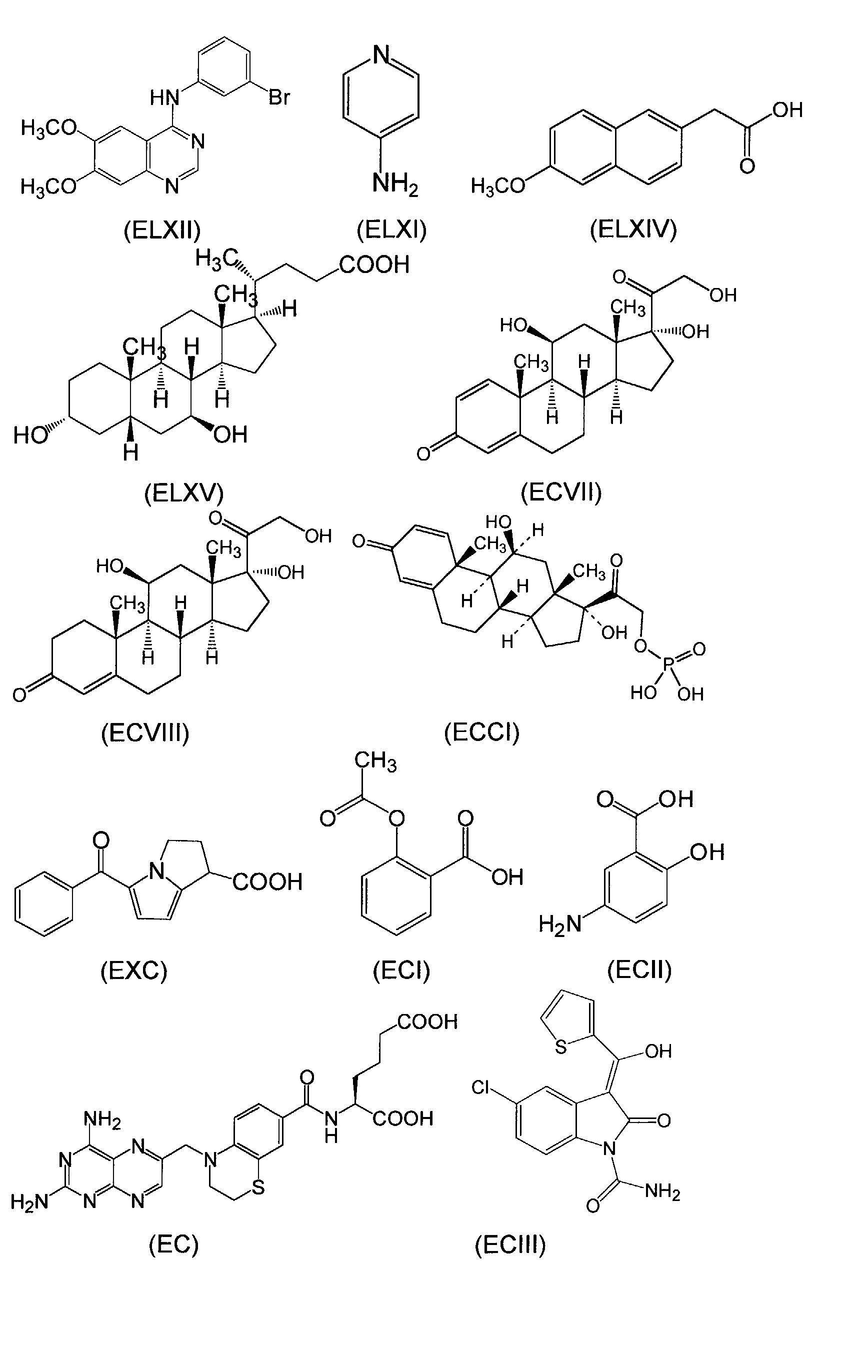

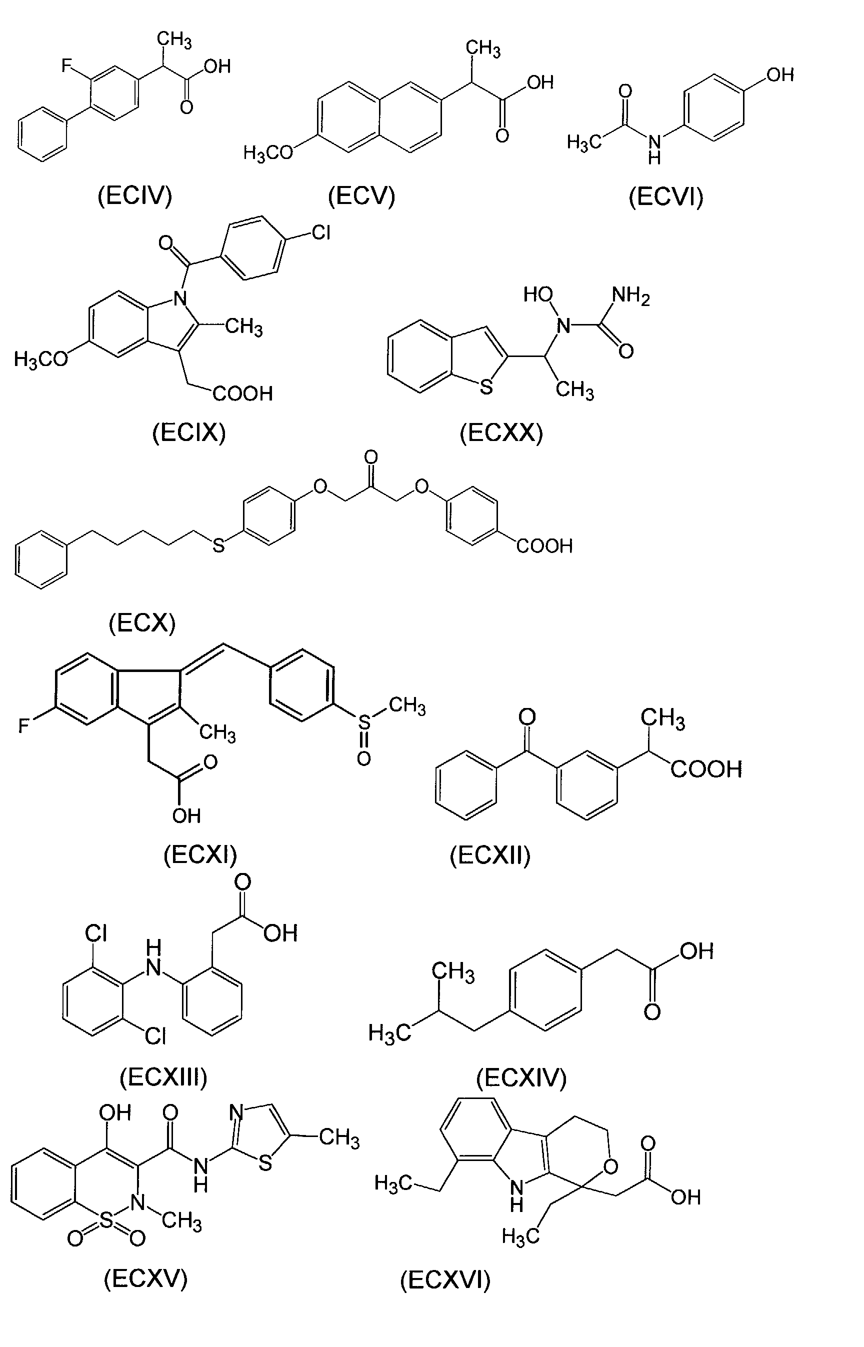

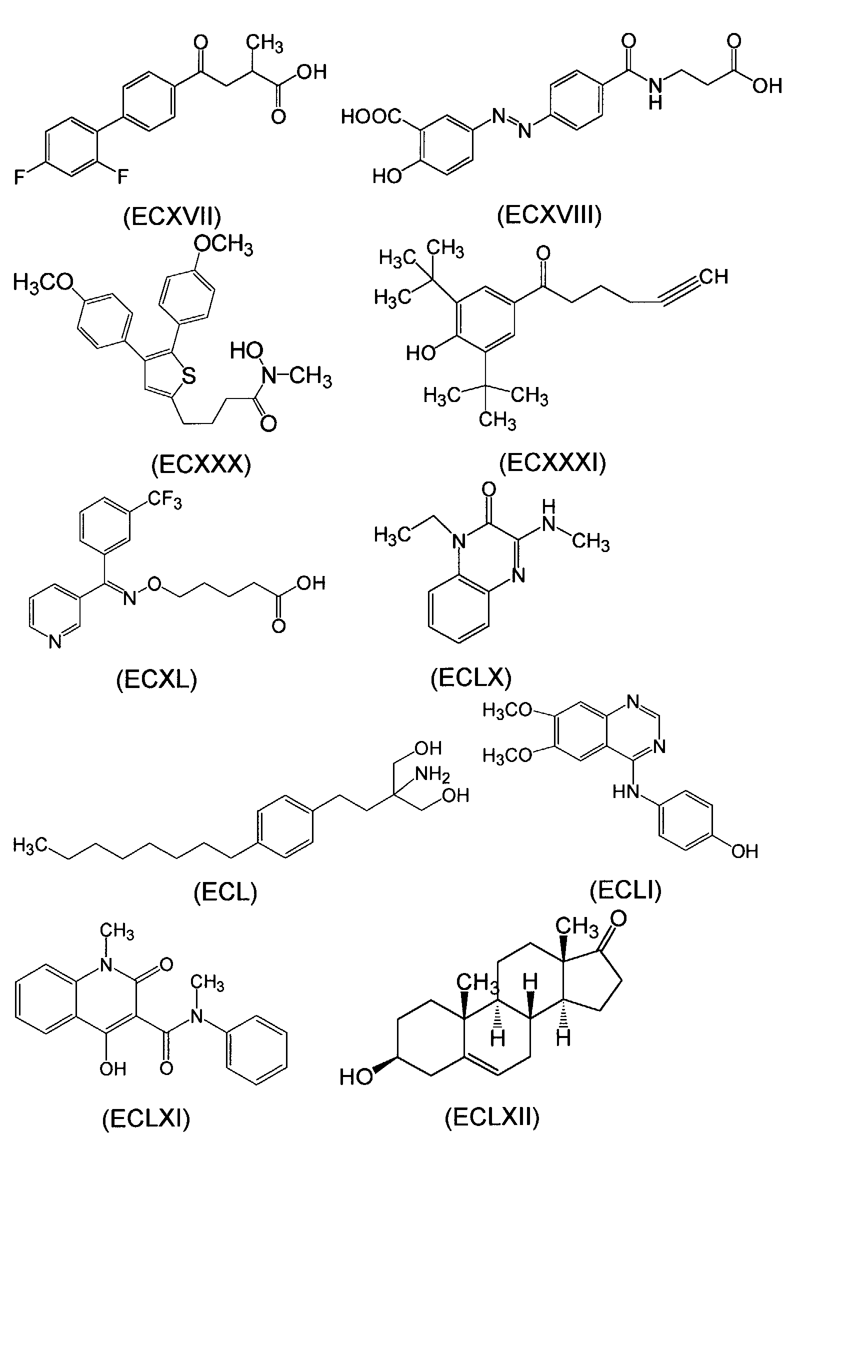

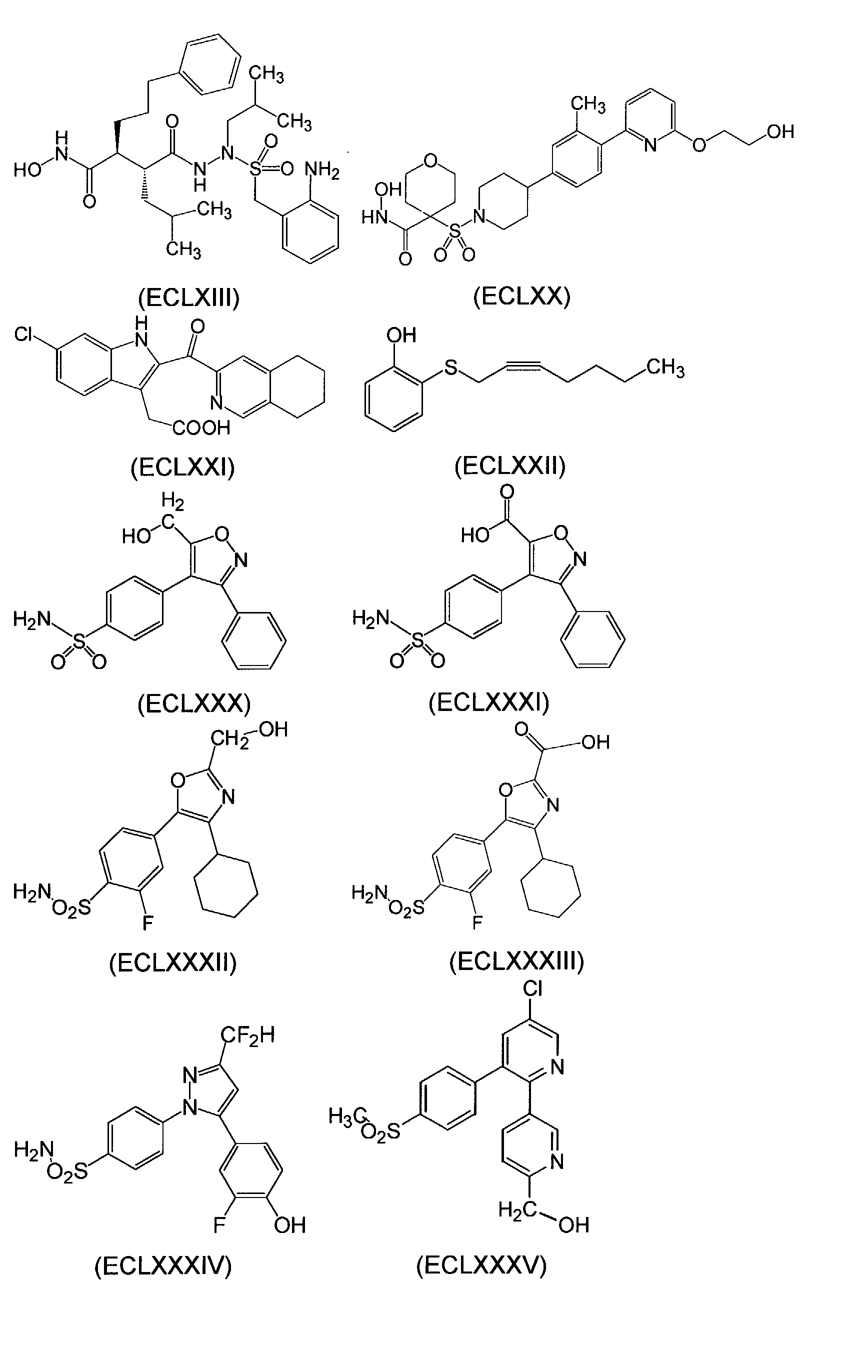

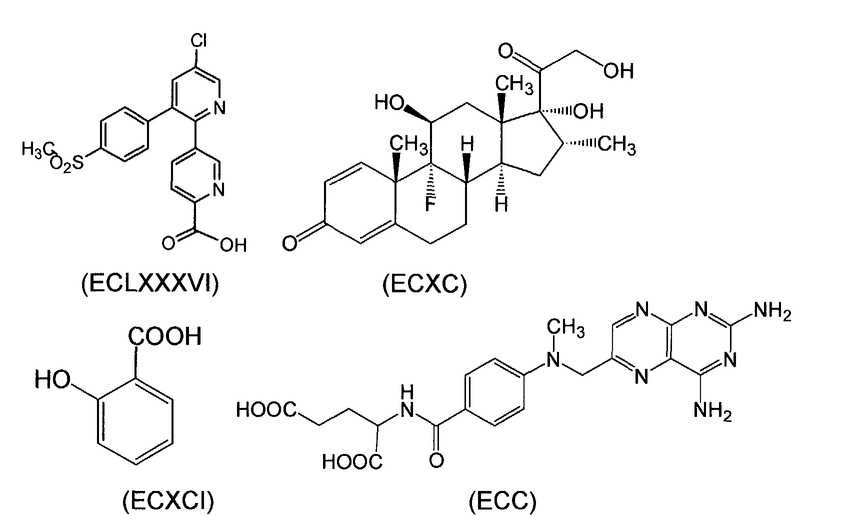

### GROUP 2B:

alendronic acid, AGN-4310, arzoxifene (SXXX), avicatonin, bazedoxifene/TSE-424, bervastatin, butedronic acid, calcipotriol, calcitriol (SVIII), CGP-77675, clodronic acid, CP-336156 (SXXXII), CP-424391, doxercalciferol, droloxifene (SVI), EB-1089 (SXXXV), ecalcidene, ED-71, EMD-84444, ERA-923, esonarimod, estradiol, etidronic acid (SXV), F-9775A, F-9775B, falecalcitriol (SXXXI), FC-1271, GW-544, HMR-3339A, 4-hydroxytamoxifene, ibandronic acid (SXX), idoxifene, incadronic acid, LGD-2226, lasofoxifene (SVII), levormeloxifene, levosimendan, lexacalcitol, lidadronic acid (SV), maxacalcitol (SXL), medronic acid, minodronic acid (SIII), miproxifene, NDE-1003, neridronic acid, NPS-2143 (SXII), NVP-AAK980, olpadronic acid (SXXI), ORG-30659, one-alpha-D₂ (SXLI), ormeloxifene, osaterone, oxidronic acid, pamidronic acid (SXXII), paricalcitol (SXXXVII), pipendoxifene (SXXXIII), piridronic acid, pitavastatin, plicamycin, pralnacasan, raloxifene (SXIII), ranelic acid, risedronic acid (SXVI), RWJ-68354, SB-242784 (SIX), SB-265123, SB-267268, SB-273005, SC-56631, secalciferol, semparatide, simendan, SUN-E3001, TAK-778, teriparatide, tiludronic acid, tibolone, toremifene (SXIV), TJN-135, trimegestone, trioxifene, TSE-424, vitamina D₂ (SII), vitamina D3 , VX-745 (SX), zindoxifene, zoledronic acid (SIV), (SXI), (SXLII), (SL)

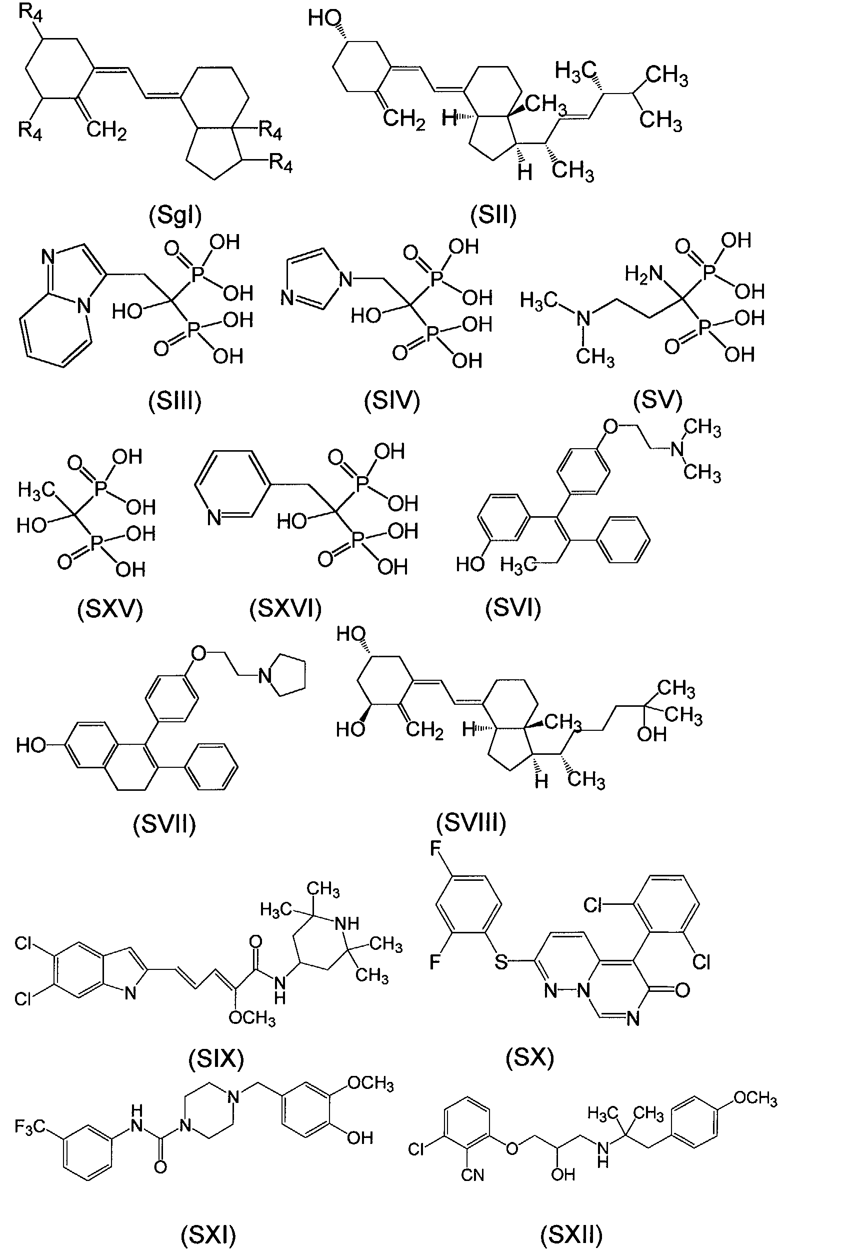

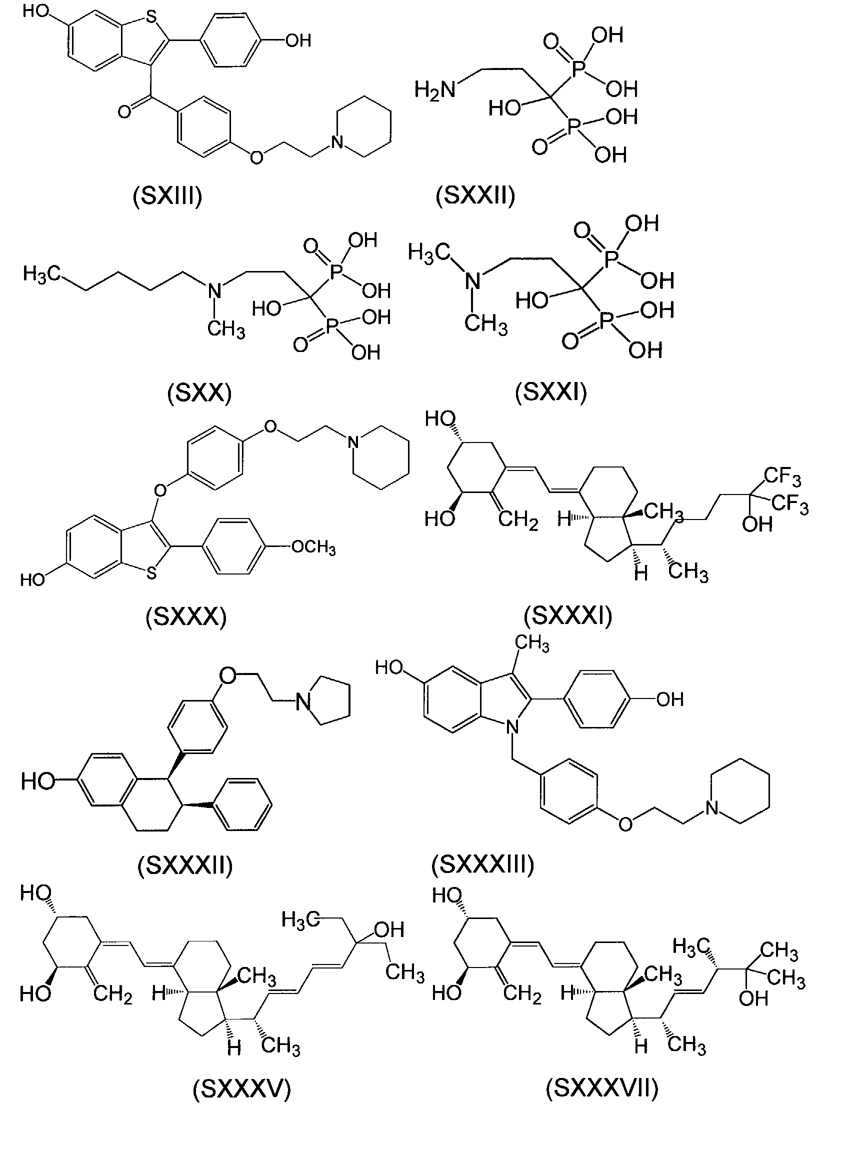

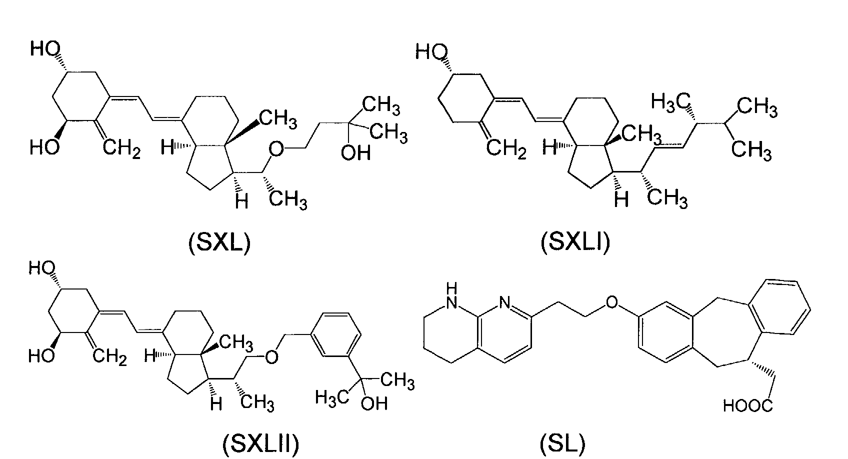

### GROUP 3B:

ABT-594, alfentanil, almotriptan, apadoline, aprepitant, asimadoline, aspirin, baclofen, benzydamine, buprenorphine, bupivacaine, butorphanol, capsavanil (PI), CJC-1008, CNS-5161, codeine (PXIV), dapitant, dextromethorphan, dihydrocodeine (PXI), devazepibe, dronabinol, dutarizine, edronocaine, etidocaine, emfilermin, enadoline, etodolac, etofenamate, ezlopitant, fenoprofen, fentanil, figopitant, flufenamic acid, flurbiprofen, gabapentin, ganaxolone, GV-196771 (PII), hydrocodone (PXII), harkoseride, icatibant, indomethacin, ipravacaine, ketoprofen, ketorolac, lamotrigine, lanepitant, levobupivacaine, LY-293558, levonantradol, lidocaine, lofentanil, lomerizine, meperidine, mepivacaine, memantine, metacetamol, morphine, nabazenil, NNC-05-1869, NW-1029, naproxen, nepadutant, osanetant, oxycodone (PX), paracetamol (PIV), pentazocine, phenazopyridine, pidolacetamol, pravadoline, propacetamol, pregabalin, propoxyphene, remifentanil, rimonabant, rizatriptan, ropivacaine, rufinamide, saredutant, SCH-50971, SDZ-249-665, sufentanil, sumacetamol, suprofen, talnetant, tonabersat, topiramate, tramadol (PV), vofopitant, ziconotide, zoltriptan, zucapsaicin, (PIll)

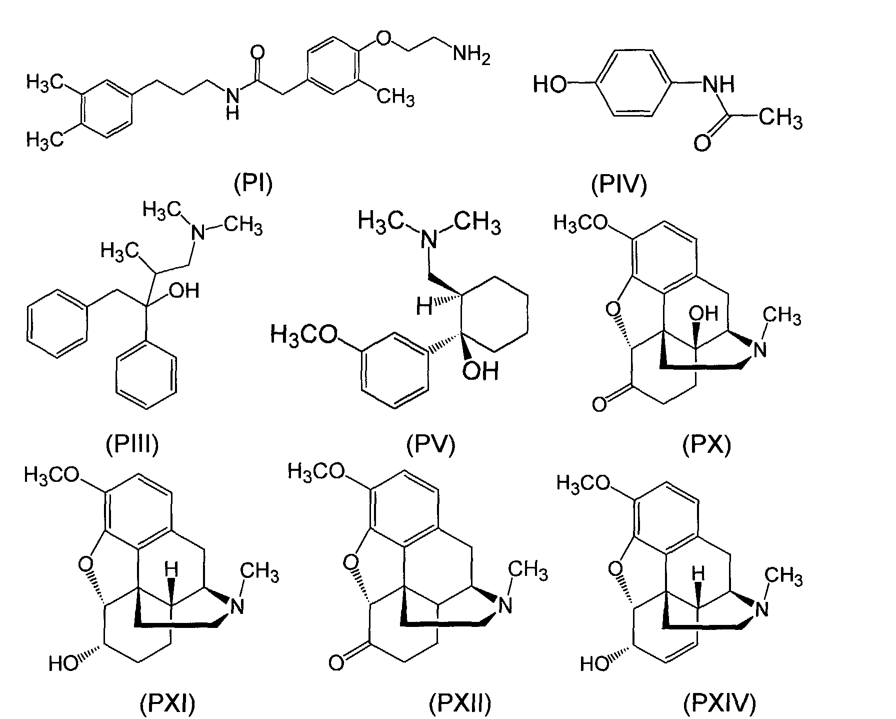

### GROUP 4B:

α-tocopherol (QIV), acitretin (QI), adapalene, amelubant, amlexanox (QIII), azathioprine, atizoram, calcitriol, calcipotriene/calcipotriol (Q10), bexarotene, dexamethasone, LPD-519, hydrocortisone, hydroxyurea, prednisolone, prednicarbate, prednisone, riboflavin, riboflavin tetrabutyrate, riboflavin 6-thioguanine, triacetate, tacalcitol, lexacalcitol, maxacalcitol, moxilubant, seocalcitol, paricalcitol, sulfasalazine, tazarotene, tepoxalin, ticolubant, thalidomide, VX-497, (QII), zidovudine,

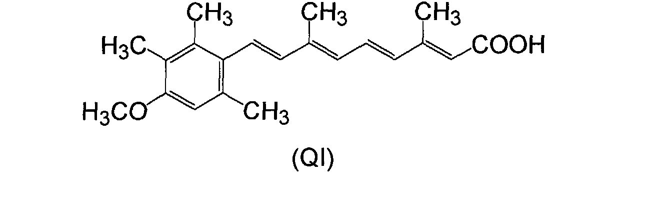

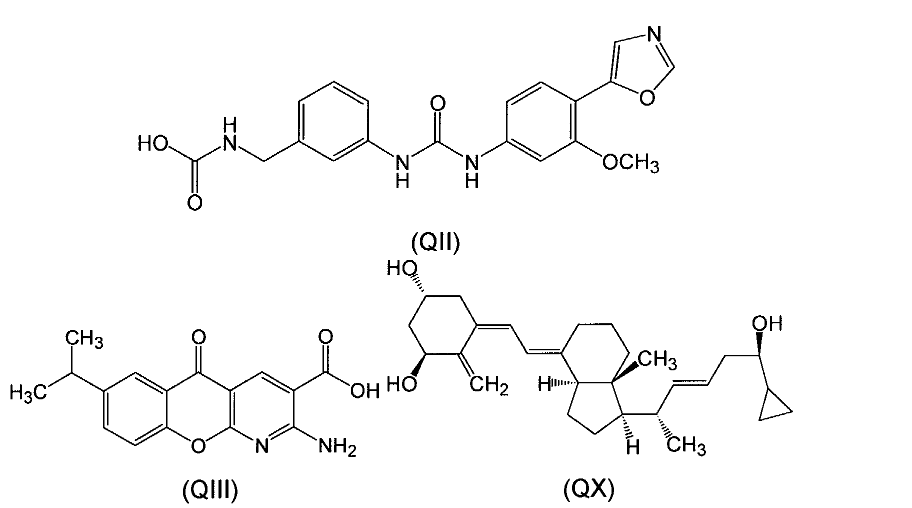

### GROUP 5B:

ablukast, ABT-761 (JVII), acetylcysteine, acitazanolast, acreozast, adapalene, albifylline, albuterol (JXXXII) (JgIII), alclometasone, alinastine, AMG-126767, R-α-methylhistamine (JCXXXI), ambicromil, ambroxol, amebucort, amelometasone, aminophylline, andolast, APT-366, apafant, apaxifylline, argatroban, argimesna, arofylline, AR-C68397AA (JXXXVI), asobamast, astemizole (JCXXXIV), ataquimast, atizoram, atreleuton, AY-0068, AWD-12-281 (JXVII), AWD-12-343, BAY-19-8004, BAY-X-1005 (JLXXX), bamaquimast, batebulast, benafentrine, bepafant, bepotastine, betotastine, betamethasone, bilastine, bimosiamose, binizolast, bitolterol, BP-294 (JCXXX), budesonide (JII) (Jgll), bunaprolast, butaprost, butixocort, camonagrel, carebastine, cartasteine, CE-1037, cetirizine (JCXXXII), ciclesonide, cilomilast/SB-207499 (JXI), cilutazoline, cinalukast, cipamfylline, Cl-1018 (JXIII), CI-1044 (JXVIII), CGH-2466, cistinexine, ciclesonide, clopidogrel, cloticasone, cromakalim, cromoglicate lisetil, contignasterol, cromoglicic acid (JI), CR-3465, cysteine, D-4418 (JXII), CPX (JLXXIII), D-4396, dacopafant, daltroban, dametralast, danosteine, dapitant, dazoquinast, DAX (JLXXIV), decarboethoxyloratadine, DF-1111301, deflazacort, deltibant, descinolone, desloratadine, desonide, dexamethasone, dexbudesonide, dexsecoverine, dextiopronin, diproteverine, DMP-777, domitroban (JXXX), doqualast, doxaprost, drotebanol, ebastine, eclazolast, efletirizine, eflumast, elziverine, emedastine, enefexine, enofelast, enoxamast, epinastine, epinephrine, eprazinone, erdosteine, espatropate, etolotifen, ezlopitant, fenleuton, fenoterol (JXXXIV), fenprinast, fexofenadine (JXC), figopitant, filaminast, flerobuterol, flezelastine, fluazacort, flunisolide, fluticasone (JV) (Jgl), fominoben, foropafant, formoterol (JXXXV), fudosteine, furegrelate, genistein (JLXXII), genleuton, guaimesal, guaifenesin, guaisteine, halometasone, HSR-609, hydroxyzine, ibudilast, icatibant, ICI-200355, idaverine, idenast, ifetroban, ilonidap, imitrodast (JCXI), INS-3653, ipratropium, IPL-4088, iralukast, isalsteine, isbogrel, isbufylline, isocromil, isoetharine, isoproterenol, israpafant (JXL), itazigrel, ketotifen, L-826141 (JXIX), L-658758, lanepitant, laprafylline, levalbuterol, levcromakalim, levmetamfetamine, levocabastine, levocetirizine, levodropropizine, levosulpiride, lexipafant, linazolast, linetastine, linotroban, lirimilast, lisofylline, lonapalene, loratadine, loxanast, mapinastine, melquinast, meribendan, mesna, metaproterenol, methylprednisolone, midaxifylline, midazogrel, midesteine, milverine, milrinone (JLXXI), minocromil, minopafant, mipitroban, mizolastine, modipafant, moguisteine, mometasone, montelukast (JC), MPB-07 (JLXXV), moxilubant, nafagrel, naphazoline, naflocort, N-acetylcysteine, nardeterol, nedocromil, neltenexine, nemazoline, nepadutant, nestifylline, NKP-608C, noberastine, norastemizole (JCXXXIV), nupafant, olopatadine, olprinone, ontazolast, osanetant (JCXX), oxalinast, oxitropium, ozagrel, pamicogrel, paramethasone, pemirolast, perbufylline, phenylbutyrate (JLXX), phenylpropanolamine (JXXII), piclamilast (JXXV), picumast, picumeterol, pirbuterol, pirmagrel, pirodomast, pobilukast, pranlukast, prednisolone, prednisone, propiverine, promethazine, proxicromil, pumafentrine (JXIV), quazolast, quiflapon, quinotolast, racephedrine, racephedrine, ramatroban (JXXXI), raxofelast, repirinast, revatropate, revenast, ridogrel, rimonabant, ritolukast, rocastine, rocepafant, roflumilast (JX), rolafagrel, rolipram, RPR-106541, rupatadine (JXCI), salmeterol (JXXXIII), salmisteine, samixogrel, saredutant, sarpogrelate, satigrel, scopinast, SCH-351591, seratrodast (JCX), setipafant, sevitropium, siguazodan, sivelestat (JL), spirofylline, stacofylline, sulotroban, sulukast, suplatast (JLX), T-440, talnetant, taurosteine, tazanolast, telmesteine, tematropium, temiverine, tenidap, terbogrel, terbucromil, terbutaline, tetrazolast, texacromil, theophylline, tiacrilast, tibenelast, ticolubant, tiotropium, tioxamast, tiprinast, tolafentrine, tofimilast, tomelukast, tranilast (JIII), toborinone (JXX), triamcinolone, tulopafant, UT-77, verlukast, vofopitant, V-11294A (JXV), YM-976 (JXVI), zafirlukast, zardaverine, zileuton (JIV), zilpaterol, zipeprol, (JXCII), (JCXXXIII), (JCXXXIV)

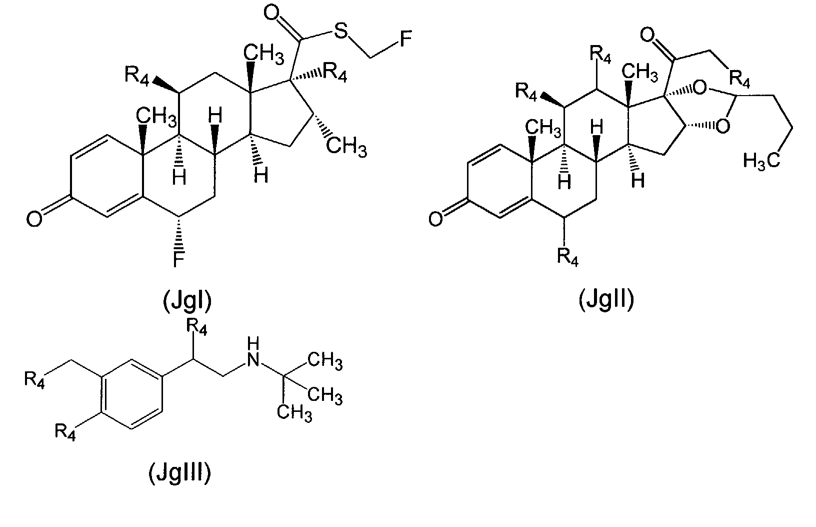

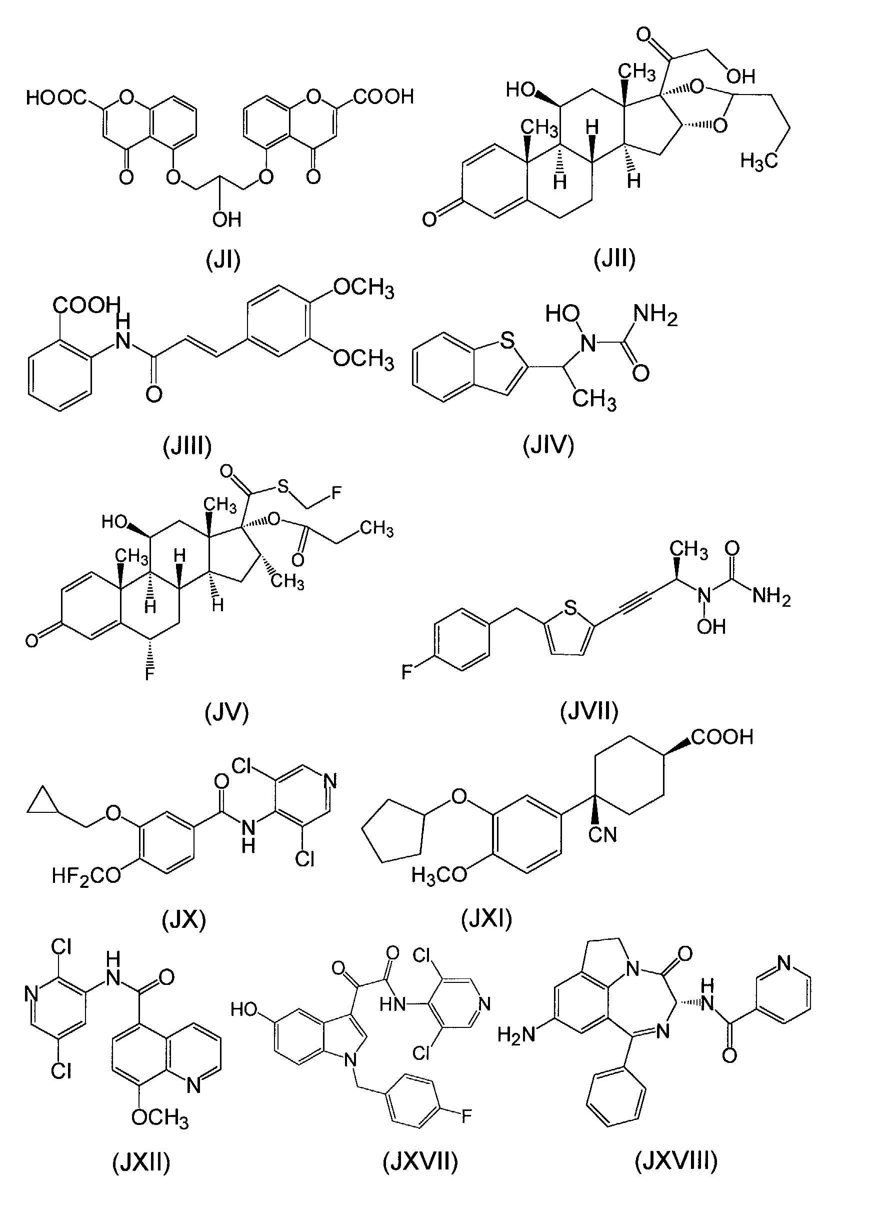

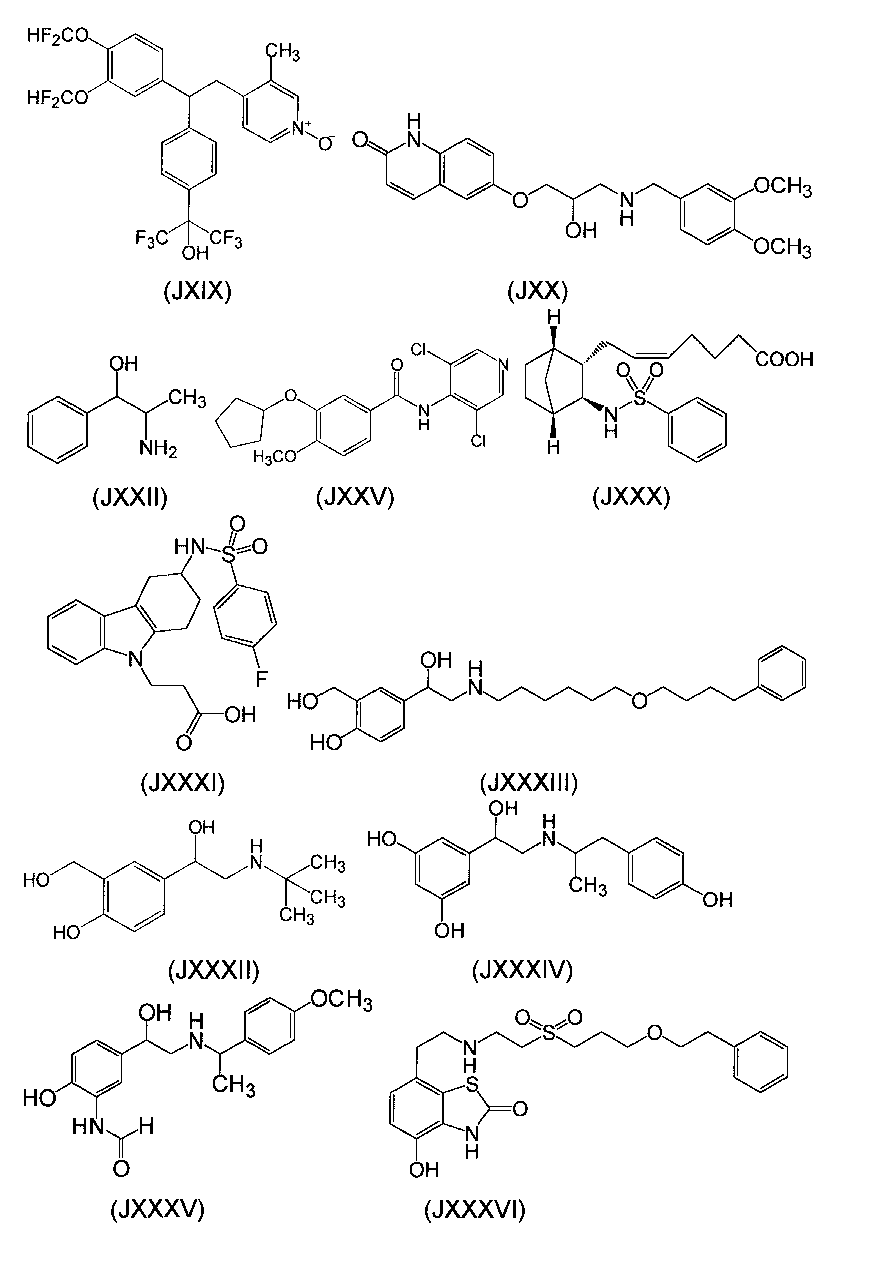

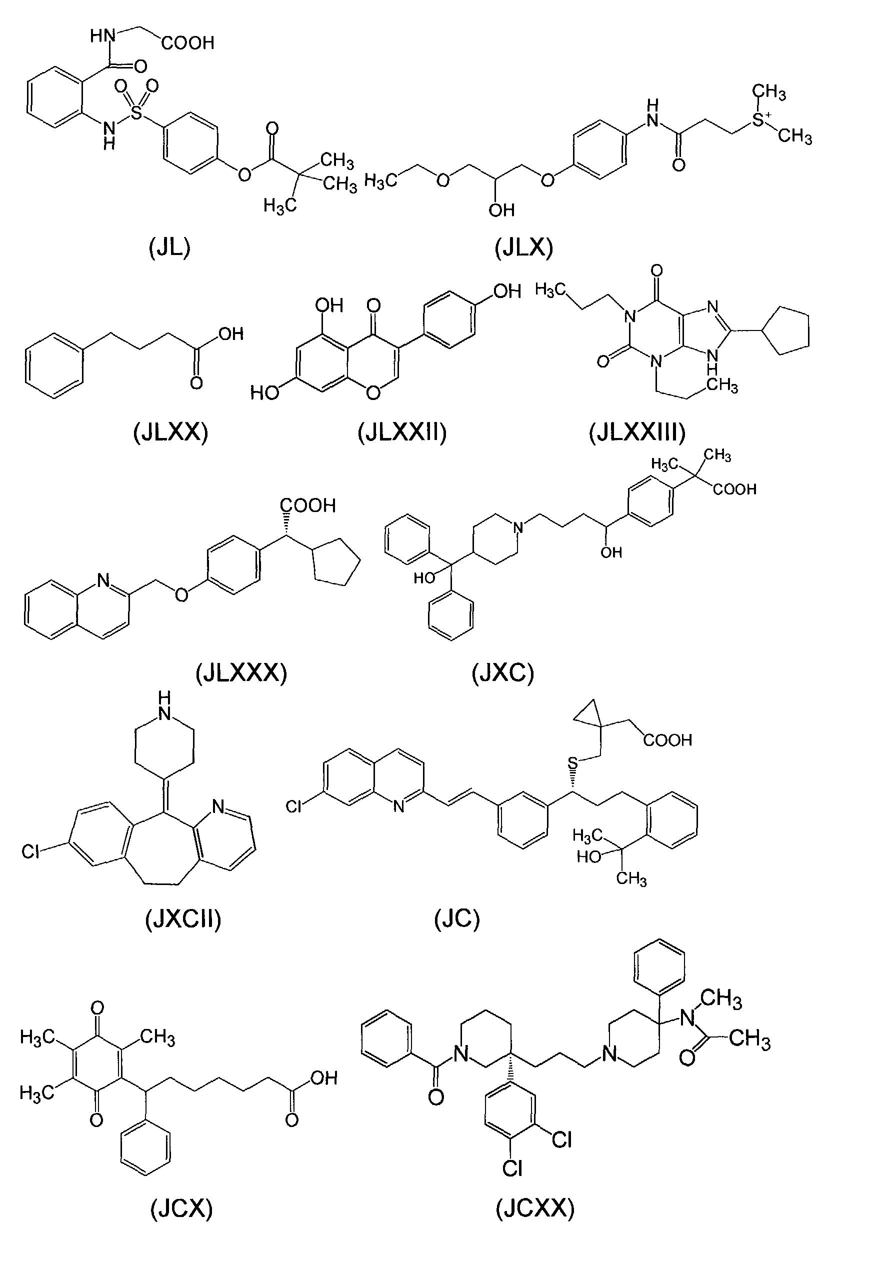

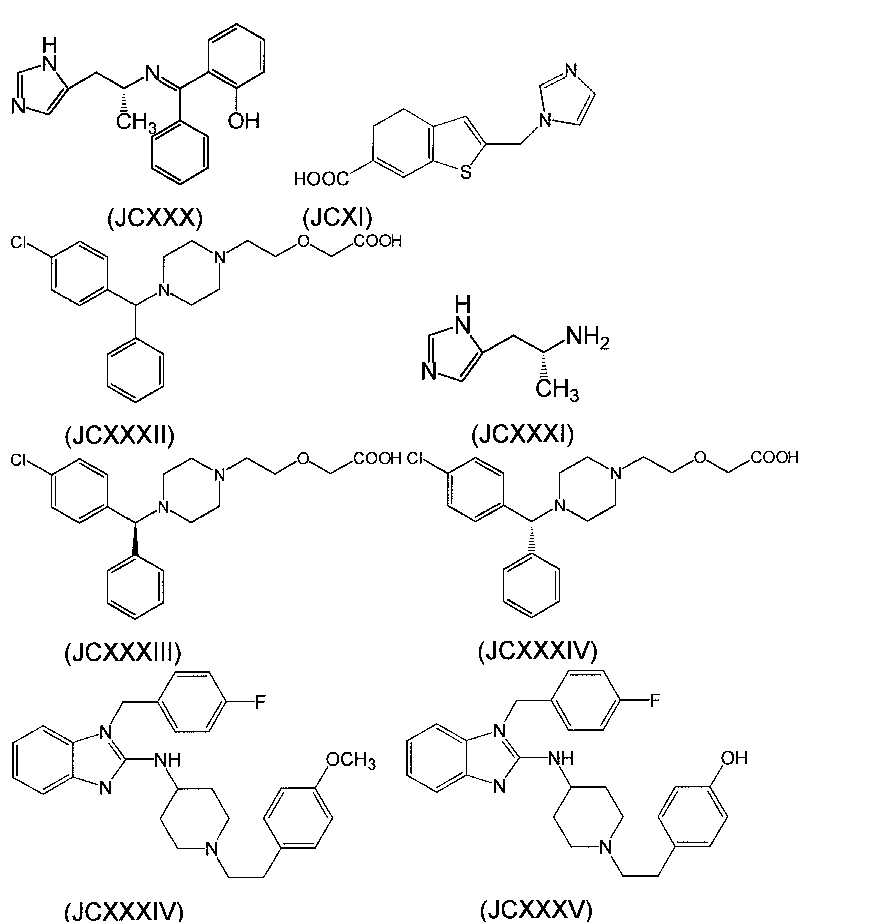

### GROUP 6B:

alizapride, alosetron, amiglumide, arbaprostil, AR-H047108, bromopride, budesonide, CAM-1028, 5-carboxyomeprazole (RXCII), 5-carboxytenatoprazole (RXCVI), 5-carboxyesomeprazole (RXCVII), chenodiol, cimetidine, cisapride, clarithromycin, clebopride, CR-2622, CR-3294, darifenacin, deprostil, desmethylrabeprazole (RXXVIII), devazepide, dexloxiglumide (RXXXI), dolasetron, domperidone, dosmalfate, egualen (RXXX), E-3620, enprostil, esaprazole, esomeprazole (RIV), esolansoprazole (RCI), esopantoprazole (RCIII), famotidine, fedotozine, granisetron, 5-hydroxylansoprazole (RC), 5-α-hydroxyomeprazole (RXC), 5-α-hydroxyotenatoprazole (RXCIV), 5-α-hydroxyesomeprazole (RXCVIII), ezlopitant, itasetron, itriglumide (RgIV) (RXXXIII), IY-81149 (RIX), L-365,260 lansoprazole (RV), lafutidine, leminoprazole (RI), levosulpiride, loperamide, loxiglumide, L-365260, LY-288513, LY-262684, LY-191009, MKC-733, MK-869, meclizine, mesalamine, mexiprostil, MKC-733, metoclopramide, misoprostol (Rgll) (RLXXII), mosapride, nepadutant, nepaprazole, nizatidine, nocloprost, norcisapride, 5-O-desmethylomeprazole (RXII), 4-O-desmethylpantoprazole (RXXII), olsalazine, omeprazole (RII), ondansetron, ornoprostil, osutidine, pantoprazole (RVI), pibutidine (RXLI), PD-135158, picoprazole (RLXIV), pipratecol, pranazepide, proglumide, pumaprazole, prucalopride, ranitidine, rabeprazole (RIII), rebamipide (RXXXIV), remiprostol, renzapride (RXXXII), rioprostil, rosaprostol (RgIII) (RLXX), roxatidine (RXL), RP-72540, RP-73870 (RLXXXI), S-0509 (RLXXXII), SB-641257, sepimostat (RL), spiroglumide, sonepiprazole, sulfasalazine, TAK-637, tarazepide, taurocholic acid, tegaserod, telenzepine, tenatoprazole (RVIII), timoprazole (RLXIII), tiropramide, trimoprostil, tropisetron, YIA-20379-1 (RLX), YM-022, YIA-20379-5 (RLXI), YIA-20379-8 (RLXII), zacopride, (RX), (RXI) (RXII), (RXIII), (RIV), (RXV), (RXVI), (RXVII), (RXVIII), (RXIX), (RXX), (RXXI), (RXXII), (RXXIII), (RXXIV), (RXXV), (RXXVI), (RXXVII), (RLXV), (RLXXI), (RLXXX), (RXCIII), (RXCI), (RCII), (RCIV), (RCVI), (RCVII)

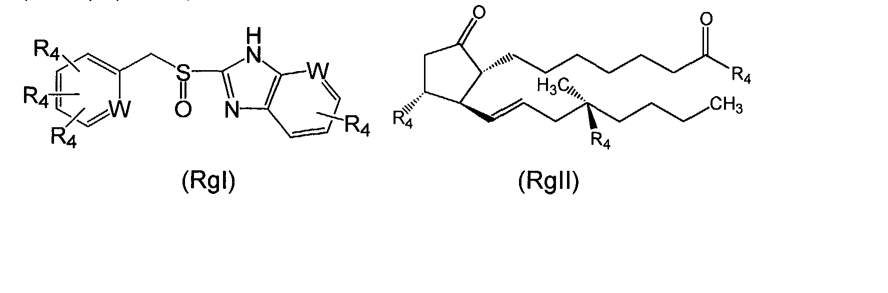

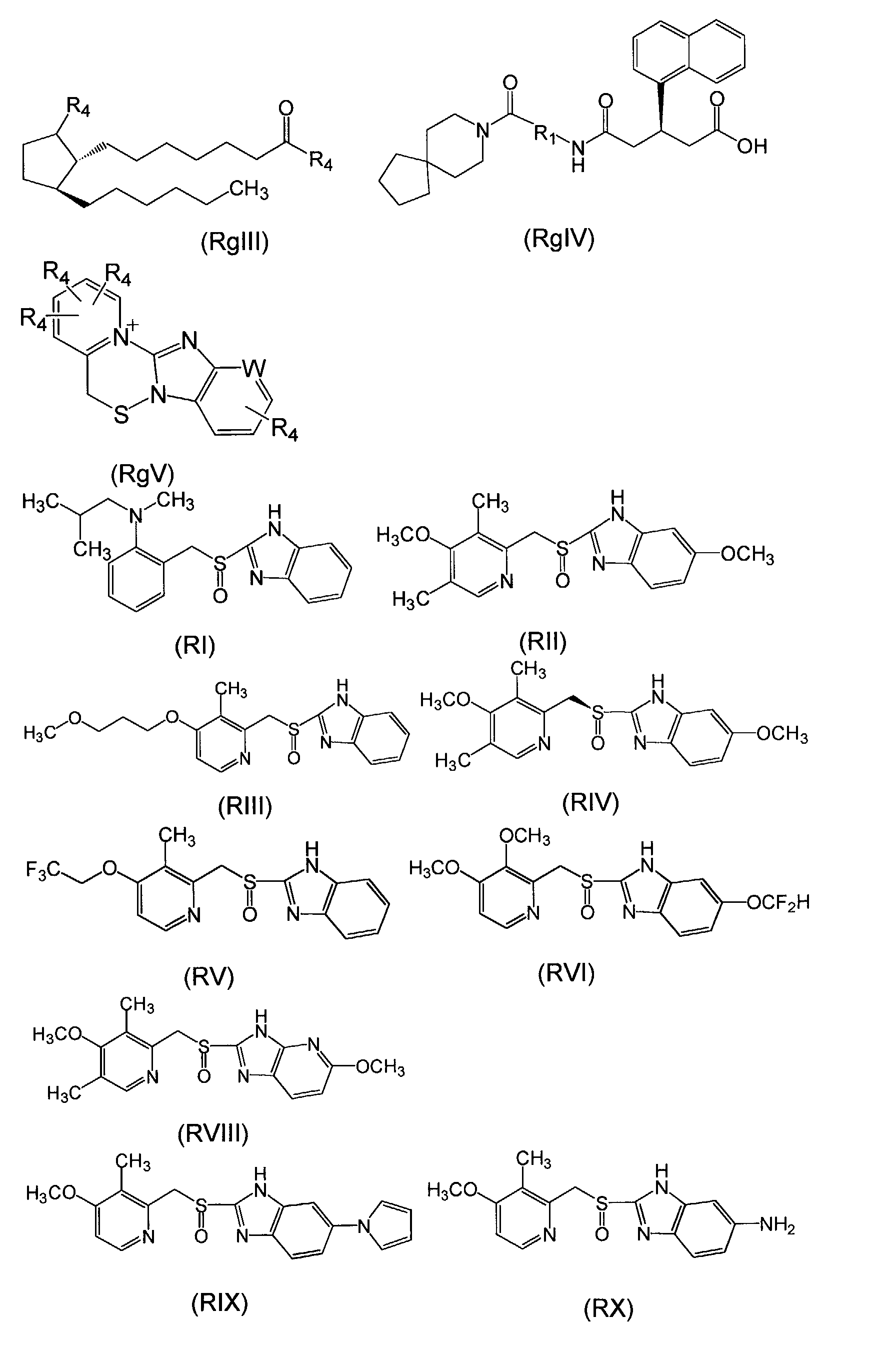

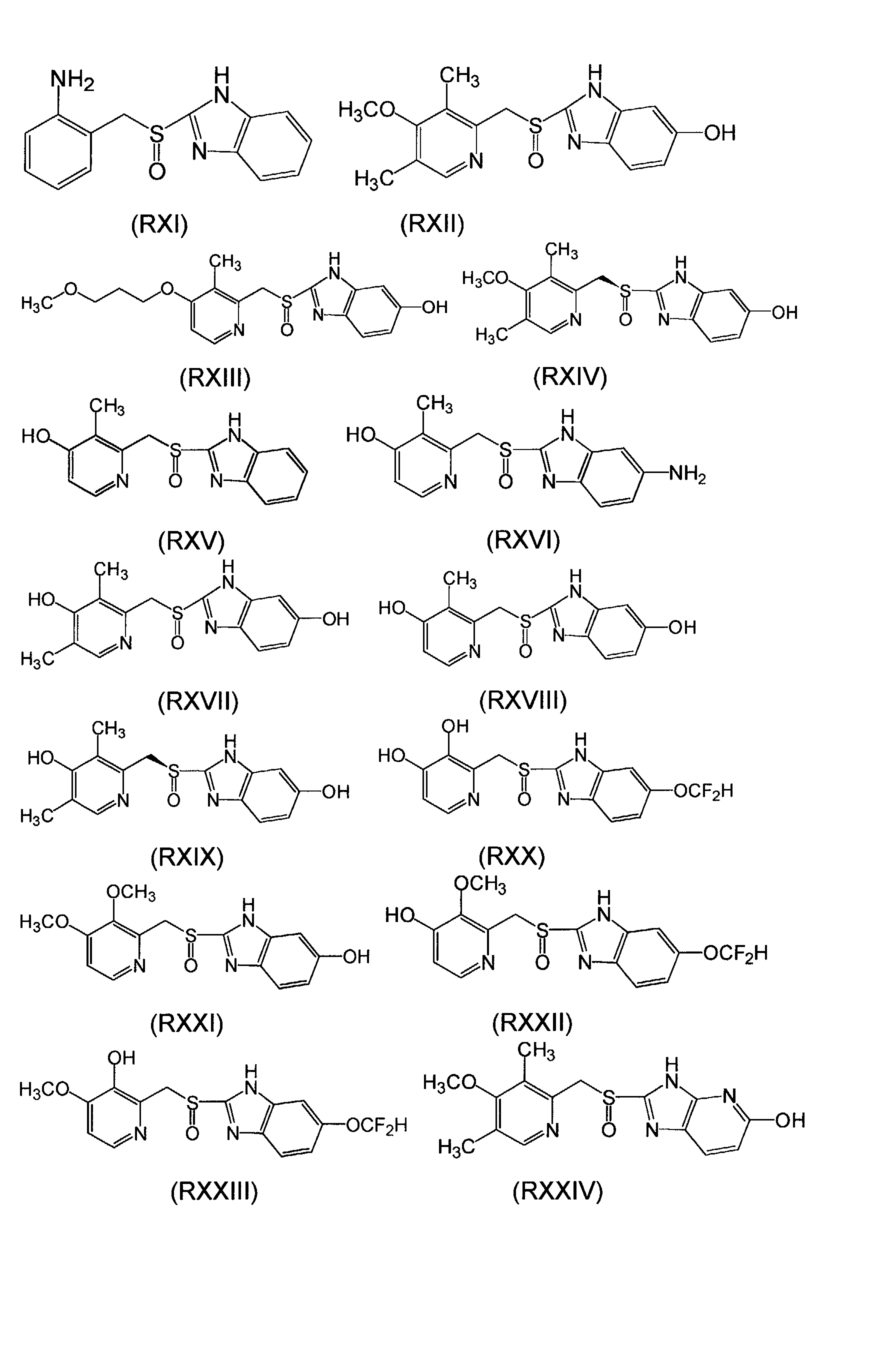

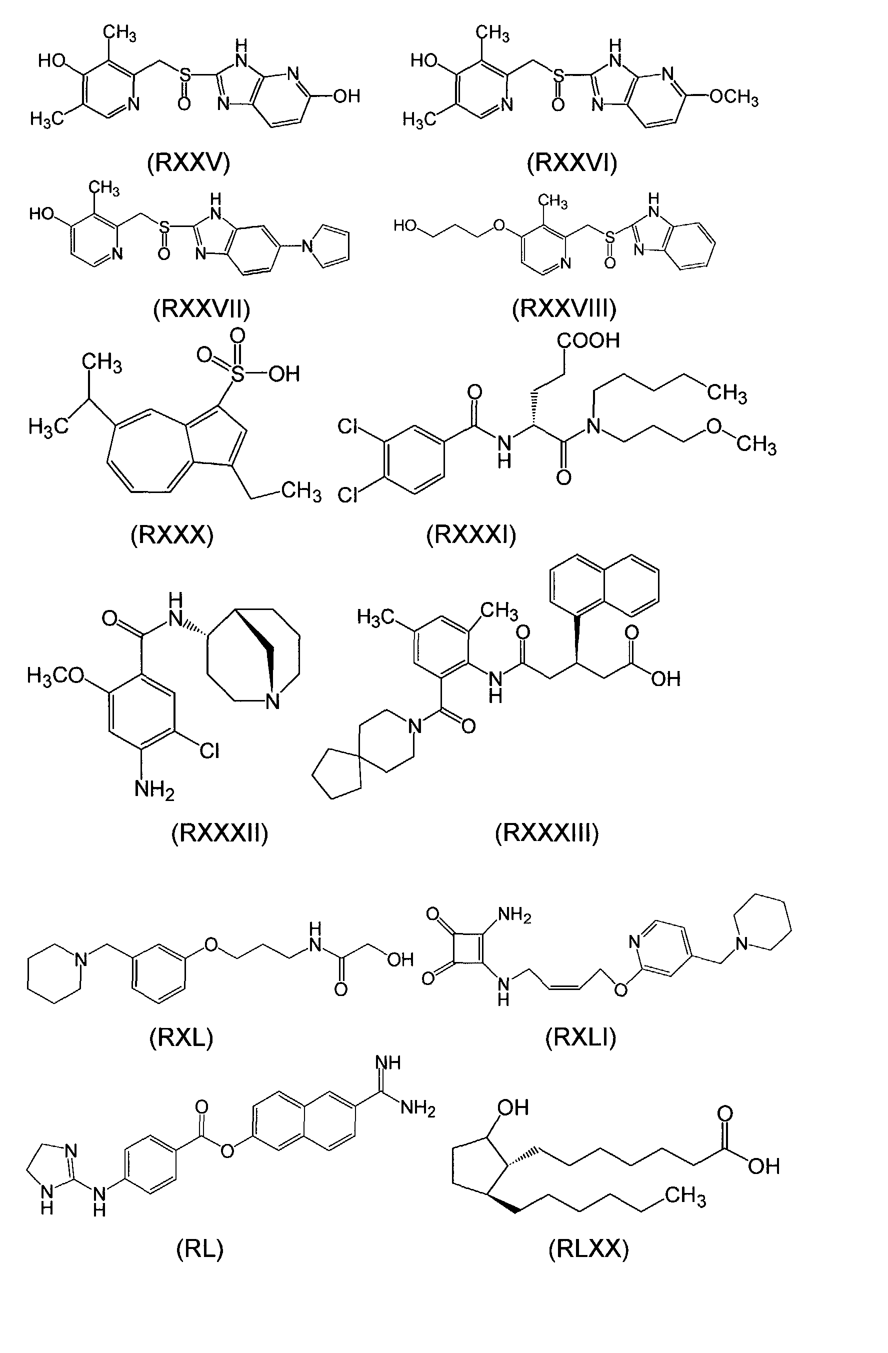

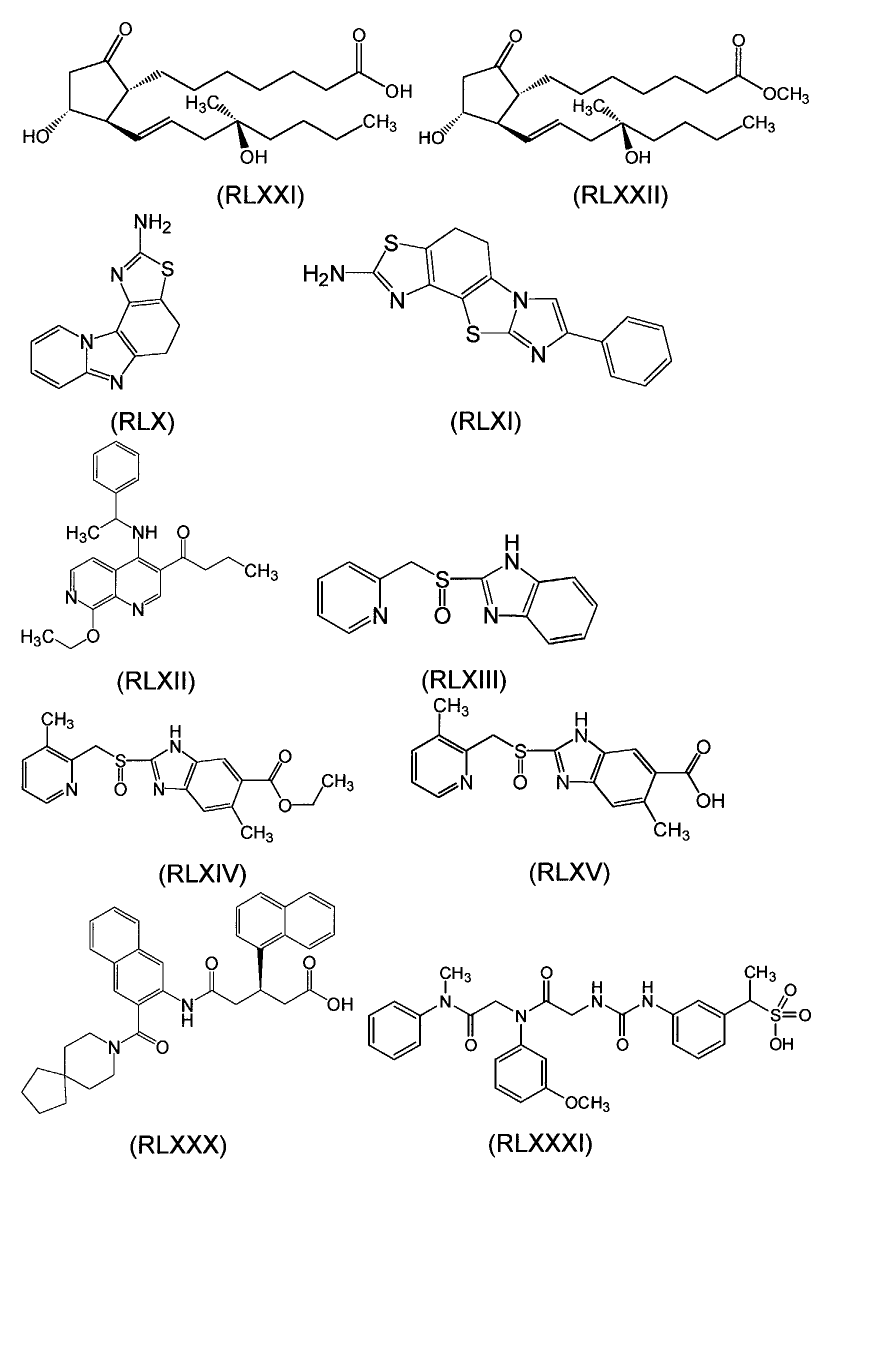

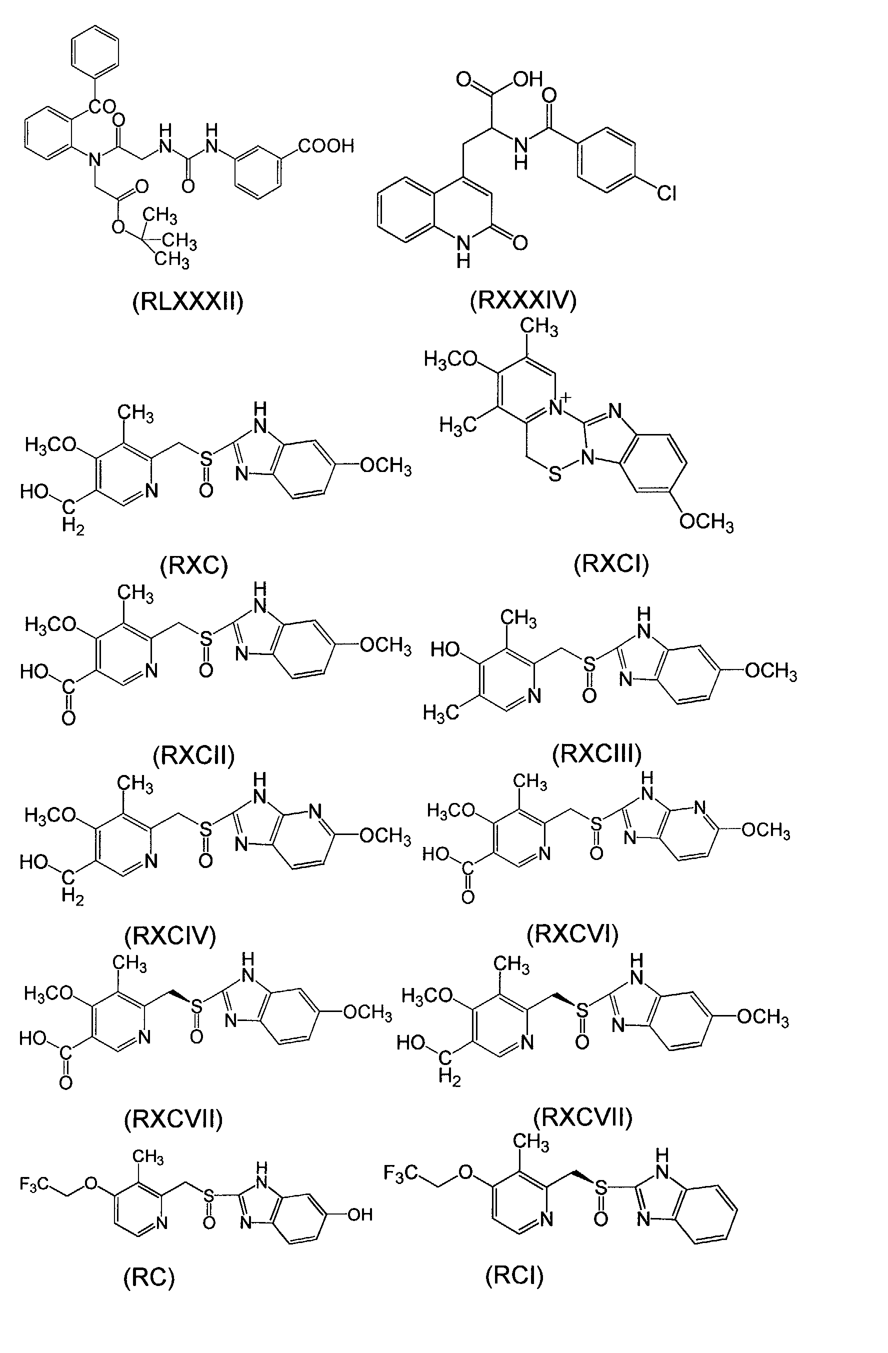

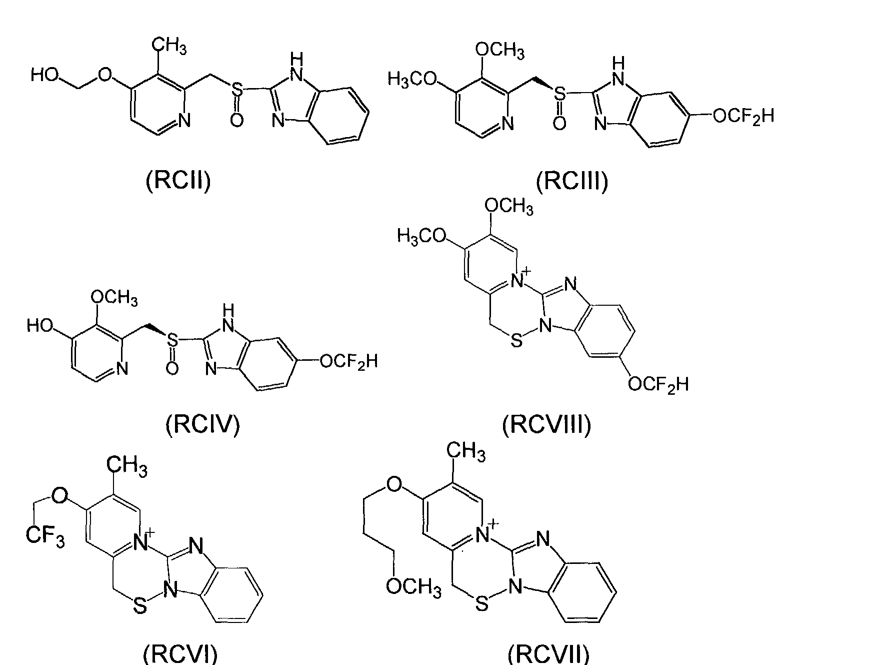

### GROUP 7B:

ABT-980, AH-9700, AIO-8507L, alfuzosin (UV), alfatradiol, alvameline (ULXX), alprostadil (UXC), atosiban, apomorphine (UCXXX), atrasentan (UCXL), bexlosteride, bicalutamide, BMS-341400, CS-891, cyproterone (UCII)), DA-8159, darifenacin (ULXXXIII), deacetylmoxisylyte, dehydrocorydaline (UXXXV), (+)-desmethylsibutramine, doxazosin (UXL), duloxetine (UXLI), dutasteride, E-4010 (UXXII), E-4021 (UXXIII), E-8010, EMD-221829, eplerenone, ESR-150, epristeride (UI), FCE-27837, fesoterodine (ULXXV), fiduxosin (UCXII), finasteride, finrozole (UCXLI), fluoxetine (UCXXXI), FK-143 (UCL), FR-119680 (UCLIII), FR-229934, gestonorone (UII-b), GI-198745, GYKI-16084 (UIX), GYKI-12743, izosteride, KMD-3213 (UCX), KCO-616, KRP-197 (ULXXXVIII), inaperisone (ULXXXV), indoramin, izonsteride, L-771688/SNAP-6383 (UCXIII), L-780945/SNAP-6991 (UCXVI), lapisteride, lanperisone, LY-320236, LY-353433, mepartricin, mepazosin, moxisylyte, naftopidil (UC), NC-1800 (ULXXXIX), NS-49 (UXCV), osanetant, osaterone (UII), oxendolone (UIV), oxybutynin, phentolamine (UVIII), PNU-157706 (UCLII), PNU-83757, PNU-156765, PNU-200577, prazosin, propantheline, prasterone (UCI), propiverine, resinferatoxin, RBX-2258, REC-15/3079, Ro-70-0004 (UCXVII), RS-100329 (UCXVIII), RWJ-38063 (UCXIV), RWJ-69736 (UCXV), SB-223412, saredutant (UXCVII), sildenafil (UXXXIV), SNA-4606-1, SNAP-6383, SNP-7915 (UCXIX), (S)-doxazosin (UCXI), (S)-oxybutynin (ULXXXVII), sibutramine (ULXXX), SPM-007, suplatast (ULXXXIV), T-1032 (UXXX), TA-1790, TAK-637, tadalafil/IC-351 (UXXXI), talnetant, tamsulosin (UIII), TF-505, temiverine (ULXXXVI), terazosin, terodiline, tolterodine (ULXXXII), turosteride, N-desmethylsildenafil/UK-103320 (UCLXX), UK-122764 (UVII), UK-343664, UK-357903, vamicamide, vardenafil (UVI), yohimbine (UCXXXII) (UgVII), zaprinast, YM-905 (ULXXXI), YM-53705, YM-46303, WAY133537, WAY151616, ZD-0947, ZD-6169 (UCXVI), Z-350 (UCLI), ZM-244085, (UX), (UXI), (UXII), (UXIII), (UXIV), (UXV), (UXVI), (UXVII), (UXVIII), (UXIX), (UXX), (UXXI), (UXXIV),(UXXV), (UCLIV), (UCLV), (UCLVI), (UCLVII), (UCLVIII), (UCLIX), (UCLXX), (UCLXXI), (UCLXXII), (UCLXXIII), (UCLXXIV)

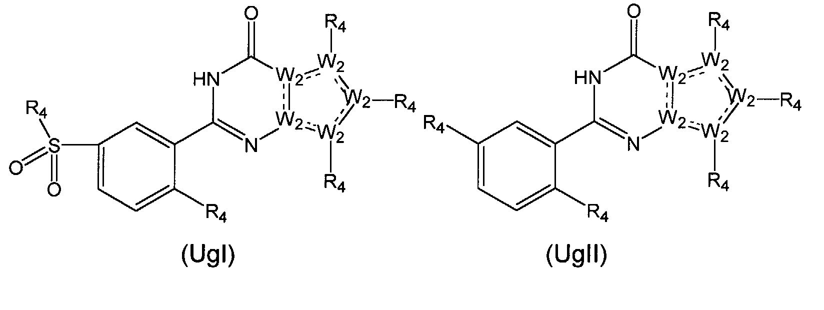

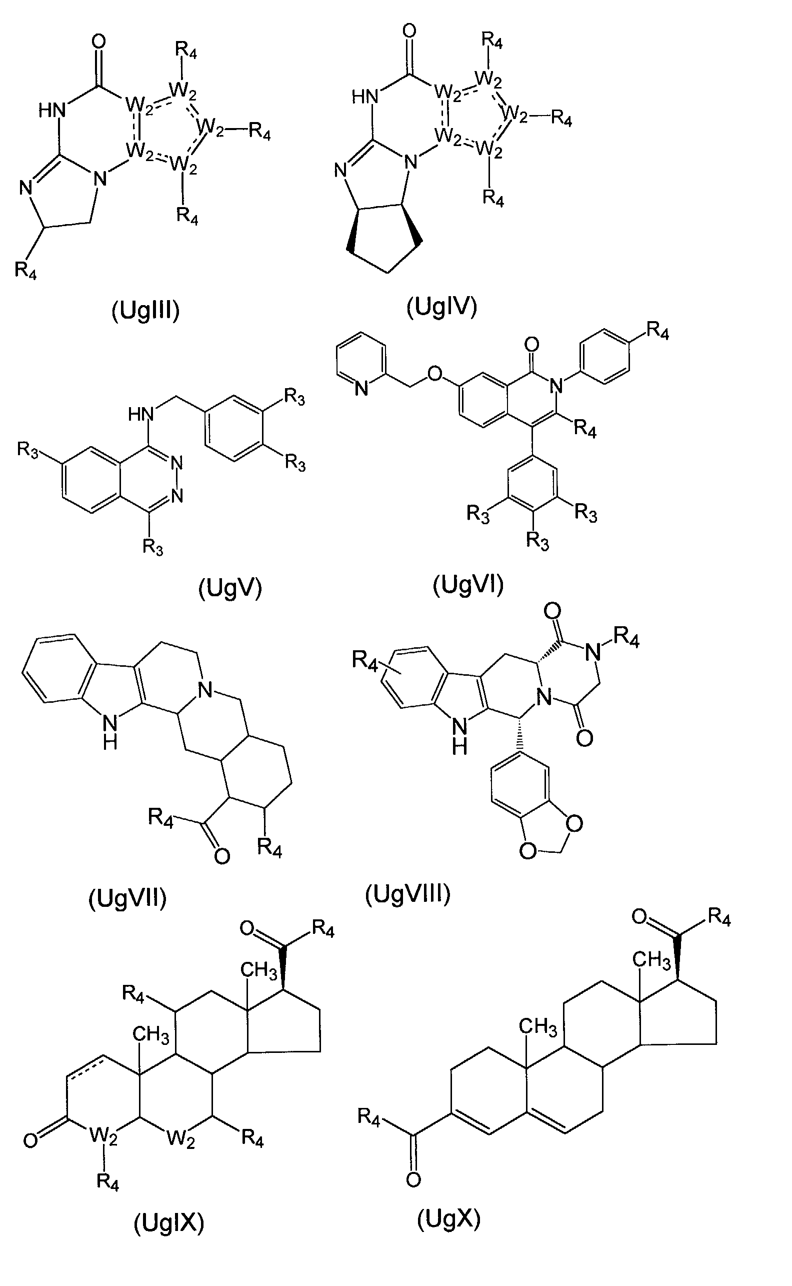

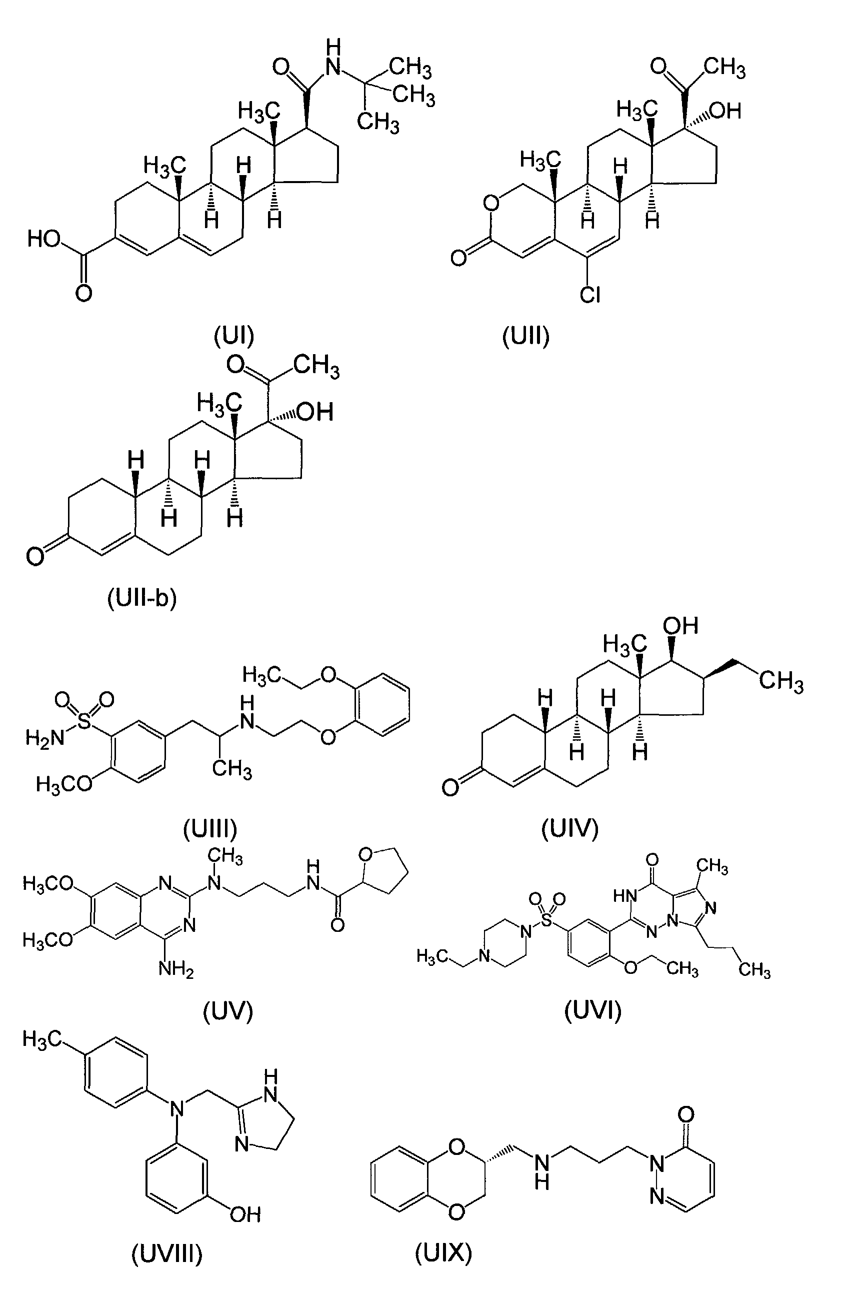

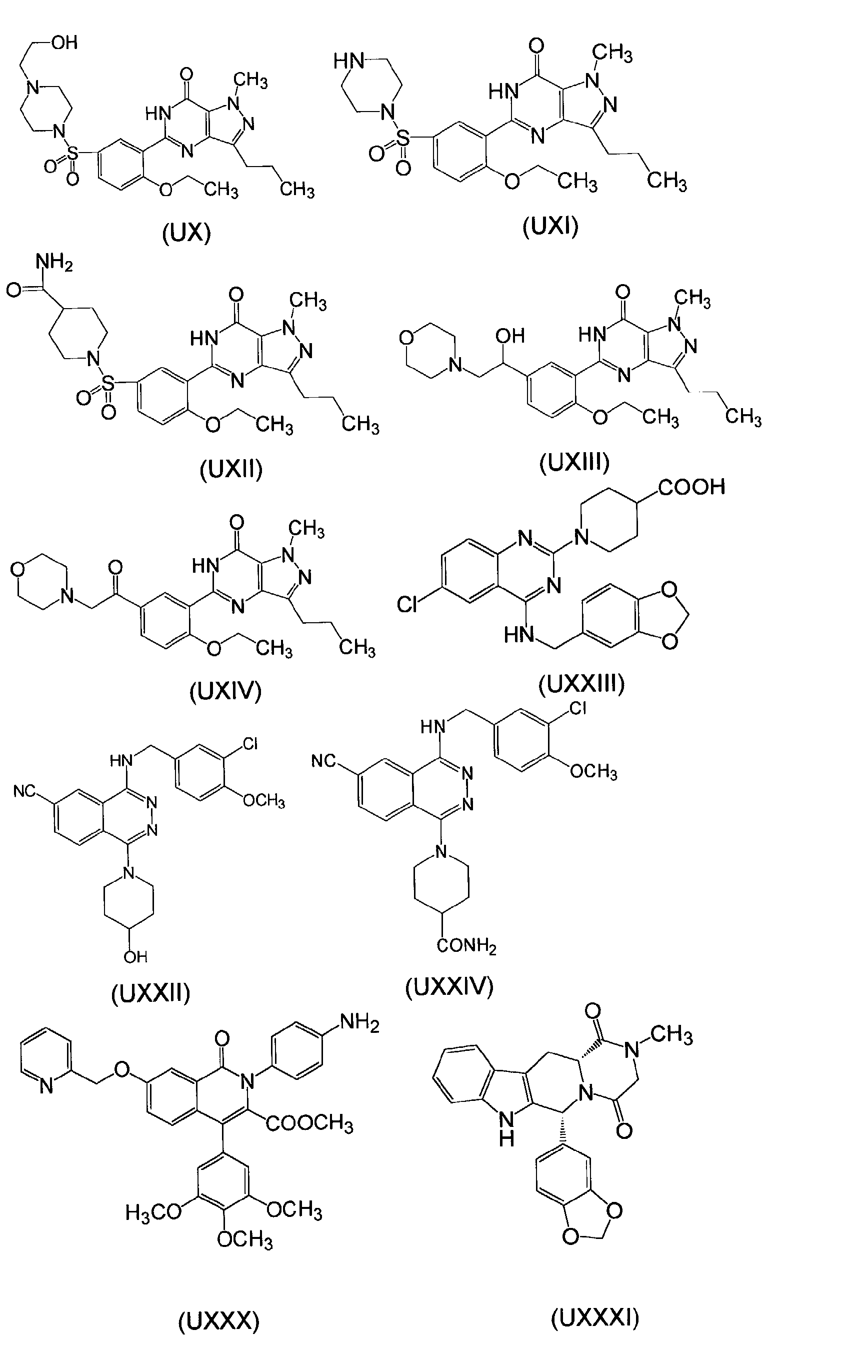

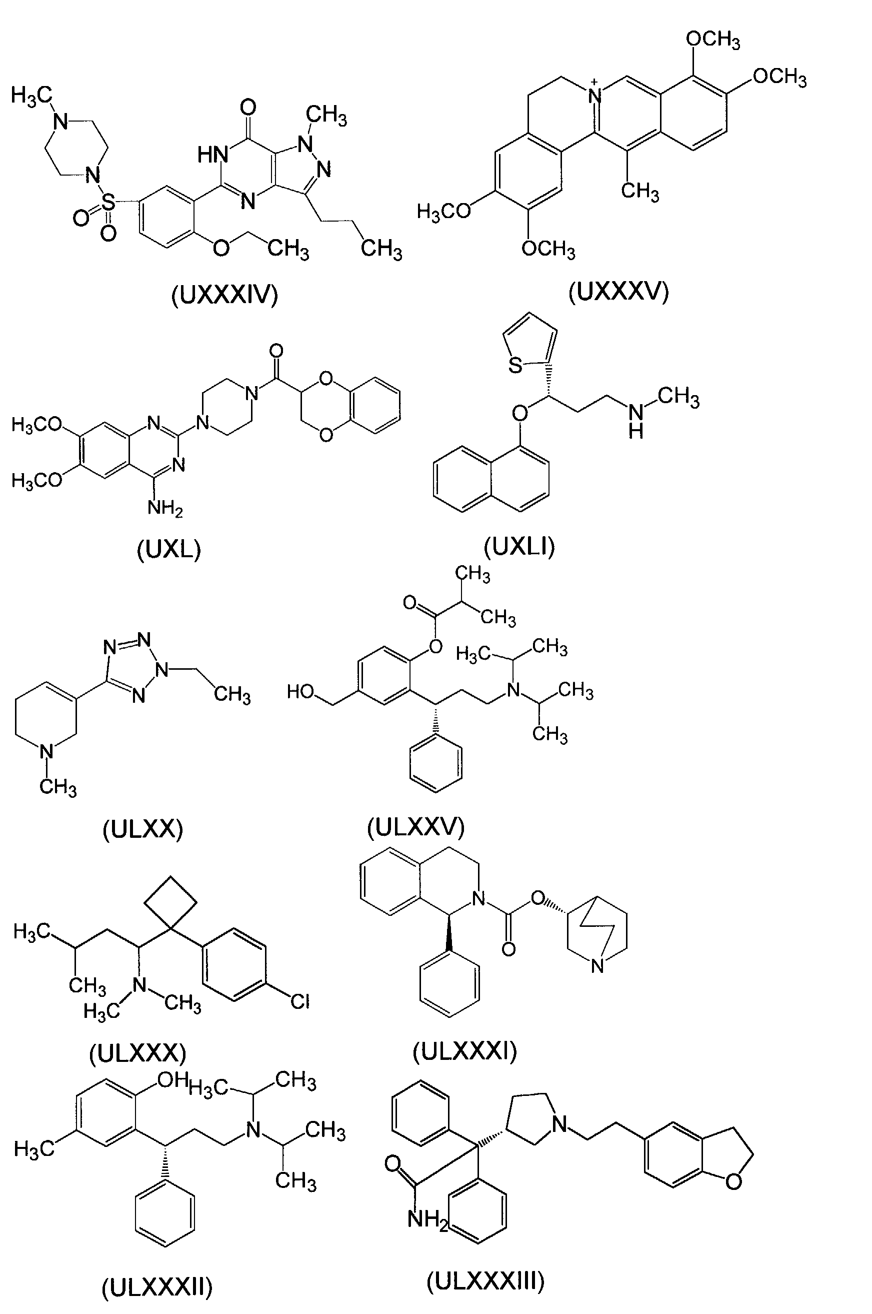

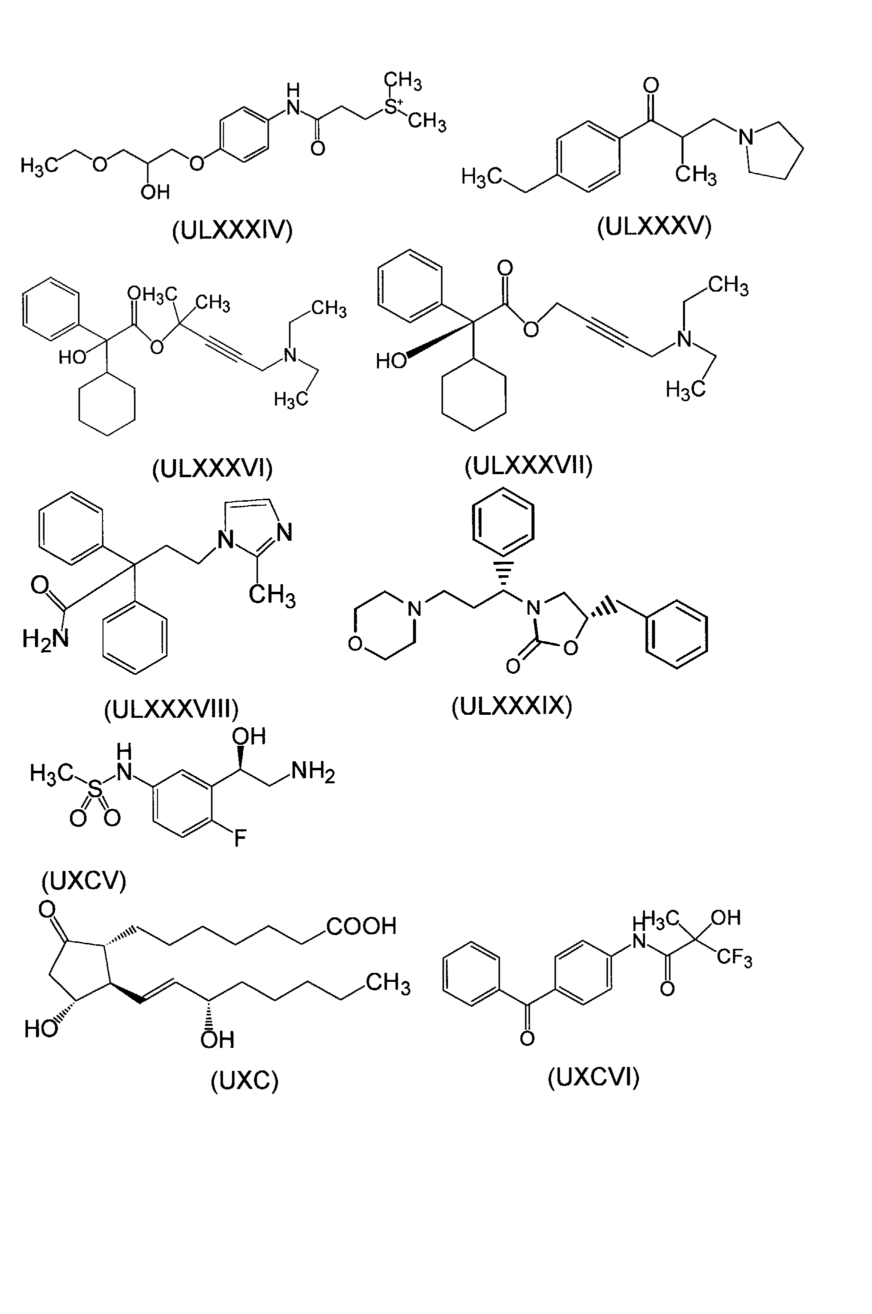

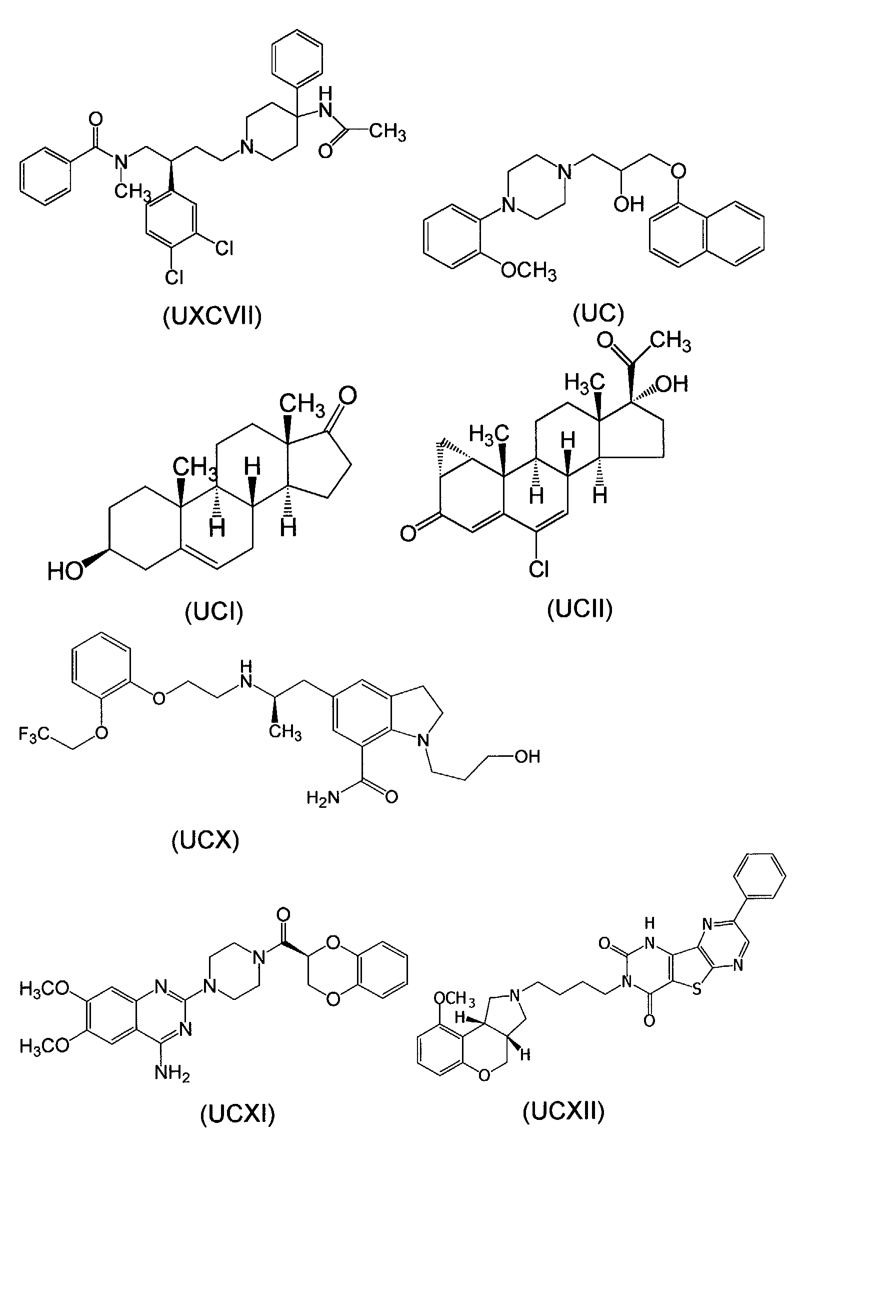

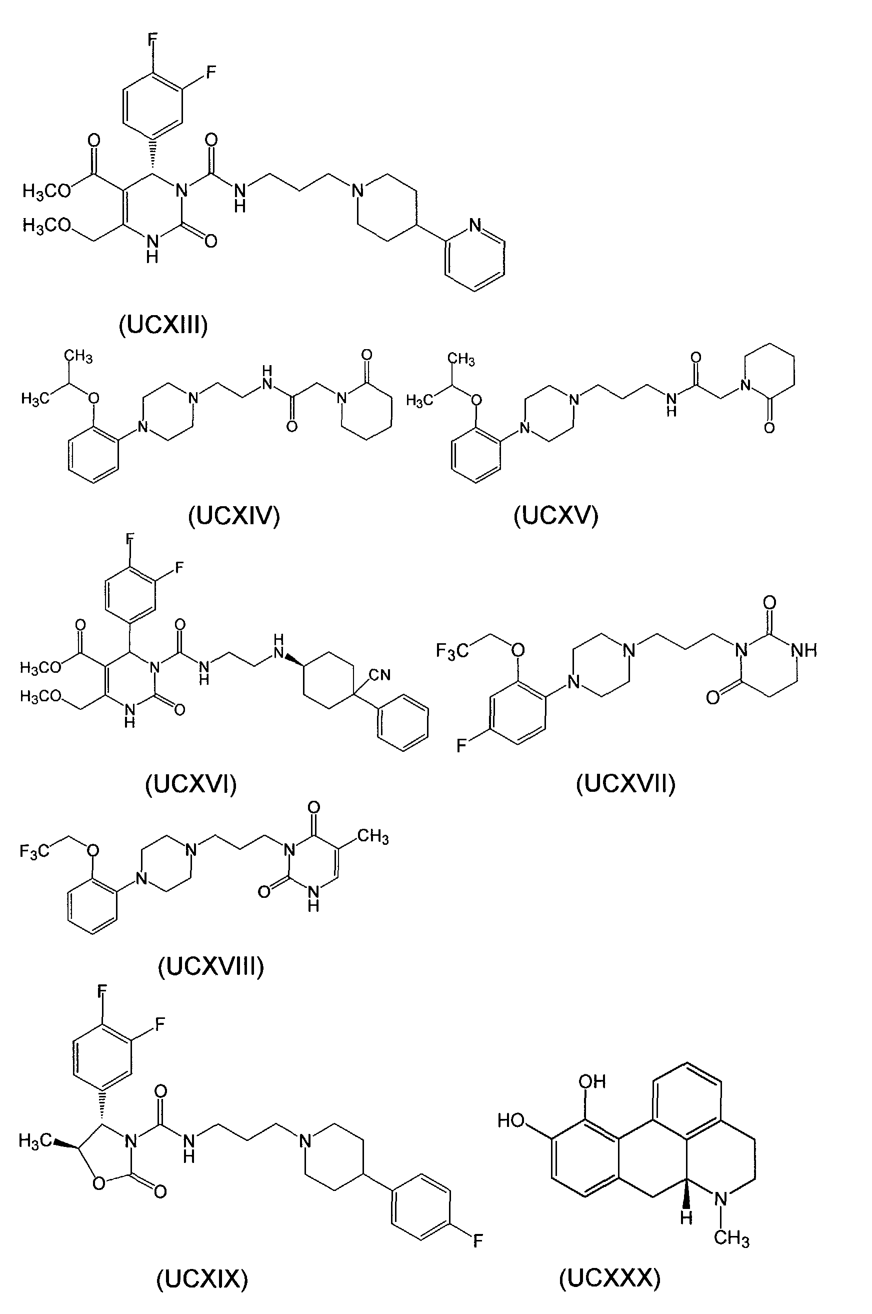

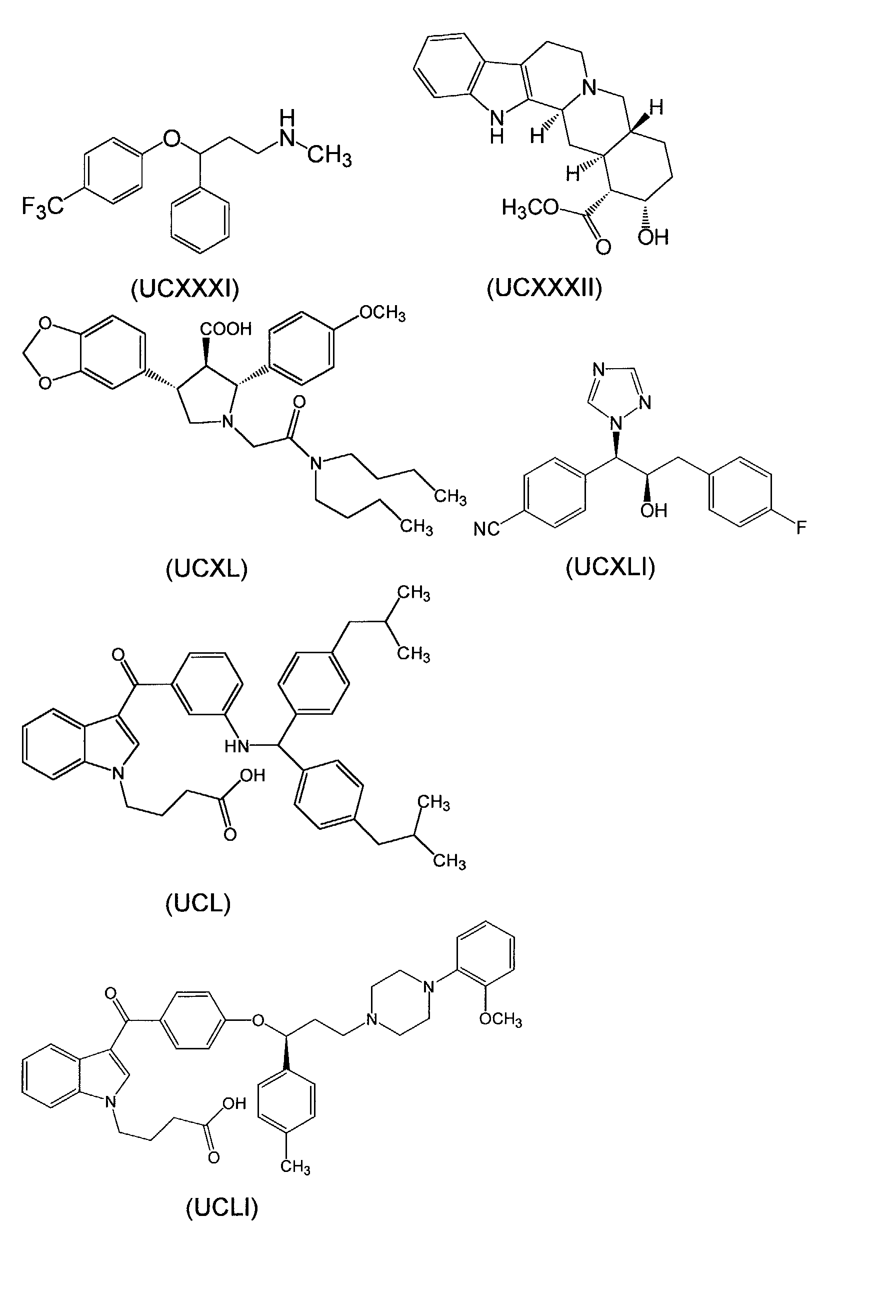

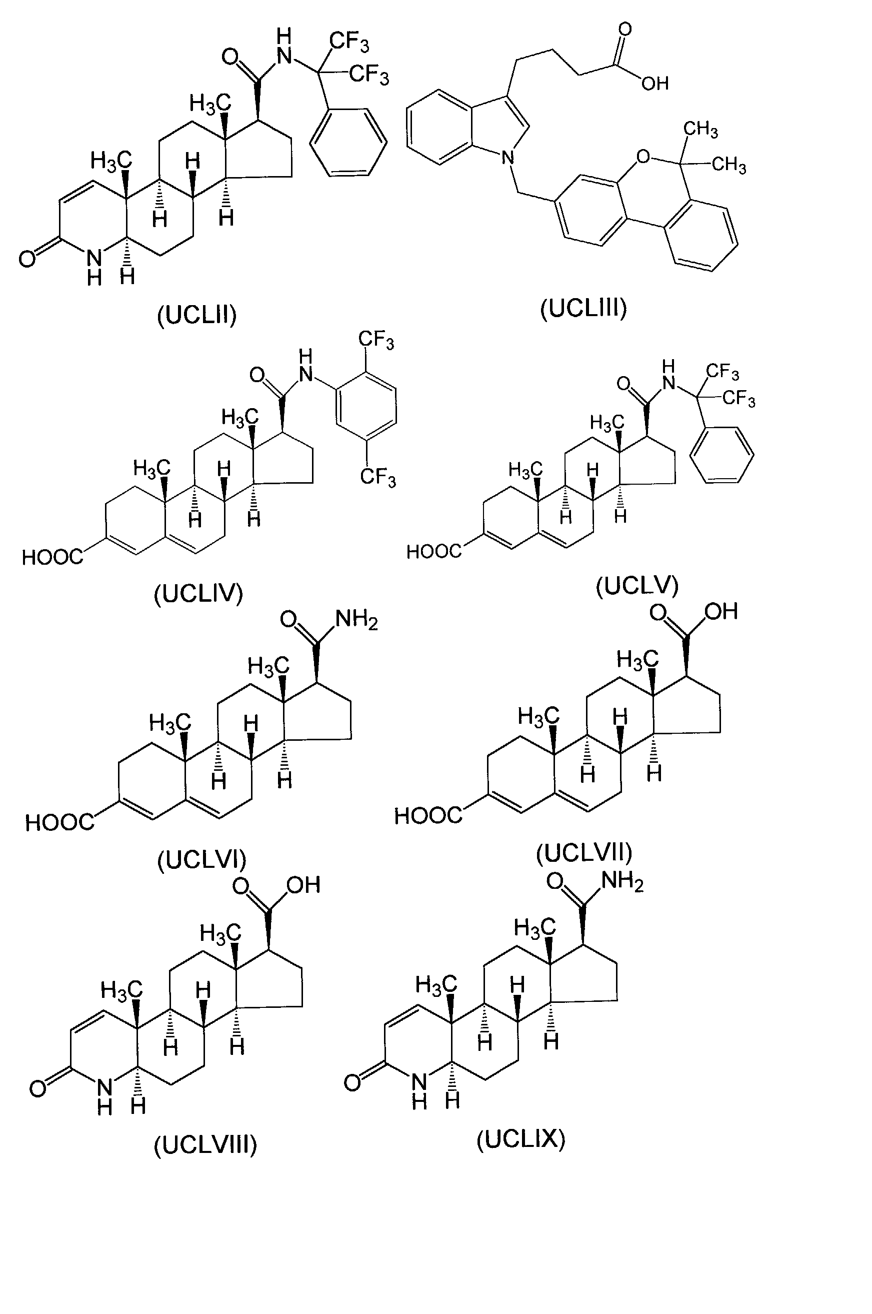

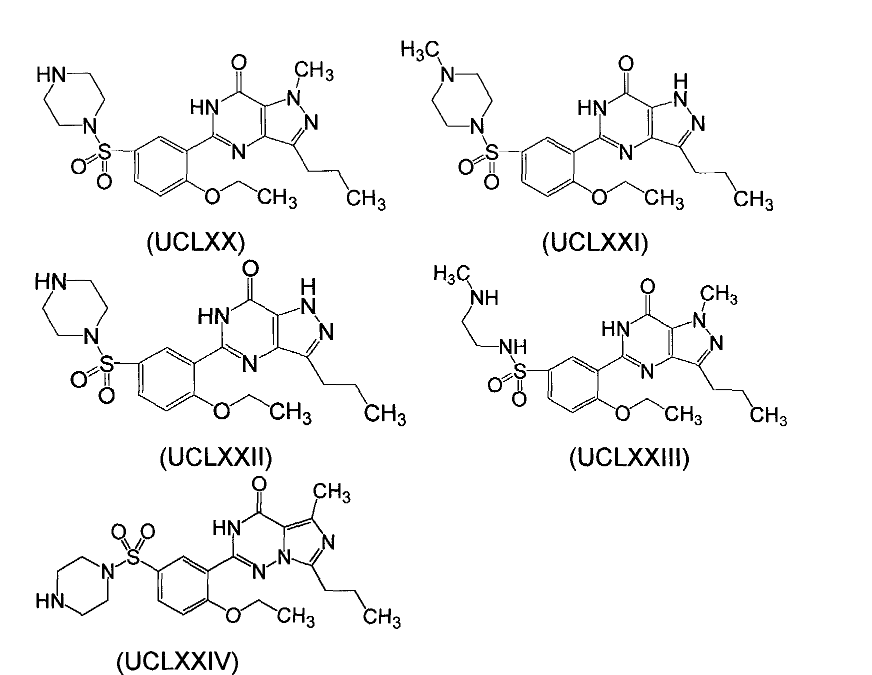

### GROUP 8B:

ABT-839 (CNdXLI), AC-7700 (CNaLXIV), AZD-3409, 3-allylfarnesol (CNdXLVI), amifostine (CNeIII), anecortave (CNaXXVI), 4-aminothalidomide (CNaIL), aminoglutethimide, anastrozole, all-trans retinoic acid (CNbLXXII), 2-(2-amino-3-metoxyphenyl)chromone (CNaXXIV), apigenin (CNbXIX), atrasentan (CNcXIII), asulacrine, BAY 59-8862, BB-3644, BMS-214662 (CNdL), BMS-275291, BBR-2778 (CNdXXI), BBR-3438 (CNdXXIV), BBR-3464, BBR-3576, bicalutamide (CNvII), buserelin, butyric acid (CNvIV), camptothecin (CNdll), 9-aminocamptothecin (CNdIII), carboxyamidotriazole (CnaXXII), capecitabine, chlorogenic acid (CNbL), chrysin (CNbXX), CP-461 (Cndl-b), CP-609754, CI-994, CI-1033 (CNcXII), CGP-77675, cilengitide (CNaXXIII), cipemastat, cladribine, combretastatin A4 (CNaLXI), combretastatin A4 phosphate (CNaLXIII), CT-2584, curcumin (CNbLIII), 5,6-dimethylxanthenone-4-acetic acid (CNaLXV), flavinic derivatives (CNbgl), flavonolic and flavonic derivatives (CNbgX), isoflavonic derivatives (CNbgXI), indolinonic derivatives (CNagl), flavanonolic and flavanonic derivatives (CNbgXII), flavanolic derivatives (CNbgXIII), anthocyanidine derivatives (CNbgXIV), thalidomide derivatives (CNagXL), 2-phthalimidino-glutaric acid derivatives (CNagXLI), phthalamidic derivatives (CNagXLII), glucose derivatives (CNdgXL), fructose derivatives (CNdgXLI), galactose derivatives (CNdgXLII), retinoid derivatives (CNbLXX), dexrazoxane (CNeI), 4-dedimethylamino-sancycline (CnaXX), diflomotecan (CNdVII), all-cis-5,8,11,14,17-eicosapentaenoic acid (CNbLXI), daidzein (CNbXXXII), docetaxel, doxorubicin (CNdXXII), D-1927, edatrexate, eflornithine (CNbLXIX), ellagic acid, EO-9 (CNdLXXX), EM-12 (CNaXLV), E-7010, epicatechin (CNbXXVI), ellagic acid, epigallocatechin-3-gallate (CNbXXVII), epirubicin, BMS-247550, BMS-214662, BMS-275291, ERA-923, erbstatin (CNcIV), erlotinib, exemestane, exatecan (CNdVI), fadrozole, fenretinide (CNbLXXIV), finrozole (CNbLXVII), flavin-adeninin-8α-hydroxy-riboflavin (CNbIII), flavine-adenine dinucleotide/FAD, flavone-8-acetic acid (CNaLXVII), flavopiridol (CNaXXV), floxuridine, 5-fluorouracil, formestane (CNbLXIII), 1-fructosamine, (CNdLXXVIII) 2-fructosamine (CNdLXXVI), fustin (CNbXXIV), fumagillol (CNaLXIV), fulvestrant, genistein (CNbXXI), gefitinib, gemcitabine (CNdXXX), 1-glucosamine (CNdLXXIII), glutathione (CNbgXXXV), 2-glucosamine (CNdLXXIV), 6-glucosamine (CNdLXXV), 2-galattosamine (CNdLXXVII), halofuginone (CNaLX), histamine (CNvI), 4-hydroxybutirroxymethyl-retinate (CNbLXXII), 5'-hydroxythalidomide (CNaXLIV), ILX-23-7553, IDN-5109, imatinib/STI-571 (CNcII), imiquimod, hyperin (CnbIL), human endostatin 6-49, indanocine (CNaVI), kaempferol (CNbXVI), kynurenic acid (CNbXXX), ketotrexate, 3-indolemethanol (CNbLXV), irinotecan (CNdIV), irofulven, itriglumide/CR-2945 (CNbXC), leuprorelin, letrozole (CNbLXVIII), losoxantrone (CNdXXIII), ledoxantrone, luteolin (CNbXVIII), lurtotecan, LY-294002 (CNcVI), L-778123 (CNdXL), marimastat (CNcI), megestrol, methotrexate, MEN-11066, mitoxantrone (CNdXX), midostaurin, mivobulin, myricetin (CNbXVII), myo-inositol (CNbLX), N-(4-carboxyphenyl)-retinamide (CNbLXXI), N-hydroxymethyl-thalidomide (CNaXLVII), N-hydroxyphthalimide (CNaXLVIII), N-acetylcysteine, naringenin (CNbXXII), nelzarabine, octreotide (CNaLXVII), oltipraz, oroxylin (CNbXXVIII), OSI-774, 2,2,5,7,8-pentamethyl-6-hydroxy-chromane (CNbXLIVI), oxandrolone (CNfI), paclitaxel, phenylbutyrate, PD-089828, PD-153035, pemetrexed, perillyl alcohol (CNbLXXV), quercetin (CNbXV), R-115777 (CNdXLII), raltitrexed, razoxane, resveratrol (CNbLI), riboflavin (CNbII), riboflavin monophosphate (CNbIV), roquinimex, RPR-130401 (CNdXLV), 2-phthalimidino glutaric acid (CNaXLVI), piroxantrone, prinomastat (CNcIII), riboflavin tetrabutyrate, riboflavin triacetate (CNbV), rubitecan (9-nitrocamptothecin) (CNdV), PTK-787 (CNaXXI), Sch-66336 (CNdXLIII), β-sitosterol (CNbLXII), seocalcitol (CNdXC), squalamine (CNbLXIV), semaxanib/SU-5416/semoxind (CNall), SCH-57068, silybin (CNbXXXI), STI-481 (CNdX), SPIKET-P (CNcVII), SPC-8490, SCH-66336, SCH-57050, S-3304, SU-6668 (CNaIII), SU-5406, SU-5614, SU-5477, SU-5402 (CNaXI), SU-4793, SU-5204, SU-4942, SU-4984, SU-5212, SU-4312, sulfuretin (CNaXV), sulindac solfone/exisulind (CNdI), tesmilifene, tamoxifene, toremifene, tanomastat (CNcVIII), TAS-103, taxifolin (CNbXXIII), teloxantrone, tezacitabine, theaflavin (CNbXXIV), thalidomide (CNaXLIII), thymitaq, tirilazad (CNbLXIX), α-tocopherol (CNbXL), trolox C (CNbXLI), 2 tocotrienoli (CNbXLII) e (CNbXLIII), topotecan (CNdXXV), triptorelin, trans ferulic acid (CNbLII), triapine, trimetrexate, troxacitabine, ubenimex, 7-hydroxystaurosporine/UCN-01 (CNcV), vorozole, vesnarinone, vitamin E acid succinate (CNbXLIV), VX-710, YM-511, wogonin (CNbXXIX), ZD-1839/Iressa (CNcIX), ZD-4190 (CNcXI), ZD-6126, ZD-9331, ZK-222584, (CNaIV), (CNaV), (CNaVII), (CNaXII), (CNaXIII), (CNaXIV), (CNaXVI), (CNaXLb), (CNagL), (CNaLXII), (CNbgXXXII), CNdVIII), (CNdIX), (CNdXLIV) (CNdXLVII), (CNdXLVIII), (CNdXLIX), (CNbXXXV), (CNbXXXVI), (CNbXXXVII), (CNbXXXVIII), (CNbXXXIX), (CNvIII), (CNcX) 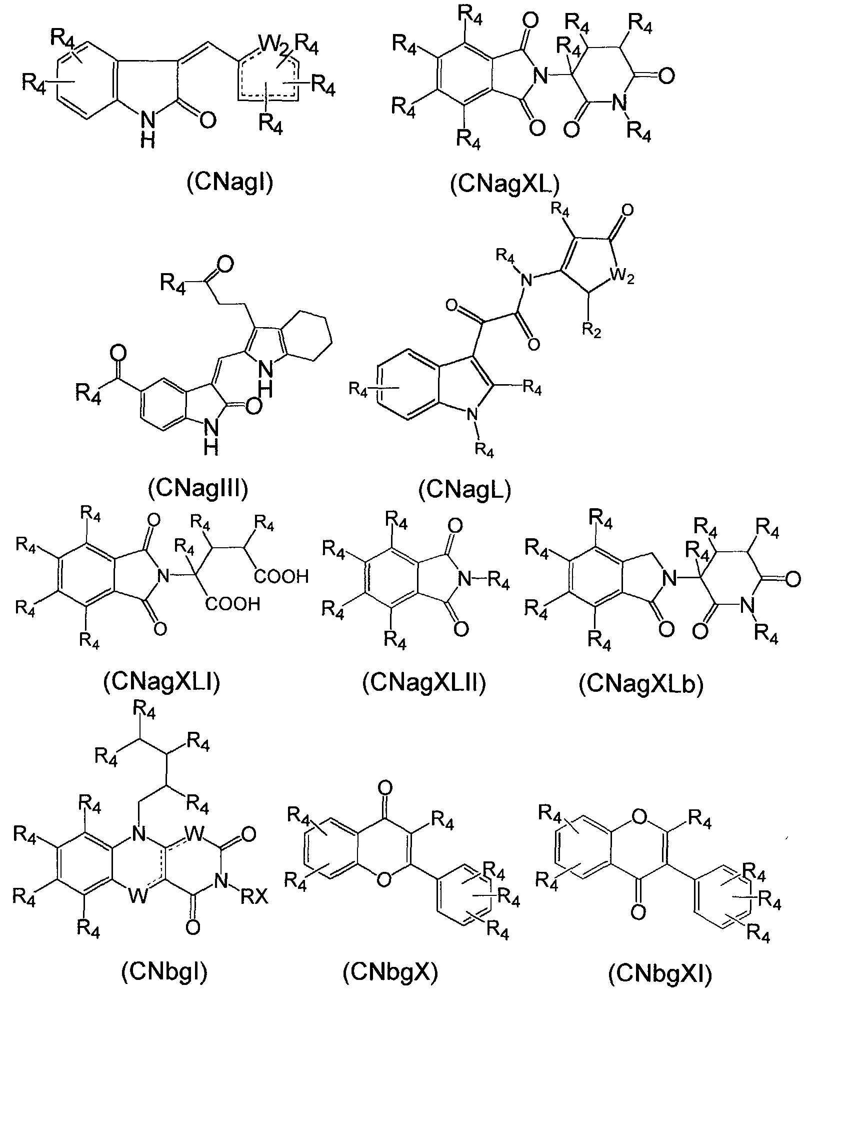 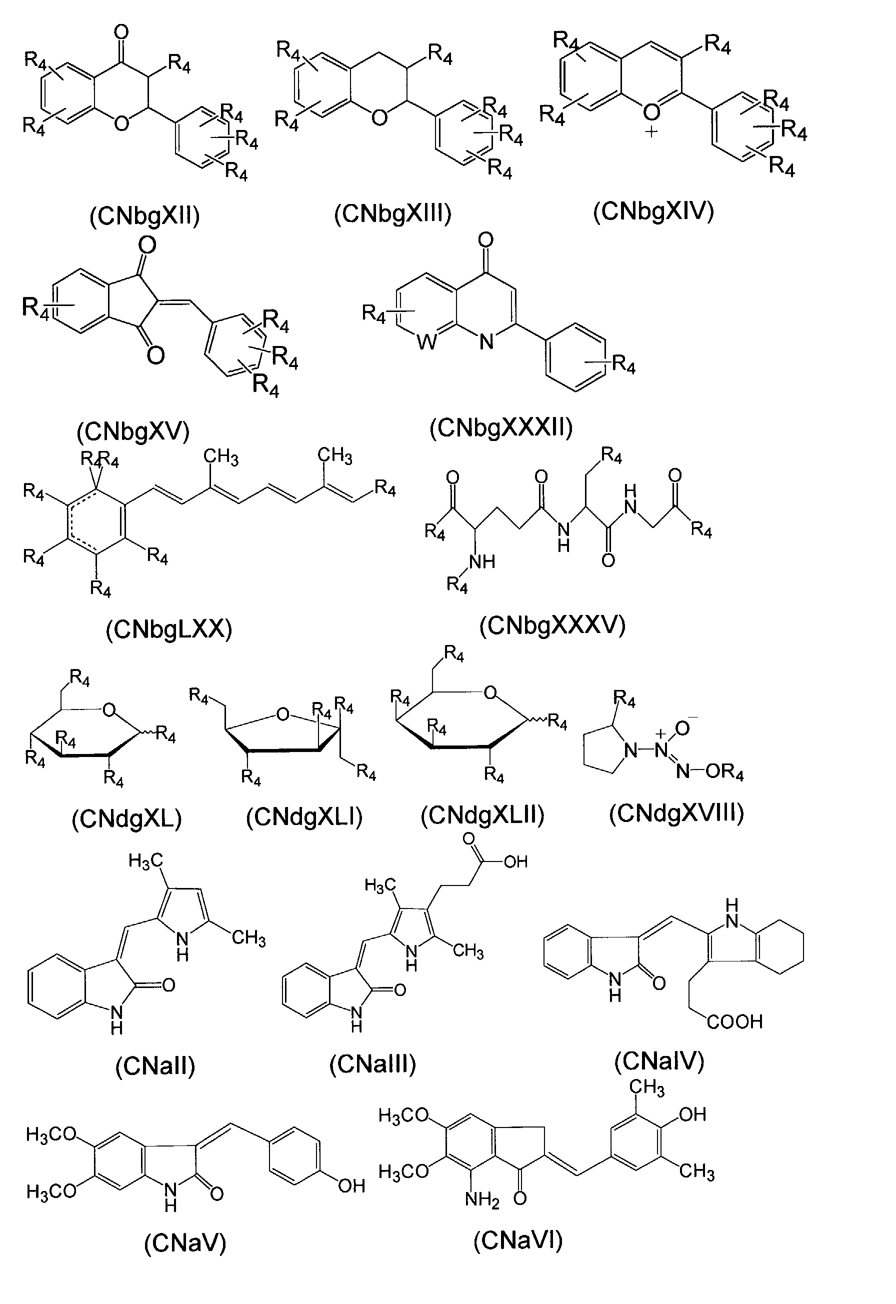 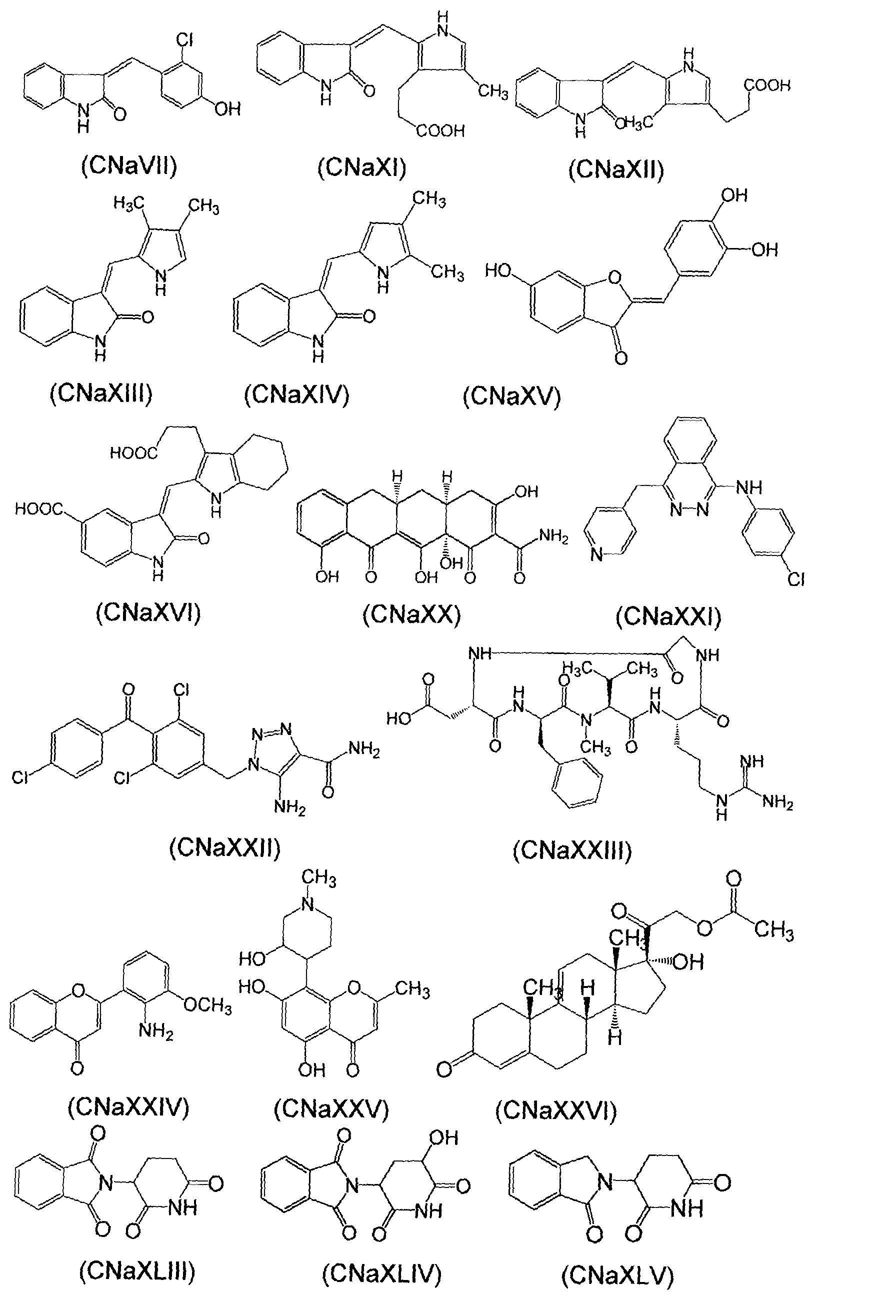 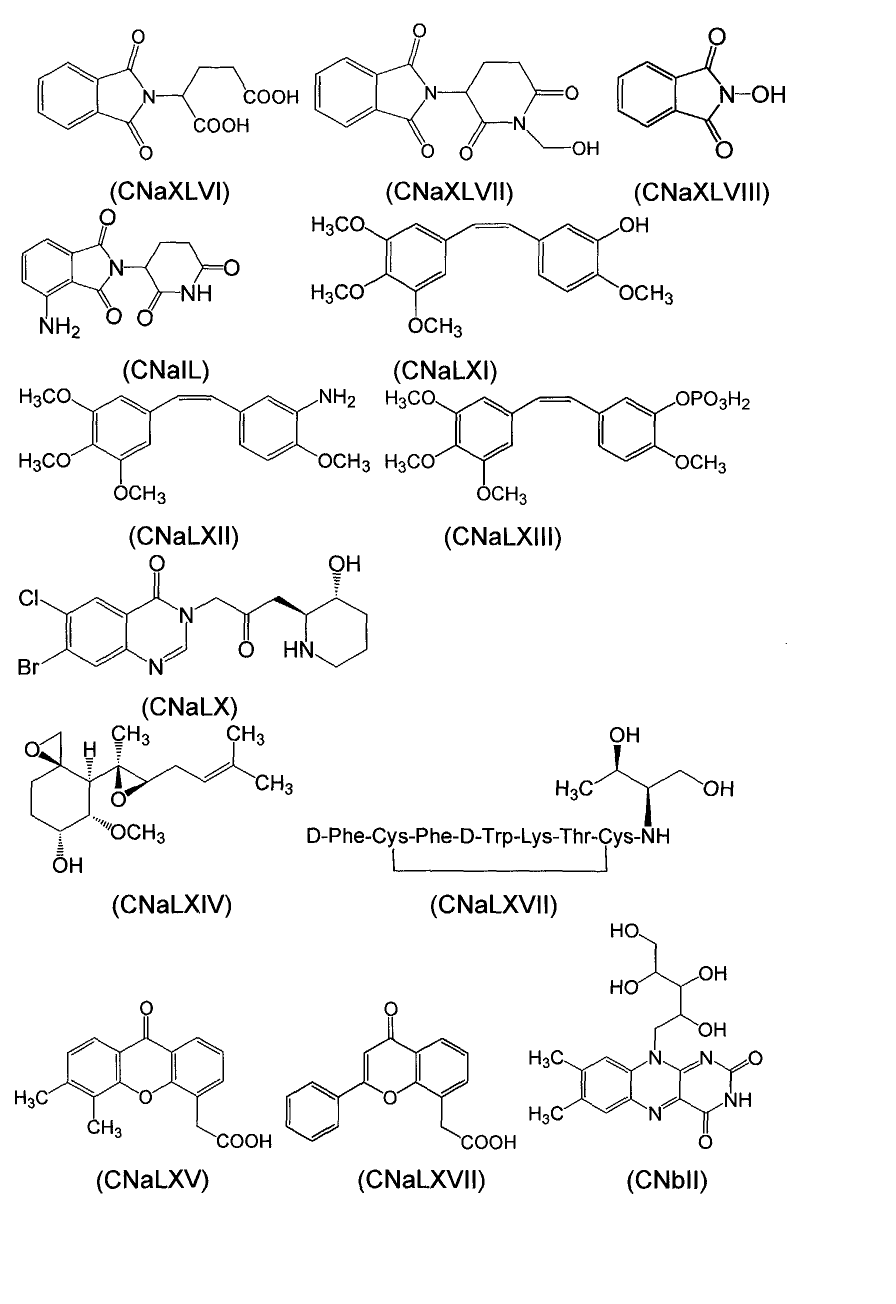 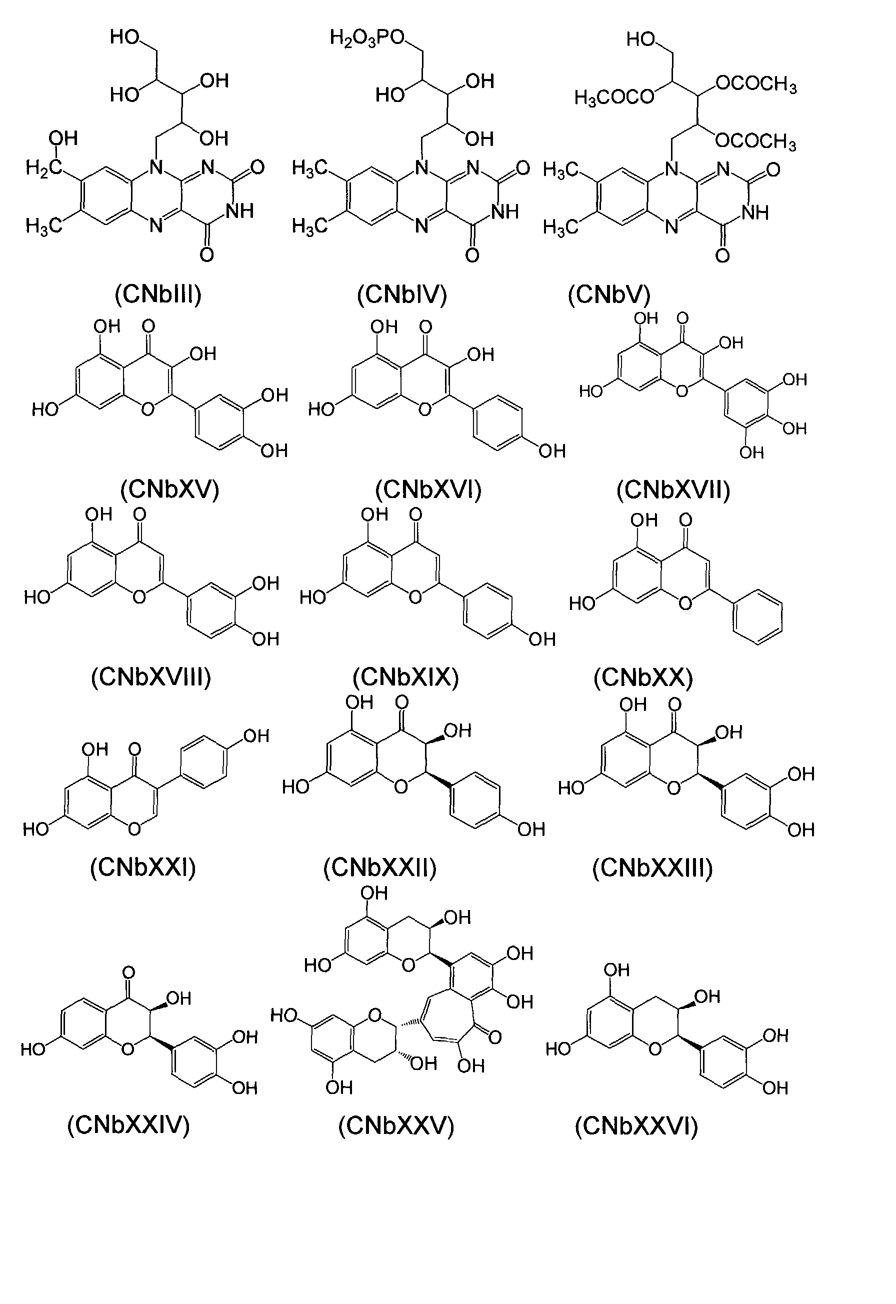 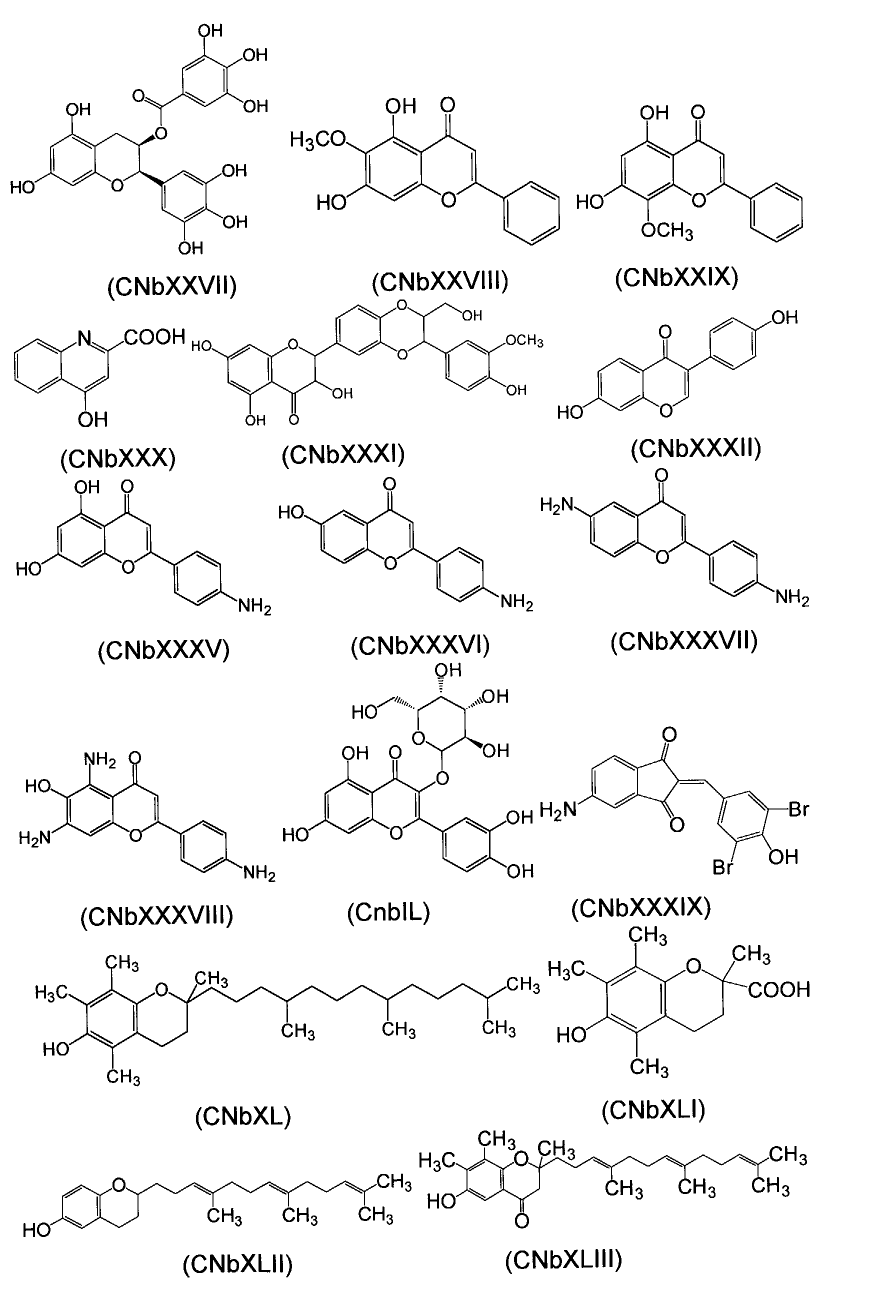 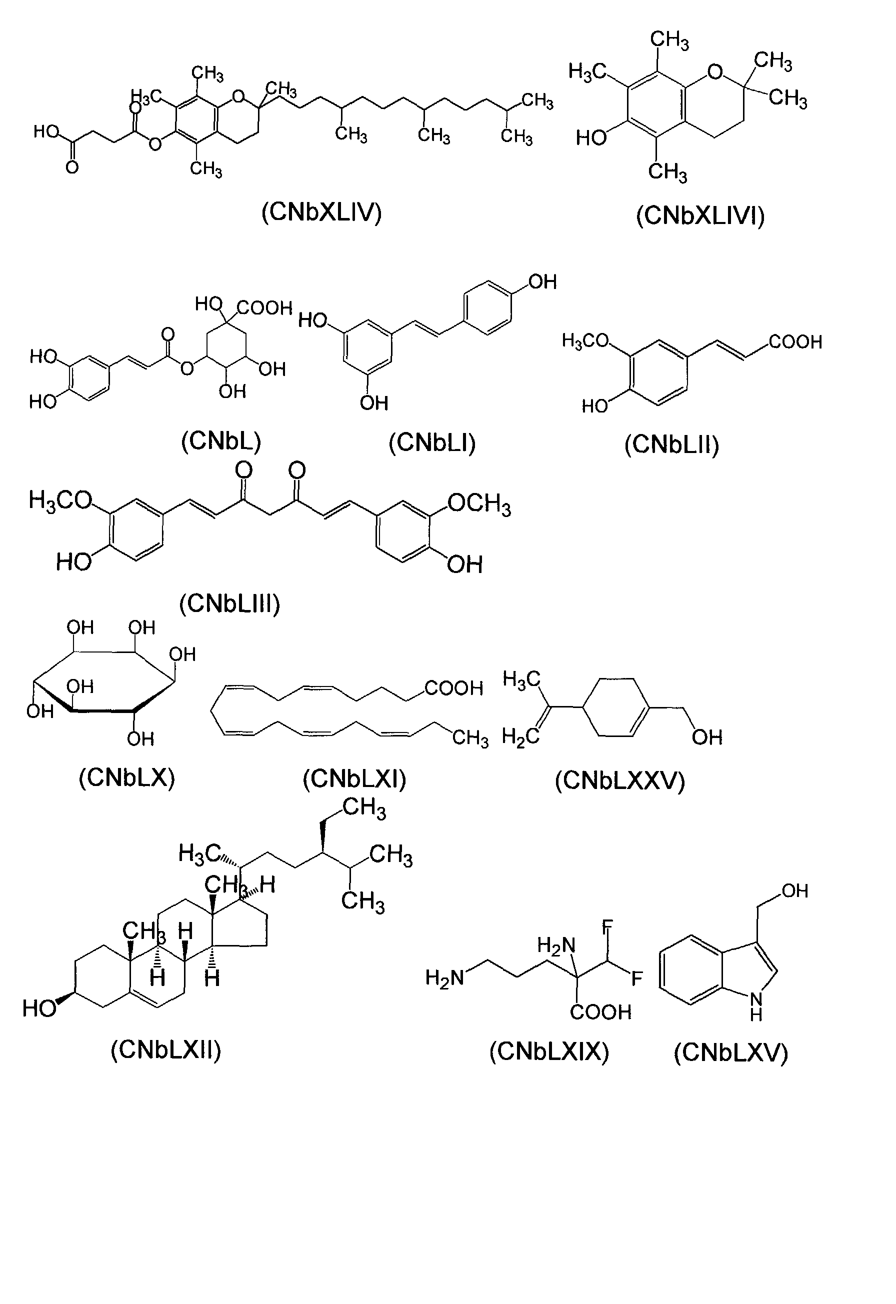 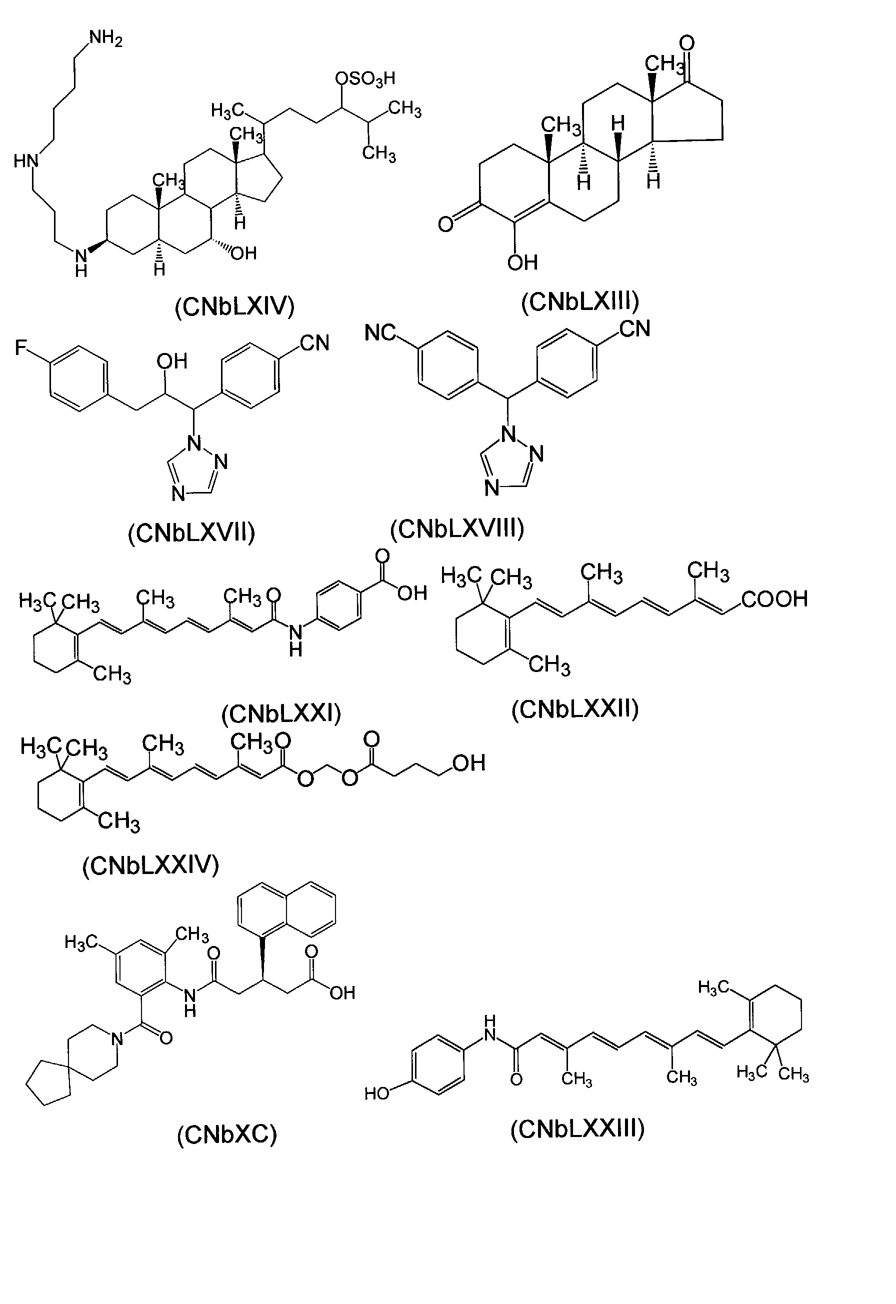 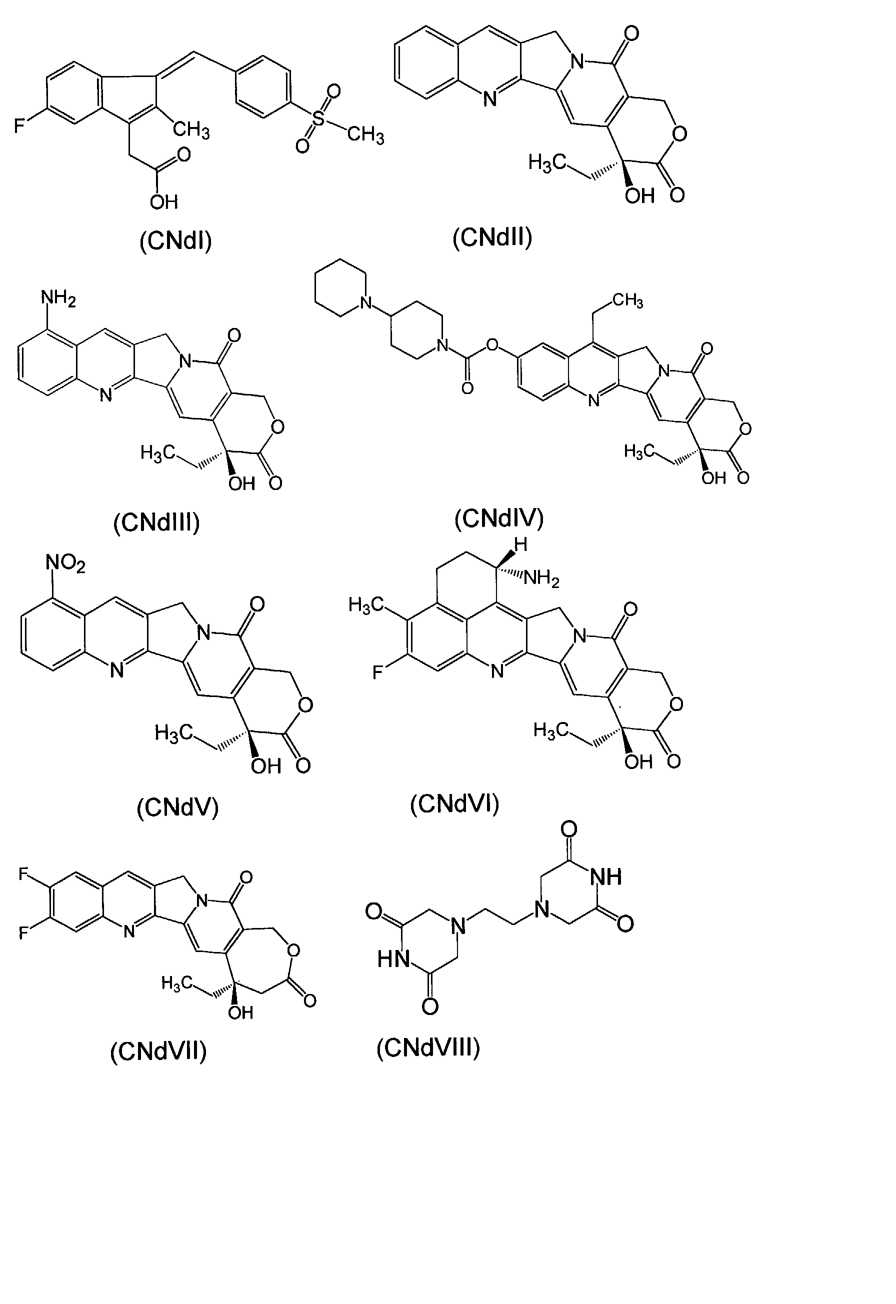 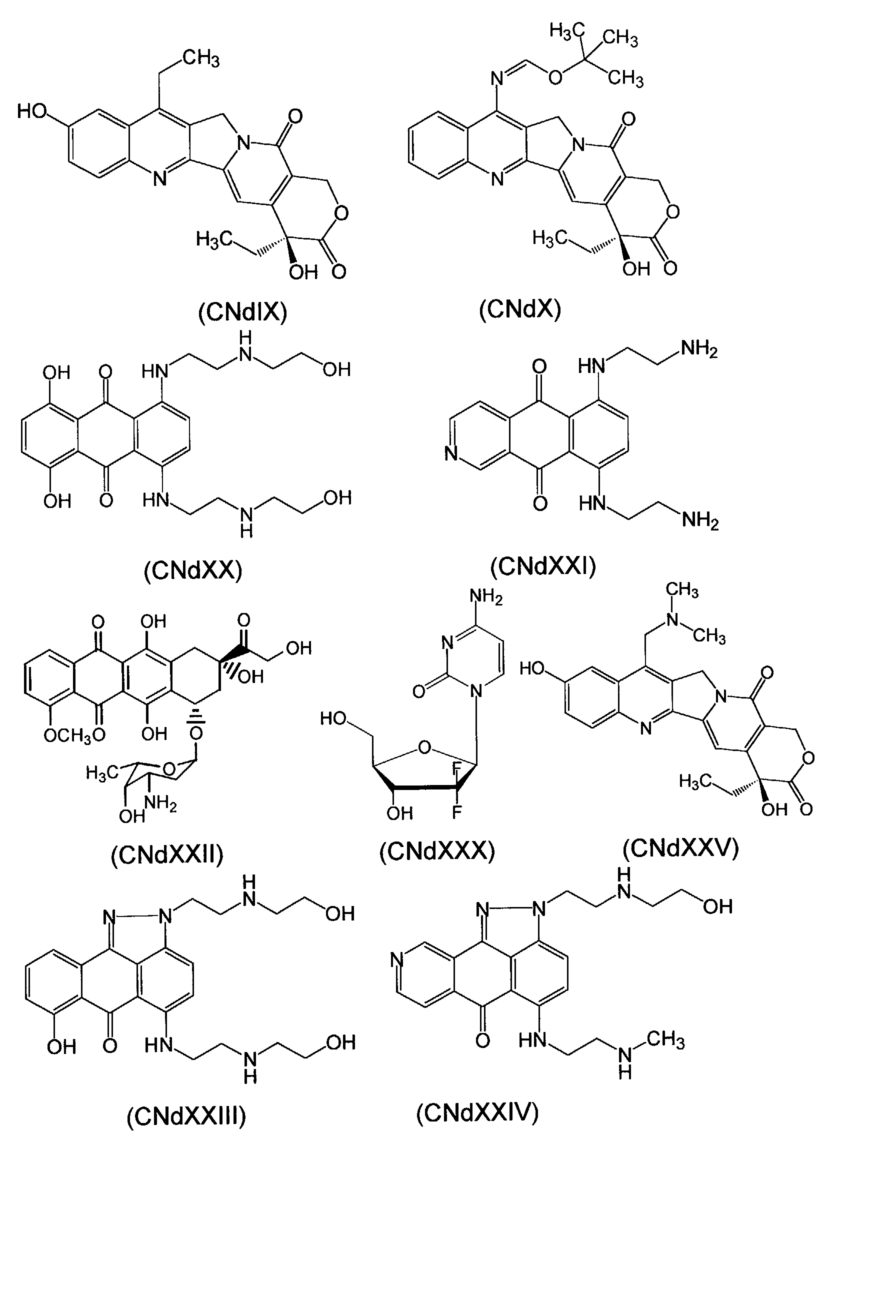 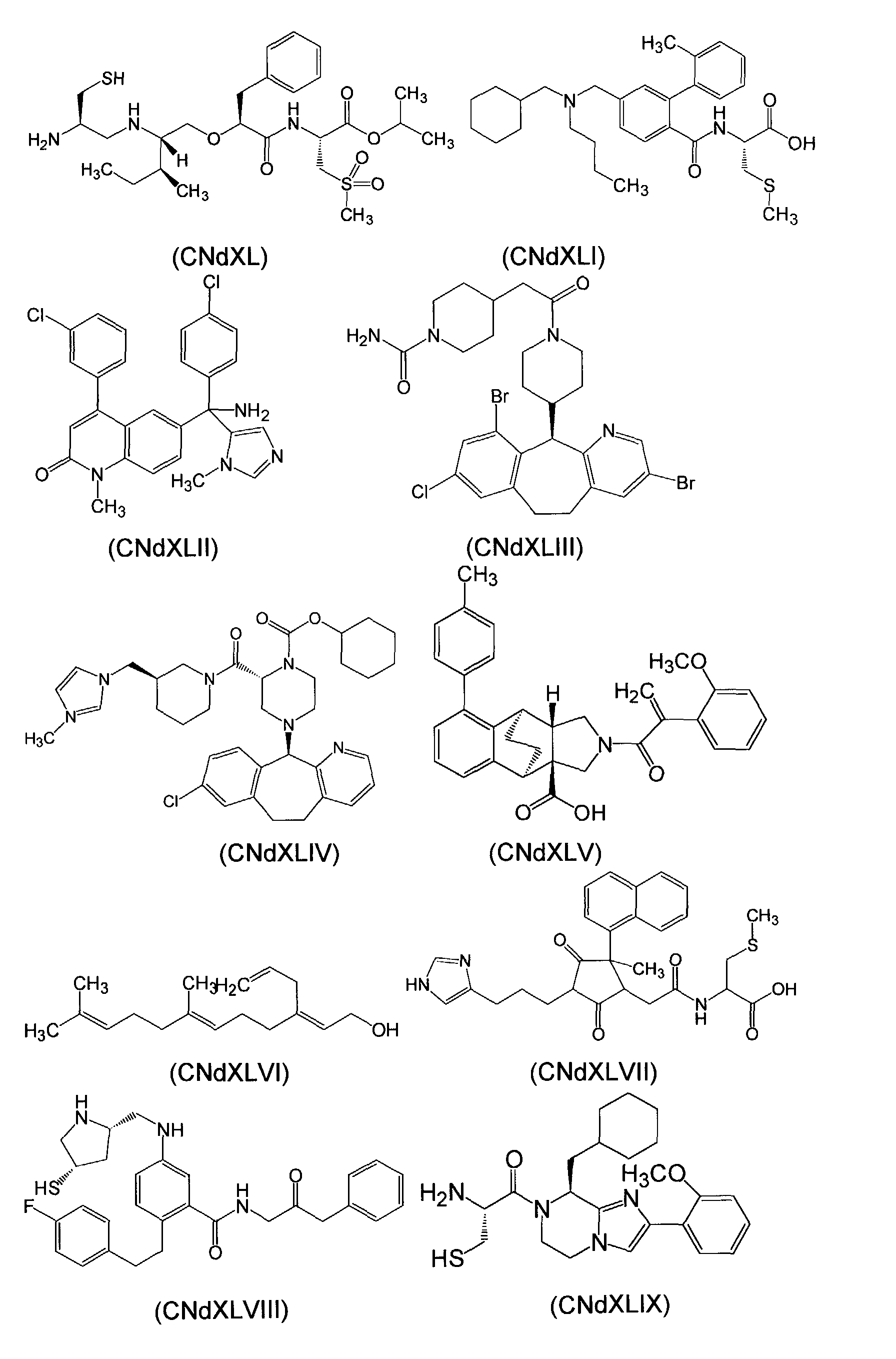 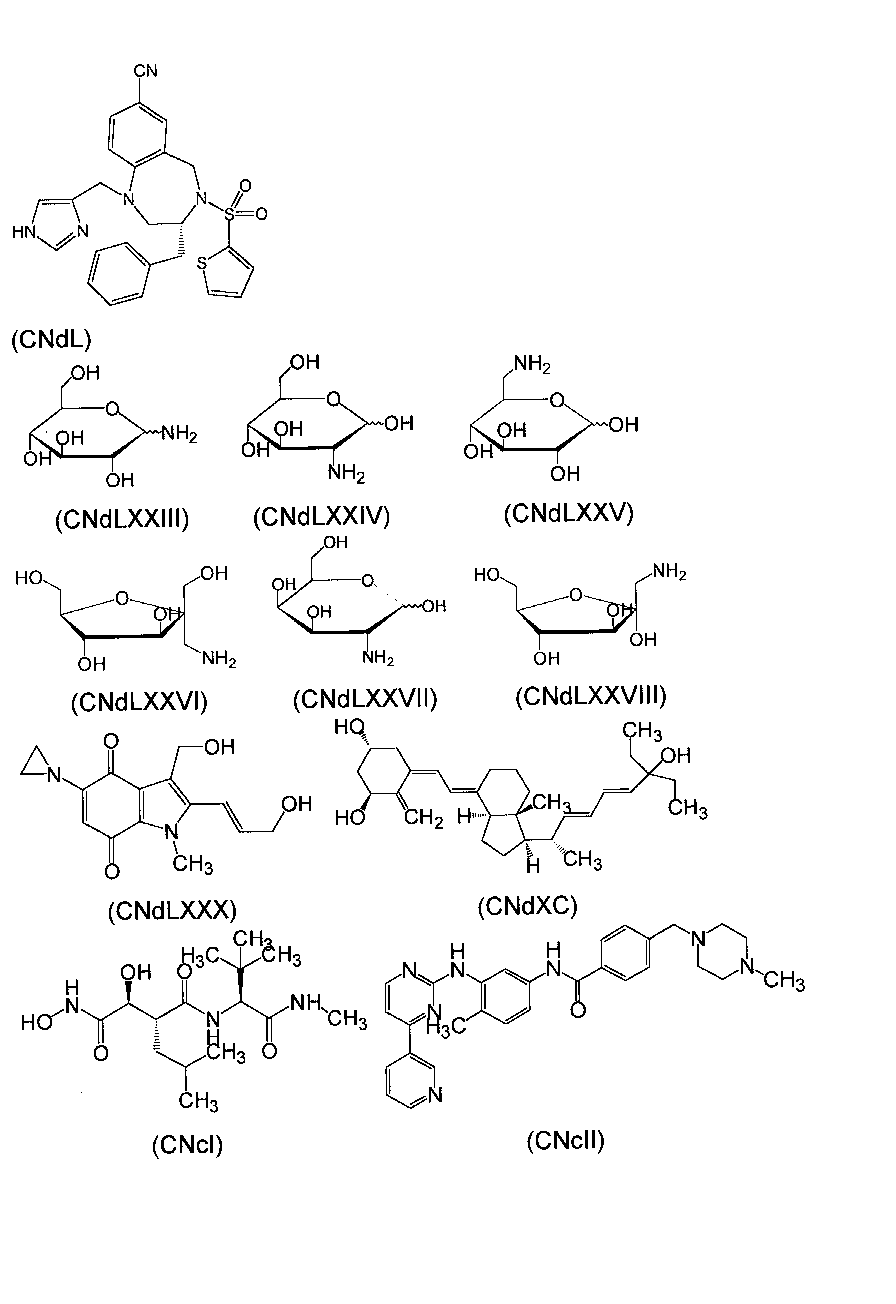 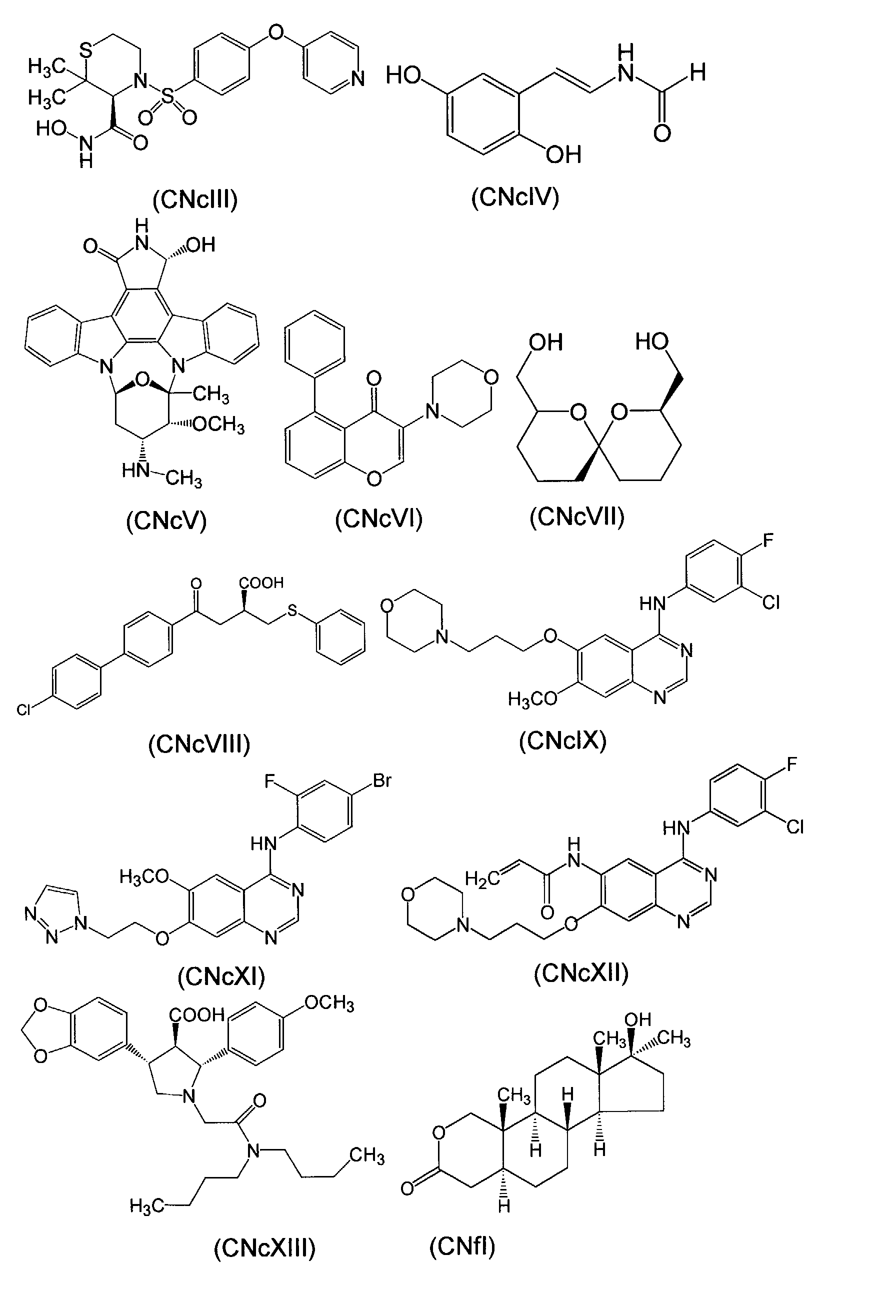 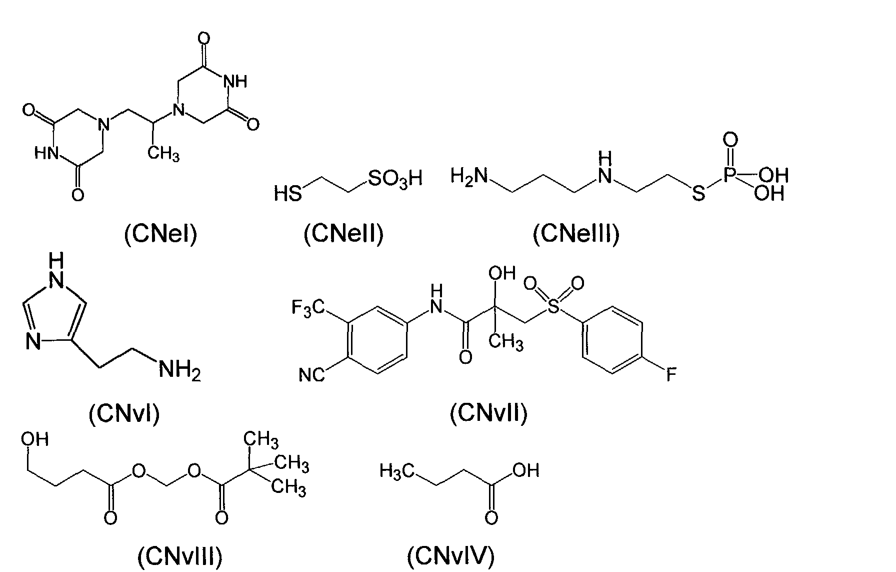 where
- R₄ is chosen among:
   - COOH; -F; -Cl; -Br; -CF₃; -NO₂, -CHO, -CN, -N₃,
   - R₂; -O-R₂; -O-(CO)-R₂; -N(R₂)₂; -N(R₂)₂-(CO)-R₂; -R₁-O-R₂;
   - R₁-S-R₂; -R₁-NR₂-R₂;-O-R₁-O-R₂;-O-R₁-S-R₂;-O-R₁-NR₂-R₂;
   - S-R₂; -S-R₁-O-R₂; -S-R₁-S-R₂; -S-R₁-NR₂-R₂;-NR₂-R₂;
   - NR₂-R₁-O-R₂; -NR₂-R₁-S-R₂; -NR₂-R₁-NR₂-R₂;-(CO)-R₂;
   - (CO)-O-R₂; -(CO)-R₁-O-R₂;-(CO)-R₁-S-R₂;-(CO)-R₁-NR₂-R₂;
   - PO(O-)O-R₂; -SO₂-R₂; -O-SO₂-R₂; -SO₂-O-R₂;
- R₁, -R₂, W and the dotted line are described as above
- W₂ is -O-, -NH-, -NH=, -N(R₂)-, -CH=CH-, -S-; =CH-
- n is an integer between 1 and 5, preferably 1
- m is an integer between 0 and 5

Preferably the compounds of the invention are chosen among the following 8 groups of compounds (from 1C to 8C):

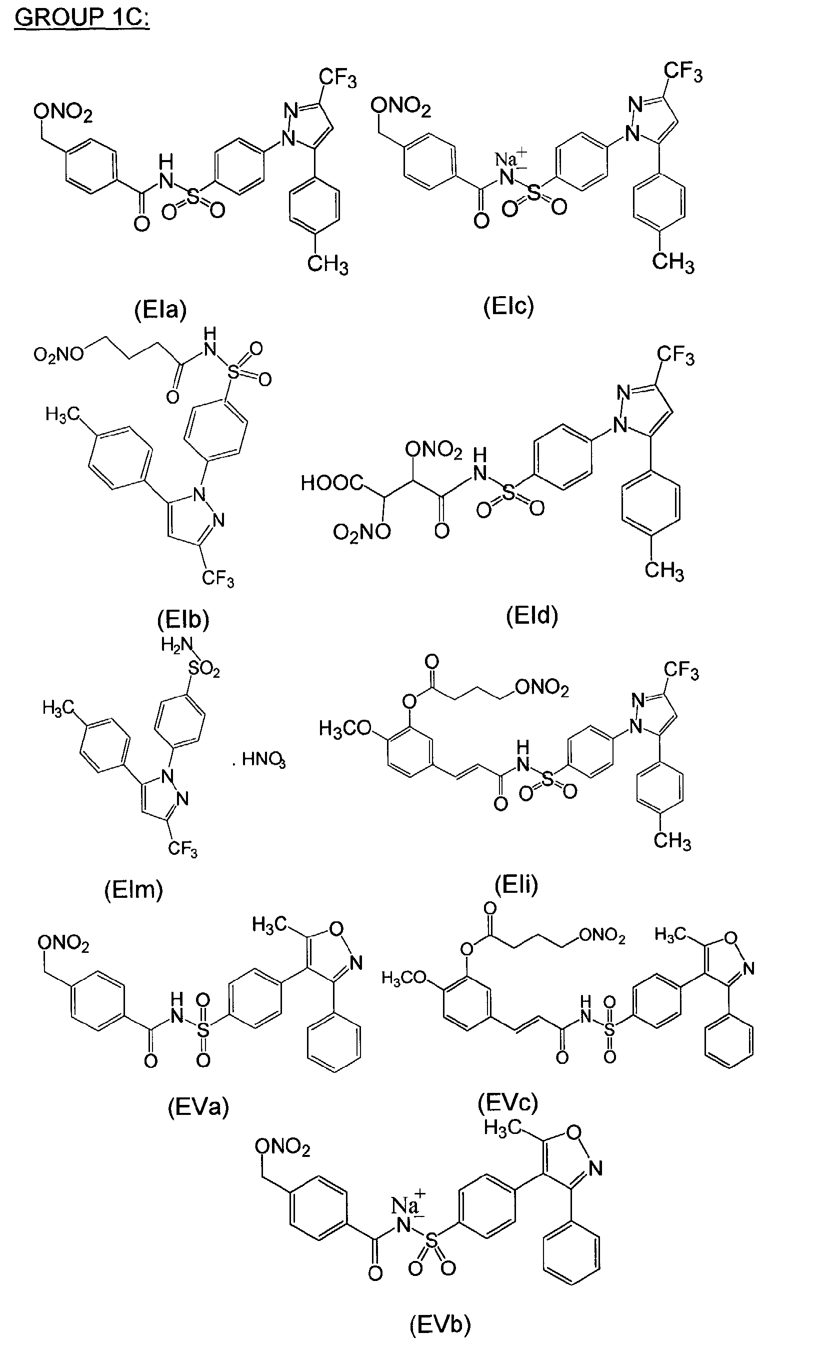

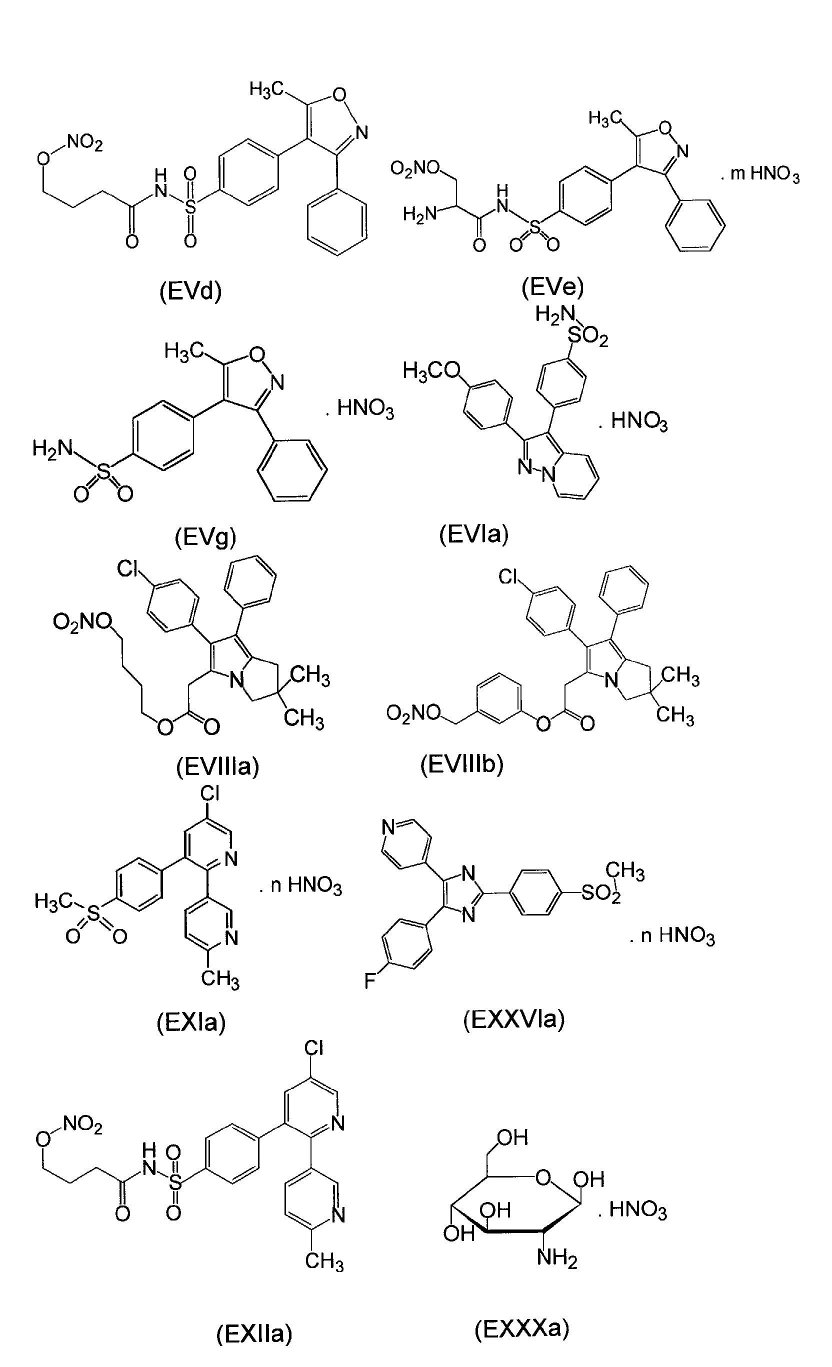

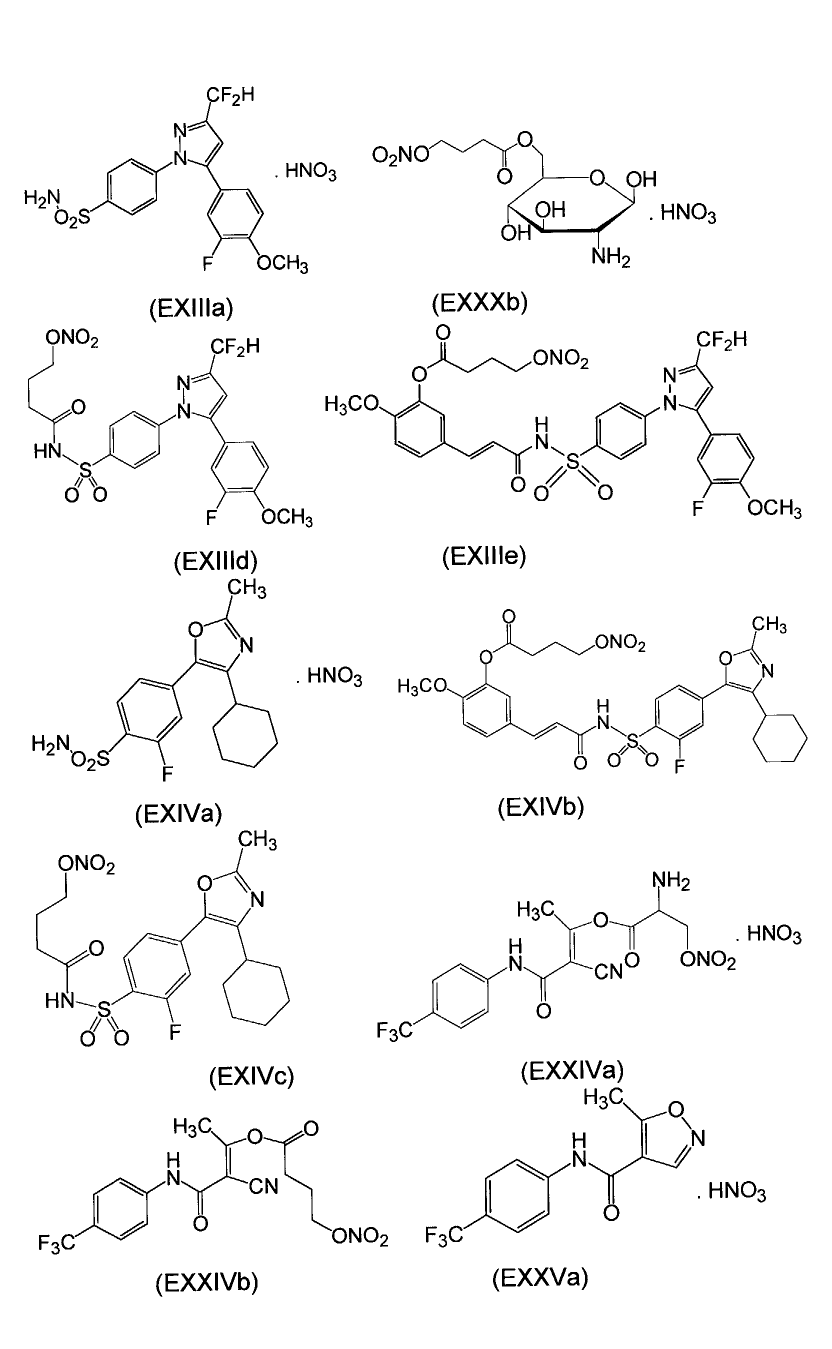

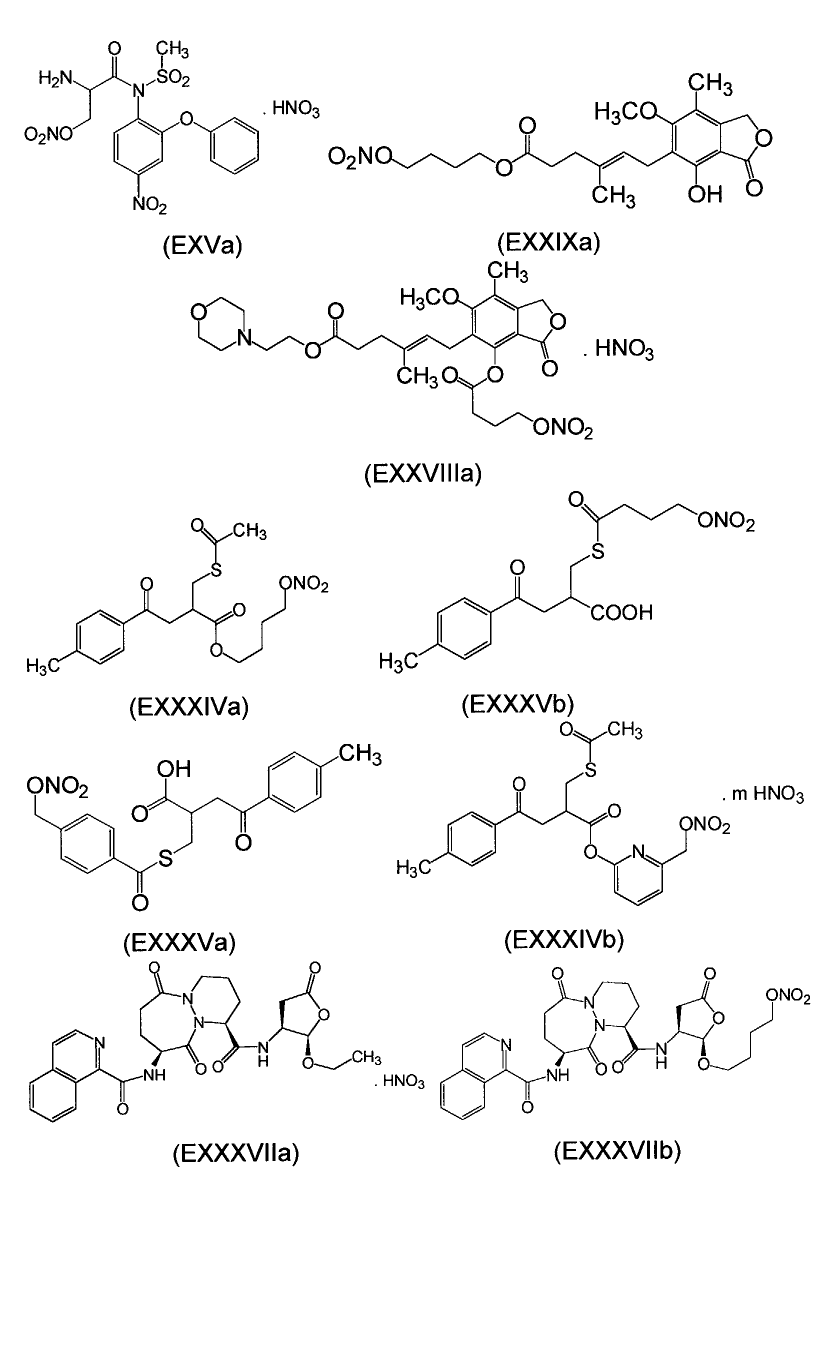

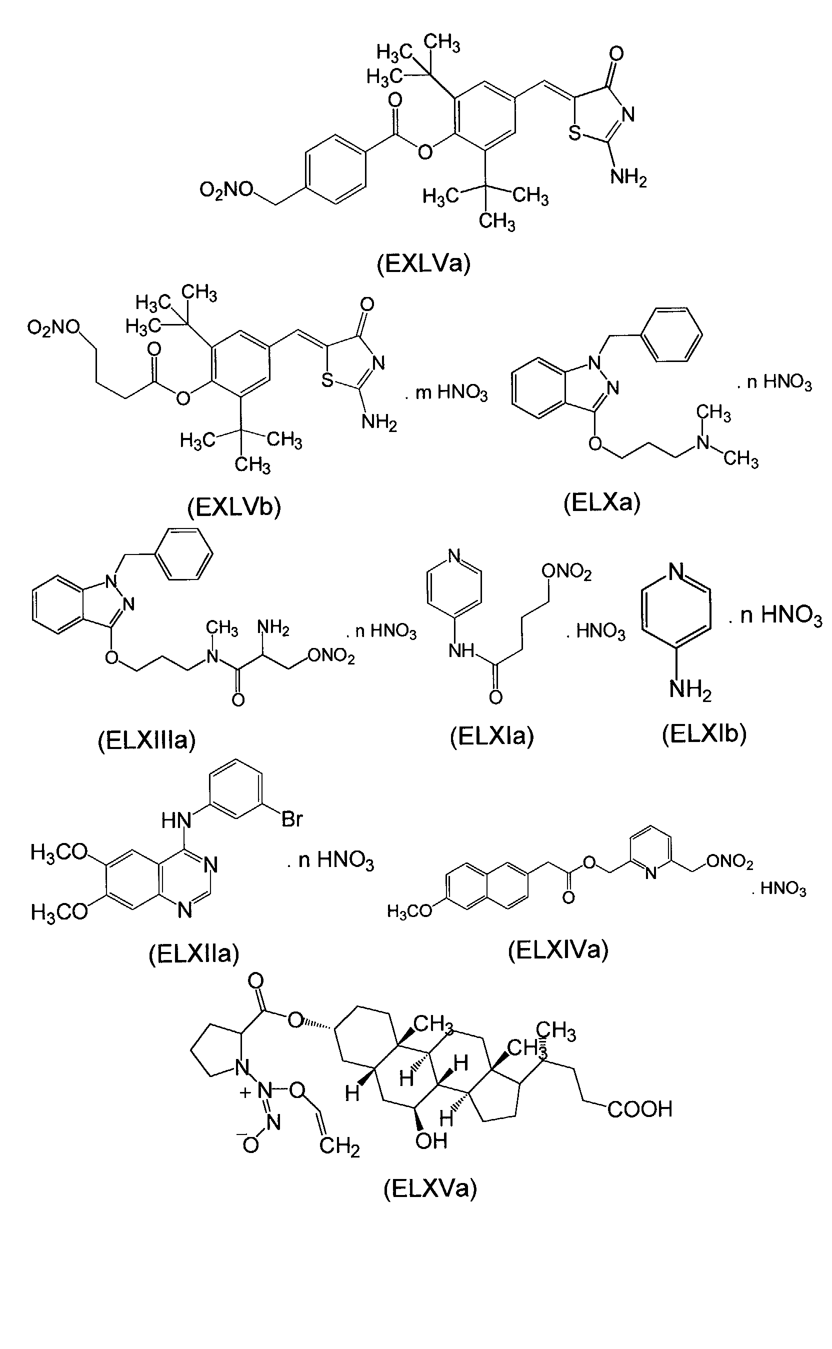

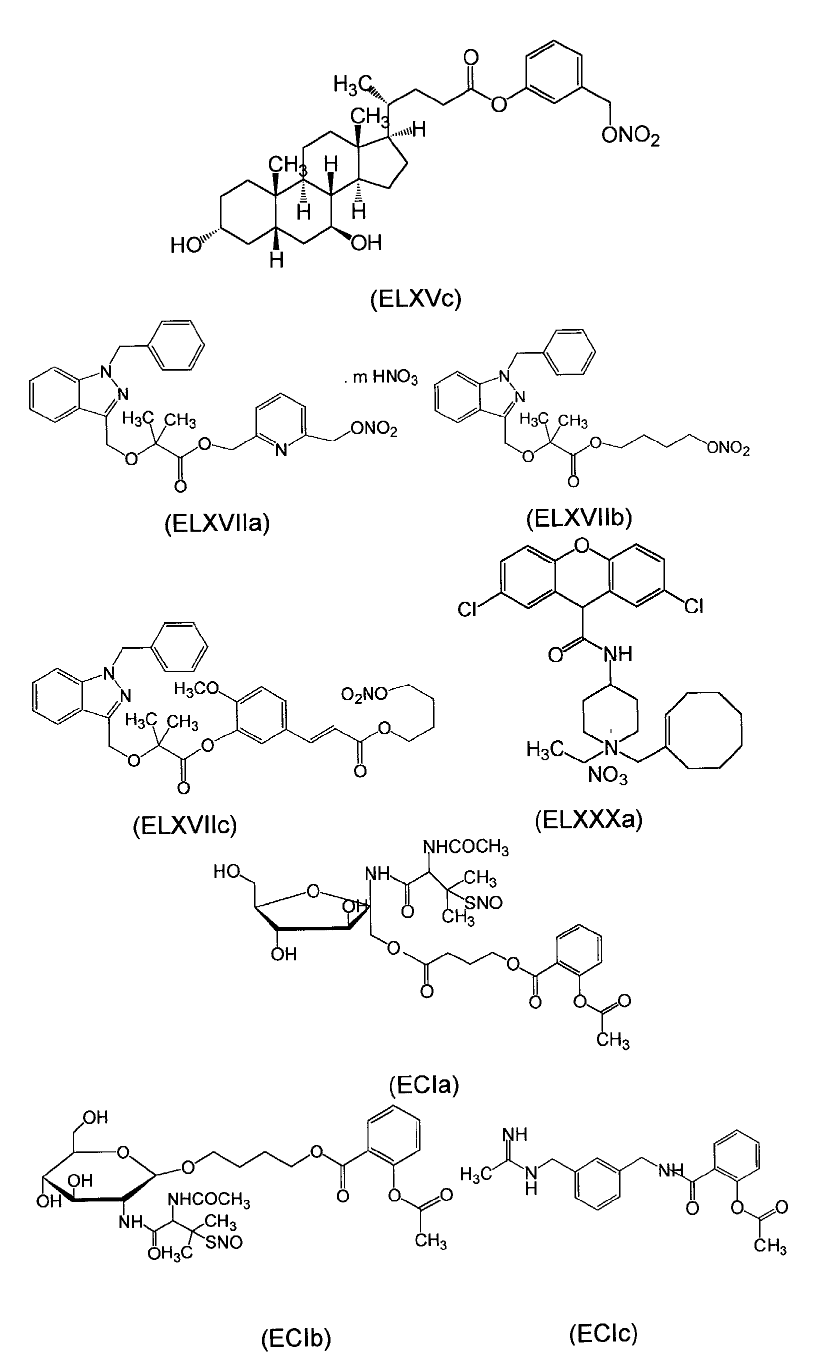

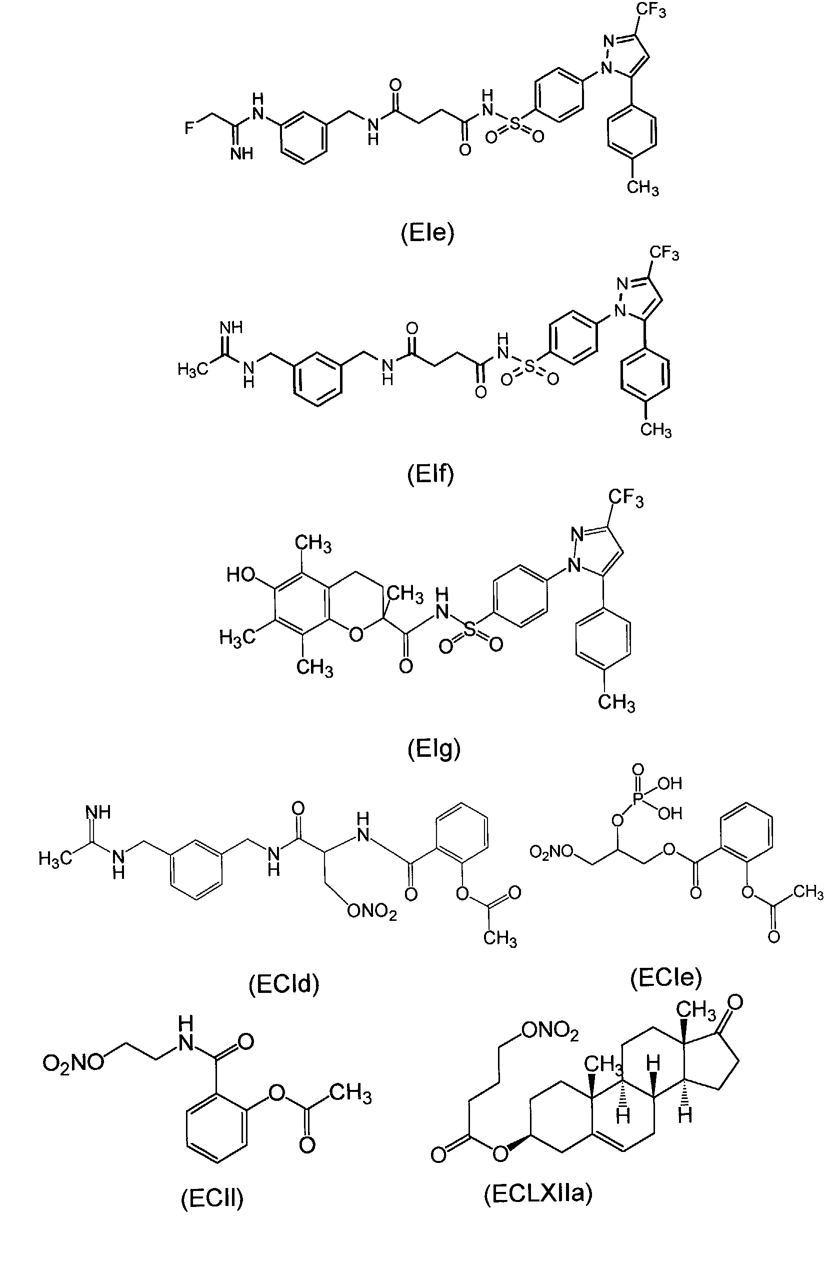

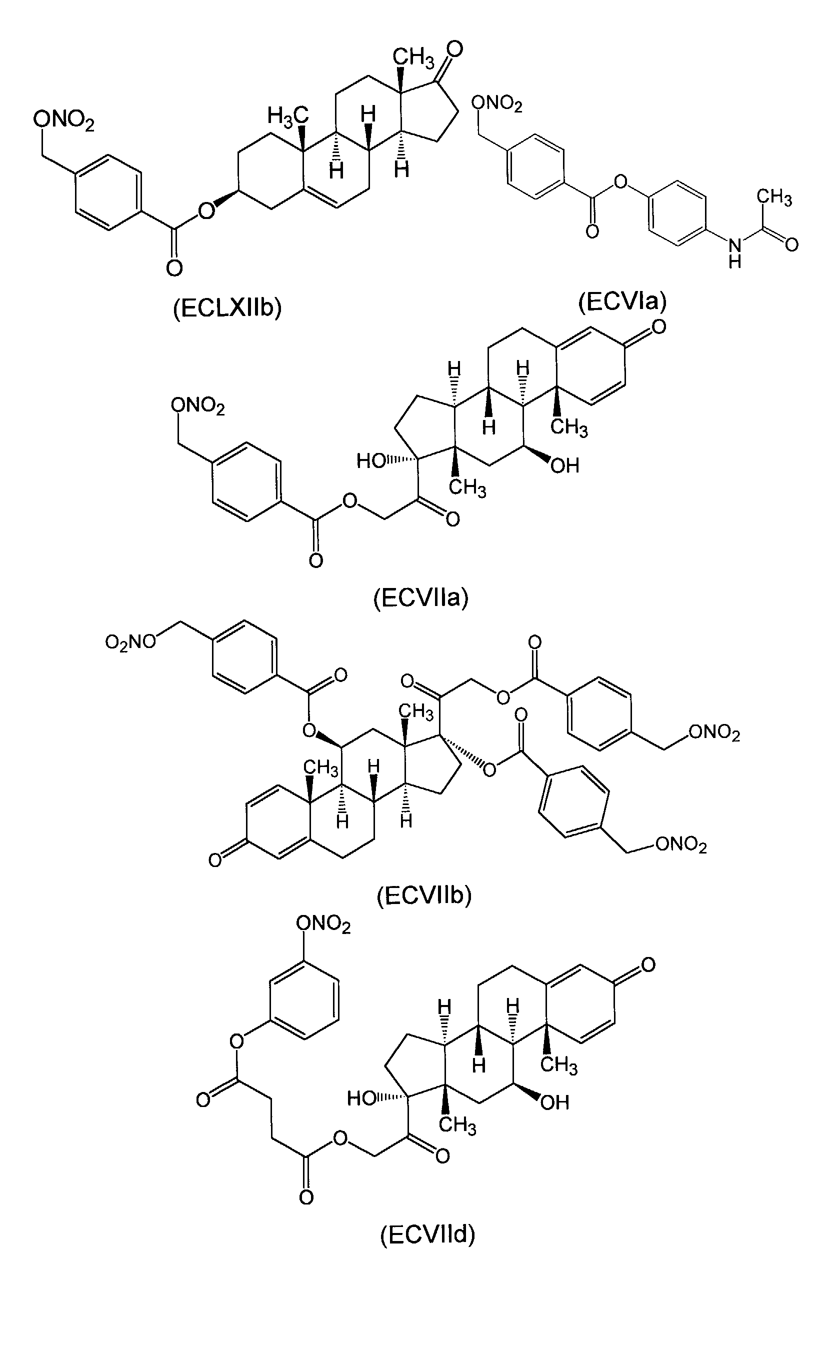

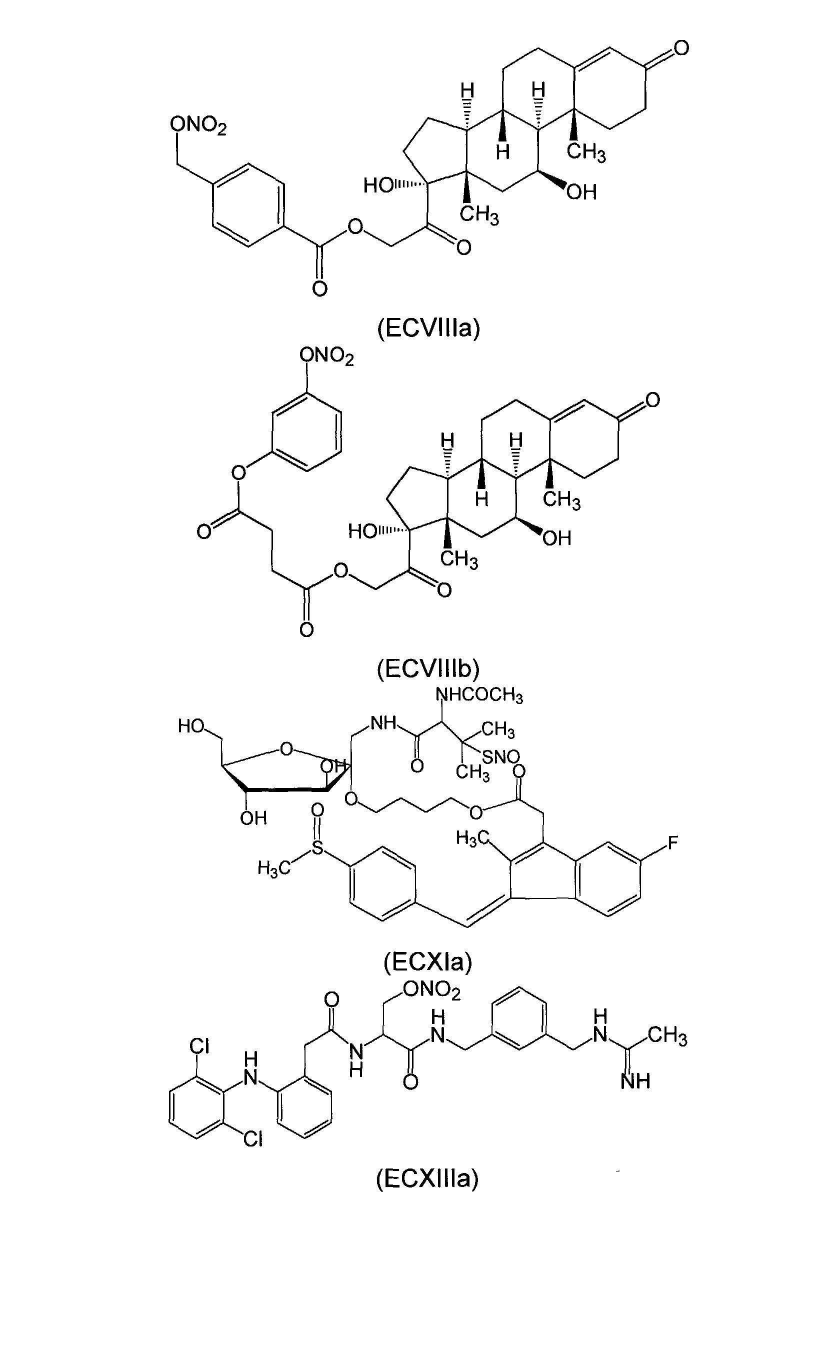

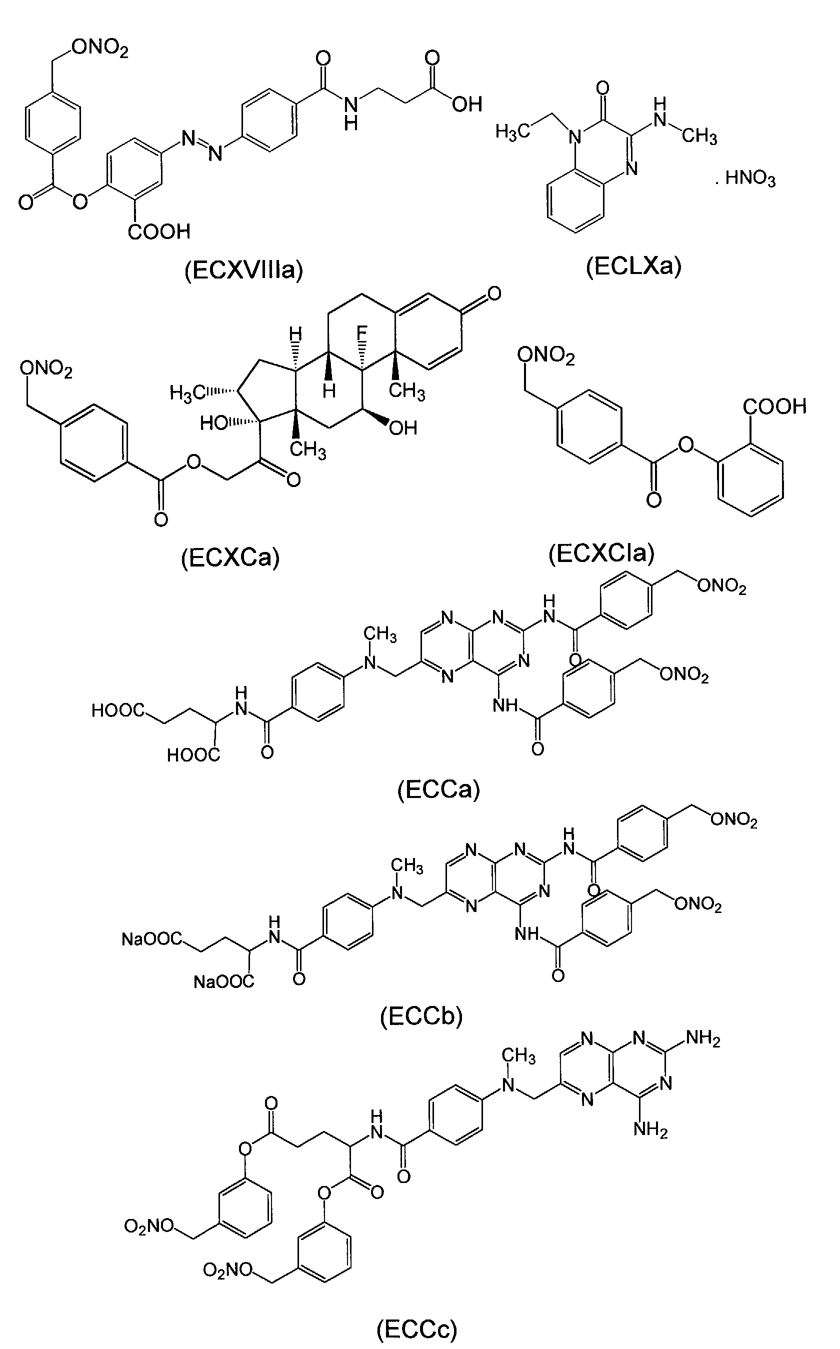

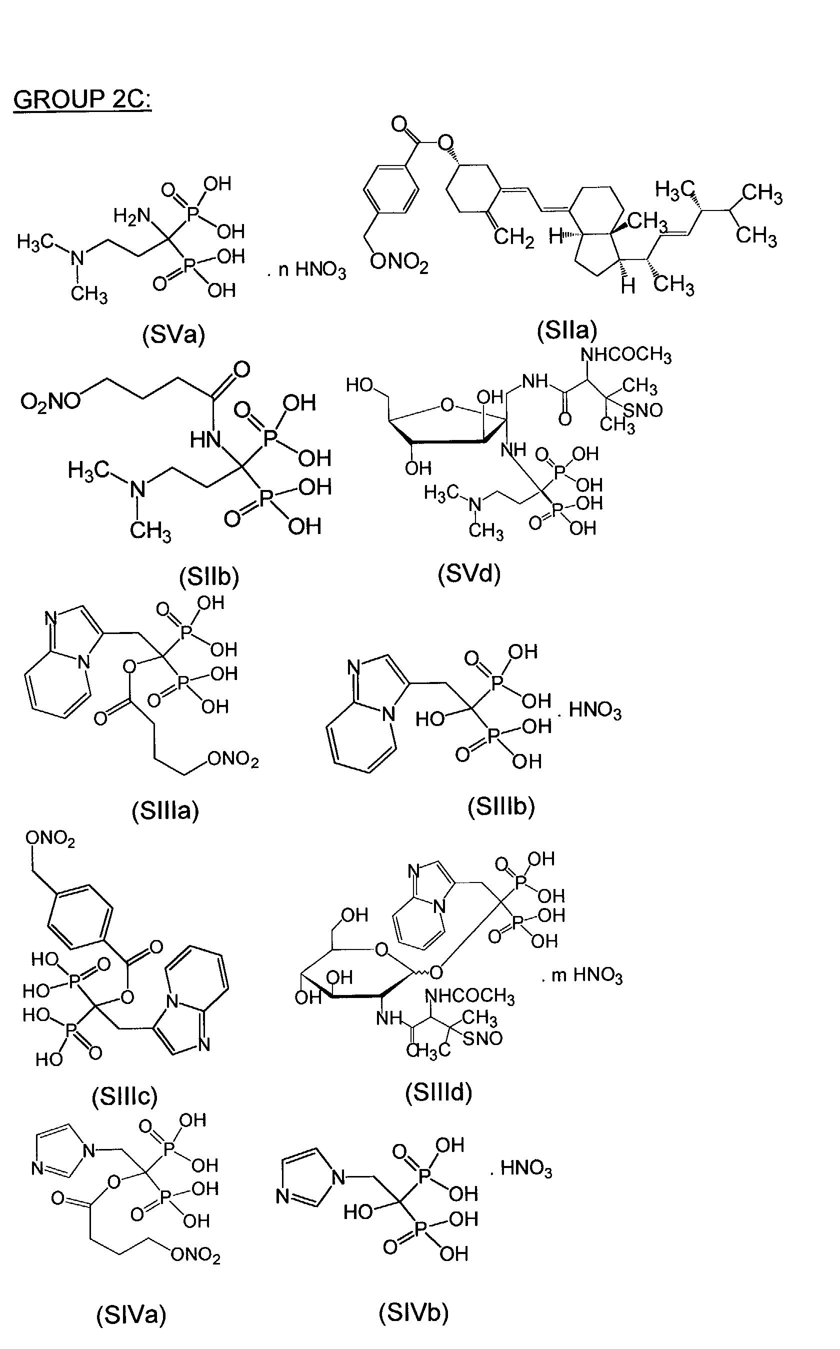

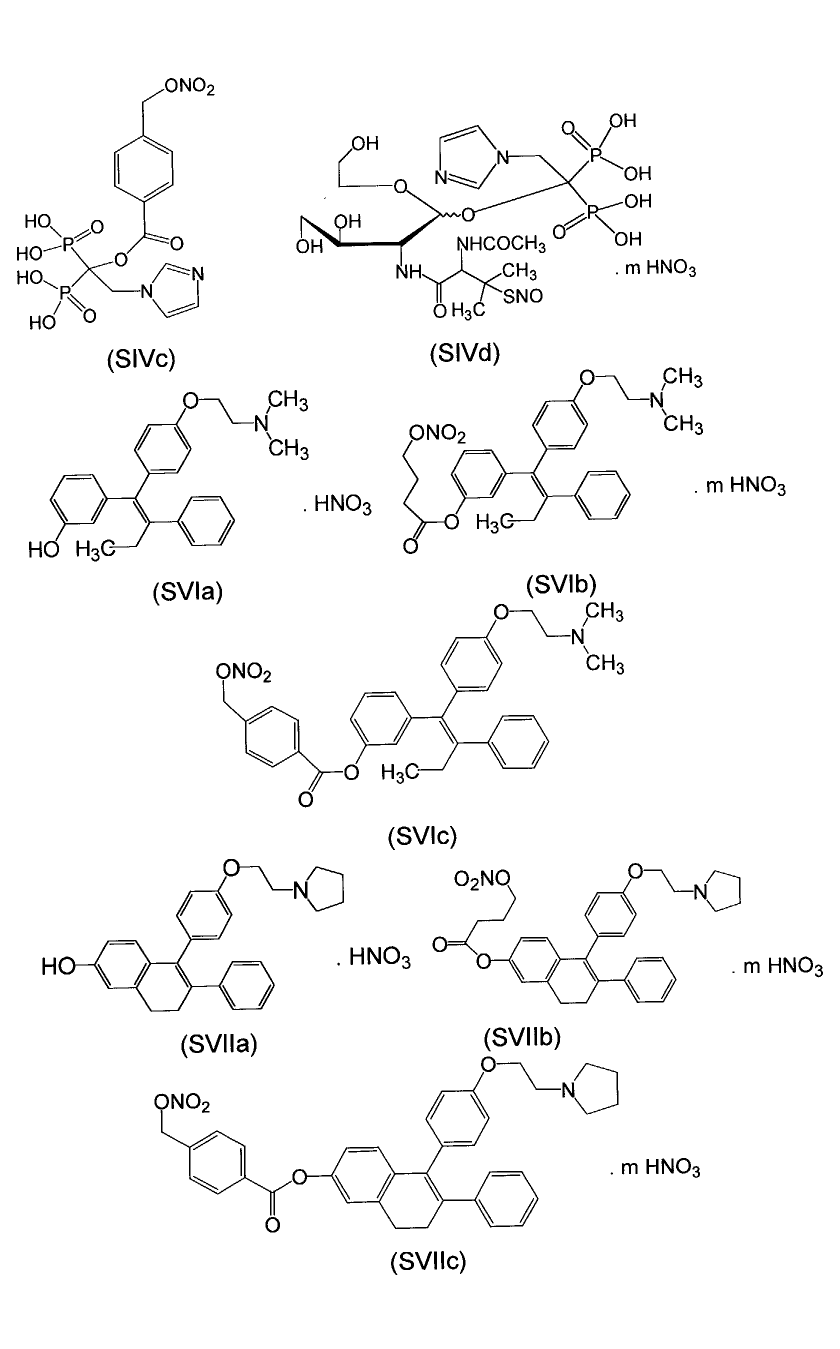

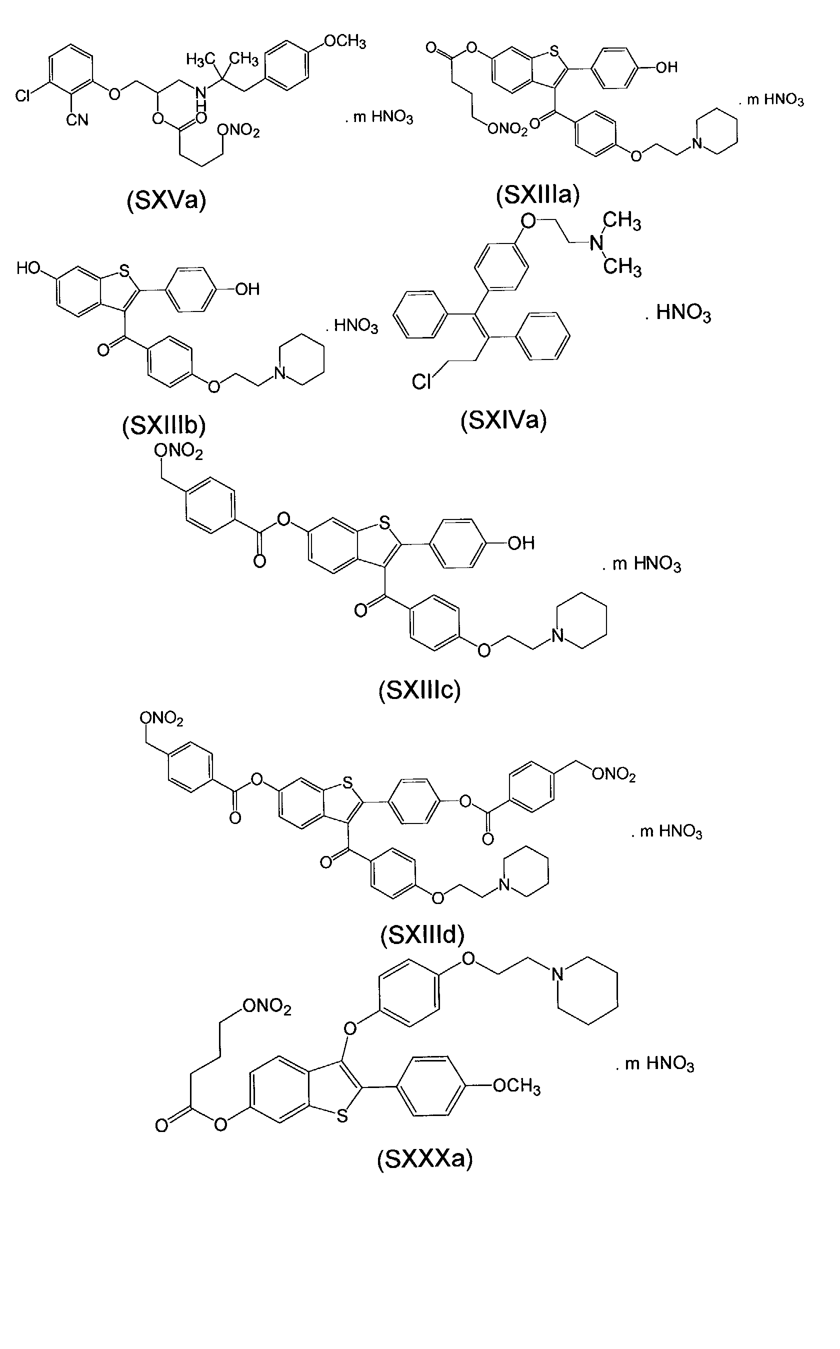

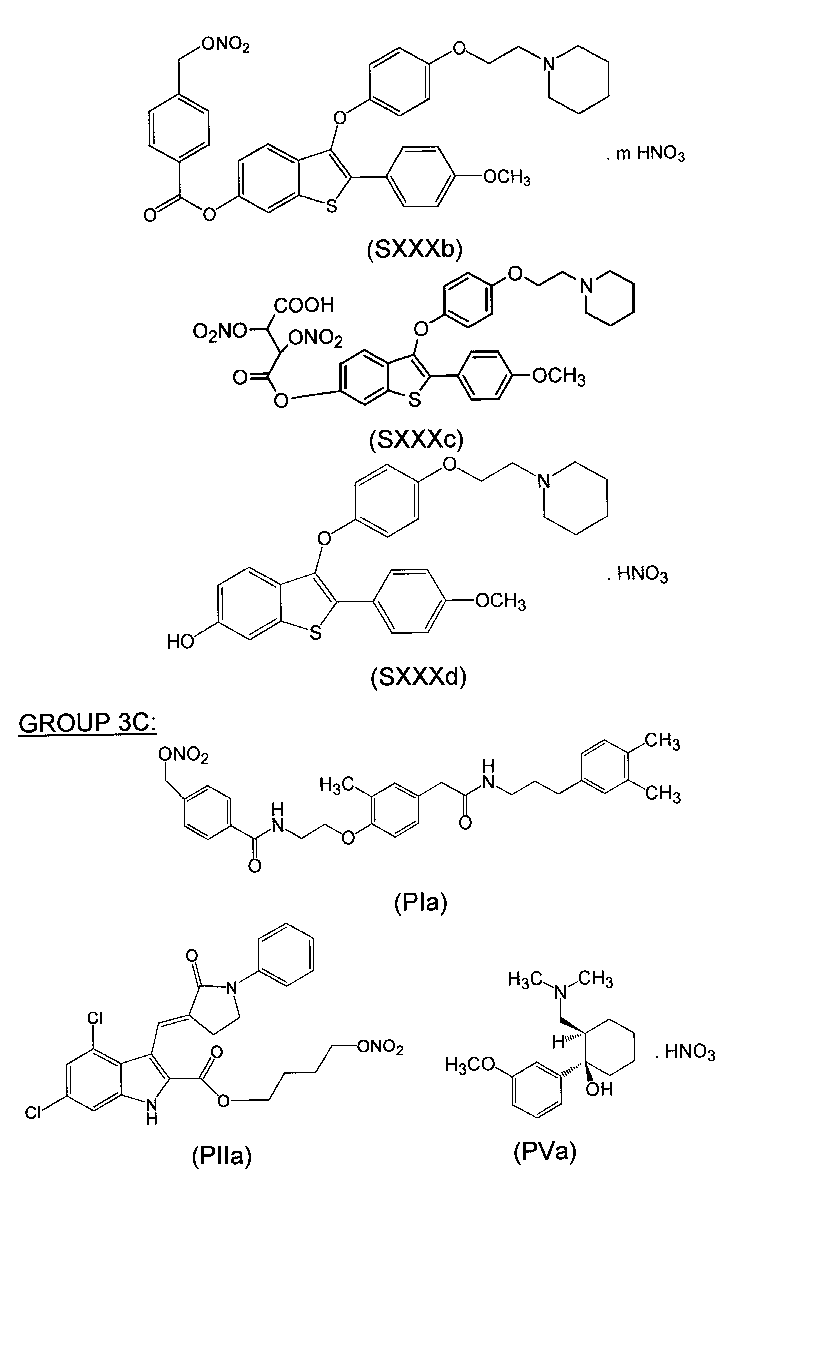

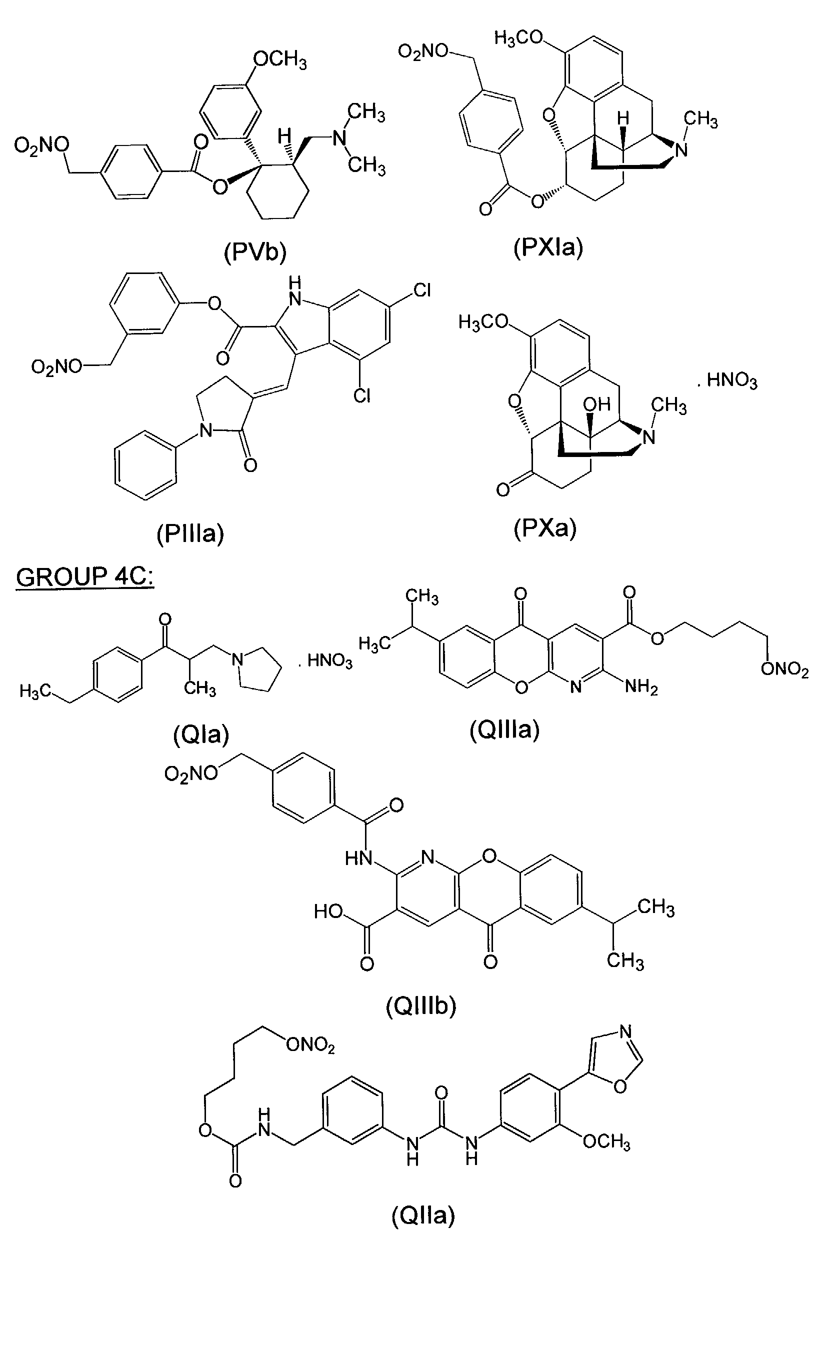

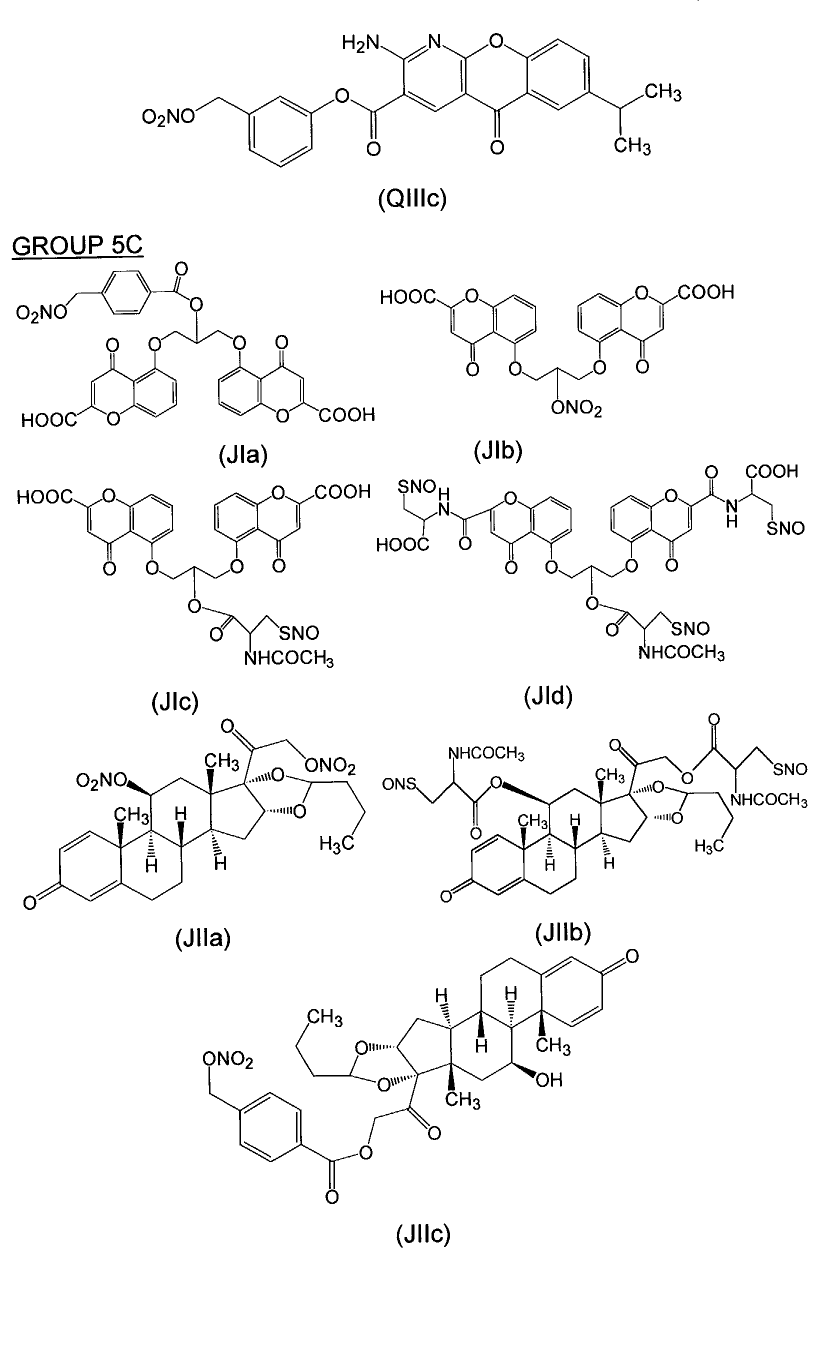

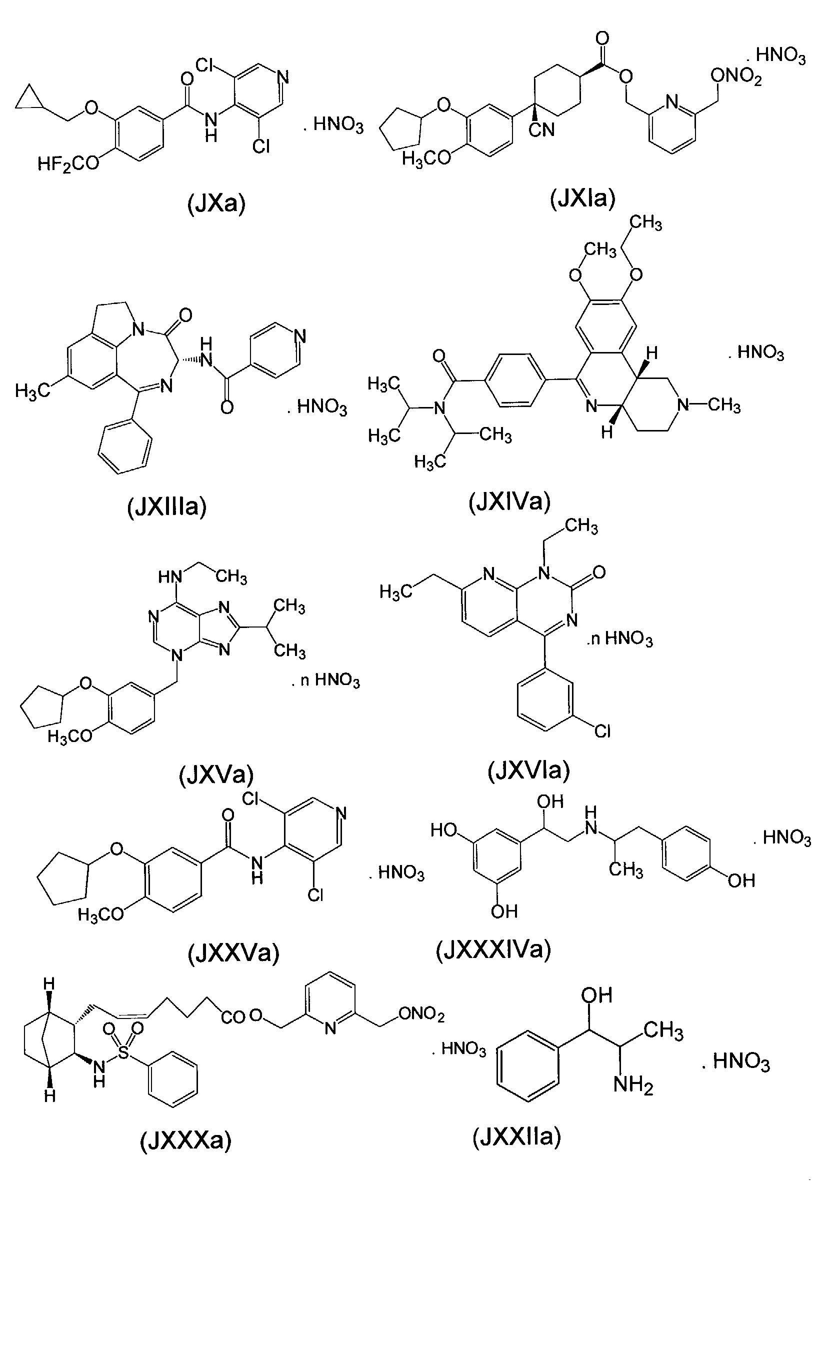

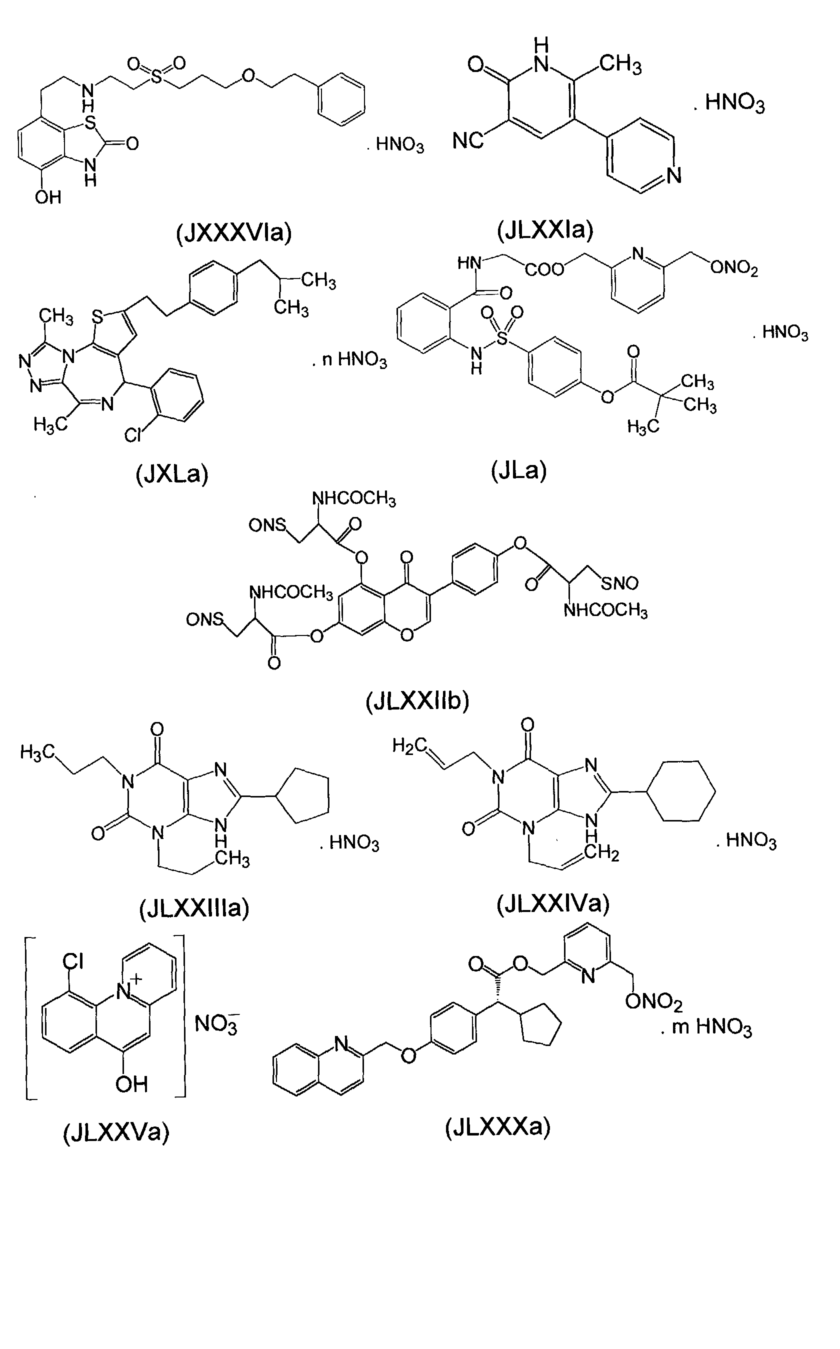

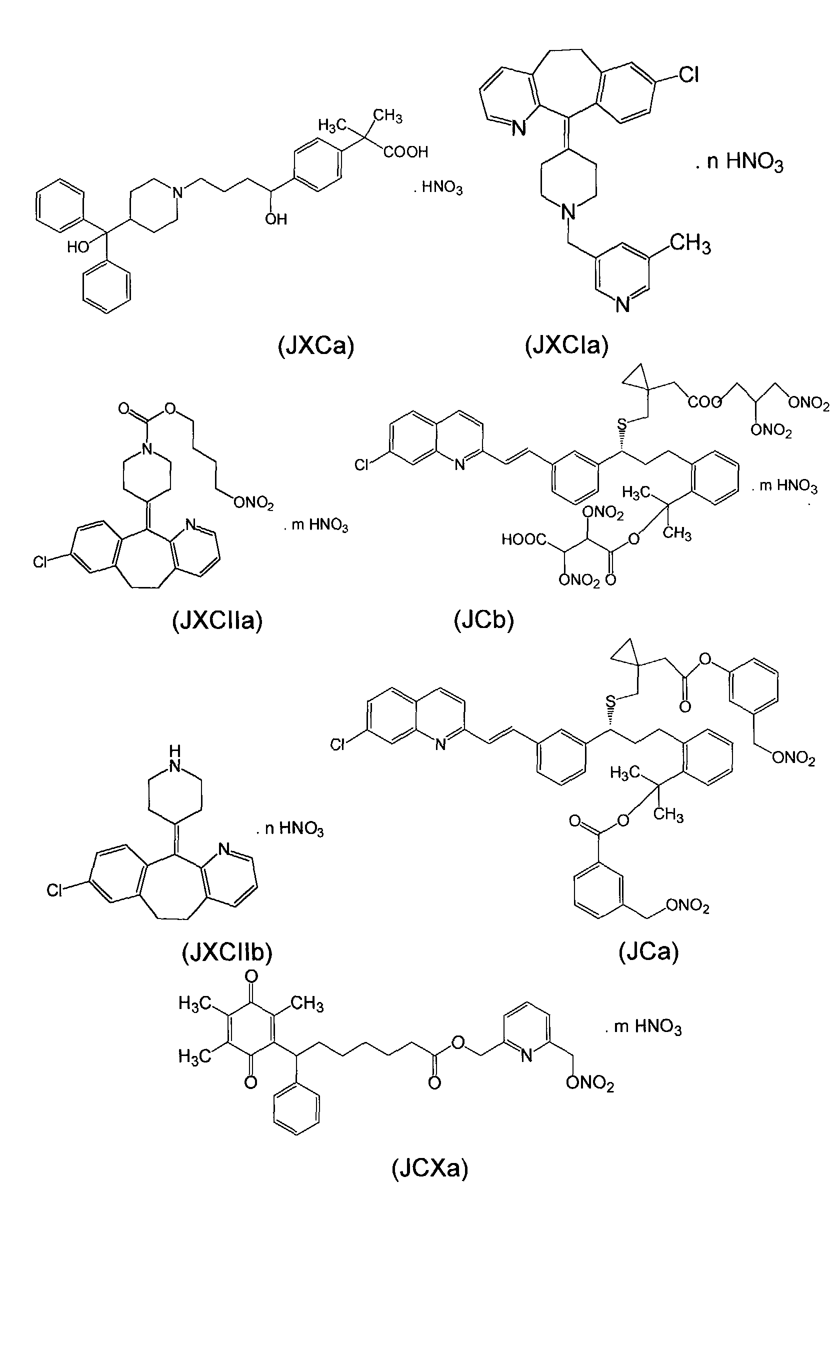

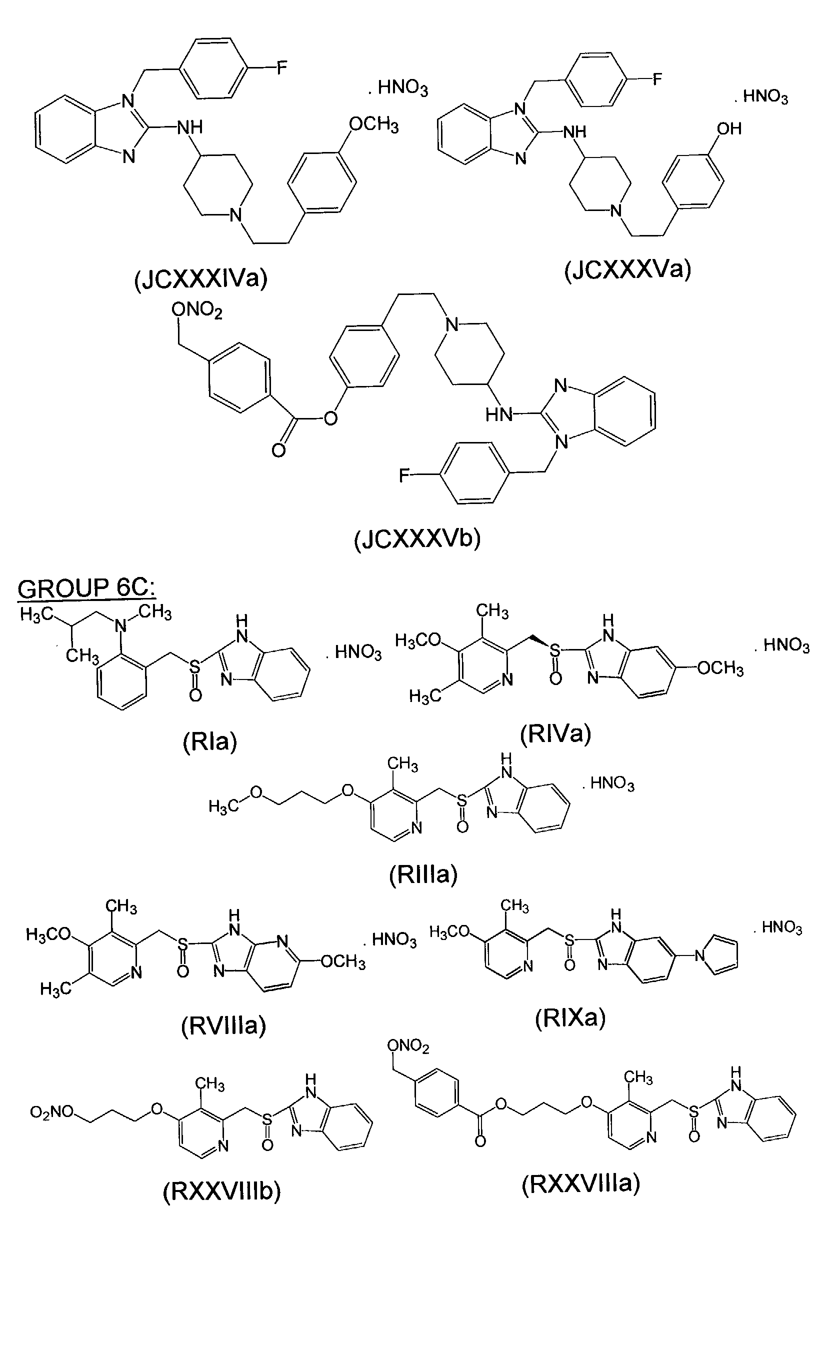

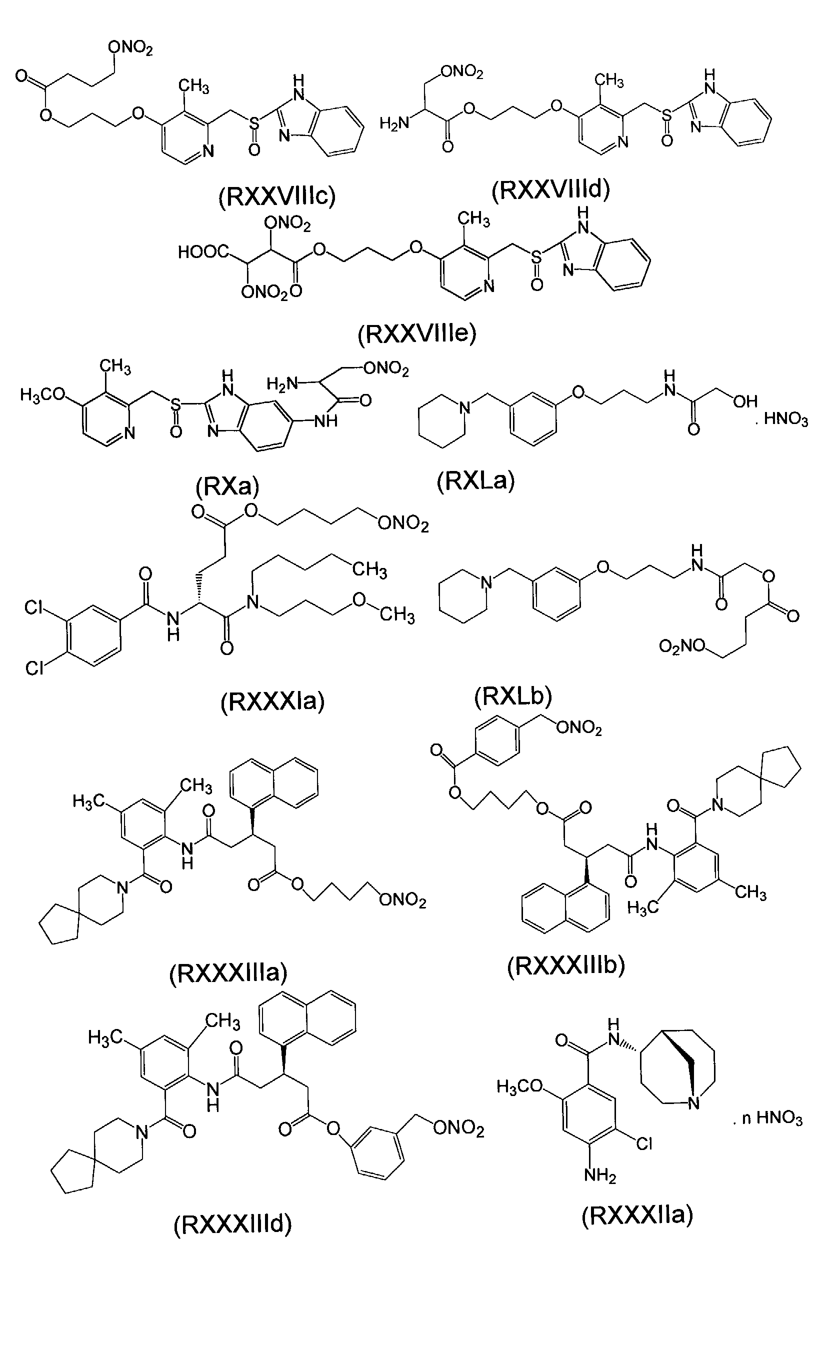

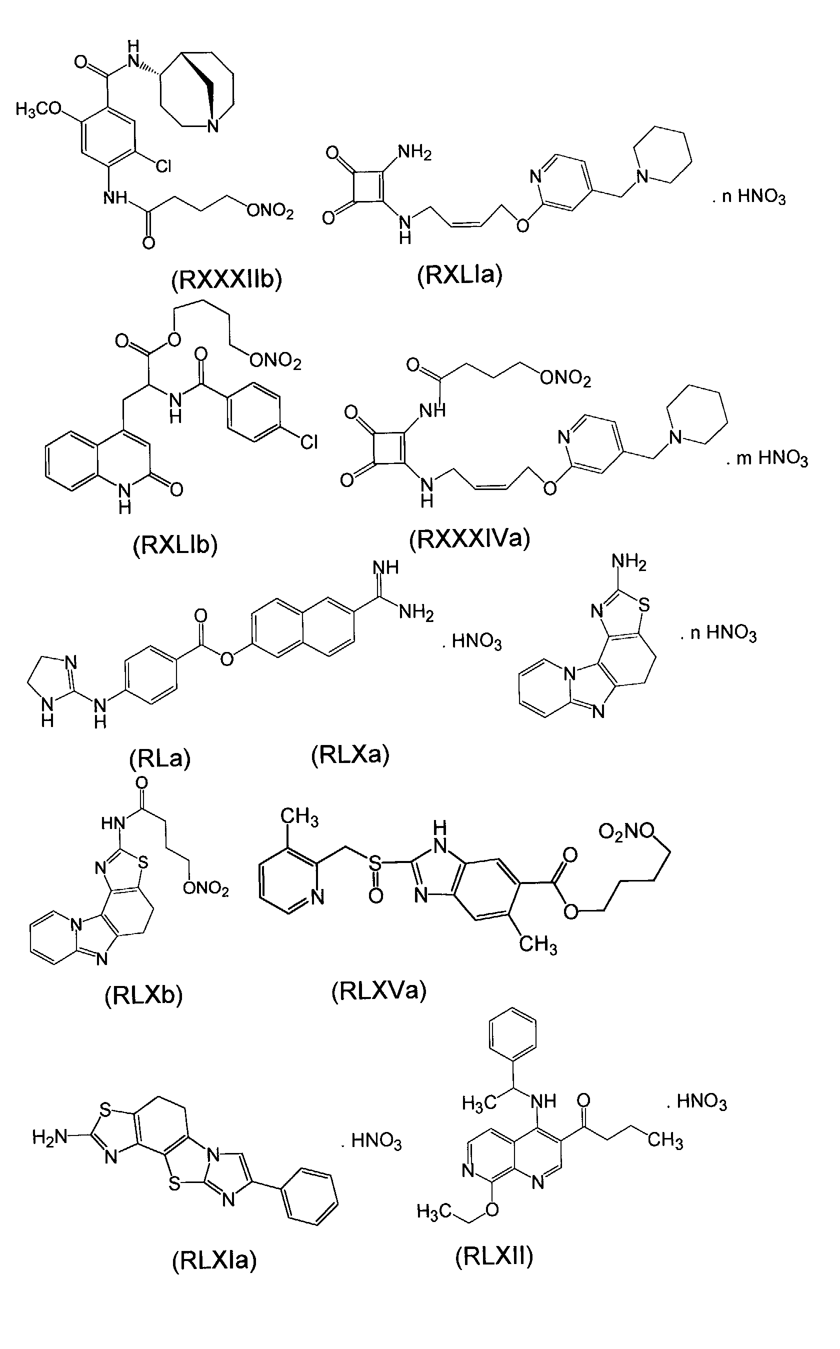

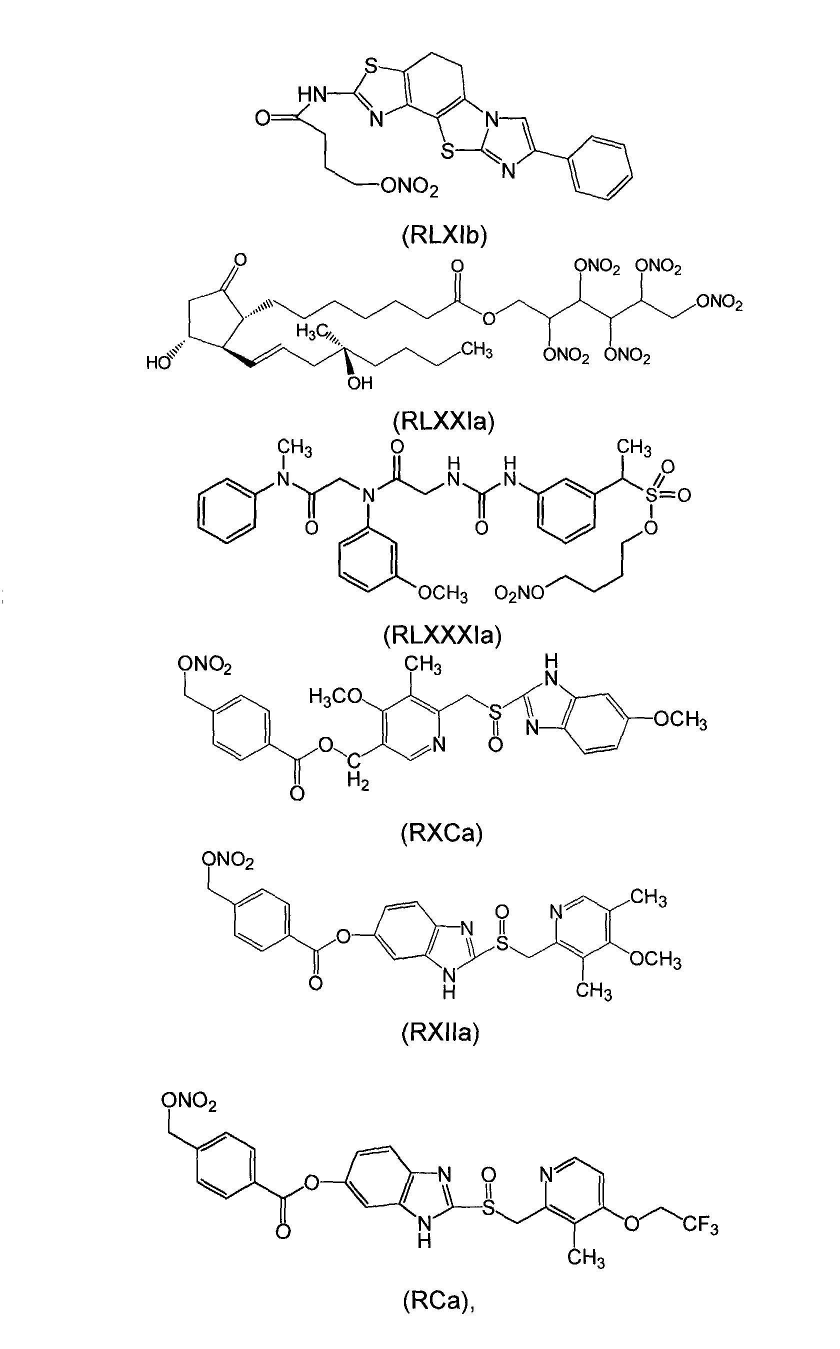

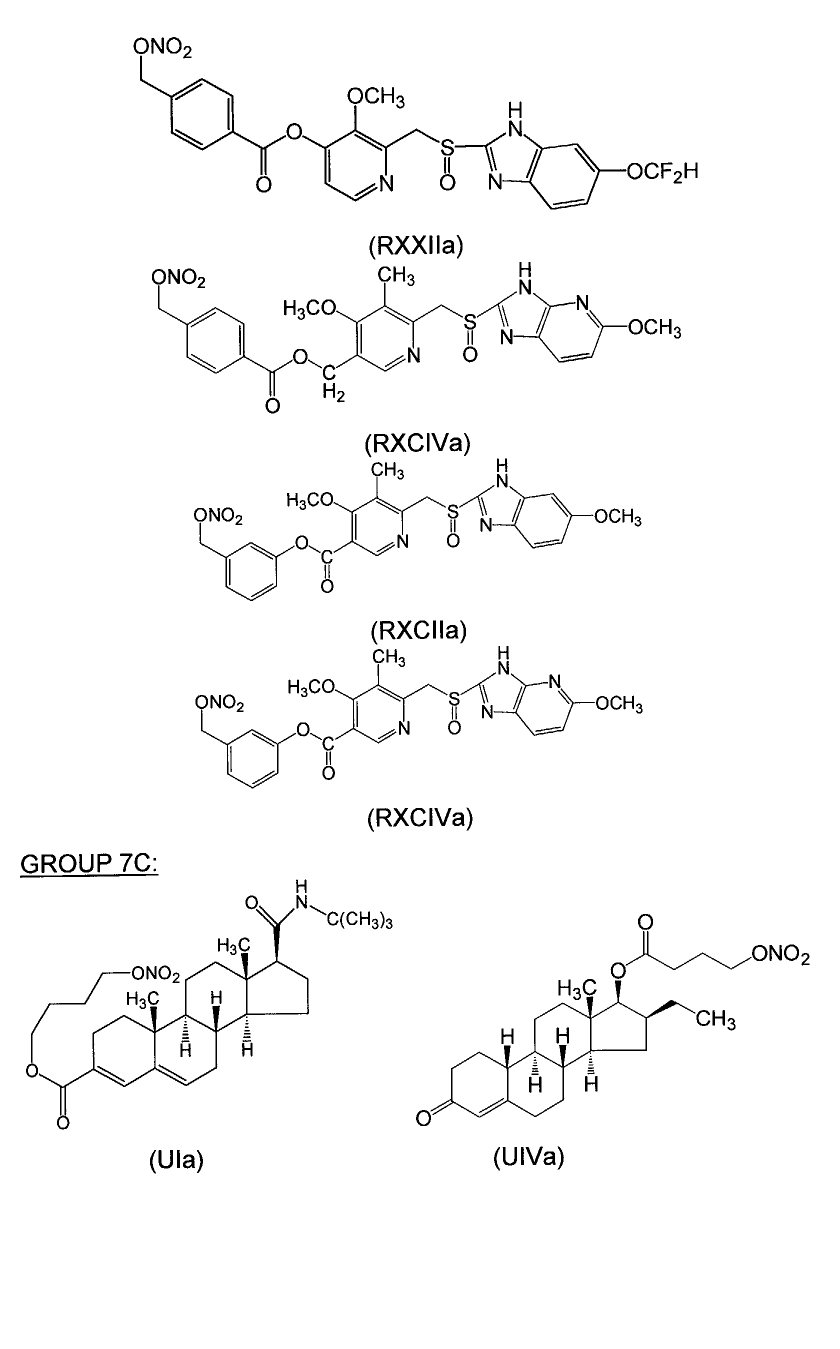

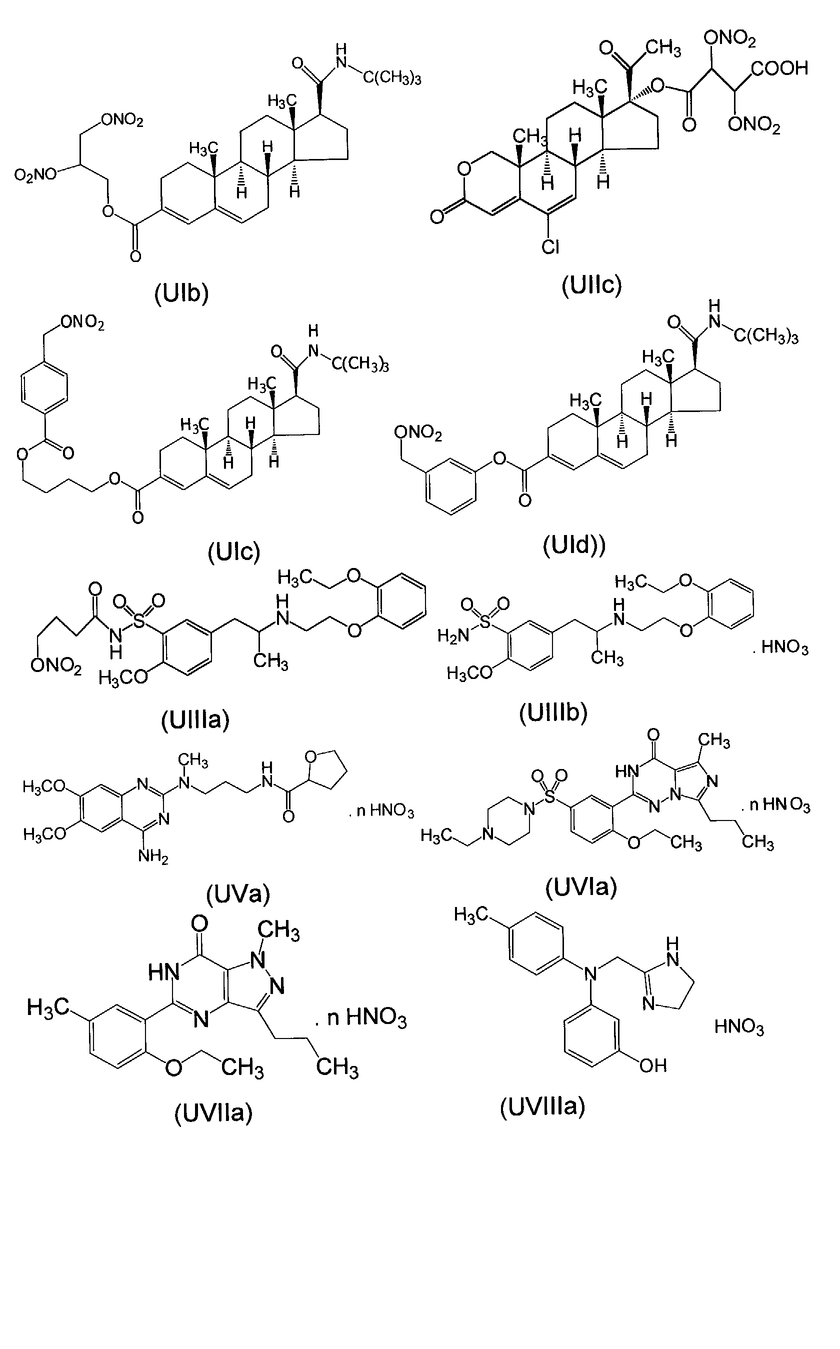

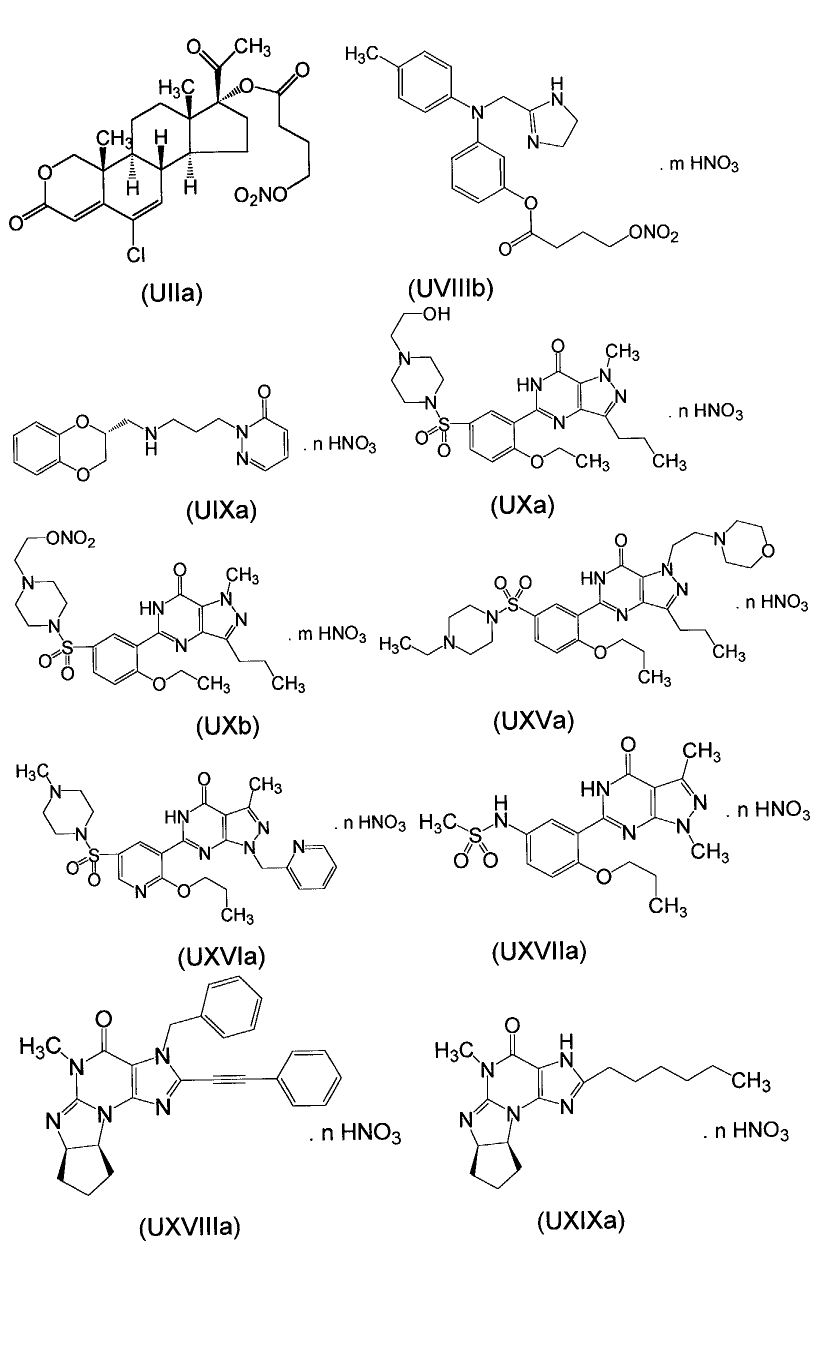

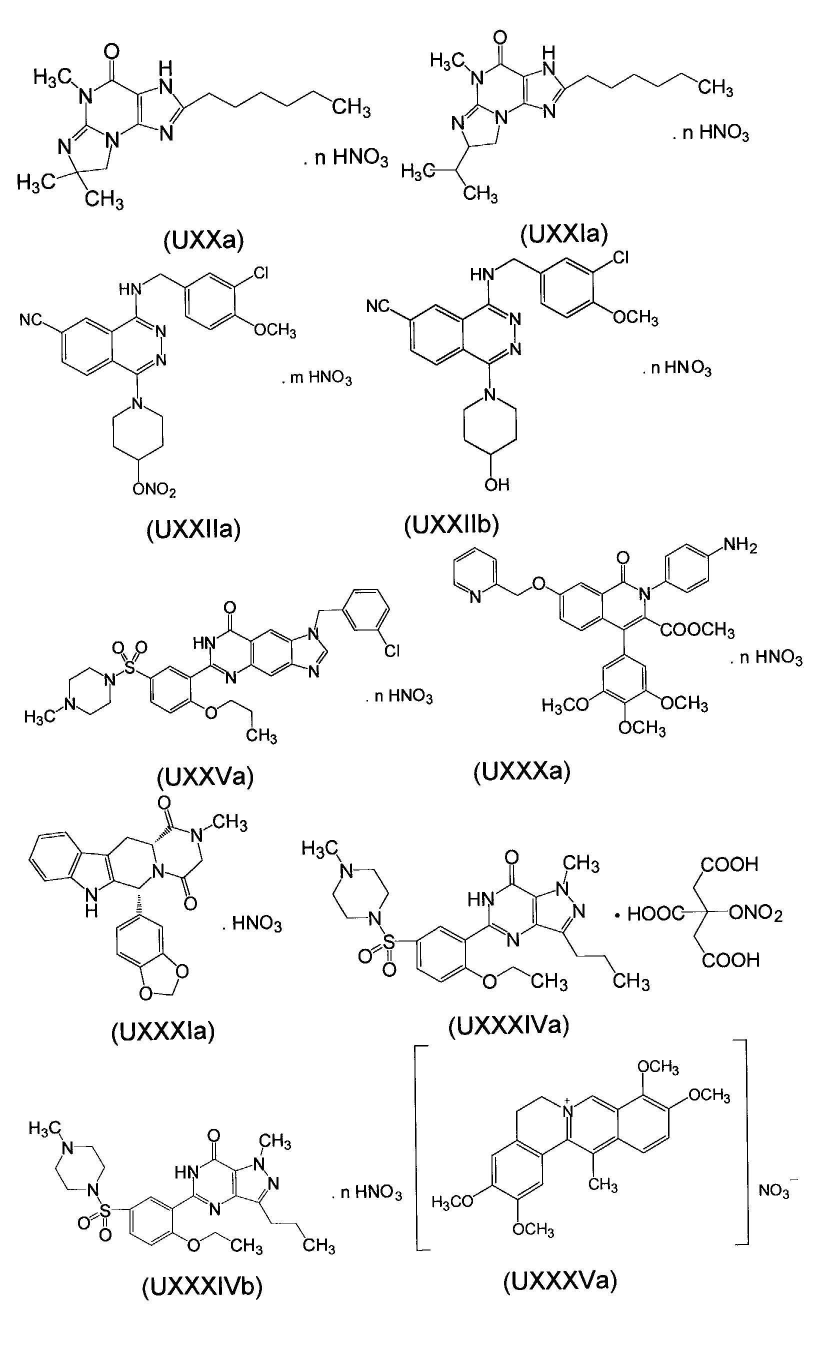

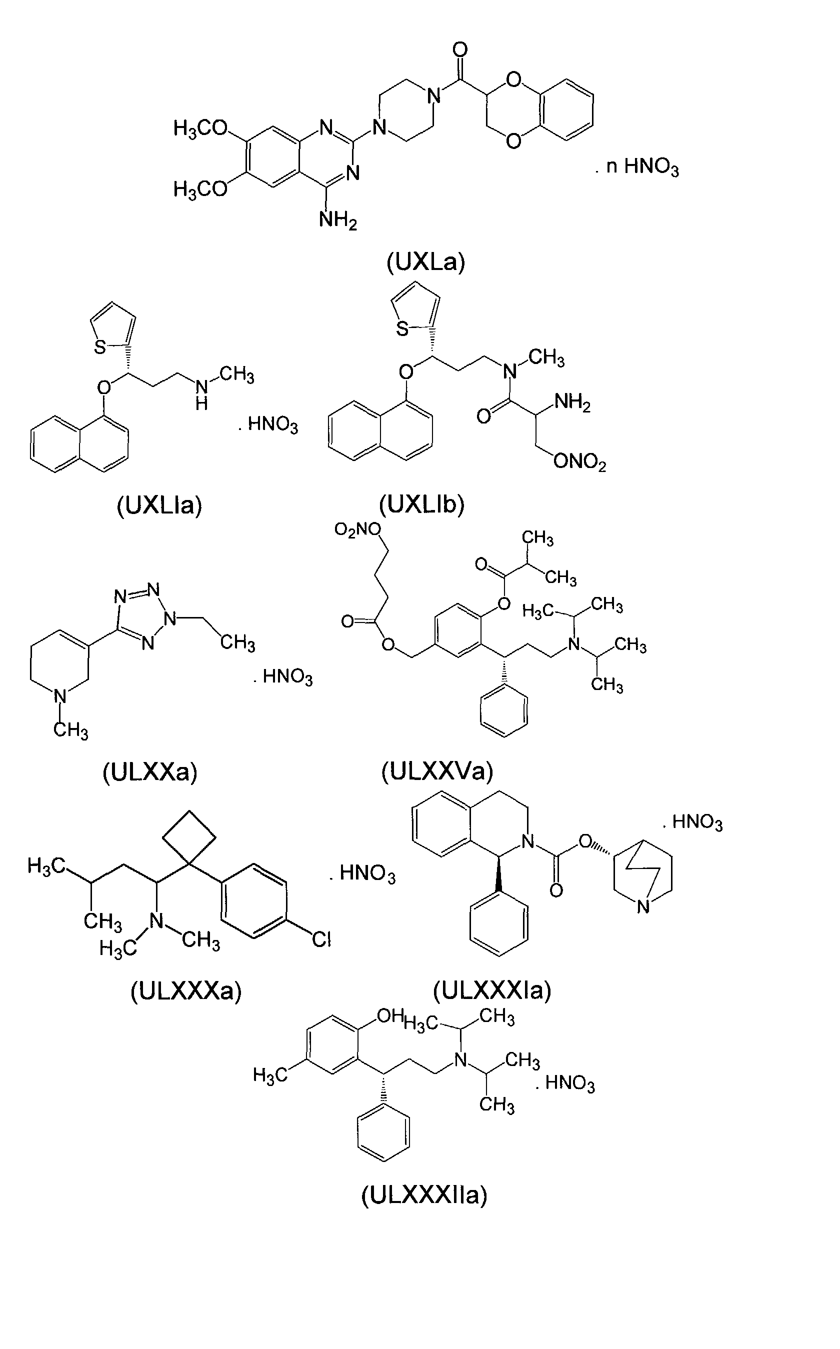

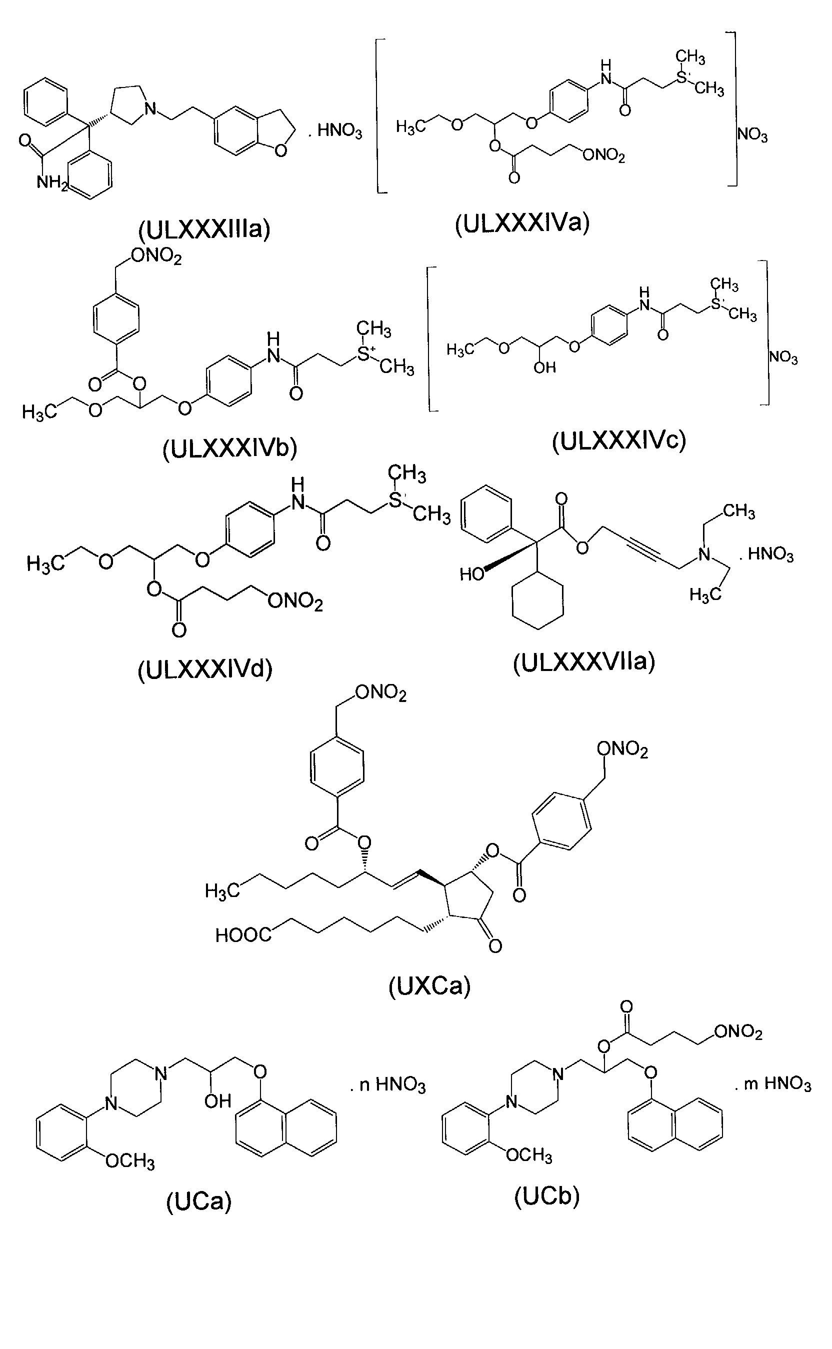

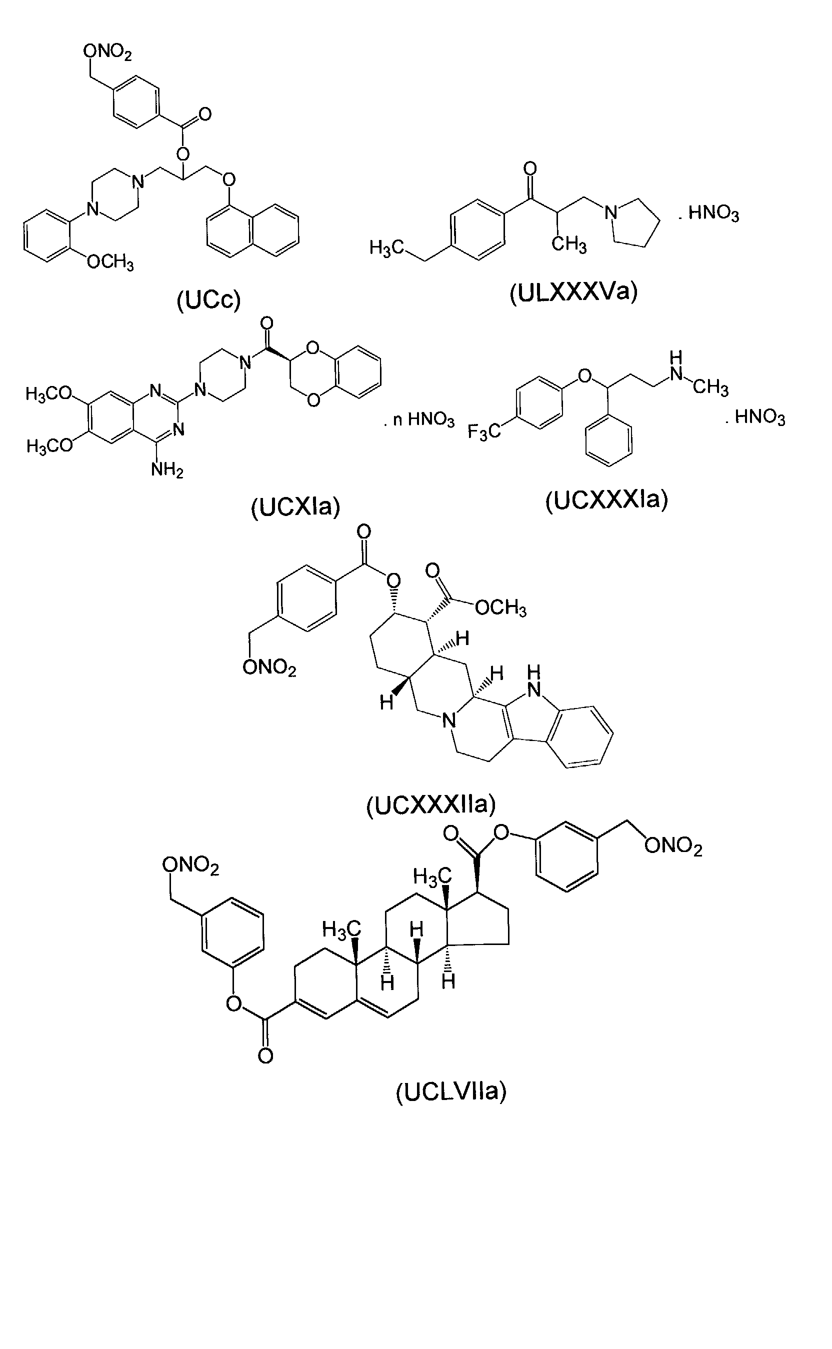

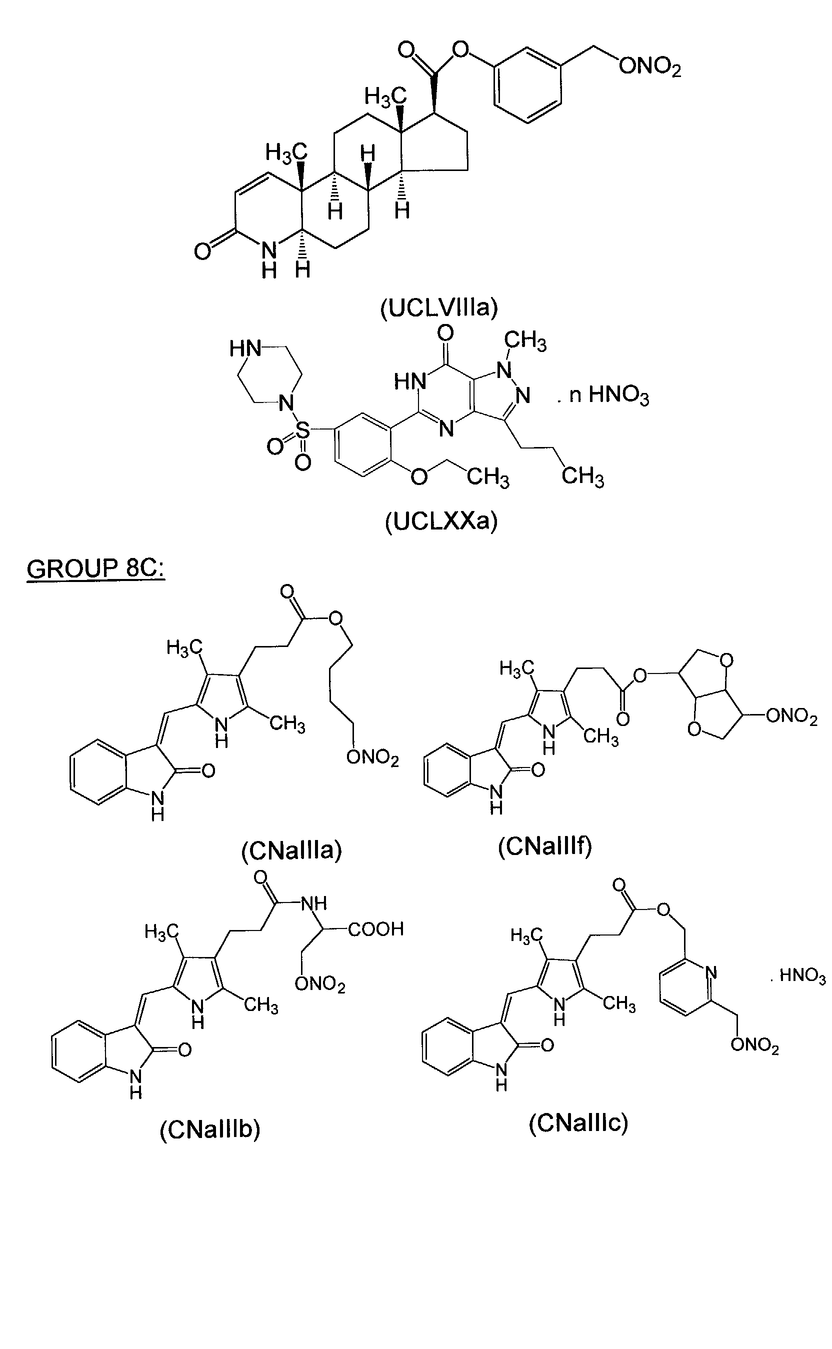

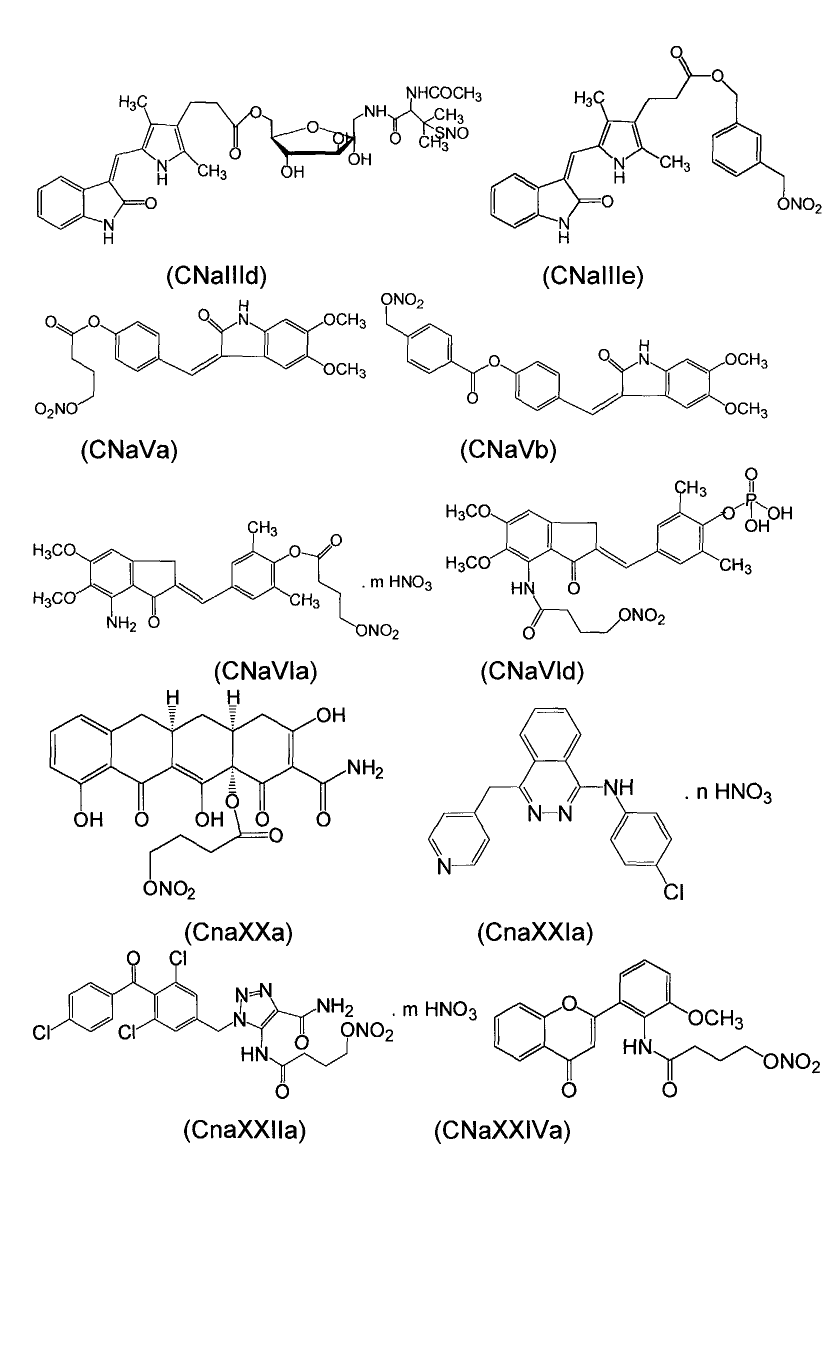

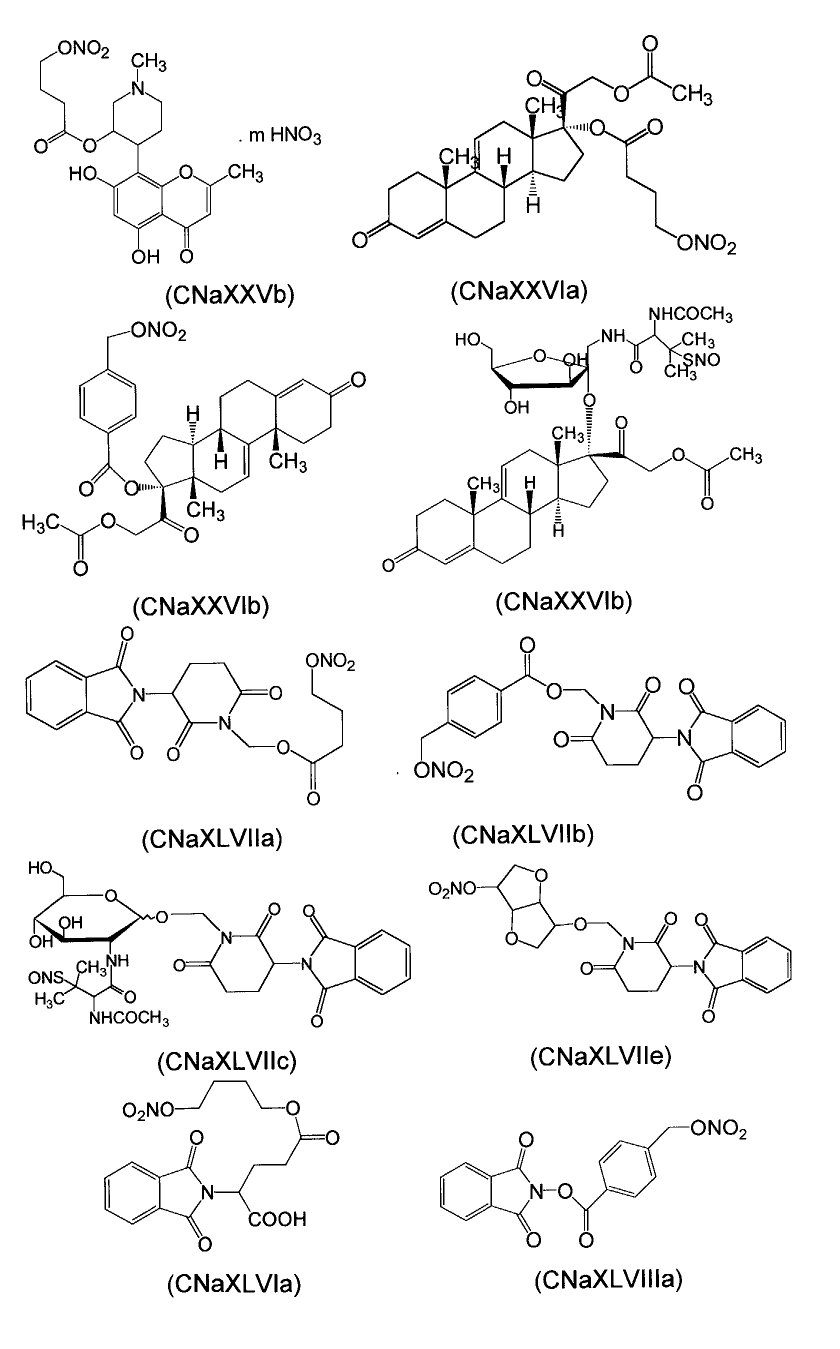

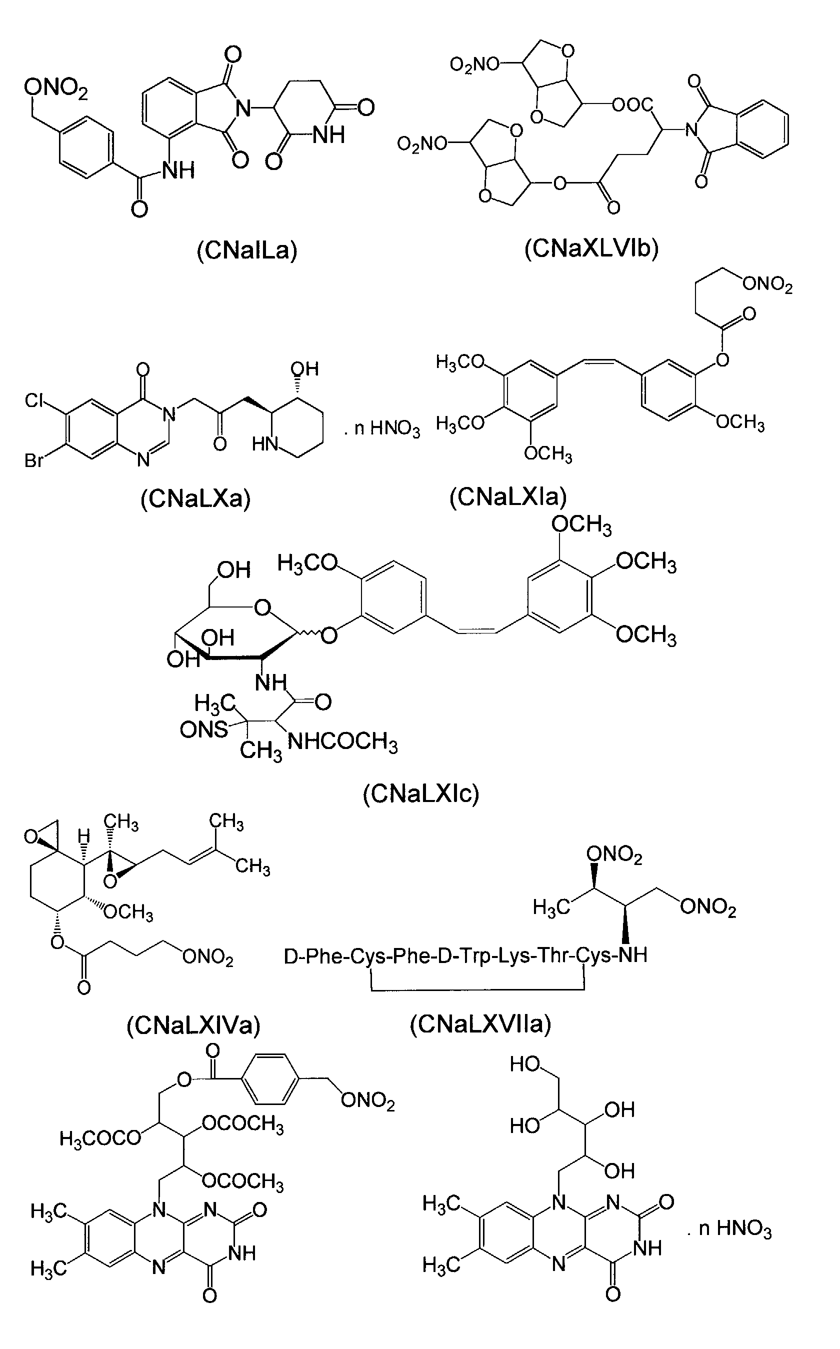

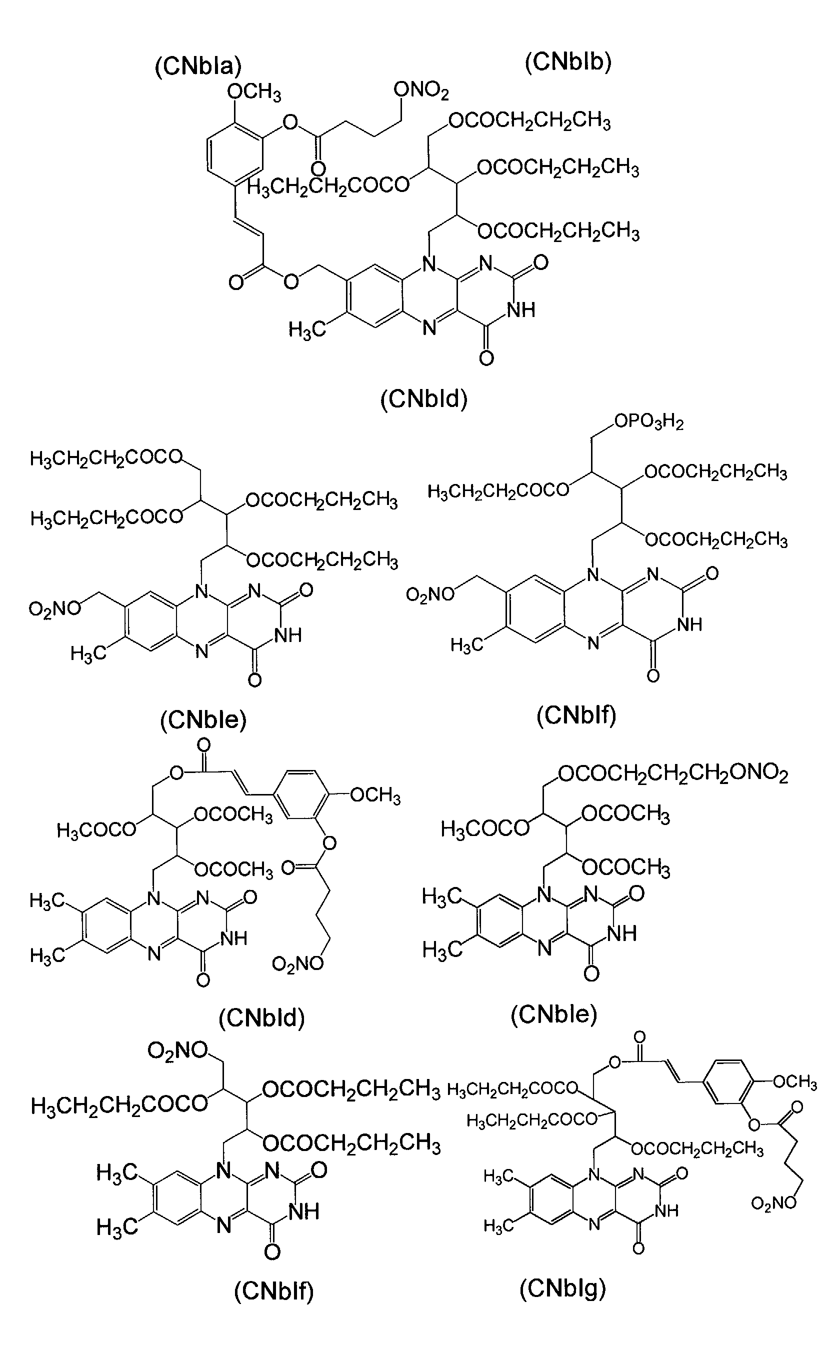

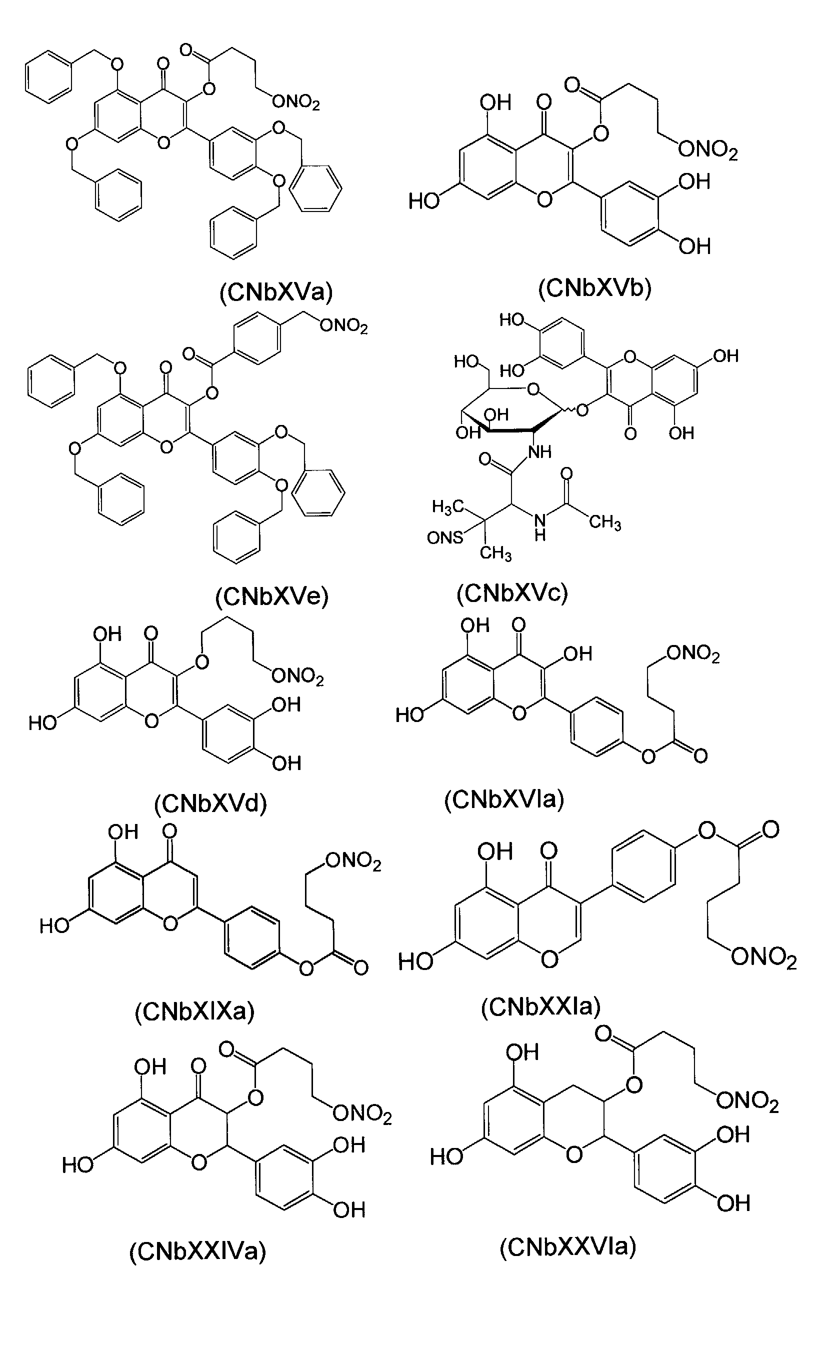

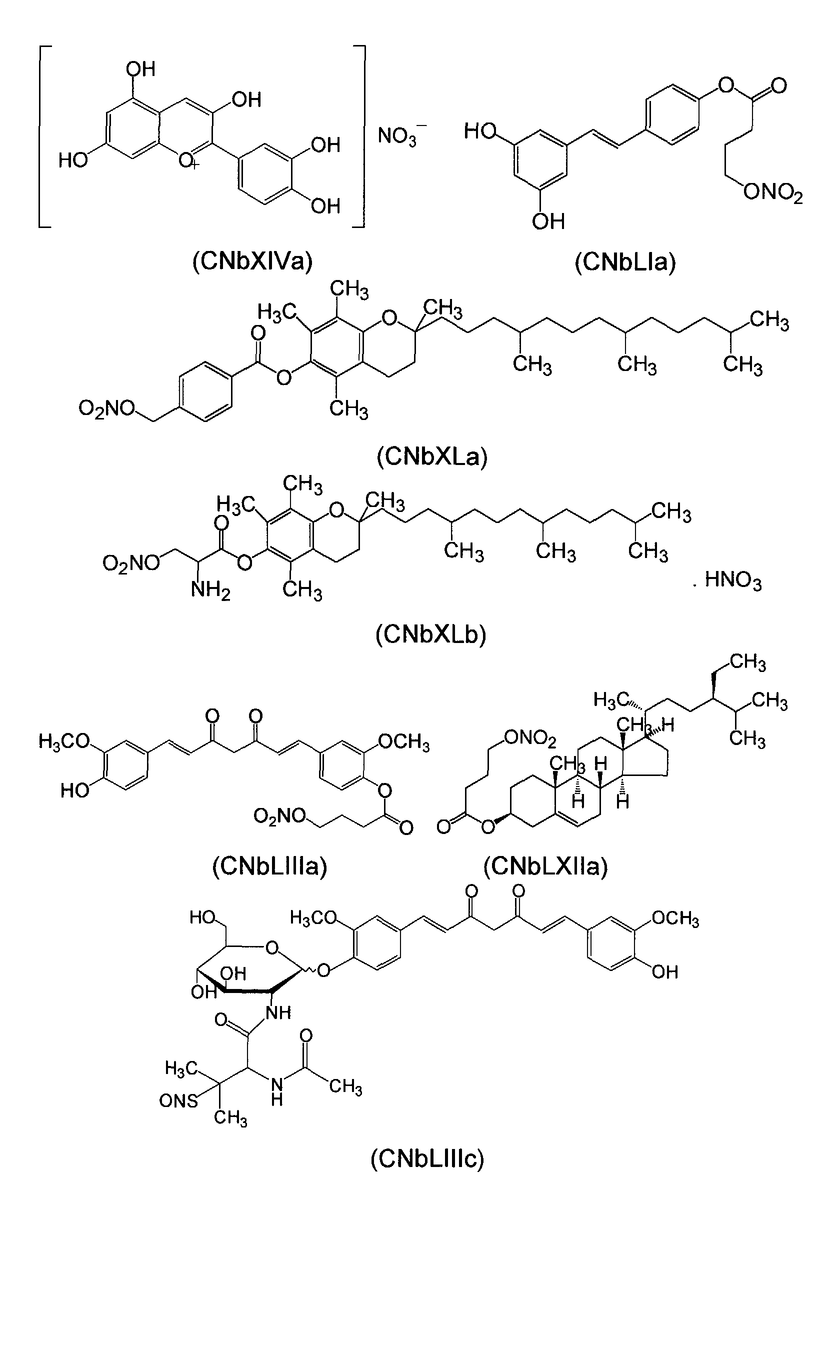

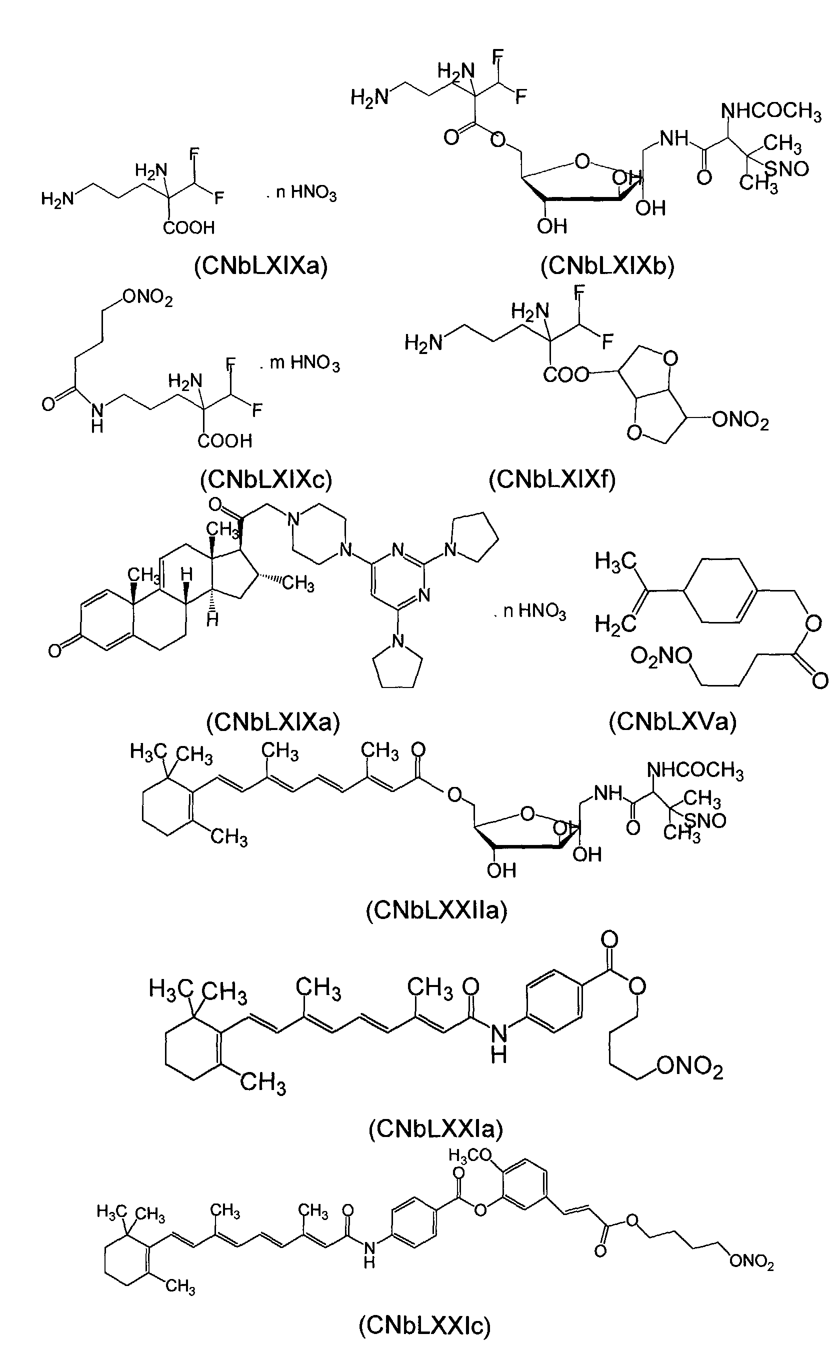

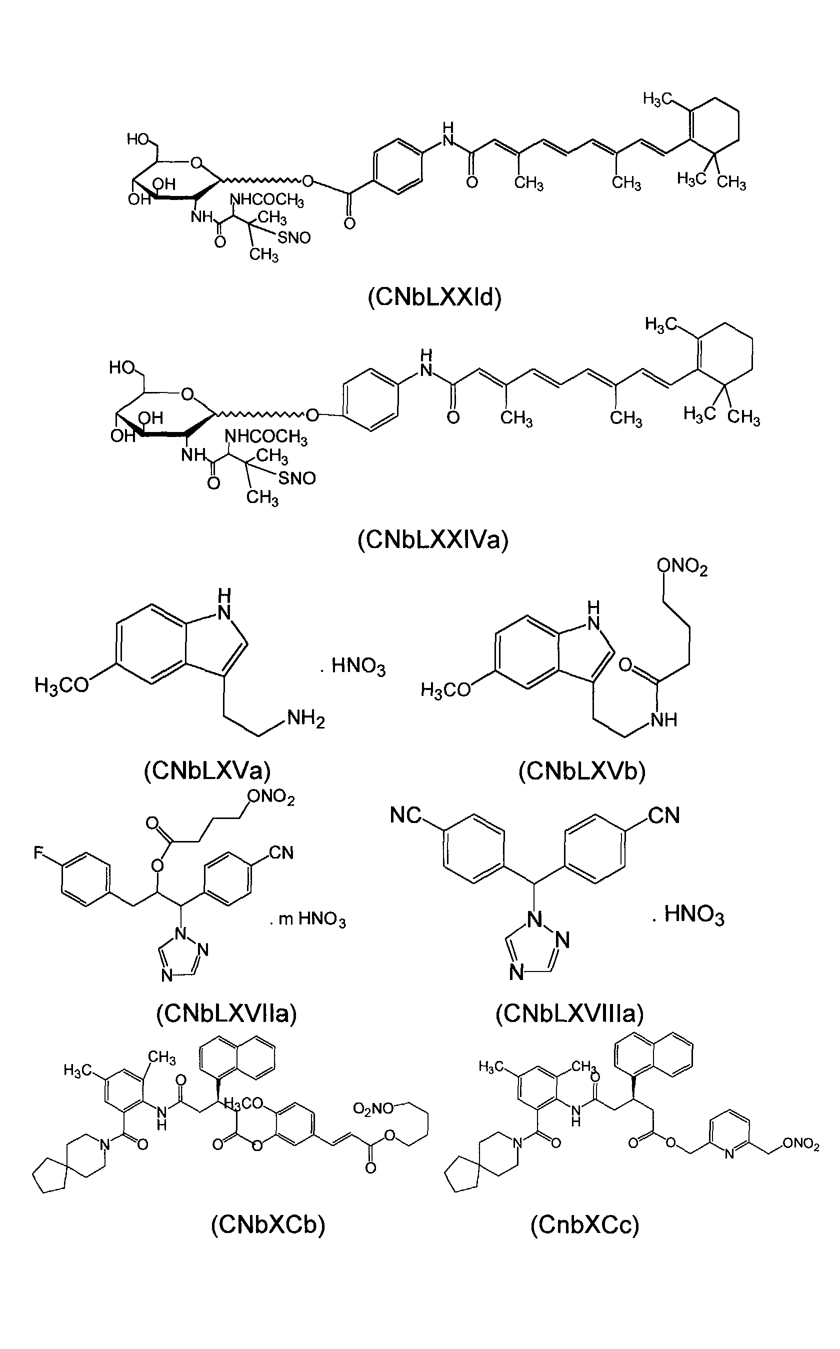

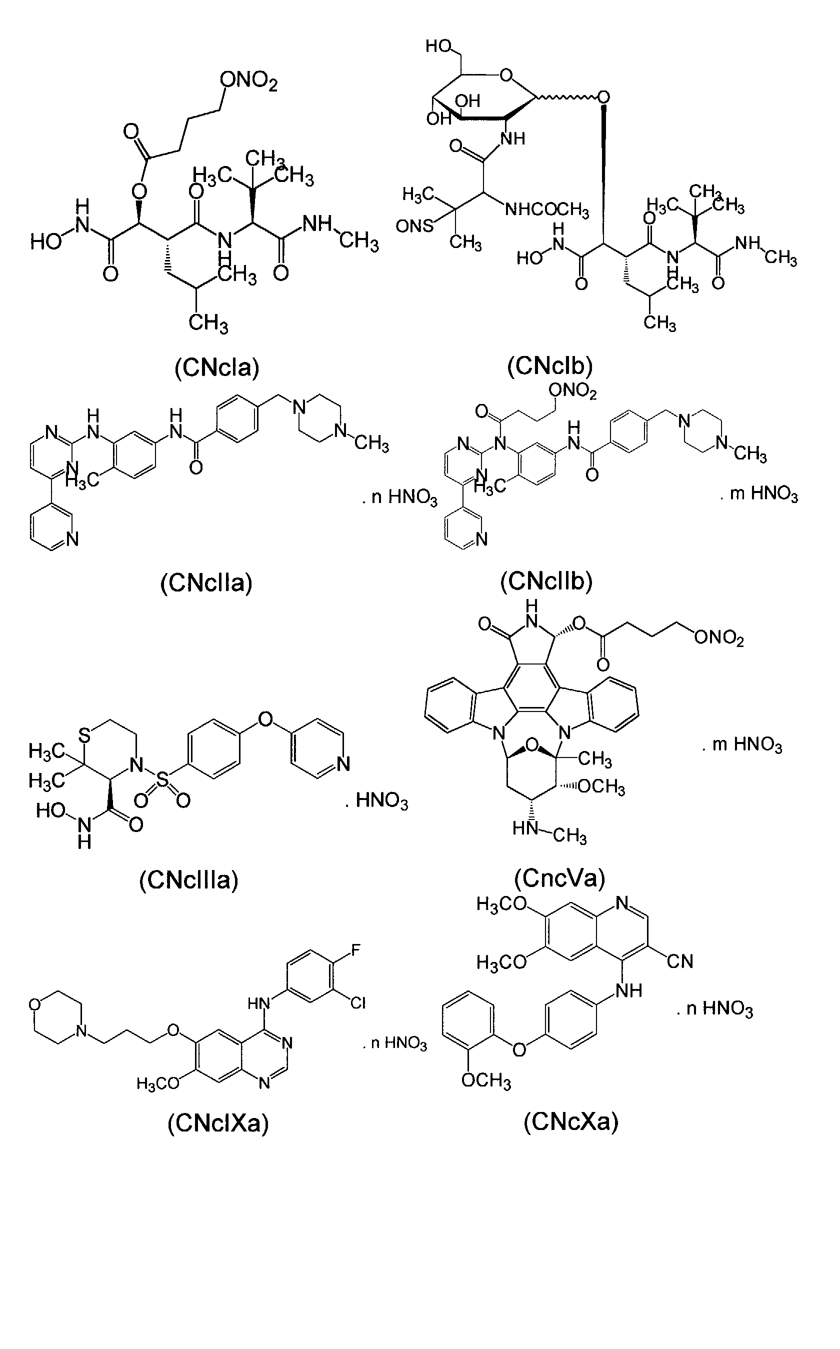

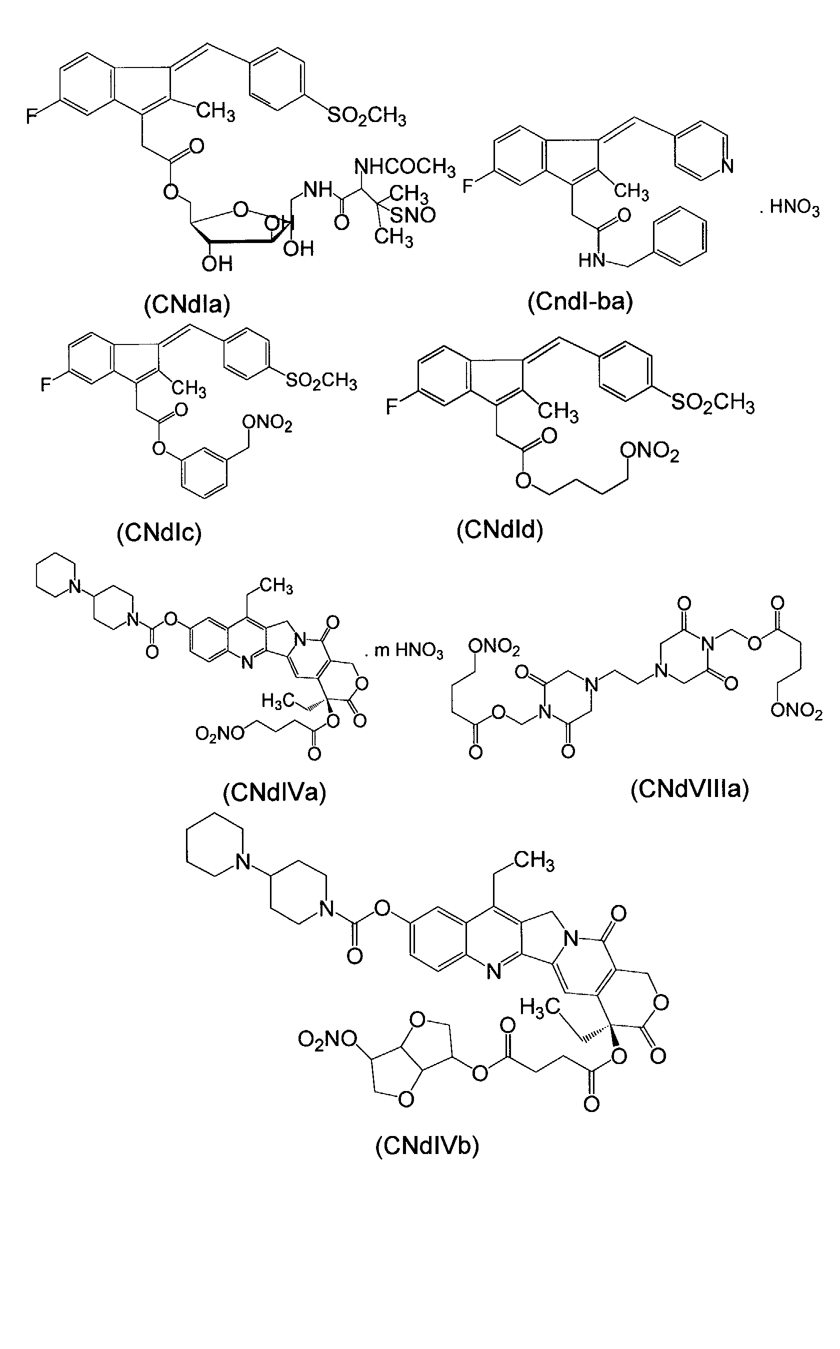

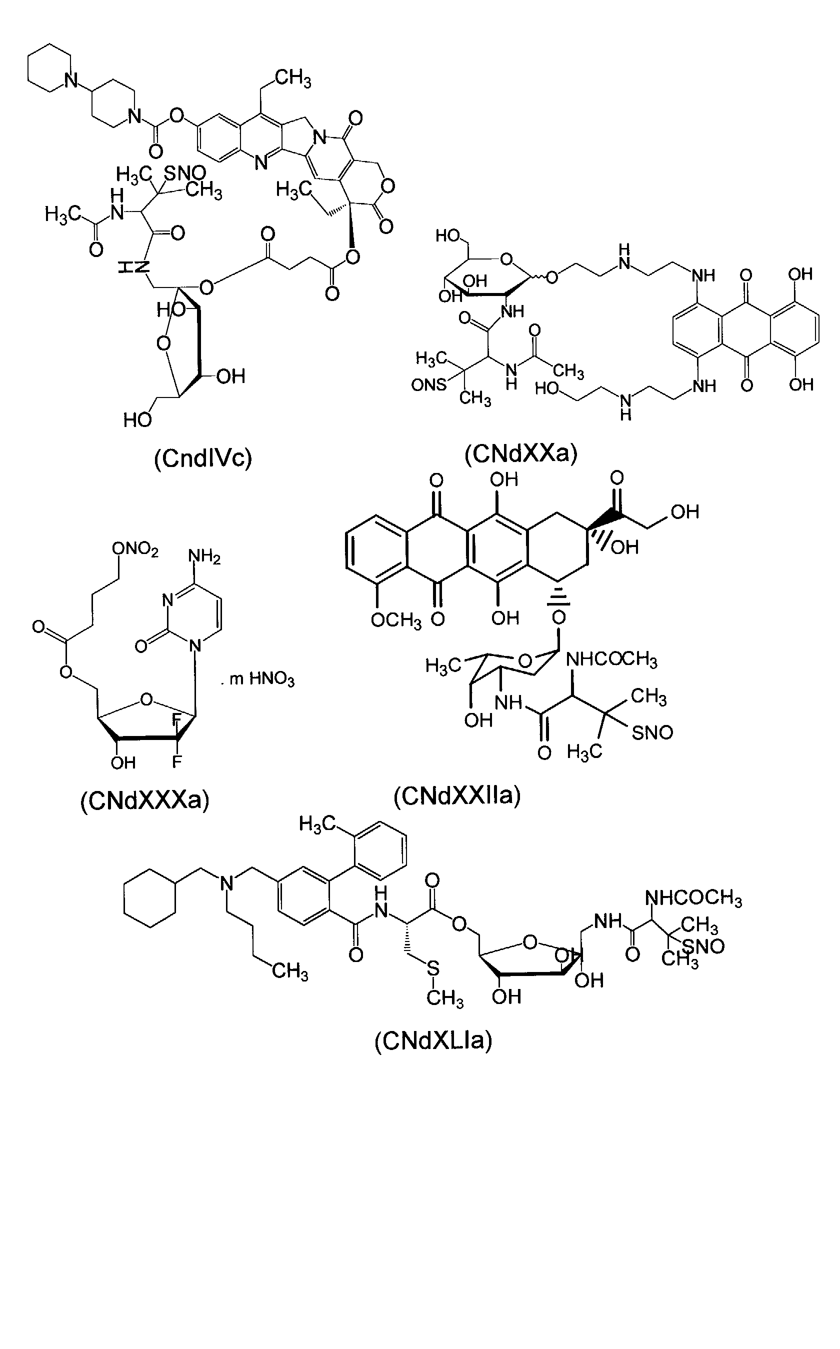

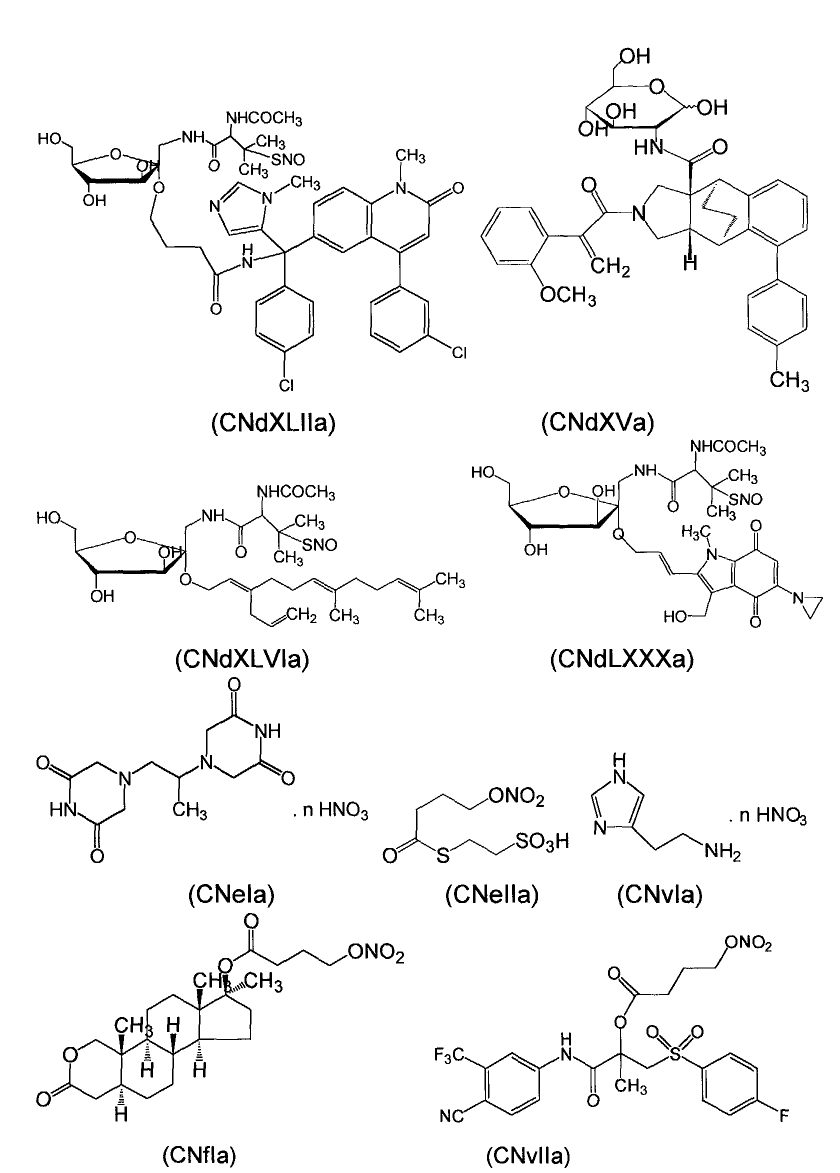

More preferably the compounds of the invention are chosen among the following 6 gropus of compounds

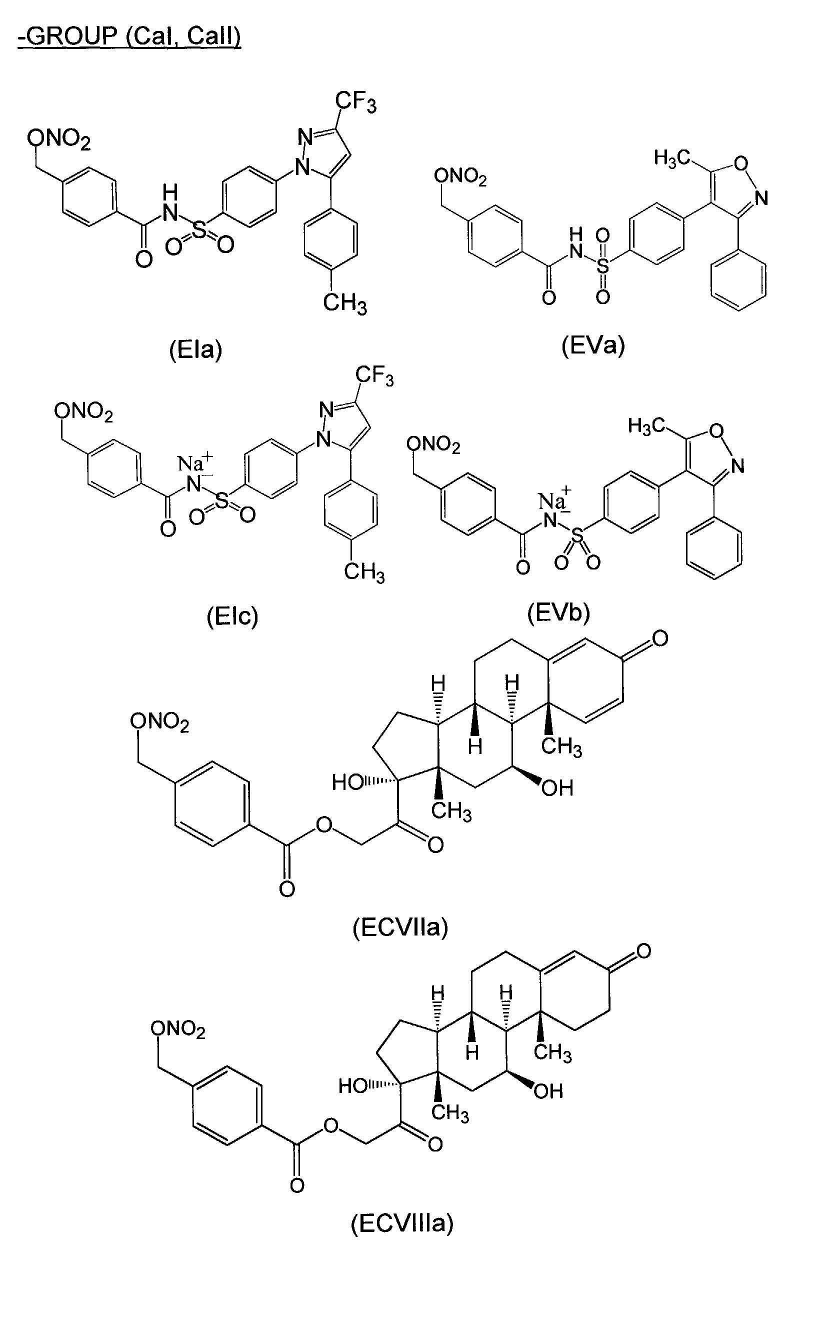

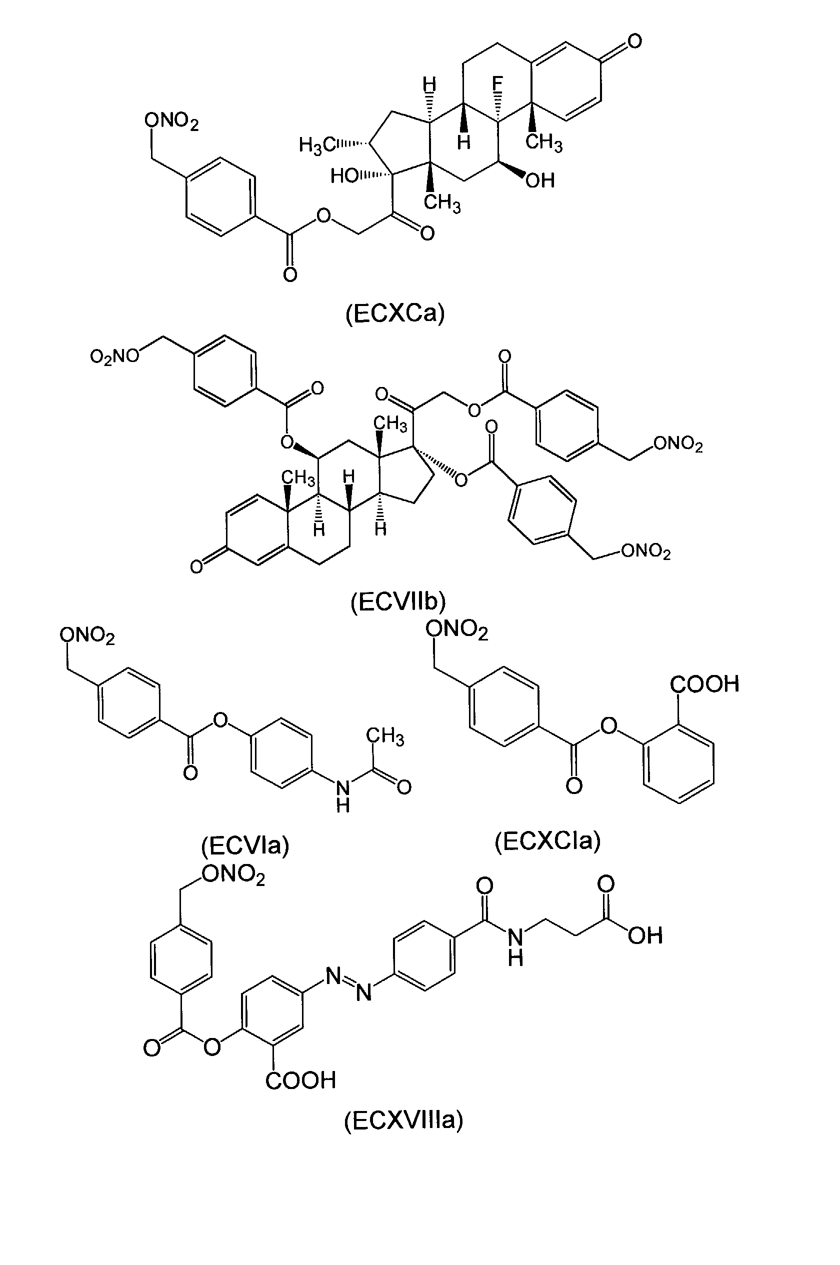

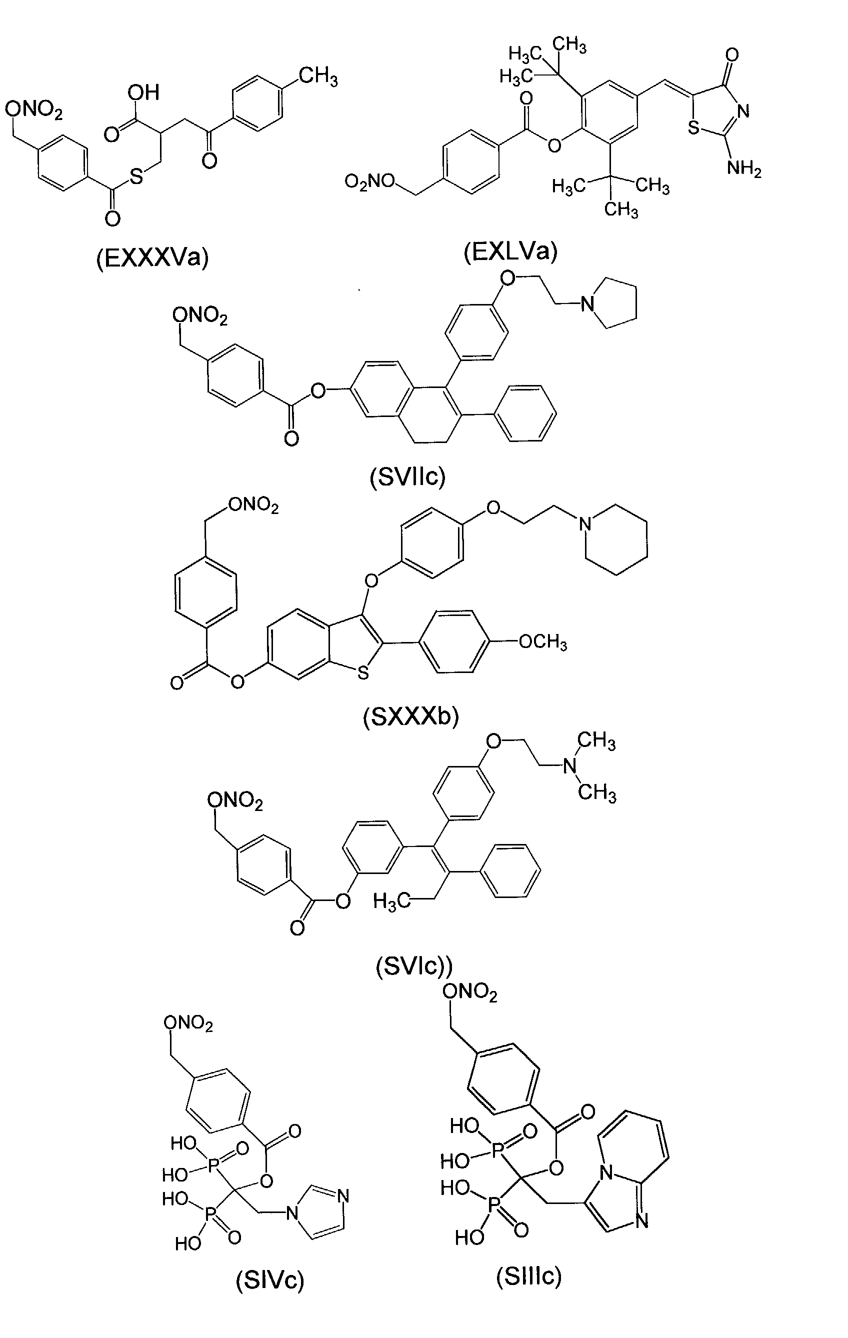

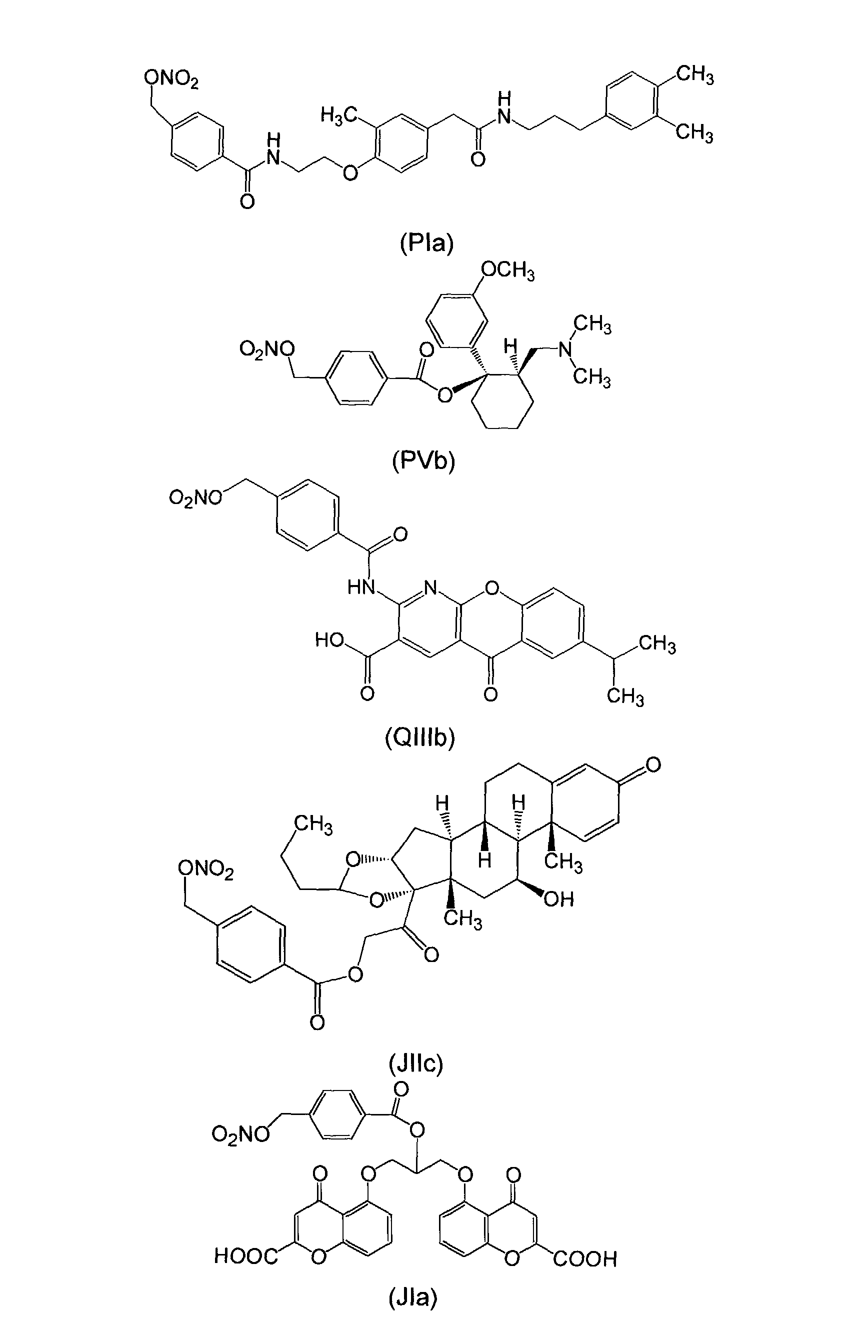

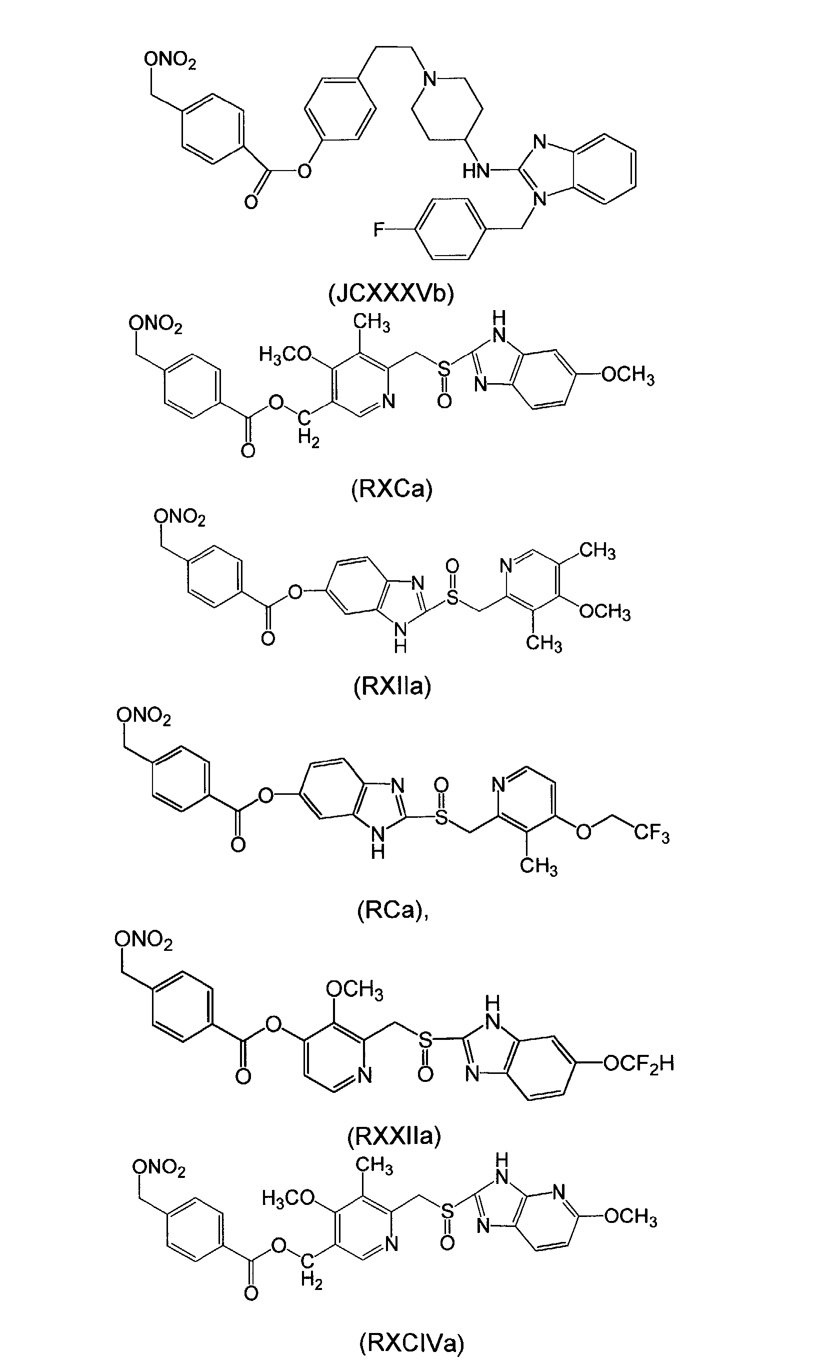

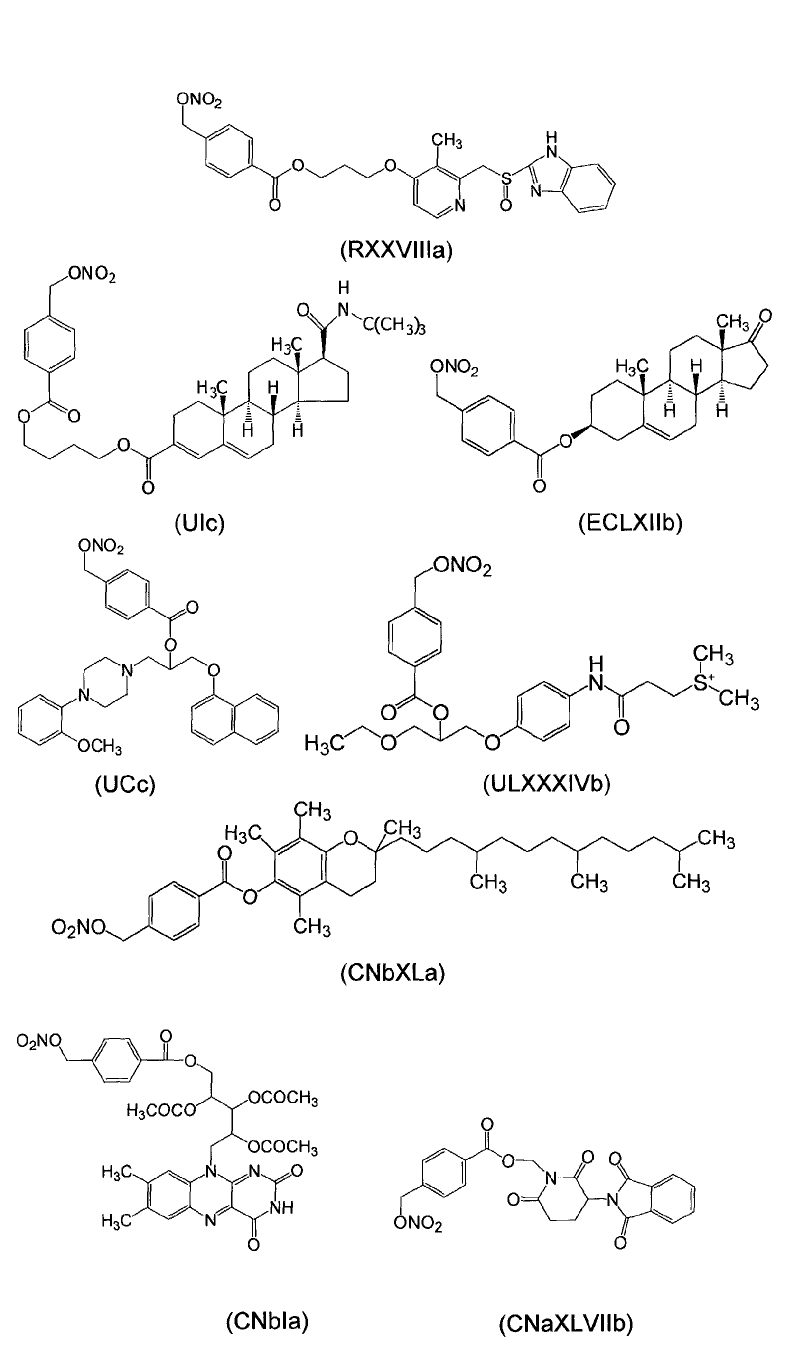

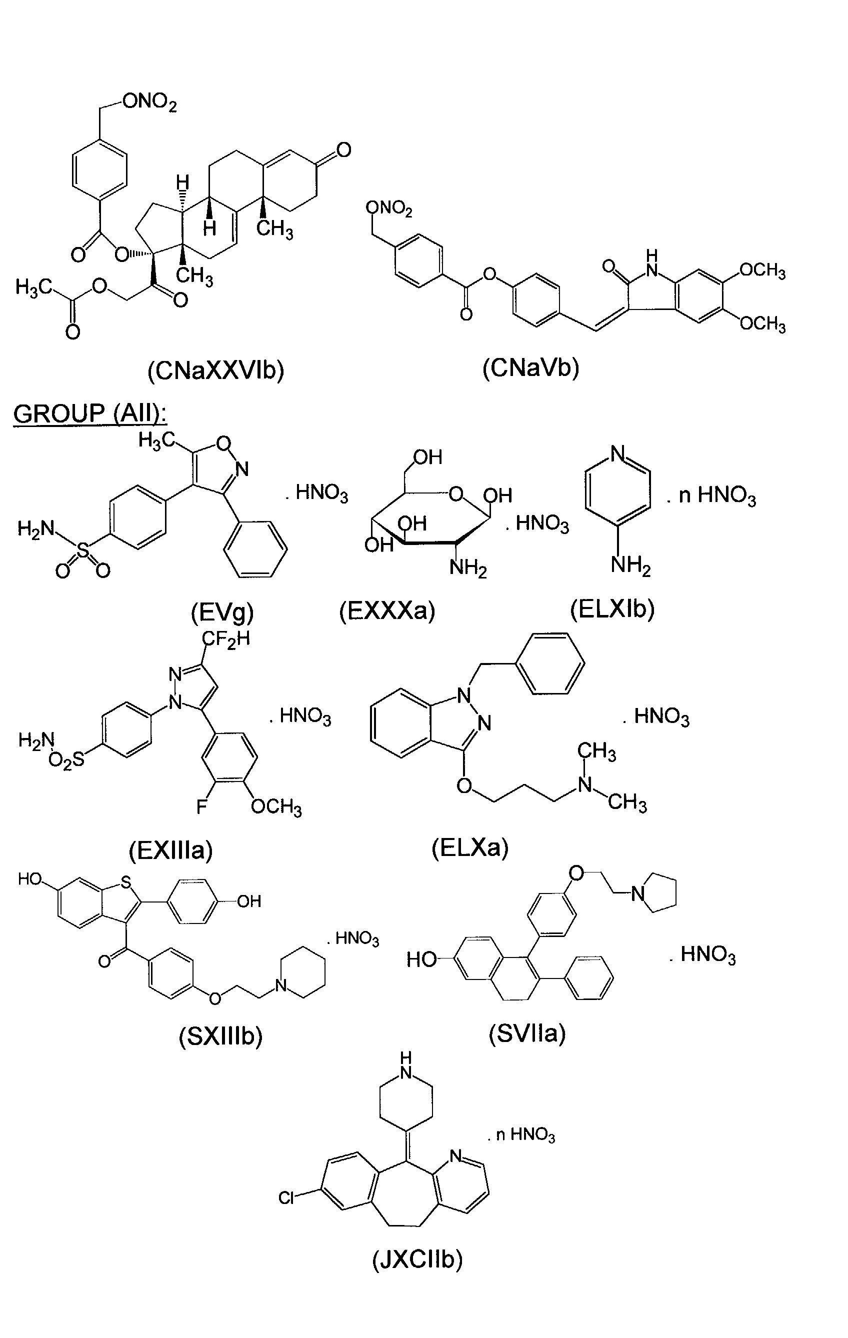

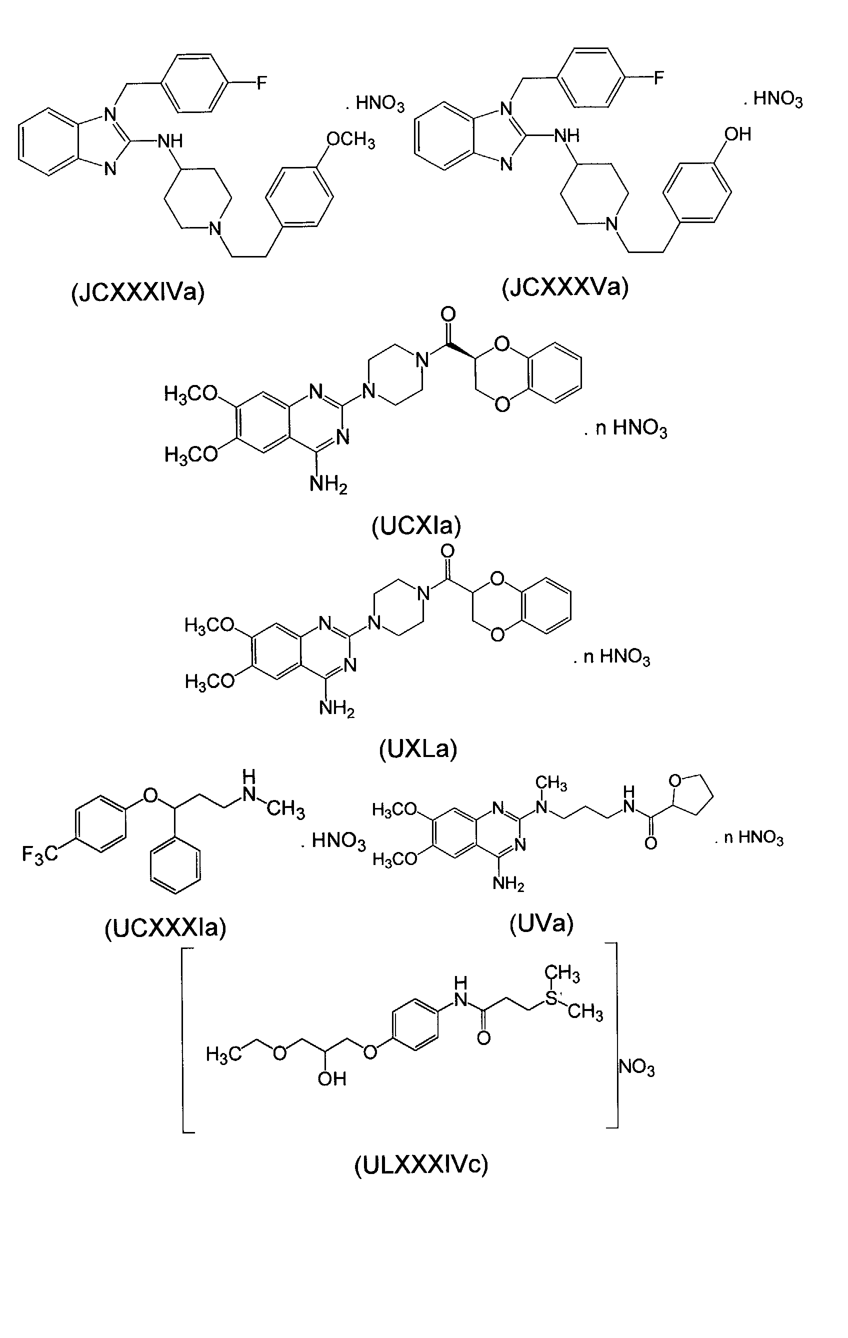

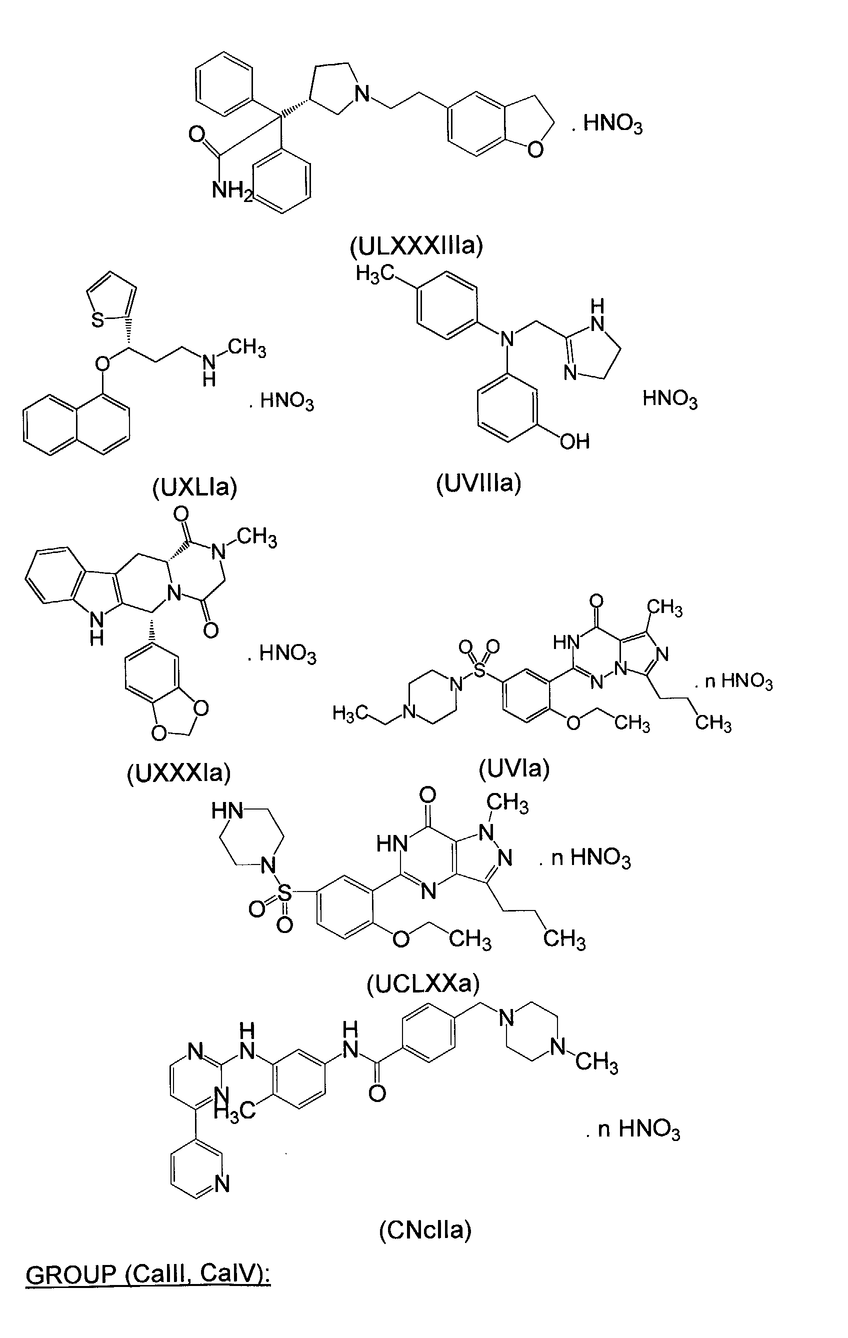

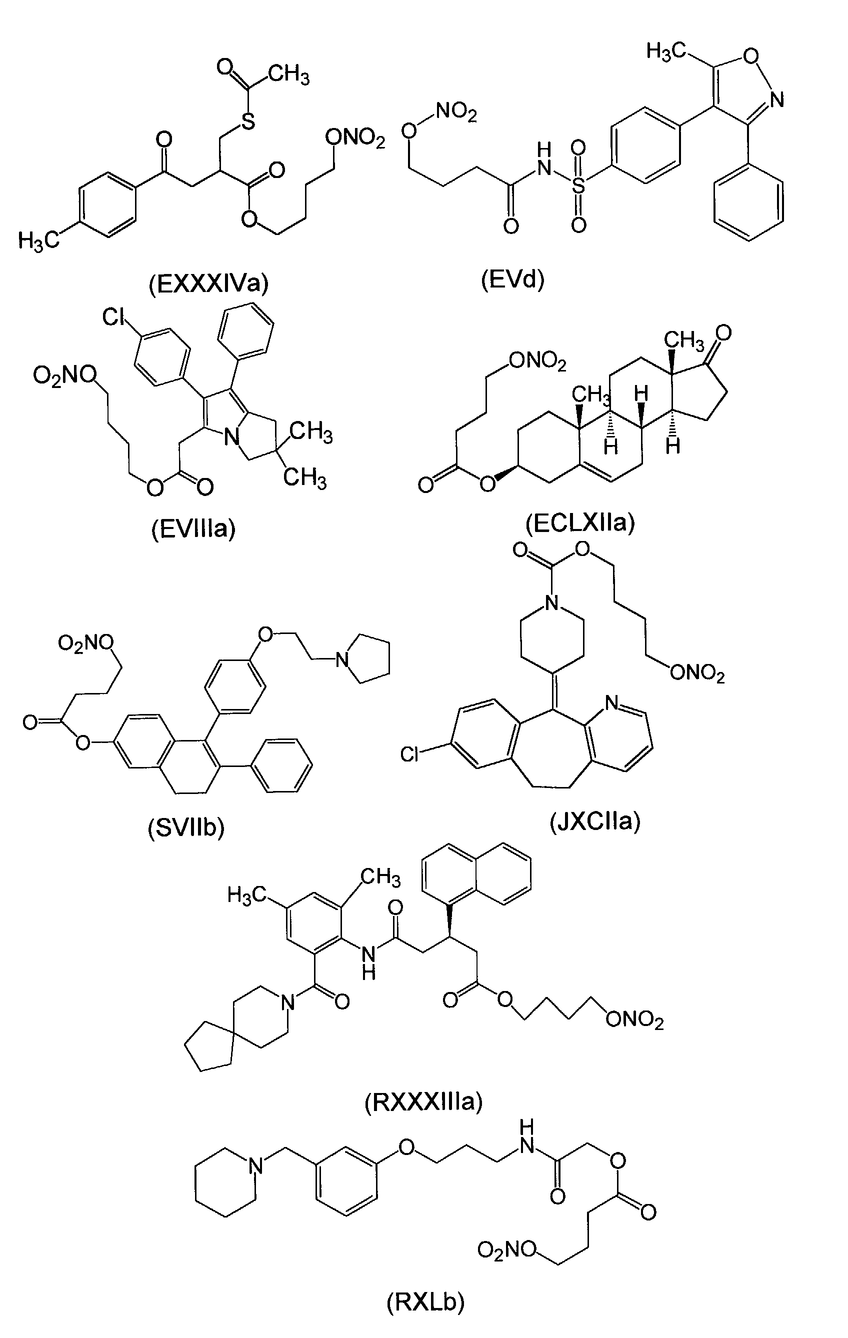

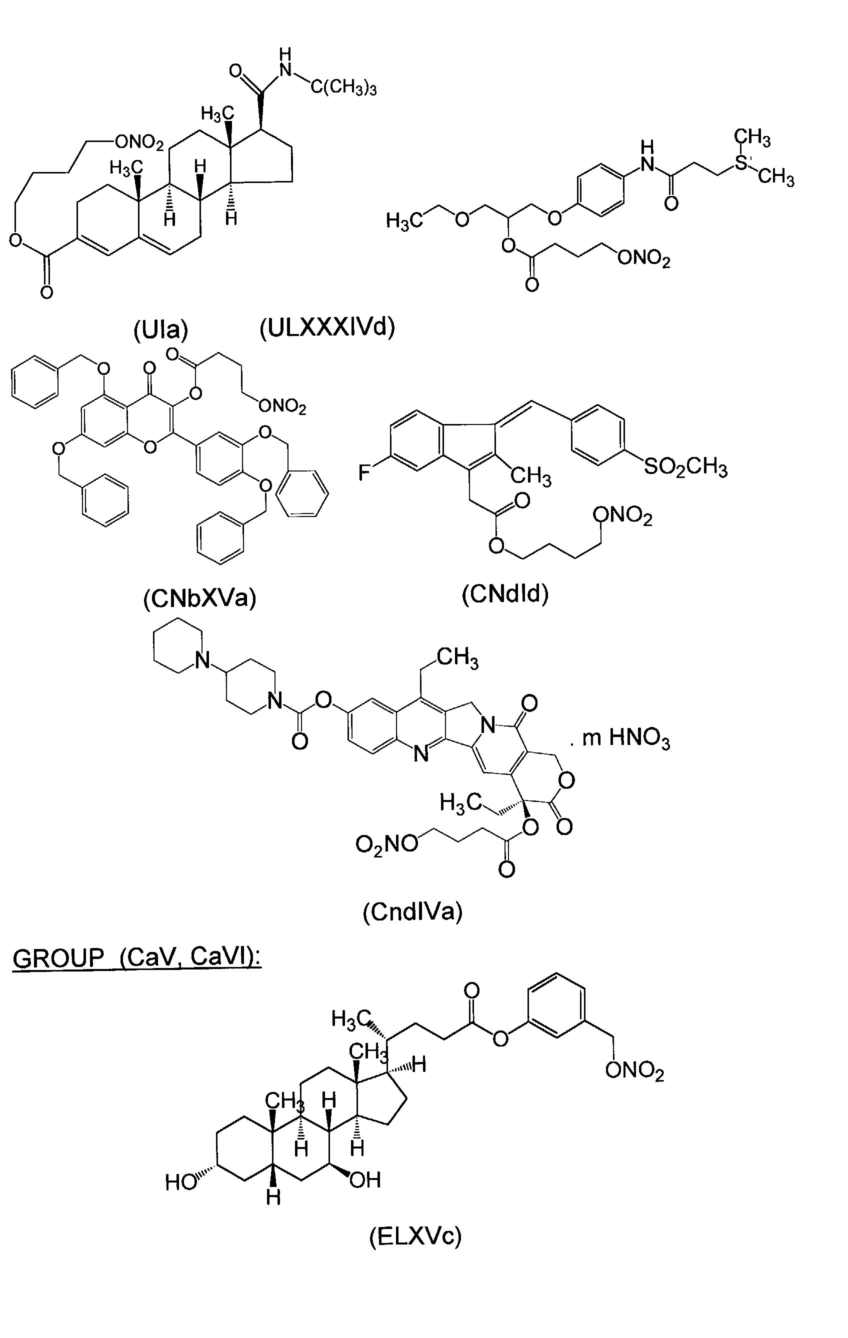

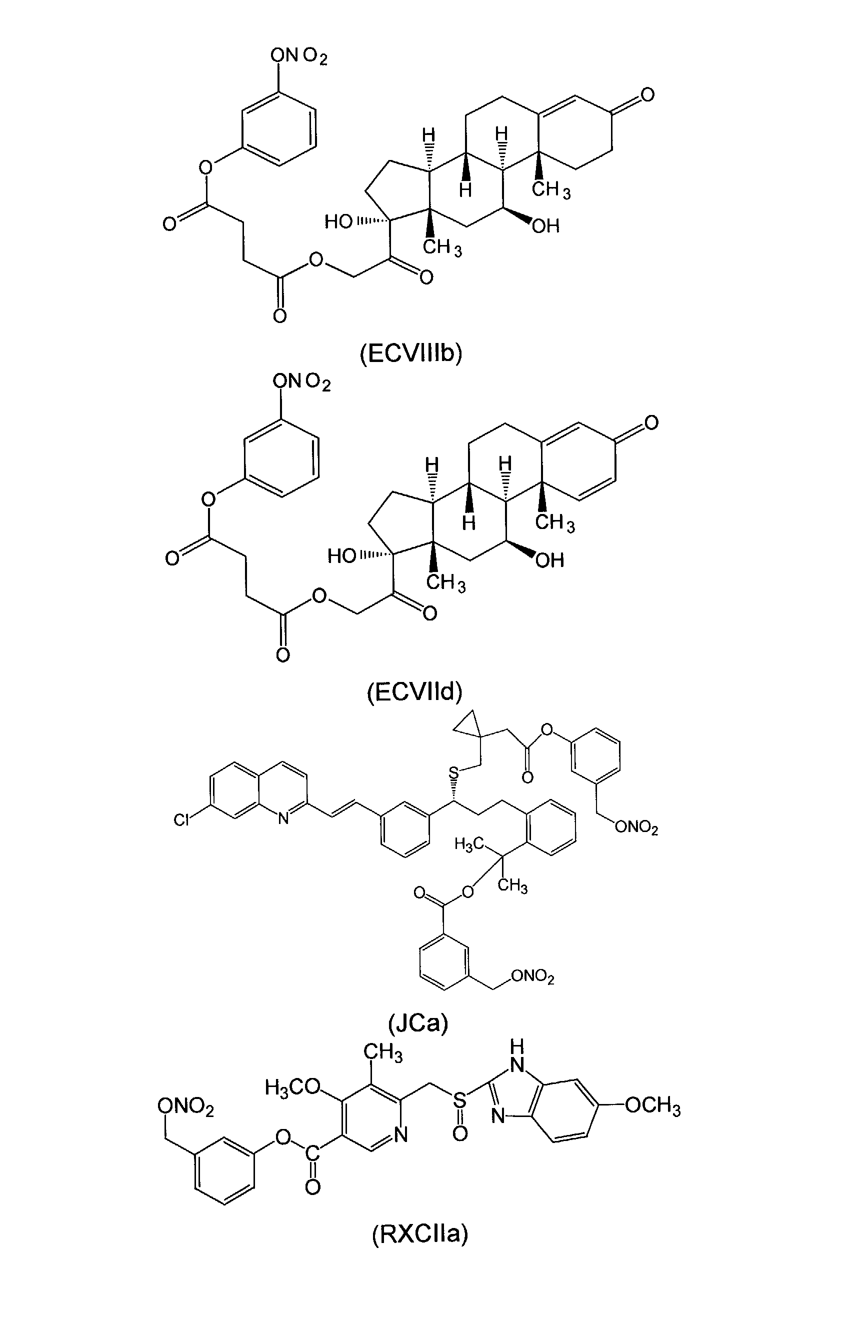

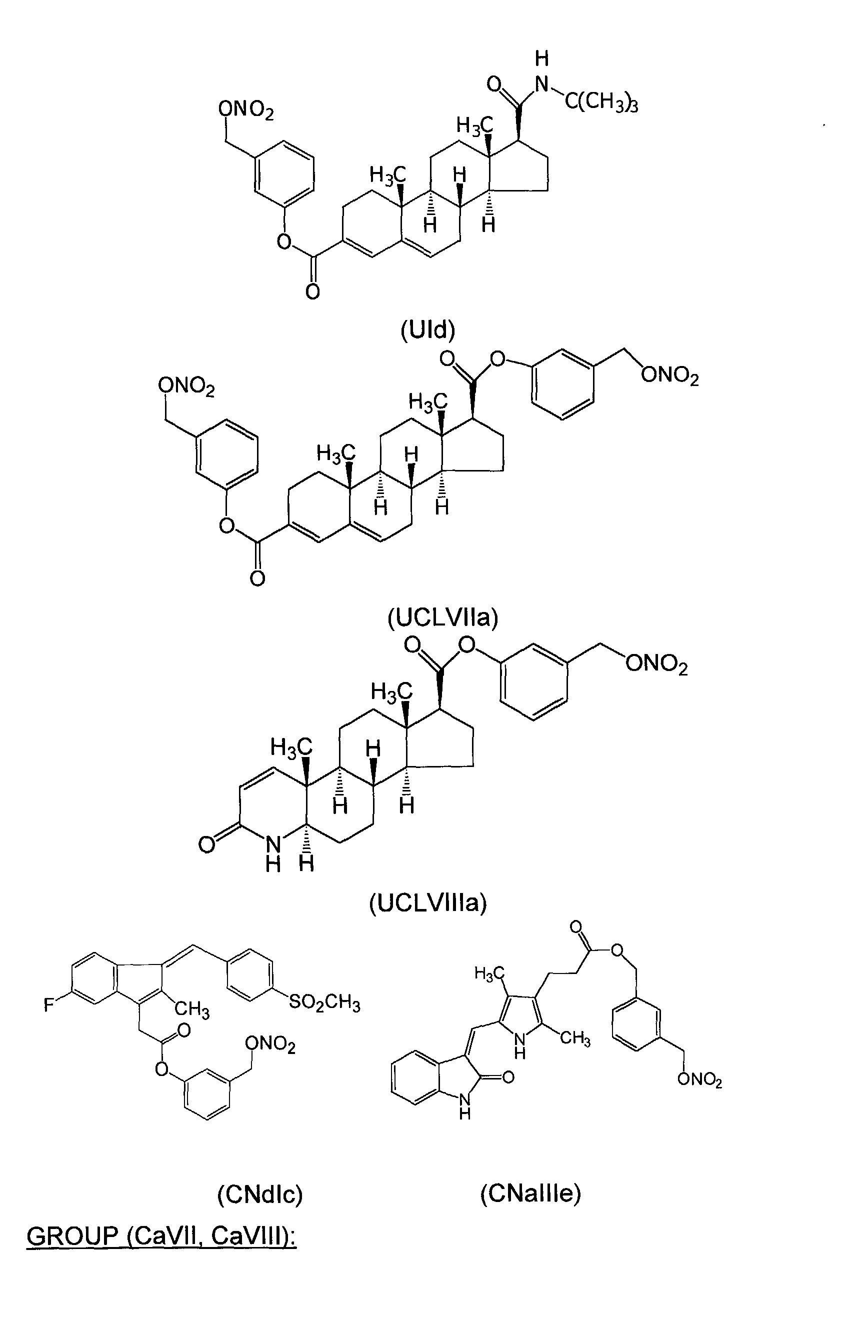

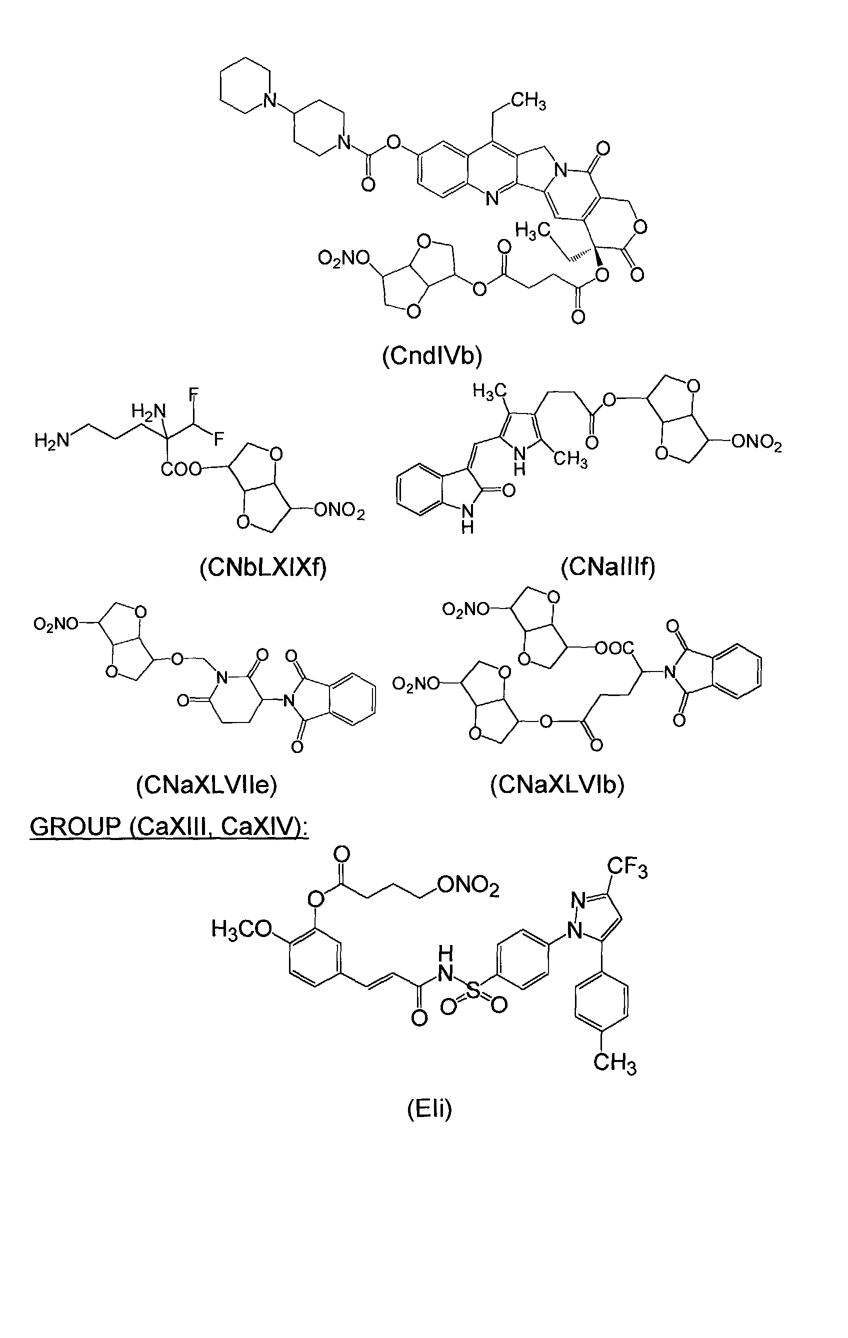

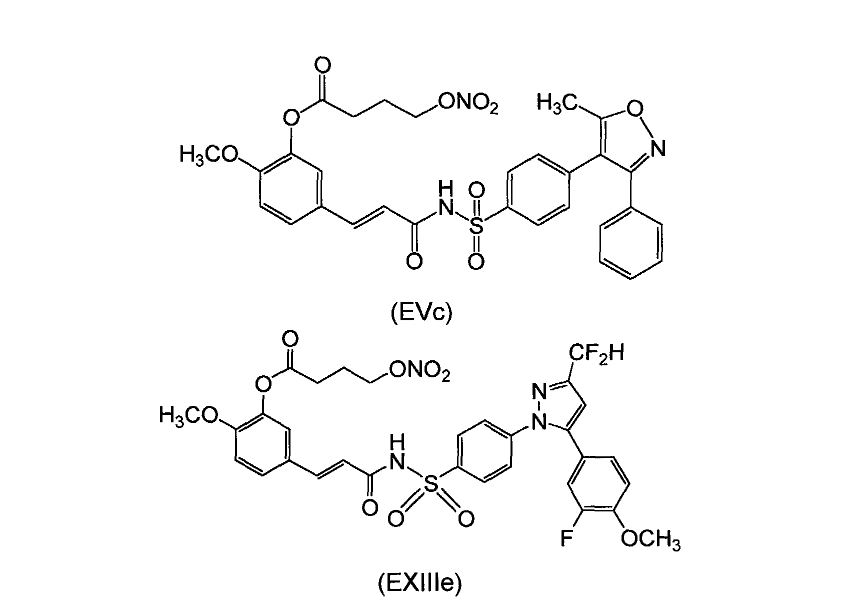

The compounds of general formula (I) are synthetized according to well known reactions described for example in "Advanced Organic Chemistry" di J. March, Wiley Interscience, IV ed. (see chemical groups index pag.1269-1300) and "Methods in Nitric Oxide Research" di M. Feelisch, J.Wiley & Sons, 1996 (from pag. 71 to pag 115). As described above , M groups can be directly bound to F drugs or to T and V groups which are bound to F drugs. Preferably F drugs are bound to -Y₁, -Y₂ and -Y₃.

The compounds of the invention can be synthetized according to known methods and in particular we can distinguish:
A) methods for the synthesis of nitrate salts
B) methods for the synthesis of nitroxy substituted linkers
C) methods for the synthesis of other NO-donors
D) methods for the synthesis of in vivo hydrolyzable bonds
E) methods for the synthesis of nitroxy substituted organic esters (e.g. benzoate, butanoate)
F) methods for the synthesis of amides

### A) METHODS FOR THE SYNTHESIS OF NITRATE SALTS

Nitric salts (AII) may be synthetized according to one of the following methods:
A1) When the substance to be salified is available as free base or as a soluble corresponding salt in an organic solvent, which preferably does not contain hydroxyl groups, for example acetonitrile, ethyl acetate, tetrahydrofuran etc., the salt is prepared by dissolving the substance in the solvent at a concentration preferably equal or higher than 10% w/v, by adding the amount of concentrated nitric acid corresponding to the moles of salifiable aminic groups present in the compound. The nitric acid is preferably diluted in the same solvent. Preferably during and after the addition the mixture is cooled to temperatures in the range 0-20 °C. The product is generally recovered by filtration and washed with the solvent.
   When the substance is not much soluble or it is available as a not much soluble salt in the above mentioned solvents, the corresponding mixtures with hydroxylated solvents may be used. Examples of such solvents are methyl alcohol, ethyl alcohol and water.
   The precipitation can be quickened by diluting then the so obtained mixture, after the addition of nitric acid, with an apolar solvent.
A2) When the starting product is salified with a halogen acid, it is possible to prepare the salt with nitric acid directing adding silver nitrate to the compound solution. After filtering the silver halide, the solution is concentrated and cooled to recover the nitrate salt.
A3) When the starting product is a salt different from halide, it is preferable to release the corresponding base by a treatment with a sodium or potassium carbonate or bicarbonate saturated solution, or with a sodium or potassium hydroxide diluted solution. The base is then extracted by a suitable organic solvent (e. g. halogenated solvents, esters, ethers) which is then dried. The organic solution is evaporated and then one proceeds according to the above described preparation methods, by dissolving the base in a solvent which does not contain hydroxyl groups (e.g. acetonitrile) or in a mixture of this solvent with a hydroxylated solvent.

### B) METHODS FOR THE SYNTHESIS OF NITROXY SUBSTITUTED LINKERS

Nitrate esters (Al) may be prepared according to one of the following methods:
B1) Direct nitration of an alcoholic group with concentrated nitric acid
B2) Nucleophilic substitution of an alkyl halide (preferably bromide or iodide) with silver nitrate. This synthesis is preferred and it is carried out in an apolar solvent (preferably acetonitrile), at room temperature or at reflux and in the dark. The starting halide is obtained by direct halogenation of hydroxyls with known methods (e.g. PBr₃) or by radical halogenation (e.g. radical HBr) of alkylic groups.

### C) METHODS FOR THE SYNTHESIS OF OTHER NO-DONORS

C1) Nitrite esters (AIII) are synthetized according the reaction of alkyl halide (preferably bromide) with NaNO₂ according to the known literature.
C2) Thionitrite (AIV) are synthetized by nitrosylation of thiolic group with NaNO₂ at acid pH (about 2,5).
C3) NONOate (AV) are synthetized by exposition of the nucleophile (usually nitrogen but also sulphur) containing molecule, dissolved in an organic solvent, to gaseous nitric oxide over a period of 1-3 days and then collecting the formed precipitate..

### D) METHODS FOR THE SYNTHESIS OF IN VIVO HYDROLYZABLE BONDS

As for the bonds between -F and -T, between -F and -V or between -F and -Y₁, -Y₂ and -Y₃, that is a carboxylic ester, a carboxylic amide, a glycoside, an acetal, a ketal, an azo compound, an hydrazide, a carbammate, a thioester, a thioamide, a phosphoric ester, a phosphonic ester, a sulphuric ester, a sulphonic amide or a sulphonic ester, they may be prepared according to several known synthetic procedures. For a complete review see "Advanced Organic Chemistry" above cited, in particular the chemical classes index: esters and amides at pag. 1275 and 1281; acid halides (esters and amides precursors) at pag. 1269; thioamides and thioesters at pag 1297 and 1298; glycosides, acetals and ketals at pag. 1269; phosphonic esters at pag. 1295; sulphonic amides and sulphonic esters a pag. 1296; azo compounds and carbammates at pag. 1280; hydrazides at pag. 1288. As for the synthesis of alkyl halides (intermediates for the synthesis of nitrate esters, nitrite esters and thionitrite via nucleophilic substitution) see at pag. 1274.

### E) METHODS FOR THE SYNTHESIS OF NITROXY SUBSTITUTED ORGANIC ESTERS (E.G. BENZOATE, BUTANOATE)

Carboxylic esters are synthetized by:
E1) Reaction of an acid and an alcohol in the presence of a dehydrating agent
E2) Acylic nucleophilic substitution reaction of an alkyl halide and an alcohol
E3) Alyfatic nucleophilic substitution reaction of an alyfatic halide, preferably bromide, and a carboxylic ion

### F) METHODS FOR THE SYNTHESIS OF AMIDES

### F1) Carboxylic amides are synthetized by acylic nucleophilic substitution of an acid halide and an amine.

The prodrugs of the invention are useful for a chronic, controlled and selective administration of nitric oxide and therefore for prevention and treatment of inflammatory, ischemic, degenerative and proliferative diseases of muscoloskeletal system (e.g. rheumatoid arthritis, osteoarthritis, lupus,osteoporosis, Paget disease),of tegumental system (e.g. ulcers, aphtha, psoriasis, dermatitis, skin tumors) of respiratory system (e.g. asthma, chronic obstructive pulmonary disease, allergy, emphysema, bronchitis, ARDS, rhinitis, cystic fibrosis, lung tumors), of gastrointestinal system (e.g. peptic ulcer, duodenal ulcer, ulcerative colitis, Crohn disease, hepatic cirrhosis, pancreatitis, esophagitis, gastritis, colon familial polyposis, colon-rectum tumors, liver tumors, oesophasus tumors, pancreas tumors, stomach tumors), of genital system (e.g. benign prostatic hypertrophy, prostatitis, sexual disorders, prostate tumors, breast tumors, uterus tumors, ovary tumors), of urinary system (urinary incontinence, bladder tumors) and of central nervous system (e.g, multiple sclerosis, Alzheimer disease, brain tumours).

Compounds of general formula (I) are preferably in a completely or partially amorphized form in order to improve their bioavailability that is to increase their AUC and/or their maximun plasmatic concentration (Cₘₐₓ) or to reduce the time necessary to obtain this concentration (Tₘₐₓ). This because it is known that an amorphized compound has usually a better solubility and a better solubilization rate than the same compound in a crystallized state.

The amorphized compounds of general formula (I) are obtainable by treating these compounds with one or more excipients, capable to amorphize these compounds. The techniques used for the amorphization are for example lyophilization, kneading and preferably spray-drying and co-grinding. In particular in the spray-drying technique the active principle is dissolved in a solvent, for example an alcohol, and the so obtained solution is mixed at room temperature with a solution or a suspension of excipients capable to amorphize the compounds of general formula (I). The resulting solution or suspension is treated in a spray-drying equipment. This last technique is also called solid dispersion technique and is particularly preferred.

The excipients are chosen from one of the following classes: polyalcohols (e.g. mannitol, sorbitol); monosaccharides or disaccharides (e.g. glucose, fructose, lactose), polysaccharides and their derivatives (e.g. microcrystalline cellulose, hydroxypropylmethylcellulose (HPMC)); polyvinylpyrrolidone (PVP); cyclodextrins and their derivatives (e.g. α-cyclodextrin, β-cyclodextrin, methyl-β-cyclodextrin, carboxymethyl-β-cyclodextrin, acetyl-β-cyclodextrin, 2-hydroxypropyl-y-cyclodextrin, Y-cyclodextrin, dimethyl-β-cyclodextrin, 2-hydroxyethyl-β-cyclodextrin, 2-hydroxypropyl-β-cyclodextrin, 3-hydroxypropyl-β-cyclodextrin, trimethyl-β-cyclodextrin); agents which can increase the membrane permeability (e.g. SNAC, sodium caprinate).

Preferably they are chosen among: polyvinylpyrrolidone, hydroxypropylmethylcellulose, lactose, microcrystalline cellulose, 2-hydroxypropyl-β-cyclodextrin, 3-hydroxypropyl-β-cyclodextrin.

The necessary amorphization degree, which can be measured by a calorimetric experiment with DSC, is at least of 5 %, but preferably greater than 80%.

Thanks to this amorphization is possible to administer compounds of general formula (I), usually more lipophilic than original drugs and consequently with solubility problems, also per os (e.g. tablets, capsules) and also through a modified release formulation: in fact both conditions require a not too low solubility.

Preferably the modified release pharmacutical formulations are parenteral, per os (diffusion systems, dissolution systems, erodable systems, osmotic systems and systems with ionic exchange resins), and transdermic release therapeutic systems (with reservoir, with membrane and with diffusion through polymeric membrane).

The compounds so obtained by spray-drying have a better bioavalability as shown by dissolution test and they can so be administered per os.

The w/w ratio among compounds of general formula (I) and the above cited excipients is preferably between 1:0,7 and 1:10.

Particularly preferred, as formulations for the compounds of this invention, are solid dispersion prepared with spray-drying technique using polyvinylpyrrolidone as excipient according to a drug/PVP ratio of 28/72 w/w.

The compounds of this invention may be administered orally, rectally, parenterally or by locally application (dermal, topical, transdermal, ocular, inhalation etc.)

The following examples are given to illustrate the invention and they are not limitative of the same.

### SYNTHESIS EXAMPLES

### EXAMPLE 1

### paranitroxymethylbenzoic acid (ZI)

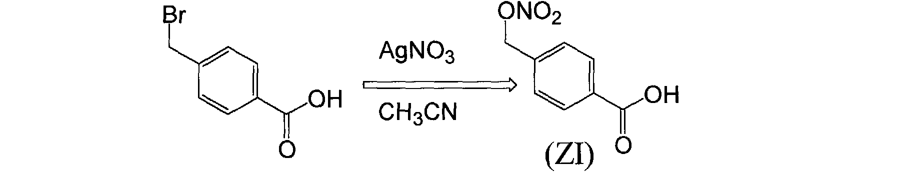

To a suspension of 5.38 g of 4-bromomethylbenzoic acid (0.025 moles) in anhydrous acetonitrile (20 mL), silver nitrate (AgNO₃) (4.95 g, 0.029 moles) dissolved in anhydrous acetonitrile (10 mL) was added drop by drop. The reaction mixture was left away from light, under stirring, at reflux (81 °C) for 8 hours and at room temperature for other 16 hours. The suspension was filtered with buchner: the precipitate, that is the product and AgBr, was dissolved in methanol (150 mL), than it was heated and finally it was filtered. The solvent was evaporated at reduced pression and 3.75 g of the desired product were obtained.
Yield: 80%.
1H-NMR (500 MHz, DMSO-*d*₆): δ 7.98 (d, 2H, J = 8.1 Hz), 7.58 (d, 2H, J = 8.1 Hz), 5.65 (s, 2H);
IR (KBr) cm⁻¹: 1690 (C=O), 1670 e 1270 (N=O).

### EXAMPLE 2

### p-nitroxymethylbenzoyl-α-tocopherol (CNbXLa)

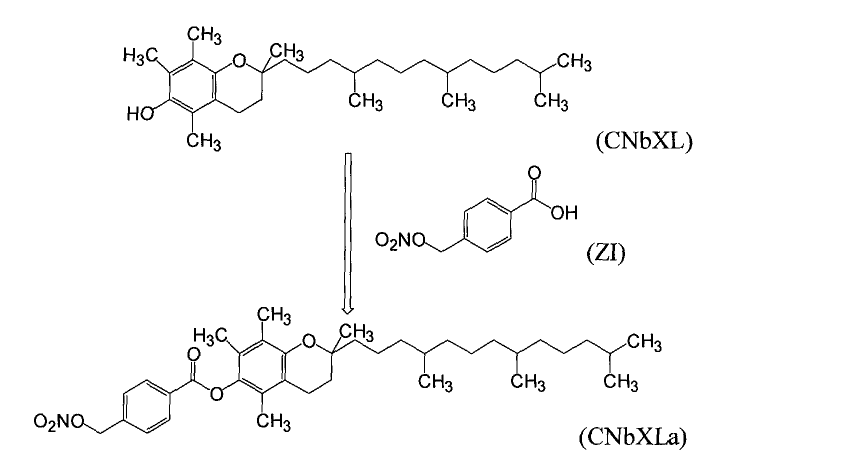

578 mg of N,N'-dicyclohexylcarbodiimide (DCC) (2.8 mmoli), 607 mg (3.08 mmoli) of paranitroxymethylbenzoic acid (ZI) (synthetized according to example 1) and a catalytic quantity of 4-dimethylilaminopyridine (4-DMAP) were added, under nitrogen atmosphere, to a solution of α-tocopherol (Sigma-Aldrich code T-3251) (1.2 g, 2.8 mmoles) in CH₂Cl₂ (34 ml).

The reaction mixture is left under stirring for 5 hours. Et₂O is added to precipitate DCU which has been produced during the reaction and then the reaction is filtered upon cotton. The solution was deprived of the solvent at reduced pressure. The crude product was purified by flash cromatography, packing the column with hexane and eluting with a mixture hexane/diisopropylic ether/CH₂Cl₂ 85/5/10.
1.497 g of the desired product were obtained. Yield: 88%.
R_{*f*} = (hexane/diisopropylic ether 6:4): 0.61
¹H-NMR (CDCl₃) δ 8.27 (2H, d, J = 8.53 Hz), 7.53 (2H, d, J = 8.53 Hz), 5.48 (2H, s), 2.62 (2H, m), 2.11 (3H, s), 2.05 (3H, s), 2.01 (3H, s), 1.85-1.70 (2H, s broad), 1.61-1.05 (26H, CH₂ e CH alyphatic chain, CH₃ ring), 0.88-0-83 (12H, m, CH₃ alyphatic chain).
¹³C-NMR (CDCl₃) δ 164.47, 149.60, 140.59, 137.78, 130.67, 128.67, 126.82, 125.04, 123.21, 117.56, 75.14, 73.66, 39.40, 37.49, 37.43, 37.42, 32.80, 27.98, 24.81, 24.45, 22.70, 22.61, 21.06, 20.64, 19.75, 19.70, 13.03, 12.17, 11.84.
MS (El, 70eV) m/Z 610.4 (M⁺H).

| Elementary analysis: | | | |
|---|---|---|---|
| Calculated | C:72.87% | H:9.09% | N:2.30% |
| Found | C:72.86% | H:9.09% | N:2.28% |

### EXAMPLE 3

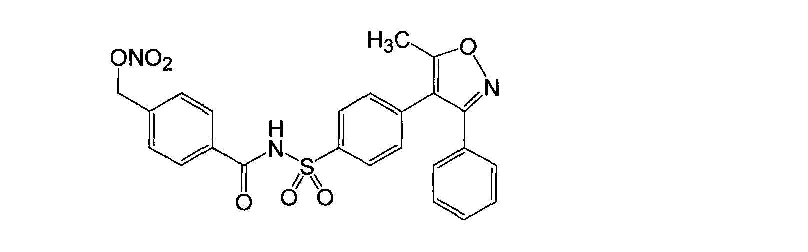

### N-[4-(5-metil-3-phenylisoxazol-4-yl)phenylsulfonyl]-p-nitroxymethylbenzamide (EVa)

Paranitroxymethylbenzoic acid (ZI) (3.5 mmoles, 689 mg) synthetized according to example 1, N,N'-dicyclohexylcarbodiimide (DCC) (3.19 mmoles, 659 mg), and 4-dimethylaminopyridine (4-DMAP) (200 mg) were added to a solution of 4-(5-methyl-3-phenylisoxazol-4-yl)benzensulfonamide (valdecoxib) (EV) (prepared as described in EP-0809636) (3.17 mmoles, 1 g) in anhydrous tetrahydrofuran. After stirring for 24 hours at room temperature, the reaction mixture was concentrated. The residue was dissolved in ethyl acetate, washed with water, dried over anhydrous magnesium sulphate and concentrated at reduced pressure.
576 mg of the desired product were obtained (yield 54 %).
MS (El, 70eV) m/Z 494.1 (M⁺H).

| Elementary analysis: | | | |
|---|---|---|---|
| Calculated | C:58.41% | H:3.88% | N:8.51% |
| Found | C:58.54% | H:3.84% | N:8.47% |

### EXAMPLE 4

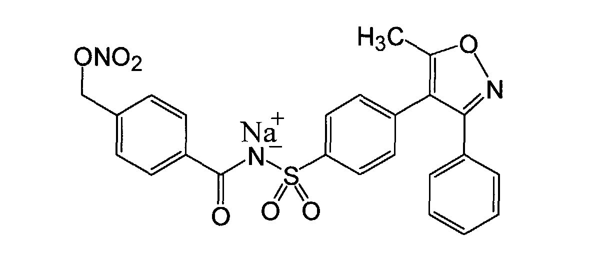

### N-[4-(5-methyl-3-phenvlisoxazol-4-vl)phenylsulfonyl]-p-nitroxymethylbenzamide sodium salt (EVb)

A mixture of N-[4-(5-metil-3-phenylisoxazol-4-yl)phenylsulfonyl]-p-nitroxymethylbenzamide (EVa) (synthetized according to example 3) (500 mg, 1.01 mmoles) and sodium hydroxide 1 N (1.01 mmoles) in ethanol was concentrated to dryness.

The residue was diluted with ehanol and was concentrated again. The residue was dried under vacuum and 494 mg of the desired product were so obtained (yield 95%).
MS (El, 70eV) m/Z 516.1 (M⁺H)

| Elementary analysis: | | | |
|---|---|---|---|
| Calculated | C:55.92% | H:3.52% | N:8.15% |
| Found | C:55.64% | H:3.39% | N:8.08% |

### EXAMPLE 5

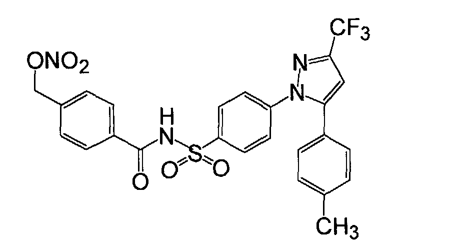

### N-[4-(5-p-tolyl-3-trifluoromethyl-1H-pyrazol-1-yl)phenylsulfonyl]-p-nitroxymethylbenzamide (Ela)

This compound was synthetized according to the synthesis described in example 3 using, as reagents, N-[4-[5-(4-methylphenyl)-3-(trifluoromethyl)-1 H-pyrazol-1-yl]]benzenesulfonamide (celecoxib) (El) (synthetized according to J Med Chem 1997, 40, pag 1347-1365) and paranitroxymethylbenzoic acid (ZI) synthetized according to example 1. Yield 58%.
MS (El, 70eV): m/z 561.1 (M⁺H)

| Elementary analysis: | | | |
|---|---|---|---|
| Calculated | C:53.27% | H:3.42% | N:10.00% |
| Found | C:53.11% | H:3.44% | N:9.97% |

### ESEMPIO 6

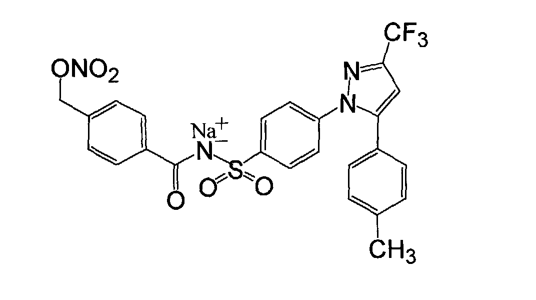

### N-[4-(5-p-tolyl-3-trifluoromethyl-1H-pyrazol-1-yl)phenylsulfonyl]-p-nitroxymethylbenzamide sodium salt (Elc)

This compound was synthetized according to the synthesis described in example 4 using, as reagents N-[4-(5-p-tolyl-3-trifluoromethyl-1H-pyrazol-1 -yl)phenylsulfonyl]-p-nitroxymethylbenzamide (Ela) (prepared as described in example 5) and sodium hydroxide. Yield 58%.
MS (El, 70eV): m/z 583.1 (M⁺H)

| Elementary analysis: | | | |
|---|---|---|---|
| Calculated | C:51.55% | H:3.11% | N:9.62% |
| Found | C:51.37% | H:3.05% | N:9.55% |

### EXAMPLE 7

### (11β)-11,17-dihydroxy-21-(p-nitroxymethylbenzoyloxy)-pregna-1,4-diene-3,20-dione (ECVIIa) (first synthesis)

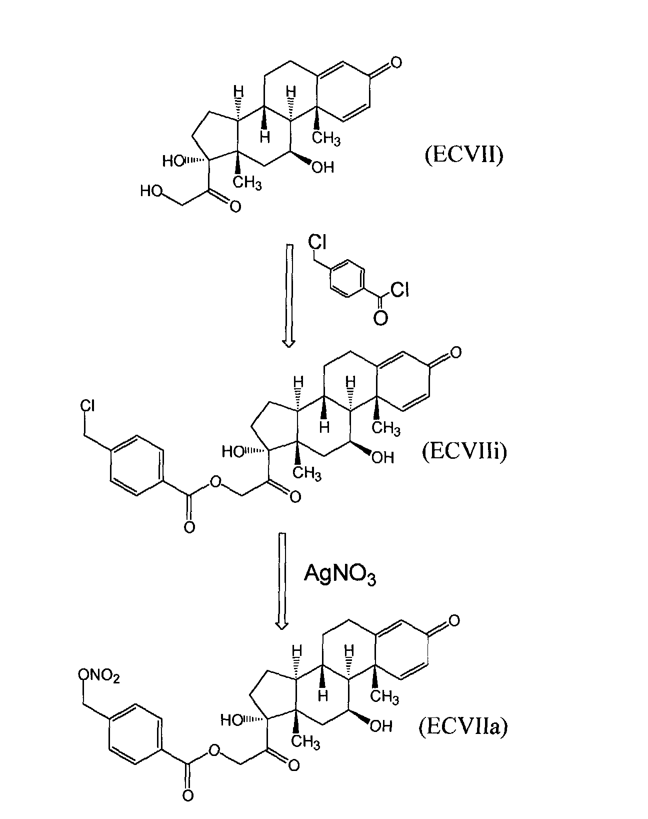

Parachloromethylbenzoyl chloride (30 mmoles) (sigma-aldrich code 270784) and triethylamine were added to a solution of (11β)-11,17,21-trihydroxy-pregna-1,4-diene-3,20-dione (prednisolone) (ECVII) (sigma-aldrich code P6004) (20 mmoles, 7.2 g) in tetrahydrofuran.

The reaction was stirred for 24 hours and then the solvent was evaporated at reduced pressure. The residue was treated with ethyl acetate, was filtered and the intermediate (ECVIIi) was obtained anhydrifying with sodium sulphate and concentration at reduced pressure (19 mmoles, 9.7 g) (yield 95%).

Silver nitrate (30 mmoles), dissolved in 20 mL of acetonitrile, was added drop by drop to the intermediate (ECVIIi) dissolved in anhydrous acetonitrile (40 mL) and tetrahydrofuran (90 mL). The reaction was stirred for 72 hours in the dark.

The precipitate was filtered, the solvent was evaporated under reduced pressure and the residue was purified by silica gel cromatography (methylene chloride/ethyl acetate 3/7) and then cristalyzed from tetrahydrofuran.

7.67 g (yield 71%) of a white powder having a melting point of 235°C are so obtained.
MS (El, 70eV) m/Z 540.2 (M⁺H)

| Elementary analysis: | | | |
|---|---|---|---|
| Calculated | C:64.55% | H:6.16% | N:2.60% |
| Found | C:64.41% | H:6.20% | N:2.51% |

### ESEMPIO 8

### (11β)-11,17-dihydroxy-21-(p-nitroxymethylbenzoyloxy)-pregna-1,4-diene-3,20-dione (ECVIIa) (second synthesis)

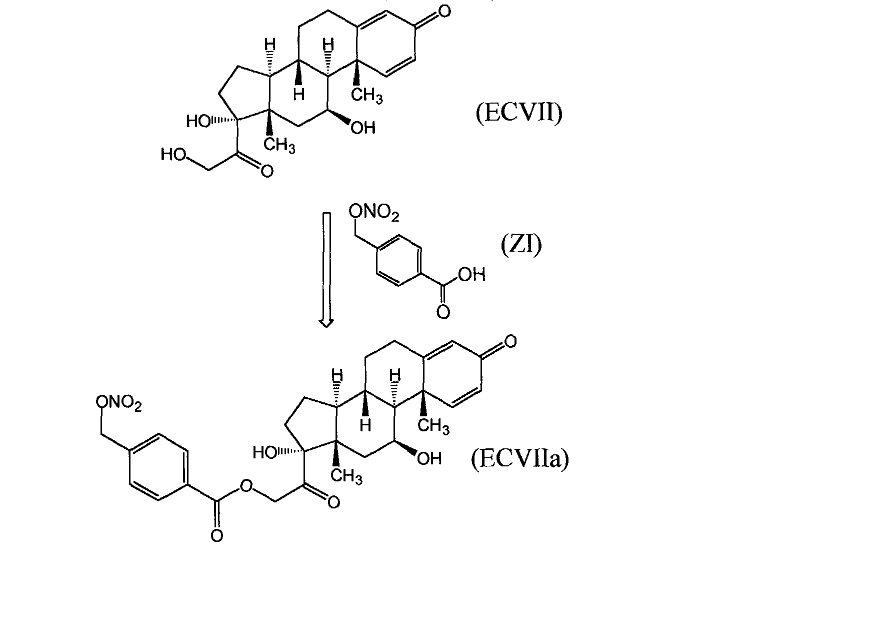

Paranitroxymethylbenzoic acid (ZI) (10 mmoles, 1.97 g) synthetized according to example 1 was added to a solution of (11β)-11,17,21-trihydroxy-pregna-1,4-diene-3,20-dione (ECVII) (prednisolone) (Sigma-Aldrich code P6004) (10 mmoles, 3.6 g) in tetrahydrofuran (90 mL).

The reaction was kept at 0 °C and under continuous stirring. N,N'-dicyclohexylcarbodiimide (DCC) (11 mmoles, 2.26 g) and a catalytic quantity of 4-dimethylaminopyridine (4-DMAP) were added.

After one hour the mixture was heated to room temperature; after 24 hours the precipitate was filtered and the solvent was evaporated at reduced pressure. The residue is treated with ethyl acetate (150 mL) and washed with water (3 X100 mL). The organic phase was anhydrified with sodium sulphate and the solvent was evaporated.

The crude product was purified by cromatography on silica gel column eluting with chloroform/ethyl acetate 3/7.
4.42 g (yield 82%) of the final product were so obtained.
MS (El, 70eV): m/z 540.2 (M⁺H)

| Elementary analysis: | | | |
|---|---|---|---|
| Calculated | C:64.55% | H:6.16% | N:2.60% |
| Found | C:64.67% | H:6.19% | N:2.63% |

### EXAMPLE 9

### (11β)-11,17-dihydroxy-21-(p-nitroxymethylbenzoyloxy)-pregna-4-ene-3,20-dione (ECVIIIa)

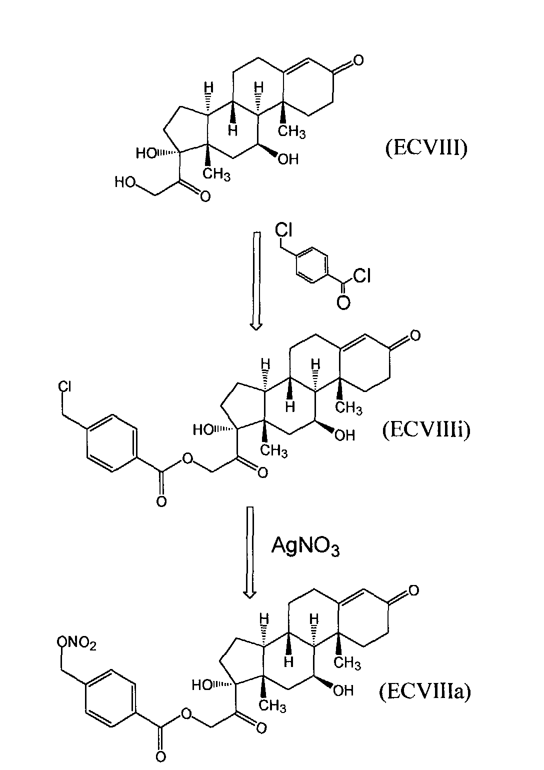

Parachloromethylbenzoyl chloride (7 mmoles) (sigma-aldrich code 270784) and triethylamine were added to a solution of (11β)-11,17,21-trihydroxy-pregna-4-ene-3,20-dione (ECVIII) (hydrocortisone) (sigma-aldrich code H3160) (5 mmoles, 1.8 g) in tetrahydrofuran.

The reaction was stirred for 24 hours and then the solvent was evaporated at reduced pressure. The residue was treated with ethyl acetate and water; it was filtered and the intermediate (ECVIIIi) was obtained anhydrifying with sodium sulphate and concentration at reduced pressure (4.8 mmol, resa 96%, 2.60 g).

Silver nitrate (7.5 mmoles), dissolved in 8 mL of acetonitrile, was added drop by drop to the intermediate (ECVIIIi) dissolved in anhydrous acetonitrile (10 mL) and tetrahydrofuran (32 mL). The reaction was stirred for 72 hours in the dark.

The precipitate was filtered, the solvent was evaporated under reduced pressure and the residue was purified by silica gel cromatography (methylene chloride/ethyl acetate 3/7) and then cristalyzed from tetrahydrofuran.
2.03 g (yield 75 %) of the desired product are so obtained.
MS (El, 70eV): m/z 542.2 (M⁺H)

| Elementary analysis: | | | |
|---|---|---|---|
| Calculated | C:64.31% | H:6.51 % | N:2.59% |
| Found | C:64.44% | H:6.40% | N:2.52% |

### EXAMPLE 10

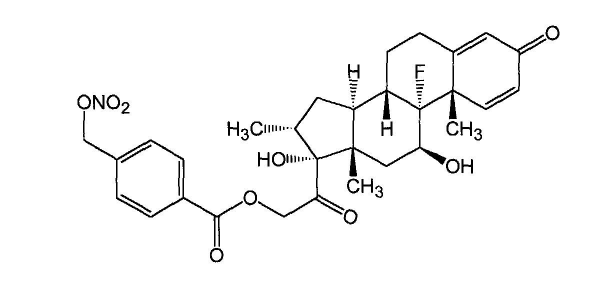

### (11β,16α)-9-fluoro-11,17-dihydroxy-21-(p-nitroxymethylbenzoyloxy)-pregna-1,4-diene-3,20-dione (ECXCa)

The compound is synthetized according to the synthesis described in example 8, using as reagents (11β,16α)-9-fluoro-11,17,21 -trihydroxy-pregna-1,4-diene-3,20-dione (dexamethasone) (ECXC) (Sigma-Aldrich code D-9184) and paranitroxymethylbenzoic acid (ZI) synthetized according to example 1.
Yield 73%.MS (El, 70eV): m/z 572.2 (M⁺H)

| Elementary analysis: | | | |
|---|---|---|---|
| Calculated | C:63.20% | H:5.95% | N:2.37% |
| Found | C:63.04% | H:6.00% | N:2.45% |

### EXAMPLE 11

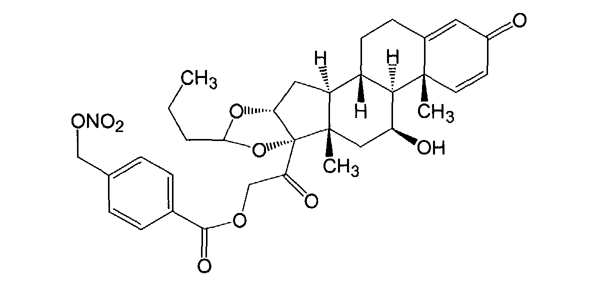

### (11β,16α)-9-fluoro-16,17-[Butylidenebis-(oxy)]-11-hydroxy-21-(p-nitroxymethylbenzoyloxy)-pregna-1,4-diene-3,20-dione (JIIc)

The compound is synthetized according to the synthesis described in example 8, using as reagents (11β,16α)-9-fluoro-16,17-[butylidenebis-(oxy)]-11,21-dihydroxy-pregna-1,4-diene-3,20-dione (budesonide) (JII) (Sigma-Aldrich code B-7777) and paranitroxymethylbenzoic acid (ZI) synthetized according to example 1.
Yield 68%. MS (El, 70eV): m/z 610.2 (M⁺H)

| Elementary analysis: | | | |
|---|---|---|---|
| Calculated | C:65.01% | H:6.45% | N:2.30% |
| Found | C:65.09% | H:6.37% | N:2.41% |

### EXAMPLE 12

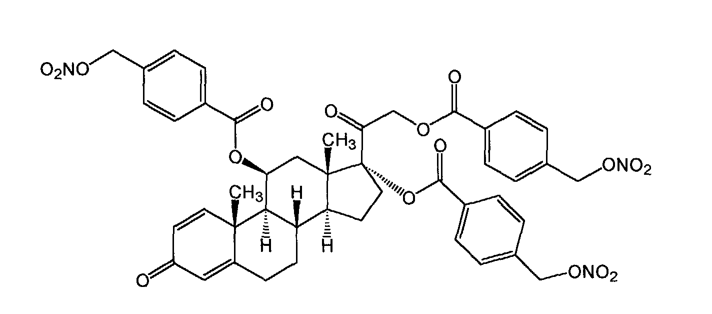

### (11β)-11,17,21-tris-(p-nitroxymethylbenzoyloxy)-pregna-1,4-diene-3,20-dione (ECVIIb)

The compound is synthetized according to the synthesis described in example 8, using as reagents (11β)-11,17,21-trihydroxy-pregna-1,4-diene-3,20-dione (ECVII) (prednisolone) (Sigma-Aldrich code P6004) and 3 equivalents of paranitroxymethylbenzoic acid (ZI) synthetized according to example 1.
Yield 72%. MS (El, 70eV): m/z 898.2 (M⁺H)

| Elementary analysis: | | | |
|---|---|---|---|
| Calculated | C:60.20% | H:4.83% | N:4.68% |
| Found | C:60.31% | H:4.69% | N:4.63% |

### EXAMPLE 13

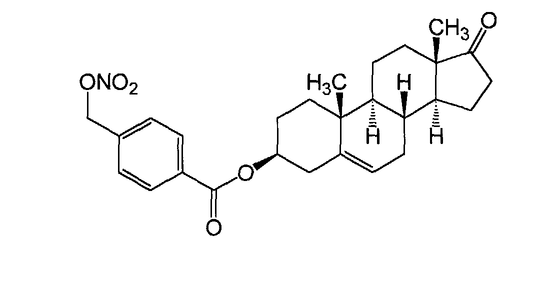

### (3β)-3-(p-nitroxvmethvlbenzovloxy)-androst-5-en-17-one (ECLXIIb)

The compound is synthetized according to the synthesis described in example 2, using as reagents (3β)-3-(p-nitroxymethylbenzoyloxy)-androst-5-en-17-one (prasterone) (ECLXII) (Sigma-Aldrich codeD-4000) and paranitroxymethylbenzoic acid (ZI) synthetized according to example 1.
Yield 79%.MS (El, 70eV): m/z 468.2 (M⁺H)

| Elementary analysis: | | | |
|---|---|---|---|
| Calculated | C:69.36% | H:7.11% | N:3.00% |
| Found | C:69.51% | H:7.09% | N:3.04% |

### EXAMPLE 14

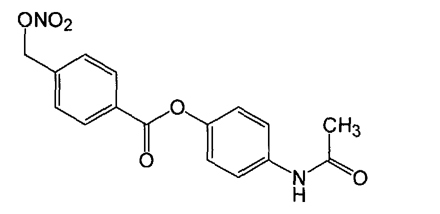

### N-[4-(p-nitroxymethylbenzoyloxy)phenyl]-acetamide (ECVIa)

The compound is synthetized according to the synthesis described in example 2, using as reagents N-(4-hydroxyphenyl)-acetamide (acetaminophen) (ECVI) (Sigma-Aldrich code 430A) and paranitroxymethylbenzoic acid (ZI) synthetized according to example 1.
Yield 82%.MS (El, 70eV): m/z 331.1 (M⁺H)

| Elementary analysis: | | | |
|---|---|---|---|
| Calculated | C:58.18% | H:4.27% | N:8.48% |
| Found | C:58.03% | H:4.22% | N:8.53% |

### EXAMPLE 15

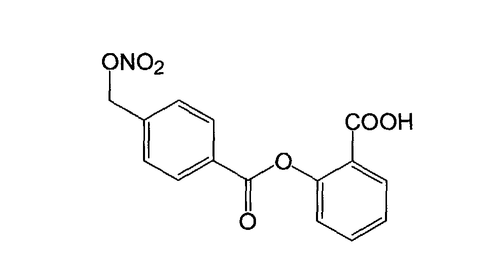

### (p-nitroxymethylbenzoyl)salicylic acid (ECXCIa)

The compound is synthetized according to the synthesis described in example 2, using as reagents salicylic acid (ECXCI) (Sigma-Aldrich code S-7401) and paranitroxymethylbenzoic acid (ZI) synthetized according to example 1.
Yield 81%.MS (El, 70eV): m/z 318.05 (M⁺H)

| Elementary analysis: | | | |
|---|---|---|---|
| Calculated | C:56.79% | H:3.49% | N:4.42% |
| Found | C:56.71% | H:3.45% | N:4.47% |

### EXAMPLE 16

### 2',3',4'-triacetvl-5'-(p-nitroxymethylbenzoyl)-riboflavin (CNbla)

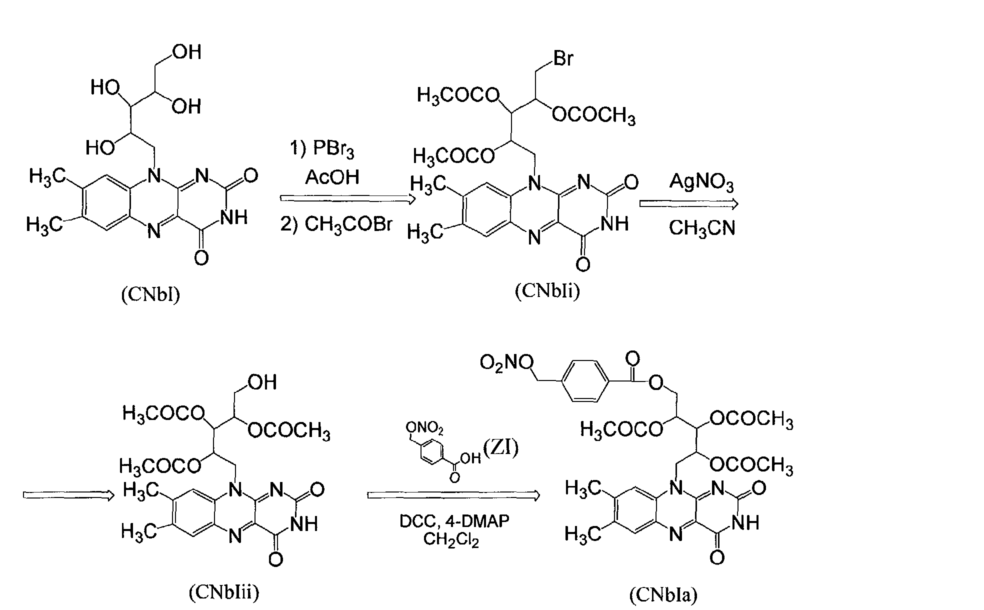

17.53 ml of a solution 1M of PBr₃ in CH₂Cl₂ (17.53 mmoles) were added to a sospension of riboflavin (CNbI) (Sigma-Aldrich code 47861) (3 g, 7.97 mmoles) in 114 ml of AcOH, kept at room temperature, under N₂ and in the dark. After 1 hour, when all was in solution, 5.3 ml of CH₃COBr (71.73 mmoles) were added. After 2 hours 400 ml of CHCl₃ were added and the solution so obtained was poured on 400 ml of H₂O. The organic phase was separated and the solvent was removed under reduced pressure. The crude product so obtained was purified by crystallization from methanol obtaining the desired product. The product was then further purified by flash cromatography, packing the column with methylene chloride and eluting with a mixture CH₂Cl₂/MeOH 99:1.

1.111 g of (CNbli) (1.96 mmoles) are so obtained
Yield: 24.5 %
R_{*f*}(CH₂Cl₂/MeOH 9:1):0.78
¹H NMR (CDCl₃) δ 8.44 (1H, s), 8.02 (1H, s), 7.58 (1H, s), 5.67 (1H, m), 5.47 (1H, dd, J = 2.75 and 6.87 Hz), 5.39 (1H, ddd, J = 3.67, 5.5 and 6.87), 5.3-4.6 (2H, broad), 3.74 (1H, dd, J = 3.67 and 11.46 Hz), 3.61 (1H, dd, J = 5.55 and 11.46 Hz), 2.58 (3H, s), 2.45 (3H, s), 2.33 (3H, s), 2.20 (3H, s), 1.78 (3H, s).
¹³C NMR (CDCl₃) δ 170.1, 169.8, 159.2, 154.5, 150.6, 148.1, 136.9, 135.9, 134.5, 132.9, 131.0, 155.6, 71.8, 69.6, 45.2, 30.7, 21.3, 21.0, 20.8, 20.3, 19.4.
MS (El, 70eV) m/Z (%) 565 (M⁺+1, 40), 242 (55), 43(100).
[α]²⁵_{D} = +131.5 (*c* 0.5, CHCl₃)

1 g of AgNO₃ was added to suspension of 1.111 g of (CNbli) (1.96 mmoles) in 10 ml of acetonitrile. The reaction was left at reflux for 24 hours. The reaction was left at room temperature, CHCl₃ was added and was centrifuged to remove AgBr so produced.

The solvent was removed at reduced pressure. The crude product was purified by flash cromatography, packing the column with methylene chloride and eluting with a mixture CH₂Cl₂/MeOH 99:1.

146 mg of (CNblii) (0.29 mmoles) were so obtained as a mixture of 2 regioisomers in a 1:2 ratio.
Yield: 26.2 %.
R_{*f*}(CH₂Cl₂/MeOH 9:1):0.51
¹H NMR (CDCl₃) δ 8.85 (2H, s), 8.07 (1H, s), 8.06 (1H, s), 7.76 (2H, s), 5.69 (1H, m), 5.49 (1H, m), 5.34 (1H, m), 5.27 (2H, d, J = 8.54 Hz), 5.1-4,7 (4H, broad), 4.51 (1H, m), 4.46 (1H, dd, J = 3.05 e 12.21 Hz), 4.39 (1H, dd, J = 3.66 e 7.94 Hz), 4.21 (1H, dd, J = 6.71 e 12.21 Hz), 4.14 (1H, m), 2.59 (6H, s), 2.45 (6H, s), 2.21 (3H, s), 2.1 (3H, s), 2.02 (3H, s), 2.0 (3H, s), 1.8 (3H, s), 1.9 (3H, s).
MS (El, 70eV) m/Z (%) 503 (M⁺+1, 67), 61 (100).

59.8 mg of N,N'-dicyclohexylcarbodiimide (DCC) (0.29 mmoles), 63 mg (0.32 mmoles) of paranitroxymethylbenzoic acid (ZI) synthetized according to example 1 and a catalytic quantity of 4-DMAP were added to a solution of (CNblii) (146 mg, 0.29 mmoles) in CH₂Cl₂ at room temperature and under N₂. After 3 hours the acid was disappeared. The crude product was purified by flash cromatography, packing the column with methylene chloride and eluting with a mixture CH₂Cl₂/MeOH 99:1.

146 mg of the desired product (CNbla) (0.21 mmoles) were so obtained as a mixture of 3 regioisomers.
Yield: 72.3 %.
R_{*f*}(CH₂Cl₂/MeOH 9:1):0.77.
¹H NMR (CDCl₃) δ 9.65 (1H, s), 9.50 (1H, s), 9.45 (1H, s), 8.24-8.21 (4H, m, aromatics), 7.95-7.85 (2H, m, aromatics), 7.71 (1H, m, aromatics), 7.63-7.45(9H, m, aromatics), 7.33 (2H, m, aromatics), 6.01-5.37 (15H, assignable to acetyls tertiary H and to benzylic H), 5.27-4.70 (6H, assignable to secondary H on N), 4.58-4.08 (6H, assignable to secondary H on acetyls), 2.52-1.65 (45H, assignable to acetyls CH₃ and to aromatic ring CH₃).
MS (El, 70eV) m/Z (%) 682 (M⁺H, 66), 663 (78), 279 (100).

### EXAMPLE 17

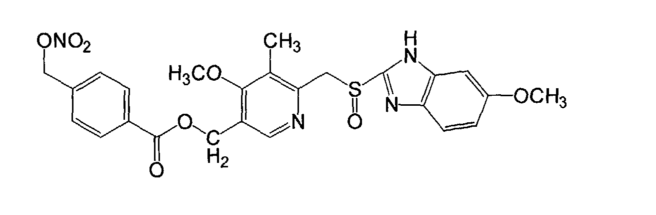

### 5-methoxy-2-[[[4-methoxy-3-methyl-5-(p-nitroxymethylbenzoyloxymethyl)-2-pyridinyl]methyl]sulfinyl]-1H-benzimidazole (RXCa)

The compound is synthetized according to the synthesis described in example 2, using as reagents 5-α-hydroxyomeprazole (RXC) synthetized according to WO99/56746 and paranitroxymethylbenzoic acid (ZI) synthetized according to example 1.
Yield 70%. MS (El, 70eV): m/z 541.1 (M⁺H)

| Elementary analysis: | | | |
|---|---|---|---|
| Calculated | C:55.55% | H:4.48% | N:10.36% |
| Found | C:55.39% | H:4.41% | N:10.31% |

### EXAMPLE 18

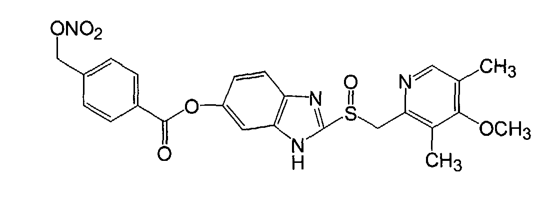

### 5-(p-nitroxymethylbenzoyloxy)-2-[[(4-methoxy-3,5-dimethyl-2-pyridinyl)methyl)sulfinyl]-1H-benzimidazole (RXIIa)

The compound is synthetized according to the synthesis described in example 2, using as reagents 5-O-desmethylomeprazole (RXII) and paranitroxymethylbenzoic acid (ZI) synthetized according to example 1.
Yield 75%. MS (El, 70eV): m/z 511.1 (M⁺H)

| Elementary analysis: | | | |
|---|---|---|---|
| Calculated | C:56.46% | H:4.34% | N:10.97% |
| Found | C:56.39% | H:4.32% | N:10.77% |

### EXAMPLE 19

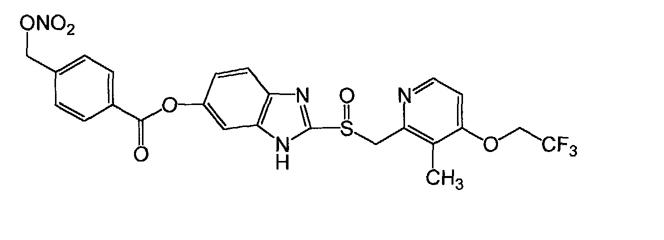

### 5-(p-nitroxymethylbenzoyloxy)-2-[[[3-methyl-4-(2,2,2-trifluoroethoxy)-2-pyridinyl]methyl]sulfinyl]-1H-benzimidazole (RCa)

The compound was synthetized according to the synthesis described in example 2, using as reagents 5-hydroxylansoprazole (RC) synthetized according to WO00/04904 and paranitroxymethylbenzoic acid (ZI) synthetized according to example 1.
Yield 78%. MS (El, 70eV): m/z 565.1 (M⁺H)

| Elementary analysis: | | | |
|---|---|---|---|
| Calculated | C:51.06% | H:3.39% | N:9.93% |
| Found | C:50.99% | H:3.35% | N:9.87% |

### EXAMPLE 20

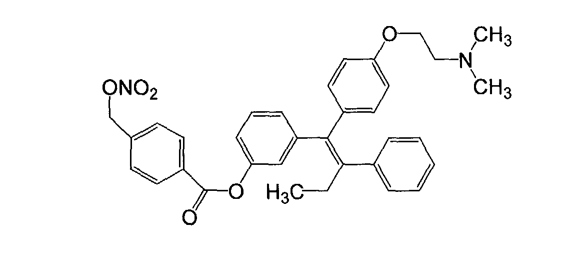

### (E)-3-[1-[4-[2-(dimethylamino)ethoxy]phenyl]-2-phenyl-1-butenyl]phenol p-nitroxymethylbenzoic acid ester (SVIc)

The compound was synthetized according to the synthesis described in example 2, using as reagents droloxifene (SVI) synthetized according to US5047431 and paranitroxymethylbenzoic acid (ZI) synthetized according to example 1.
Yield 80%. MS (El, 70eV): m/z 567.2 (M⁺H)

| Elementary analysis: | | | |
|---|---|---|---|
| Calculated | C:72.07% | H:6.05% | N:4.94% |
| Found | C:72.01% | H:5.98% | N:4.89% |

### EXAMPLE 21

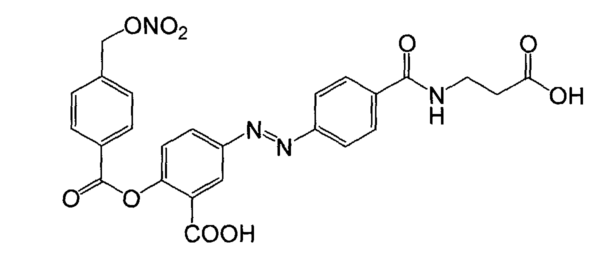

### 5-[4-(2-Carboxy-ethylcarbamoyl)-phenylazol-2-(4-nitroxymethylbenzoyloxy)-benzoic acid (ECXVIIIa)

The compound was synthetized according to the synthesis described in example 2, using as reagents balsalazide (ECXVIII) and paranitroxymethylbenzoic acid (ZI) synthetized according to example 1.
Yield 67%. MS (El, 70eV): m/z 537.1 (M⁺H)

| Elementary analysis: | | | |
|---|---|---|---|
| Calculated | C:55.97% | H:3.76% | N:10.44% |
| Found | C:55.91% | H:3.74% | N:10.47% |

### EXAMPLE 22

### 2-Acetylsulfanylmethyl-4-oxo-4-p-tolyl-butyric acid 4-nitroxy-butyl ester (EXXXIVa)

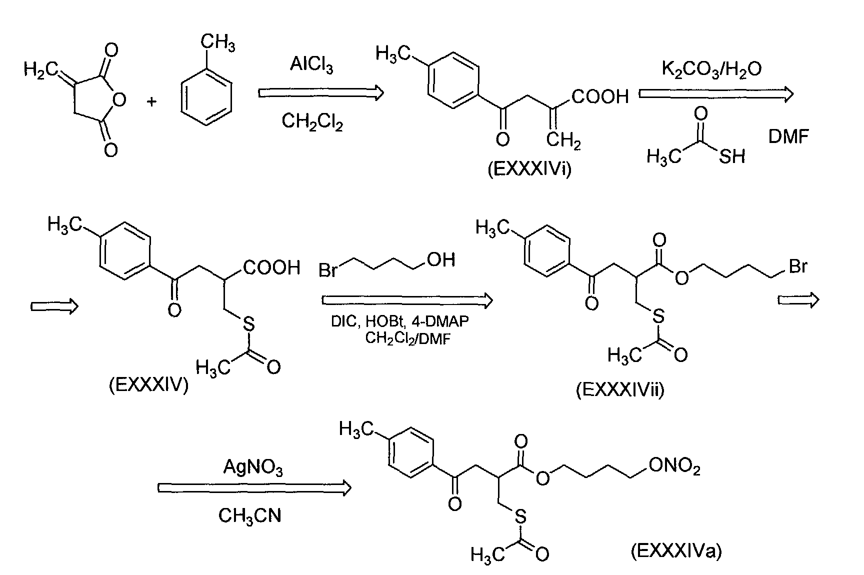

1.9 mL of toluene (17.8 mmoles) were added to a suspension of itaconic anhydride 95% (17.8 mmoles, 2.1 g) in 70 mL of CH₂Cl₂. In the span of 30 minutes 4.7 g of AlCl₃ were added. The reaction was stirred for other 4 hours. The solvent was removed at reduced pressure and water and ice were added to the crude product. 3 mL of concentrated HCI were added and the product was extracted with ethyl acetate.

The product was anhydrified with Na₂SO₄, was filtered and deprived of the solvent at reduced pressure. 3.4 g of the product (EXXXIVi) were obtained. Yield 93.6%.
¹H NMR (400 MHz, CDCl₃) : δ 7.87 (d, 2H, J=8.3 Hz), 7.25 (d, 2H, J=8.3 Hz), 6.50 (s, 1H), 5.77 (s, 1H), 3.95 (s, 2H), 2.41 (s, 3H).

K₂CO₃ (50 mg, 0.15 mmoles) in H₂O (200 µL) was added drop by drop to a solution of (EXXXIVi) (500 mg, 2.4 mmoles) and thiolacetic acid (214 µL, 2.88 mmoles) in DMF (5 mL) kept at 30 °C. The reaction was stirred for 2 hours. Water was added to the reaction mixture and then HCL 10% was added to reach pH 5. The product was extracted with ethyl acetate, was washed with water and brine, was anhydrified with Na₂SO₄ and the solvent was removed at reduced pressure. The product was purified by crystallization from Et₂O/hexane. 1.1 g of the desired product (EXXXIV) (3.8 mmoles) were obtained.
Yield: 77.5%.
¹H NMR (500 MHz, CDCl₃): δ: 7.84 (d, 1H, J=8.6 Hz), 7.25 (d, 1H, J=8.6 Hz), 3.50-3.16 (m, 5H), 2.41 (s, 3H), 2.32 (s, 3H).

4-Br-butanol (82 µL, 0.94 mmoles), HOBt (85 mg, 0.63 mmoles), DIC (100 µL, 0.63 mmoles) and a catalytic quantity of 4-DMAP were added to a solution of (EXXXIV) (176 mg, 0.63 mmoles) in 5 mL of CH₂Cl₂/DMF 9:1 kept under N₂. The reaction was stirred for 6 hours, the solvent was removed at reduced pressure, CHCl₃ was added and the reaction mixture was washed 3 times with brine. The mixture was anhydrified with Na₂SO₄ and the solvent was removed at reduced pressure. The product was purified by flash cromatography (eluting: hexane/AcOEt 85:15). 76 mg of the desired product (EXXXIVii) (0.19 mmoles) were obtained.
Yield: 30 %. R_{*f*} (hexane/AcOEt 85:15):0.22.
¹H NMR (500 MHz, CDCl₃): δ: 7.84 (d, 2H, J=8.3), 7.25 (d, 2H, J=8.3), 4.25 (t, 1H, J=6.3), 3.49-3.41 (m, 3H), 3.31-3.12 (m, 4H), 2.41 (s, 3H), 2.34 (s,3H), 2.18 (m, 2H).

AgNO₃ (97 mg, 0.57 mmoles) was added to a solution of (EXXXIVi) (76 mg, 0.19 mmoles) in CH₃CN (5 mL). The reaction was left at reflux for 6 hours. The solution was filtered to remove AgBr so produced and the solvent was removed at reduced pressure. CHCl₃ was added and AgNOₛ was filtered. The solvent was removed at reduced pressure and the product was purified by flash cromatography (eluting : AcOEt/hexane 2:8).

50 mg of the desired product (EXXXIVa) (0.13 mmoles) were so obtained.
Yield: 69%. R_{*f*} (hexane/AcOEt 8:2):0.20
¹H NMR (500 MHz, CDCl₃): δ: 7.83 (d, 2H, J=8.2), 7.25 (d, 2H, J=8.2), 4.54 (m, 2H), 4.21 (m, 2H), 3.48-3.43 (m, 1H), 3.29-3.15 (m, 4H), 2.40 (s, 3H), 2.32 (s, 3H), 2.05 (m, 2H).

### EXAMPLE 23

### 4-Nitroxy-butyric acid 5,7-bis-benzyloxy-2-(3,4-bis-benzyloxy-phenyl)-4-oxo-4H-chromen-3-yl ester (CNbXVa)

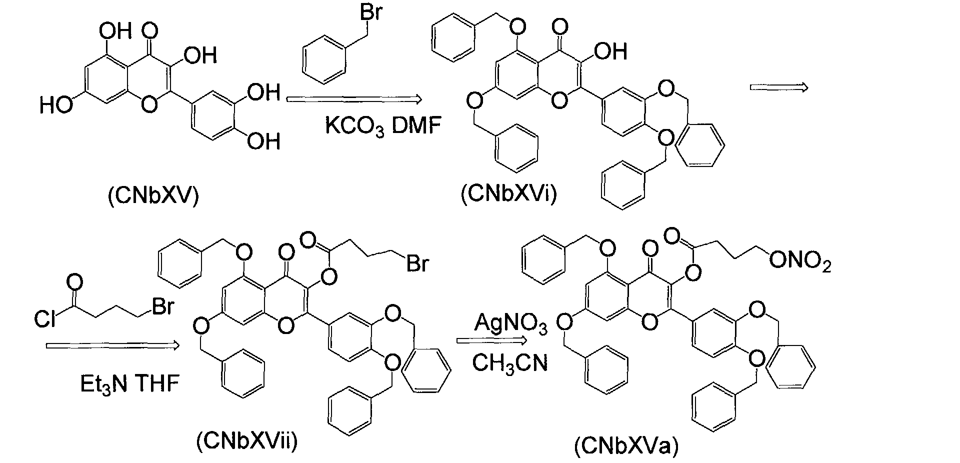

1.12 g of dihydrated quercetin (CNbXV) (3.3 mmoles) in dry DMF (10 mL) were added drop by drop to a suspension of K₂CO₃ (1.96 g, 14.2 mmoles) in dry DMF (3 mL) kept under N₂. The reaction was stirred for 1 hour. Benzyl bromide (1.8 mL, 14.8 mmoles) was added drop by drop and the reaction was stirred all the night. DMF was removed at reduced pressure and ethyl acetate and water were added to the crude product. The organic phase was separated and washed 2 times with brine. The mixture was anhydrified with Na₂SO₄, was filtered and the solvent was removed at reduced pressure. The crude product so obtained was purified by flash cromatography eluting with a mixture CH₂Cl₂/hexane 9:1.

1.484 g of the desired product (CNbXVi) (2.24 mmoles) were obtained.
Yield: 67.9% R_{*f*} (AcOEt/hexane 2:8): 0.38
¹H NMR (CDCl₃) δ 12.68 (1H, s), 7.71 (1H, d, J=1.97 Hz), 7.56 (1H, dd, J=8.86 e 1.97 Hz), 7.50-7.21 (20H, m), 6.97 (1H, d, J=8.86 Hz), 6.46 (1H, d, J=2.45), 6.44 (1H, d, J=8.86 Hz), 5.24 (2H, s), 5.12 (2H, s), 5.05 (2H, s), 4.99 (2H, s).

4-Bromo-butyryl chloride (768 µL, 6.3 mmoles) and Et₃N (878 µL, 6.3 mmoles) were added to a solution of CNbXVi (1.4 g, 2.1 mmoles) in 30 ml of THF: a complex chloride-Et₃N, insoluble in THF, was formed. The reaction was stirred 5 hours at room temperature. The complex was filtered and THF was removed at reduced pressure. The product was obtained by precipitation from THF-hexane. 1.372 g of (CNbXVii) were obtained (1.69 mmoles).
Yield: 80 %. R_{*f*} (AcOEt/hexane 2:8): 0.32
¹H NMR (CDCl₃) δ 7.66 (d, 1H, J=1.88 Hz), 7.49 (dd, 1H, J=8.45 e 1.88 Hz), 7.44-7.14 (m, 20H), 6.94 (d, 1H, J=8.45 Hz), 6.81 (d, 1H, J=1.87 Hz), 6.81 (d, 1H, J=1.87 Hz), 5.22 (s, 2H), 5.13 (s, 2H), 4.98 (s, 2H), 4.96 (s, 2H), 3.62 (t, 2H, J=6.57 Hz), 2.96 (t, 2H, J=7.51 Hz), 2.40 (quintet, J=7.51 e 6.57).

AgNO₃ (862 mg, 5.07 mmoles) was added to a suspension of (CNbXVii)(1.372 g, 1.69 mmoles) in 50 mL of CH₃CN. The reaction was left at reflux for 5 hours. The solution was left to clear to remove AgBr. The solvent was removed at reduced pressure. The product was obtained by precipitation from THF-hexane: 1,1 g (1.38 mmol) of (CNbXVa) were obtained.
Yield: 82%. R_{*f*} (AcOEt/hexane 3:7): 0.64
¹H NMR (CDCl₃) δ 7.65 (d, 1H, J=1.87 Hz), 7.49 (dd, 1H, J=8.45 and 1.87 Hz), 7.46-7.15 (m, 20H), 6.94 (d, 1H, J=8.45 Hz), 6.83 (d, 1H, J=2.34 Hz), 6.67 (d, 1H, J=2.34 Hz), 5.22 (s, 2H), 5.14 (s, 2H), 4.98 (s, 2H), 4.95 (s, 2H), 4.68 (t, 2H, J=6.58 Hz), 2.91 (t, 2H, J=7.51 Hz), 2.28 (quintet, J=7.51 and 6.58).

### EXAMPLE 24

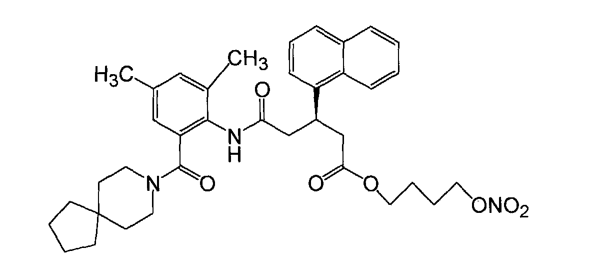

### beta-[2-([2-(8-azaspiro[4.5]dec-8-ylcarbonyl)-4,6-dimethylphenyl]amino)-2-oxoethyl]-(R)-1-naphthalene-propanoic acid-(4-nitroxybutyl)-ester (RXXXIIIa)

This compound was synthetized according to the synthesis described in the third and the fourth step of example 1 (from (EXXXIV) to (EXXXIVa)), using itriglumide (RXXXIII) (sigma-aldrich code 19778) as reagent.

### EXAMPLE 25

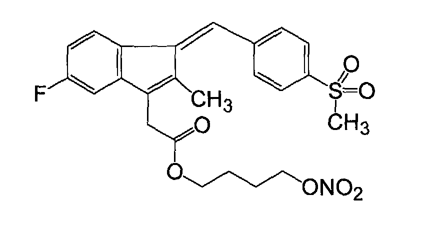

### (Z)-5-fluoro-2-methyl-1-[[4-(methyl-sulfonyl)phenyl]methylene]-1H-indene-3-acetic acid--(4-nitroxybutyl)-ester (CNdld)

This compound was synthetized according to the synthesis described in the third and the fourth step of example 1 (from (EXXXIV) to (EXXXIVa)), using exisulind (CNdI) as reagent.

### EXAMPLE 26

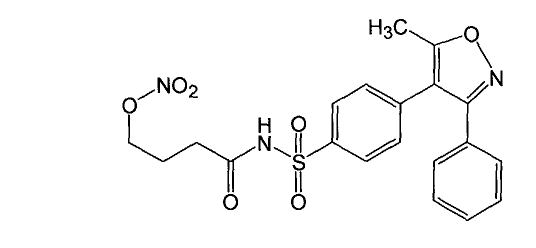

### N-[4-(5-metil-3-phenylisoxazol-4-yl)phenylsulfonyl]-p-nitroxymethylbutanamide (EVd)

This compound was synthetized according to the synthesis described in the third and the fourth step of example 1 (from (EXXXIV) to (EXXXIVa)), using 4-(5-methyl-3-phenylisoxazol-4-yl)benzensulfonamide (valdecoxib, (EV), synthetized as described in EP-0809636) as reagent.
MS (El, 70eV) m/Z 446.1 (M⁺H).

| Elementary analysis: | | | |
|---|---|---|---|
| Calculated | C:53.93% | H:4.30% | N:9.43% |
| Found | C:53.99% | H:4.27% | N:9.41% |

### EXAMPLE 27

### m-nitroxymethylphenol (ZI)

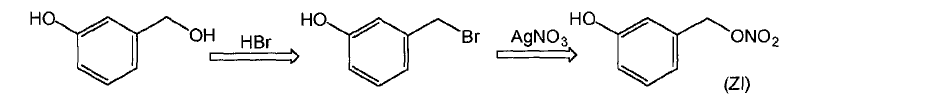

3-hydroxybenzylic alcohol (5 g, 40.30 mmoles) in methylene chloride (16 mL) was added to hydrobromic acid (48%) (25 mL). The reaction was left at room temperature for 4 hours. The reaction mixture was washed with aqueous sodium bicarbonate and was anhydrified with anhydrous sodium sulphate. Methylene chloride was removed at reduced pressure. The oily residue was dissolved in anydrous acetonitrile (25 mL) and a solution of silver nitrate (6.85 g, 40.32 mmoles) dissolved in anhydrous acetonitrile (10 mL) was added drop by drop and in the dark.

After 12 hours the precipitate (silver bromide) was filtered and the solvent was removed at reduced pressure. The so obtained oily residue was dissolved in toluene (22 mL) and the solution was purified by silica gel cromatography (toluol/ethyl acetate 7/3 v/v). The eluate was dried at reduced pressure and 2.65 g of the desired product (yield 39%) were so obtained.

### EXAMPLE 28

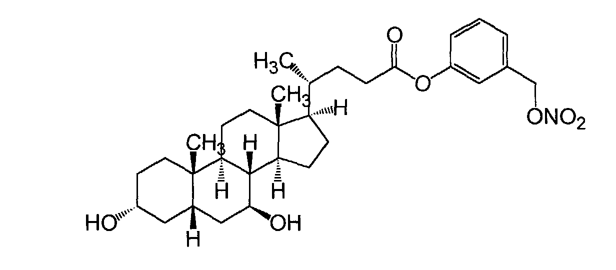

### (3α,5β,7β)-3,7-dihydroxycholan-24-oic acid (m-nitroxymethylphenol)ester (ELXVc)

M-nitroxymethylphenol (ZI) (7 mmoles, 1.18 g) prepared according to example 27 was added under stirring to a solution of ursodiol (Sigma-Aldrich code U5127) (2 g, 5.09 mmoles) dissolved in chloroform (18 mL) and dimethylacetamide (17 mL).

N,N'-dicyclohexylcarbodiimide (1.44 g, 7 mmol) and 4-dimethylaminopyridine (70 mg) were added to the solution cooled at a 0°C and kept under stirring. After 1 hour the mixture was heated to room temperature. After 24 hours the precipitate was filterd and the solvent was removed at reduced pressure. The residue was treated with ethyl acetate (100 mL) and washed with water (3 X 70 mL).

The organic phase was anhydrified with sodium sulphate and the solvent was removed at reduced pressure.

The residue was purified by silica gel cromatography eluting with n-hexane/ethyl acetate 1/9. 1.24 g of the desired product were obtained.
Yield 45%. MS (El, 70eV): m/z 544.3 (M⁺H)

| Elementary analysis: | | | |
|---|---|---|---|
| Calculated | C:68.48% | H:8.34% | N:2.58% |
| Found | C:68.41% | H:8.32% | N:2.60% |

### EXAMPLE 29

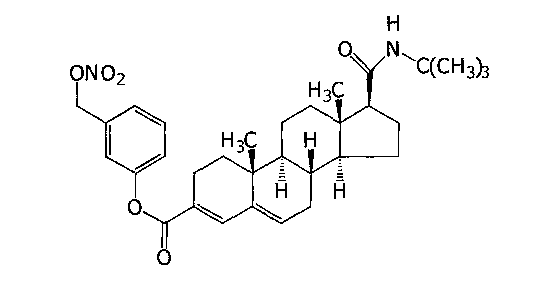

### (17β)-17[[(1,1-dimethylethyl)amino]carbonyl]androsta-3,5-diene-carboxylic acid (m-nitroxymethylphenol)ester (Uld)

This compound was synthetized according to the synthesis described in example 28 using, as reagents, epristeride (UI) (synthetized according to EP289327) and m-nitroxymethylphenol (ZI) synthetized according to example 27.
Yield 47%. MS (El, 70eV): m/z 551.3 (M⁺H)

| Elementary analysis: | | | |
|---|---|---|---|
| Calculated | C:69.79% | H:7.69% | N:5.09% |
| Found | C:69.84% | H:7.68% | N:5.11% |

### EXAMPLE 30

### glucosamine nitrate salt (EXXXa)

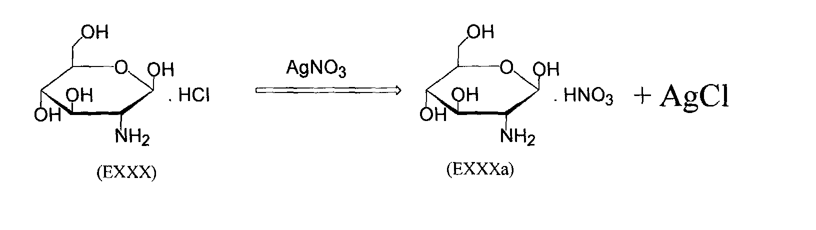

AgNO₃ (78 mg, 0.46 mmoles) dissolved in 300 µl of H₂O was added to a solution of di D-Glucosamine hydrochloride (Sigma-Aldrich code G2206) (100 mg, 0.46 mmoles) in H₂O (1 mL) under stirring and at room temperature; a precipitate was formed (AgCI). The reaction was stirred for 10 minutes, was cooled at 0°C and the solid was removed by centrifugence. The solvent was removed and the product was solid/sticky. The solid was washed many times with EtOH to obtain a powdery solid. mp 90-93°C

### EXAMPLE 31

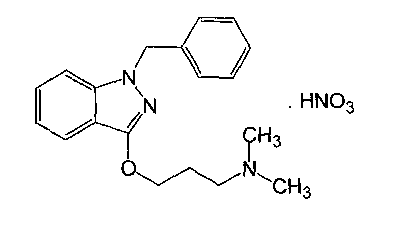

### 1-benzyl-3-[3-(dimethylamino)propoxy]-1H-indazole nitric salt (ELXa)

This compound was synthetized according to the synthesis described in example 30 using, as reagent, benzydamine hydrochloride (ELXa) (Sigma-Aldrich code B5524). Yield 95%.

| Elementary analysis: | | | |
|---|---|---|---|
| Calculated | C:61.28% | H:6.50% | N:15.04% |
| Found | C:61.23% | H:6.69% | N:15.12% |

### EXAMPLE 32

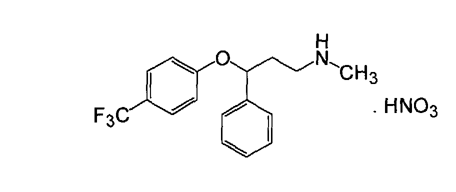

### N-methyl-3-(p-trifluoromethylphenoxy)-3-phenylpropylamine nitric salt (UCXXXIa)

This compound was synthetized according to the synthesis described in example 30 using, as reagent, fluoxetine hydrochloride (UCXXXI).
Yield 93%.

| Elementary analysis: | | | |
|---|---|---|---|
| Calculated | C:54.84% | H:5.14% | N:7.52% |
| Found | C:54.92% | H:5.37% | N:7.62% |

### EXAMPLE 33

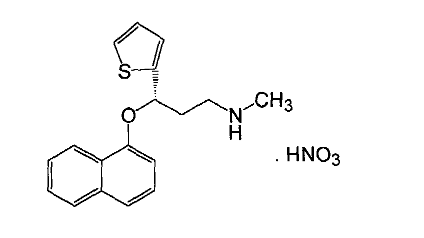

### (+)-N-methyl-3-(1-naphthalenyloxy)-3-2-thienylpropylamine nitric salt (UXLIa)

This compound was synthetized according to the synthesis described in example 30 using, as reagent, duloxetine hydrochloride (UXLIa).
Yield 89 %.

| Elementary analysis: | | | |
|---|---|---|---|
| Calculated | C:59.98% | H:5.59% | N:7.77% |
| Found | C:60.07% | H:5.56% | N:7.71% |

### EXAMPLE 34

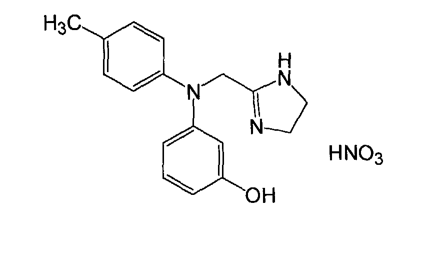

### 2-(m-hydroxy-N-tolylanilinomethyl)-2-imidazoline nitric salt (UVIIIa)

This compound was synthetized according to the synthesis described in example 30 using, as reagent, phentolamine hydrochloride (UVIII) (Sigma-Aldrich code P7547). Yield 92%

| Elementary analysis: | | | |
|---|---|---|---|
| Calculated | C:72.57% | H:6.81% | N:14.94% |
| Found | C:72.51% | H:6.83% | N:14.87% |

### PHARMACOLOGICAL EXAMPLES

### EXAMPLE 1

### Absorption of the compounds of the invention by passive diffusion through biological membranes (gastrointestinal and cutaneous absorption) according to the model described by the first Fick law, in comparison with that of all known nitrate vasodilators

A model describing the absorption of molecules by passive diffusion through biological membranes (gastrointestinal and cutaneous absorption) is described by the first Fick law (see Amorosa "Principi di tecnologia farmaceutica" pag 491).

We have calculated DA (mg/cm²) that is the absorbed dose per unit area during a period of 15 minutes according to the model described by the first Fick law, as described in the document EPA/600/8-91-011B, January 1992, Interim Report. U.S. Environmental Protection Agency, Exposure Assessment Group, Office of Health and Environmental Assessment, Washington, DC.

Since DA=Kp·Cw·t, we have before calculated LogP (logarithm of the distribution coefficient n-ottanol/water) as described in Meylan et al J. Pharm. Sci. 1995; 84; pag. 83-92. Then, with it, we have calculated Cw that is water solubility (mg/cm³) (according to Meylan et al Environ. Toxicol. Chem. 1996; 15; pag. 100-106). Finally we have calculated the permeability coefficient of the molecule from water (Kp, (cm/hr)) (as described in EPA/600/8-91-011B, January 1992, Interim Report. U.S. Environmental Protection Agency, Exposure Assessment Group, Office of Health and Environmental Assessment, Washington, DC).

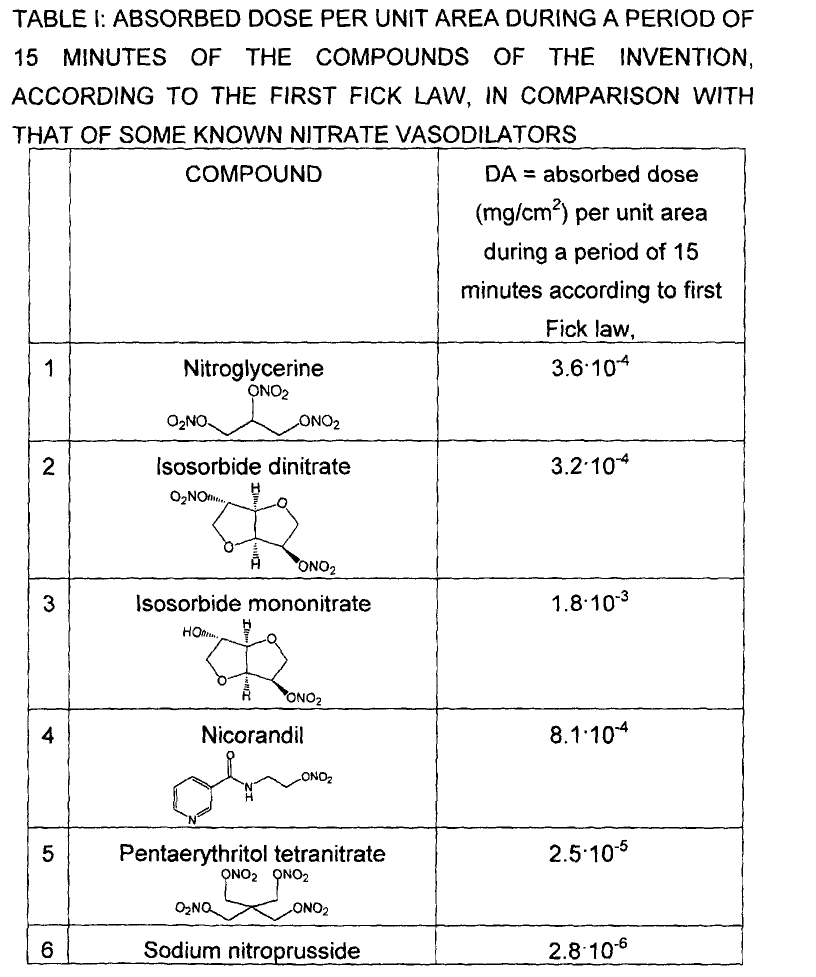

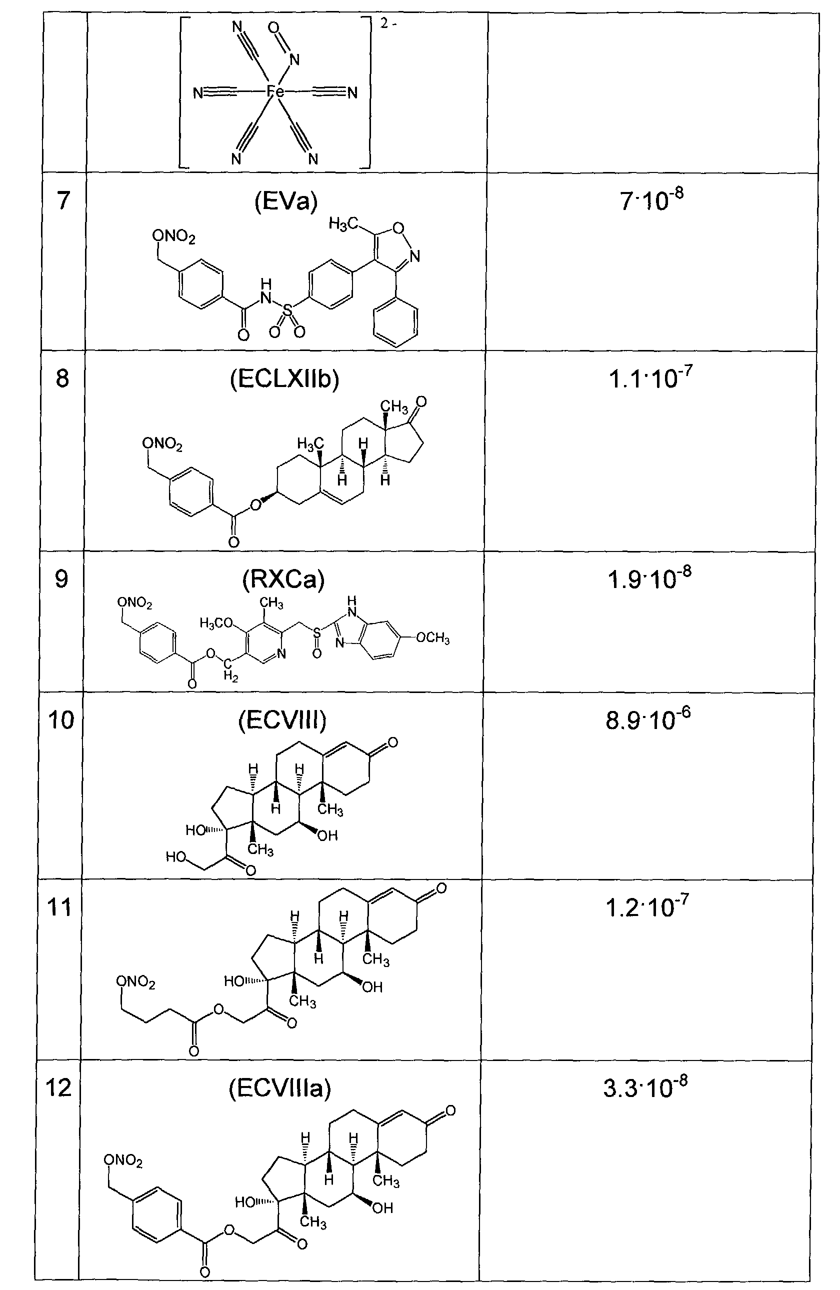

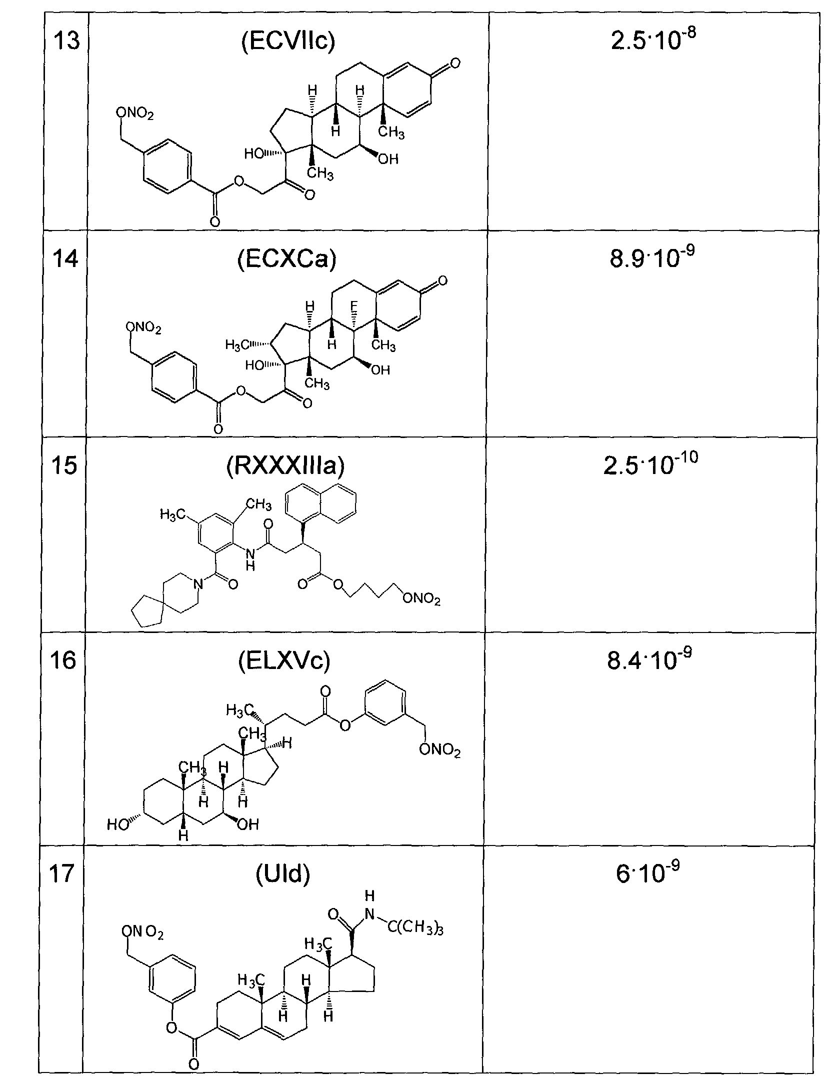

As shown by the results of table I, we have unexpectedly found that the compounds of the invention have an absorption from 10 to 1000 times slower than classic nitrate vasodilators (nitroglycerine, isosorbide dinitrate, isosorbide mononitrate, nicorandil, pentaerythritol tetranitrate).

This is an important therapeutic advantage because it is a controlled release of nitric oxide, that is nitrates are transformed metabolically in vivo into nitric oxide but, as said before, is essential that the rate of this release is not high to avoid hypotensive side effects and also potentially proinflammatory and proapoptotic side effects.

The compounds of the invention can so be adsorbed much more slowly than classic nitrate vasodilators and for this reason they are devoid of hypotensive side effects as proved by example 3.

### EXAMPLE 2

### Endogenous S-nitrosohemoglobin (SNO-Hb) levels in rat blood and after administration of N-[4-(5-p-tolyl-3-trifluoromethyl-1H-pyrazol-1-yl)phenylsulfonyl]-p-nitroxymethylbenzamide (Ela)

A highly sensitive photolysis-chemiluminescence methodology was used. Nitric oxide, released from Hb-SNO through photolysis, was detected in a chemiluminescence spectrometer by reaction with ozone (McMahon, T. J., and Stamler, J. S. (1999) Methods Enzymol. 301, 99-114).

Blood was obtained from the left ventricle (arterial) and jugular vein (venous) of anesthetized Sprague-Dawely rats: 12 were controls and 12 were treated with 100 mg/Kg of N-[4-(5-p-tolyl-3-trifluoromethyl-1H-pyrazol-1-yl)phenylsulfonyl]-p-nitroxymethylbenzamide (Ela). Red blood cells were isolated by centrifugation at 800 g, washed three times in phosphate buffered saline at 4 °C, lysed by the addition of 4-fold excess volume of deionized water containing 0.5 mM EDTA and desalted rapidly according to the method of Penefsky at 4 °C.

In 24 rats, Hb samples were divided in two aliquots which were then treated or not treated with 10-fold excess HgCl₂ which can remove the signal of Hb(Fell)NO.

N-[4-(5-p-tolyl-3-trifluoromethyl-1H-pyrazol-1-yl)phenylsulfonyl]-p-nitroxymethylbenzamide (Ela) can significantly increase S-nitrosohemoglobin (SNO-Hb) levels both in rat arterial and in rat venous blood.

**TABLE III:**

| Endogenous S-nitrosohemoglobin (SNO-Hb) levels in rat blood and after administration of N-[4-(5-p-tolyl-3-trifluoromethyl-1H -pyrazol-1-yl)phenylsulfonyl]-p-nitroxymethylbenzamide (Ela) | | |
|---|---|---|
| | Arterial SNOERR-Hb (nM) | Venous SNO-Hb (nM) |
| Endogenous levels | 311 +- 55 | 32 +- 14 |
| Levels after administration of (Ela) (100 mg/Kg i.p.) | | |

### EXAMPLE 3

### Arterial blood pressure effects of oral administration of sodium nitroprusside and (11β,16α)-9-fluoro-11,17-dihydroxy-21-(p-nitroxymethylbenzoyloxy)-pregna-1,4-diene-3,20-dione (ECXCa)

Sodium nitroprusside (0.5 mg/kg) in carboxymethylcellulose 0.5% or an equimolar dose (1.09 mg/Kg) of (11β,16α)-9-fluoro-11,17-dihydroxy-21-(p-nitroxymethylbenzoyloxy)-pregna-1,4-diene-3,20-dione (ECXCa) in carboxymethylcellulose 0.5% or the vehicle alone (control group) were administered per os to 15 male wistar rats divided into 3 groups and after anaesthesia.

Blood pressure was continuously recorded for 4 hours.

**TABLE II:**

| RAT ARTERIAL BLOOD PRESSURE EFFECTS OF ORAL ADMINISTRATION OF SODIUM NITROPRUSSIDE (0.5 MG/KG) AND (11B,16A)-9-FLUORO-11,17-DIHYDROXY-21-(P-NITROXYMETHYLBENZOYLOXY)-PREGNA-1,4-DIENE-3,20-DIONE (ECXCA) (1.09 MG/KG) | | |
|---|---|---|
| Control (%) | Sodium nitroprusside (%) | (ECXCa) (%) |
| 100 | 76 | 100 |

These results prove that (11β,16α)-9-fluoro-11,17-dihydroxy-21-(p-nitroxymethylbenzoyloxy)-pregna-1,4-diene-3,20-dione (ECXCa) does not modify systemic blood pressure while sodium nitroprusside can cause a marked hypotension.

## Claims

1. Compounds of general formula (I), their pharmaceutically acceptable salts, their in vivo hydrolyzable prodrugs, their tautomers and their diastereomers
F-(X)_{q} (I)
where:
- q is an integer from 1 to 5;
- F is a molecule or a drug for prevention and treatment of inflammatory, ischemic, degenerative and proliferative diseases of musculoskeletal, tegumental, respiratory, gastrointestinal, genito-urinary and central nervous systems;
- X is chosen among 3 groups of substituents, -M, -T and -V where:
- M is chosen among the following groups which can release nitric oxide in vivo: nitrate ester (Al) -ONO₂; nitrate salt (All); nitrite ester (AIII)
- ONO; thionitrite (AIV) -SNO; NONOate (AV) -NO(NO) 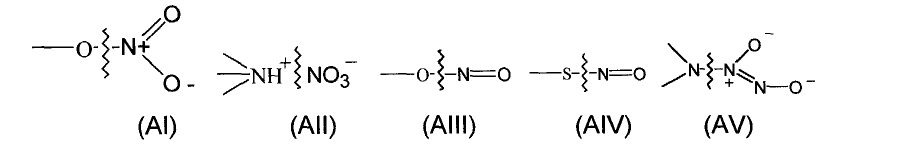
- the undulated line shows where and how is the bond which is necessary to link M (on the right of the line) and the drug F (on the left of the line);
- T is chosen among the following radicals, optionally salified:
- O-R₁-M; -O-R₁-O-R₁-M;-O-R₁-S-R₁-M;-O-R₁-NR₂-R₁-M;
- S-R₁-M; -S-R₁-O-R₁-M; -S-R₁-S-R₁-M; -S-R₁-NR₂-R₁-M;
- NR₂-R₁-M; -NR₂-R₁-O-R₁-M; -NR₂-R₁-S-R₁-M; -NR₂-R₁-NR₂-R₁-M;
- (CO)-R₁-M; -(CO)-R₁-O-R₁-M; -(CO)-R₁-S-R₁-M;-(CO)-R₁-NR₂-R₁-M;
- O-(CO)-R₁-M;
- R₁-O-R₁-M; -R₁-S-R₁-M; -R₁-NR₂-R₁-M;
- PO(O-)O-R₁-M;
where M is described as above;
- R₁- is a saturated or unsaturated, linear or branched alkylene, having from 1 to 21 carbon atoms or a saturated or unsaturated, optionally heterosubstituted or branched cycloalkylene, having from 3 to 7 carbon atoms or an optionally heterosubstituted arylene having from 3 to 7 carbon atoms;
- R₂ is H or a saturated or unsaturated, linear or branched alkyl, having from 1 to 21 carbon atoms or a saturated or unsaturated, optionally heterosubstituted or branched cycloalkyl, having from 3 to 7 carbon atoms or an optionally heterosubstituted aryl having from 3 to 7 carbon atoms;
- R₁- and -R₂ may be substituted with -OH, -SH, -F, -Cl, -Br, -OPO₃H₂, -COOH, NR₂ or with a saturated or unsaturated, linear or branched alkyl, having from 1 to 10 carbon atoms or with a saturated or unsaturated, optionally heterosubstituted or branched cycloalkyl, having from 3 to 7 carbon atoms;
- the bond between -F and -T is hydrolyzable in vivo by metabolic or enzymatic activity and in particular is a carboxylic ester, a carboxylic amide, a glycoside, an acetal, a ketal, an azo compound, an hydrazide, a carbammate, a thioester, a thioamide, a phosphoric ester, a phosphonic ester, a sulphuric ester, a sulphonic amide, a sulphonic ester.
- the above cited pharmaceutically acceptable salts are salts from inorganic acid (e.g. nitrate, nitrite, chlorhydrate, bromhydrate, sulphate, phosphate), salts from organic acids (e.g. citrate tartrate, acetate, maleate, fumarate, ossalate, p-toluensulphonate, methanesulphonate, ethansulphonate, benzenesulphonate), from inorganic bases (e.g. ammonium, sodium, litium, potassium, magnesium, calcium and zinc salts) or from organic bases (e.g. ammonium salts of organic amines).
The bond between -R₁ and -M is described by the above cited formulae from (Al) to (AV) that is the undulated line shows here where and how is the bond which is necessary to link -M (on the right of the line) and -R₁ (on the left of the line);
- V is chosen among -Z-M₂, -O-Z-M₂, -N(R₂)-Z-M₂, -R₁-Z-M₂, -O-R₁-M₂, -O-R₁-Z-M₂ where:
- M₂ is chosen among -M, -R₁-M, -O-R₁-M, -S-R₁-M, -N(R₂)-R₁-M
where R₁, R₂ and M are described as above;
- Z-M₂ is chosen among the following radicals, optionally salified with nitric acid: 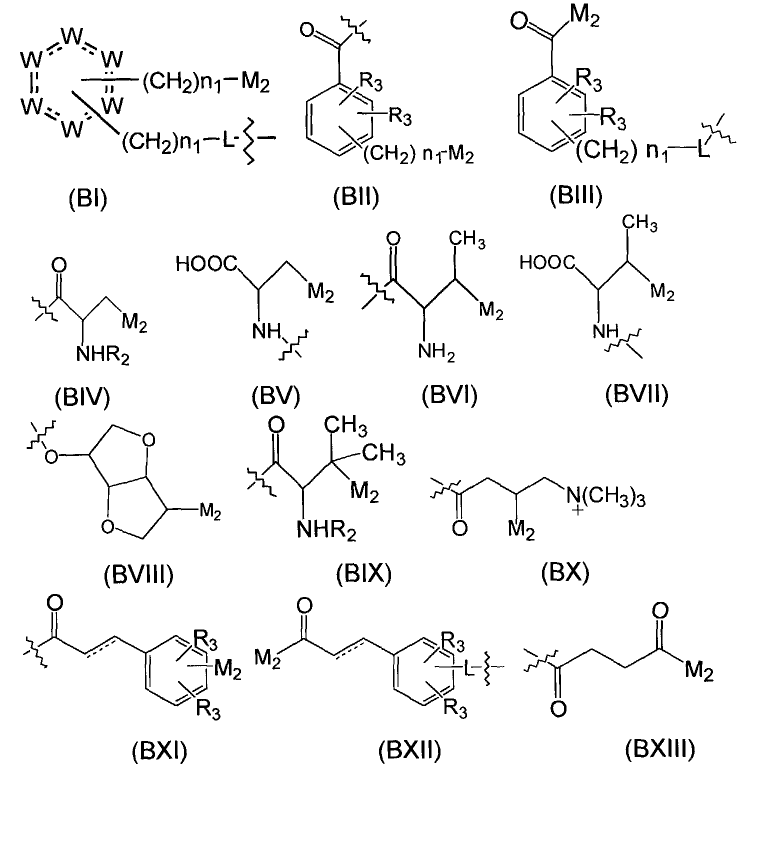 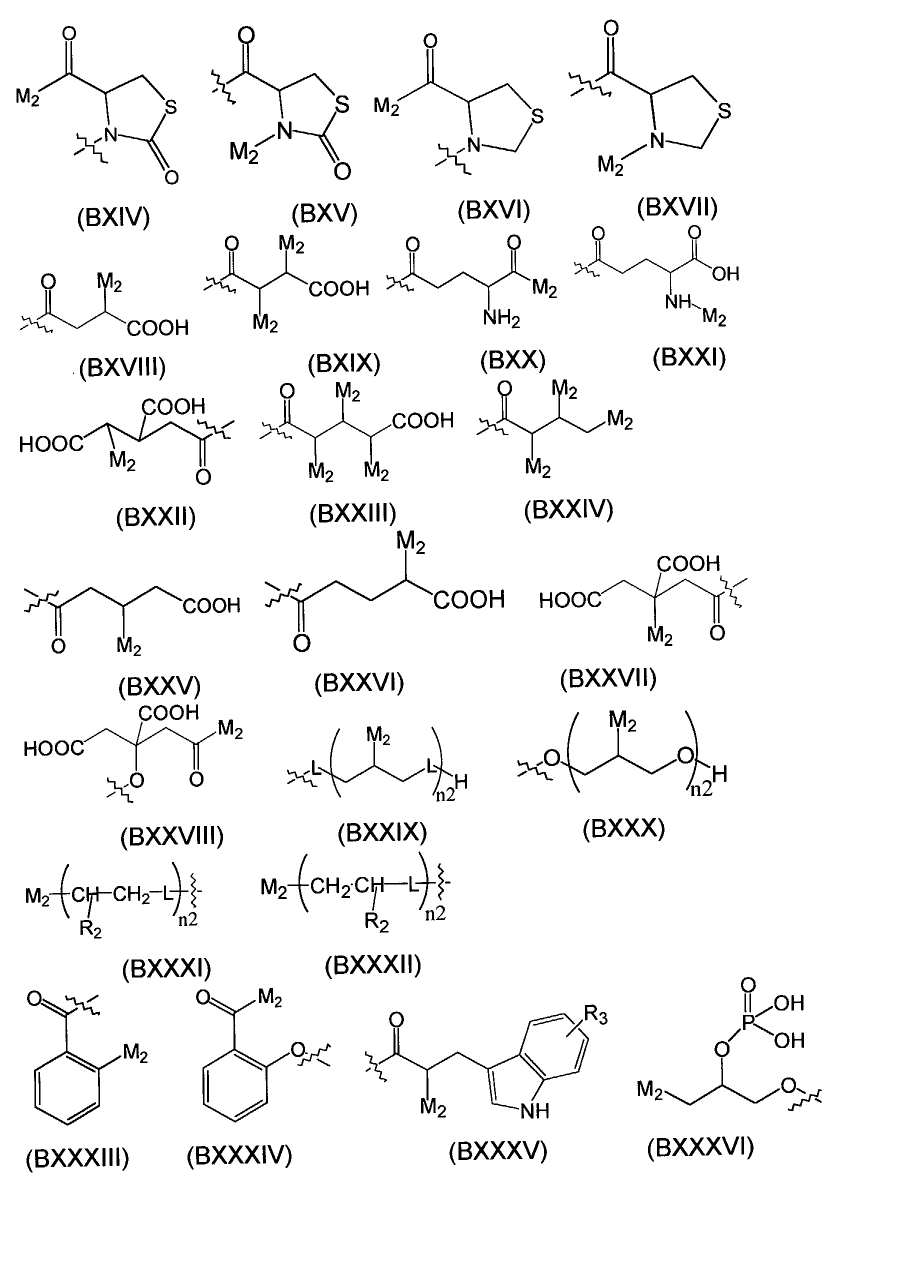 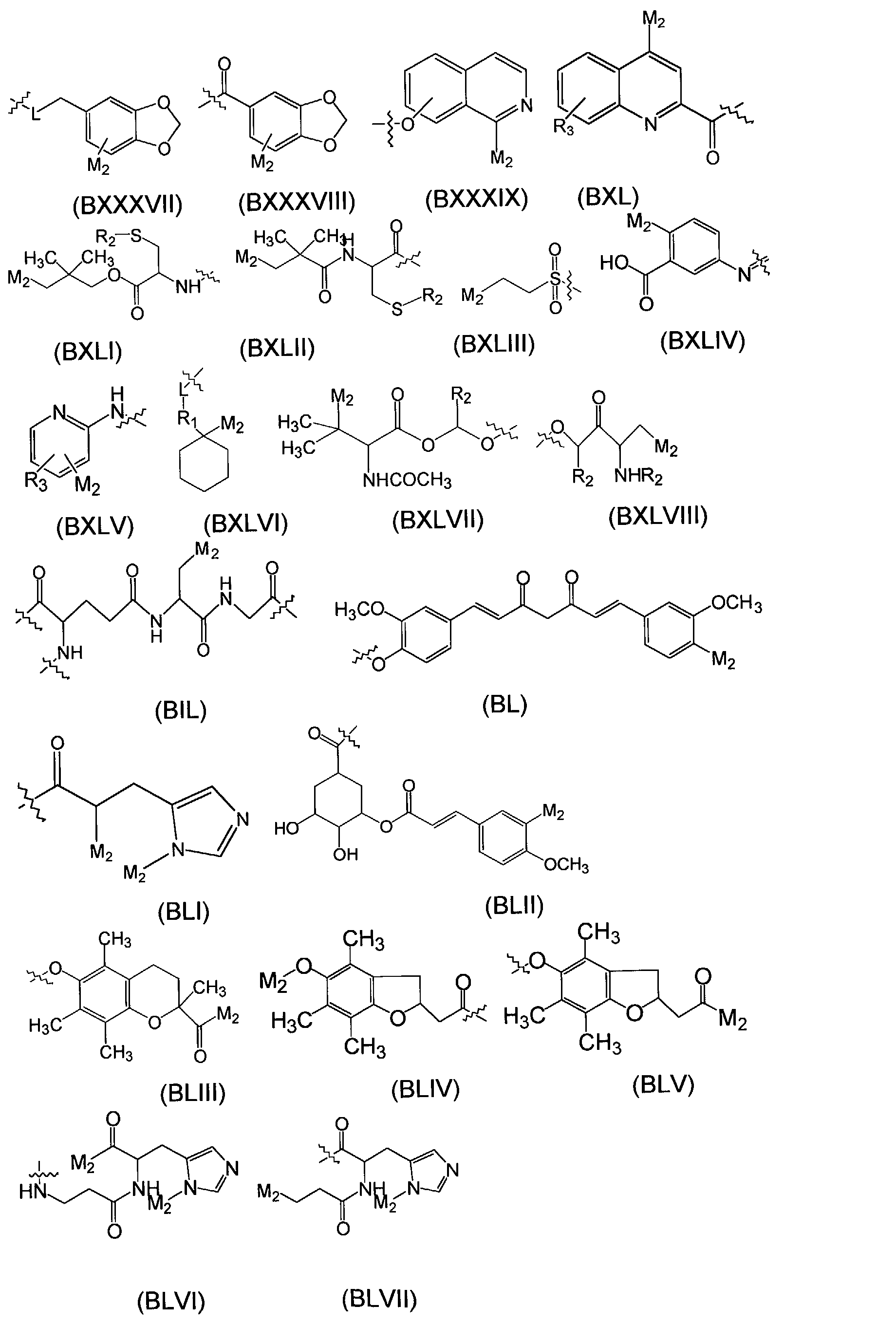 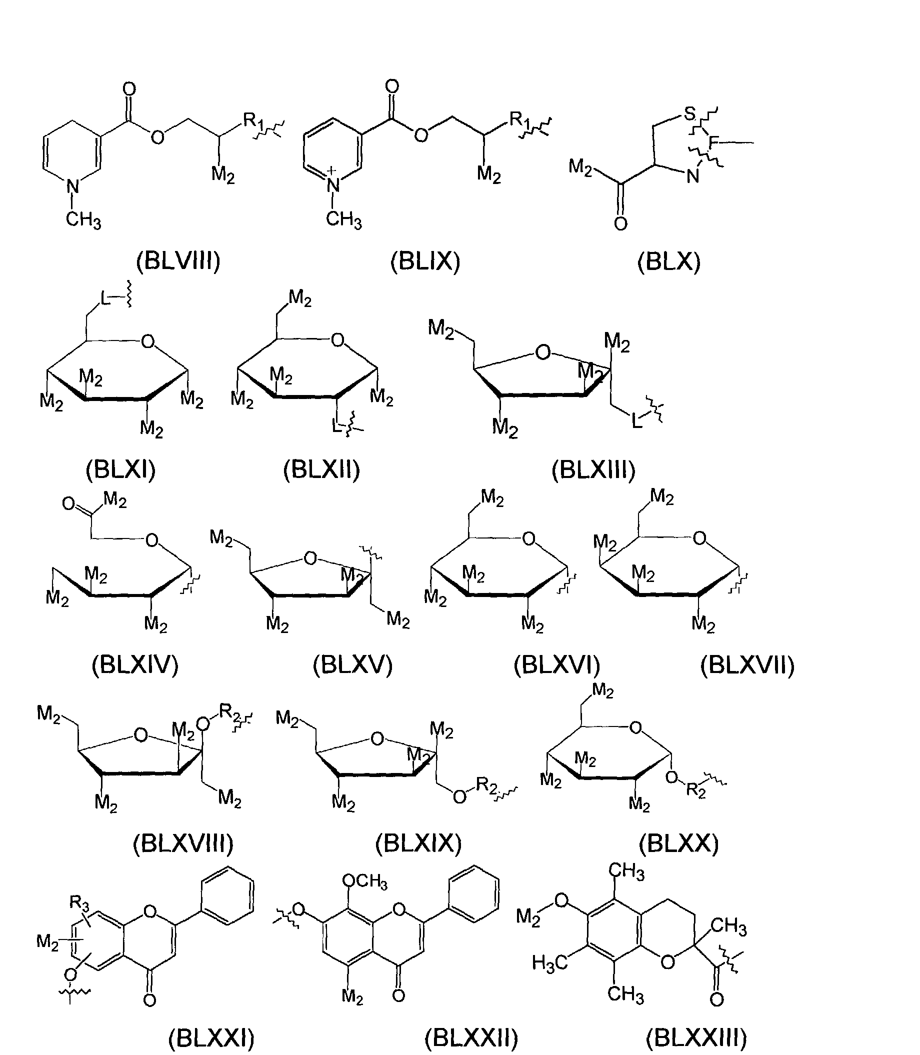
where:
- R₃ is chosen among -H, -OH, -NH₂ ,-NO₂, -CH₃, -CN, -OCH₃ , - CH₂OH, -COOH, -CHO, -F, -Cl, -Br, -CF₃;
- "L" is an oxygen atom -O-, a sulphur atom -S-, a nitrogen atom - NR₂-, a selenium atom -Se- or a carbon atom -CR₂R₂-;
- W is chosen among -CH=, =N-, -N+(O-)=; n₁ is an integer from 0 to 3; n₂ is an integer from 1 to 6, preferably from 1 to 4;
- R₁, R₂, are described as above:
- the dotted line is a simple bond or a double bond; -Ar₁- is an optionally heterosubstituted arylene having from 3 to 7 carbon atoms;
- the undulated line shows where is the bond between -V and the drug -F;
- the bond between -F and -V is hydrolyzable in vivo by metabolic or enzymatic activity and in particular is a carboxylic ester, a carboxylic amide, a glycoside, an acetal, a ketal, an azo compound, an hydrazide, a carbammate, a thioester, a thioamide, a phosphoric ester, a phosphonic ester, a sulphonic amide, a sulphonic ester.

2. Compounds according to claim 1, in which -X is chosen among nitrate salt (All) in a stoichiometric ratio with F of at least 1:1 and the following monovalent radicals -Y₁ optionally salified with nitric acid: 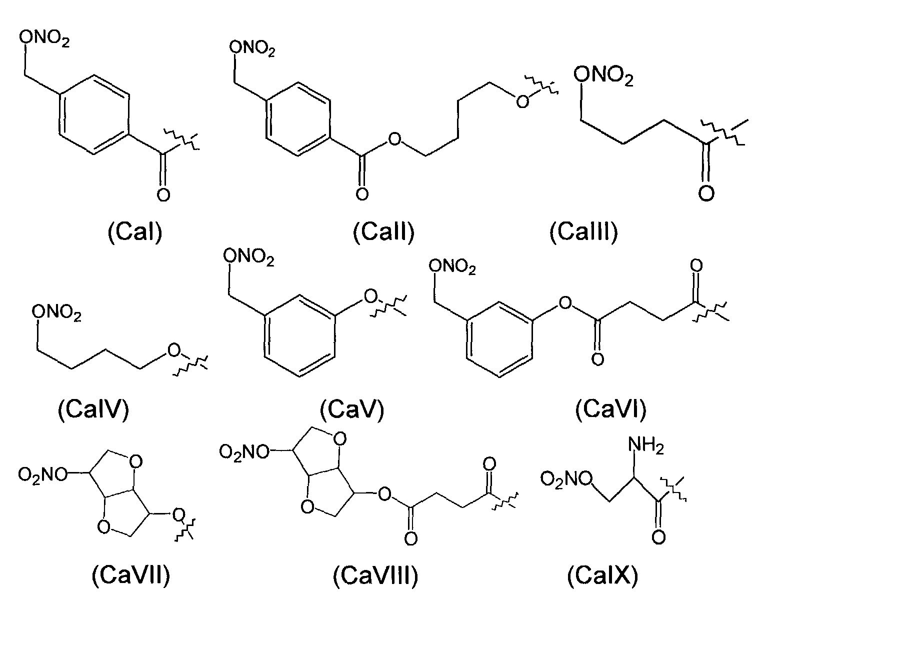 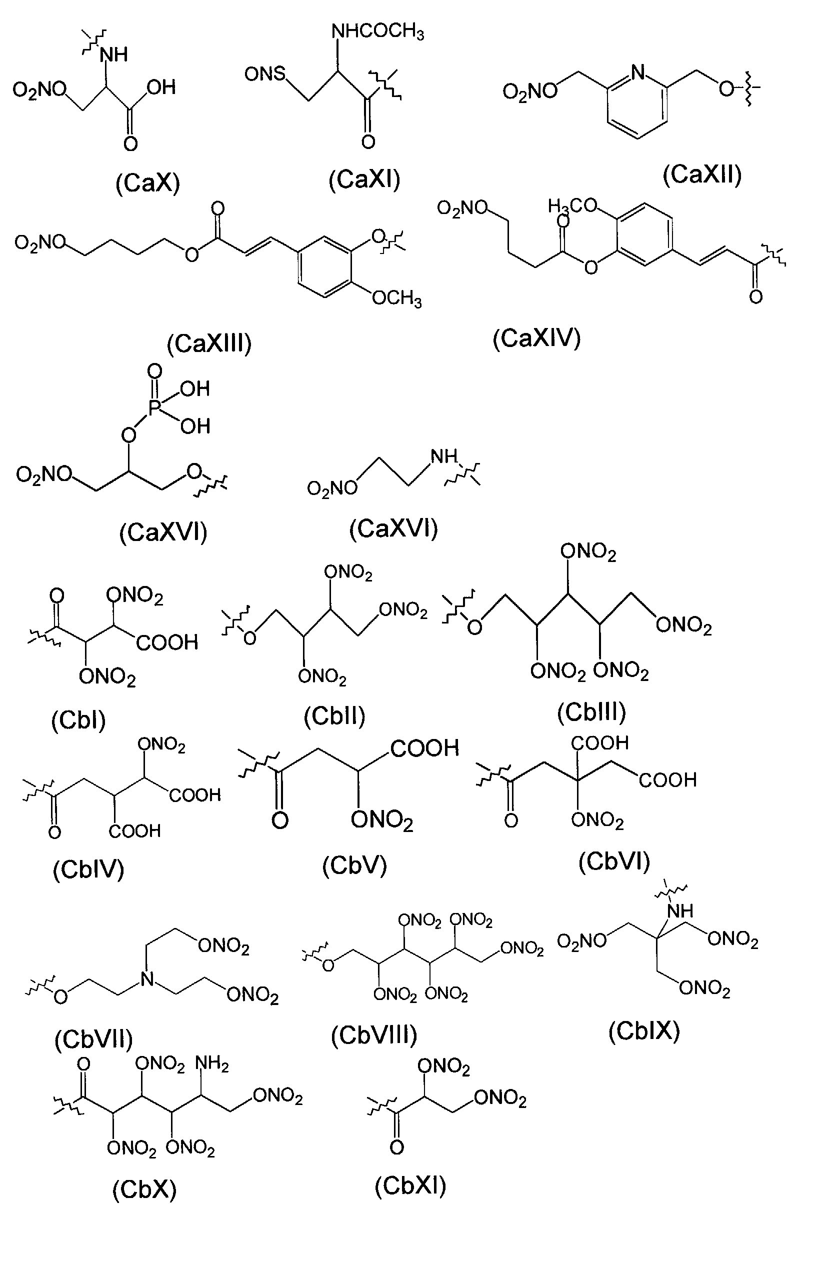 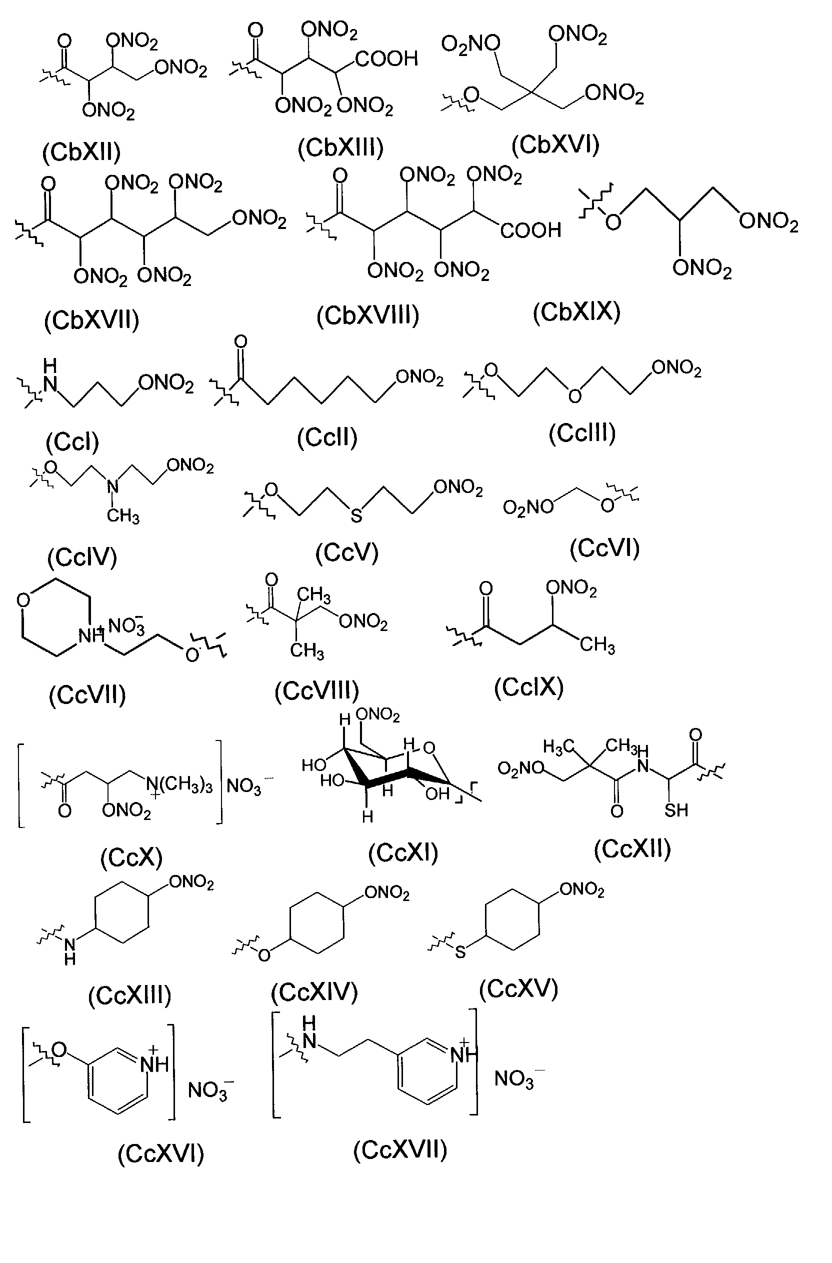 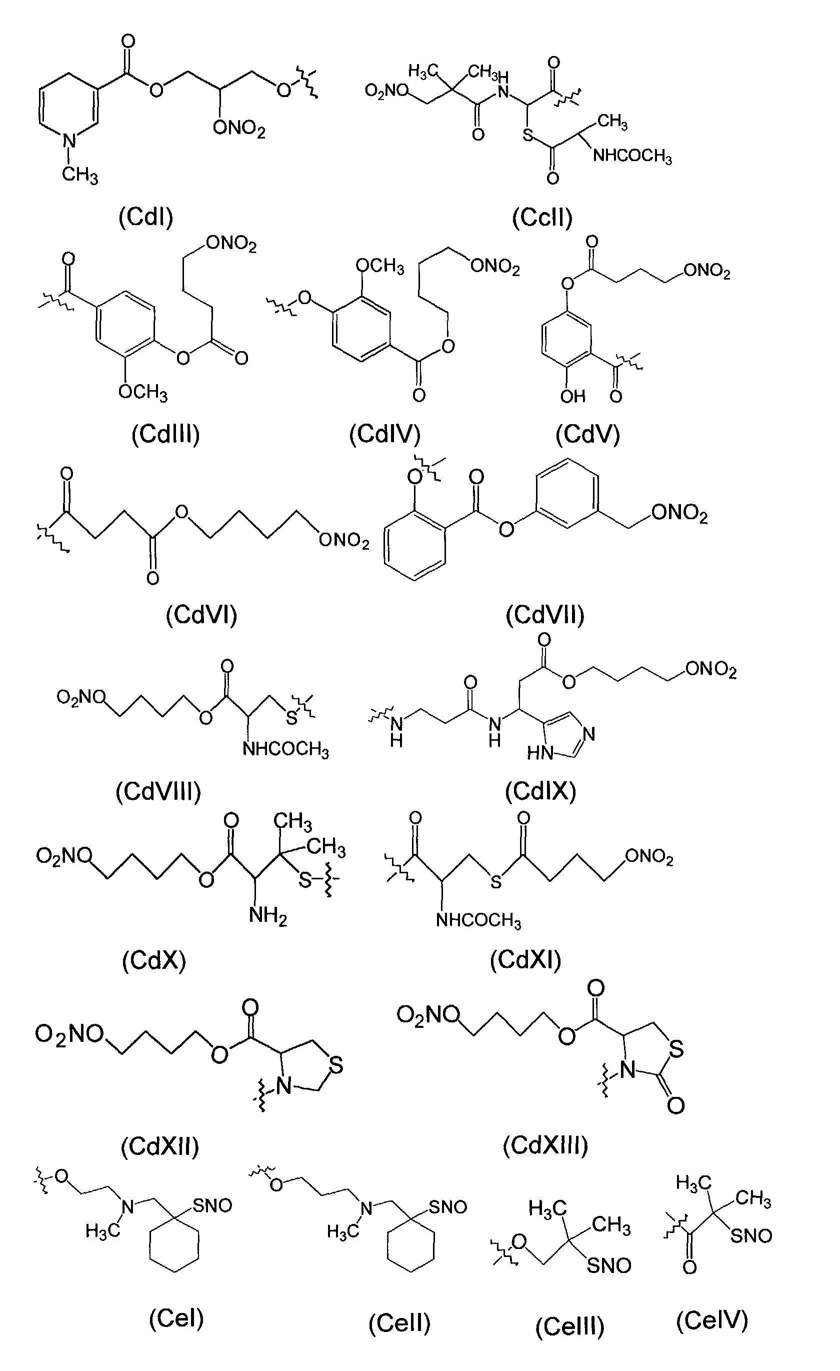 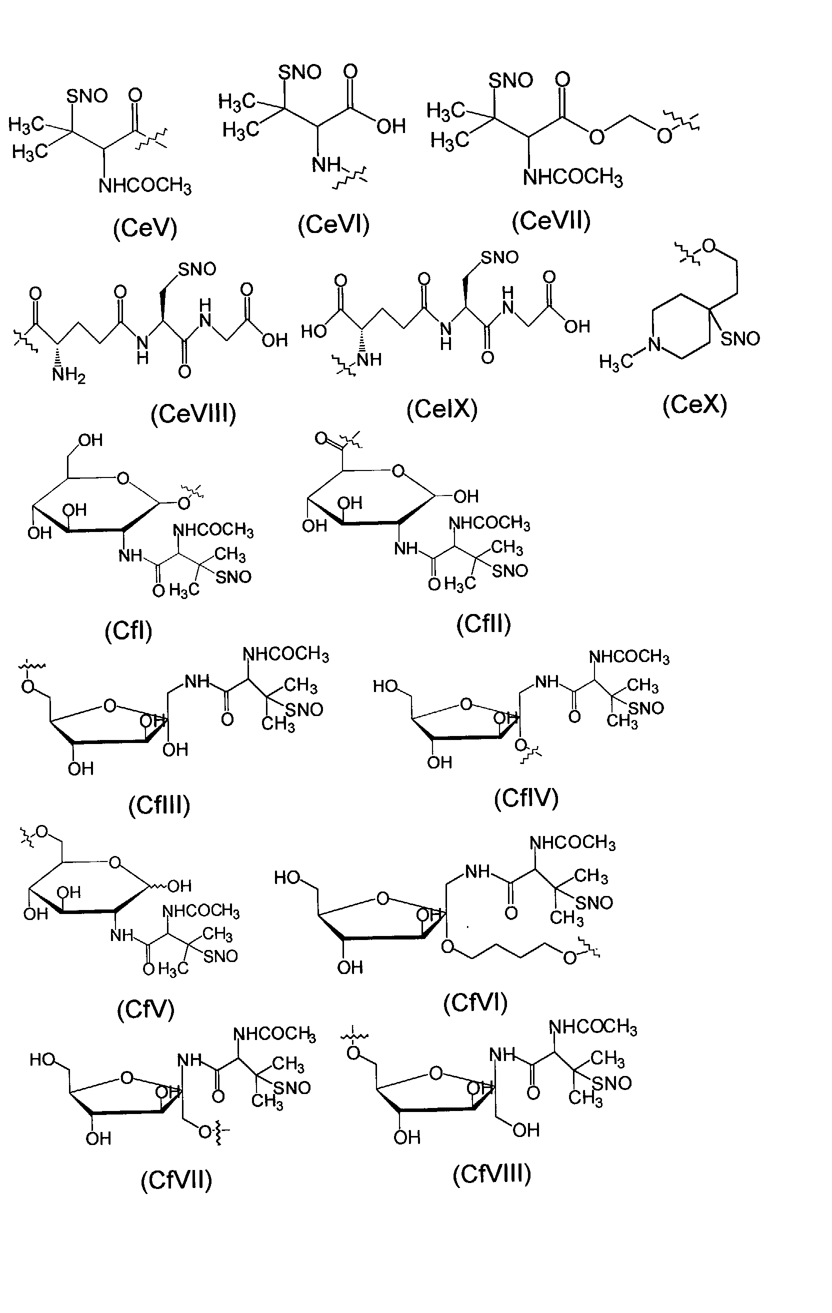 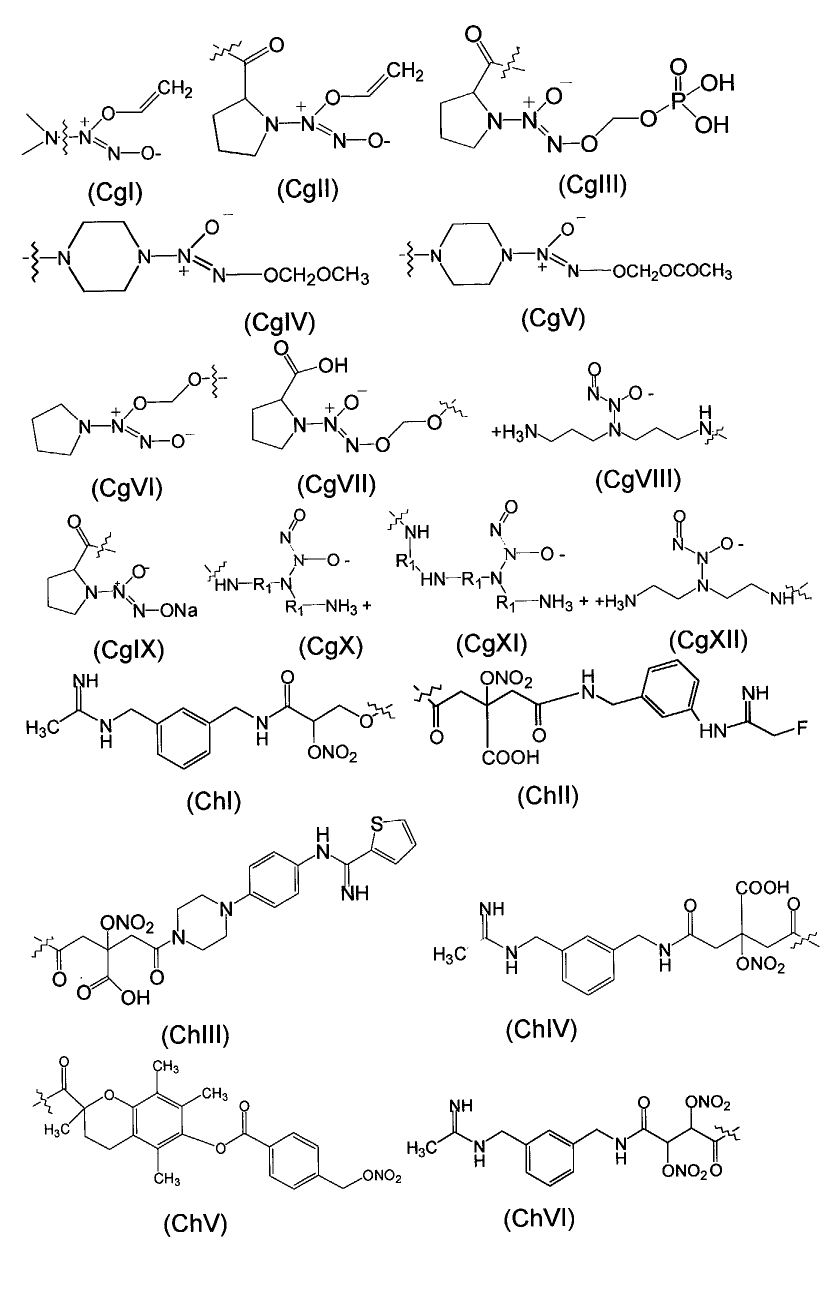 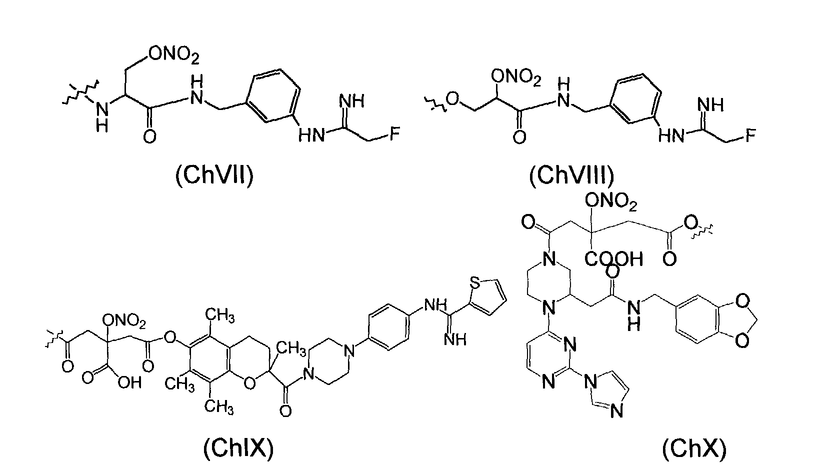 where:
- the dotted line is a simple bond or a double bond;
- the undulated line shows where is the bond between -Y₁ and the drug -F;
- the bond between -F and -Y₁ is hydrolyzable in vivo by metabolic or enzymatic activity and in particular is a carboxylic ester, a carboxylic amide, a glycoside, an acetal, a ketal, an azo compound, an hydrazide, a carbammate, a thioester, a thioamide, a phosphoric ester, a phosphonic ester, a sulphonic amide, a sulphonic ester.

3. Compounds according to claim 2, in which -X is chosen among nitrate salt (All) in a stoichiometric ratio with F of at least 1:1 and the following monovalent radicals -Y₂ optionally salified with nitric acid: 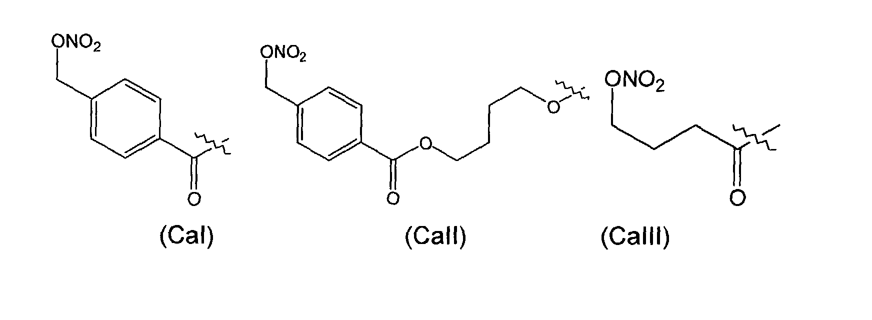 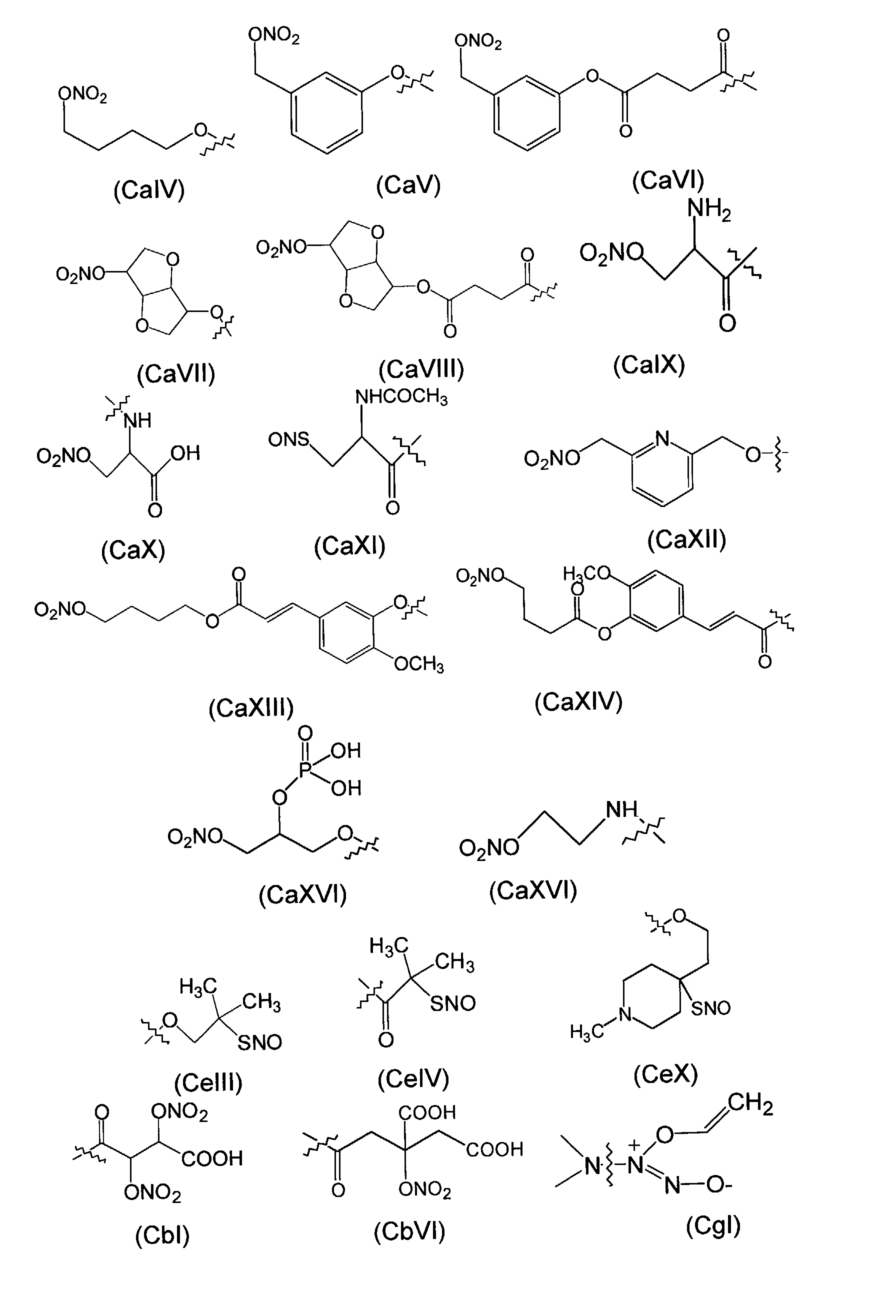
- where the bond between -Y₂ and the drug -F, in the point shown by the undulated line is a carboxylic ester or a carboxylic amide.

4. Compounds according to claim 3 in which -X is the following monovalent radical -Y₃ (paranitroxymethylbenzoyl): 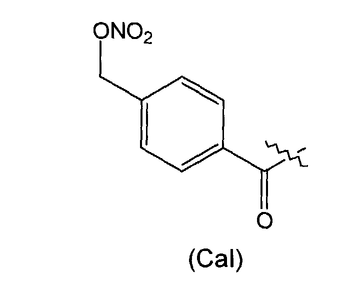 where the bond between -Y₃ and the drug -F, in the point shown by the undulated line, is a carboxylic ester or a carboxylic amide.

5. Compounds according to claims 1-4 in which q is 1.

6. Compounds according to claims 1-5 in which -F is chosen among the following 8 groups of molecules (from 1A to 8A) which are identified by their own INN ("International Nonproprietary Name", from "World Health Organization" database) or by their own company code or by their own chemical name.
GROUP 1A:
ζ₁-tocopherol, ζ₂-tocopherol, δ-tocopherol, ε-tocopherol, η-tocopherol, β-tocopherol, γ-tocopherol, α-tocopherol, 17β-estradiol, 21-acetoxypregnenolone, 6-mercaptopurine, 8α-hydroxy-riboflavin, abetimus, aceclofenac, acemetacin, acetaminosalol, acetoxolone, acexamic acid, aconitine, 3-acetylconitine, actarit, adapalene, adxanthromycin, ajulemic acid, alclofenac, alclometasone, aldesleukin, algestone, alonacic, allopurinol, alminoprofen, amcinafal, amcinonide, amebucort, amelometasone, amfenac, amiprilose, amixitrene, ampiroxicam, amrinone, amtolmetin guacil, anakinra, anirolac, anisperimus, anitrazafen, apafant, apazone, aprepitant, araprofen, artecanin, asimadoline, asobamast, aspirin, ascomycin, ataquimast, atiprimod, atizoram, atliprofen, atrasentan, atreleuton, aurothioglucose, aurothiomalat, aurothiomalic acid, azapropazone, azaspirane, azastene, azathioprine, azimexon, azure A, azure B, azure C, bakeprofen, bamaquimast, balsalazide, basiliximab, batimastat, beclometasone, bendazac, benorylate, benoxaprofen, benzbromarone, benzpiperylone, benzydamine, bepafant, berberine, bermoprofen, betamethasone, betamethasone acibutate, beiwutine, bilirubin, biliverdin, bifeprofen, bimosiamose, bindarit (ELXVII), bosentan, boswellic acids, brasilicardin a, brequinar, bromfenac, bromhexine, bromisoval, bromosaligenin, broperamole, bucillamine, bucloxic acid, bucolome, budesonide, bufexamac, bufezolac, bulleyaconitine, bumadizone, busulfan, butanixin, butibufen, butixirate, butixocort, carbenoxolone, canin, carprofen, celecoxib, chenodeoxycholic acid, chloroprednisone, chloroquine, ciamexon, ciclesonide, cicloprofen, cinaproxen, cinfenoac, cinmetacin, cipemastat, ciproquazone, clamidoxic acid, clidanac, cliprofen, clobetasol, clobetasone, clobuzarit, chlorin e6, chlorophyll, clocortolone, clofenamic acid, clofexamide, clofurac, clometacin, clonixin, clopirac, cloticasone, cloprednol, cloximate, cobanamide, colchicine, colfenamate, coproporphyrin (I, II, III), cormetasone, cortisone, cortivazol, cortodoxone, cridanimod, cyclophosphamide, cyclosporin (A,B,C,D,G), 10-deazaaminopterin, dacopafant, daltroban, dapitant, darbufelone, darusentan, deflazacort, defoslimod, dehydrocholic acid, delmetacin, deloxolone, deltibant, deracoxib, descinolone, desonide, desoximetasone, dexamethasone, dexamethasone acefurate, dexamethasone-21-phosphate, dexbudesonide, dexibuprofen, dexindoprofen, dexketoprofen, dexpemedolac, dextiopronin, deuteroporphyrin IX, diacerein, diclofenac, diclonixin, difenamizole, difenpiramide, diflorasone, diflucortolone, diflumidone, diflunisal, difluprednate, diftalone, diethylhomospermine, dimepranol, dipyrocetyl, dipyrone, diphenyleneiodonium, ditazole, domoprednate, drocinonide, dronabinol, droxicam, duometacin, edatrexate, eltenac, emorfazone, emoctakin, endrysone, enfenamic acid, enolicam, enoxolone, enrasentan, epirizole, eremophyllene a, esculamine, esculentin A, esflurbiprofen, esonarimod, esonarimod, estriol, etersalate, etodolac, 12-epi-scalaradial, etofenamate, etoricoxib, everolimus, ezlopitant, falcinonide, famprofazone, felbinac, fenamole, fenbufen, fenclofenac, fenclorac, fenclozic acid, fendosal, fenflumizole, fenleuton, fenoprofen, fenpipalone, fentiazac, fepradinol, feprazone, figopitant, flavine-adenine dinucleotide, flavonone hydrazone, flazalone, flosulide, flobufen, fluarandrenolide, fluazacort, flucloronide, fludrocortisone, flufenamic acid, flumetasone, flumizole, flunisolide, flunixin, flunoxaprofen, fluocinolone acetonide, fluocinonide, fluocortin, fluocortolone, fluorometholone, fluperolone, fluprednidene, fluprednisolone, fluprofen, fluproquazone, fluquazone, flurbiprofen, fluretofen, flutiazin, fluticasone, folinic acid, forfenimex, formocortal, foropafant, frabuprofen, furaprofen, furcloprofen, furobufen, furofenac, ganaxolone, gentisic acid, gestodene, glaspimod, glatiramer, glucametacin, glycol salicylate, glucosamine, gold thiomalic acid, guaiazulene, gusperimus, halcinonide, halobetasol, halometasone, halopredone, hepatyrix, hemin, hematoporphyrin IX, hematoporphyrin D, hemipyocyanine, hexaprofen, higenamine, hydrocortamate, hydrocortisone, hydroxychloroquine, 3-hydroxyparthenolide, kutkoside, ibufenac, ibuprofen, ibuproxam, icariin, icatibant, ilomastat, ilonidap, imiquimod, indobufen, indometacin, indoprofen, inosine, intrazole, iotroxic acid, iodinin, isatoribine, isofezolac, isoflupredone, isonixin, isoprednidene, isoprofen, isoxepac, isoxicam, israpafant, ivarimod, ketoprofen, ketorolac, ketorolac tromethamine, ketotrexate, kobiin, laflunimus, labradimil, lanepitant, lappaconitine, leflunomide, levamisole, lexipafant, lexofenac, limazocic, licovek, livax, lisofylline, lobenzarit, lobuprofen, lofemizole, lonaprofen, lonazolac, lornoxicam, losmiprofen, loteprednol etabonate, lotifazole, loxoprofen, lubeluzole, mabuprofen, mafosfamide, marimastat, mazipredone, meclofenamic acid, meclorisone, medrysone, mefenamic acid, melittin, meloxicam, meprednisone, mesalazine, meseclazone, mesoporphyrin IX, metacetamol, metanixin, metbufen, methotrexate, methocarbamol, methylprednisolone, methylprednisolone suleptanate, methylcobalamin, methimazole, metiazinic acid, metronidazole, mexoprofen, minocycline, minopafant, mipragoside, miroprofen, misoprostol, mitoxantrone, mizoribine, modafinil, modipafant, mobenakin, mofebutazone, mofetil, mofezolac, mometasone, morazone, morniflumate, moxilubant, mycophenolate mofetil, myoral, N-deacetylranaconitine, N-deacetylfinaconitine, N-deacetyllappaconitine, N-methyl-protoporphyrin IX, nabumetone, naflocort , nagarine, napirimus, naproxen, nemorobucin, nepadutant, nepafenac, nicortonide, niflumic acid, nimesulide, niometacin, nitraquazone, N-desmetylbenzydamine ,nivacortol, nolpitantium, nupafant, octisalate, odalprofen, olsalazine, oleanolic acid, onapristone, oprelvekin, orpanoxin, orazipone, osanetant, oxaceprol, oxametacin, oxaprozin, oxepinac, oxindanac, oxipurinol, oxolamine, oxyfenamate, oxyphenbutazone, paclitaxel, paeony, paracetamol, paramethasone, paranyline, parecoxib, parthenolide, parsalmide, pelubiprofen, pemedolac, penicillamine, pentostatin, pentoxifylline, perisoxal, perizoxal, petrosaspongiolide M, phenylbutazone, piclamilast, picroside I, pidolacetamol, pidotimod, pidotimod, piketoprofen, pilocarpine, pimecrolimus, pimetacin, pipebuzone, piperylone, pirazolac, pirfenidone, piroxicam, pirprofen, pitonakin, pralnacasan, pranoprofen, prasterone, prednazate, prednazoline, prednicarbate, prednisolone, prednisolone 25-diethylaminoacetate, prednisolone sodium phosphate, prednisone, prednival, prednazoline, prednylidene, prefenamate, prifelone, prinomastat, prinomide, probenecid, procodazole, proglumetacin, propacetamol, propyphenazone, proquazone, prospidium, protizinic acid, protoporphirin IX, quercetin, rameswaralide, ramifenazone, ranaconitine, rapamycin, resiquimod, resiquimod, resocortol, rhein, ribavirin , riboflavin, riboflavin monophosphate, riboflavin tetrabutyrate, riboflavin triacetate, triptolide, ridogrel, rimexolone, rimonabant, rocepafant, rofecoxib, romazarit, romurtide, roquinimex, s-adenosylmethionine, salacetamide, salicylamide, salicylamide o-acetic acid, salicylsulfuric acid, salaspermic acid, salnacedin, salsalate, saredutant, scyphostatin, seclazone, secotanaparthenolide, seprilose, sermetacin, setipafant, sibenadet, sirolimus, sitaxentan, sodium hycluronate, sonermin, solimastat, stepronin, stercobilin, sudoxicam, sufosfamide, sulfaloxic acid, sulfasalazine, sulfinpyrazone, sulindac, sumacetamol, suprofen, susalimod, susalimod, suxibuzone, tacrolimus, talmetacin, talnetant, talniflumate, talosalate, tanetin, tanomastat, tarasentan, tauroselcholic acid, tasonermin, tazeprofen, tazofelone, tebufelone, temarotene, tenidap, tenoxicam, teceleukin, tepoxalin, teriflunomide, terofenamate, terprenin, tesicam, tesimide, tetrandrine, tetriprofen, tezosentan, thalidomide, therafectin, thielocin B3, thiazolinobutazone, thiethazole, thymocartin, thymopentin, thymotrinan, tianafac, tiaprofenic acid, tiaramide, ticolubant, tifacogin, tiflamizole , tifurac, tilmacoxib, tilnoprofen arbamel, timegadine, tinoridine, tiopinac, tiopronin, tioxaprofen, tiprotimod, tixocortol, tobramycin , tolfenamic acid, tolmetin, tomoxiprole, tresperimus, triamcinolone, 1-(2-trifluoromethylphenyl) imidazole, 1H-[1,2,4]Oxadiazolo[4,3-a]quinoxalin-1-one, tribuzone, triclonide, trifezolac, triflumidate, trimetrexate, triptolide, tripedane, triptochlorolide, tripdiolide, triptonide, triptolidenol, triptriolide, 16-hydroxytriptolide, tripterine, triptotriterpenic acid B, 12-methoxy triptonoterpene, tropesin, tucaresol, tulopafant, ubenimex, ufenamate, ularitide, ursodeoxycholic acid, ursulcholic acid, urobilin, uroporphyrin (I, III), valacyclovir, valdecoxib, valganciclovir, vedaprofen, vitamin E acid succinate, vitamin E nicotinate, vofopitant, xanoxic acid, xenbucin, ximoprofen, zaltoprofen, zidometacin, zileuton, zoliprofen, zomepirac, α-bisabolol, yunaconitine, wilforlide A, wilforlide B, A-176120, A-197574, A-119435, A-205804, A-241548, A-252444.0, A-293507, A-771726, A-77951, ABS-103, ABT-281, ABT-839, AGIX-4207, AGT-1, ALX-0600, AM-6898A, AM-6927, AM-724, AM-80, AP-1903, APC-2059, APO-501, AR-C73346XX, ARM-434, ATM-027, ATM-027, ATL-2502, BASB024, BASB033, BASB034, BAY-12-9566, BAY-U-3405, BB-2275, BBIC, BB-2827, BB-76163, BCX-1777, BCX-34, BF-389, BMS-184000, BMS-188667, BMS-193884, BMS-214662, BMS-453, Bio-1211, Bio-1272, BX-471, BXT-51072, CARN-1000, C-220, CBF-BS2, CBP-1011, CC-1069, CC-1088, CC-3052, CDB, CDC-501, CDC-801, CDF-571, CGP-28238, CGS-33090A, CHI 621, CK-17, CK-101A, CK-102, CK-103A, CK-112, CK-113, CK-114, CK-115, CK-116, CK-119, CK-120, CK-122, CK-111, CK-117, CK-118, CK-121, CK-123, CK-124, CK-125, CK-126, CK-127, CK-128, CK-130, CK-131, CK-132, CK-133, CK-134, CK-135, CK-136, CK-137, CK-138, CK-139, CK-140, CK-141, CK-142, CK-143, CK-144, CK-145, CK-146, CK-147, CK-148, CK-149, CK-150, CK-151, CK-152, CK-153, CK-154, CK-156, CK-160, CK-163, CK-CLASP-1, COX-189, CPH-82, CP-123369, CPI-1714, CR-3294, CS-670, CSIMM-1, CT-12441, CT-5219, CT-757, CT-767, CT-5269, CY-9701, DA-9601, D-1927, D-609, D-9120, DMP777, DPC-789, DU-6712, DUP-697, DX-88, E-5110, E-5531, E-5564, E-6080, ER-34122, ERL-080, FK-506, FO-5904, FR-167344, FR-153657, FR-228352, FTY-720, G009, GI-245402, GL-701, GPI-1046, GPI-6150, GR-205171, GR-253035, GW-406381, GW-468816, GW-273629, GW-274150, HCL-113, HE-2200, HEP-689, HMR-3408A, HS-378, IC-14, IC-202A, IC-202B, IC-485, ICN-17261, IM-46068, IR-208, IR-208, IR-501, ISA(TX)247, ISIS-104838, ISIS-13280, ISIS-13989, ISIS-16374, ISIS-17044, ISIS-17470, ISIS-18155, ISIS-18669, ISIS-19493, ISIS-2302, ISIS-3082, ISIS-25302, ISIS-27104, IVL-745, J-104132, J-113397, J-113863, JJ-319, JTE-522, K-1115A, KE-298, KE-748, KE-758, KF-20444, KME-4, KT-651, L-167307, L-3002, L-685818, L-732531, L-733725, L-745337, L-761000, L-766112, L-776967, L-778123, L-778736, L-784506, L-784512, L-788123, L-791456, L-818571, LDP-02, LF-173657, LF-160335, LF-160687, LJP-394, LJP-712, LK-413, LU-135252, LY-262691, LY-242040, LY-311727, LY-733725, M-40403, MC-1R, MCP-3, MDL-108180, MDL-201112, MK-591, MK-0663, ML-3000, ML-3116, ML-3176, ML-3177, MNA-715, MSP-771, MSP-771 , MX-32, MX-56, MX-68, NBI-5788, NMI-377, NS-398, OB-101, OB-186, OM-89, OM-294DP, ONO-NT-126, ONO-4817, ONO-AE-1329, OP-229-648, OP-2000, PCM-4, PD-057081, PD-098059, PD-159879, PD-56840, PD-980589, PEP-1261, PGE-2946979, PGV-20229, PIXY-321, PMX 622, PNU-168727, PNU-190192, PP1D-1, PS-508, PTI-601, R-115777, R-125224, R-173262, RAD-001, RO-320-1195, RO-32-3555, RPR-130401, RPR-200765A, RS-130830, RS-57607, RVC-588, RVC-589, RVC-593, RVC-595, RVC-596, RVC-597, RVC-598, RVC-599, RVC-600, RVC-601, RWJ-57504, RWJ-67657, RWJ-68354, S-19812, S-2474, S-27609, S-33516, SA-6541, SA-9499, SB-202190, SB-203580, SB-203580, SB-206718, SB-209670, SB-210313, SB-217242, SB-217969, SB-220025, SB-226882, SB-242235, SB-262105, SB-273005, SB-683698, SC-236, SC-26196, SC-77537, SC-299, SC-558, SC-560, SC-57461A, SC-57666, SC-58125, SC-58236, SC-58451, SCH-218157, SCH-23863, SCH-66336, SCIO-469, SCV-07, SDZ-RAD, SDZ MDL-987, SDZ-281240, SDZ ASM-981, SEK-1005, SH-636, SI-3501, SIT-115, SKF-105809, SKF-86002, SP-057, SP-4-1, SP-600125, SPA-1761B, SR-31747, SR-140333A, SU-101, SU-20, T-614, TAK-044, TAK-603, TAN-2474A, TBP-1, TBC-3486, TBC-3342, TMC-2A, TMC-2B, TMC-2C, TMC-95A, TMX-67, TR-9109, TP-10, TRK-530, TS-932, TSL-225, U-19451A, VK-19911, VML-295, VRT-37742, VX-148, VX-702, VX-740 (HMR-3480), VX-765, VX-944, VX-954, WA-8242B, WF-27082B, WIN-64338, WY-14643, Y-39041, WY-48489, Y-700, Z-53, ZD-2315, ZD-6416, ZD-7349, ZD-7349A, ZK-809984, ZK-810000, 1400W
GROUP 2A:
1-α-hydroxy-vitamin D₂, 17β-estradiol, 22-oxy-calcitriol, 24,28-methylene-1-α-hydroxy-vitamin D₂, 3-allylfarnesol, 4B4-6-21, 4-hydroxy-tamoxifen, A-176120, A-197574, abiraterone , ABT-839, AGIX-4207, AGT-1, alendronic acid, altrenogest, AP-21946, AP-22188, AP-22408, AP-22409, AP-22650, APC-3328, arginylglycylaspartic acid, arzoxifene, avicatonin, bafilomycin A1, BB-2275, BF-389, BMP-17, BMS-214662, butedronic acid, calcifediol, calcipotriol, calcitonin, calcitriol, celecoxib, CGP-77675, CHF-3142, CHF-3150, CHF-3316.01, CLIK-071, CLIK-164, CLIK-166, clodronic acid, clomifene, colecalciferol, concanamycin A, COX-189-, CP-336156, CP-336156, CP-424391, cystatin C, D-609, DCR5, deaminohydroxy-toremifene, deracoxib, desogestrel, DF-1601A, dienogest, dihydroraloxifene, dihydrotachysterol, 21,25-dihydroxycholecalciferol, doxercalciferol, droloxifene, E2-BP, E-5110, ED-71, elcatonin, EMD-84444, enclomifene, ergocalciferol, esomeprazole, esonarimod, estradiol, etidronic acid, etonogestrel, examorelin, 775B, FA-70D, falecalcitriol, FC-1271, flocalcitriol, formebolone, FR-167356, FR-177995, GGTI-2166, GGTI-298, HEP-187, HEP-689, HMR-3339A, HMR-3408A, ibandronic acid, idoxifene, IGF-I/BPII, IM-46068, incadronic acid, inocoterone, ipriflavone, ISIS-104838, ISIS-17044, ISIS-27104, KE-298, KE-748, KE-758, KME-4, KW-8232, L-167307, L-778123, L-788123, lansoprazole, lasofoxifene, leminoprazole, levormeloxifene, levosimendan, lidadronic acid, liposomal isa-13-1, MDL-201000, MDL-201003, MDL-201053, MDL-201112, medronic acid, medroxyprogesterone , mesabolone, minodronic acid, miproxifene, NDE-1003, 4-hydroxy-tamoxifen, N-desmethyl-tamoxifen, neridronic acid, nitromifene, NNC-45-0095, nomegestrol, norelgestromin, norethindrone , norethisterone, NPS-2143, NPS-2143, NSL-1406, NVP-AAK980, NVP-AAK-980, olpadronic acid, omeprazole, OPG, ORG-30659, ormeloxifene, osaterone, oxidronic acid, pamidronic acid, panomifene, pantoprazole, PCM-4, PD-098059, PD-098059, PD-980589, PGE-336646, pipendoxifene, piridronic acid, plicamycin, pralnacasan, progesterone, promegestone, R-115777, rabeprazole, raloxifene, ranelic acid, risedronic acid, RO-26-9228, roxibolone, RPR-130401, RPR-200765A, RPR-200765A, RWJ-67657, RWJ-68354, S-2474, SB-202190, SB-203580, SB-206718, SB-210313, SB-220025, SB-223245, SB-242235, SB-242784, SB-242784, SB-265123, SB-267268, SB-273005, SC-56631, SC-68448, SCH-60663, SCH-66336, SCIO-469, SCRC-2941-18, secalciferol, secalciferol, semparatide, SH-636, simendan, SKF-105809, SKF-86002, solimastat, SR-31747, SUN-E3001, T-614, TAK-778, TAN-2483A, TBP-1, tamoxifen, tenatoprazole, teriflunomide, teriparatide, teriparatide, tibolone, tilmacoxib, tiludronic acid, TJN-135, toremifene, trenbolone, trimegestone, trimgesterone, trioxifene, TSE-424, valdecoxib, vinigrol, vitamin D₁, vitamin D₂, vitamin D₃, vitamin D₃-1-retinate, vitamin D₄, vitamin D₅, vitamin K₂, VK-19911, VX-702, VX-740, VX-745, VX-765, VX-954, WY-47766, XT-238, XT-44, XT-533, XW-630, YM-175, zanoterone, ZG-2807A, zindoxifene, zoledronic acid, zuclomifene
GROUP 3A:
ABT-594, ADL-10-0101, ALX-0646, 5-bromosalicylic acid acetate, acetaminophen, acetylsalicylsalicylacid, 2-acetylsalicylamido-4-picoline, alendronic acid, alfentanil, alimadol, alletorphine, allylprodine, almotriptan, alniditan, aloxiprin, alphaprodine, alpiropride, ALX-0388, ALX-0646, AM-374, AM-404, aminochlorthenoxazin, aminopropylon, aminopyrine, anandamide, anileridine, anilopam, antipyrine, antrafenine, apadoline, aprepitant, asimadoline, aspirin, avitriptan, benzydamine, benzylmorphine, bezitramide, BCH-2963, BIBN-4096BS, botulinum toxin type A, bremazocine, brifentanil, bromadoline, bucetin, buprenorphine, butacetin, bupivacaine, butinazocine, butorphanol, calcium acetylsalicylate, capsavanil, carbamazepine, carbiphene, carfentanil, carsalam, chlorthenoxazine, cinchophen, ciprefadol, ciprofloxacin, ciramadol, cizolirtine, clodronic acid, clonitazene, clonixeril, cloracetadol, codeine, cogazocine, CP-96345, CP-99994, CP-55940, cropropamide, crotethamide, dapitant, delmopinol, desomorphine, dexoxadrol, dextromoramide, dexmedetomidine, dezocine, diampromide, dibusadol, didemnin B, difenamizole, dihydrocodeine, dihydroergotamine, dihydromorphine, dimenoxadol, dimepheptadol, dimethylthiambutene, dioxaphetyl, dipipanone, diproxadol, dipyrocetyl, dipyrone, divalproex, dolasetron, donitriptan, dotarizine, doxpicomine, E-2078, eletriptan, emorfazone, enadoline, epirizole, eptazocine, epibatidine, ergocornine, ergocorninine, ergocryptine, ergotamine, eseridine, esketamine, ET-142, ethenzamide, ethoheptazine, ethoxazene, ethylmethylthiambutene, ethylmorphine, etodolac, etofenamate, etonitazene, ezlopitant, F-11356, fampridine, fenoprofen, fentanyl, figopitant, filenadol, FK-888, floctafenine, flufenamic acid, flumedroxone, flumexadol, fluoresone, fluoxetine, flupirtine, fluradoline, flurbiprofen, fonazine, fosfosal, frakefamide, frovatriptan, gaboxadol, ganaxolone, GI-87084B, glafenine, GR-73632, GR-82334, GR-203040, GV-150526, GV-228869, GV-213237, GV-196771, GV-217828, GW419458, hydrocodone, hydroxypethidine, HU-210, ibandronic acid, ibazocine, icatibant, ICI-174864, incadronic acid, indomethacin, iprazochrome, isoladol, IS-159, isomethadone, JTE-522, JWH-051, JWH-015, ketazocine, ketobemidone, ketoprofen, ketorfanol, ketorfanol, ketorolac, L-365260, lanepitant, lappaconitine, lefetamine, letimide, levonantradol, levorphanol, levobupivacaine, lidadronic acid, lisuride, lofentanil, lomerizine, lorcinadol, L-732138, L-758298, L-759633, L-759656, L-759787, L-768242, L-775606, LY-235959, LY-274614, LY-303870, LY-334370, LY-344864, LY-334370, MBT, MDL-747216, meperidine, meptazinol, metacetamol, metazocine, metergotamine, methadone, methotrimeprazine, methyldihydromorphine, methysergide, metkephamid, metoclopramide, metofoline, metopon, mexiletine, mimbane, minodronic acid, mirfentanil, MK-801, molinazone, morphine, moxazocine, MR-2266, MT-500, myfadol, myrophine, nabitan, nafoxadol, nalbuphine, nalpitantium, nalmexone, nantradol, naproxen, naproxol, naratriptan, narceine, nefopam, nefopam, nepadutant, nerbacadol, nexeridine, nicocodine, nicodicodine, nicomorphine, nifenazone, nonivamide, norcodeine, norlevorphanol, normethadone, normorphine, norpipanone, NS-398, ocfentanil, OHM3295, olvanil, osanetant, oxapadol, oxetorone, oxitriptan, oxycodone, oxymorphone, pamidronic acid, paracetamol, parsalmide, p-bromoacetanilide, PD-117302, PD-154075, pentazocine, perisoxal, phenacetin, phenadoxone, phenazocine, phenazopyridine, pheniramidol, phenocoll, phenoperidine, picenadol, pidolacetamol, piminodine, pinadoline, pipradimadol, pipramadol, piritramide, pizotyline, PL-017, p-lactophenetide, PNU-109291, pravadoline, pravadoline, prodilidine, prodolic acid, profadol, proheptazine, promedol, propacetamol, propiram, propoxyphene, propofol, proxorphan, quadazocine, R-84760, remifentanil, rimazolium, rimonabant, risedronic acid, rizatriptan, ropivacaine, RP-60180, RP-67580, RP-68651, RPR-100893, RS-127445, S-19014, salethamide, salicin, sameridine, salverine, saredutant, SB-209509, SB-218842, SB-220453, SB-235863, SCH-23390, SCH-50971, SDZ-NKT-343, sergolexole, simetride, SNF-8702, SNF-8814, SNF-8820, SNF-9007, spiradoline, SR-140333, SR-141716A, SR-142801, SR-144528, SR-48965, SR-48968, sufentanil, sumacetamol, sumatriptan, suprofen, talnetant, tetrandrine, thiambutene, tidembersat, tifluadom, tilidine, tiludronic acid, tinoridine, tolpadol, tonaberstat, tonazocine, topiramate, tramadol, trefentanil, triclacetamol, tropoxin, U-50488H, U-62066, U-69593, veradoline, verilopam, viminol, vofopitant, volazocine, xorphanol, WIN-55212-2, zatosetron, zenazocine, zoledronic acid, zolmitriptan, zucapsaicin, ziconotide
GROUP 4A:
ζ₁-tocopherol , ζ₂-tocopherol, α-tocopherol , δ-tocopherol, ε-tocopherol, η-tocopherol, β-tocopherol, γ-tocopherol, atizoram, 6-azauridine, 8α-hydroxy-riboflavin, acitretin, adapalene, algestone, γ-aminolevulinic acid, amlexanox, anthralin, azaribine, azelaic acid, APC-2059, ASM-981, becaplermin, bergaptene, bexarotene, BMS-185411, BMS-297208, bucladesine, butantrone, calcipotriene, cedefingol, chrysarobin, cioteronel, CMI-392, CP-80633, CPR-1152, cycloheximide, cyproterone, cytoxazone , dapsone, dexamethasone, FR-900520, domoprednate, enazadrem, ER-38930, ER-41666, etretinate, FK-506, H-1305, HU1124, hyaluronic acid, hydrocortisone, IR-502, ISIS-12854, ISIS-18268, L-762943, lactic acid, lexacalcitol, lobophorin A, lonapalene, maxacalcitol , momordin Ic, motretinide, MRE-0094, NAB2, NIP-530, pantoprazole, pauciflorine a , peldesine, pexiganan, prednisolone, prednisone, pyrogallol, R-115866, repifermin, resorcinol, retinoic acid, riboflavin, riboflavin monophosphate, riboflavin tetrabutyrate, riboflavin triacetate, seocalcitol, paricalcitol, RO-201724, SDZ-ASM-981, safingol, SB-216754, SB-235699, SB-332235, SCH-47112, sulfapyridine, tacalcitol, TAK-427, tazarotene, temarotene, tepoxalin, terbinafine, tetroquinone, thalidomide, ticolubant, tioxolone, TP-508, TRK-820, vitamin E acid succinate, VX-497
GROUP 5A:
4-phenylbutyrate, ablukast, acefylline clofibrol, acetylcysteine, acitazanolast, acreozast, acrivastine, adamexine, adapalene, adibendan, albifylline, albuterol, alclometasone, alifedrine, alinastine, alloclamide, almeterol xinafoate, almitrine, alprogen , altapizone, ambicromil, ambroxol, amebucort, amelometasone, amicibone, amipizone, amlexanox, amphotericin B, andolast, antazoline, apafant, apaxifylline, argatroban, argimesna, arofylline, asmanex, asobamast, astemizole, ataquimast, atizoram, atreleuton, azatadine, azelastine, baclofen, baicalein, bamaquimast, bambuterol, bamipine, barmastine, batebulast, beclometasone, becotide, beloxamide, benafentrine, bencisteine, benproperine, benzonatate, bepafant, bepotastine, betamethasone, bietanautine, bilastine, bimosiamose, binizolast, bitolterol, bromhexine, bromodiphenhydramine, brompheniramine, brovanexine, broxaterol, budesonide, bufenadrine, bufrolin, bunaprolast, butamirate, butaprost, butethamate, butixocort, cabastine, caffeine, calfactant, calcitrol, camonagrel, caramiphen, carbapenem, carbetapentane, carbinoxamine, carbocisteine, carebastine, carmantadine, cartasteine, cefaclor, cefpimizole, cetirizine, cetoxime, chlophedianol, chlorcyclizine, chloropyramine, chlorothen, chlorpheniramine, ciclesonide, ciclotropium, cilomilast, cilutazoline, cimaterol, cimetropium, cinalukast, cinnarizine, cipamfylline, ciproquazone, cirazoline, cistinexine, clemastine, clemizole, climbazole, clobenzepam;, clobenztropine, clobutinol, clocinizine, cloperastine, clopidogrel, closiramine, cloticasone, cloxacepride, codeine, codeine N-oxide, codeine phosphate, colterol, cormetasone, cromakalim, cromoglicate lisetil, cromoglicic acid, cromolyn sodium , cyclexanone, cycliramine, cyproheptadine, cysteine, dacemazine, dacisteine, dacopafant, dalbraminol, daltroban, dametralast, danosteine, dapitant, darodipine, dazmegrel, dazoquinast, decominol, deflazacort, deltibant, dembrexine, denbufylline, deptropine, descarboethoxyloratadine, descinolone, desloratadine, desonide, desoximetasone, dexamethasone, dexamethasone-21-phosphate, dexbrompheniramine, dexbudesonide, dexchlorpheniramine, dexetozoline, dexsecoverine, dextiopronin, dextromethorphan, dibenzonium, difeterol, dihydrocodeine, dihydrocodeinone enol acetate, dimemorfan, dimesna, dimethindene, dimethoxanate, dimetorfan, dioxethedrin, diphenhydramine, diphenylpyraline, diproteverine, divabuterol, docebenone, domipizone, domitroban, doqualast, dorastine, doxaminol, doxaprost, doxepin, doxofylline, doxylamine, dropopizine, drotebanol, duramycin, ebastine, eclazolast, efletirizine, eflumast, elziverine, embramine, emedastine, enefexine, enazadrem phoshate, enofelast, enoxamast, enprofylline, ensulizole, epinastine, epinephrine, eprazinone, erdosteine, esculetin, espatropate, etacepride, etanterol, ethylmorphine, etolotifen, etymemazine, ezlopitant, fenethazine, fenleuton, fenoterol, fenprinast, fexofenadine, figopitant, filaminast, flerobuterol, flezelastine, flixotide, fluazacort, flixoride, flufylline, flumetasone, flunisolide, fluprofylline, fluproquazone, fluticasone, flutropium, fominoben, formoterol, foropafant, fudosteine, furafylline, furegrelate, furoate, gamfexine, genleuton, genistein, glimepiride, guaiactamine, guaiapate, guaietolin, guaifenesin, guaifylline, guaimesal, guaisteine, halometasone, hexopyrronium, heterocodeine, histamine, histapyrrodine, hydrocodone, ibudilast, hydroxychloroquine, hydroxyzine, icatibant, idaverine, idenast, ifetroban, ilonidap, imitrodast, imoxiterol, indanazoline, ipratropium , iproheptine, iralukast, isalsteine, isamoxole, isbogrel, isbufylline, isoaminile, isocromil, isoetharine, isopromethazine, isoproterenol, isothipendyl, israpafant, itazigrel, ketotifen, lanepitant, laprafylline, letosteine, levalbuterol, levcromakalim, levmetamfetamine, levocabastine, levocetirizine, levodropropizine, levopropoxyphene, levopropylhexedrine, levosulpiride, lexipafant, lifarizine, linazolast, linetastine, linotroban, lisofylline, lodoxamide, lofemizole, lombazole, lomerizine, lonapalene, loratadine, loxanast, luteolin, mabuterol, mapinastine, masoprocol, mebhydrolin, mecysteine, medrylamine, melquinast, menthol, mepixanox, mequitamium, mequitazine, meribendan, meropenem, mesna, metaproterenol, metron s, metaterol, methafurylene, methaphenilene, methapyrilene, methotrexate, methylprednisolone, mexafylline, midaxifylline, midazogrel, midesteine, mifentidine, milrinone, milverine, minocromil, minopafant, mipitroban, mizolastine, mizolastine , mizolastine, modipafant, moguisteine, mometasone, mometasone, montelukast, morclofone, morphine sulphate, motapizone, moxaverine, moxifloxacin, moxilubant, nafagrel, naflocort, naminterol, napactadine, naphazoline, narceine, nardeterol, nedocromil, neltenexine, nemazoline, nepadutant, neraminol, nesosteine, nestifylline, nicogrelate, nisbuterol, nitraquazone, nivimedone, noberastine, norastemizole, norgestrel, normethadone, noscapine, nuclomedone, nupafant, nuvenzepine, olopatadine, olpimedone, olprinone, omonasteine, ontazolast, orphenadrine, osanetant, oxabrexine, oxagrelate, oxalinast, oxarbazole, oxatomide, oxeladin, oxolamine, oxomemazine, ozagrel., pamicogrel, paramethasone, pargeverine, pemirolast , pentoxifylline,, perbufylline, phenindamine, pheniramine, phenylephrine , phenylpropanolamine, phenyltoloxamine, phenylbutyrate, pholcodine, piclamilast, picoperine, picumast, picumeterol, pimobendan, pinacidil, pipazethate, pirbuterol, pirmagrel, pirodomast, pirolate, pobilukast, pranlukast, prednisolone, prednisolone farnesylate, prednisone, prenalterol, prenisteine, prenoverine, prenoxdiazine, procaterol, promethazine, propentofylline, propiverine, proxicromil, pseudoephedrine, pumafentrine, pyribenzamine, pyrilamine, pyroxamine, pyrrobutamine, quazolast, quifenadine, quiflapon, quillaic acid, quinotolast, racemethorphan, racephedrine, ramatroban, radolmidine, raxofelast, repirinast, resiquimod, revatropate, revenast, ridogrel, rimiterol, rilopirox, rimonabant, rispenzepine, ritolukast, rocastine, rocepafant, rociverine, roflumilast, rolafagrel, rolipram, rotoxamine, rupatadine, salbutamol, salmeterol, salmeterol xinafoate, salmisteine, samixogrel, saredutant, sarpogrelate, satigrel, scopinast, secoverine, seratrodast, seretide, setipafant, sevitropium mesilate, siguazodan, siltenzepine, sintropium, sivelestat , somantadine, sophocarpine , spirofylline, stacofylline, stepronin, sudexanox, sulotroban, sulukast, sunagrel, suplatast, tagorizine, talnetant, tasuldine, taurosteine, tazanolast, tazifylline, taziprinone, telmesteine, tematropium , temelastine, temiverine, tenidap, tepoxalin, terbogrel, terbucromil, terbutaline, tetrazolast, texacromil, thenaldine, theophylline, thonzylamine, tiacrilast, tibenelast, ticolubant, tiflamizole, tilbroquinol, tiliquinol, tinazoline, tiotropium, tioxamast, tipepidine, tiprinast, tixanox, tobramycin, tobuterol, tolafentrine, tolazoline, tolpropamine, tomelukast, torbafylline, tranilast, tranilast, traxanox, triamcinolone, triamcinolone acetonide, trifenagrel, tripolidine, tritoqualine, troleandomycin, tulobuterol, tulopafant, tyloxaphol, ularitide, uridine 5'-triphosphate, urushiol, verlukast, verofylline, vinpocetine, vofopitant, xamoterol, xanoxic acid, zafirlukast, zanamivir, zaprinast, zardaverine, zileuton, zilpaterol, zindotrine, zinterol, zipeprol, zolamine, A-277249, A-64077, A-76745, A-78773, A-78773, A-79175, A-85761, AA-861, ABT-761, AH-21132, AMG-126737, APC-366, AR-C68397, AR-C89855, AR-D111421, AWD-12-281, AWD-12-343, AY-0068, BA-679-BR, BAY-19-8004, BAY-19-9996, BAY-K-8644, BAY-U-3405, BAY-U-9773, BAY-X-1005, BAY-X-7195, BAY-X-9773, BBR-2221, BBR-2222, BIIC-1996, BIIL-284, BIIX-1, BIO-1211, BI-RM-270, BIRZ-227, BM-113, BM-16.2115, BOM-1006, BRL-52974, BPC-15, BW-755C, BWA-78U, BY-217, CDP-840, CDS-90910, CE-2072, CE-1037, CGH-2466, CGH-2466, CGP-45715A, CGP-49823, CGP-69669A, CGP-73400, CGS-21680, CGS-25019C, CH-13584, CI-1018, CI-1044, CMI-392, CMI-977, CP-105696, CP-195494, CP-195543, CP-199330, CP-199331, CP-220629, CP-288886, CP-293121, CP-80633, CP-99994, CPX, CS-560, CS-615, CT-5219, CT-747, DAX, D-22888, D-4396, D-4418, D-58, DCF-987, DF-1681, DMP-777, DPC657, E-25, E-3040, EF-40, EPI-2010, ER-27319, ETH-615, F-1322, FK-224, FK-355, FK-888, FPL-55712, FR-134043, FR-184280, FUB-593, GR-213487, GT-261163X, GW-215864, GW-250495, GW-311616A, GW-328267, HMR-4011, HMR-4011A, HP-3, ICI-198615, ICI-204219, ICI-211965, ICI-D2138, ICI-200355, IDEC-152, IL-4-DM, INS-365, INS-37217, IPL-4088, IPL-576092, IVL-745, J-104129, K-5993, KAA-276, L-615919, L-648051, L-649923, L-651392, L-689065, L-658758, L-691816, L-697198, L-699333, L-708780, L-733321, L-739010, L-746530, L-743986, L-826141, LAS-31025, LDP-977, LM-1484, LM-1507.NA, LY-170680, LY-171883, LY-213024, LY-210073, LY-223982, LY-233469, LY-255283, LY-264086, LY-292728, LY-293111, LY-303870, MAFP, MDL-105212A, MDL-105172A, MDL-108207DA, MEN-10627, MEN-11467, MJ-451, MK-0476, MK-0591, MK-0679, MK-571, MK-679, MK-886, MKS-492, ML-3000, ML-3176, MOL-294, MPB-07, MPB-07, MRS-1523, NA-00226A, NA-00226B, NCS-613, NCS-630, NCS-631, NCS-632, NIP-520, NKP-608C, NPC-18915, OC-229-648, ONO-LB457, ONO-LB448, ONO-1078, ONO-4057, ONO-5046, ONO-6818, OR-1958, ORG-20241, ORG-30029, PA-1806, PF-5901, PF-10042, PNU-142731A, PR-001337, R-113281, R-133663, R-146225, R-840, RK-0202, REV-5901, RG-12,525, RG-12525, RO-20-1724, RP-23618, RP-64966, RP-66153, RP-73401, RPR-106541, RPR-106541, RPR-114597, RPR-122818, RPR-132294, RPR-132703, RS-601, RS-635, RU-486, RWJ-2048, RWJ-63556, S-1319, S-1674, S-2474, SB 683698 , SB-201146, SB-209247, SB-205312, SB-207499, SB-209247, SB-223412, SB-227122, SB-244525, SB-332235, S-36496, SC-41930, SC-53228, SC-50605, SC-51146, SC-53228, SCH-205528, SCH-217048, SCH-351591, SCH-55700, SCH-60059, SIL-13RA2-FC, SKF-104353, SHF-104493, SKF-106203, SKF-94836, SKF-96231, SM-15178, SR-140333, SR-144190, SR-48212, SR-48968, SYN-1390, SYN-1396, T-0757, T-2585, TA-270, TAK-661, TASP-V, TBC-1269, TBC-3342, TBC-3586, TEI-8362, TEI-9874, TMC-120B, TP10, TMK-688, TRK-851, TR-14531, TR-9109, U-50488-H, U-62066E, U-875302, V-11294, VB-5122, VML-530, VUF-5087, VUFB-19363, VUF-K-8707, VUF-K-8788, WAY-121006, WHI-P180, WHI-P97, Y-27632, YM-38336, YM-44778, YM-49244, YM-976, ZD-0892, ZD-2138, ZD-4407, ZD-6021, ZD-8321, ZM-230487, ZM-274773, YM-976
GROUP 6A:
4-amino-salicylic acid, 5-hydroxy-omeprazole, aceglutamide, acetorphan, acetoxolone, aldioxa, alemcinal, alizapride, alkofanone, alosetron, amiglumide, amoxicillin, arbaprostil, asperlicin, azacitidine, azasetron, azithromycin, bemesetron, benexate, benzotript, bergenin, bisfentidine, bromcresol green, bromcresol purple, budesonide, buzepide, carbenoxolone, casofekamide, catechin, cetraxate, cilansetron, cimetidine, cinitapride, cisapride, clarithromycin, dalcotidine, darenzepine, darifenacin, deboxamet, deprostil, devazepide, devazepide, dexloxiglumide, didanosine, difenoxin, dimeticone, diphenoxylate, dolasetron, donetidine, dosmalfate, ebrotidine, ecabapide, ecabapide, ecabet, egualen, enprostil, esaprazole, esomeprazole, etintidine, exisulind, fabesetron, famotidine, famotidine, fedotozine, fenoctimin, galdansetron, gefarnate, goodyeroside A, granisetron, idremcinal, indisetron, irsogladine, irsogladine, isotiquimide, itasetron, itopride, itriglumide, lafutidine, lagatide , lamivudine, lamtidine, lansoprazole, lavoltidine, leminoprazole, lerisetron, letrazurile, lidamidine, lintitript, lirexapride, loperamide, lorglumide, loxiglumide, lozilurea, lupitidine, lurosetron, mebiquine, meciadanol, 6-mercaptopurine, mesalamine, mesalazine, mesalazine., methylnaltrexone, metoclopramide, metronidazole, mexiprostil, mifentidine, mirisetron, mosapride, mosapride, mosapride, nepaprazole, niperotidine, nizatidine, nolinium, norcisapride , norcisapride, nuvenzepine, octeotride, olsalazine, olsalazine, omeprazole, omeprazole sulphone, ondansetron, ornoprostil, osutidine, oxmetidine, palonosetron, pantoprazole, phenolphthalein, piboserod, pibutidine, picoprazole, pifarnine, pipratecol, pirenzepine, pirenzepine, plaunotol, porfimer, pranazepide, proglumide, prucalopride, pumaprazole, rabeprazole, racecadotril, ramixotidine, ramosetron, ranitidine, rebamipide, remiprostol, renzapride, ricasetron, ridogrel, rifaximin, rioprostil, rispenzepine, rofleponide, rosaprostol, rotraxate, roxatidine, saviprazole, siltenzepine, simeticone, sofalcone, somatostatin, sopromidine, spiriprostil, spiroglumide, spizofurone, sucralfate, sucrosofate, sufotidine, sulamserod, sulfasalazine, sulisatin, tarazepide, tazofelone, tegaserod, tegaserod, telenzepine, telenzepine, tenatoprazole, teprenone, thalidomide, ticalopride, timoprazole, tinidazole, tiotidine, tizanidine, tolimidone, tomoglumide, tranexamic acid, trapencaine, triletide, triletide, trimoprostil, tritiozine, tropisetron, troxipide, tuvatidine, ufiprazole, uzarin, venritidine, zaldaride, zaltidine, zatosetron, zolenzepine, zolenzepine, zolimidine, ε-acetamidocaproic acid, 5-ASA-CYS, A-71378, A-71623, ABT-229, AD-2698, ADL-8-2698, APC-2059, AR-R15849, AT-61, ATL-2502, AU-006, AU-130, AU-224, AU-413, AU-461, BIMU-8, BILN-303-SE, BXT-51072, BXT-51108, BY-112, CA-1028, CAM-CAM-1028, 1481, CDC-801, CDP-571, CHF-17454, CJ-11974, CI-988, CP-212454, CP-122721, CR-1392, CR-1409, CR-1505, CR-2345, CR-2622, CR-2945, CR-2194, DA-3934, DA-6034, DA-9601, DA-9601, DZ-3514, E-3620, EB-3810, EM-574, FK-480, FK-1052, FR-127519, FR-145715, FR-180102, FR-182024, FR-193879, FR-208419, G-009, GR-205171, GM-611, GS-332, GW-5823, GW-7178, H199, H-335/25, HC-62, HC-70II, HC-74, HP-0310, ICM-3, IQM-95333, ISIS-2302, ISIS-14803, ISIS-22023, IT-9302, IY-81149, J-104129, J-106366, JB-9315, JK-1085, KC-11458, L-364718, L-365260, L-368730, L-368935, L-740093, L-754030, LDP-02, LY-206890, LY-219057, LY-246736, LY-315535, LY-316108, MK-329, MKC-733, NABI-3700001, NE-0080, OP-2000, OTC SB-300, P371A1, PA53, PD-134308, PD-135158, PD-138450, PD-140548, PTR-3046, Q-33160A, R-116712, RO-32-6167, RO-48-5695, RP-69758, RP-73870, S-15176, SB-207266, SC-50998, SCH-28080, SCH-33405, SDZ-HTF-919, SHANVAC-B, SK-896, SK-951, SKK-47029, SKF-96067, SKF-97574, SL-65.0102, SLV-305, SPI-447, SR-27897, SR-27897B, SR-58611, T-0632, T-0970, TA-510, TP-1202, TP-680, TR-14035, TS-951, TT-S24, VRT-21493, XYZ-033MP, Y-34867, Y-34959, Y-36912, YH-1885, YJA-20379, YJA-20379-5, YJA-20379-8, YM-022, YM-114, YM-53389, YNS-15P, Z-338, ZNC-2381, Y-905, YNS-15P,
GROUP 7A:
17β-estradiol, 1-docosanol, 3-allylfarnesol, 7-morpholinomethyltheophylline, abarelix, acefylline, acetazolamide, alfuzosin, alprostadil, altizide, alverine, amanozine, ambuside, amikacin, amiloride, amitriptyline, apomorphine, arbutin, atosiban, atropine, azosemide, baclofen, bendroflumethiazide, benzthiazide, bethanechol, bexlosteride, botulinum toxin A, bumetanide, butizide, candesartan cilexetil, canrenoic acid, cetrorelix, chloraminophenamide, chlorazanil, chlorothiazide, ciprofloxacin, clenbuterol, clinafloxacin, clindamycin, clofenamide, clopamide, clorexolone, cyclopenthiazide, cyclothiazide, dantrolene, darifenacin, deacetylmoxisylyte, desacetylspironolactone, 6β-idrossi-7a-tiometil spironolactone, desipramine, desmopressin, desogestrel, dicycloverine, dicyclomine, dienestrol, dihydrotestosterone , disulfamide, doxazosin, doxepin, doxercalciferol, doxiverin, drospirenone, duloxetine, dutasteride, eflornithine, emepronium, ephedrine, epitizide, eplerenone, epristeride, estradiol, estriol, ethacrynic acid, ethiazide, ethinylestradiol, ethoxzolamide, etonogestrel, etozolin, fenquizone, fesoterodine, fiduxosin, finasteride, flavoxate, flumethiazide, fluoxetine, flurbiprofen, fosfomycin, furosemide, ganirelix acetate, gatifloxacin, gestonorone, hydracarbazine, hydrochlorothiazide, hydroflumethiazide, hyoscyamine, hyoscine, imidapril, imipramine, inaperisone, indapamide, indometacin, ipratropium, isosorbide, izonsteride, lanperisone, loperamide, losartan, mannitol, mebeverine, mefruside, mercaptamine, mepartricin, mespirenone, mesterolone, methazolamide, methocalcone, methyclothiazide, methyltestosterone, meticrane, metolazone, midodrine, midodrine, moxisylyte, muzolimine, naftopidil, naftopidil, nepadutant, nifedipine, niguldipine, nitrofurantoin, norethindrone , norfloxacin, nortriptyline, ofloxacin, oleandrin, osanetant, osaterone, oxendolone, oxybutynin, oxybutynin, papaverine, paroxetine, perhexiline, phenoxybenzamine, phentolamine, phenylephrine, phenylpropanolamine, pimagedine, pinacidil, piretanide, piretanide, polythiazide, prasterone, prazosin, propantheline, propiverine, prostaglandin E₁, prostaglandin F_{2α}, prostavasin, protheobromine, pseudoephedrine, quinethazone, resiniferatoxin, roxithromycin, salbutamol, saredutant, sevelamer, sildenafil, sirolimus, suplatast, tacrolimus, tacrolimus, talnetant, tamsulosin, teclothiazide, temiverine, terazosin, terbutaline, terodiline, testosterone, theobromine, tibolone, ticrynafen, tolterodine, torsemide, trans-retinoic acid, trazodone, tretinoin, triamterene, trichlormethiazide, trimegestone, tripamide, triptorelin, troglitazone, trospium, turosteride, upidosin, vamicamide , vardenafil, vardenafil, xenoxin-1, xipamide, yohimbine, A-176120, A-197574, ABT-232, ABT-839, ABT-866, ABT-980, AH-9700, AIO-8507L, ALRT-4204, ALRT-4326, AZD0947, AZD5106, BAY-38-9456, BMS-214662, BMS-284756/T-3811ME, BMS-341400, BMY-7378, CDB-4124, Chi-MrIA, CL-385004, CS-891, CV787, DA-8159, DecbSM, DV-182, DWH-146e, E-4010, E-8010, EMD-221829, ESR-1150, FK-176, FK-453, FR-119680, FR-149175, FR-229934, GI-198745, GS-332, GW-419458, GYKI-16084, HP-4, HP-7, HY-770U, IC-351, IM-46068, ISIS-24195, J-811, JTP-20993, K-4610178, K-4610422, KCO-616, KRP-197, KW-3902, KW-7158, L-372662, L-374943, L-757464, L-771688, L-778123, L-780945, L-788123, LG-120746, LG-121071, LG-121104, LU25-109, LU-25-109, LU-302872, MEDI-491, MEDI-501, MEDI-503, MEDI-504, melanotan II, MEN-10627, MK-826-, MX-6, NC-1800, NK-433, NMI-187, NMI-221, NMI-678-11, NNC-45-0781, NS-21, NS-49, NS-8, NZ-419, ONO-5816,ONO-8815, OPC-41061, OPC-51803, Org-30659, PNU-157706, PNU-156765, PNU-156804, PNU-200577, PT-14, R-115777, RCC-36, RCC-36, Rec-15/2615, Rho-TIA, RO-60-0332, RO-70-0004, RPR-100579, RPR-130401, RS-100329, RSD-992, RU-58642, RWJ-38063-, SB-223412, Sch-66336, SGN-00101, SL-5770499, SL-251039, SNA-4606-1, SNAP-5089, SNAP-5399, SNAP-6201, SNAP-6383, SNAP-5540, SNAP-6543, SNAP-6552, SNAP-6991, SNAP-7242, SNAP-7915, SPM-007, SR-144190, SR-48968, SR-49059, SR-59026A, T-1032, TA-1790, TA-1790, TAK-637, TSE-424, UK-343664, UK-357903, VIPGC-NO, VLM-588, VNA-932, VP-365, WAY-133537, WAY-141608, WAY-151616, YM-31758, YM-32906, YM-36117, YM-58790, YM-905, YM-934, Z-15, Z-350, ZD-0947, ZD-6169, MUDE, EDEX, TOPIGLAN, ALPROX-TD, INVICORP
GROUP 8A:
β sitosterol, α tocopherol, β tocopherol, γ tocopherol, δ tocopherol, ε tocopherol, η tocopherol, α1 sitosterol, ζ₁ tocopherol, ζ₂ tocopherol, α-carotene, β-carotene, γ-carotene, δ-carotene, β-citraurin, γ-sitosterol, β-sitosterol, α-tocopherol, 10-bromopaullone, 10-desacetylbaccatin III, 13-cis-retinoic acid, 16-Methyloxazolomycin, 17-(Allylamino)-17-demethoxygeldanamycin, 2-(2-amino-3-metoxyphenyl)-chromone, 2,2,5,7,8-pentamethyl-6-hydroxy-chromane, 2-hydroxychalcone, 2-methoxyestradiol, 3-allylfarnesol, 3-Aminopyridine-2-carboxaldehyde, 3-deoxysilychristin, 3-hydroxyflavone, 2-phthalimidino glutaric acid, 3,4-didehydroretinoic acid, 3-indolemethanol, 4'-bromoflavone, 4-aminothalidomide, 4-bromothenylguanine, 4-dedimethylaminosancycline, 4-Demethylpenclomedine, 4-hydroxybutirroxymethyl-retinate, 5,6-dimethylxanthenone-4-acetic acid, 5'-hydroxythalidomide , 5-aza-2-deoxycytidine, 5-azacytidine, 5-fluorouracil, 5-hydroxytryptophan, 5-methoxytriptamine, 6-azauridine, 6-gingerol , 6-hydroxymethyl-acylfulvene, 6-mercaptopurine, 6-shogaol , 6-thioguanine, 7-Hydroxycoumarin, 7-hydroxystaurosporine, 9-cis retinoic acid, 9-aminocamptothecin, 9-aminocamptothecin, 9-Hydroxycrisamicin A, 9-nitrobromopaullone, 14-hydroxy-4,14-retroretinol, abarelix, acacetin, aceglatone, acetylharpagide, acetyldinaline, acitretin, acrimarin F, acyfulvene, adefovir, adenine, adozelesin, aeruginosamide, alanine, alanosine, albendazole, aldesleukin, alitretinoin, allantoin, allcis-5,8,11,14,17-eicosapentaenoic acid, allicin, allin, allixin, allopurinol, all-trans retinoic acid, all-trans-4-hydroxyretinol, all-trans-4-hydroxyretinoic acid, all-trans-4-oxoretinol, all-trans-4-oxo-retinoic acid, almuheptolide A, alpha-amyrin, alpha-curcumene, alpha-linolenic-acid, alpha-pinene, alpha-terpinene, altretamine, alsterpaullone, altretamine, amamistatin A, amentoflavone, amifostine, aminoglutethimide, aminopterin, anguillosporal, ampelopsin, amphidinolide T, amrubicin, amsacrine, amygdalin, anastrozole, ancestim, ancitabine, anecortave, anethole, angiopoietins, angiostatin, anhydrovinblastine, annamycin, annomuricin D, annopentocins, anserine, anthocyanins, anthocyanosides, anthranilic acid, antimycin A1, antimycin A3, antineoplaston A10, anvirzel, apigenin, apigetrin, apiin, apiole, aplidine, apocyanin (A, B, C, D), apomine, arabinogalactan, arbutin , arcyriacyanin A, arginine, arglabin, ariculatin, asimin, asimicin, asiminocin, asmarine B, asperuloside, astacin, astaxanthin, astragalin, avrainvillamide, axillarin, azacitidine, azafrin, azaserine, azidodideoxyguanosine, baicalein, baptigenina, batimastat, bengamide, bergapten, beta-alethine, betaine, berberine, beta-ionone, betulin, bexarotene, bicalutamide, bifendati, bilobalide, bilobols, biochanin A, biotin, biricodar, bisantrene, bisnafide, bixin, bizelesin, bohemine, borneol, bornyl-acetate, brassicolene, brassinin, brevicollin, bromelain, bromotaxane, bropirimine, bryostatin 1, buserelin, busulfan, busurelin, bullanin, buthionine, butylidene-phthalide, butyric acid, caffeic acid, caffeic acid, caloporoside, calicheamycin, calusterone, calphostin S, camphene, camphor, camptothecin, canavaninelupeol, canpagin, canstatin, cantharidin, canthaxanthin, canventol, capecitabine, capsaicin, capsanthin, carboplatin, carboxyamidotriazole, cardanols, carmofur, carmustine, carnosic acid, carnosine, carnosol, carnosolic acid, carvacrol, carolin (A, B, C), carvone, caryophyllene, caryophyllene-oxide, carzelesin, carzinophilin, carboquone, catechin, catechin, ceftiofur, cemadotin, centaurein, cercosporin, cetrorelix, chavicol, chalcones, cherimoline, chloptosin, chlormadinone, chlorogenic acid, chlorophyll, chloroquinoxaline sulfonamide, chromocarb, chrysanthemaxanthin, chrysin, chrysoeriol, chlorambucil, chlornaphazine, chlorozotocin, cichoric acid, cilengitide, cineole, cinnamic acid, cipemastat, cirsimaritrin, cirsineol, cis-inochiresinol, cisplatin, citral, citric acid, citrusine, cladribine, clofarabine, cnicin, cnidilide, cobalamin, combretastatin, combretastatin A4, combretastatin A4 phosphate, conophylline, contortrostatin, conyferyl alcol, coporang, cordycepin, cortivazol, cotara, coumarin, coumestrol, crellastatin A, CRM-51005, crocetin, cryptophycin, cryptophycin-1, cryptophycin-52, cryptoxanthin (α, β, γ, δ), curacin A, cuscenol B, curcumin, cyanidin, cyanidin 3 galactoside, cyanidin 3 glucoside, cyanidin 3,5 diglucoside, cyanidin 3-rhamnoglucoside, cyanidin 3-soforoside, cyasterone, cycloartenol, cyclophosphamide, cymene, cystemustine, cytarabine, cytotrienin IV, daidzein, dacarbazine, daidzin, damnacanthal, daphnezomine G, daphnodorin A, datiscetin, daunorubicin, decitabine, declopramide, defosfamide, dehydrodidemnin B, delphinidin, delphinidin 3,5 diglucoside, delta-3-carene, delta-7-sterols, 5,7-dichloroemodin, demecolcine, denileukin diftitox, denopterin, depsipeptide, desmethyleleutherobin, desmopressin, desoxyepothilone a, desoxyepothilone B, cis-2'-deoxy-2'-fluoromehtylene-cytidine, dexamethasone, dexormaplatin, dexrazoxane, detirelix, diazaphilonic acid, diaziquone, dibromodulcitol , diethylnorspermine, diethylstilbestrol monophosphate, diflomotecan, dihydroxycalcitriol, dimefline, dimesna, diosmetin, diosmin, discodermolide, discorhabdin P, diphyllins, docetaxel, dolastatin-10, dolastatin-15, dolostatin, doxifluridine, doxil, doxorubicin, dromostanolone, echinacoside, echinenone, ecteinascidin-757, ecteinascidin-743, edatrexate, edelfosine, edeine, edotreotide, eflornithine, elacridar, elemene, eleutherobin, elinafide, ellagic acid, emfilermin, emitefur, emtricitabine, emodin, endostatin, eniluracil, enloplatin, enocitabine, epicatechin, epigallocatechin, epigallocatechin-3-O-gallate, epipachysamine E, epirubicin, epitiostanol, epothilone (A, B, C, N-oxide, E, D), eptaplatin, equol, erbstastin, eriodictyol, eruboside-b, esculetin, estragole, espelicin, estramustine, ethanidazole, etoglucid, etoposide, etoposide phosphate, etretinate, eugenol, eupatorin, eudistomin H, exatecan, exemestane, exisulind, fadrozole, falcarindiol, fagopyrin, falnidamol, farnesylthiosalicylic acid, fenretinide, ferulic-acid, feruloyl-4-hydroxycinnamoylmethane, fialuridine, finrozole, fisetin, flavin-adeninin-8α-hydroxy-riboflavin, flavine-adenine dinucleotide, flavone-8-acetic acid, flavopiridol, flavoxanthin, floxuridine, fluasterone, fludarabine, fludarabine phosphate, fluoropyrimidine, fluorouracil, flutamide, fluvirucin B2, folic acid, folinic acid, formamicin, formestane, formononetin, fosfestrol, fraxinol, fucoxanthin, fulvestrant, fumagillol, fumagillol chloroacetylcarbamate, fustin, galangin, galarubicin, gallic acid, gallocatechin, galocitabine, gamma-terpinene, ganirelix, gardenin (A, B, C, D, E), geldanamycin, gemcitabine, gemfibrozil, genistein, genistein-7-phosphate, genistin, genkwanin, geldanamycin, gentisic acid, geraniol, gimeracil, goniodonin, ginkgolide A, ginkgolide B, glufosfamide, glutathione, glutethimide, glucose, 1-glucosamine , 2-glucosamine, 6-glucosamine, 2-galattosamine, 1-fructosamine, 2-fructosamine, glyceollin, glyceollin-II, glycine, glycitein, glycitin, gnidimacrin, goserelin, gossypol, griseolic acid, halimide, harmine, harmaline, harmalol, harman, halofuginone, heliquinomycin, herbimycin A, hemiasterlin, hepaxanthin, hesperetin, hesperidin, heteroxylan, hexestrol, hispidulin, histamine, histidinol, homoharringtonine, hycamtin, hypericin, 7,7'-dichlorohypericin, hydrocaffeic acid, α-hydroxyfarnesylphosphonic acid, hydroxymethylacylfulvene, hydroxyurea, 5-hydroxynoracronycine, hycamtin, hyperin, hyperoside, hypocrellin (A, B), hispidospermidine, human endostatin 6-49, idarubicin, ilatreotide, ilomastat, imatinib, imexon, imidacrine, improsulfan, indanocine, indirubin, indole-3-acetic-acid, inosine, inositol, intoplicine, ipriflavone, iproplatin, irigenin, irinotecan, irisquinone, irofulven, iseganan, isatoribine, isochlorogenic acid, isoeugenol, isofraxidin, isoliquiritogenin, isopimpinellin isoquercetin, isoquercitrin, isorhamnetin, isorosmanol, isovitexin, iturelix, justicidin A, kaempferol, kahalalide F, karenitecin, ketotrexate, krestin, kynurenic acid, kushenol (B, H, E, K, N, M), kuraninone, kosamol A, kuraridin, labiatic-acid, lactacystin, ladirubicin, lapachol, lavendustin, I-carnosine, ledoxantrone, lentinan, leridistim, letrozole, leucocyanidin, leucotropin, leucovorin, leuprolide, leuprorelin, leurubicin, liarozole, licocoumarone, licoflavanone, licochalcone A, lignin, limonene, linalol, linarin, linoleic-acid, liquiritigenin, lisofylline, lobaplatin, lobradimil, lometrexol, lonidamine, losoxantrone, luminacin D, lupeol, lurtotecan, lutein, luteolin, luteolin-7-glucoside, luzindole, lycopene, lycobetaine, lycophyll, lycoxanthin, macrocarpin A, malvidine 3 glucoside, malvidine 3,5 diglucoside, mammastatin, mannomustine, marimastat, maslinic-acid, maspin, mechlorethamine, megestrol, melatonin, melengestrol, melphalan, menadiol, menogaril, menthone, mepitiostane, mesna, meshima-ex, methotrexate, 1-methoxyagroclavine, 3-methoxyquercetin, miboplatin, midostaurin, mifepristone, migrastatin, miltefosine, mirostipen, mispyric acid, mitalactol, mitobronitol, mitoguazone, mitolactol, mitomycin C, mitotane, mitoxantrone, mivobulin, mofarotene, mopidamol, morin, motretinide, myo-inositol, myrcene ,myroridin K, myricetin, myristicin, 14-methylpentadecanoic acid, 16.methyllinoleic acid methyl ester, N-(2 hydroxyethyl)-retinamide, N-(2 hydroxyphenyl)-retinamide, N-(2-carboxyphenyl)retinamide, N-(4-carboxyphenyl)-retinamide, N-(4-carboxyphenyl)-retinamide, N-(ethyl)retinamide, N-(4-etoxycarbonylphenyl)-retinamide, N-(4-hydroxyphenyl)-retinamide, N-(4-hydroxyphenyl)-retinamide-O-glucoronide, naamidine A, N-acetyl-5-hydroxytriptamine, naringenin, naringin, nedaplatin, nemorubicin, neohesperidin, neplanocin A, neparensinol B, nepetrin, N-hydroxymethyl-thalidomide, nicardipine, N-hydroxyphthalimide, nicotinamide, nilutamide, nimustine, nitracrine, nobiletin, nolatrexed, nonataxel, nordihydroguaiaretic acid, noscapine, novembicin, O-6-benzylguanine, octreotide, oleanolic-acid, oliverine, olomoucine, oltipraz, onapristone, oncocidin A1, oprelvekin, orientin, ormaplatin, orotic acid, oroxylin, oteracil, oxaliplatin, oxostephanine, ozogamicin, paclitaxel, pachystaudine, pancratistatin phosphate, paeciloquinone A, pantothenic acid, pantothenic acid, p-coumaric acid, pavetannin B1, p-cymene, panorex, pectolinarigenin, pelargonidin, pelargonidin 3 glucoside , pelargonidin 3,5 diglucoside, peloruside a, peltatins, pemetrexed, Penicillamine, Pentacea, pentamidine, pentetreotide, pentostatin, peonidin, perifosine, perillyl alcohol, petunidin, phenamet, phenesterine, phenylahistin, phenylbutyrate, phloretin, phloroglucinol, phosphatidyl-choline, phthalascidin, p-hydroxybenzoic-acid, phytic-acid, pibrozelesin, picibanil, piceatannol, pifithrin-A, pinitol, pirarubicin, piritrexim, pironetin, piroxantrone, podophyllinic acid, podophyllotoxin, porfimer, pratensein, prednimustine, prinomastat, proanthocyanidins, probenecid, procarbazine, progenipoietin G, propionic acid, protocatechuic acid, prunetin, prunellin, psoralen, pteropterin, purpurogallin, pyrazoloacridine , pyridoxine, pyrisulfoxin a, pyroxamide, Q-12713, quercetagetin, quercetin, quercetin -O-β-D-galactoside, quercetin-3-galactoside, quercetin-3-rhamnoglycoside, quercimeritrin, quercitrin, raltitrexed, ranimustine, ranpirnase, razoxane, radicicol, rebeccamycin, reblastatin, repifermin, resveratrol, retinal, retinilhydroxymethylether, retinol, rhamnetin, rhodomycin, riboflavin, riboflavin monophosphate, riboflavin tetrabutyrate , riboflavin triacetate, rhinacanthin (C, D), robinetin, robinin, rohitukine, roquinimex, rosmarinic acid, roscovitine, rosmadial, rosmanol, rosmaridiphenol, rosmarinic acid, rosmariquinone, rubitecan , rubixanthin, rutin, safingol, safrole, sakuranetin, salicylic-acid saponin, salicylihalamide A, sanguinarine, s-allyl-I-cysteine, saponaretin, saponins , sarcodictyin A, sarcophytol A, sardomozide, sargramostim, saskatchewan, satraplatin, scoparin, scopoletin, scopafungin, scutellarein, schizophyllan, scytonemin, sebriplatin, secobatzelline A, sedoxantrone, selenocysteine, selenomethionine, sensamide, serotonin, shermilamine D, sigmastanol, silandrin, silybin, silychristin, silydianin, silymarin, silymonin, sinapic acid, sitosterol, sizofiran, sobuzoxane, soblidotin, solasodine, solimastat, somatostatin, sophoricoside, sophoraflavone G, spiroplatin, sparfosic acid, spisulosine, spongistatin, spinencin, squalamine, β-tethymustine, staurosporine, stigmasterol, streptonigrin, streptothricin B, streptozocin, suberanilohydroxamic acid, sulfuretin , sulidac solfone, supidimide, suradista, suramin, syringaldehyde, syringic acid, taglutimide, talaporfin, taiwanhomoflavone A, tamandarin A, tannic acid, tannin, tanomastat, tasonermin, taurine, taurolidine, tautomycin, taxifolin, taxol, tazarotene, tecogalan, tectorigenin, tegafur, teloxantrone, temozolomide, tengeretin, teniposide, tenuazonic acid, terpinen-4-ol, tesmilifene, testolactone, tetrahydrocortisol, tetramethylpyrazine, tetrandrine, tetrocarcin A, tezacitabine, thalidomide, theaflavin, theaflavine, thielavin B, thiamine, thiamiprine, thionine, thyrsiferyl 23-acetate, thioctic acid, thioguanine, thymalfasin, thymol , tipifarnib, tiazofurin, timcodar, tirapazamine, tirazone, tirilazad, tocladesine, tocol, tonkinecin, tokaramide A, tomudex, topostatin, topotecan, torularhodin, trans ferulic acid, trapoxin b, triaziquone, tributyrin, trichostatin (A, B, C, D), tridolgosir, triethylenemelamine, trigonelline , trilostane, trimetrexate, trimidox, triptorelin, triptofordin C2, trolox C, troponin I, troxacitabine, troxerutin, trunkamide A, tryptamine, tryptophan, tubercidin, tubulysin A, tumerone, tumstatin, tyrphostin, ubenimex, umbelliferone, uncarinic acid A, uracil mustard, urethan, ursolic acid, valereinic acid , valeric acid, valrubicin, valspodar, vanillic acid, vanillin, vapreotide, vatalanib, verapamil, verteporfin, vesnarinone, vinblastine, vincristine, vindesine, vinflunine, vinorelbine, violaxanthin, vitamin a2, vitamin E acid succinate, vitexin , vitilevuamide, virulizin, vorozole, wogonin, xanthophyll, xanthotoxin, xanthotoxol, xenomin 1, zacopride, zalcitabine, zeaxanthin, zeniplatin, ziconotide, zingerone, zingibain , zinostatin, zorubicin, 506U78, 5F-203, 6-MCDF, 6-MPG, 776C85, A-007, A-105972, A-176120, A-125800, A-177430, A-177775, A-197574, A-198401, A-228839, A-316023, ABT-518, ABT-627, ABT-839, AC-7700, AC-9301, ACRP30R2, AD-198, AE-941, AG-99, AG-12275, AG-12286, AG-1288, AG-1478, AG-18, AG-2034, AG-2037, AG-3340, AG-3433, AG-494, AG-555, AG-7352, AG-825, AG-879, AGN-190168, AGN-193109, AGM-1883, AGR-129, AH-12, AH-27, AH-30, AH-7, AL-245, AL-6, AM-580, AMD-473, AMD-3100, AMP-404, AN-207, AN-238, APC-8015, APC-8024, AR54-, AR5LAC20, ARH460--23, AS5-2H, AT-5015, AVI-4126, AZD-3409, AZD-6474, B-317, B-429, B-581, B-956, B-1086, BAL-9602, BAM-002, BAY-12-9166, BAY-12-9566, BAY-38-3441, BAY-43-9006, BB-1277, BB-21295, BB-67357, BB-76163, BB-2516, BB-3644, BB-94, BBL22, BBR-2778, BBR-3438, BBR-3464, BBR-3576, BBR-3610, BE-41956A, BE-43547A1, BE-45985X, BE-54238A, BE-55051, BE-56384, BE-56980, BE-60828, BE-67251, BEC2, BGP-15, BIBH-1, BIBX-1382, BIM-46068, BIWA4, BLP-25, BLS-0597, BMD-188, BMP-6, BMS-27291, BMS-181174, BMS-182751, BMS-184476, BMS-184477, BMS-186511, BMS-188797, BMS-200659, BMS-214662, BMS-247550, BMS-247615, BMS-247616, BMS-265246, BMS-275291, BN-80245, BN-80915, BN-80927, BNP-1100, BNP-1350, BNP-7787, BP-04, BP-1, BPHA, BR-630, BSU-1075, BSU-9057, BZA-2B, BZA-5B, C-1027, C-1311, C-75, CB-96, CB-300907, CB-300919, CB-30865, CB-7646, CC-3052, CC-4047, CC-5013, CC-7085, CCI-779, CD-1599, CD-437, CD-2314, CDC-501, CDC-801, CEP-2563, CEP-701, CGP-41251, CGP-48664, CGP-52608, CGP-74514, CGP-79787, CGP-79807, CGS20267, CGS-24592, CGS-27023A, CHAP-31, CHAP-81, CHS-828, CHT-22, CI-1006, CI-1021, CI-1027, CI-1031, CI-1033, CI-1042, CI-958, CI-980, CI-994, CJ-11974, CKD-602, CKD-731, CL-387785, CM101, CM-101, CMA-676, CMB401, CN-706, CN-787, COL-3, CP-1, CP-248, CP-263114, CP-358744, CP-358774, CP-461, CP-609754, CPC-405, CPD-5, CPR-2003, CPR-2005, CPR-3005, CPT-111, CRL-1005, CRL-8246, CRL-8256, CRM-51005, CRM-646-A, CSA, CS-682, CT-052923, CT-17, CT-2103, CT-2584, CT-2584, CTP-37, CV706, CV-787, CWJ-A-5, CYA-246, CYS-3-AMBA-Met, D-2163, D-24241, D-24851, D-609, DAB389IL-7, DB-67, DB-81, DC-TA-46, DDE-131, DDE-261, DDE-313, DDE-380, DE-310, DJS-811, DOX-GA3, DP-242/3-, DP-71, DR-70, DT388-GM-CSF, DTI-015, DTPA-BRE-3, DW-2143, DW-2282, DX-8951F, DZ-3358, E21R, E-350, E-3620, E6-BP, E-7070, E-7869, EB-1089, ED-501, EDP-137, EG009, EKB-569, ELF3, EM-12, EM-800, EMD-1211974, EO-9, EPO-906, ERA-923, ER-34617, ER-35794, ER-38925, ER-68203, ER-68203-00, ERA-923, ES-936, ET-743, ETM-775, F-11782, FB-642, FC-2.15, FCE-28948, FCE-29381, FCE-29771, FD-594, FDUMP, FDU-NOAC, FE-200486, FE-200486,, FE-399, FJ-5002, FK317, FPP-33, FR-143430, FR-182877, FR-186054, FR-901228, FRI-20, FT-ALZ, FTI-2148, FTI-2153, FTI-276, FTI-277, FY21-AA09, GI-198745, G-207, G-3139, GBC-590, GF-120918, GD0039, GEM-231, GEX1Q5, GF-109203X, GFB-111, GFMO-1, GGTI-2166, GGTI-298, GI-129471, GL-331, GLI-328, GM2-KLH/QS-21, GM-95, GMK, GnRH-III, GPI-0100, GR-891, GR-128107, GR-135533, GRN-5384, GSDI-126, GTI-2040, GTI-2501, GTX-006, GV-1301, GVH-313, GW-1843, GW-2059, GW-395058, GW-5181, GW572016, GW-8510, GW-9499, GW-974, H-10, HA-1, HA-14-1, HI-368, HPKCI, HSCS-1, HSPE-7, HSPPC-96, HWL-12, HX-600, HYB-0432, HYB-165, IB-367, IIK7, IB-96212, ICI-164384, ICI-245991, IDEC-Y2B8, IDN-5005, IDN-5109, ILX23-7553, ILX-295501, ILX-651, IM-46068, IM862, IMC-C225, INGN-201, INGN-241, INGN-251, INX-3001, INX-3280, INXC-6295, IRX-2, ISIS-10639, ISIS-12539, ISIS-12884, ISIS-14864, ISIS-15999, ISIS-16009, ISIS-16518, ISIS-16601, ISIS-16609, ISIS-16834, ISIS-17509, ISIS-19387, ISIS-20772, ISIS-21028, ISIS-22783, ISIS-23722, ISIS-23905, ISIS-2503, ISIS-25513, ISIS-27067, ISIS-28313, ISIS-28949, ISIS-3521, ISIS-4189, ISIS-5132, ISIS-5132, ISIS-7597, J-104134, J-104871, J-107088, J-109404, JBT-3002, JF-05-59, JK-1624S02, JKC-362, JM-216, JMV-1802, JTE-522, K-135, K-252a, K-22387, KAN-108308, KAN-104141, KF-22678, KF-25706, KF-41399, KF-58333, KHPC-10, KI-6783, KM-043, KNK-437, KOCH-5, KR-30035, KRN-5500, KRN-7000, KRN-8602, KS-505a, KT-5720, KT-6149, KT-6352, KW-2170, L-377202, L-39, L-452958, L-651582, L-731734, L-731735, L-739749, L-739750, L-744832, L-745631, L-778123, L-779450, L-779575, L-783277, LB-42908, LCS-640, LDI-200, LDP-341, LFM-A12, LFM-A13, LG-120744, LG-120753, LG-190119, LGD-1069, LGD-1550, LM-4108, LMB-2, LMB-7, LMS-83177, L-NDDP, LU-103793, LU-223651, LY-231514, LY-294002, LY-309887, LY-320236, LY-329146, LY-333531, LY-335979, LY-355703, MAG-283, MCC, MC-1473, MDL-101731, MDL-101986, MDL-73811, MDL-73811, MDX-11, MDX-210, MDX-220, MDX-447, MDX-H210, MEDI-503, MEDI-507, MEDI-517, MEN-10710, MEN-11066, MEN-11951, MER-1020DA, MER-WF1726, MG-132, MG98, MGI-114, MGV, MIBG, MK-4, MK571, MK-869, MK-886, MKT-077, MI-1544, MI-1892, MM-36, MMI-270, MPC-7869, M-PGA, MPI-5020, MPV-2213AD, MR-20492, MS-209, MS-247, MS-27-275, MS-275, MS-325, MSI-1857, MTP-PE, MUP-98176, MV9411, MX6, MT965-A, MT965-B, N276-15, NA-22598B1, NAMI-A, NB-506, NB-1011, NBI-3001, NC100150, NCA-0424, NCO-700, NF-062, NF-06307, NG-60, NK-611, NIK-250, NHTS, NLCQ-1, NM-3, NS-398, NSC-314622, NSC-330507, NSC-354258, NSC-614491, NSC-614491, NSC-649488, NSC-655649, NSC-658875, NSC-674495, NSC-676632, NSC-678515, NSC-686288, NSC-693569, NSC-693632, NSC-7103505, NSC-D699127, NU/ICRF-505, NU:UB-31, NU:UB-73, NU-2058, NU-3076, NU-3121, NV1020, NX-1838, NX-211, OC-1012, OC-144-093, ODN-2009, ODN-698, ODN-83, OGT-719, ON-5543, ONO-4007, ONO-8711, ONYX-015, ONYX-838, OPB-3206, ORI-1313, OSI-774, P-4055, PADP, PAM4, PAK-104, PAK-104P, PD-089828, PD-145709, PD-0174073, PD-0183812, PD-090560, PD-098059, PD-128763, PD-153035, PD-158780, PD-160678, PD-166285, PD-166866, PD-168393, PD-173074, PD-173955, PD-173956, PD-180970, PD-183805, PD-184352, PI-88, PI-CA91, PKC-412, PKI-166, PLQ-22, PNU 145156E, PNU-144113, PNU 153429, PNU-157914, PNU-157977, PNU-159548, PNU-166196, PNU-177496, PP-33, PR-39, PR-69, PRO-533, PS-341, PS-724, PS-990, PSC-833, PSP-94, PT-523, PT-624, PTK-787, PX12, P-X-1544, Q-85, QM16-A, QS-21, QW-1624F2-2, R-101933, R-109339, R115777, R-116010, R123942, RB94, RK-0202, RL-0903, RMP-7, RO-09-4889, RO-25-4020, RO-25-6760, RO-25-9716, RO-28-2653, RO-31-7453, RO-31-7453, RO-41-5253, RO-50-4258, RO-65-7674, RP-527, RPR-108518, RPR-108514A, RPR-109881, RPR-115781, RPR-116258A, RPR-130401, RSR13, S-1127, S-16020-2, S179D-PRL, S-20928, S-20929, S-23906-1, S-30372, S-30972-1, S-3304, S-8184, S-836, SAHA, SAM-484, SB 249553, SB-203580, SB-209763, SB-238039, SB-247464, SB-251353, SB-408075, SB 596168, SBAS2, SBR-11-2897, SBR-11-3702, SBR-170-, SB-RA-131012, SB-T-1213, SB-T-1250, SB-T-101187, SC-1, SC-68448, SC-72115, SC-AAD9, SCH-207758, SCH-56396, SCH-58500, SCH-66336, SCHL-705, SD-7, SGI-101, SGN-10, SGN-15, SH-207, SJG-136, SL11047, SL-11093, SN-38, SMT-487, SN-38, SNF-4435C,SQ-67454, SP-1053C, SPC8490, SPD-424, SPIKET-P, SR 2789, SR 27897, SR-271425, SR-271425-, SR29142, SR-29142, SR-45023A, SR-4895, SR-49059, SRL-172, SS-555, ST-1481, STA-BX909, STI-412, STI-481, STI-571, SU-0879, SU-101, SU-1433, SU-1498, SU4312, SU-4793, SU-4942, SU-4984, SU-5204, SU-5212, SU-5402, SU-5406, SU-5416, SU-5477, SU-5614, SU-6668, SU-9516, SU-9902, SW-163E, T12, T-138067, T-2591, T-3782, T-607, T-64, T-67, T-900607, T-904064, T-98475, TA-2516, TA-2620-, TAS-103, TAS106, TER-117, TER-199, TER-286, TG-1031, TIP-19.40, TJN-151, TLC-144, TLC-D-99, TLC-ELL-12, TLK-199, TLK286, TMC-169, TMC-96, TNP-470, TOP-53, TRK-710, TTNPB, TT-2, TT-232, TX-1123, TX-1920, TXD-258, TZT-1027, U-0126, UCN-01, UCS-1025A-, UCT-1072M1, V-93, VGA1102, VLTS-587, VN/85-1, VN/87-1, VNP-20009, VNP40101M, VP-16, VUF-5000, VUF-5434, VX-710, VX-853, WAY-170523, WHI-D11, WHI-D12, WHI-P154, WHI-P273, WIN-61651, WMC-26, WP-631, WP-745, WP-769, WR-1065, WT-126-34, WX-293T, XK-469, XR-11576, XR-3054, XR5000, XR-5944, XR-9576, YH-137, YH-138, YM294, YM511, YM529, YMB-6H9, ZD-0473, ZD-1626, ZD-1839, ZD-2767, ZD-4190, ZD-6126, ZD-6474, ZD-9063P, ZD-9331, ZK-119010, ZK-112993, ZK-191703, ZK-222584, ZK-229561, ZK-230211, ZM-445526, ZNPCF64, IRESSA, NEOQUIN

7. Compounds according to claims 1-6 in which -F is chosen among the following 8 groups of molecules (from 1 B to 8B) which are identified by their own structural formula or by their own INN ("International Nonproprietary Name" from "World Health Organization" database) or by their own company code or by their own chemical name.
GROUP 1B:
ABT-963, α-tocopherol, 8α-hydroxy-riboflavin, aceclofenac, acemetacin, actarit, alclofenac, alclometasone, AGIX-4207, ajulemic acid (EL), amebucort, amelometasone, amiprilose, ampiroxicam, amtolmetin guacil, anakinra, anirolac, anisperimus, aprepitant, aspirin (ECI), AR-C73346XX (ECX), ataquimast (ECLX), atiprimod, atizoram, atreleuton, balsalazide (ECXVIII), bendazac, benorylate, benoxaprofen, benzydamine (ELX), bermoprofen, beclomethasone, betamethasone, betamethasone acibutate, bindarit, brequinar, BF-389 (EXLVIII), bromfenac, bucillamine, bucillamine, budesonide, bufexamac, bumadizone, butixocort, CB-2431, CC-1069, CC-1088, CEP-1347, celecoxib (El) and 2 metabolites of it ((EII), (EIII)), chenodeoxycholic acid, chloroquine, ciclesonide, cinaproxen, cinmetacin, clidanac, clobuzarit, clofenamic acid, CGS-33090A, CLX-0921, CLX-120500, CK-135, COX-189, CS-502, cloticasone, CR-3558, cridanimod, cyclosporin A, darbufelone (EXLV), deflazacort, dehydrocholic acid, deltibant, deracoxib (EXIII), descinolone, desonide, dexamethasone (ECXC), dexketoprofen, dexamethasone acefurate, dexamethasone-21-phosphate, dexbudesonide, dexpemedolac, dextiopronin, diacerein, diclofenac (ECXIII), diflunisal, dipyrone, DPC-333, droxicam, erlotinib, esonarimod (XXXIV), etodolac (ECXVI), etofenamate, etoricoxib (EXI), E-5110 (EXLVI), everolimus, ezlopitant, fampridine (ELXI), felbinac, fenbufen, fenleuton, fentiazac, figopitant, flavine-adenine dinucleotide, flobufen (ECXVII), flosulide (EXXII), fluazacort, flufenamic acid, flunisolide, flurbiprofen (ECIV), fluticasone, fluocortin, FR-228352 (EVI), FTY-720 (ECL), ganaxolone, glucosamine (EXXX), ginkgolide B, GR-205171, GW-4459, halometasone, hydroxychloroquine, hydrocortisone (ECVIII), hyperin, ibufenac, ibuprofen (ECXIV), icatibant, ilonidap, indometacin (ECIX), indoprofen, ISIS-104838, isoxicam, ketoprofen (ECXII), ketorolac (EXC), KME-4 (EXLVII), KE-758 (EXXXV), KE-748 (EXXXVI), LL-Z-1640-2, laquinimod, lanepitant, leflunomide (EXXV), limazocic, lobenzarit, L-745337 (EXX), lornoxicam, loteprednol, J-113863 (ELXXX), meclofenamic acid, mesalazine (ECII), meloxicam (ECXV), methotrexate (ECC), 6-methoxy-2-naphthylacetic acid (ELXIV), mofetil, ML-3000 (EVIII), mizoribine, mivotilate, mofezolac, mometasone, moxilubant, mycophenolate mofetil (EXXVIII), mycophenolic acid (EXXIX), MX-68 (EC), nabumetone, naflocort, naproxen (ECV), N-desmethylbenzydamine (ELXIII), nepadutant, nepafenac, niflumic acid, nimesulide (EXV), NS-398 (EXXXIII), olsalazine, oxaprozin, oxolamine, oxisopred, paracetamol (ECVI), paramethasone, parecoxib (EIV), pemedolac, penicillamine, pentostatin, pimecrolimus, piroxicam, PNU-156804, pralnacasan (EXXXVII), prasterone (ECLXII), prifelone, prednisone, prednisolone (ECVII), prednazoline (ECCI), propacetamol, proglumetacin, quercetin, reminertant, resiquimod, rhein, riboflavin, riboflavin monophosphate, riboflavin tetrabutyrate, riboflavin triacetate, ridogrel (ECXL), rimonabant, rofecoxib (EIX), romazarit, rofleponide, roquinimex (ECLXI), RWJ-67657, RWJ-68354, salicylic acid (ECXCI), S-19812 (ECXXX), SB-202190, SB-203580, SB-220025, SCIO-469, sinomenine, sirolimus, sivelestat, solimastat, SP-600125, spiradolene, sulfasalazine, S-2474 (EXXVII), SU-5271 (ELXII), sulindac (ECXI), suprofen, suxibuzone, tacrolimus, talnetant, tanomastat, tauroselcholic acid, tazofelone, tazadolene, tebufelone (ECXXXI), tenidap (ECIII), tenoxicam, tepoxalin (EVII), teriflunomide (EXXIV), thalidomide, tiaramide, ticolubant, tifurac, tilmacoxib (EIV), tilnoprofen arbamel, tiopronin, tomoxiprole, tolfenamic acid, tresperimus, T-614 (EXXI), TBC-1269, TSL-225, TTL-3, triamcinolone, triptolide, ursulcholic acid, UR-8813, ursodiol (ELXV) (EgXII), valdecoxib (EV), vofopitant, VX-745, WHI-P131 (ECLI), Y-39041, zileuton (ECXX), zomepirac, (EX), (EXII), (EXVI), (EXVII) (EXVIII), (EXIX), (EXXI-b), (EXXXI), (EXXXII), (EXXXIII), (ELXX), (EXXXVIII), (EXXXIX), (EXL),(EXLI), (EXLII) (ECLXIII), (ECLXX) (ECLXXI), (ECLXXII), (ECLXXX), (ECLXXXI), (ECLXXXII) (ECLXXXIII). (ECLXXXIV). (ECLXXXV). (ECLXXXVI) 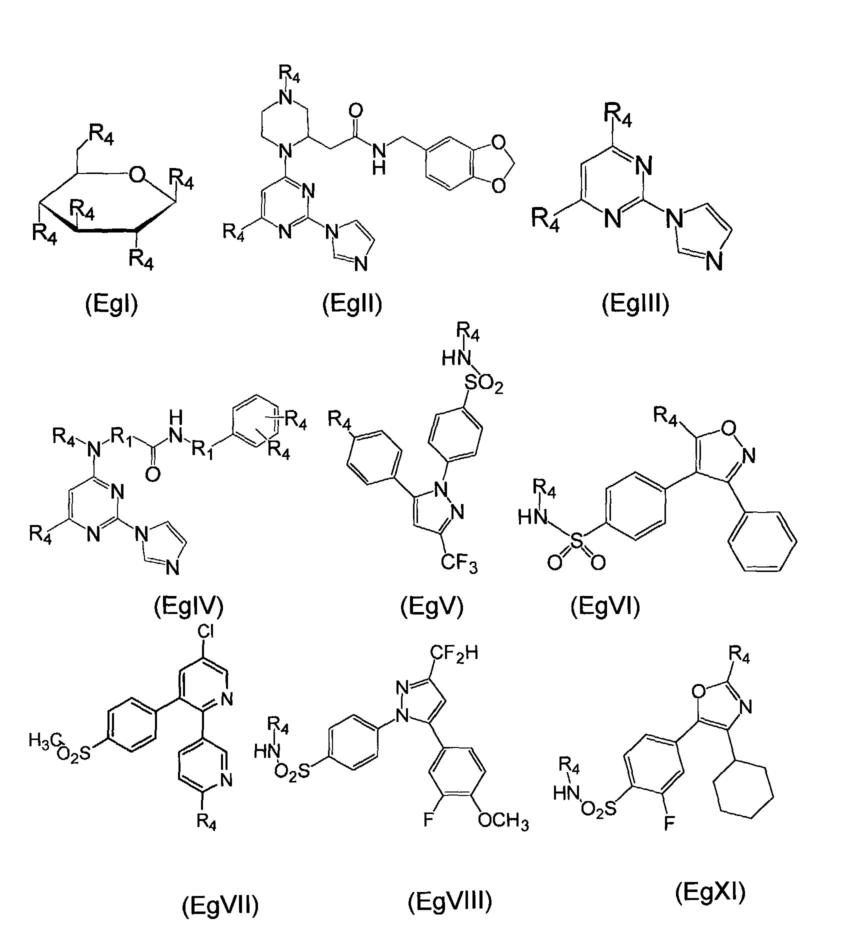 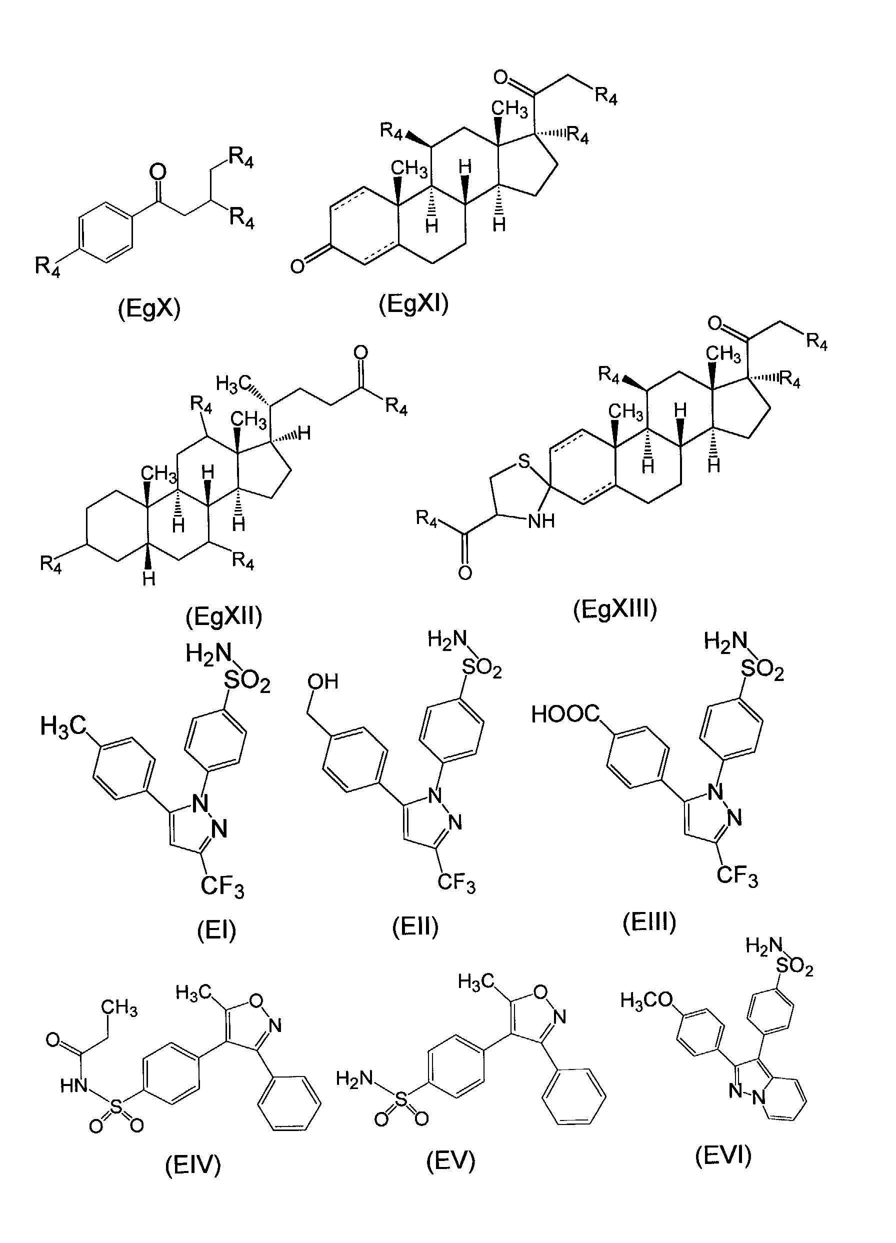 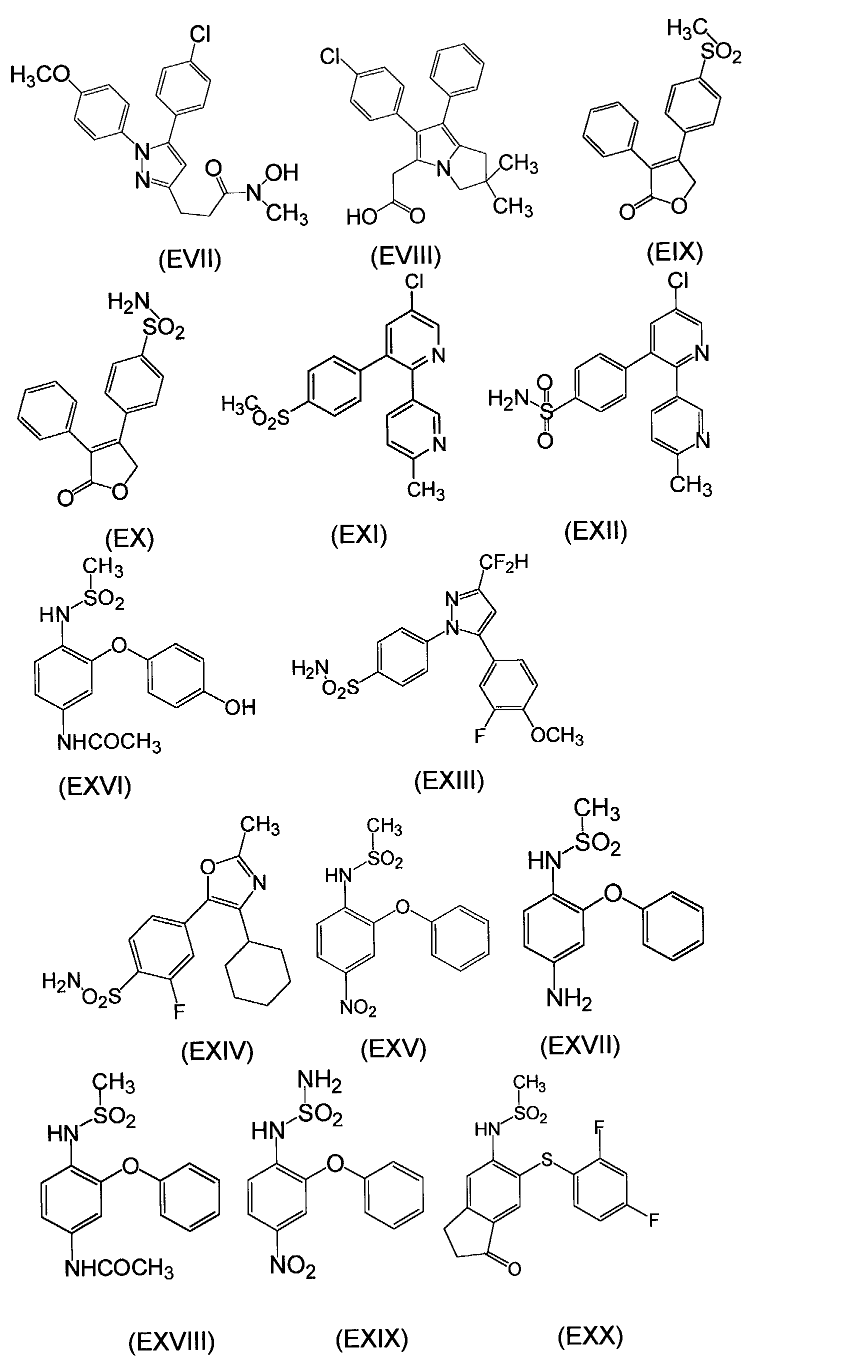 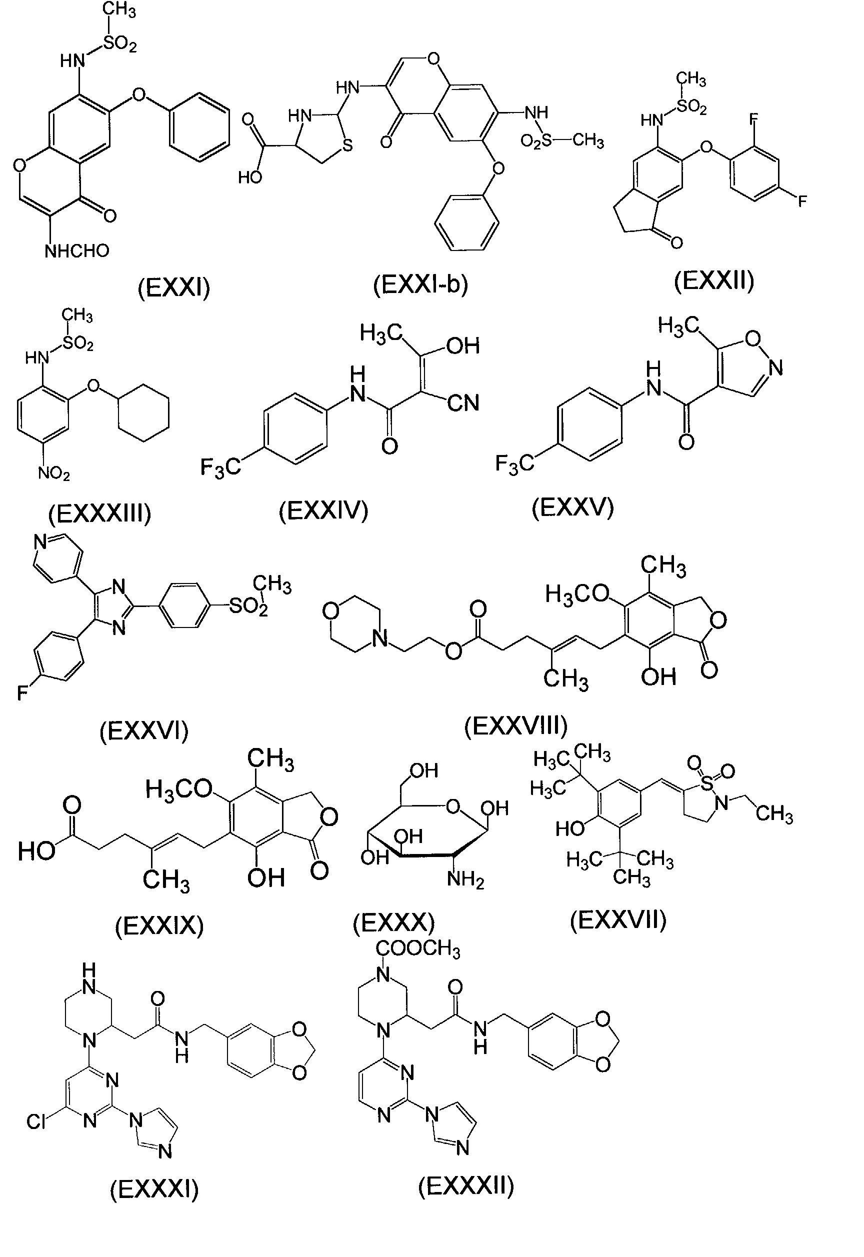 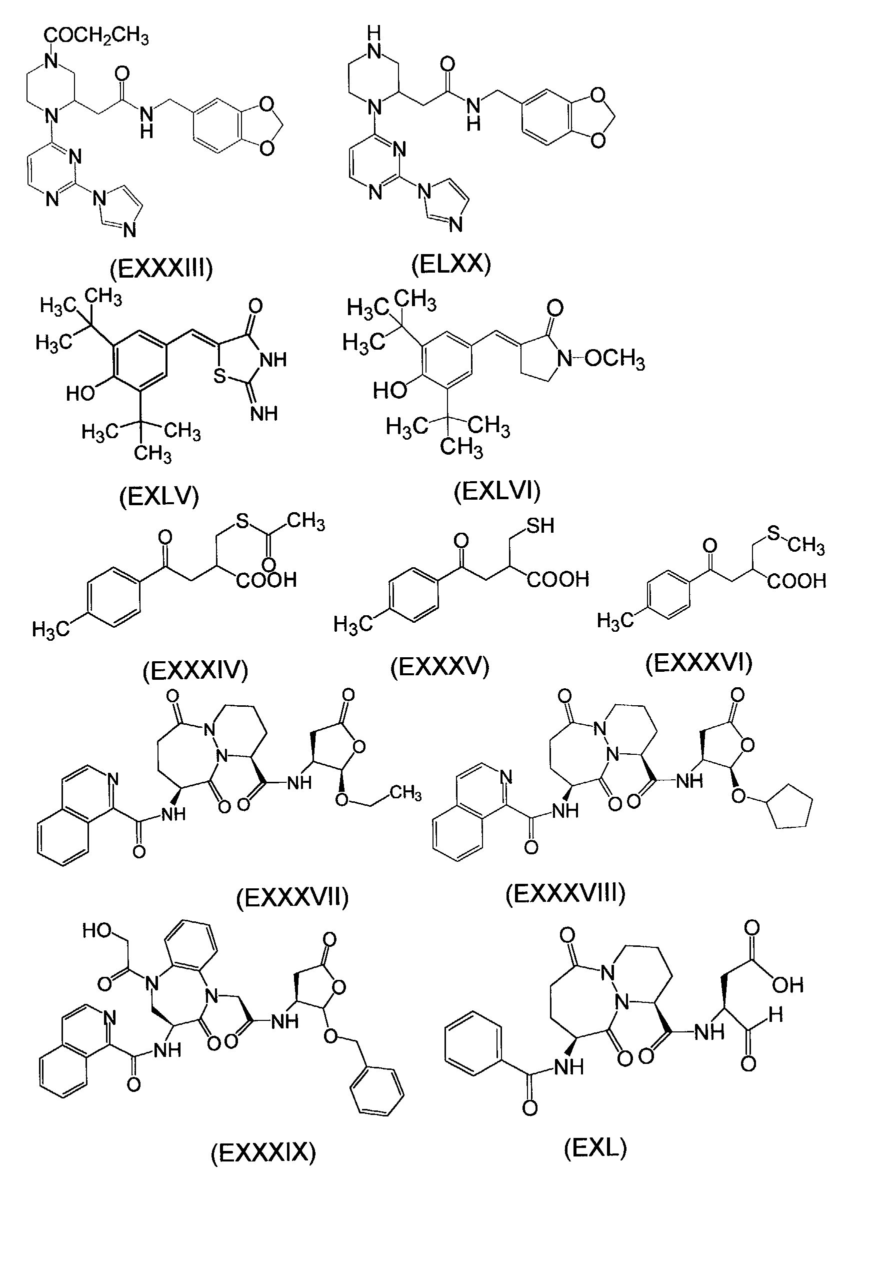 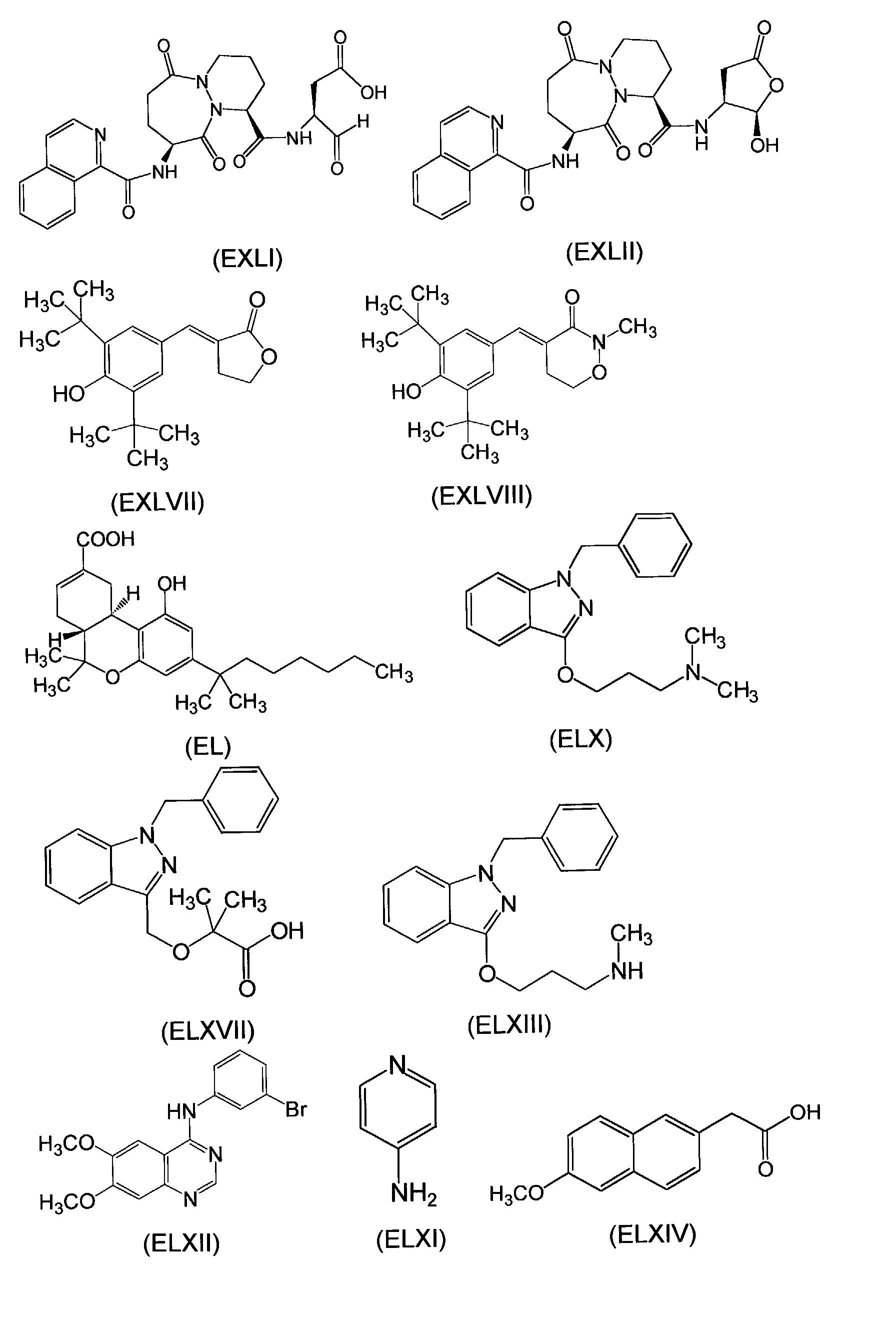 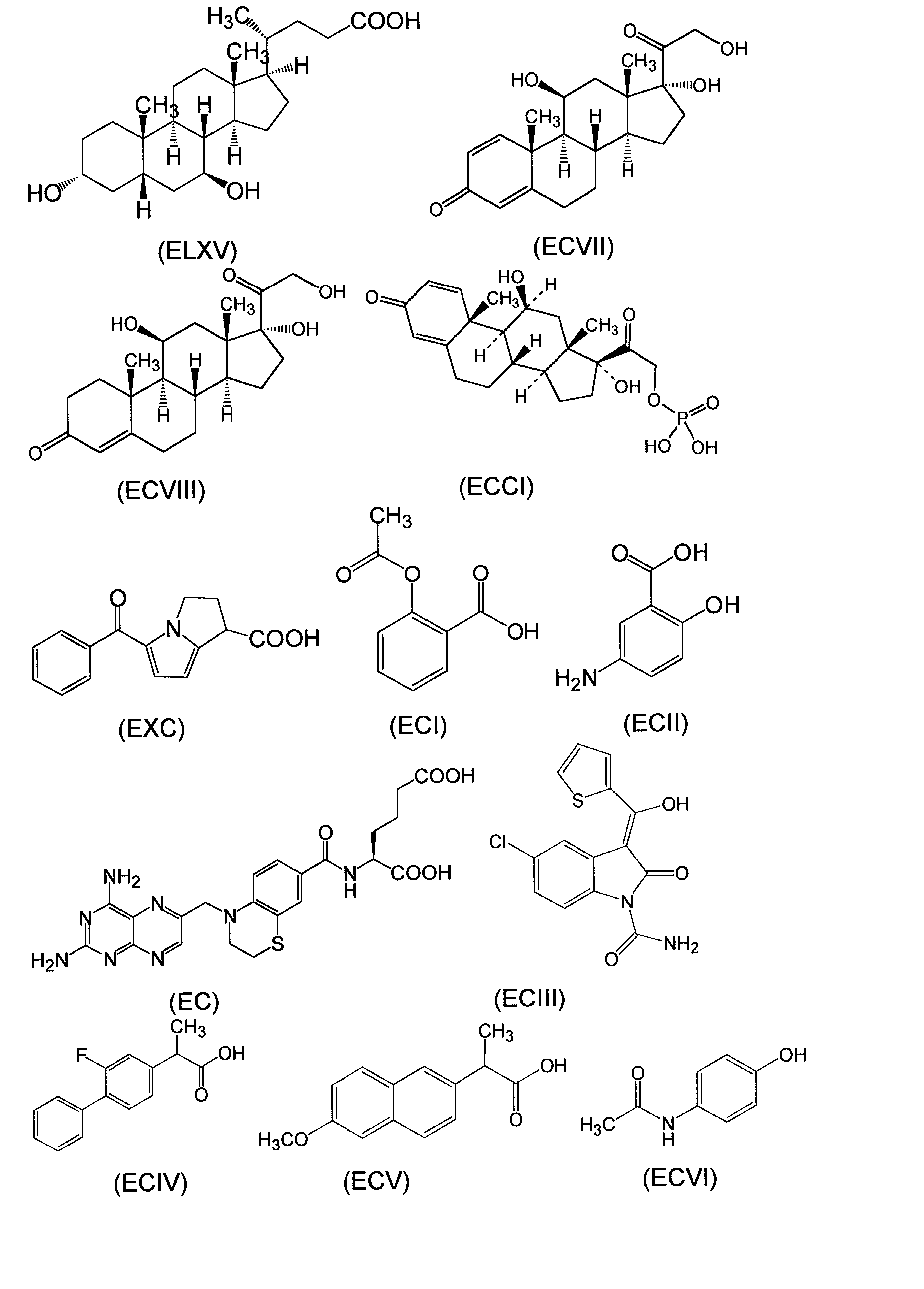 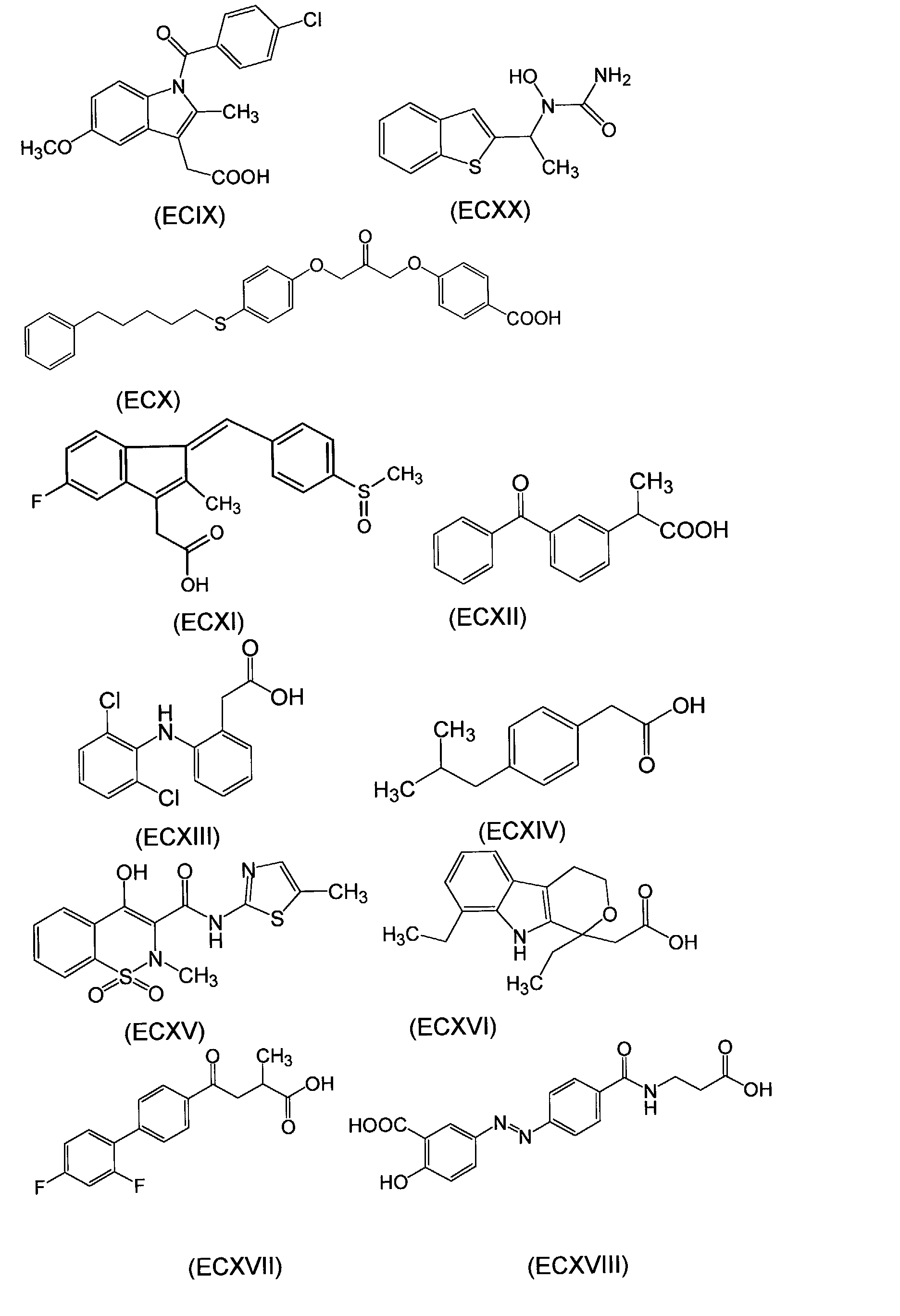 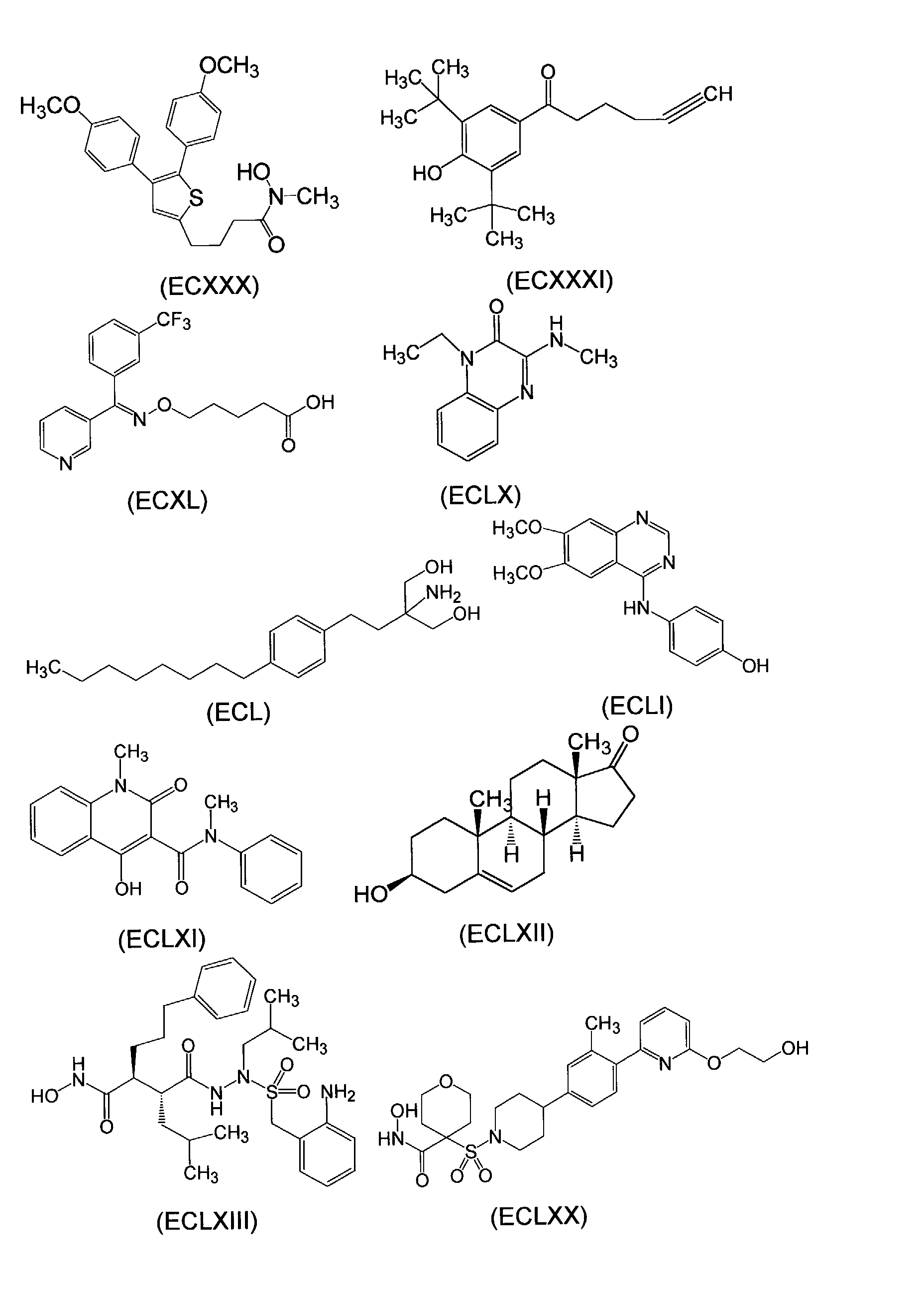 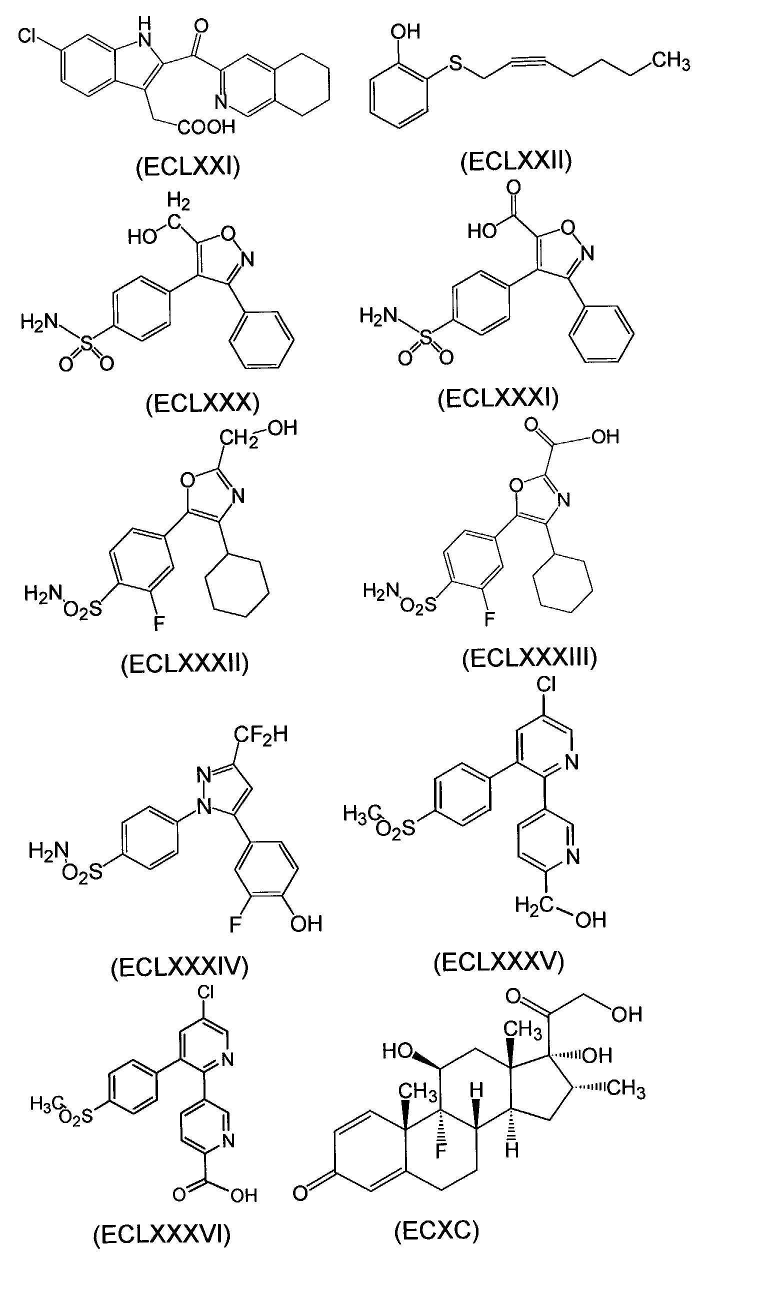 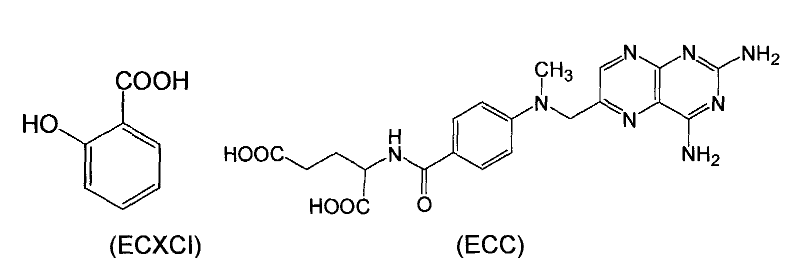 GROUP 2B:
alendronic acid, AGN-4310, arzoxifene (SXXX), avicatonin, bazedoxifene/TSE-424, bervastatin, butedronic acid, calcipotriol, calcitriol (SVIII), CGP-77675, clodronic acid, CP-336156 (SXXXII), CP-424391, doxercalciferol, droloxifene (SVI), EB-1089 (SXXXV), ecalcidene, ED-71, EMD-84444, ERA-923, esonarimod, estradiol, etidronic acid (SXV), F-9775A, F-9775B, falecalcitriol (SXXXI), FC-1271, GW-544, HMR-3339A, 4-hydroxytamoxifene, ibandronic acid (SXX), idoxifene, incadronic acid, LGD-2226, lasofoxifene (SVII), levormeloxifene, levosimendan, lexacalcitol, lidadronic acid (SV), maxacalcitol (SXL), medronic acid, minodronic acid (SIII), miproxifene, NDE-1003, neridronic acid, NPS-2143 (SXII), NVP-AAK980, olpadronic acid (SXXI), ORG-30659, one-alpha-D₂ (SXLI), ormeloxifene, osaterone, oxidronic acid, pamidronic acid (SXXII), paricalcitol (SXXXVII), pipendoxifene (SXXXIII), piridronic acid, pitavastatin, plicamycin, pralnacasan, raloxifene (SXIII), ranelic acid, risedronic acid (SXVI), RWJ-68354, SB-242784 (SIX), SB-265123, SB-267268, SB-273005, SC-56631, secalciferol, semparatide, simendan, SUN-E3001, TAK-778, teriparatide, tiludronic acid, tibolone, toremifene (SXIV), TJN-135, trimegestone, trioxifene, TSE-424, vitamina D₂ (SII), vitamina D3, VX-745 (SX), zindoxifene, zoledronic acid (SIV), (SXI), (SXLII), (SL) 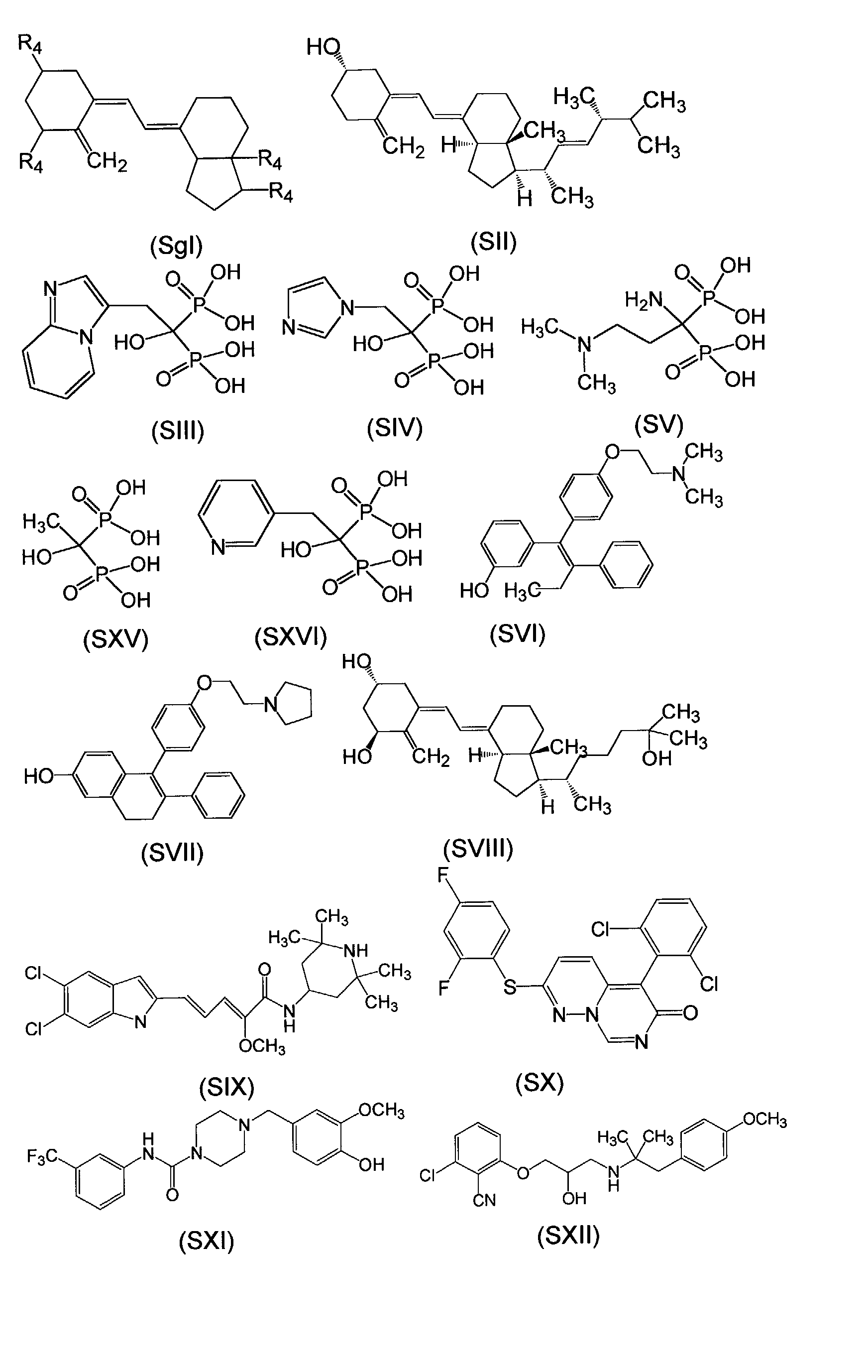 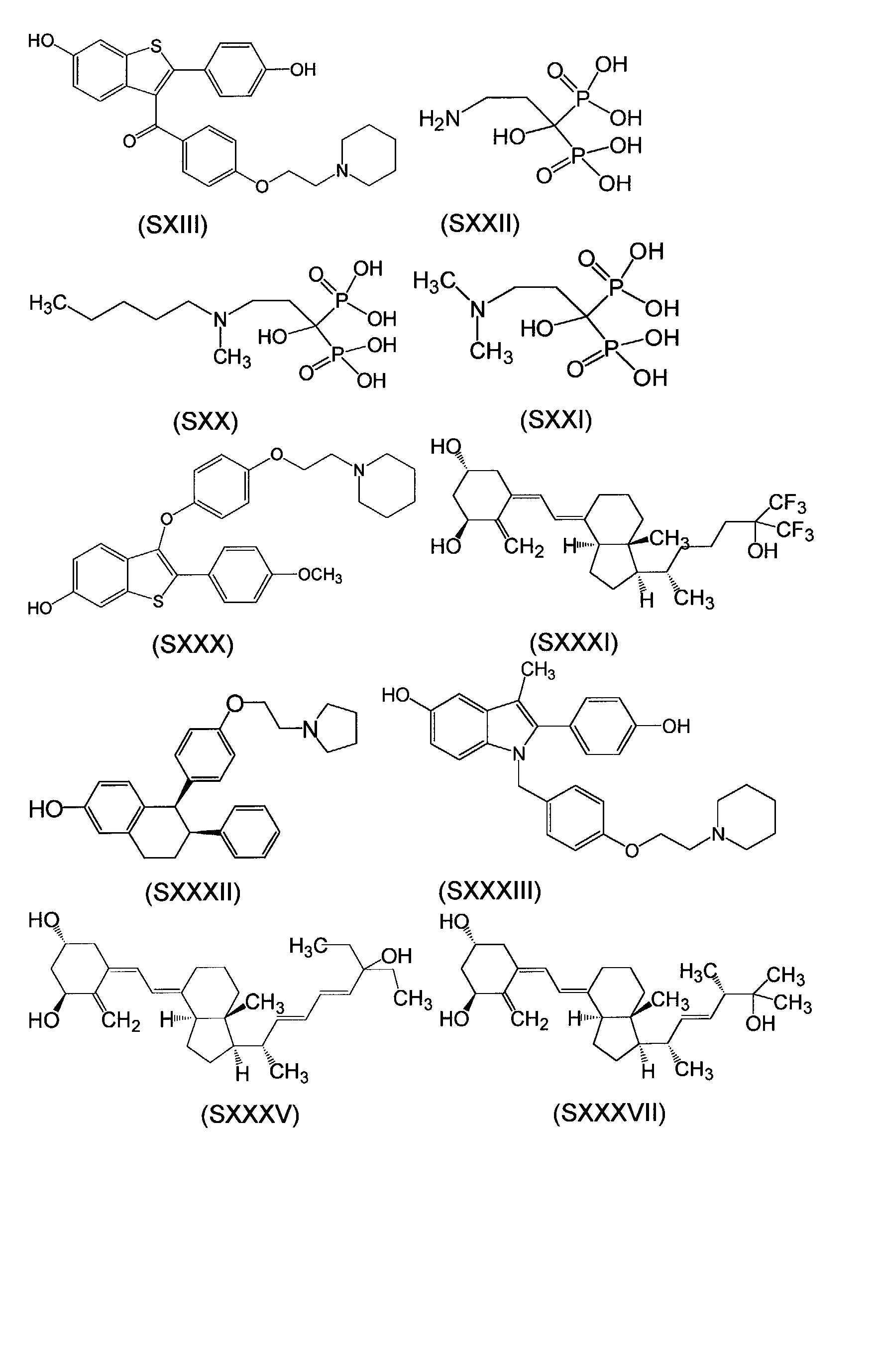 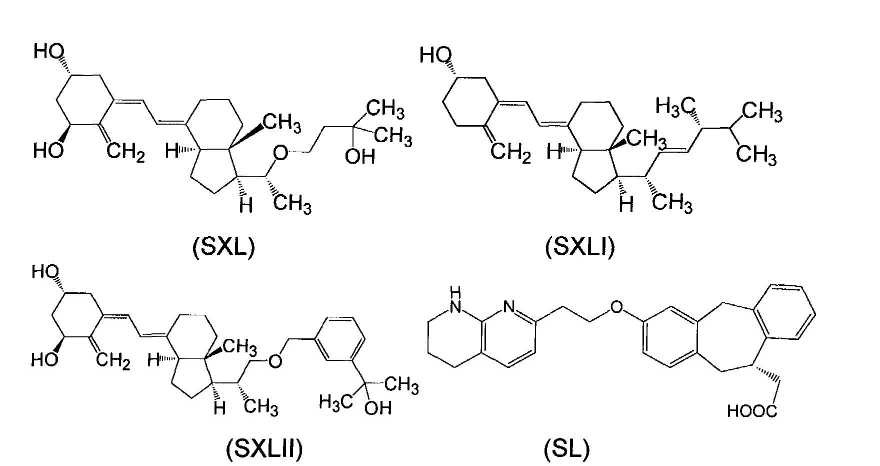 GROUP 3B:
ABT-594, alfentanil, almotriptan, apadoline, aprepitant, asimadoline, aspirin, baclofen, benzydamine, buprenorphine, bupivacaine, butorphanol, capsavanil (PI), CJC-1008, CNS-5161, codeine (PXIV), dapitant, dextromethorphan, dihydrocodeine (PXI), devazepibe, dronabinol, dutarizine, edronocaine, etidocaine, emfilermin, enadoline, etodolac, etofenamate, ezlopitant, fenoprofen, fentanil, figopitant, flufenamic acid, flurbiprofen, gabapentin, ganaxolone, GV-196771 (PII), hydrocodone (PXII), harkoseride, icatibant, indomethacin, ipravacaine, ketoprofen, ketorolac, lamotrigine, lanepitant, levobupivacaine, LY-293558, levonantradol, lidocaine, lofentanil, lomerizine, meperidine, mepivacaine, memantine, metacetamol, morphine, nabazenil, NNC-05-1869, NW-1029, naproxen, nepadutant, osanetant, oxycodone (PX), paracetamol (PIV), pentazocine, phenazopyridine, pidolacetamol, pravadoline, propacetamol, pregabalin, propoxyphene, remifentanil, rimonabant, rizatriptan, ropivacaine, rufinamide, saredutant, SCH-50971, SDZ-249-665, sufentanil, sumacetamol, suprofen, talnetant, tonabersat, topiramate, tramadol (PV), vofopitant, ziconotide, zoltriptan, zucapsaicin, (PIII) 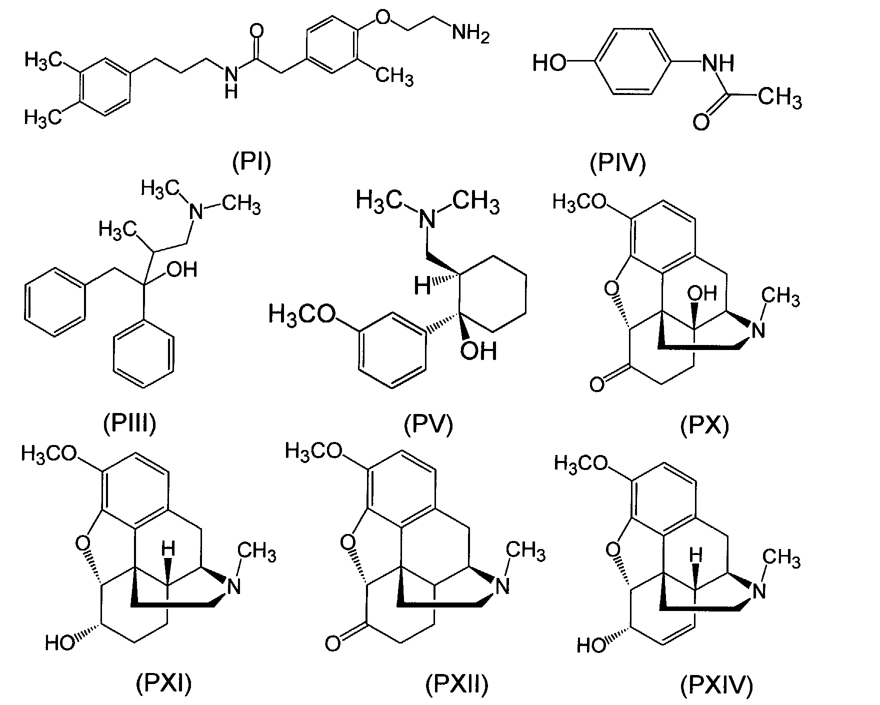 GROUP 4B:
α-tocopherol (QIV), acitretin (QI), adapalene, amelubant, amlexanox (QIII), azathioprine, atizoram, calcitriol, calcipotriene/calcipotriol (Q10), bexarotene, dexamethasone, LPD-519, hydrocortisone, hydroxyurea, prednisolone, prednicarbate, prednisone, riboflavin, riboflavin tetrabutyrate, riboflavin 6-thioguanine, triacetate, tacalcitol, lexacalcitol, maxacalcitol, moxilubant, seocalcitol, paricalcitol, sulfasalazine, tazarotene, tepoxalin, ticolubant, thalidomide. VX-497. (QII), zidovudine. 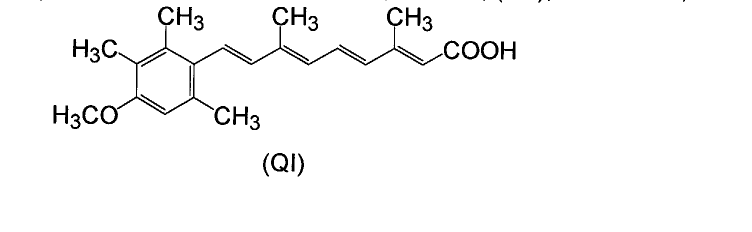 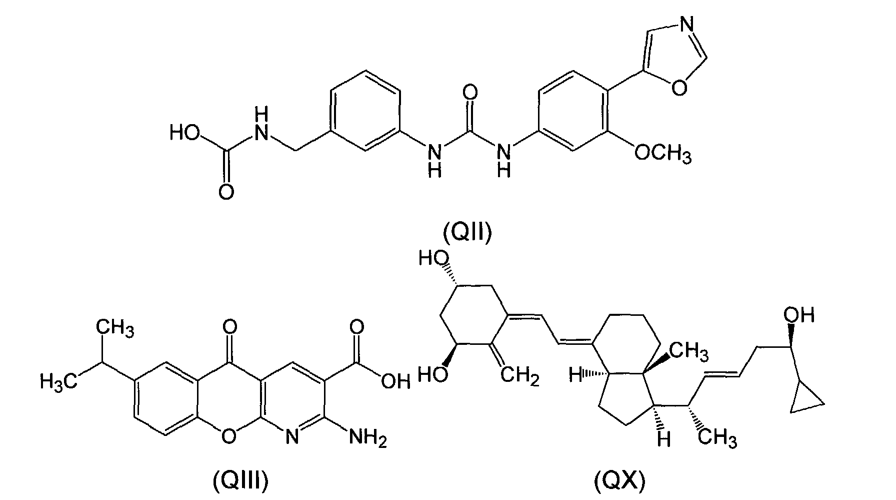 GROUP 5B:
ablukast, ABT-761 (JVII), acetylcysteine, acitazanolast, acreozast, adapalene, albifylline, albuterol (JXXXII) (Jglll), alclometasone, alinastine, AMG-126767, R-α-methylhistamine (JCXXXI), ambicromil, ambroxol, amebucort, amelometasone, aminophylline, andolast, APT-366, apafant, apaxifylline, argatroban, argimesna, arofylline, AR-C68397AA (JXXXVI), asobamast, astemizole (JCXXXIV), ataquimast, atizoram, atreleuton, AY-0068, AWD-12-281 (JXVII), AWD-12-343, BAY-19-8004, BAY-X-1005 (JLXXX), bamaquimast, batebulast, benafentrine, bepafant, bepotastine, betotastine, betamethasone, bilastine, bimosiamose, binizolast, bitolterol, BP-294 (JCXXX), budesonide (JII) (Jgll), bunaprolast, butaprost, butixocort, camonagrel, carebastine, cartasteine, CE-1037, cetirizine (JCXXXII), ciclesonide, cilomilast/SB-207499 (JXI), cilutazoline, cinalukast, cipamfylline, Cl-1018 (JXIII), CI-1044 (JXVIII), CGH-2466, cistinexine, ciclesonide, clopidogrel, cloticasone, cromakalim, cromoglicate lisetil, contignasterol, cromoglicic acid (JI), CR-3465, cysteine, D-4418 (JXII), CPX (JLXXIII), D-4396, dacopafant, daltroban, dametralast, danosteine, dapitant, dazoquinast, DAX (JLXXIV), decarboethoxyloratadine, DF-1111301, deflazacort, deltibant, descinolone, desloratadine, desonide, dexamethasone, dexbudesonide, dexsecoverine, dextiopronin, diproteverine, DMP-777, domitroban (JXXX), doqualast, doxaprost, drotebanol, ebastine, eclazolast, efletirizine, eflumast, elziverine, emedastine, enefexine, enofelast, enoxamast, epinastine, epinephrine, eprazinone, erdosteine, espatropate, etolotifen, ezlopitant, fenleuton, fenoterol (JXXXIV), fenprinast, fexofenadine (JXC), figopitant, filaminast, flerobuterol, flezelastine, fluazacort, flunisolide, fluticasone (JV) (Jgl), fominoben, foropafant, formoterol (JXXXV), fudosteine, furegrelate, genistein (JLXXII), genleuton, guaimesal, guaifenesin, guaisteine, halometasone, HSR-609, hydroxyzine, ibudilast, icatibant, ICI-200355, idaverine, idenast, ifetroban, ilonidap, imitrodast (JCXI), INS-3653, ipratropium, IPL-4088, iralukast, isalsteine, isbogrel, isbufylline, isocromil, isoetharine, isoproterenol, israpafant (JXL), itazigrel, ketotifen, L-826141 (JXIX), L-658758, lanepitant, laprafylline, levalbuterol, levcromakalim, levmetamfetamine, levocabastine, levocetirizine, levodropropizine, levosulpiride, lexipafant, linazolast, linetastine, linotroban, lirimilast, lisofylline, lonapalene, loratadine, loxanast, mapinastine, melquinast, meribendan, mesna, metaproterenol, methylprednisolone, midaxifylline, midazogrel, midesteine, milverine, milrinone (JLXXI), minocromil, minopafant, mipitroban, mizolastine, modipafant, moguisteine, mometasone, montelukast (JC), MPB-07 (JLXXV), moxilubant, nafagrel, naphazoline, naflocort, N-acetylcysteine, nardeterol, nedocromil, neltenexine, nemazoline, nepadutant, nestifylline, NKP-608C, noberastine, norastemizole (JCXXXIV), nupafant, olopatadine, olprinone, ontazolast, osanetant (JCXX), oxalinast, oxitropium, ozagrel, pamicogrel, paramethasone, pemirolast, perbufylline, phenylbutyrate (JLXX), phenylpropanolamine (JXXII), piclamilast (JXXV), picumast, picumeterol, pirbuterol, pirmagrel, pirodomast, pobilukast, pranlukast, prednisolone, prednisone, propiverine, promethazine, proxicromil, pumafentrine (JXIV), quazolast, quiflapon, quinotolast, racephedrine, racephedrine, ramatroban (JXXXI), raxofelast, repirinast, revatropate, revenast, ridogrel, rimonabant, ritolukast, rocastine, rocepafant, roflumilast (JX), rolafagrel, rolipram, RPR-106541, rupatadine (JXCI), salmeterol (JXXXIII), salmisteine, samixogrel, saredutant, sarpogrelate, satigrel, scopinast, SCH-351591, seratrodast (JCX), setipafant, sevitropium, siguazodan, sivelestat (JL), spirofylline, stacofylline, sulotroban, sulukast, suplatast (JLX), T-440, talnetant, taurosteine, tazanolast, telmesteine, tematropium, temiverine, tenidap, terbogrel, terbucromil, terbutaline, tetrazolast, texacromil, theophylline, tiacrilast, tibenelast, ticolubant, tiotropium, tioxamast, tiprinast, tolafentrine, tofimilast, tomelukast, tranilast (JIII), toborinone (JXX), triamcinolone, tulopafant, UT-77, verlukast, vofopitant, V-11294A (JXV), YM-976 (JXVI), zafirlukast, zardaverine, zileuton (JIV), zilpaterol, zipeprol, (JXCII), (JCXXXIII), (JCXXXIV) 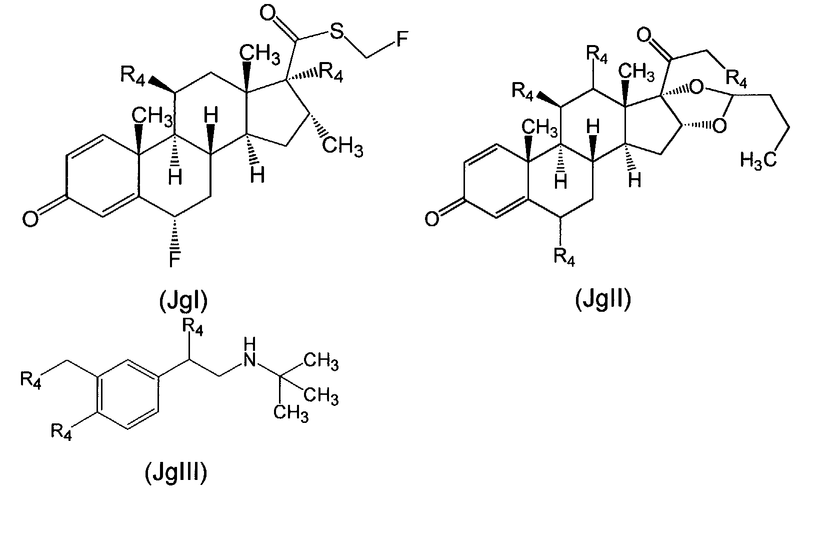 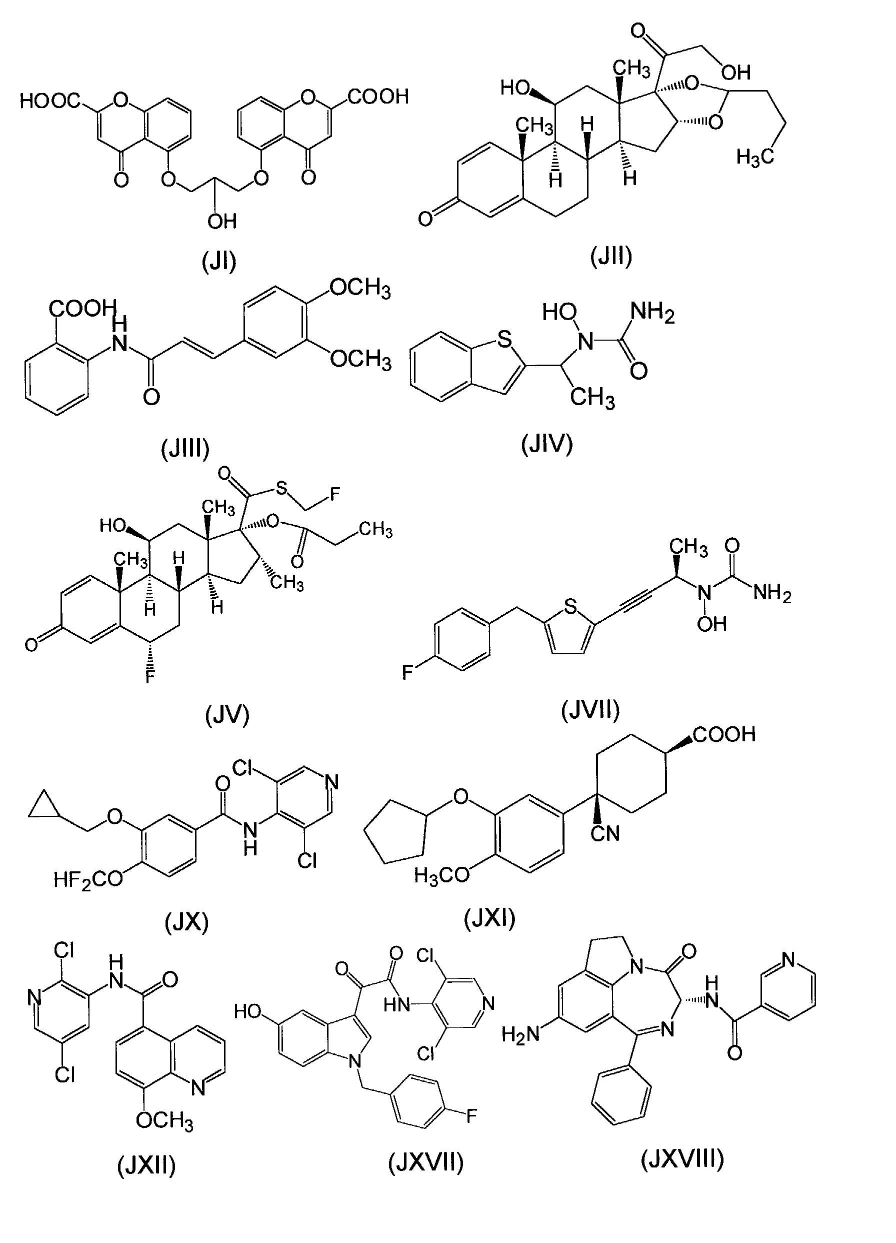 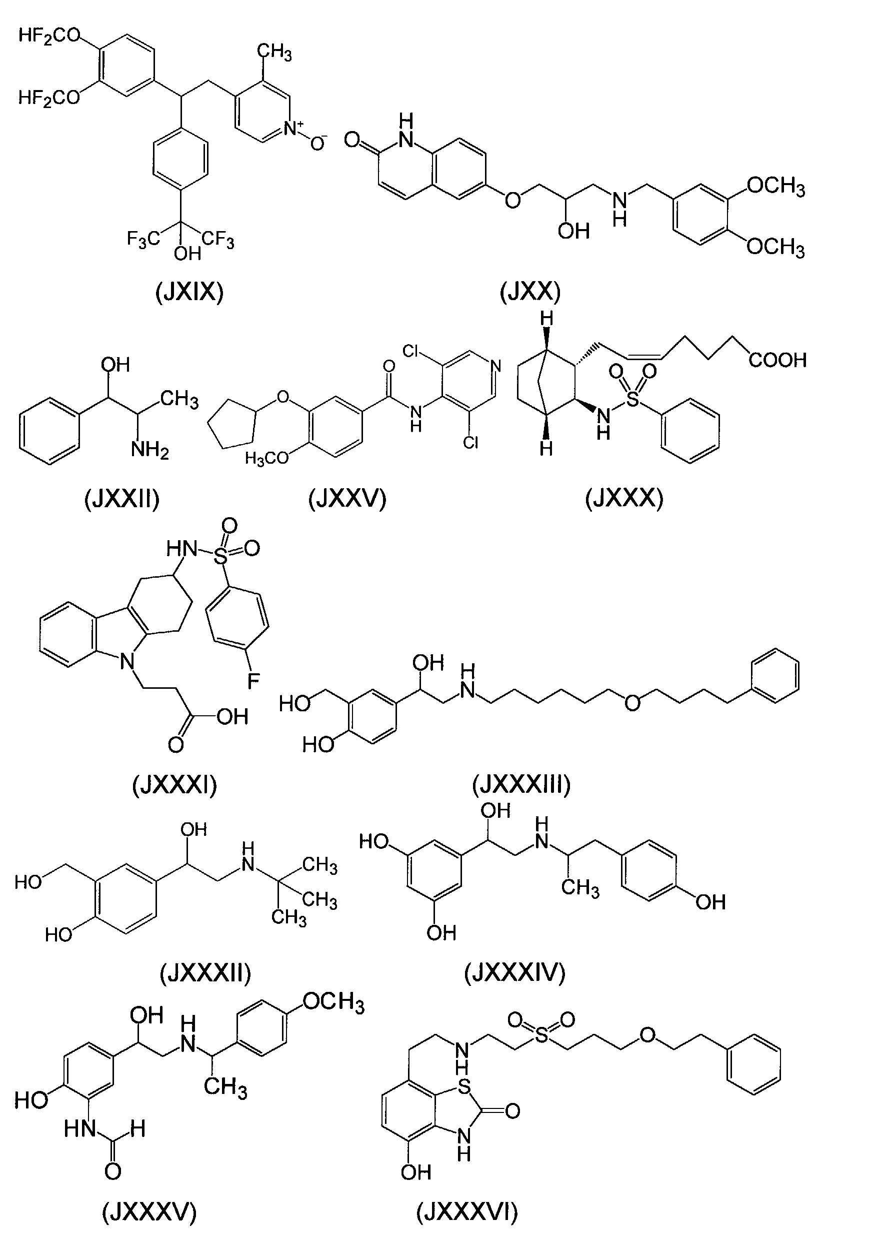 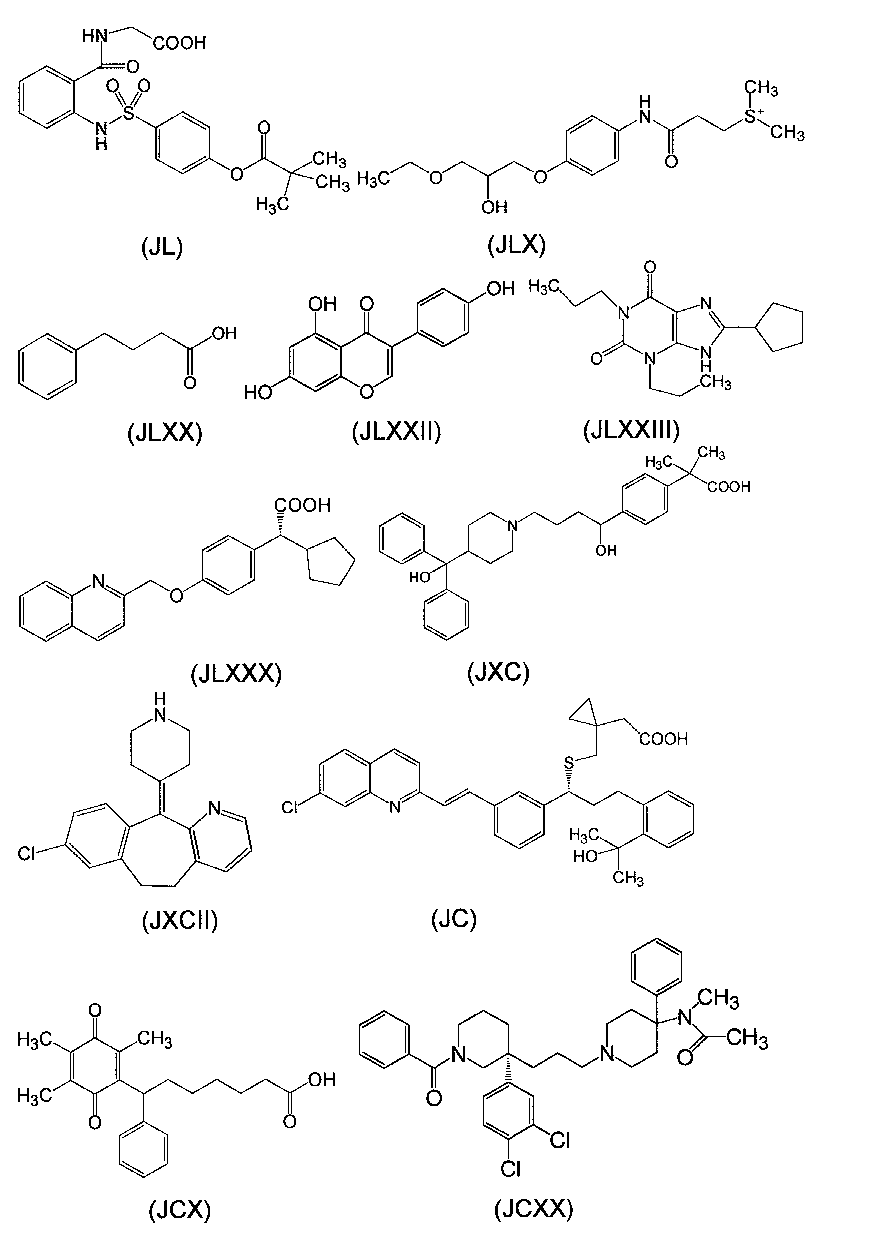 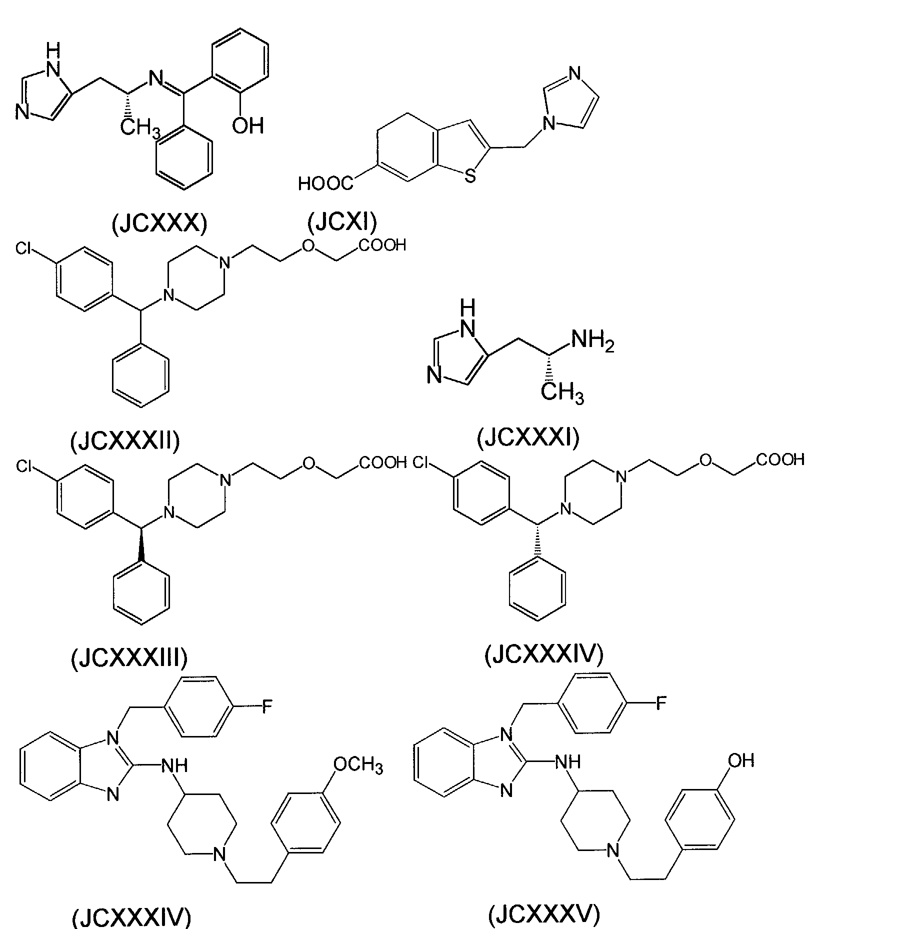 GROUP 6B:
alizapride, alosetron, amiglumide, arbaprostil, AR-H047108, bromopride, budesonide, CAM-1028, 5-carboxyomeprazole (RXCII), 5-carboxytenatoprazole (RXCVI), 5-carboxyesomeprazole (RXCVII), chenodiol, cimetidine, cisapride, clarithromycin, clebopride, CR-2622, CR-3294, darifenacin, deprostil, desmethylrabeprazole (RXXVIII), devazepide, dexloxiglumide (RXXXI), dolasetron, domperidone, dosmalfate, egualen (RXXX), E-3620, enprostil, esaprazole, esomeprazole (RIV), esolansoprazole (RCI), esopantoprazole (RCIII), famotidine, fedotozine, granisetron, 5-hydroxylansoprazole (RC), 5-α-hydroxyomeprazole (RXC), 5-α-hydroxyotenatoprazole (RXCIV), 5-α-hydroxyesomeprazole (RXCVIII), ezlopitant, itasetron, itriglumide (RgIV) (RXXXIII), IY-81149 (RIX), L-365,260 lansoprazole (RV), lafutidine, leminoprazole (RI), levosulpiride, loperamide, loxiglumide, L-365260, LY-288513, LY-262684, LY-191009, MKC-733, MK-869, meclizine, mesalamine, mexiprostil, MKC-733, metoclopramide, misoprostol (Rgll) (RLXXII), mosapride, nepadutant, nepaprazole, nizatidine, nocloprost, norcisapride, 5-O-desmethylomeprazole (RXII), 4-O-desmethylpantoprazole (RXXII), olsalazine, omeprazole (RII), ondansetron, ornoprostil, osutidine, pantoprazole (RVI), pibutidine (RXLI), PD-135158, picoprazole (RLXIV), pipratecol, pranazepide, proglumide, pumaprazole, prucalopride, ranitidine, rabeprazole (RIII), rebamipide (RXXXIV), remiprostol, renzapride (RXXXII), rioprostil, rosaprostol (RgIII) (RLXX), roxatidine (RXL), RP-72540, RP-73870 (RLXXXI), S-0509 (RLXXXII), SB-641257, sepimostat (RL), spiroglumide, sonepiprazole, sulfasalazine, TAK-637, tarazepide, taurocholic acid, tegaserod, telenzepine, tenatoprazole (RVIII), timoprazole (RLXIII), tiropramide, trimoprostil, tropisetron, YIA-20379-1 (RLX), YM-022, YIA-20379-5 (RLXI), YIA-20379-8 (RLXII), zacopride, (RX), (RXI) (RXII), (RXIII), (RIV), (RXV), (RXVI), (RXVII), (RXVIII), (RXIX), (RXX), (RXXI), (RXXII), (RXXIII), (RXXIV), (RXXV), (RXXVI), (RXXVII), (RLXV), (RLXXI), (RLXXX), (RXCIII), (RXCI), (RCII), (RCIV), (RCVI), (RCVII) 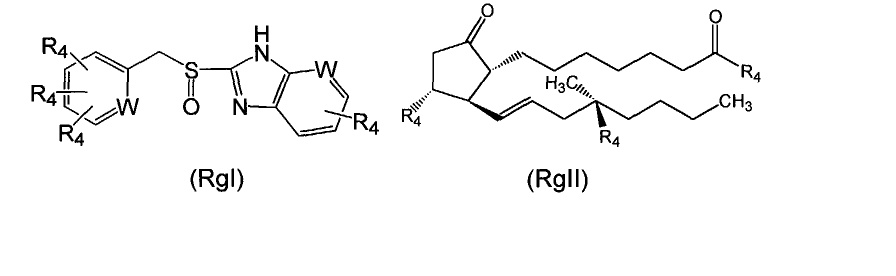 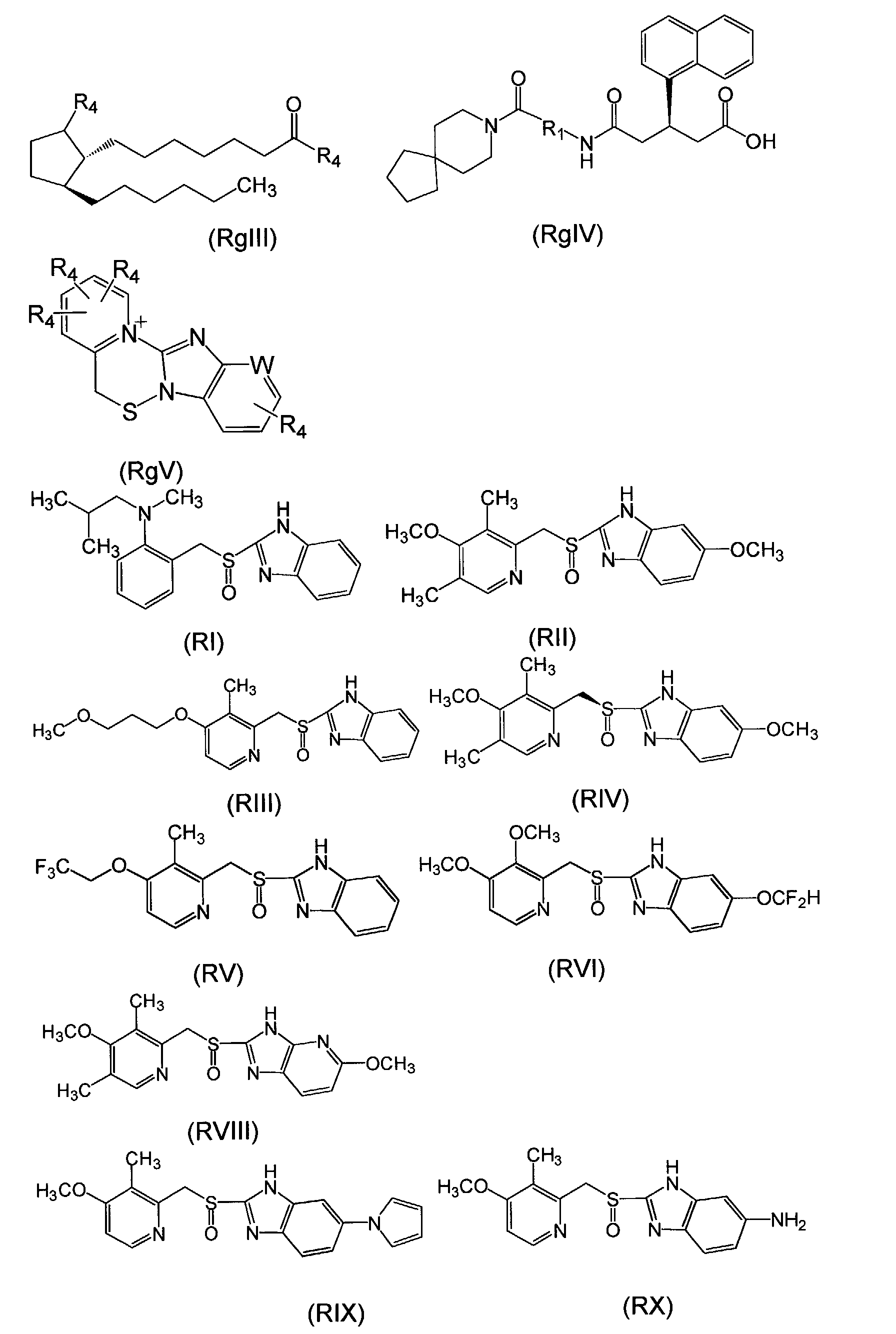 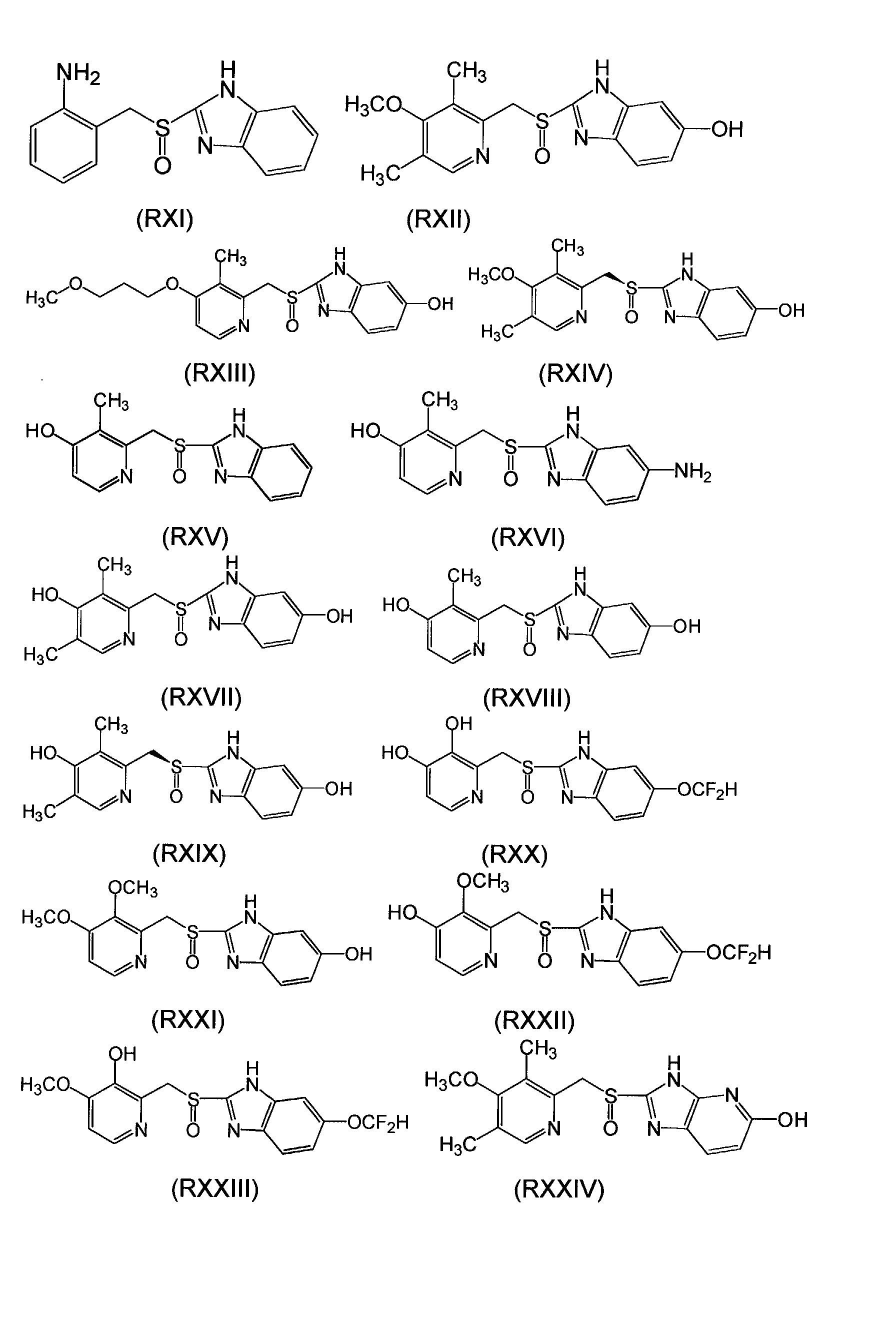 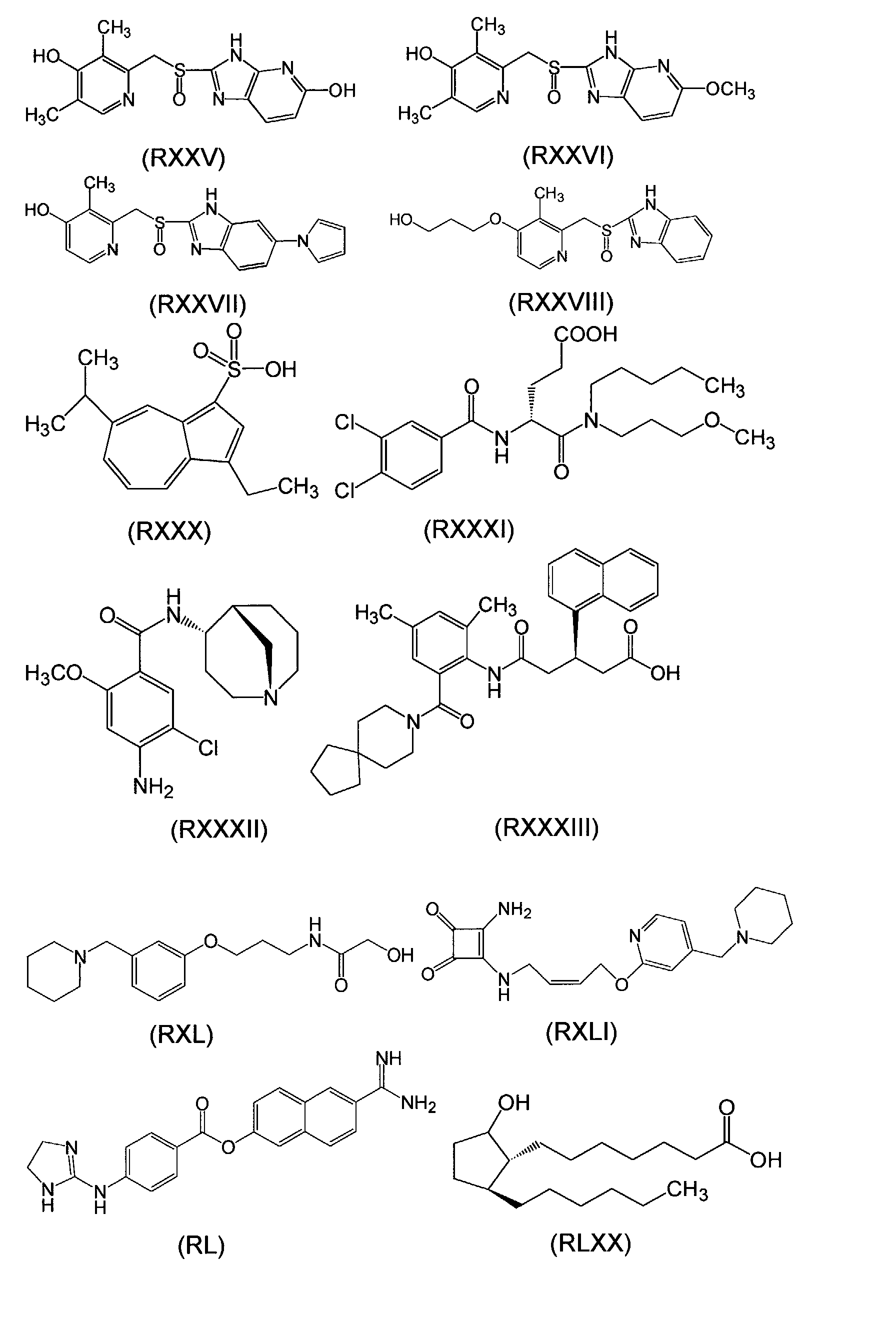 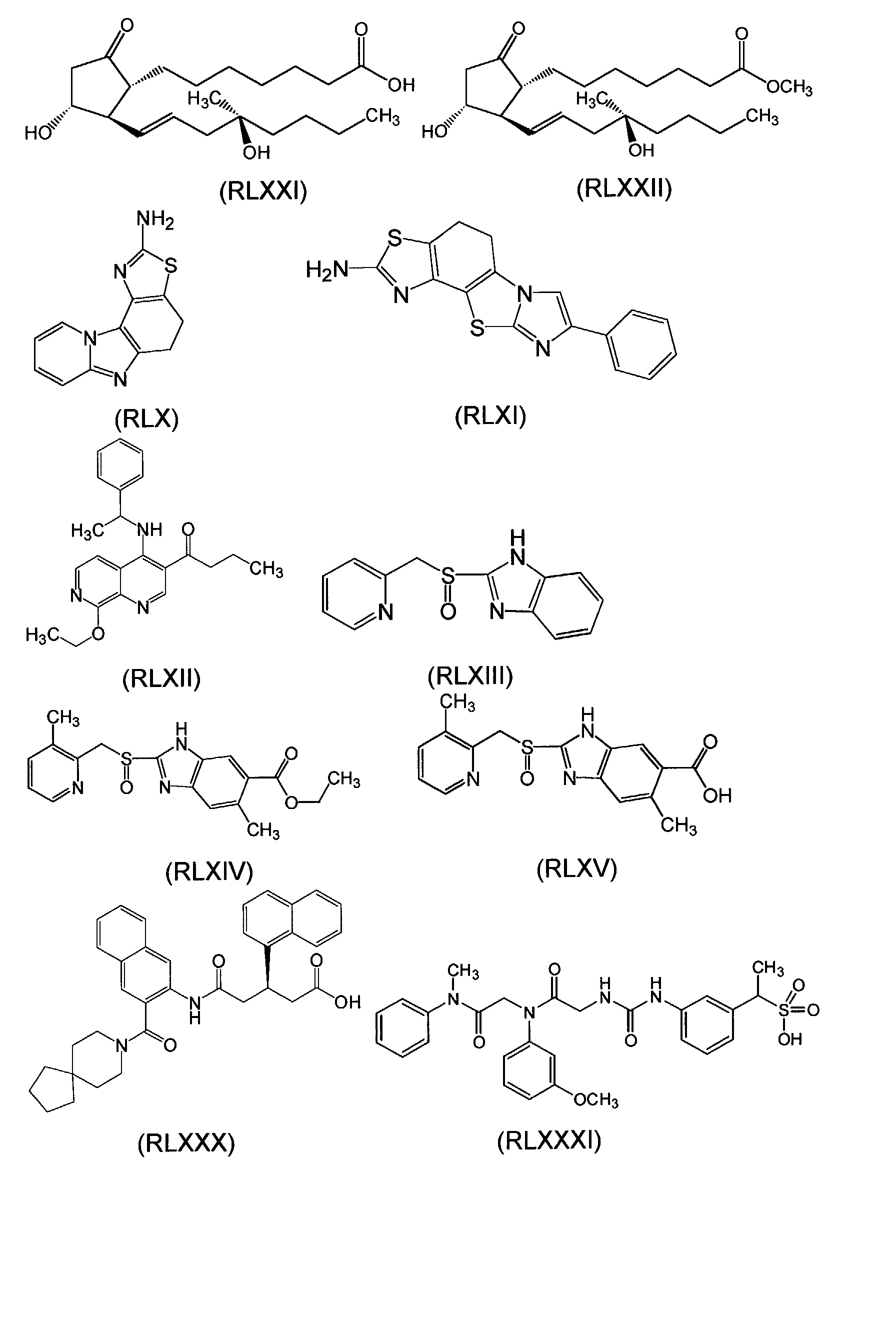 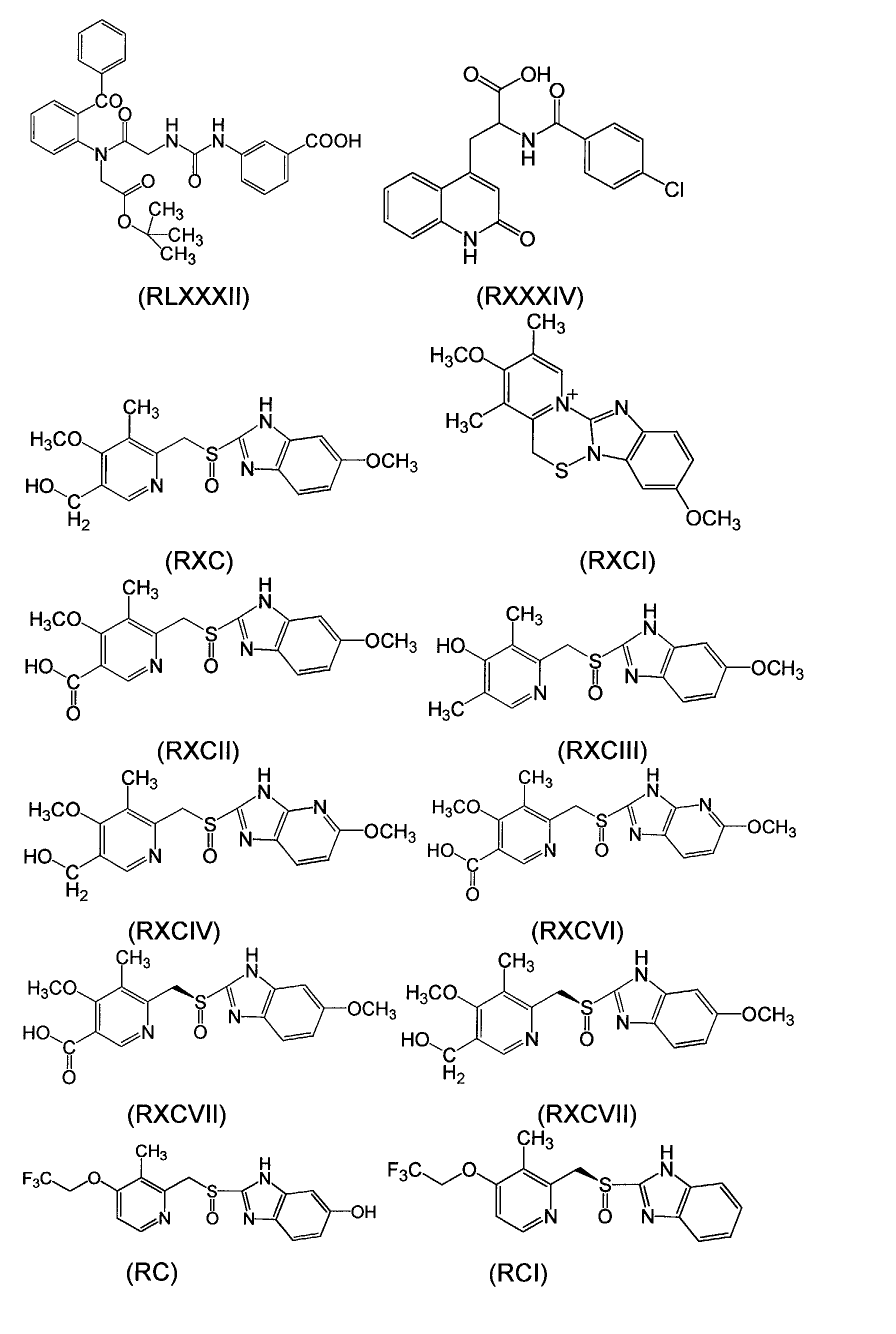 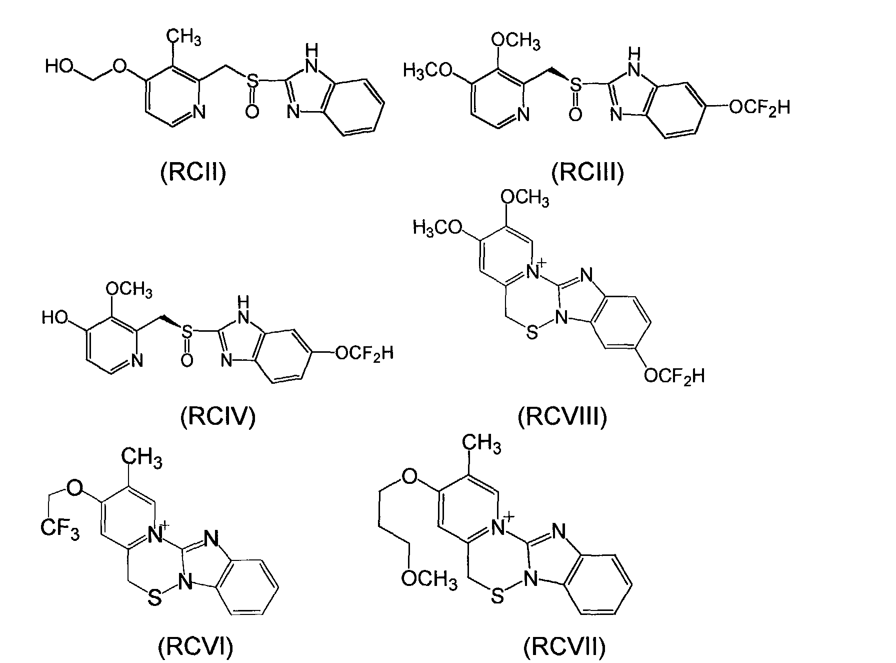 GROUP 7B:
ABT-980, AH-9700, AIO-8507L, alfuzosin (UV), alfatradiol, alvameline (ULXX), alprostadil (UXC), atosiban, apomorphine (UCXXX), atrasentan (UCXL), bexlosteride, bicalutamide, BMS-341400, CS-891, cyproterone (UCII)), DA-8159, darifenacin (ULXXXIII), deacetylmoxisylyte, dehydrocorydaline (UXXXV), (+)-desmethylsibutramine, doxazosin (UXL), duloxetine (UXLI), dutasteride, E-4010 (UXXII), E-4021 (UXXIII), E-8010, EMD-221829, eplerenone, ESR-150, epristeride (UI), FCE-27837, fesoterodine (ULXXV), fiduxosin (UCXII), finasteride, finrozole (UCXLI), fluoxetine (UCXXXI), FK-143 (UCL), FR-119680 (UCLIII), FR-229934, gestonorone (UII-b), GI-198745, GYKI-16084 (UIX), GYKI-12743, izosteride, KMD-3213 (UCX), KCO-616, KRP-197 (ULXXXVIII), inaperisone (ULXXXV), indoramin, izonsteride, L-771688/SNAP-6383 (UCXIII), L-780945/SNAP-6991 (UCXVI), lapisteride, lanperisone, LY-320236, LY-353433, mepartricin, mepazosin, moxisylyte, naftopidil (UC), NC-1800 (ULXXXIX), NS-49 (UXCV), osanetant, osaterone (UII), oxendolone (UIV), oxybutynin, phentolamine (UVIII), PNU-157706 (UCLII), PNU-83757, PNU-156765, PNU-200577, prazosin, propantheline, prasterone (UCI), propiverine, resinferatoxin, RBX-2258, REC-15/3079, Ro-70-0004 (UCXVII), RS-100329 (UCXVIII), RWJ-38063 (UCXIV), RWJ-69736 (UCXV), SB-223412, saredutant (UXCVII), sildenafil (UXXXIV), SNA-4606-1, SNAP-6383, SNP-7915 (UCXIX), (S)-doxazosin (UCXI), (S)-oxybutynin (ULXXXVII), sibutramine (ULXXX), SPM-007, suplatast (ULXXXIV), T-1032 (UXXX), TA-1790, TAK-637, tadalafil/IC-351 (UXXXI), talnetant, tamsulosin (UIII), TF-505, temiverine (ULXXXVI), terazosin, terodiline, tolterodine (ULXXXII), turosteride, N-desmethylsildenafil/UK-103320 (UCLXX), UK-122764 (UVII), UK-343664, UK-357903, vamicamide, vardenafil (UVI), yohimbine (UCXXXII) (UgVII), zaprinast, YM-905 (ULXXXI), YM-53705, YM-46303, WAY133537, WAY151616, ZD-0947, ZD-6169 (UCXVI), Z-350 (UCLI), ZM-244085, (UX), (UXI), (UXII), (UXIII), (UXIV), (UXV), (UXVI), (UXVII), (UXVIII), (UXIX), (UXX), (UXXI), (UXXIV),(UXXV), (UCLIV), (UCLV), (UCLVI), (UCLVII), (UCLVIII), (UCLIX), (UCLXX), (UCLXXI), (UCLXXII), (UCLXXIII), (UCLXXIV) 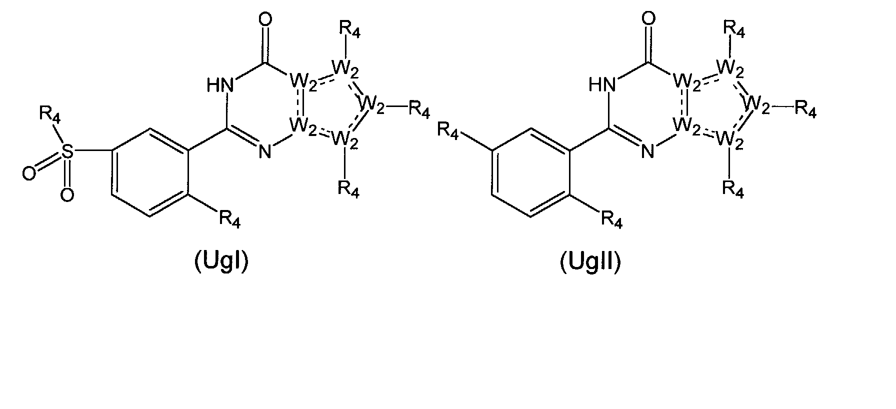 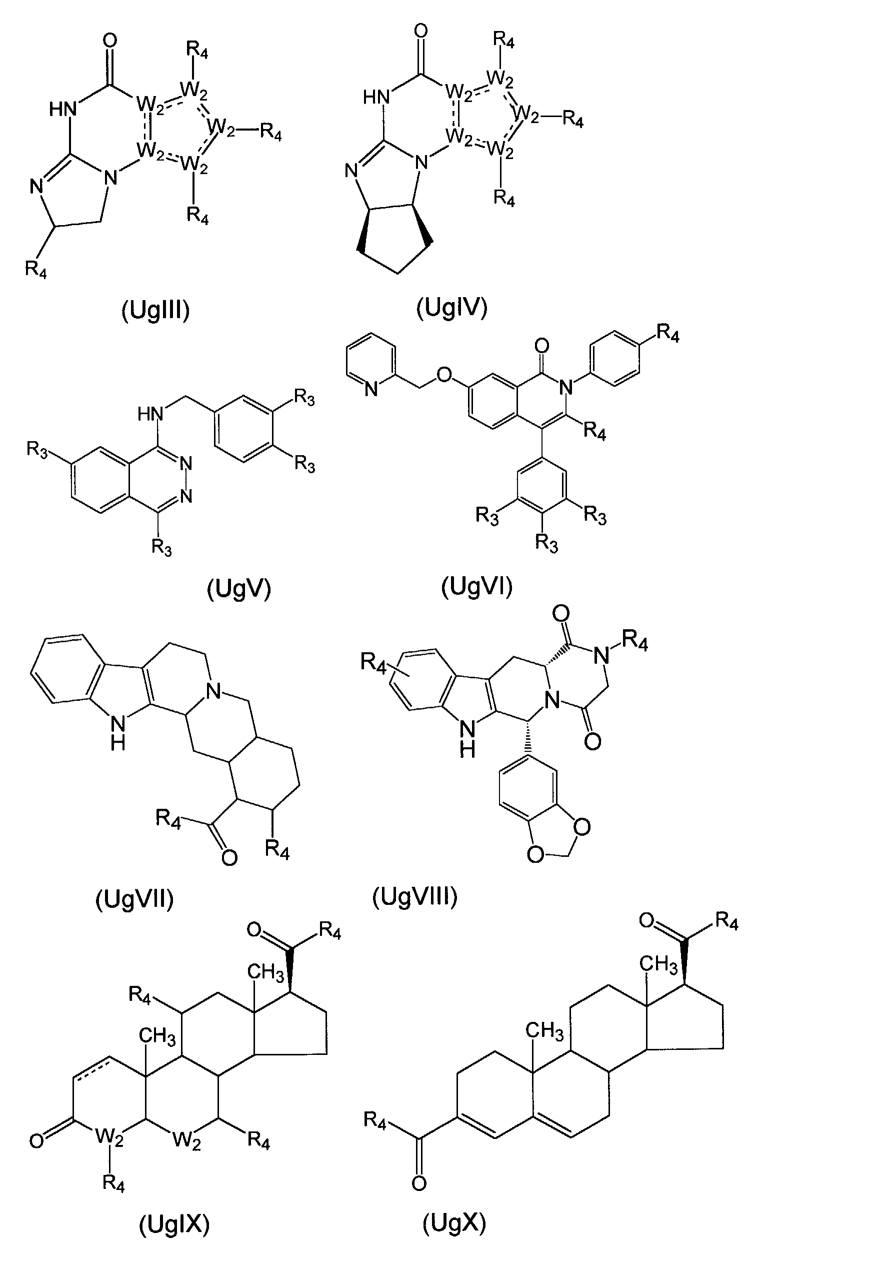 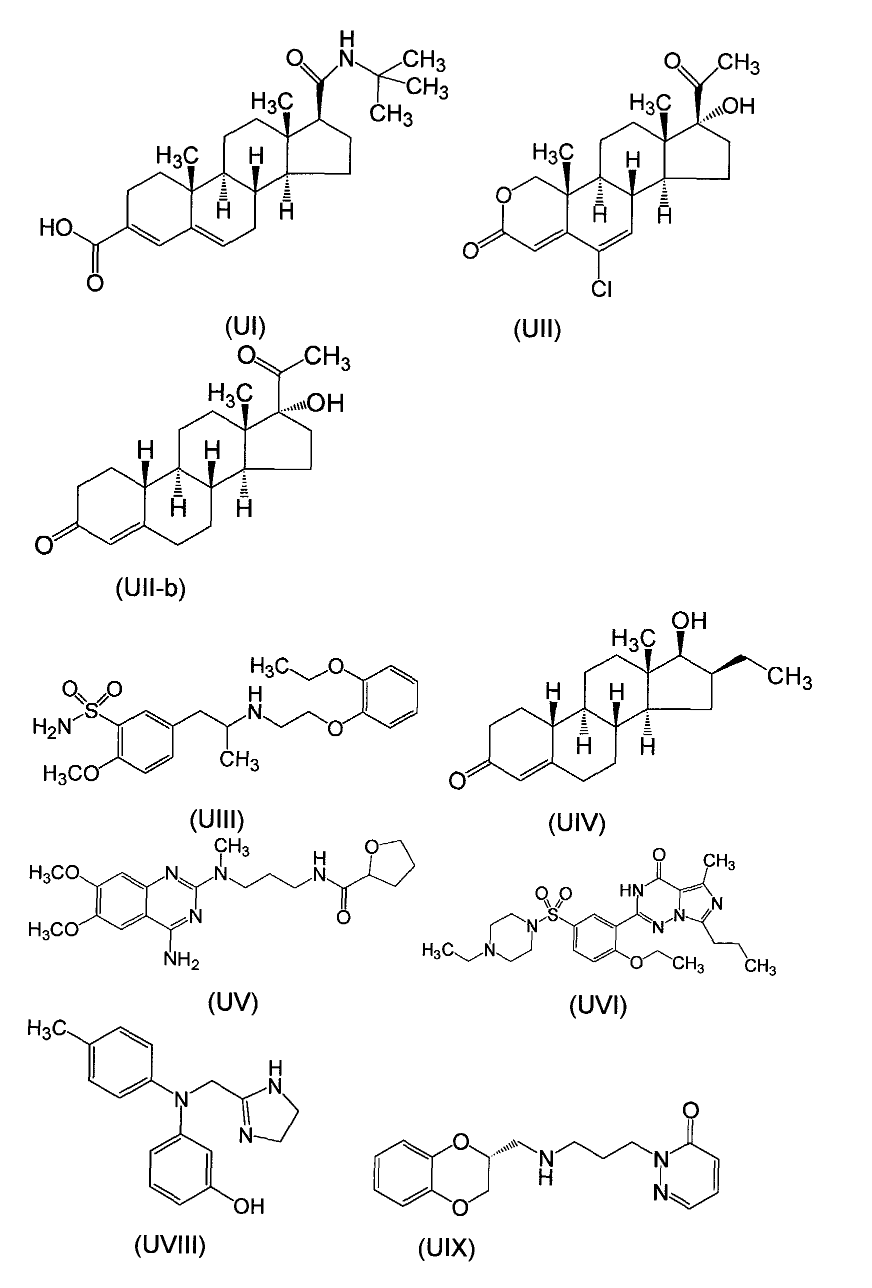 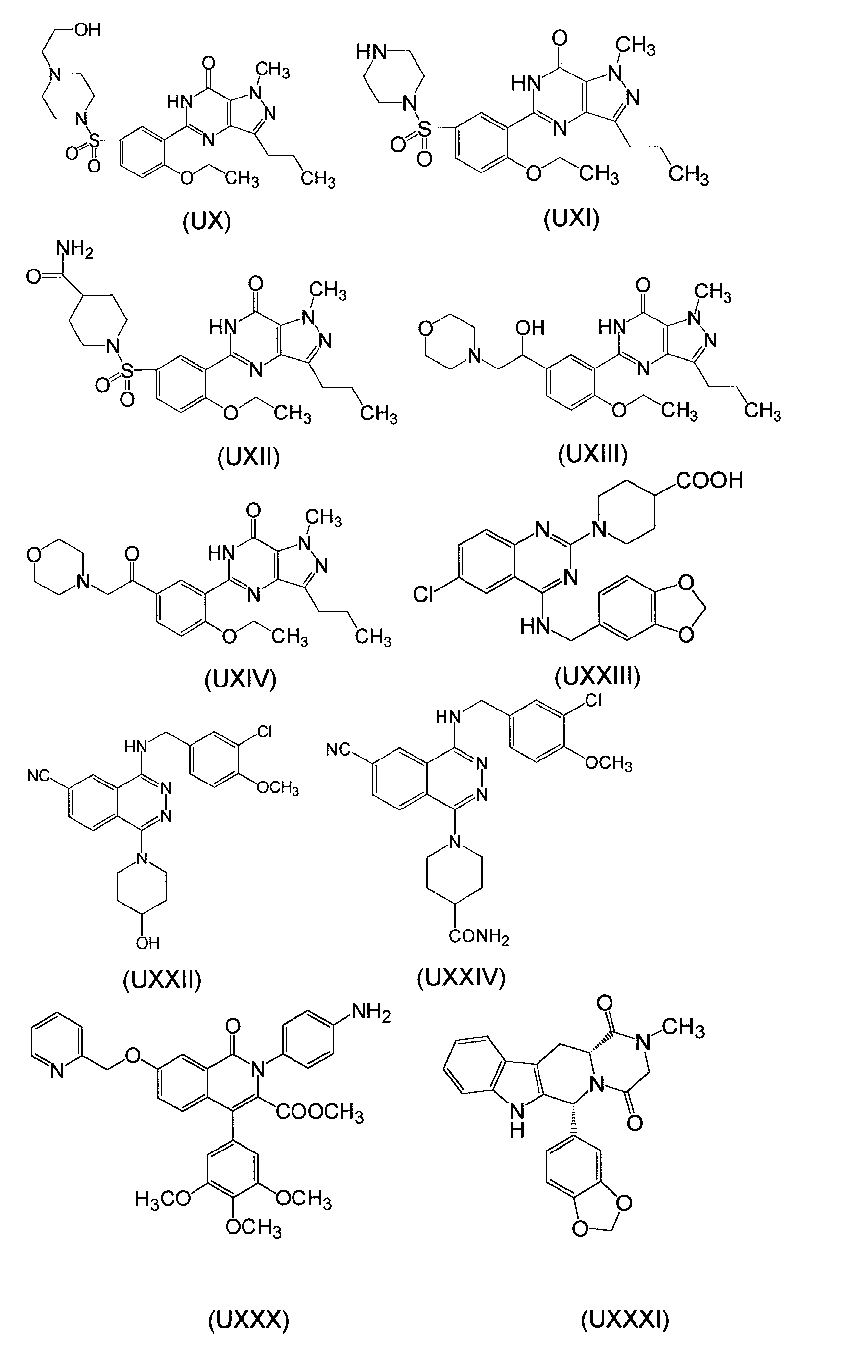 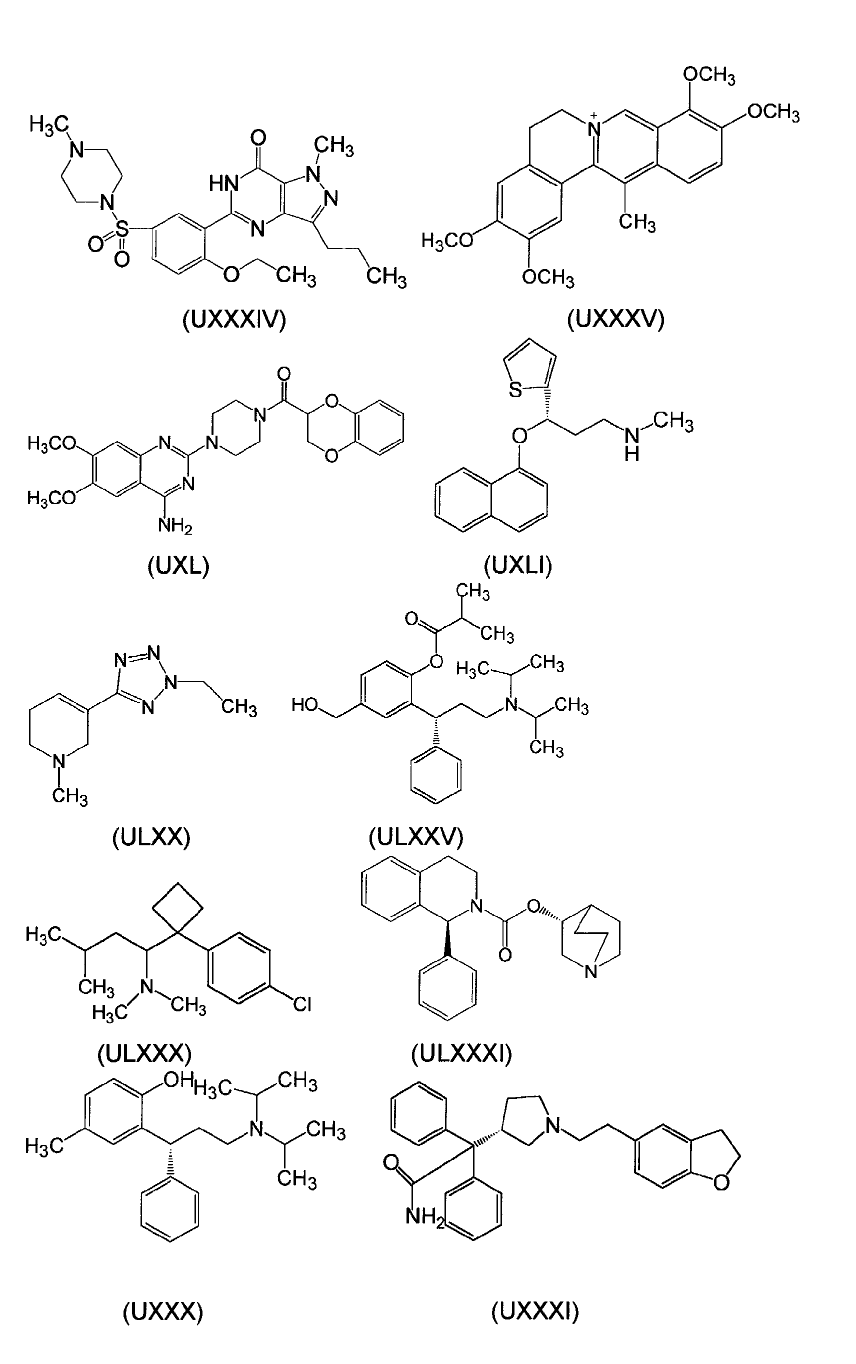 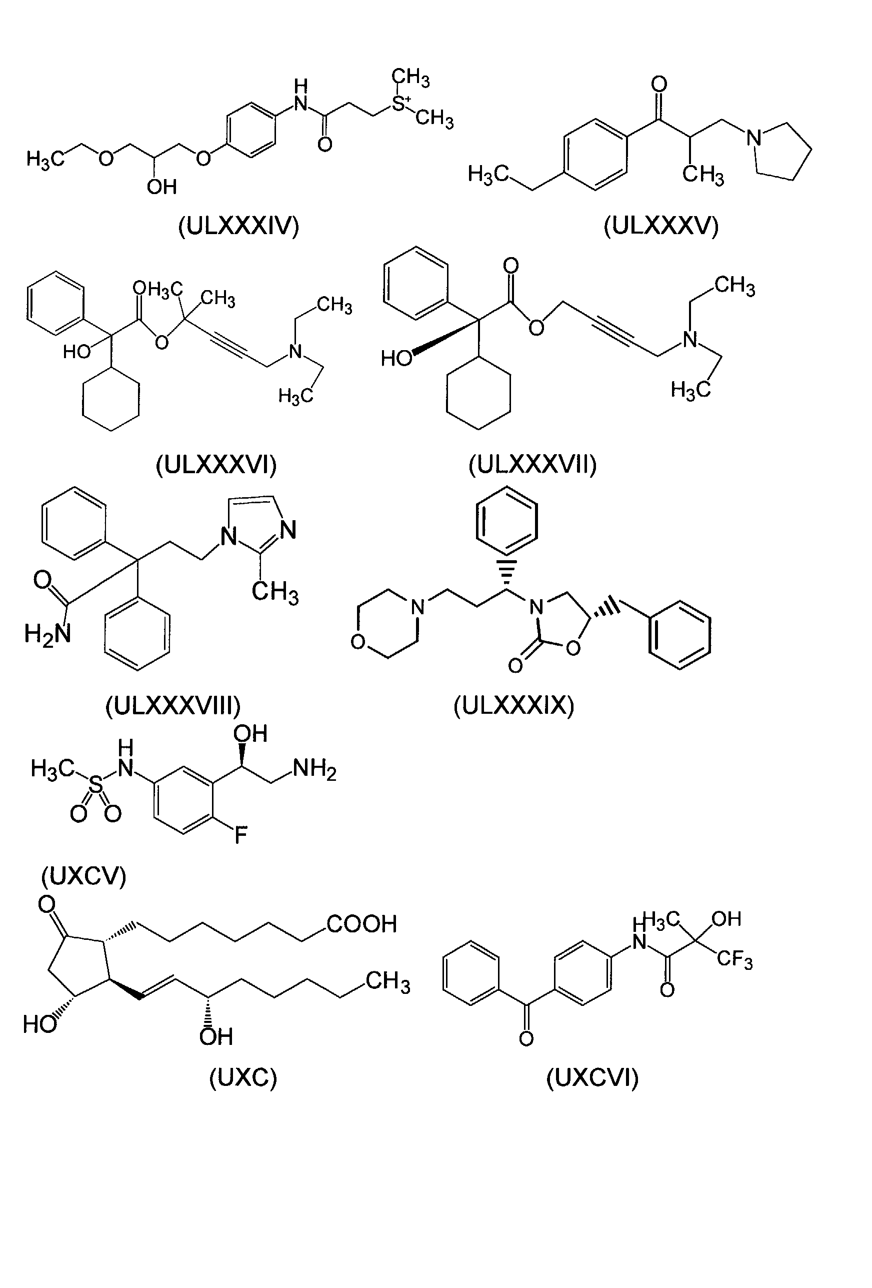 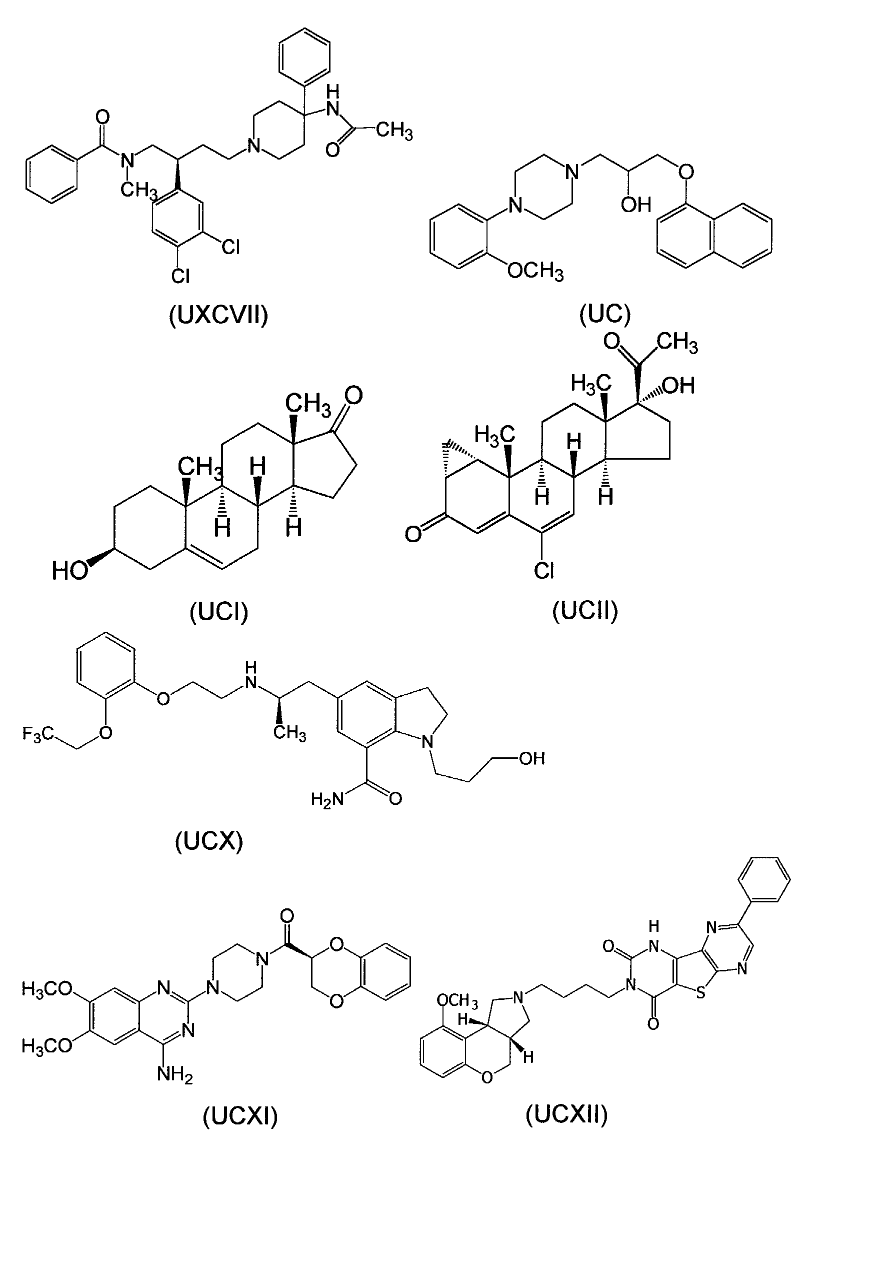 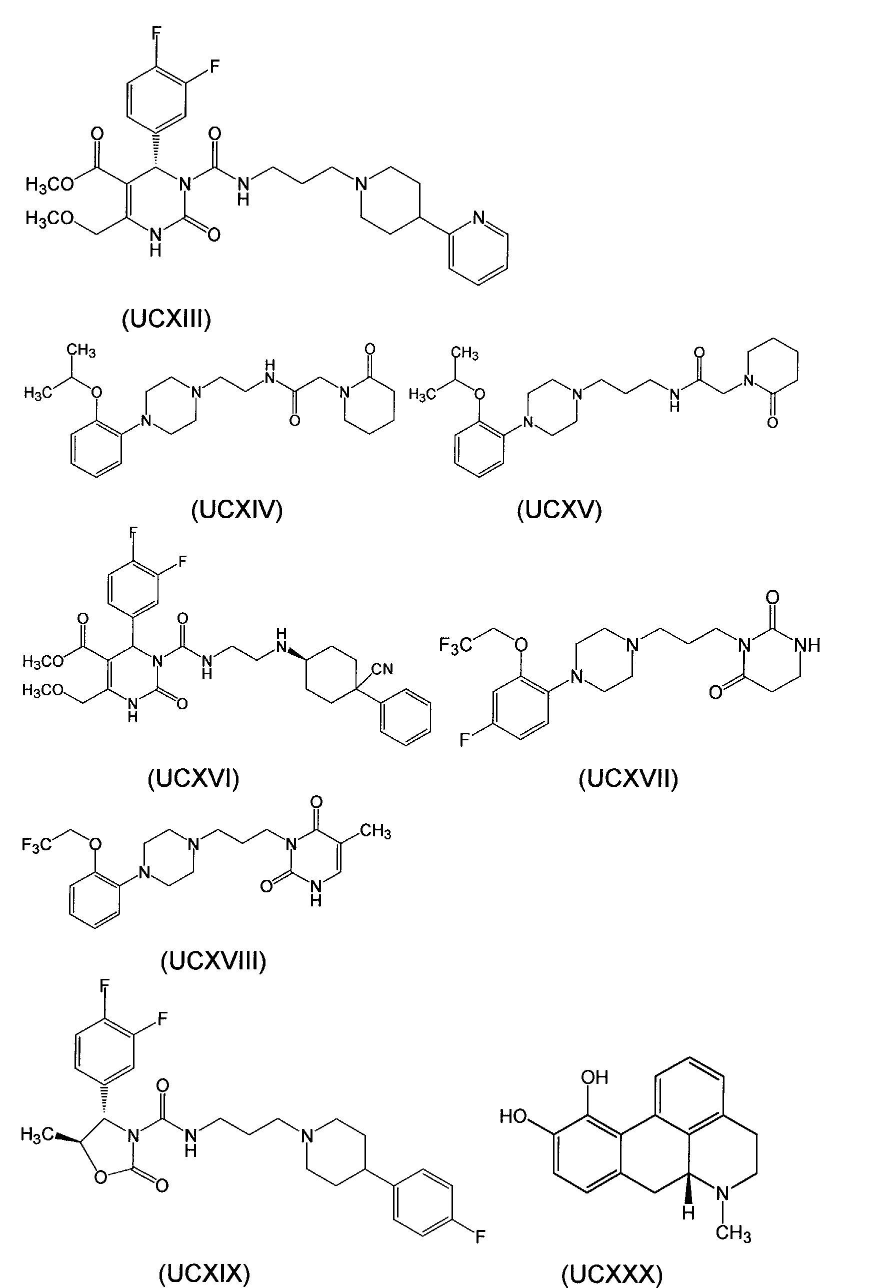 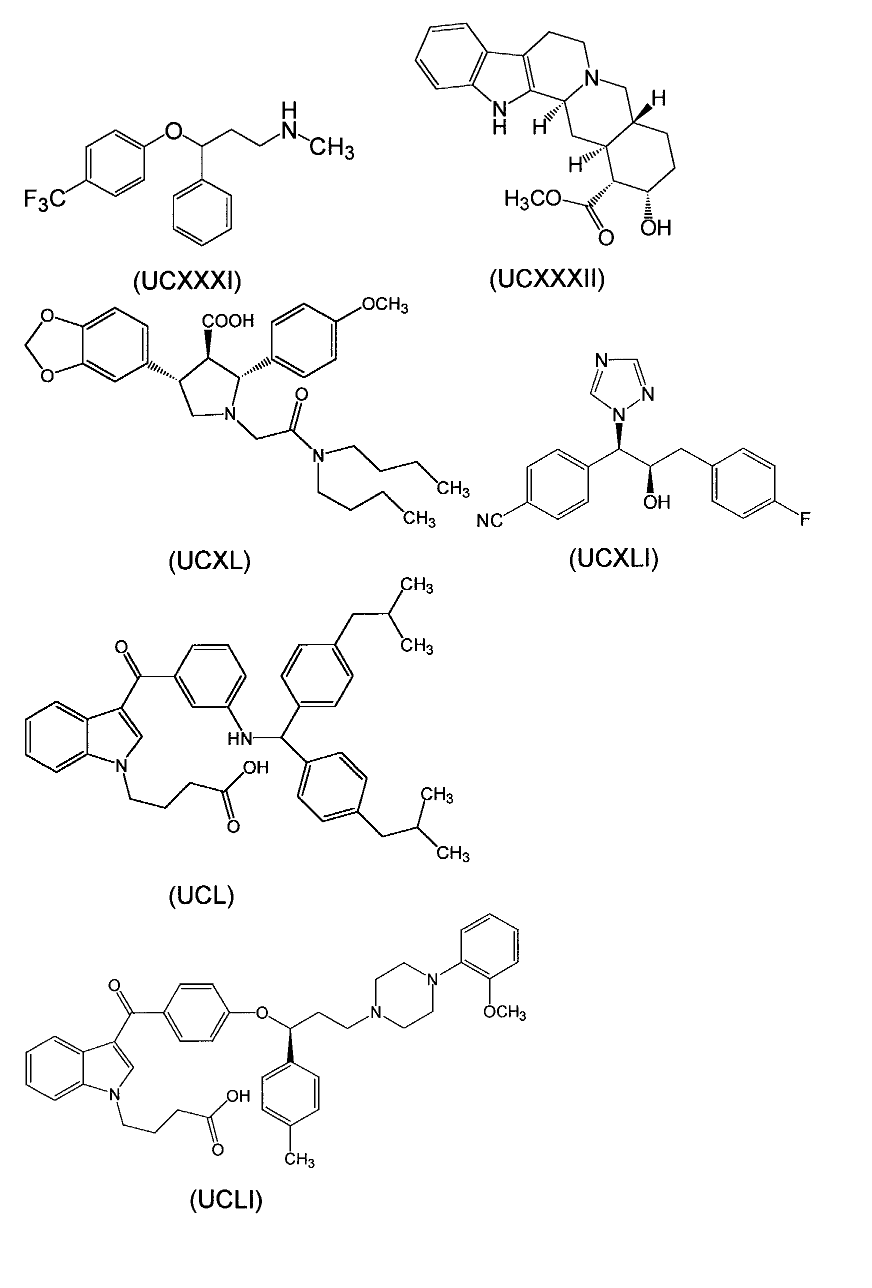 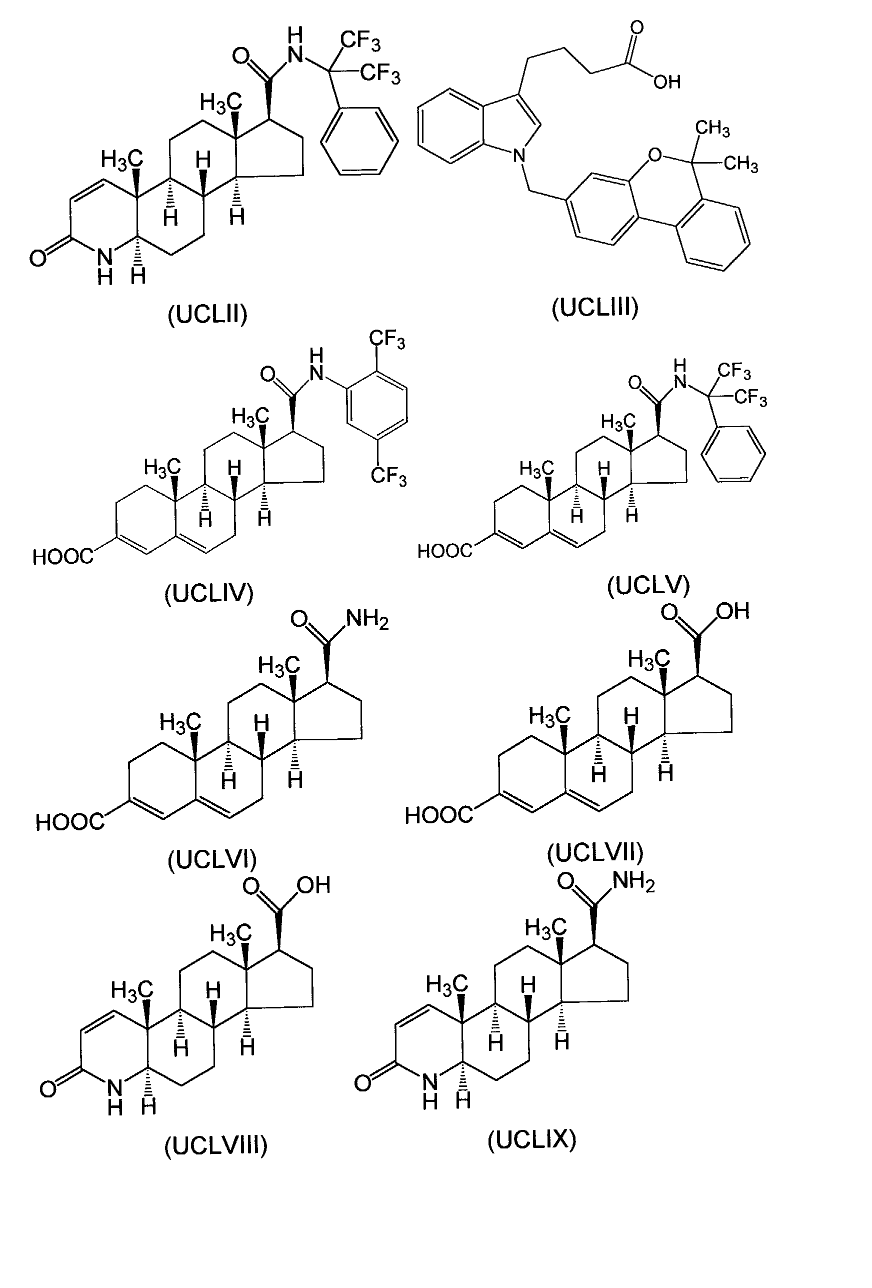 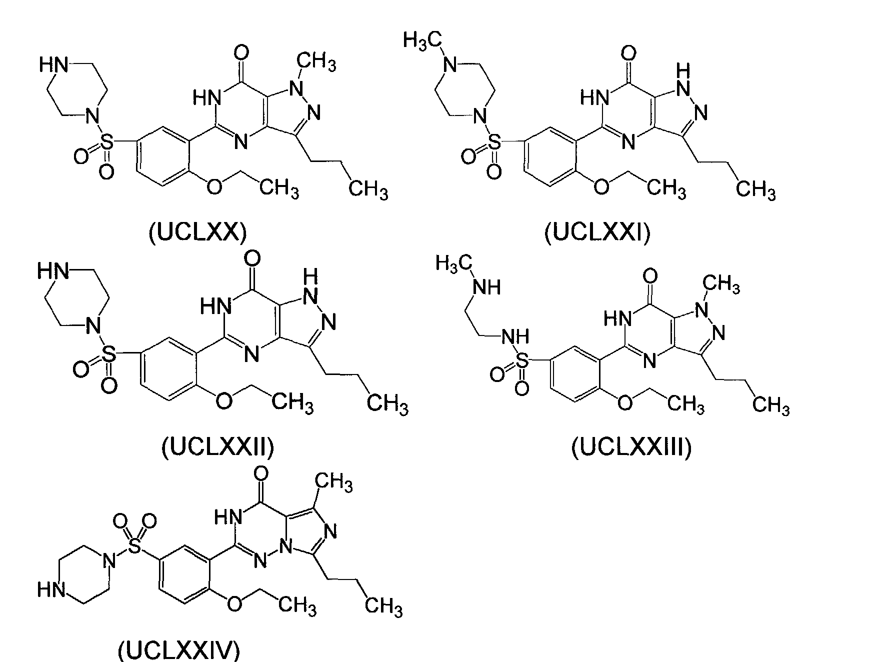 GROUP 8B:
ABT-839 (CNdXLI), AC-7700 (CNaLXIV), AZD-3409, 3-allylfarnesol (CNdXLVI), amifostine (CNeIII), anecortave (CNaXXVI), 4-aminothalidomide (CNaIL), aminoglutethimide, anastrozole, all-trans retinoic acid (CNbLXXII), 2-(2-amino-3-metoxyphenyl)chromone (CNaXXIV), apigenin (CNbXIX), atrasentan (CNcXIII), asulacrine, BAY 59-8862, BB-3644, BMS-214662 (CNdL), BMS-275291, BBR-2778 (CNdXXI), BBR-3438 (CNdXXIV), BBR-3464, BBR-3576, bicalutamide (CNvII), buserelin, butyric acid (CNvIV), camptothecin (CNdll), 9-aminocamptothecin (CNdIII), carboxyamidotriazole (CnaXXII), capecitabine, chlorogenic acid (CNbL), chrysin (CNbXX), CP-461 (Cndl-b), CP-609754, CI-994, CI-1033 (CNcXII), CGP-77675, cilengitide (CNaXXIII), cipemastat, cladribine, combretastatin A4 (CNaLXI), combretastatin A4 phosphate (CNaLXIII), CT-2584, curcumin (CNbLIII), 5,6-dimethylxanthenone-4-acetic acid (CNaLXV), flavinic derivatives (CNbgl), flavonolic and flavonic derivatives (CNbgX), isoflavonic derivatives (CNbgXI), indolinonic derivatives (CNagl), flavanonolic and flavanonic derivatives (CNbgXII), flavanolic derivatives (CNbgXIII), anthocyanidine derivatives (CNbgXIV), thalidomide derivatives (CNagXL), 2-phthalimidino-glutaric acid derivatives (CNagXLI), phthalamidic derivatives (CNagXLII), glucose derivatives (CNdgXL), fructose derivatives (CNdgXLI), galactose derivatives (CNdgXLII), retinoid derivatives (CNbLXX), dexrazoxane (CNeI), 4-dedimethylamino-sancycline (CnaXX), diflomotecan (CNdVII), all-cis-5,8,11,14,17-eicosapentaenoic acid (CNbLXI), daidzein (CNbXXXII), docetaxel, doxorubicin (CNdXXII), D-1927, edatrexate, eflornithine (CNbLXIX), ellagic acid, EO-9 (CNdLXXX), EM-12 (CNaXLV), E-7010, epicatechin (CNbXXVI), ellagic acid, epigallocatechin-3-gallate (CNbXXVII), epirubicin, BMS-247550, BMS-214662, BMS-275291, ERA-923, erbstatin (CNcIV), erlotinib, exemestane, exatecan (CNdVI), fadrozole, fenretinide (CNbLXXIV), finrozole (CNbLXVII), flavin-adeninin-8α-hydroxy-riboflavin (CNbIII), flavine-adenine dinucleotide/FAD, flavone-8-acetic acid (CNaLXVII), flavopiridol (CNaXXV), floxuridine, 5-fluorouracil, formestane (CNbLXIII), 1-fructosamine, (CNdLXXVIII) 2-fructosamine (CNdLXXVI), fustin (CNbXXIV), fumagillol (CNaLXIV), fulvestrant, genistein (CNbXXI), gefitinib, gemcitabine (CNdXXX), 1-glucosamine (CNdLXXIII), glutathione (CNbgXXXV), 2-glucosamine (CNdLXXIV), 6-glucosamine (CNdLXXV), 2-galattosamine (CNdLXXVII), halofuginone (CNaLX), histamine (CNvI), 4-hydroxybutirroxymethyl-retinate (CNbLXXII), 5'-hydroxythalidomide (CNaXLIV), ILX-23-7553, IDN-5109, imatinib/STI-571 (CNcII), imiquimod, hyperin (CnbIL), human endostatin 6-49, indanocine (CNaVI), kaempferol (CNbXVI), kynurenic acid (CNbXXX), ketotrexate, 3-indolemethanol (CNbLXV), irinotecan (CNdIV), irofulven, itriglumide/CR-2945 (CNbXC), leuprorelin, letrozole (CNbLXVIII), losoxantrone (CNdXXIII), ledoxantrone, luteolin (CNbXVIII), lurtotecan, LY-294002 (CNcVI), L-778123 (CNdXL), marimastat (CNcI), megestrol, methotrexate, MEN-11066, mitoxantrone (CNdXX), midostaurin, mivobulin, myricetin (CNbXVII), myo-inositol (CNbLX), N-(4-carboxyphenyl)-retinamide (CNbLXXI), N-hydroxymethyl-thalidomide (CNaXLVII), N-hydroxyphthalimide (CNaXLVIII), N-acetylcysteine, naringenin (CNbXXII), nelzarabine, octreotide (CNaLXVII), oltipraz, oroxylin (CNbXXVIII), OSI-774, 2,2,5,7,8-pentamethyl-6-hydroxychromane (CNbXLIVI), oxandrolone (CNfI), paclitaxel, phenylbutyrate, PD-089828, PD-153035, pemetrexed, perillyl alcohol (CNbLXXV), quercetin (CNbXV), R-115777 (CNdXLII), raltitrexed, razoxane, resveratrol (CNbLI), riboflavin (CNbII), riboflavin monophosphate (CNbIV), roquinimex, RPR-130401 (CNdXLV), 2-phthalimidino glutaric acid (CNaXLVI), piroxantrone, prinomastat (CNcIII), riboflavin tetrabutyrate, riboflavin triacetate (CNbV), rubitecan (9-nitrocamptothecin) (CNdV), PTK-787 (CNaXXI), Sch-66336 (CNdXLIII), β-sitosterol (CNbLXII), seocalcitol (CNdXC), squalamine (CNbLXIV), semaxanib/SU-5416/semoxind (CNall), SCH-57068, silybin (CNbXXXI), STI-481 (CNdX), SPIKET-P (CNcVII), SPC-8490, SCH-66336, SCH-57050, S-3304, SU-6668 (CNaIII), SU-5406, SU-5614, SU-5477, SU-5402 (CNaXI), SU-4793, SU-5204, SU-4942, SU-4984, SU-5212, SU-4312, sulfuretin (CNaXV), sulindac solfone/exisulind (CNdI), tesmilifene, tamoxifene, toremifene, tanomastat (CNcVIII), TAS-103, taxifolin (CNbXXIII), teloxantrone, tezacitabine, theaflavin (CNbXXIV), thalidomide (CNaXLIII), thymitaq, tirilazad (CNbLXIX), α-tocopherol (CNbXL), trolox C (CNbXLI), 2 tocotrienoli (CNbXLII) e (CNbXLIII), topotecan (CNdXXV), triptorelin, trans ferulic acid (CNbLII), triapine, trimetrexate, troxacitabine, ubenimex, 7-hydroxystaurosporine/UCN-01 (CNcV), vorozole, vesnarinone, vitamin E acid succinate (CNbXLIV), VX-710, YM-511, wogonin (CNbXXIX), ZD-1839/Iressa (CNcIX), ZD-4190 (CNcXI), ZD-6126, ZD-9331, ZK-222584, (CNaIV), (CNaV), (CNaVII), (CNaXII), (CNaXIII), (CNaXIV), (CNaXVI), (CNaXLb), (CNagL), (CNaLXII), (CNbgXXXII), CNdVIII), (CNdIX), (CNdXLIV) (CNdXLVII), (CNdXLVIII), (CNdXLIX), (CNbXXXV), (CNbXXXVI), (CNbXXXVII), (CNbXXXVIII), (CNbXXXIX), (CNvIII), (CNcX) 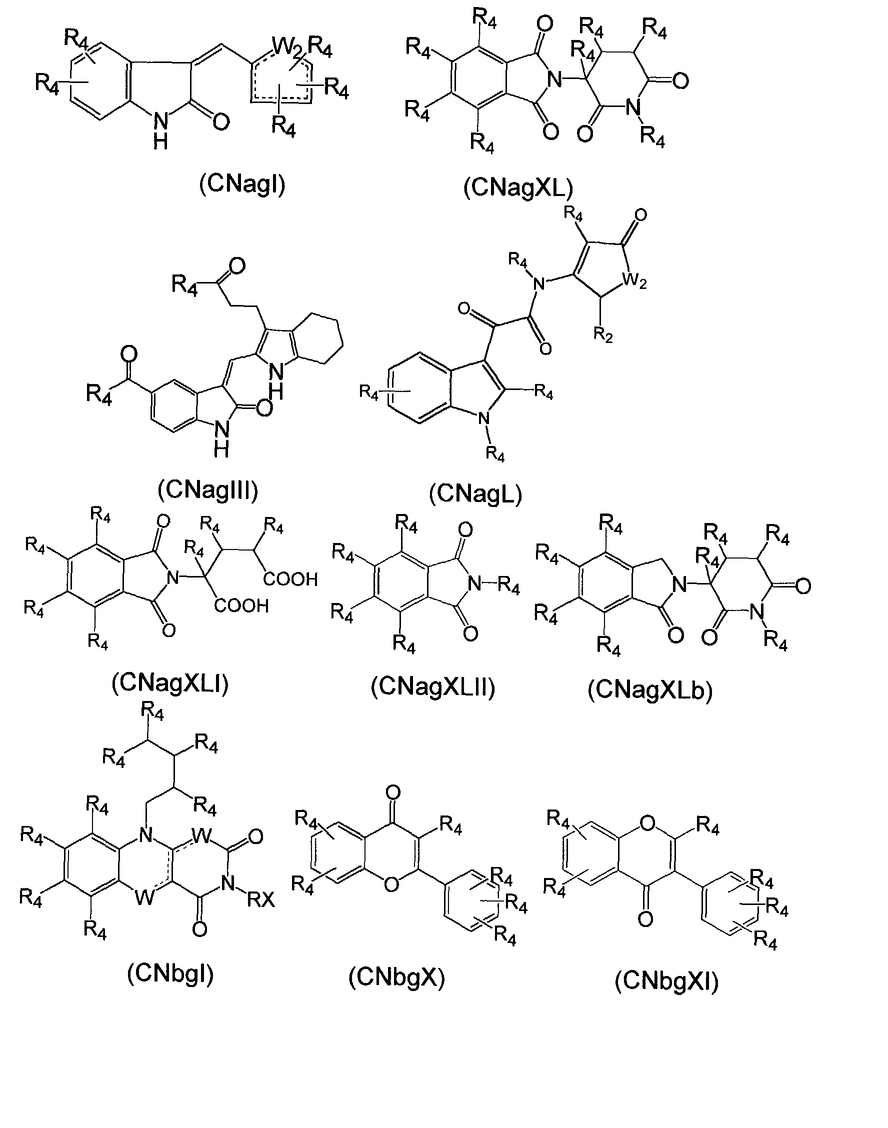 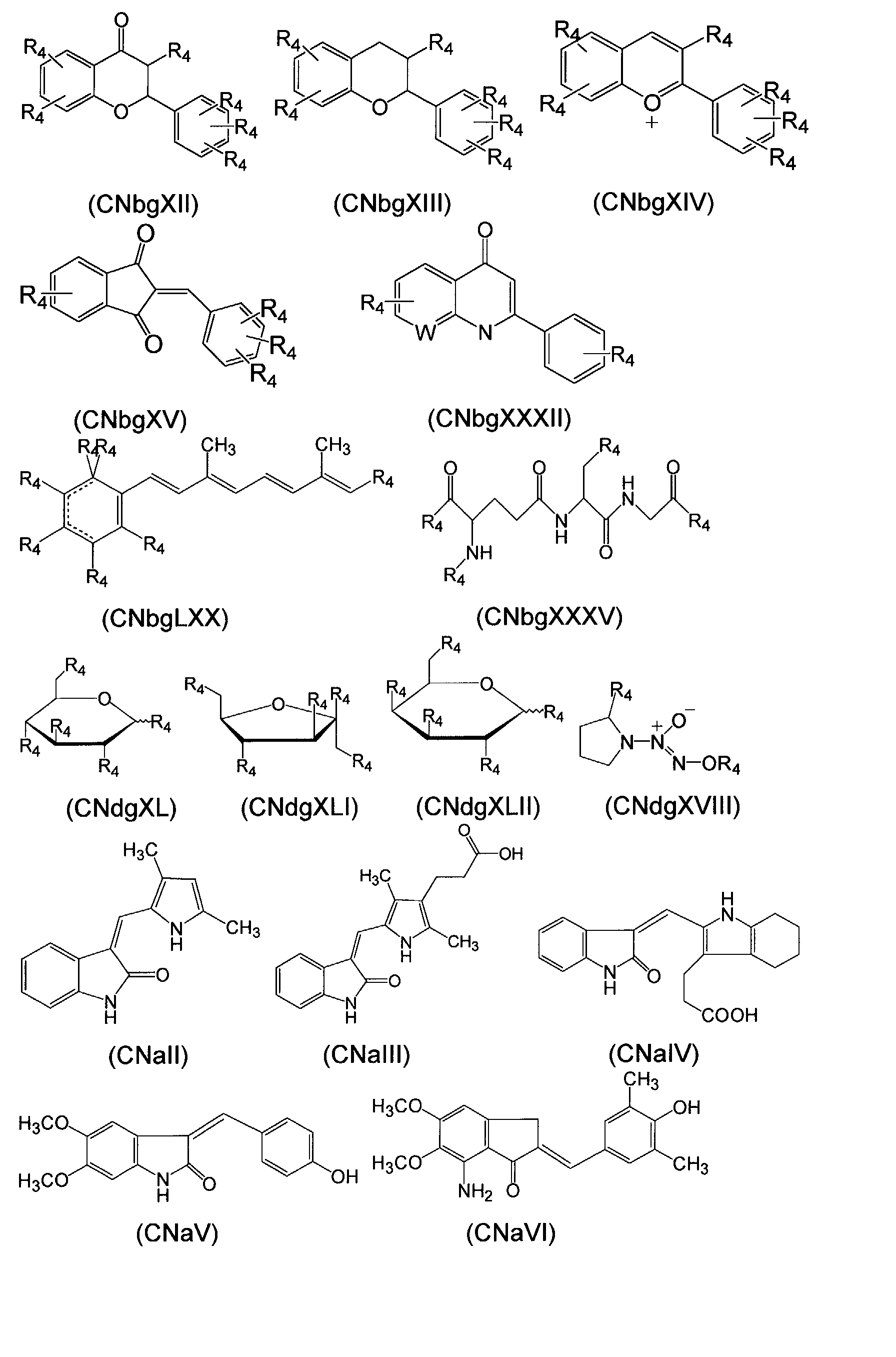 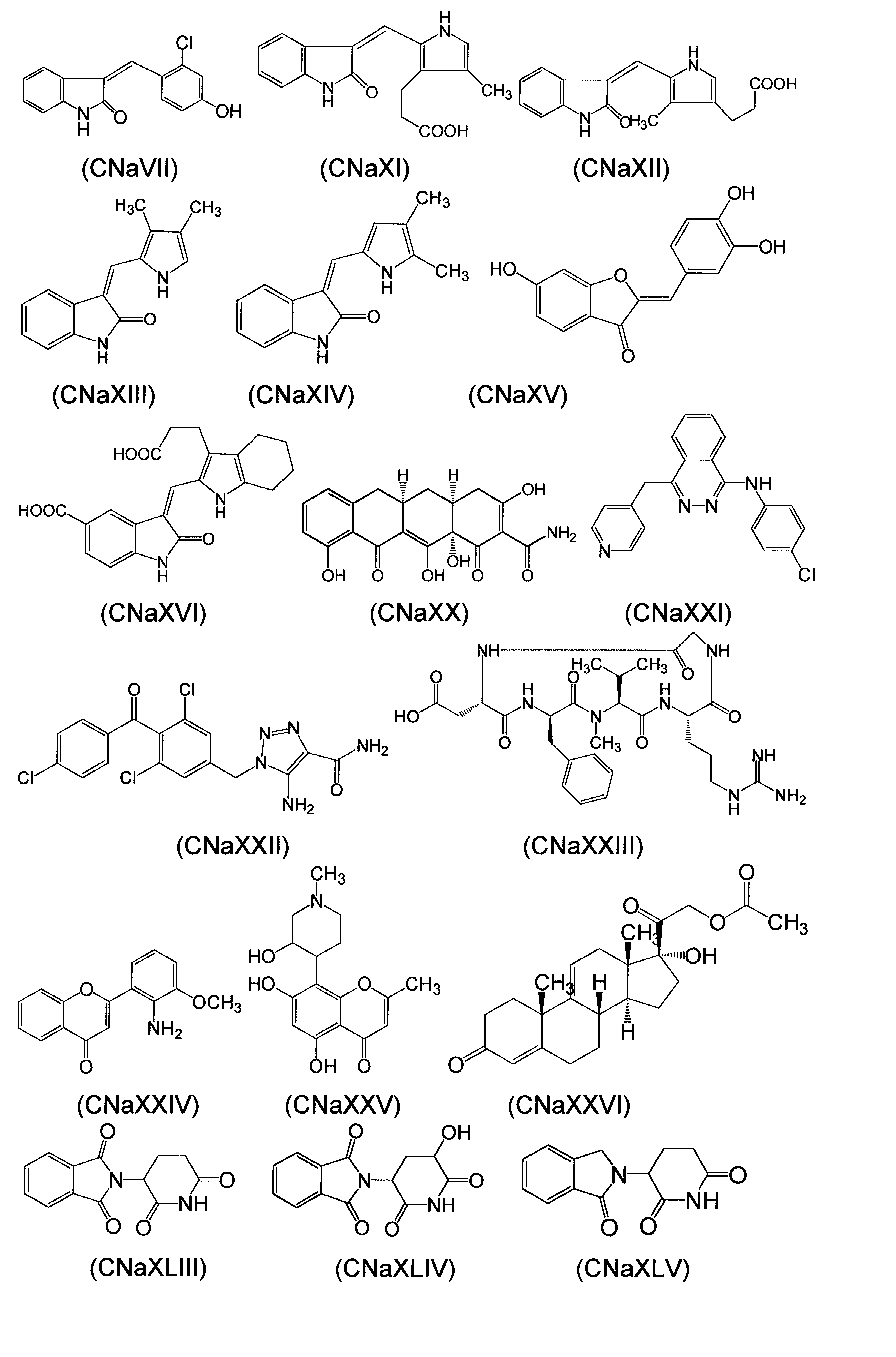 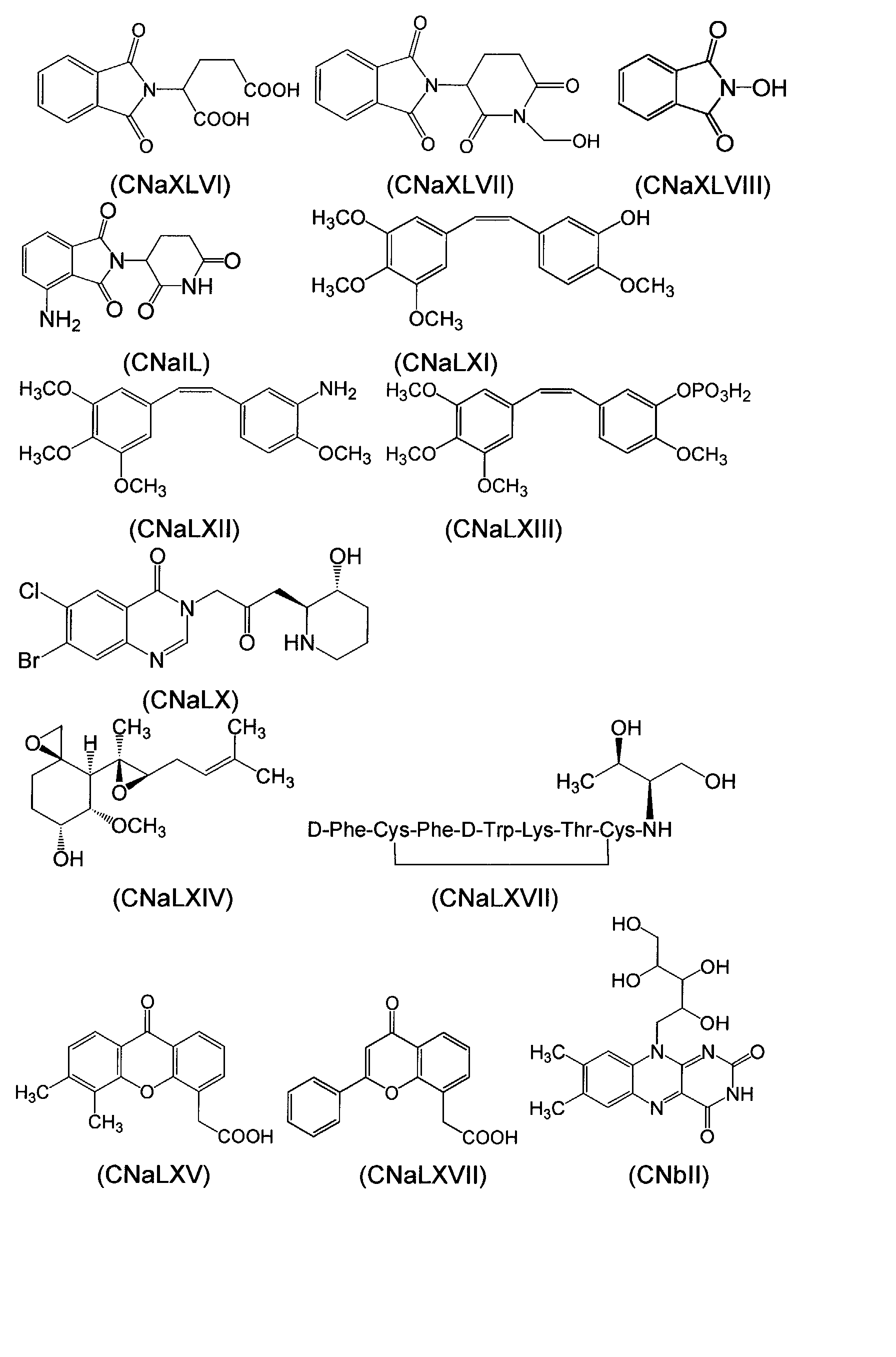 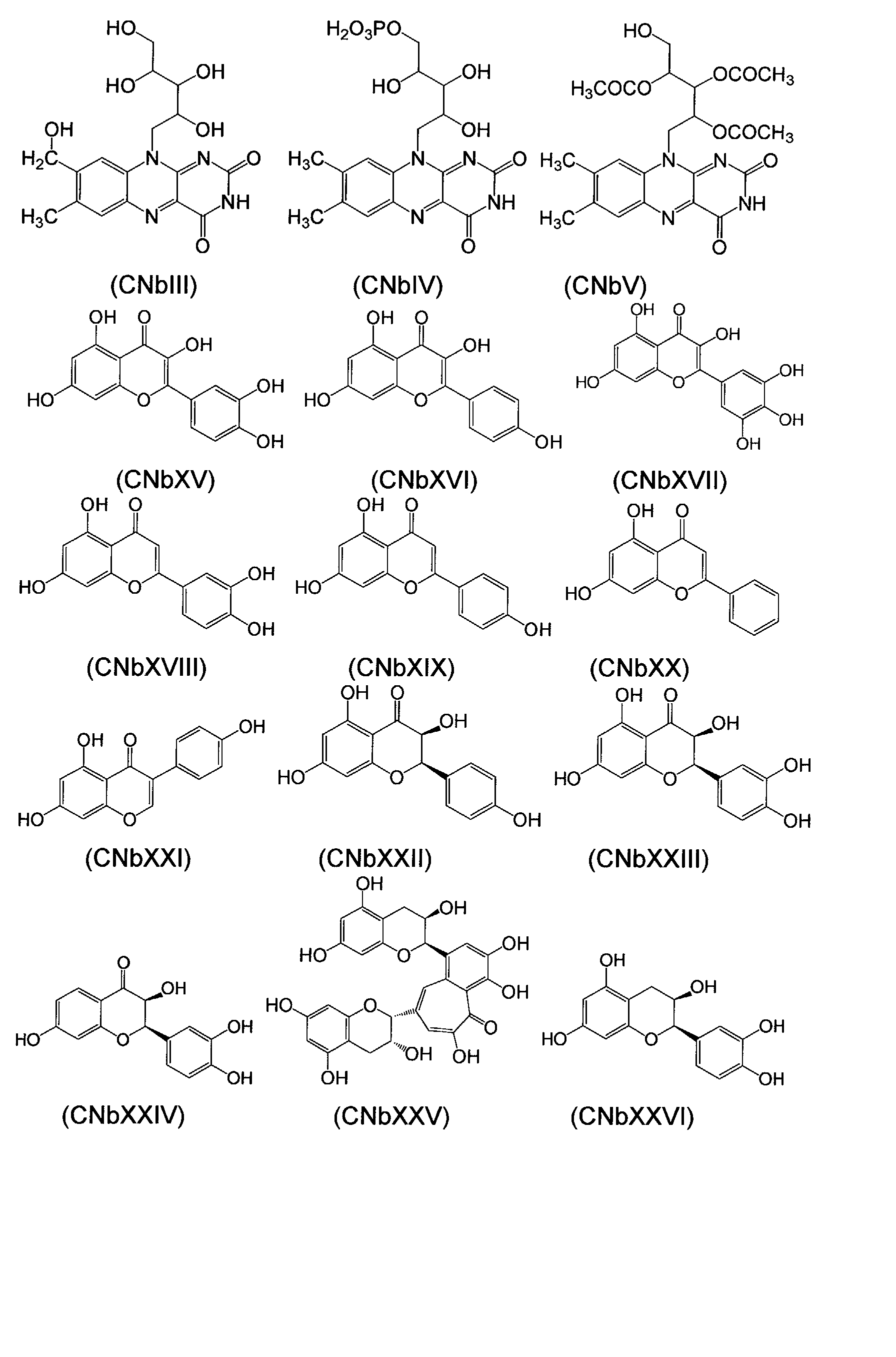 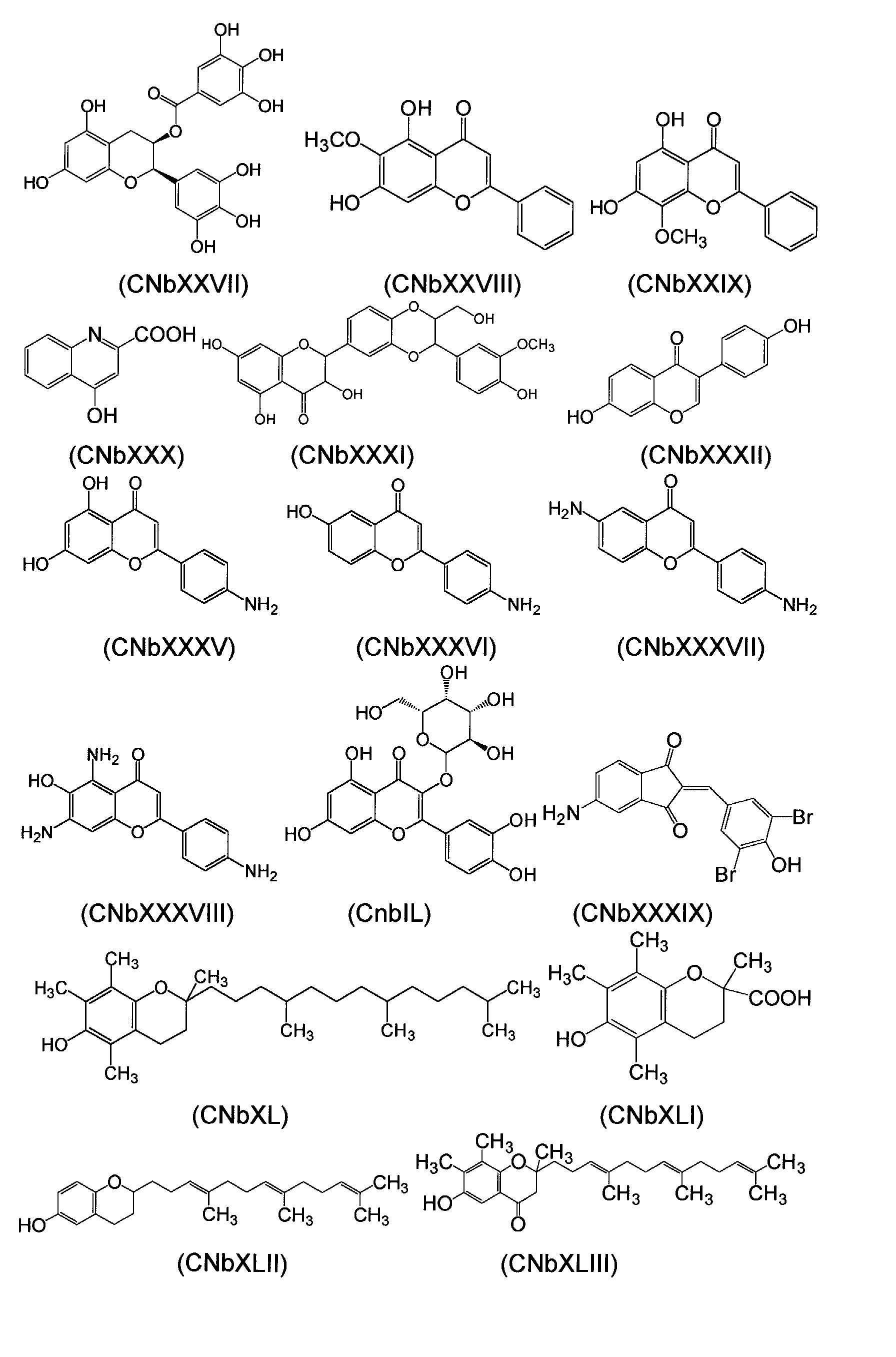 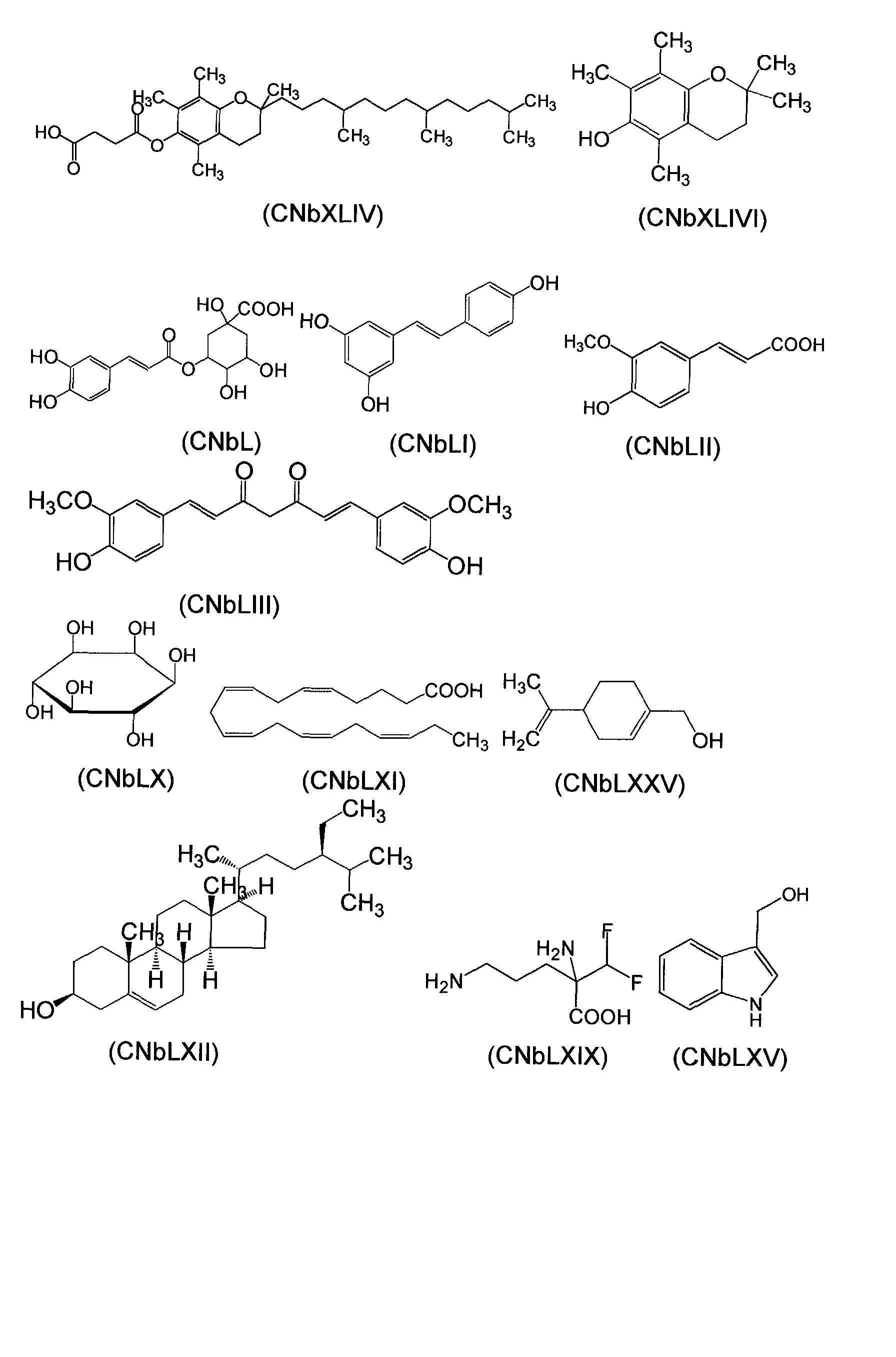 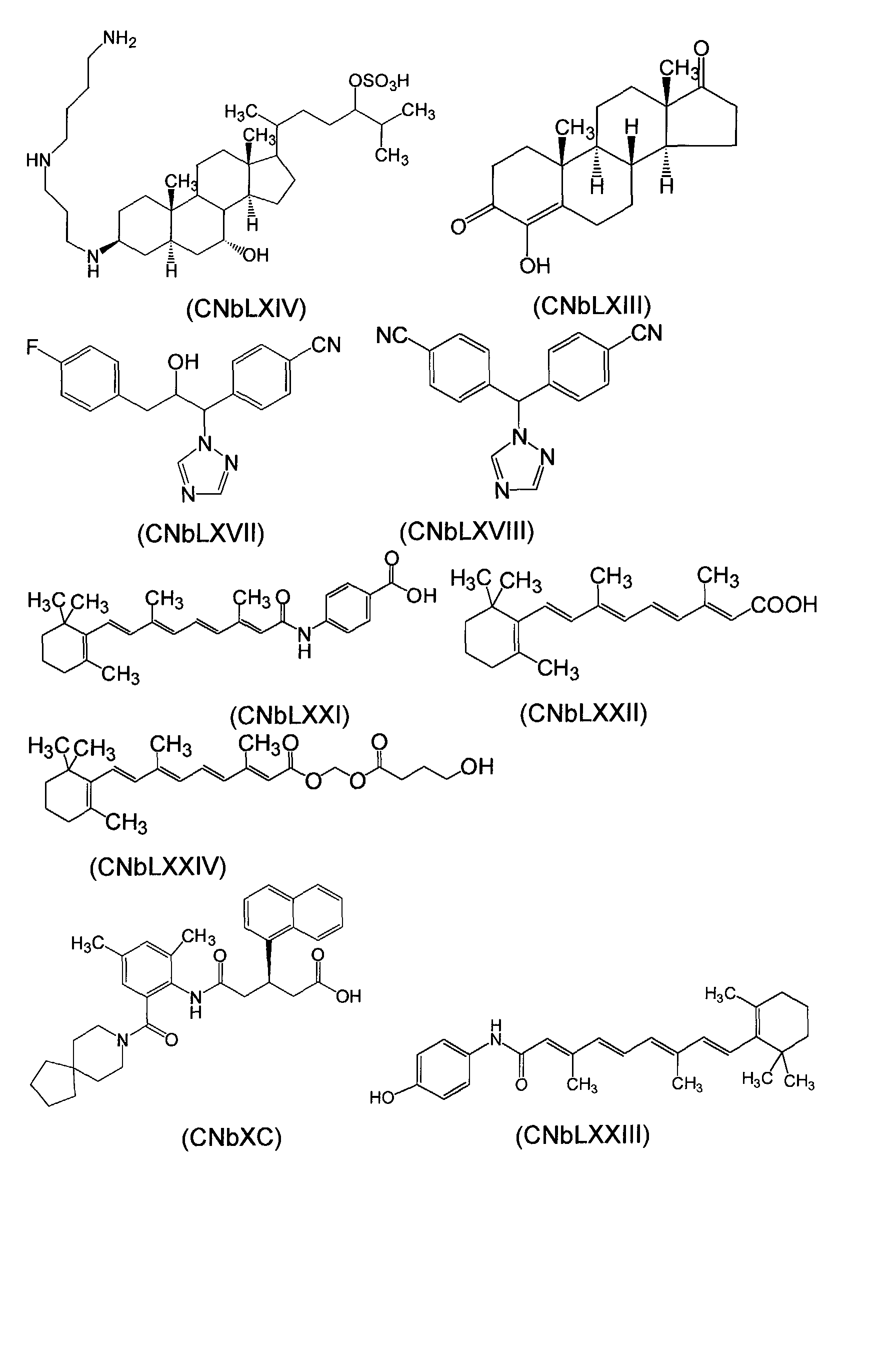 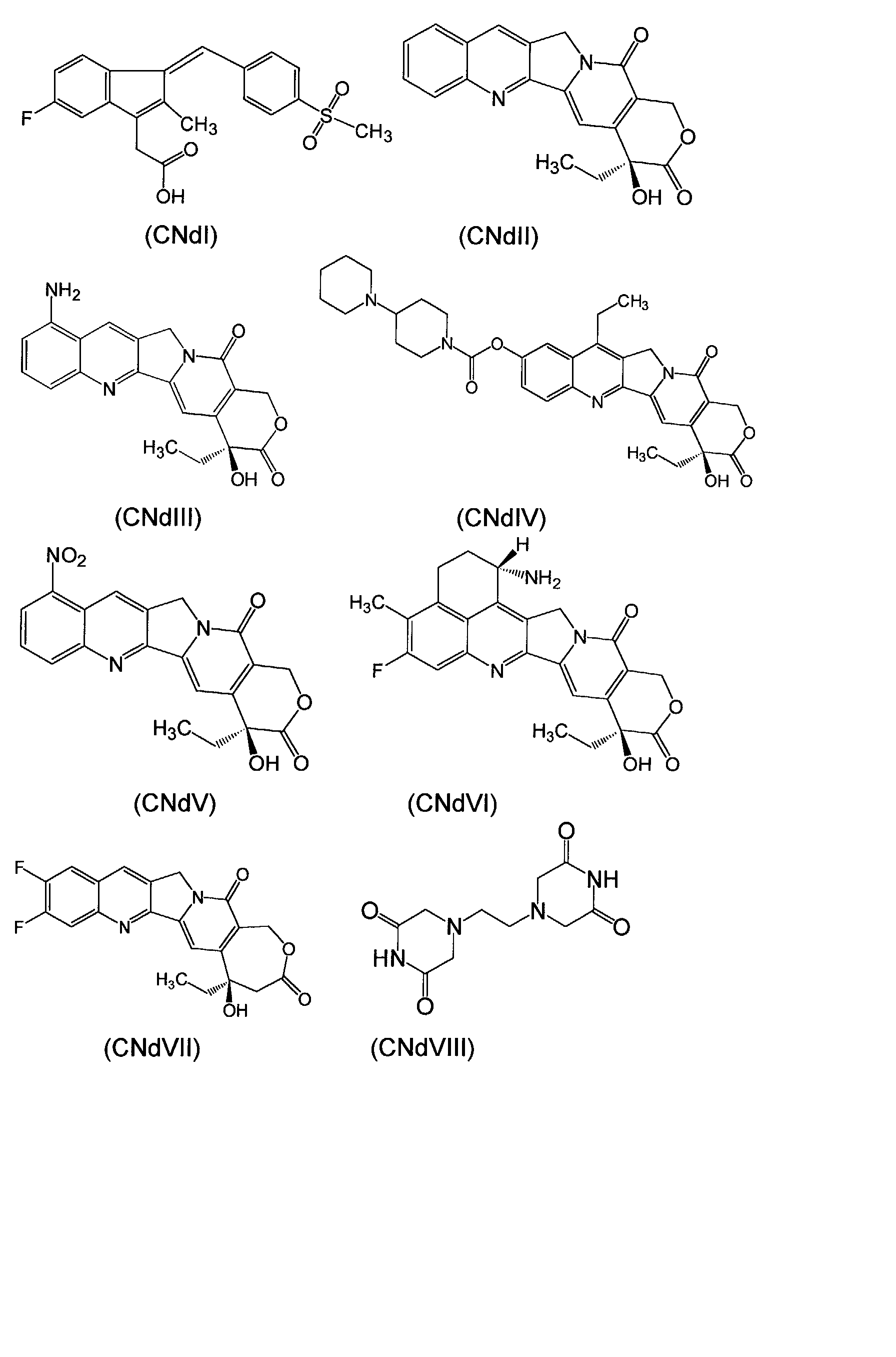 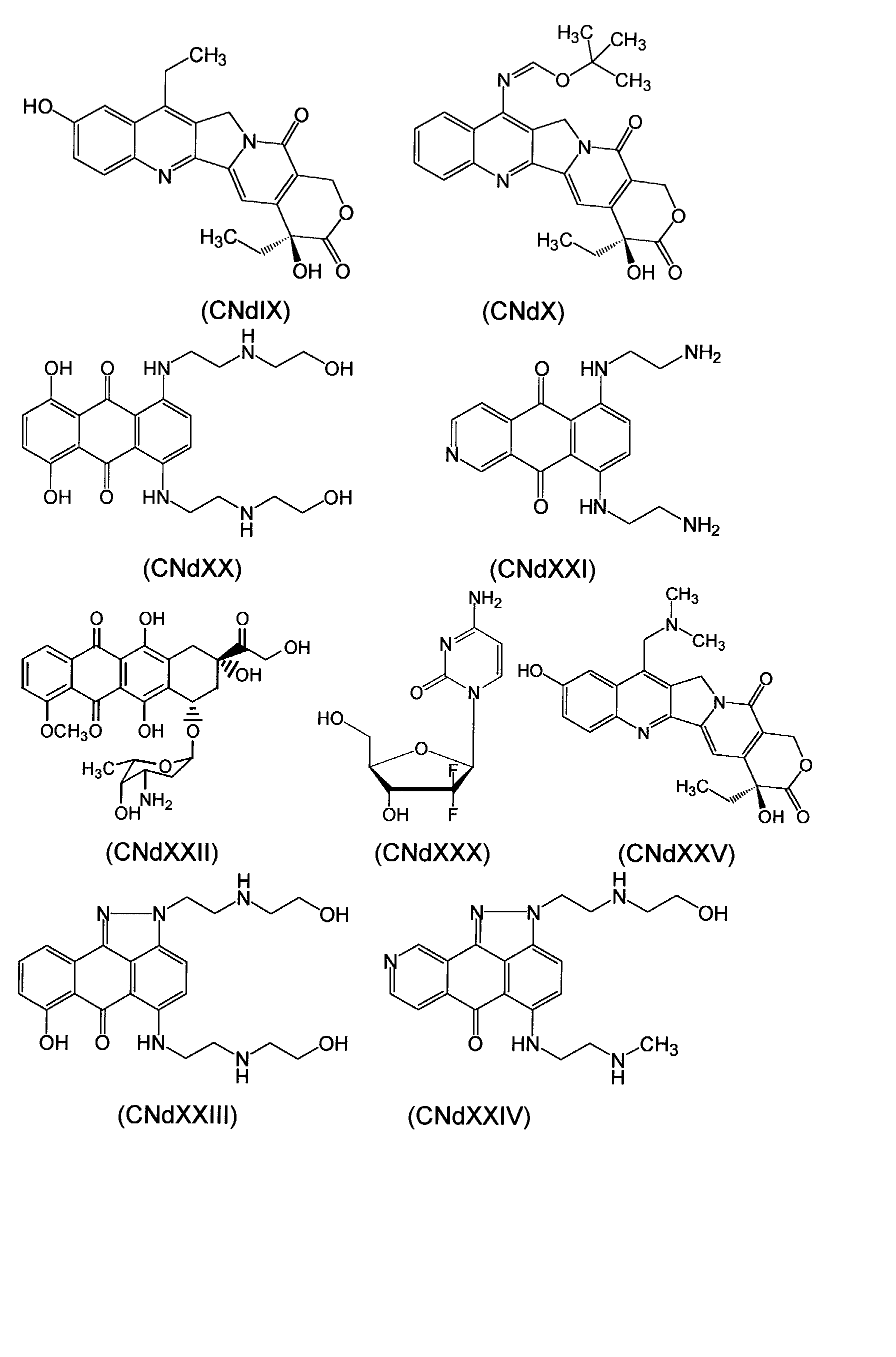 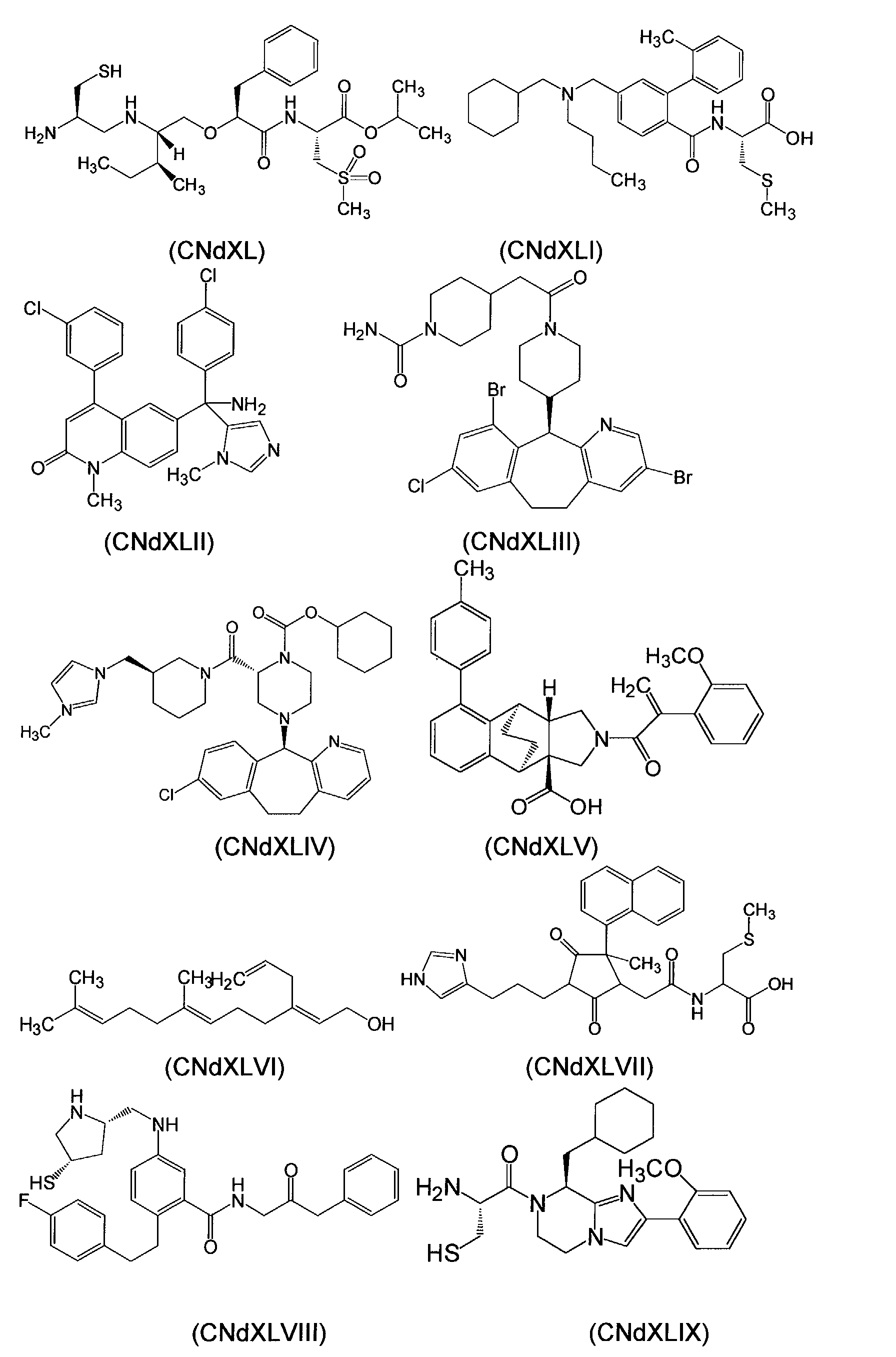 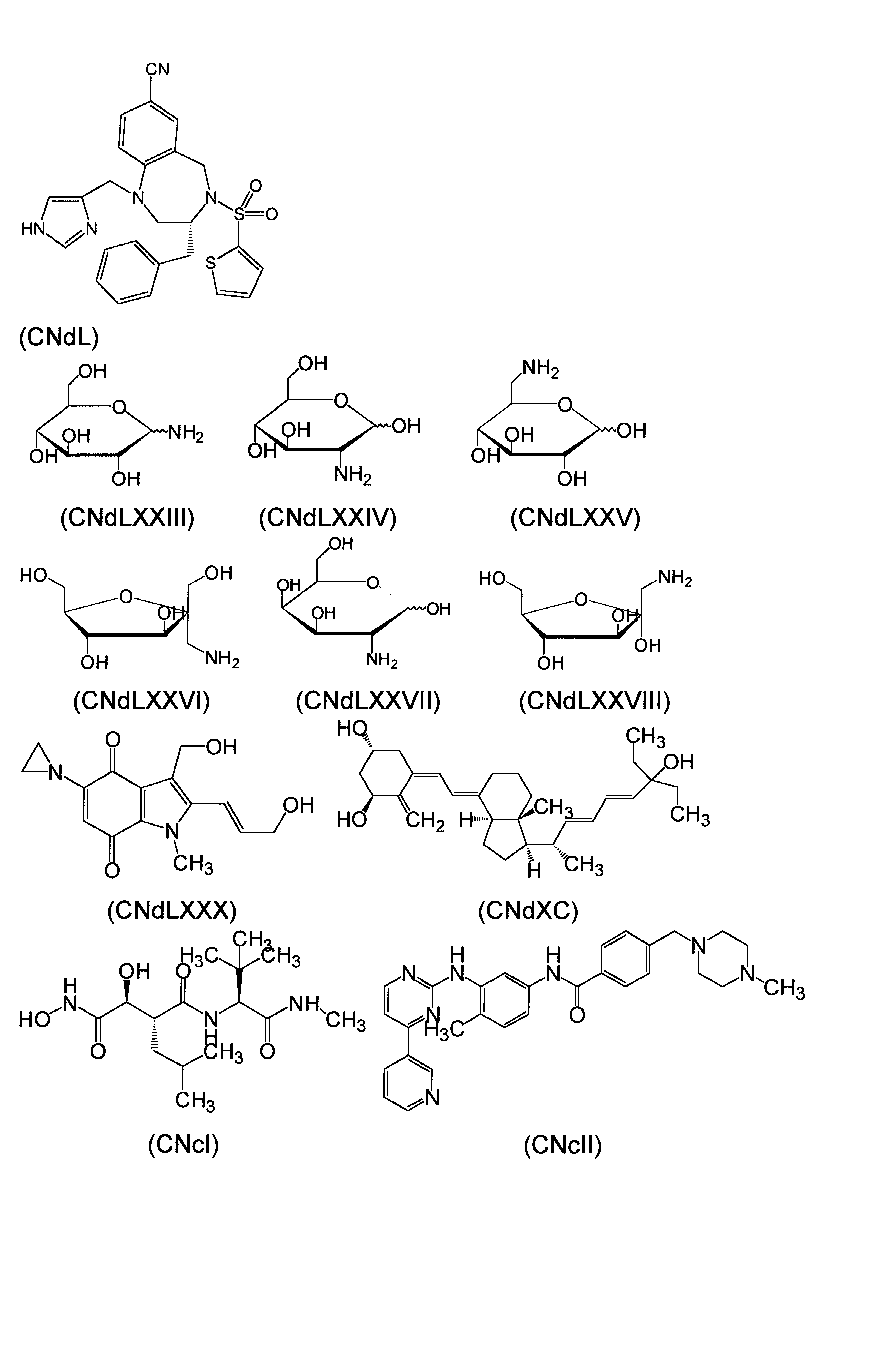 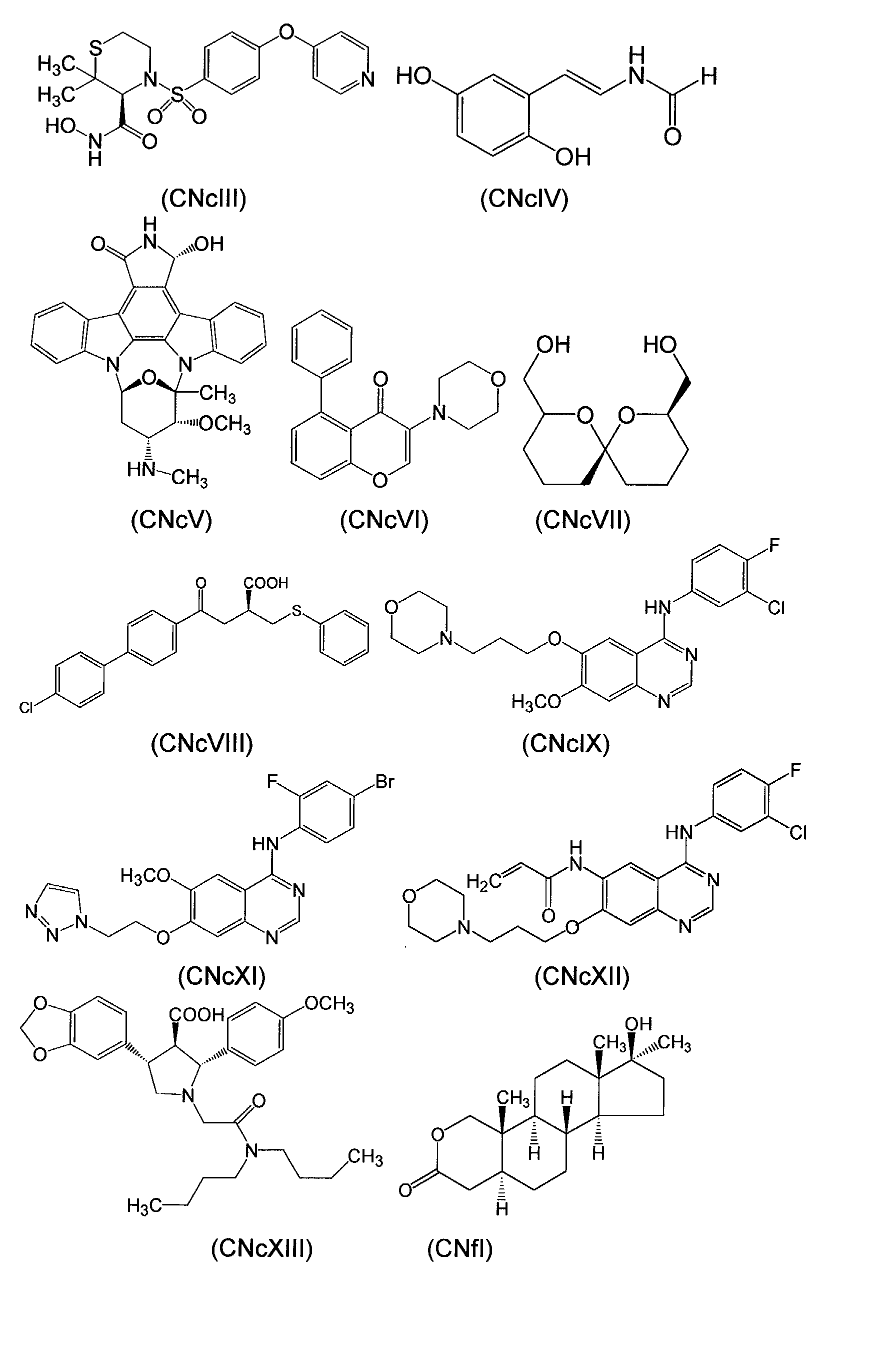 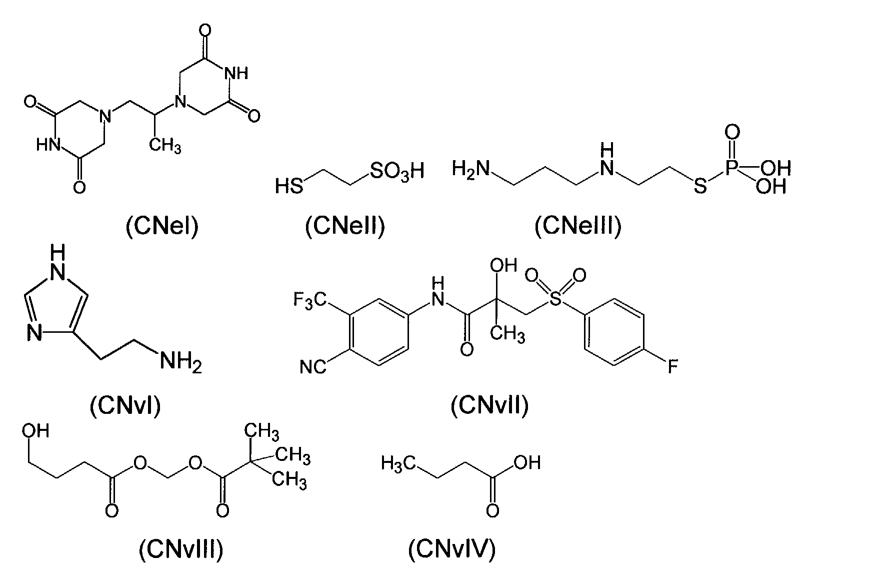 where:
- R₄ is
- COOH; -F; -Cl; -Br; -CF₃; -NO₂, -CHO, -CN, -N₃,
- R₂ ; -O-R₂; -O-(CO)-R₂; -N(R₂)₂; -N(R₂)₂-(CO)-R₂; -R₁-O-R₂;
- R₁-S-R₂; -R₁-NR₂-R₂;-O-R₁-O-R₂;-O-R₁-S-R₂;-O-R₁-NR₂-R₂;
- S-R₂; -S-R₁-O-R₂; -S-R₁-S-R₂; -S-R₁-NR₂-R₂;-NR₂-R₂;
- NR₂-R₁-O-R₂; -NR₂-R₁-S-R₂; -NR₂-R₁-NR₂-R₂;-(CO)-R₂;
- (CO)-O-R₂; -(CO)-R₁-O-R₂;-(CO)-R₁-S-R₂;-(CO)-R₁-NR₂-R₂;
- PO(O-)O-R₂; -SO₂-R₂; -O-SO₂-R₂; -SO₂-O-R₂;
- R₁, -R₂, W and the dotted line are described as above
- W₂ is -O-, -NH-, -NH=, -N(R₂)-, -CH=CH-, -S-; =CH-
- n is an integer between 1 and 5, preferably 1
- m is an integer between 0 and 5

8. Compounds according to claims 1-7 chosen among the following 8 groups of compounds 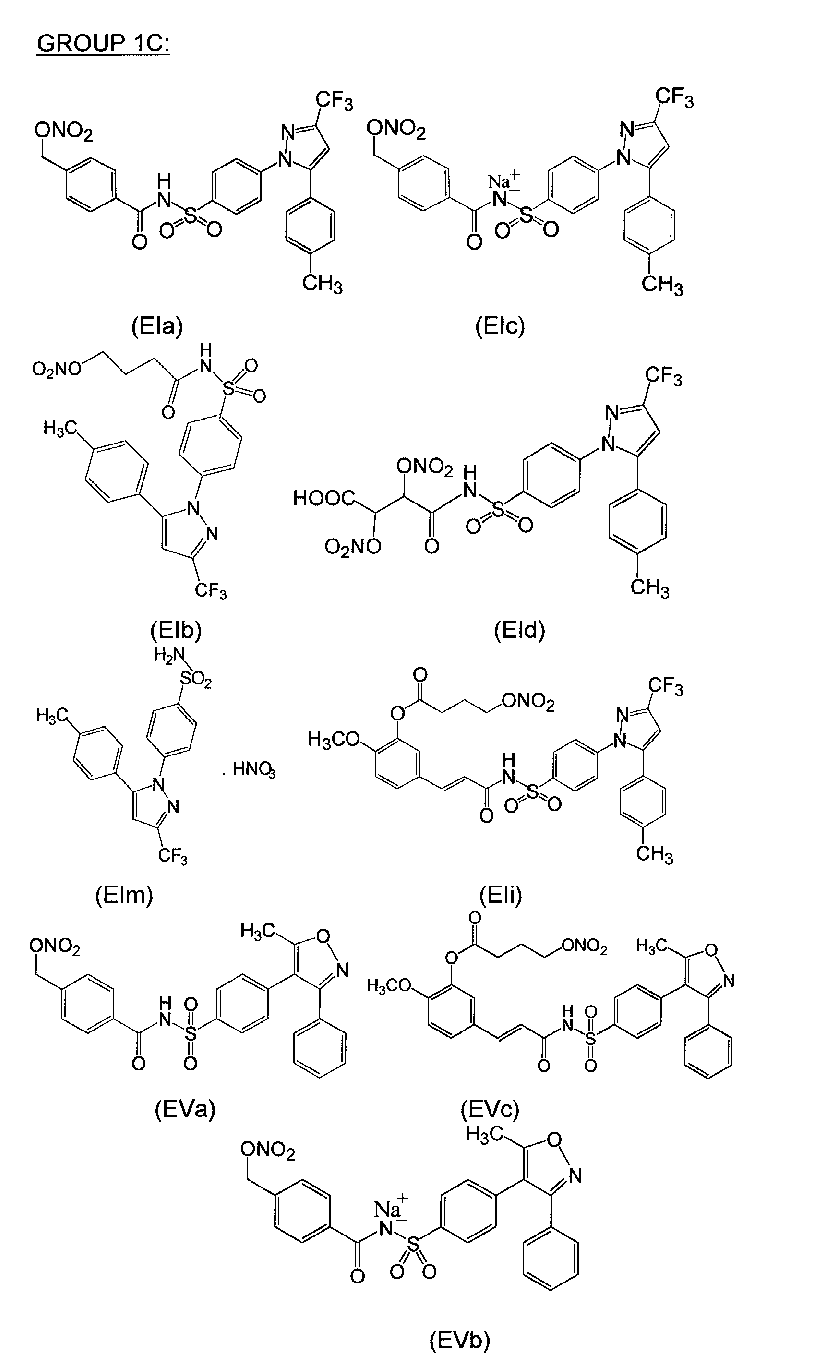 7

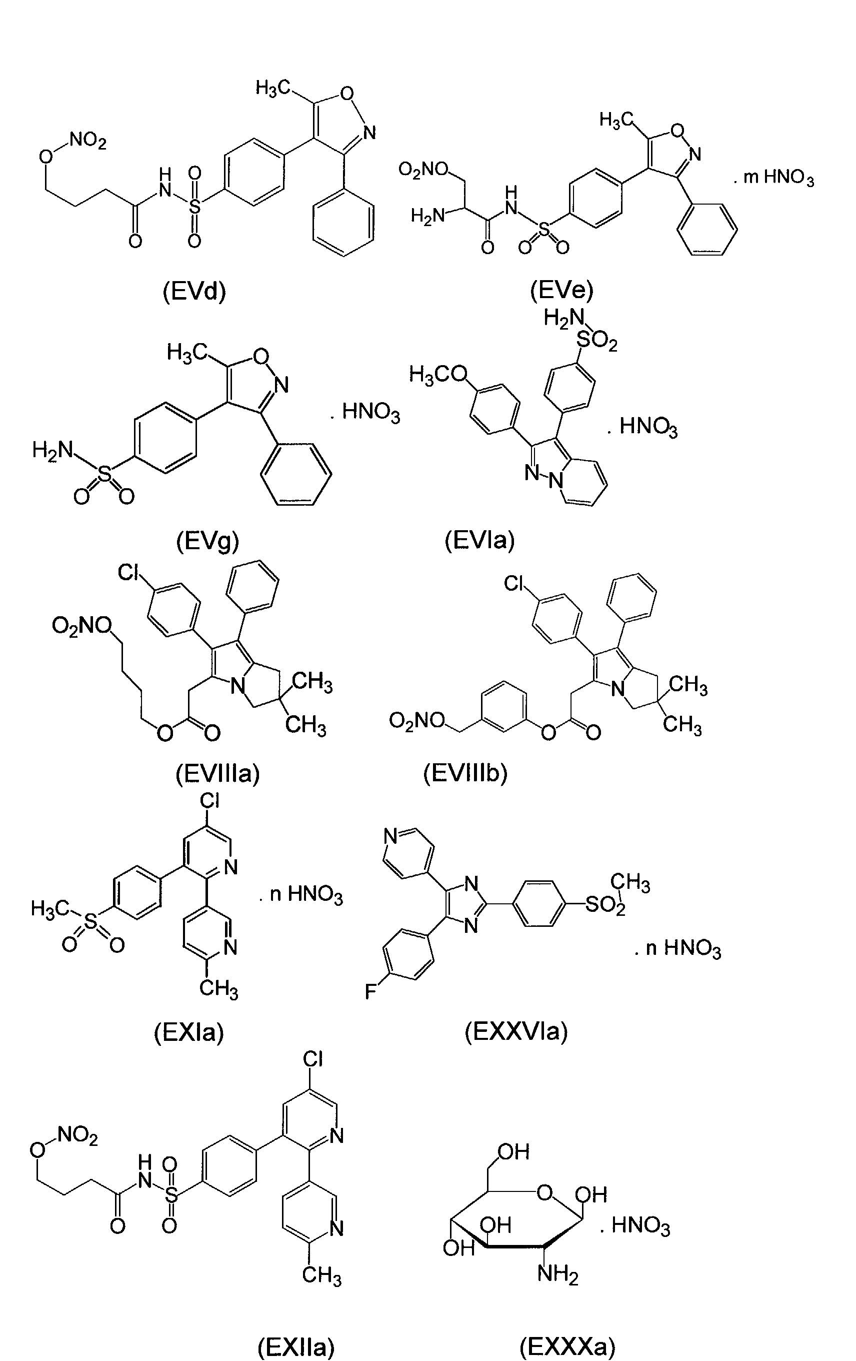

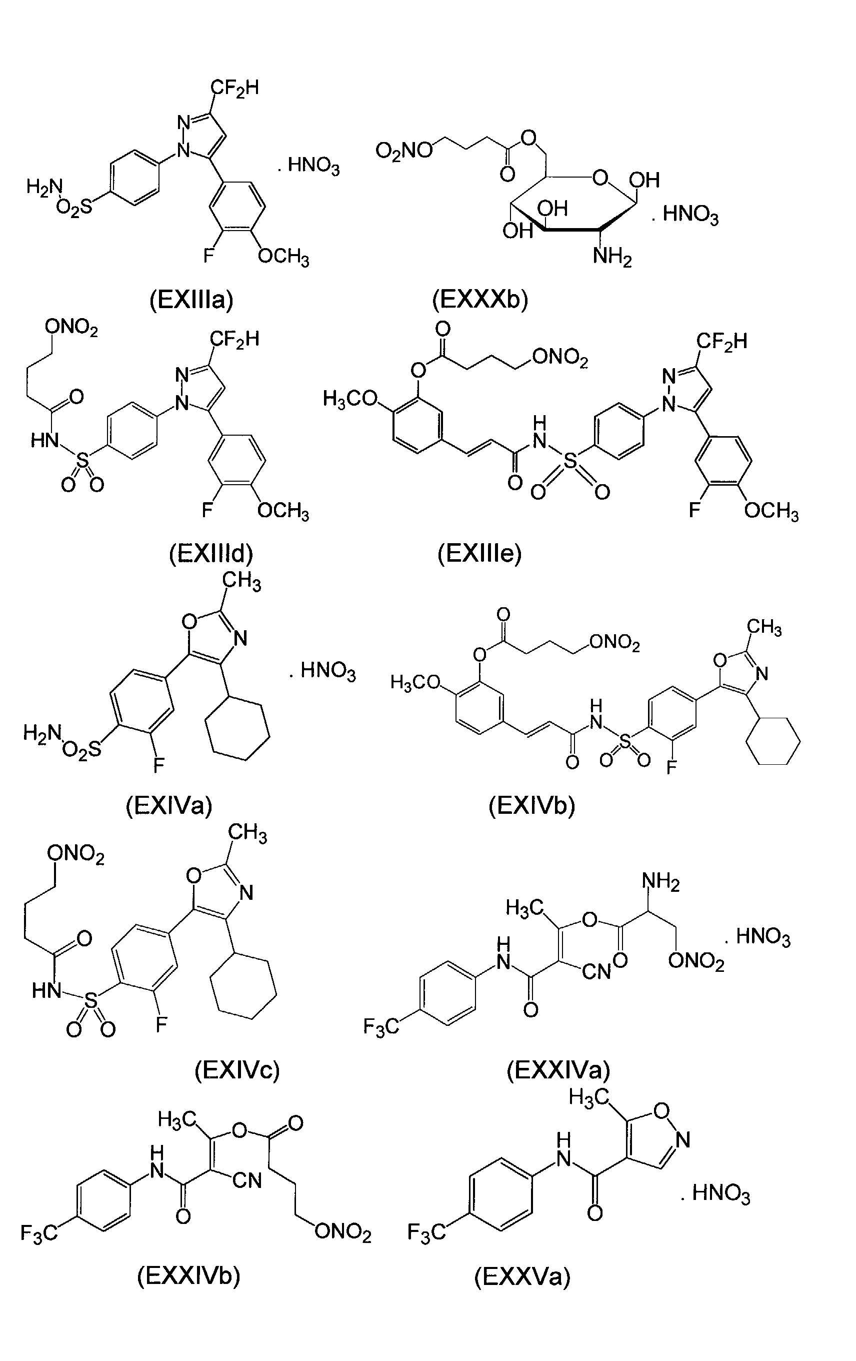

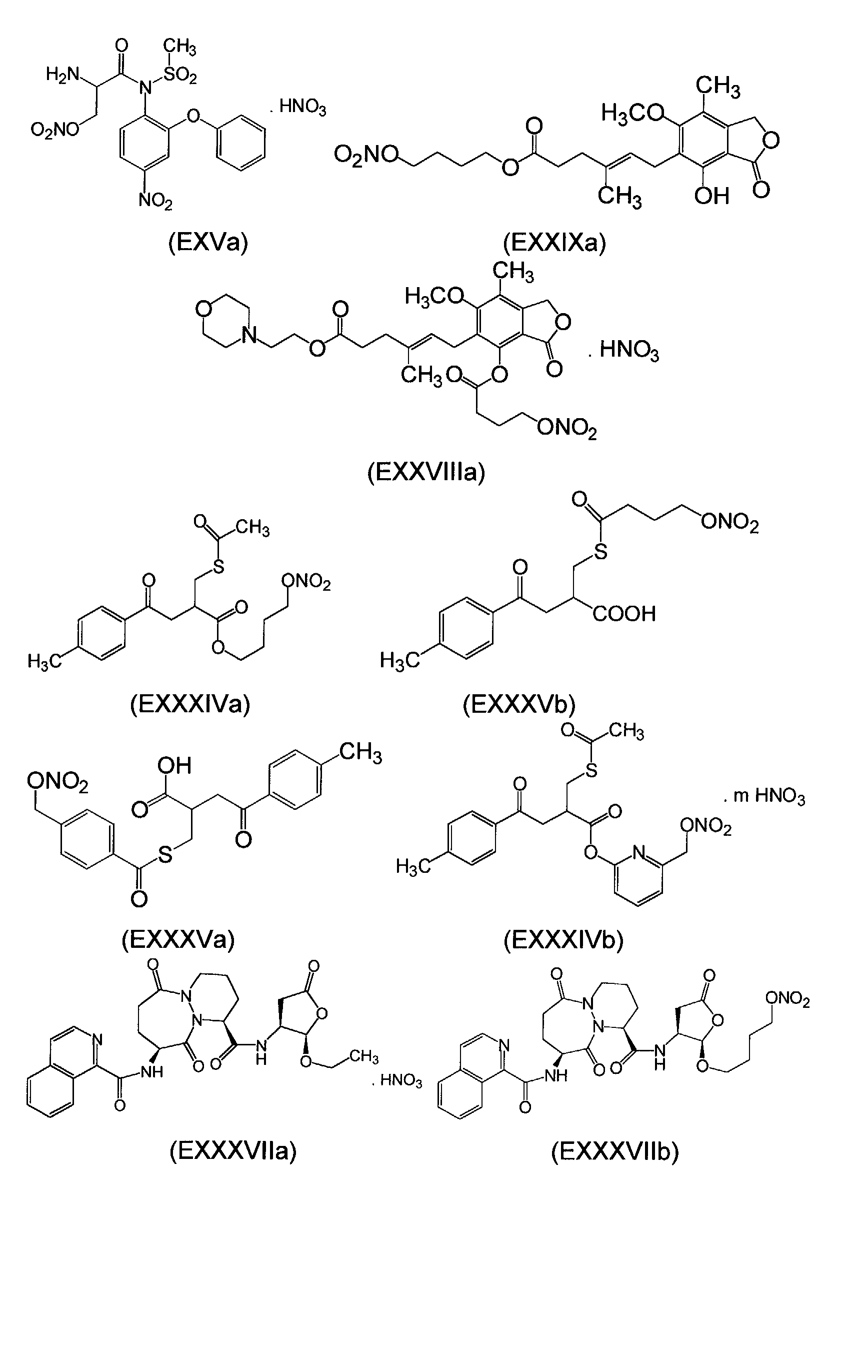

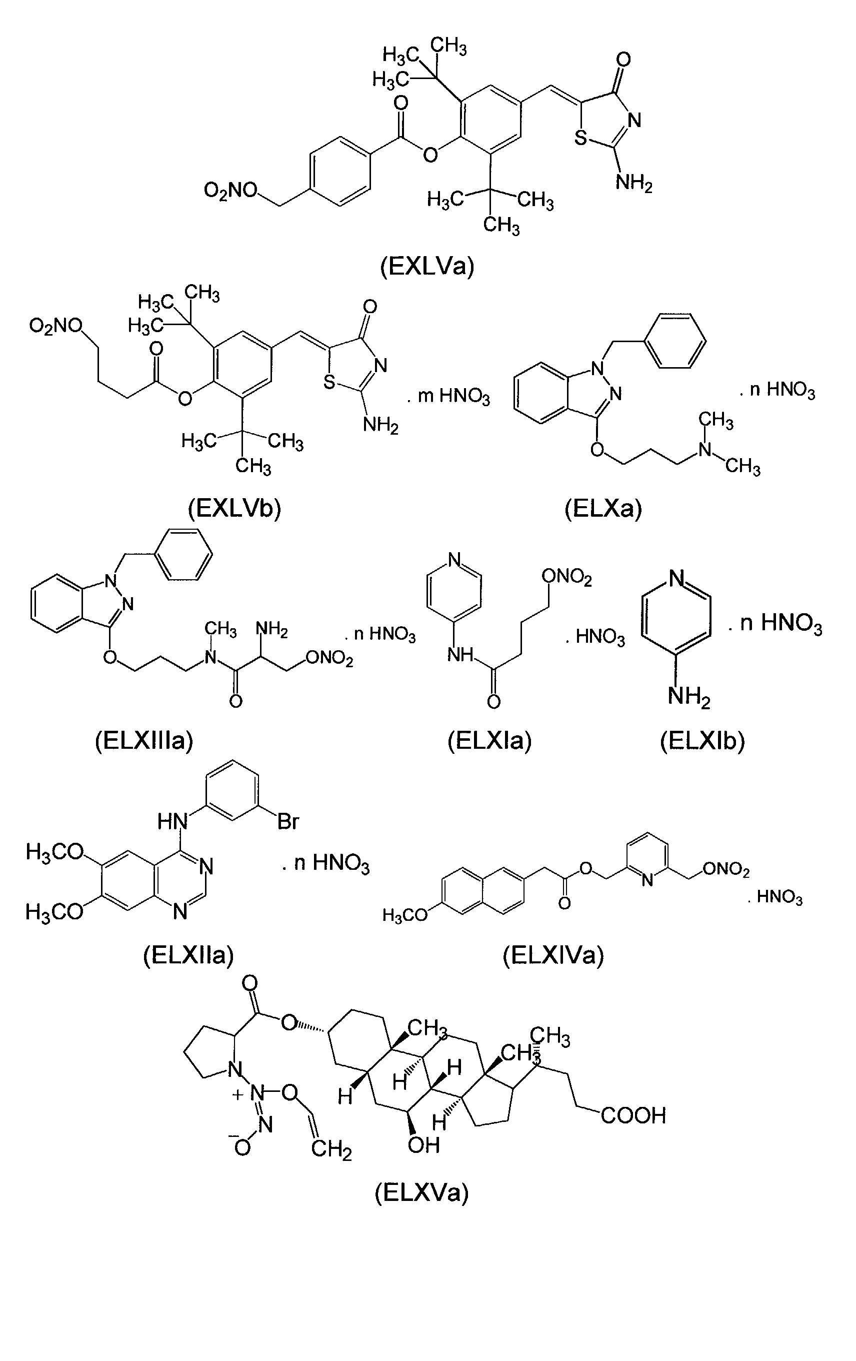

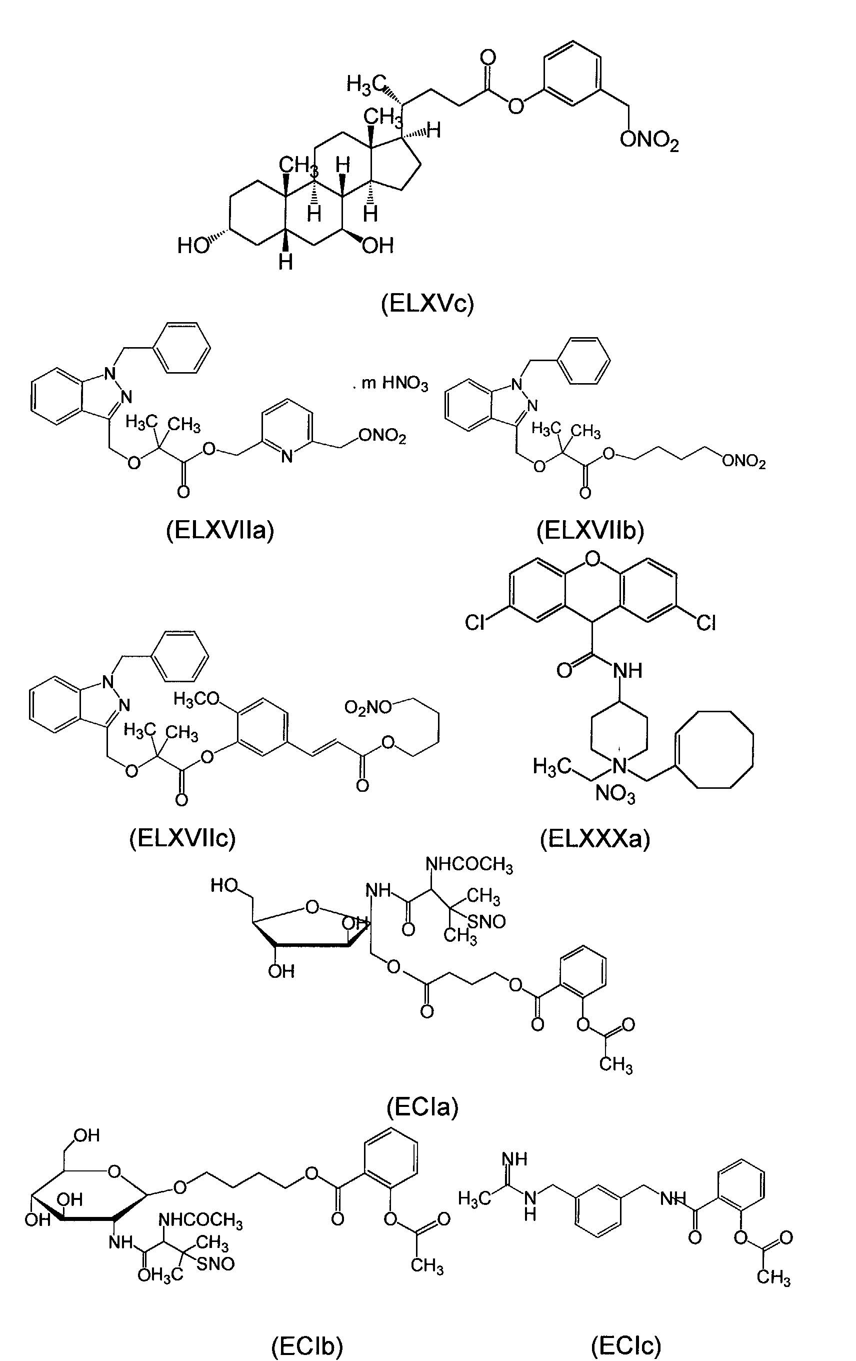

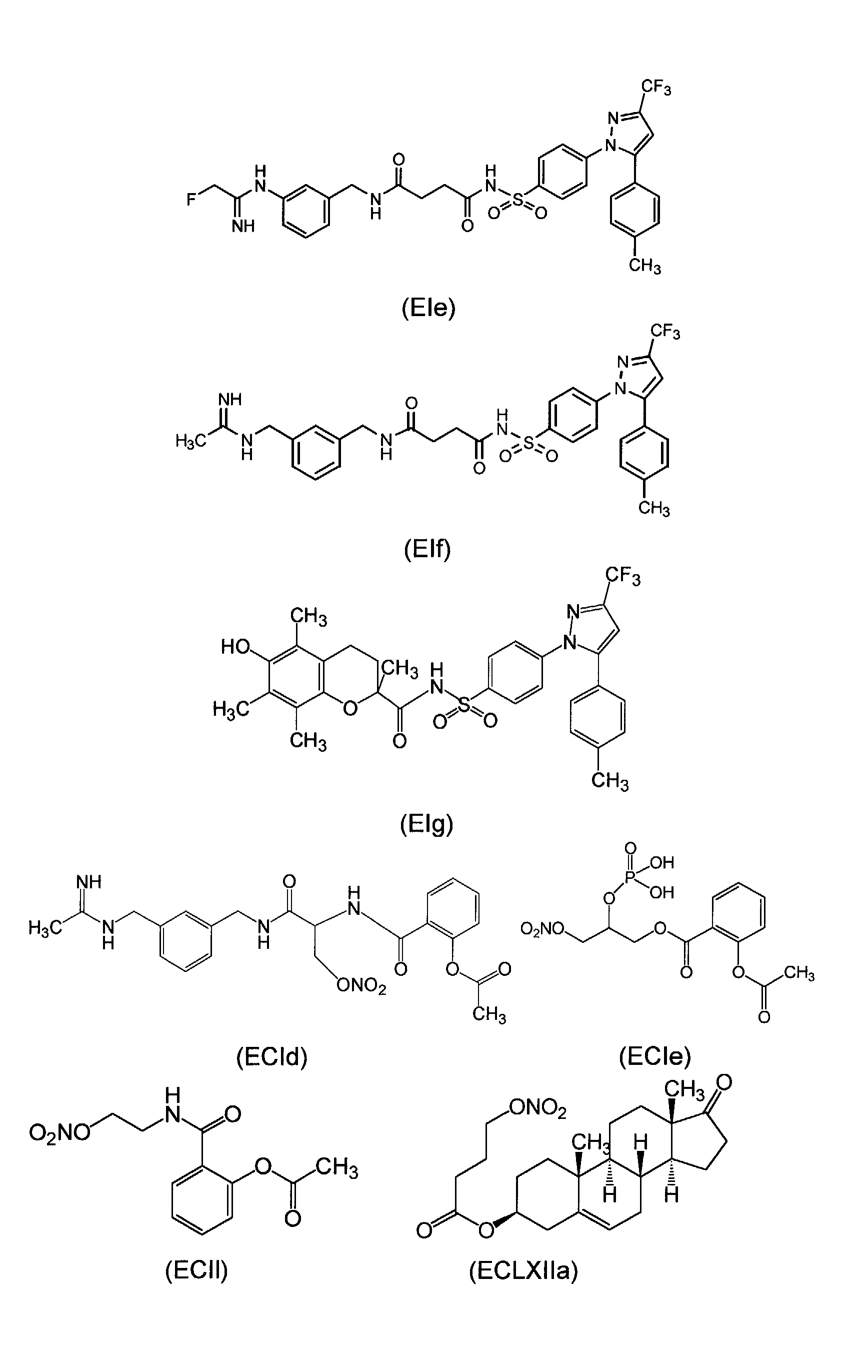

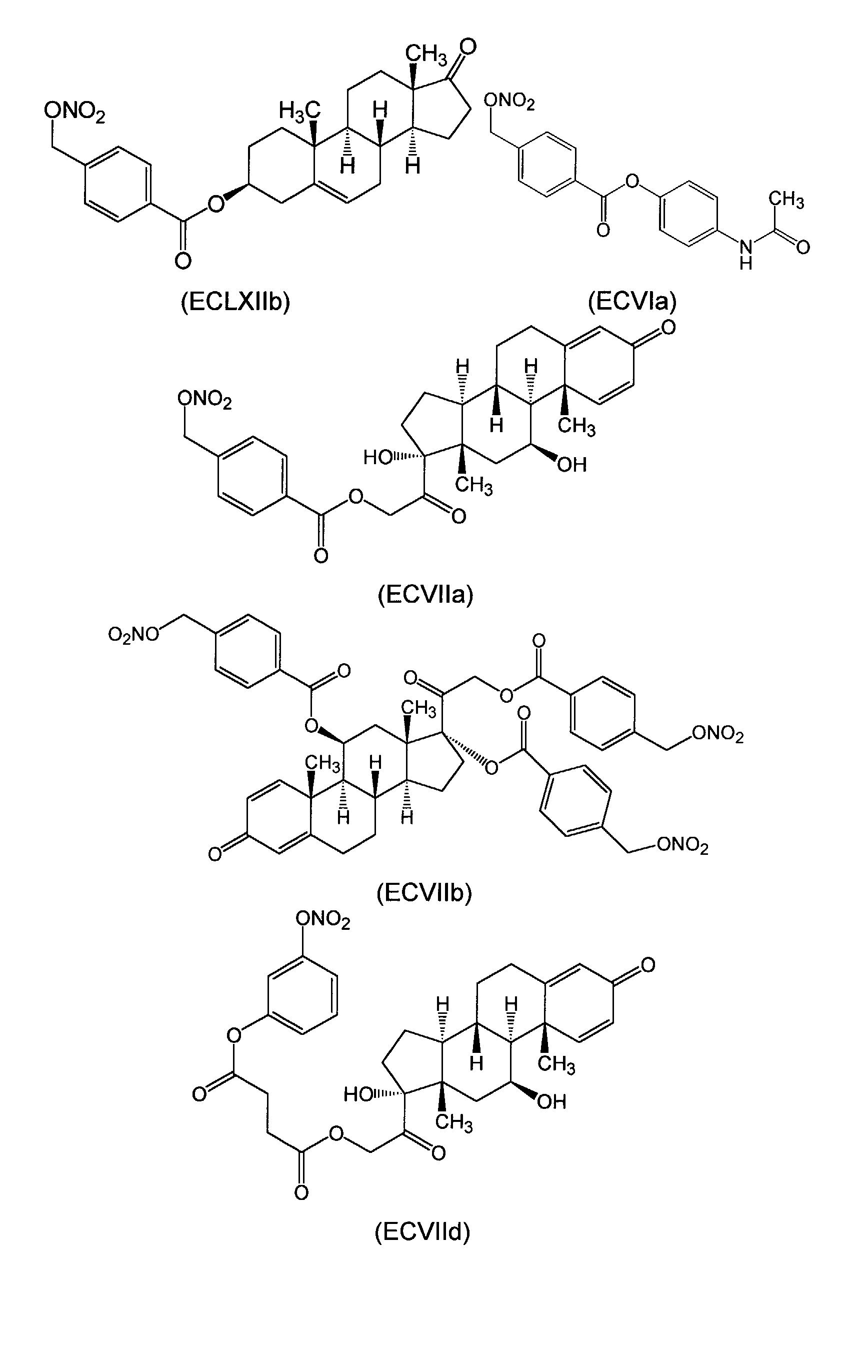

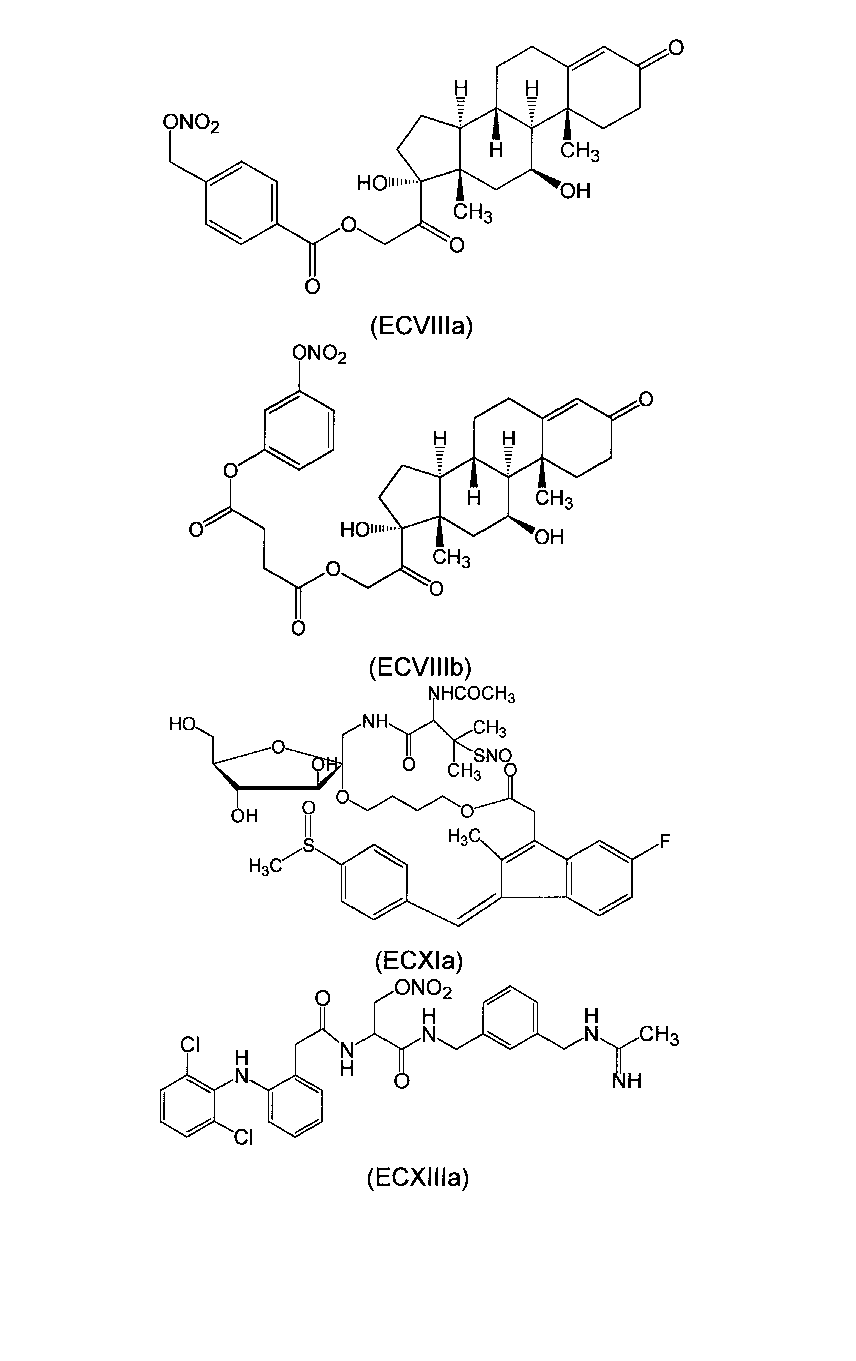

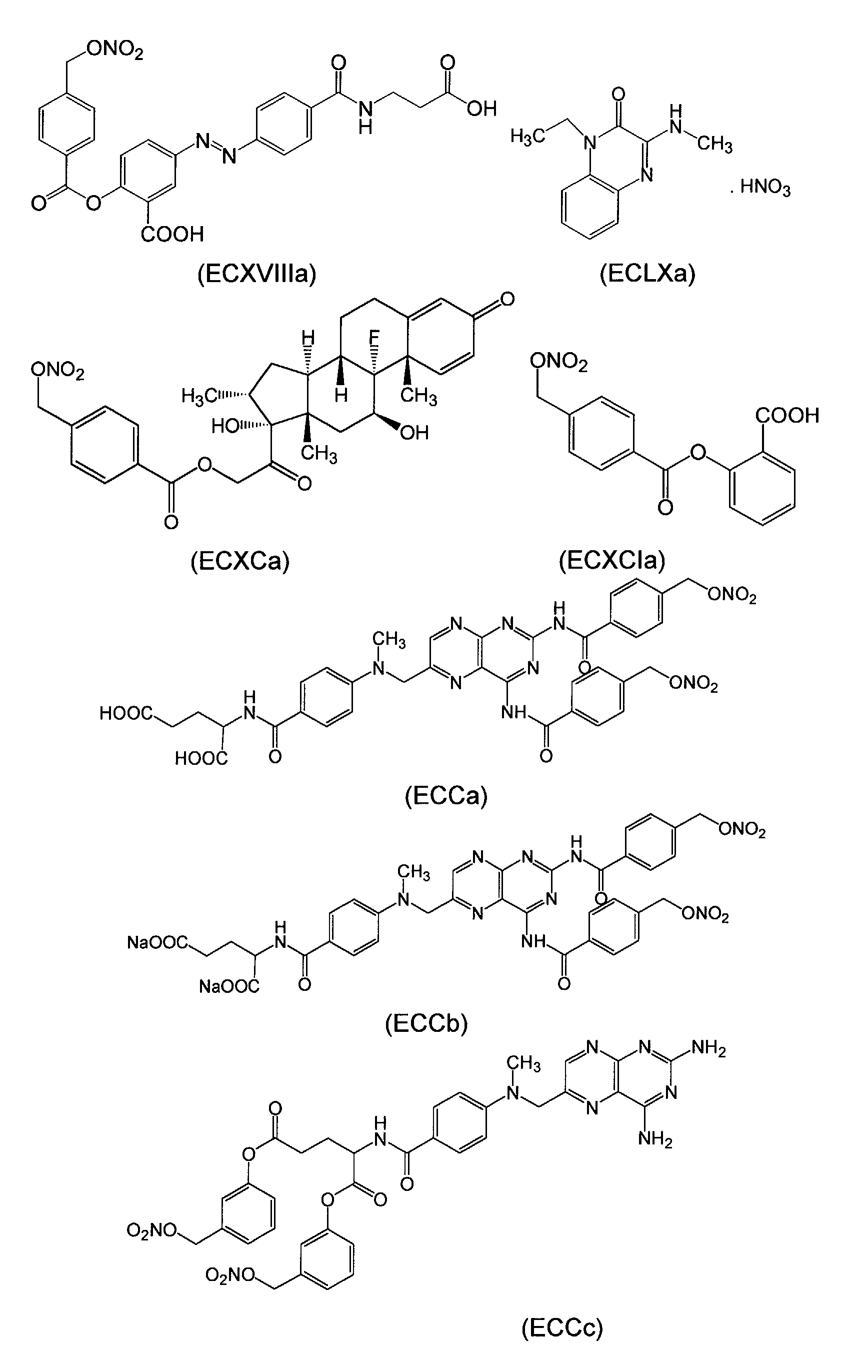

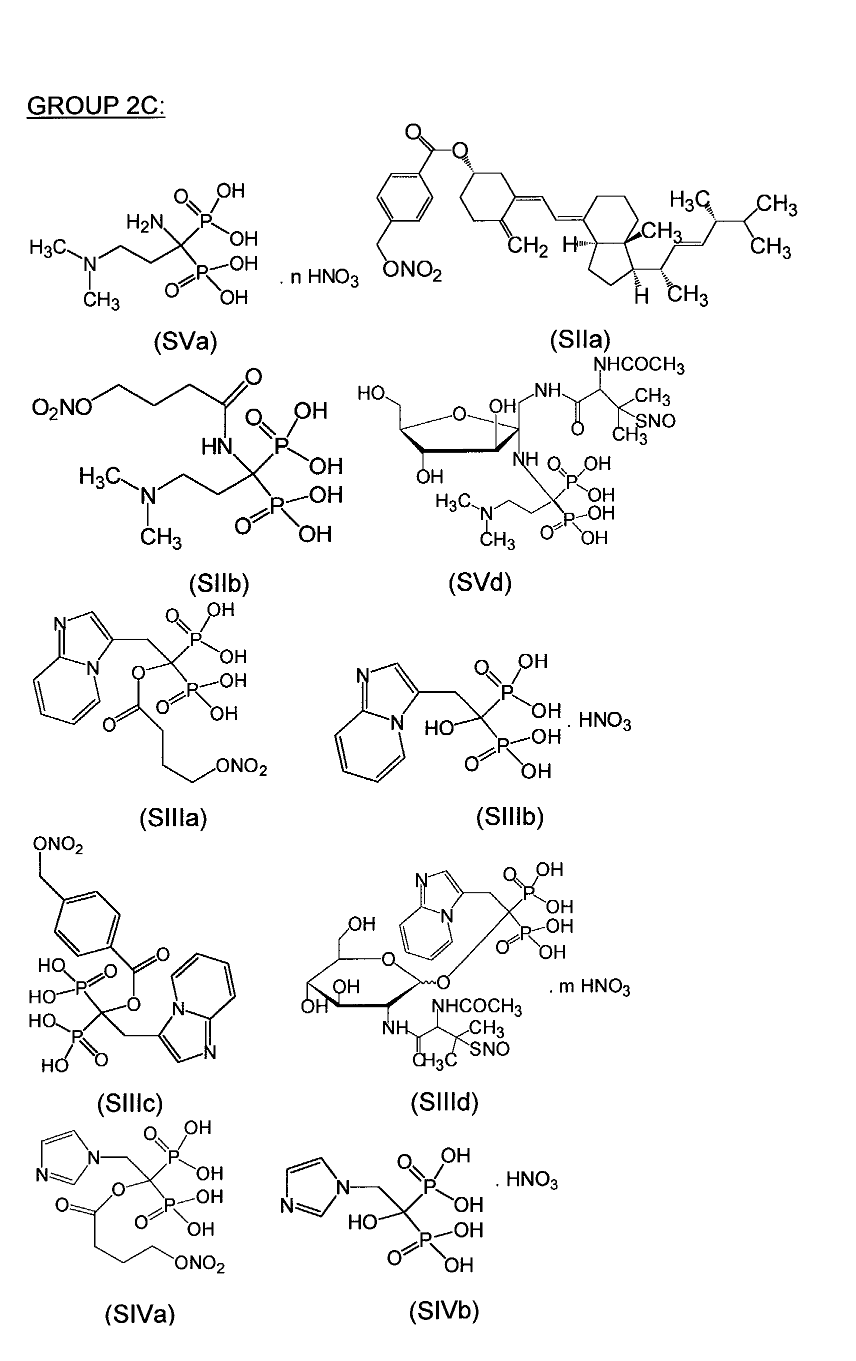

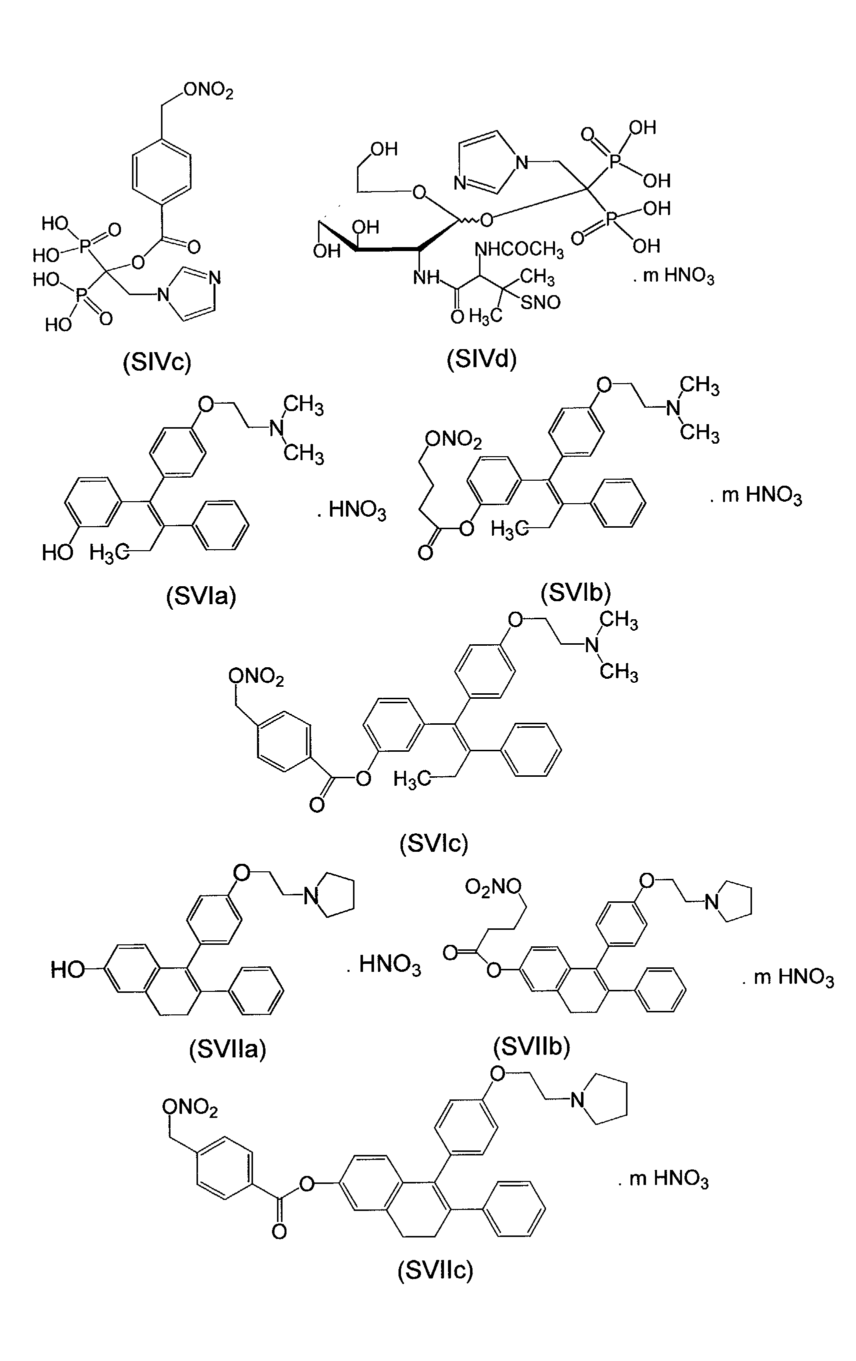

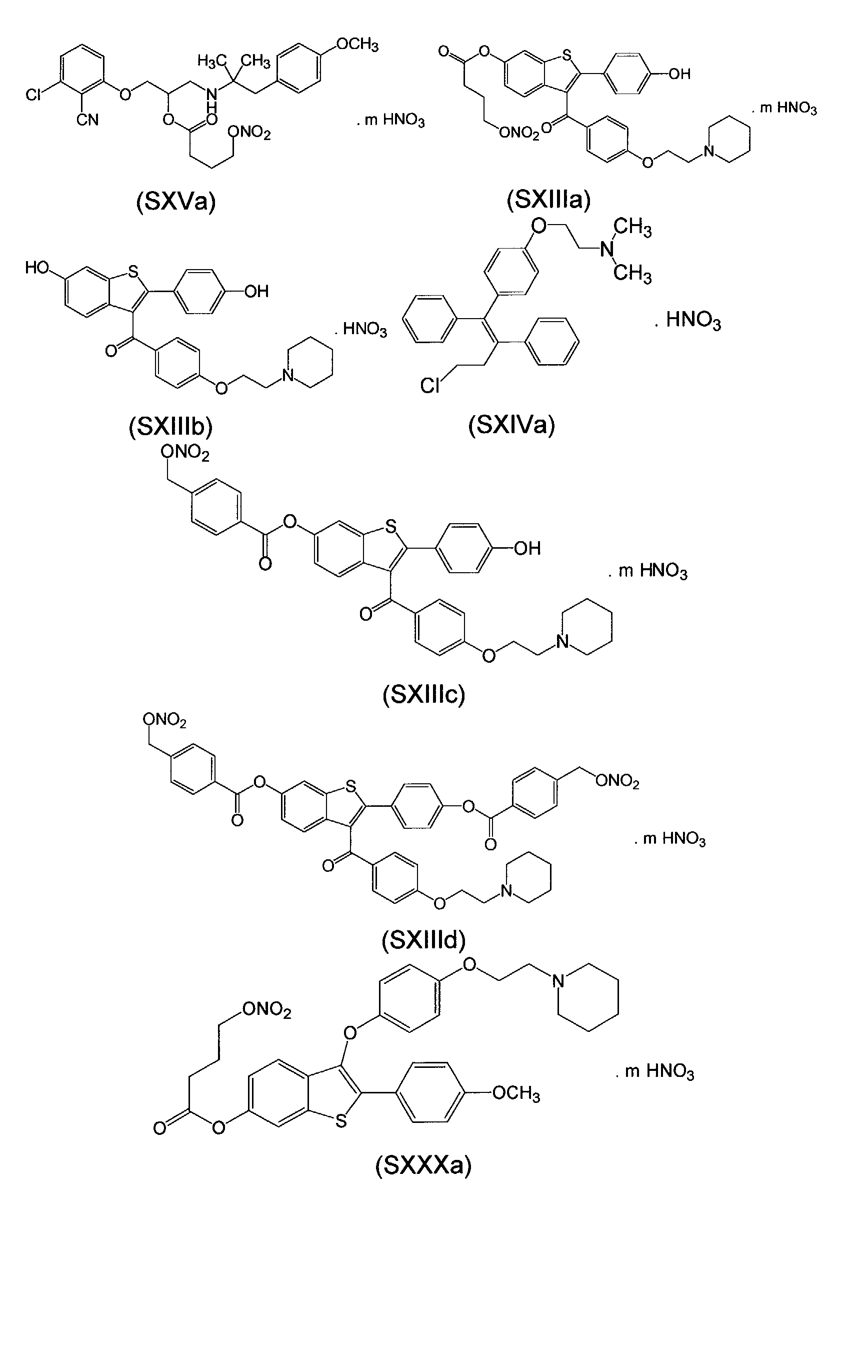

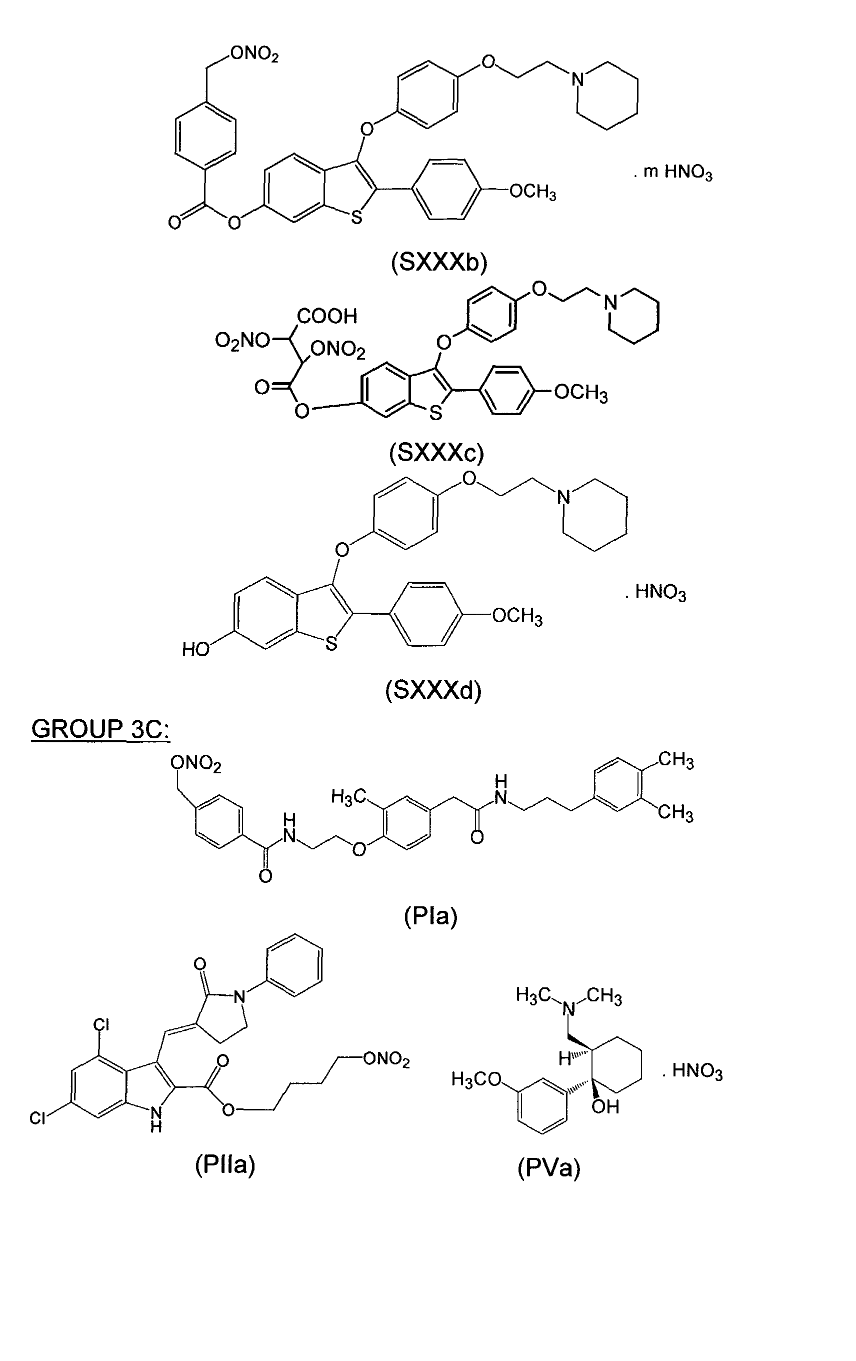

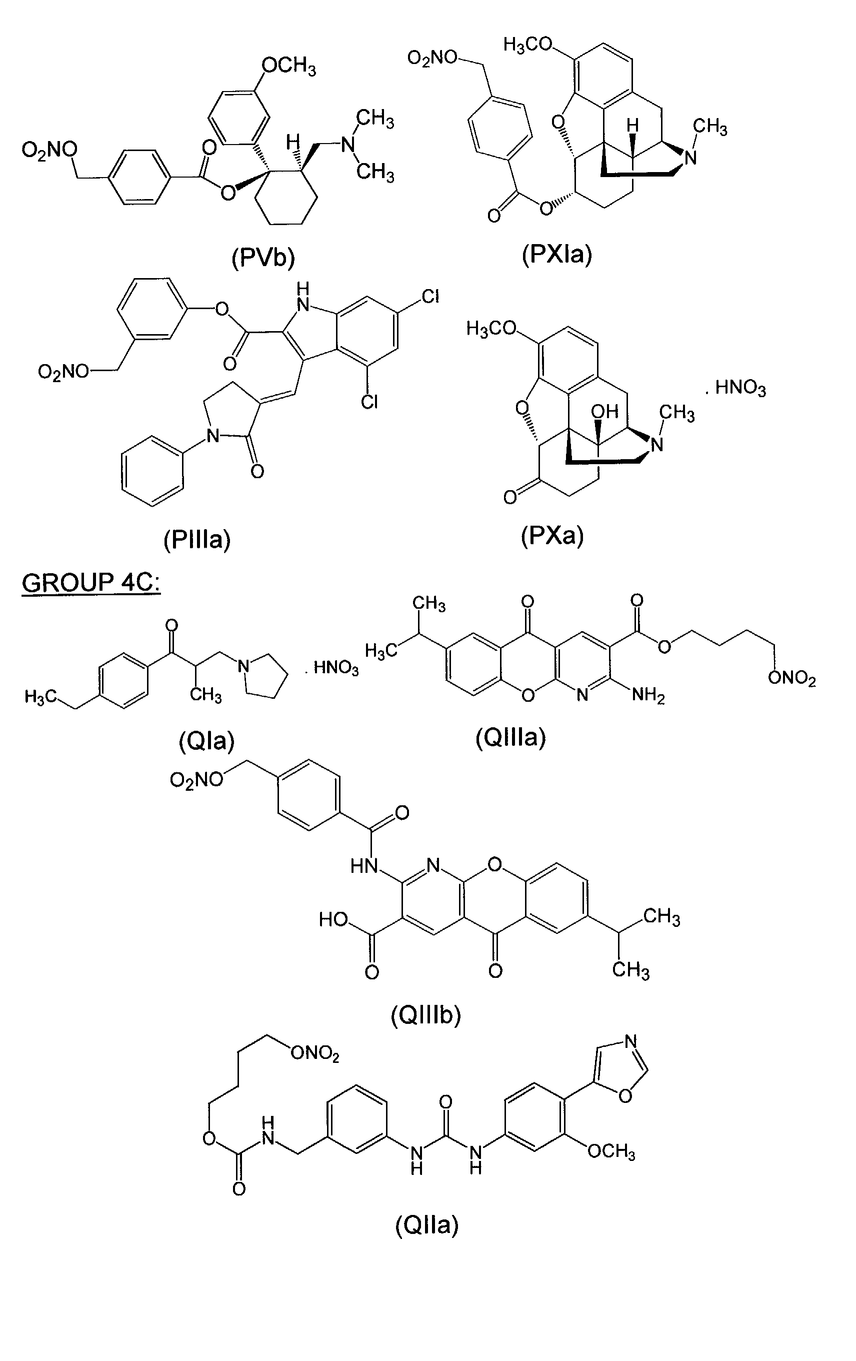

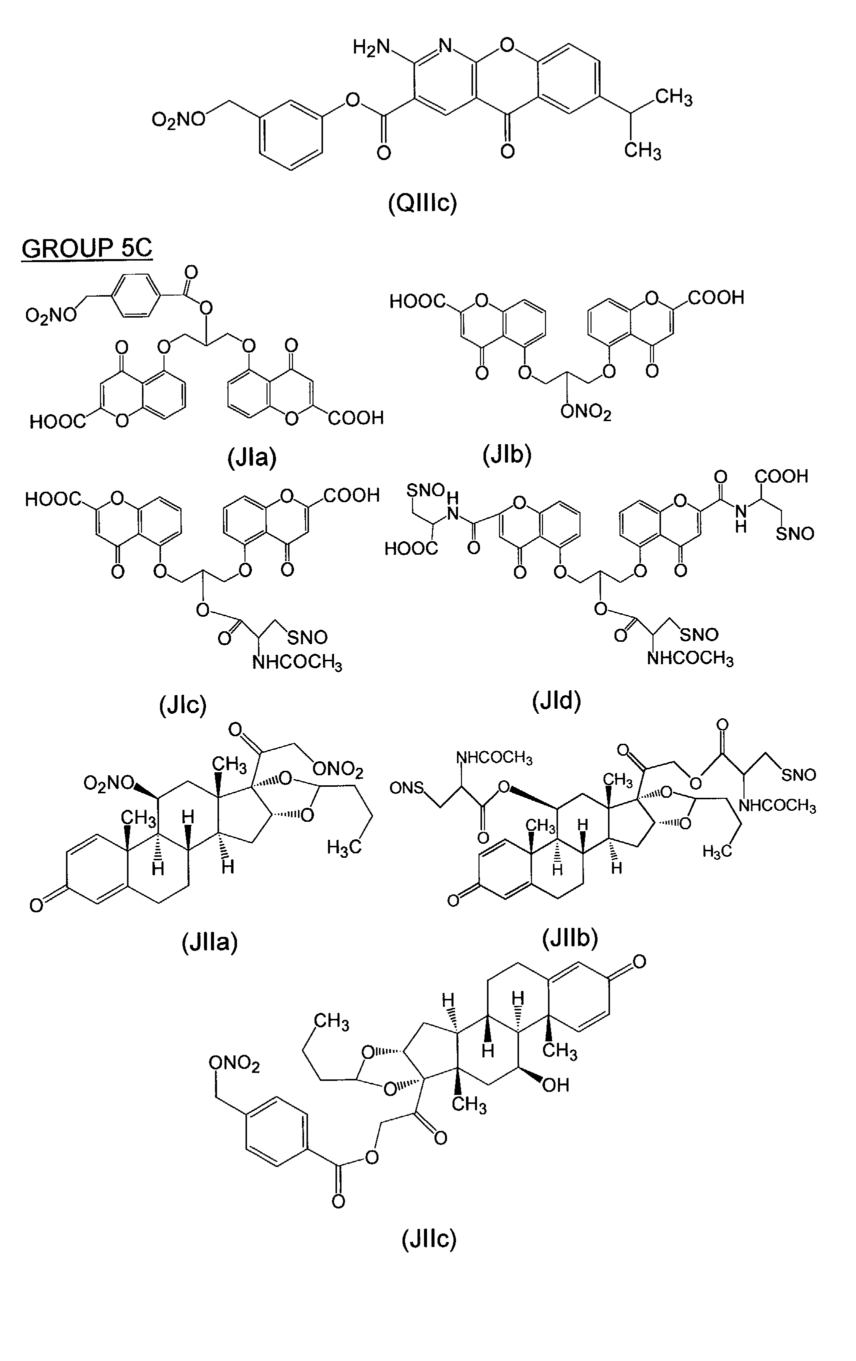

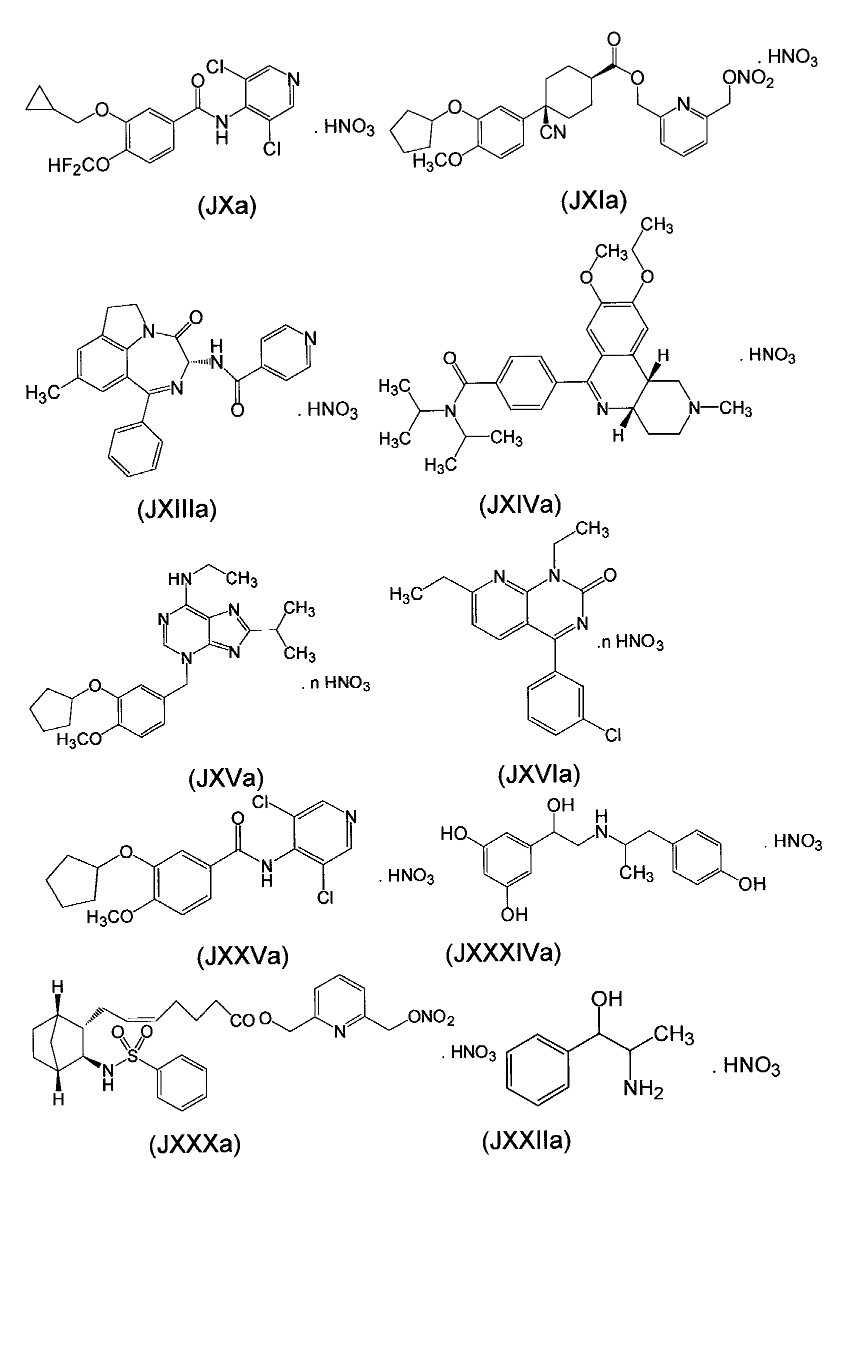

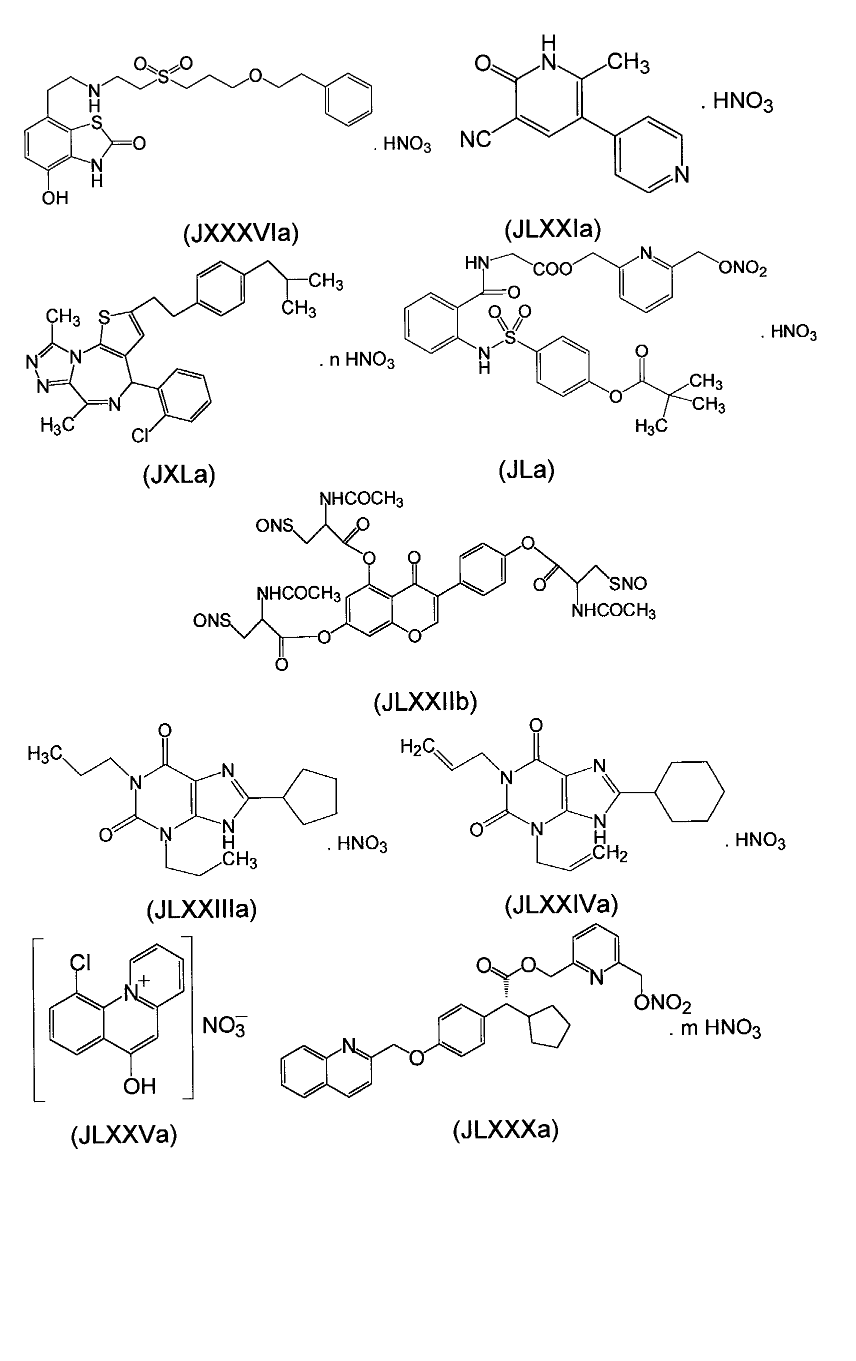

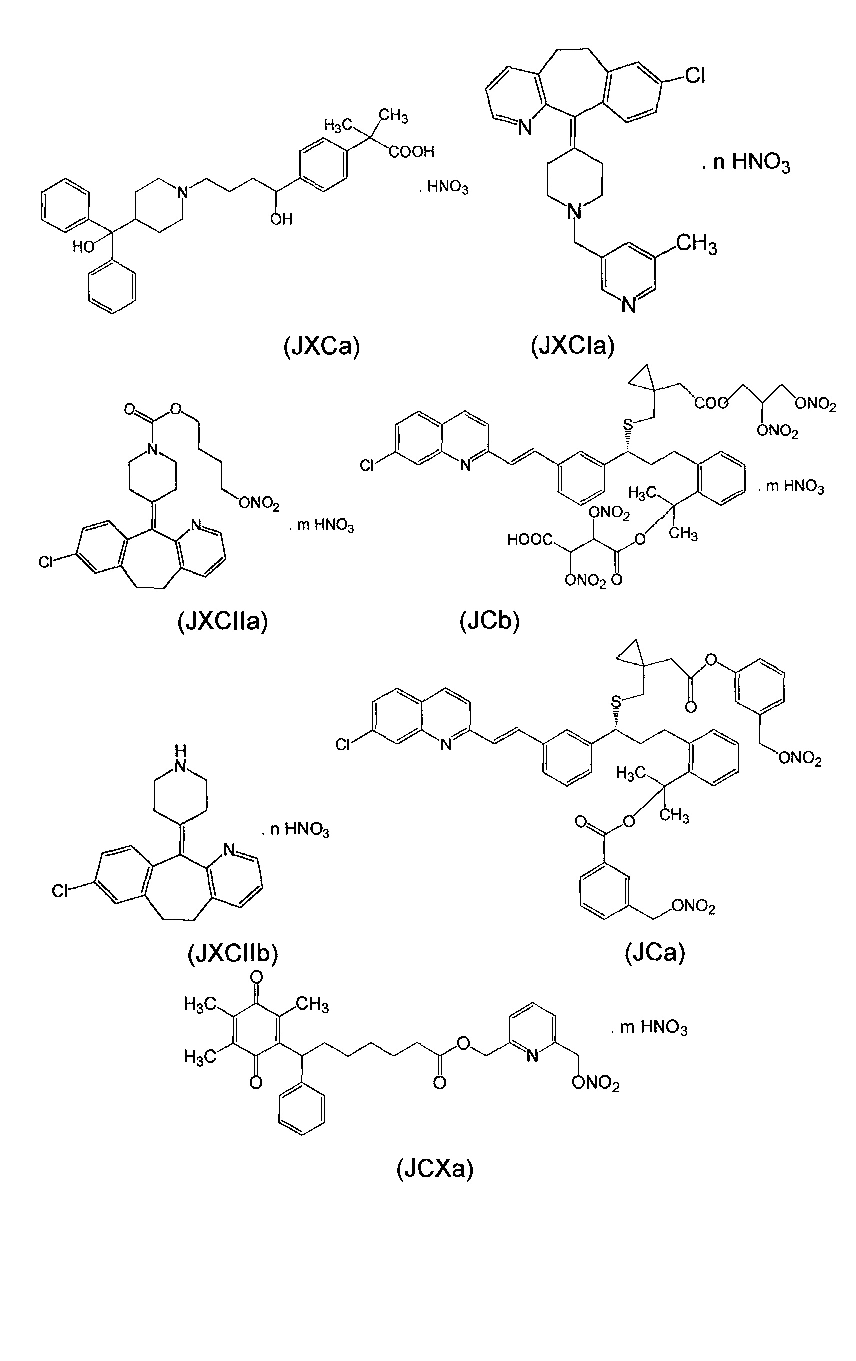

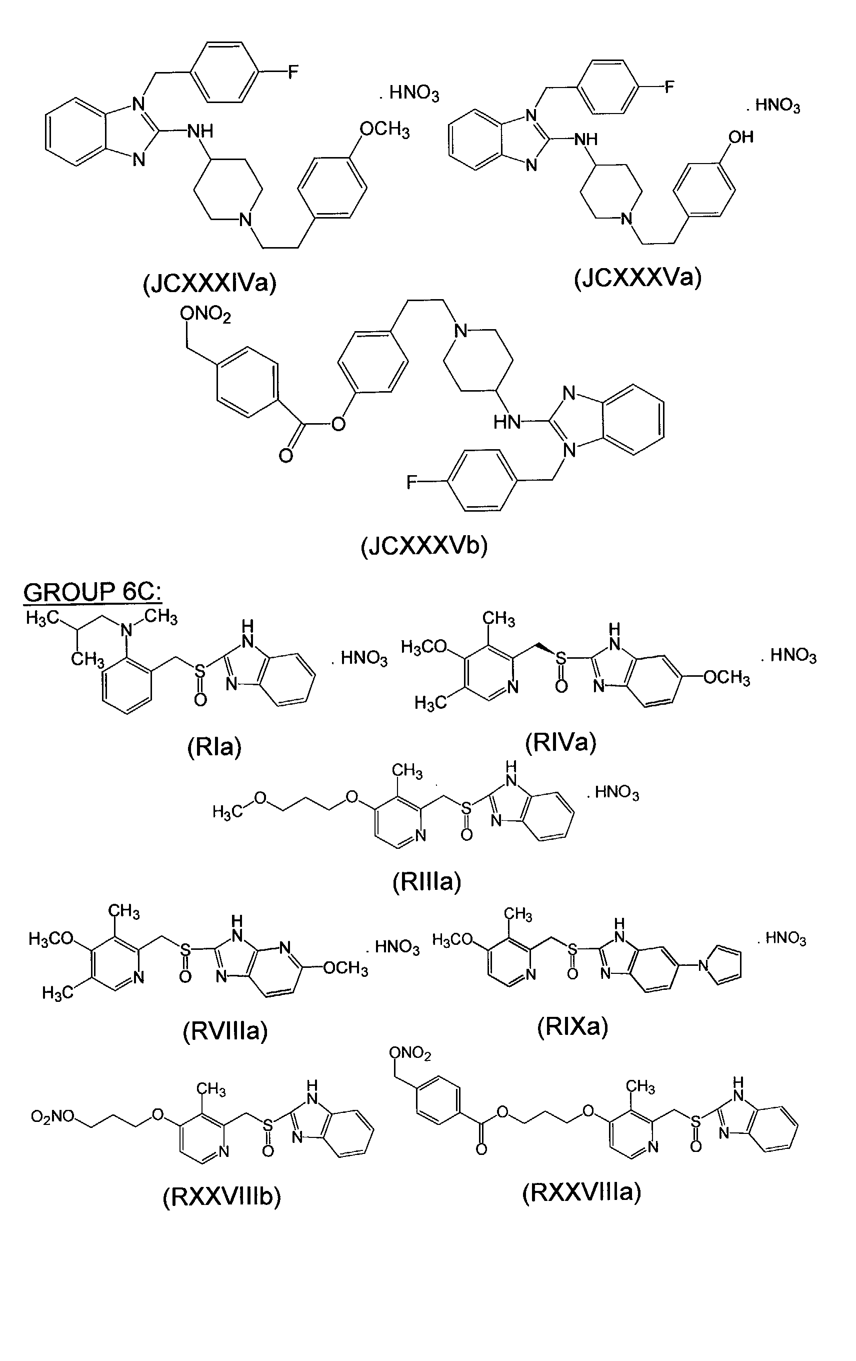

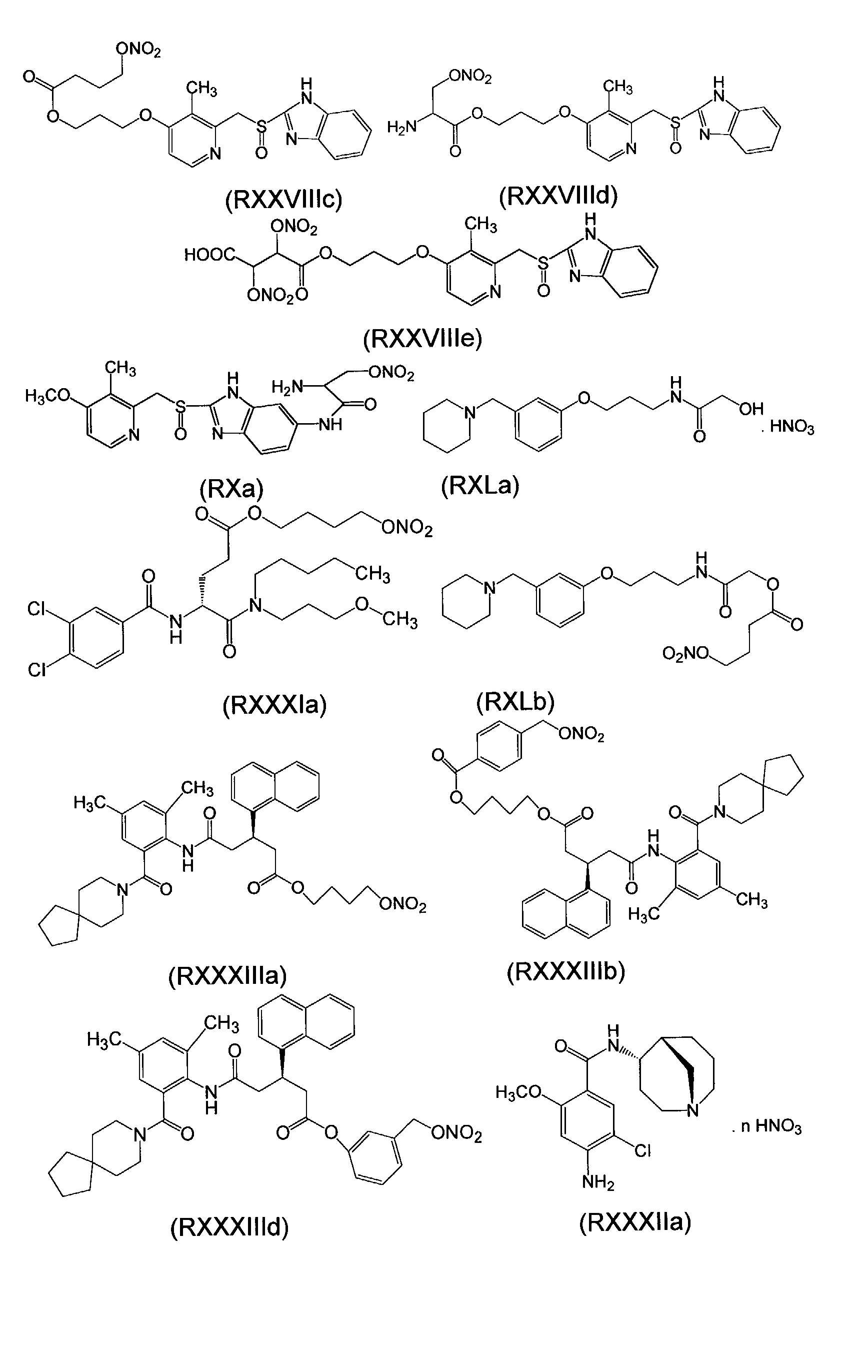

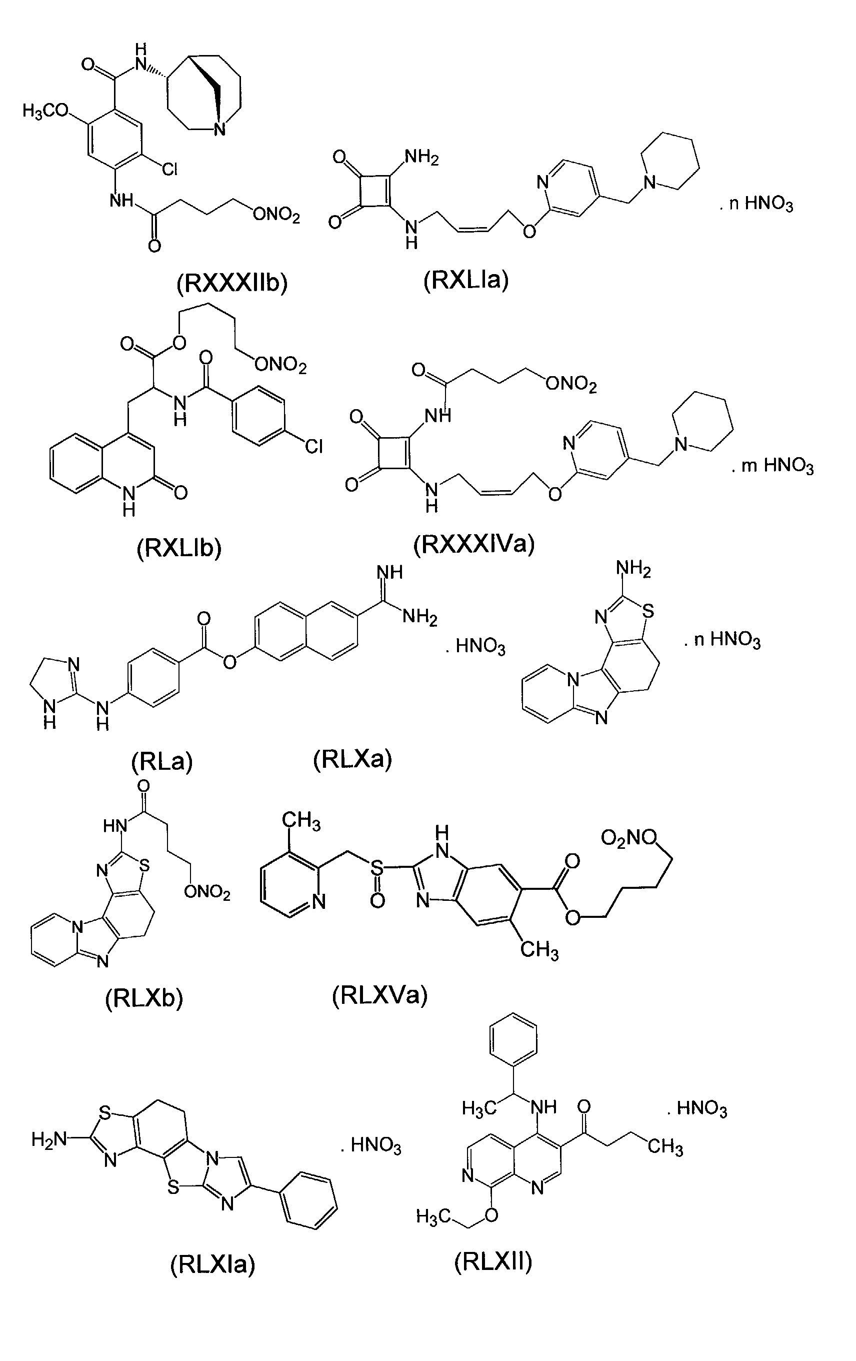

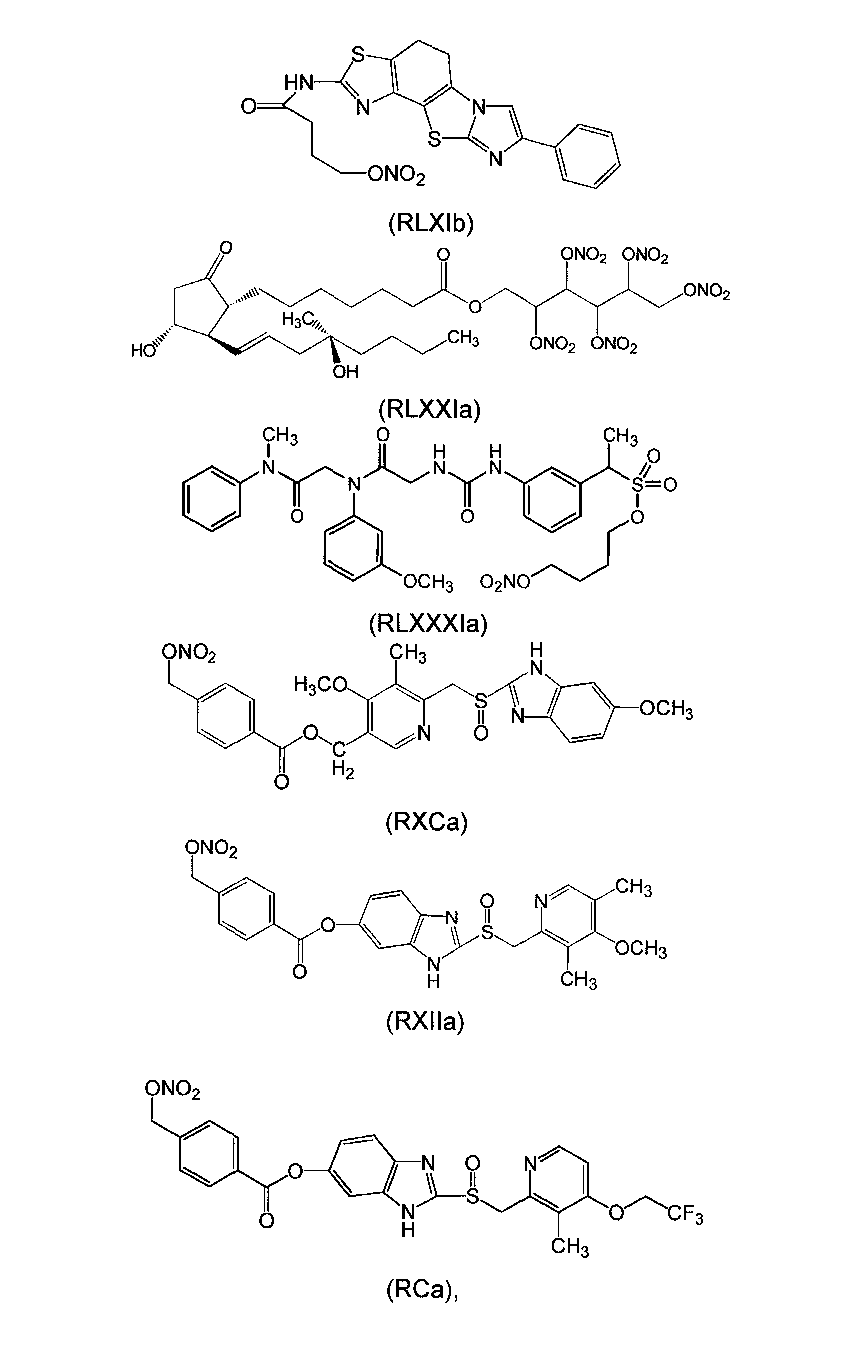

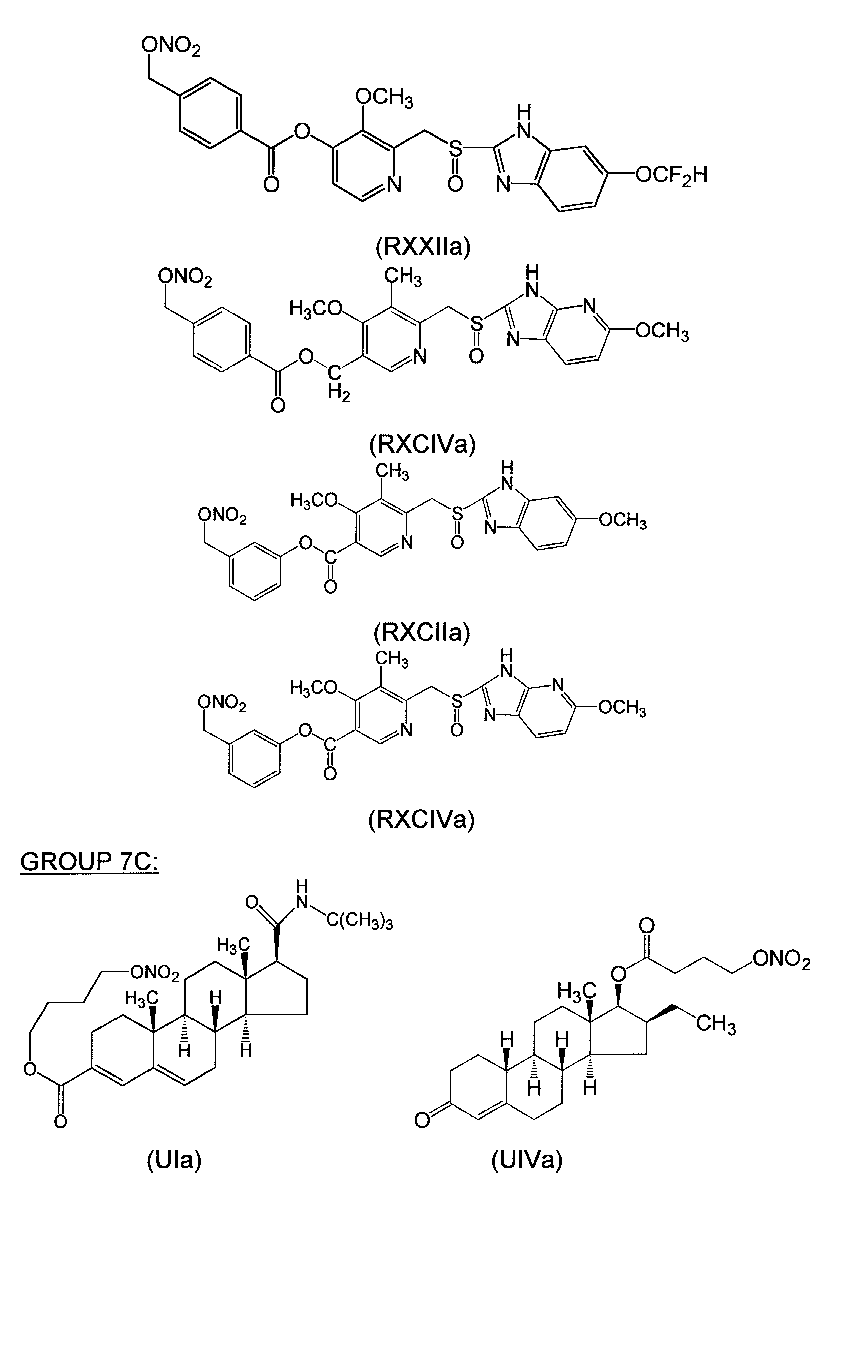

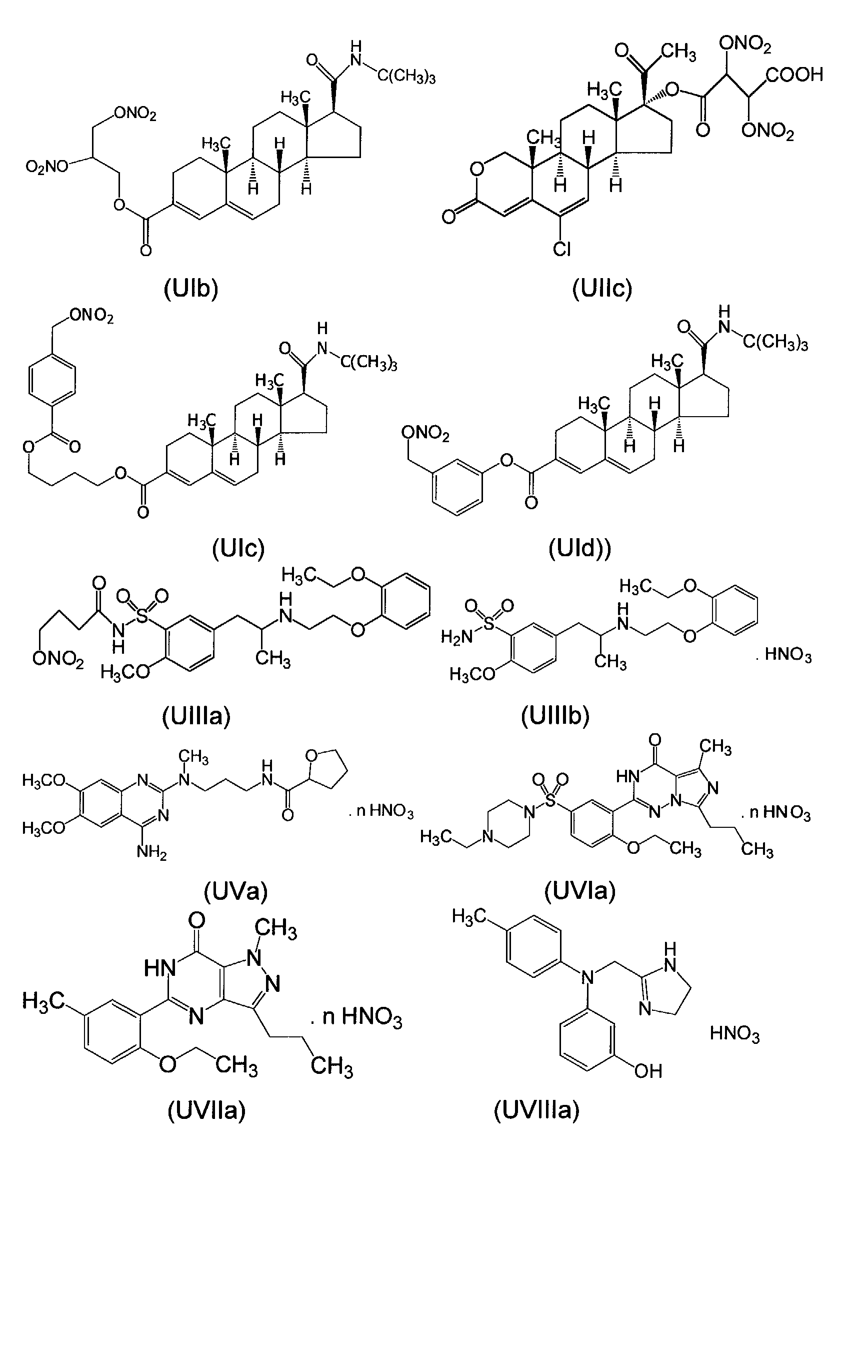

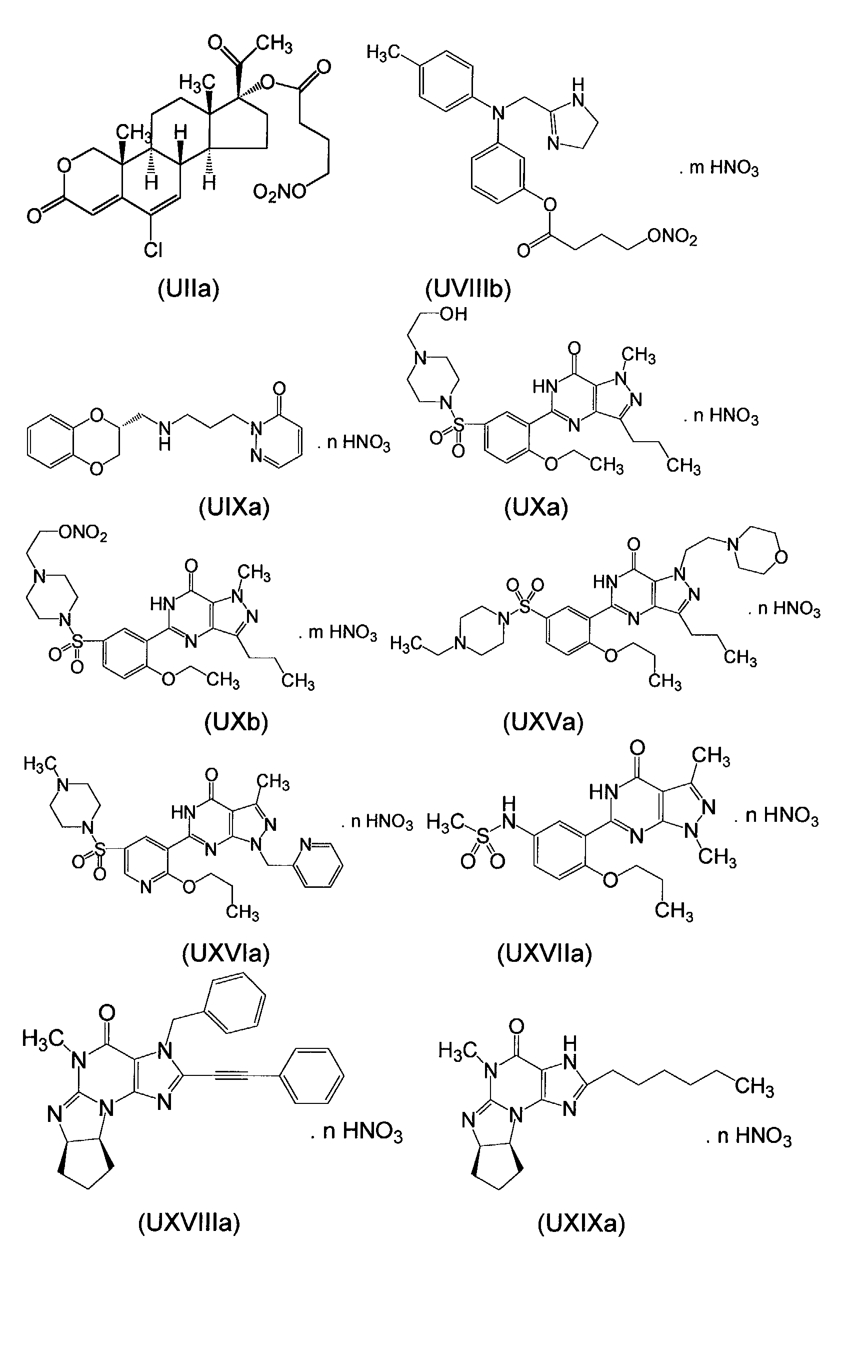

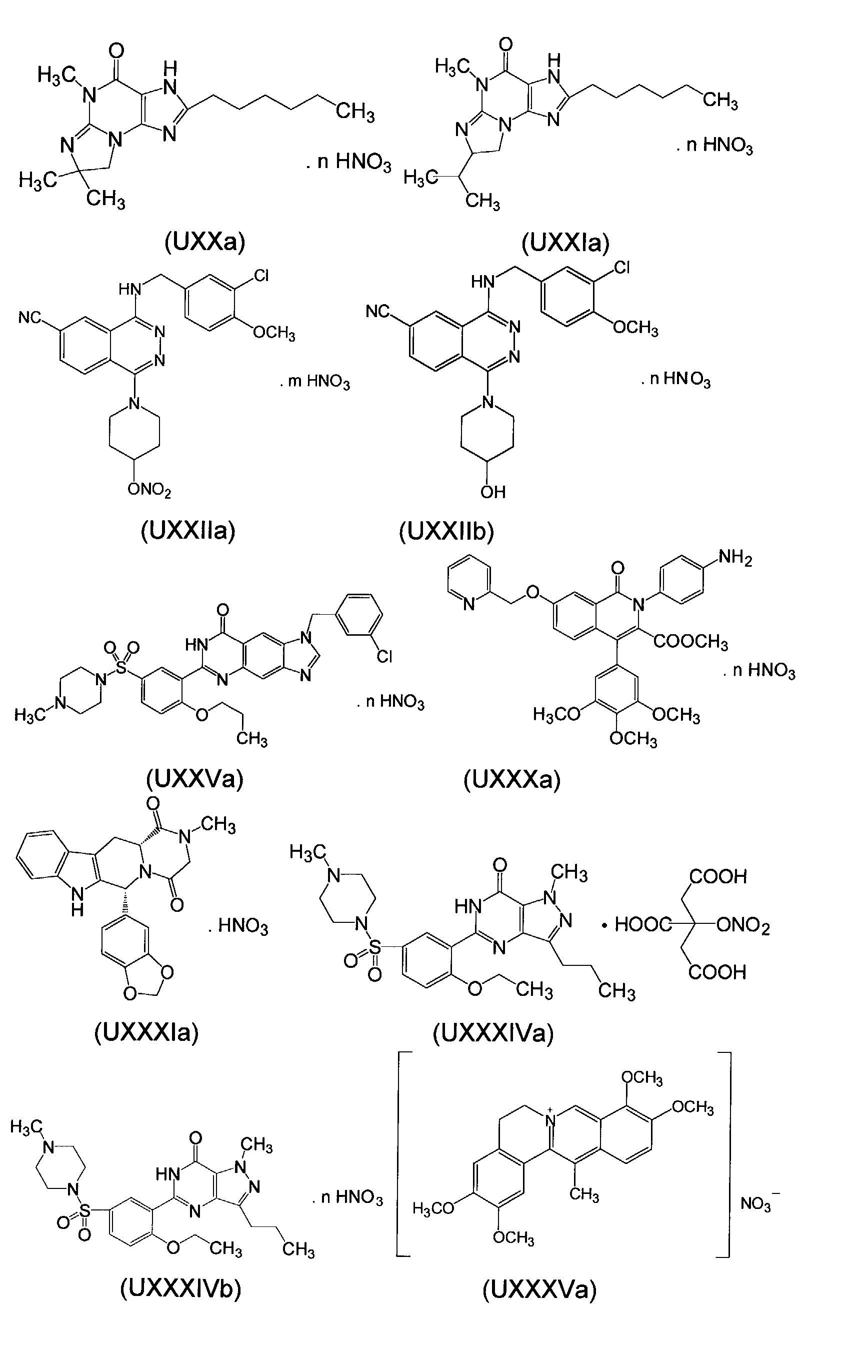

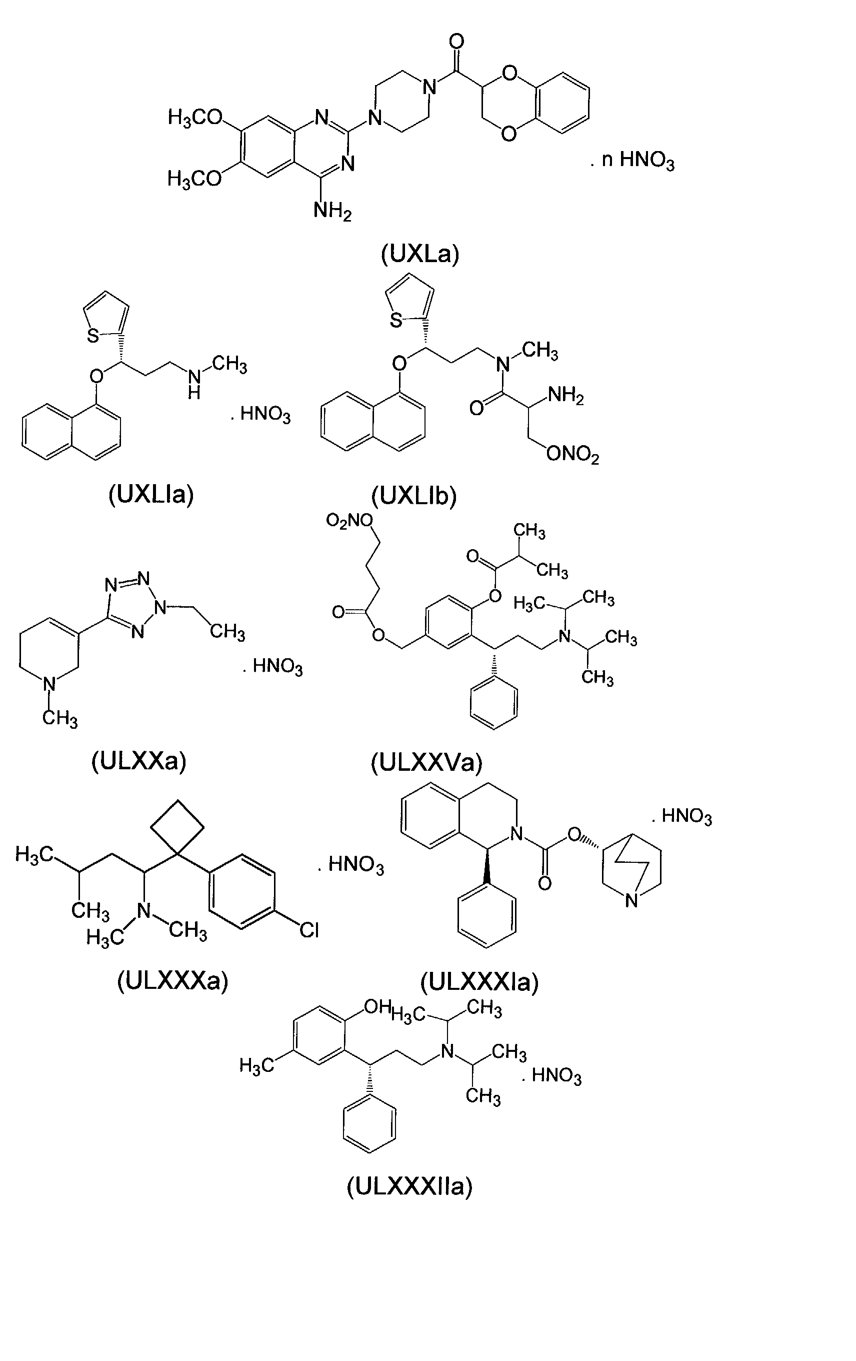

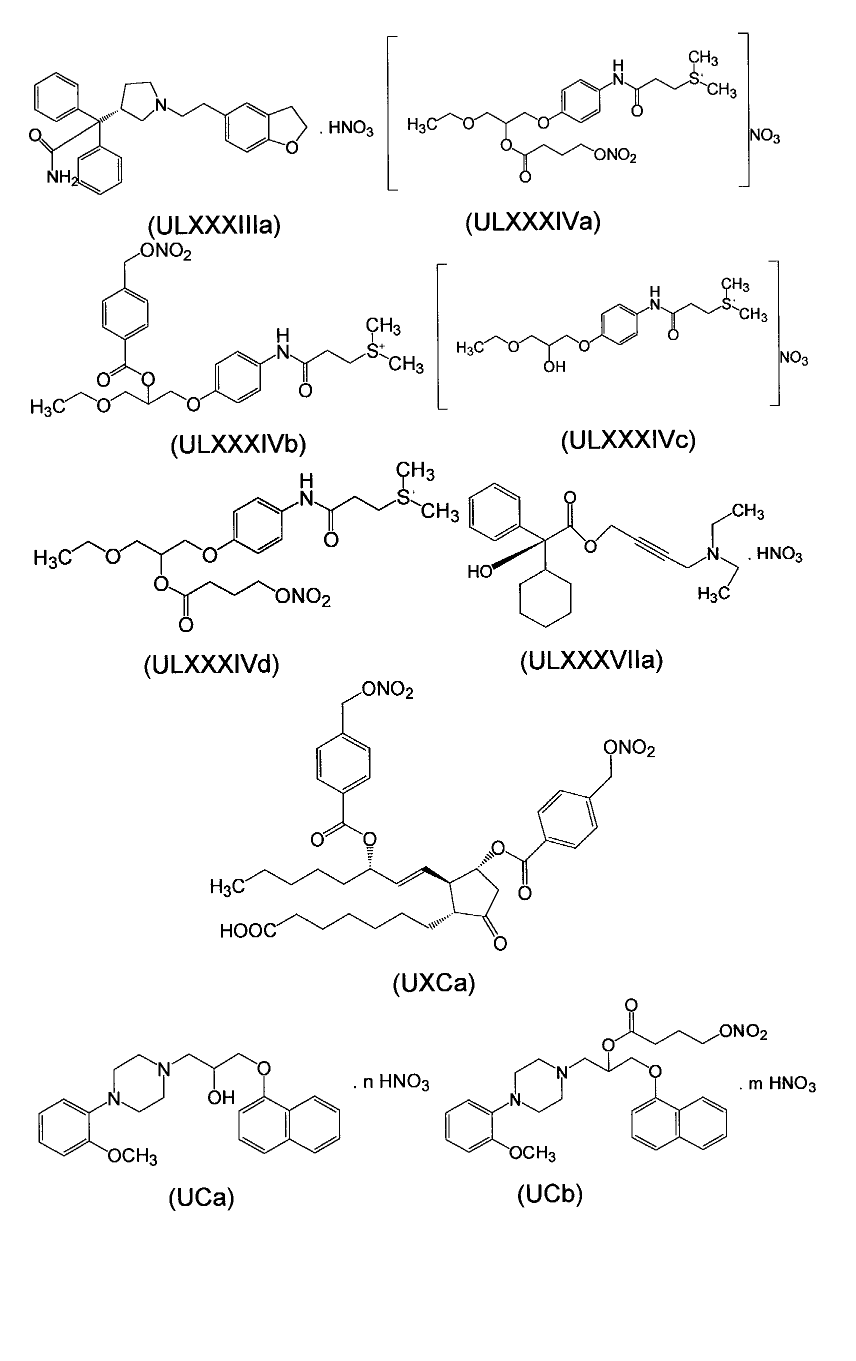

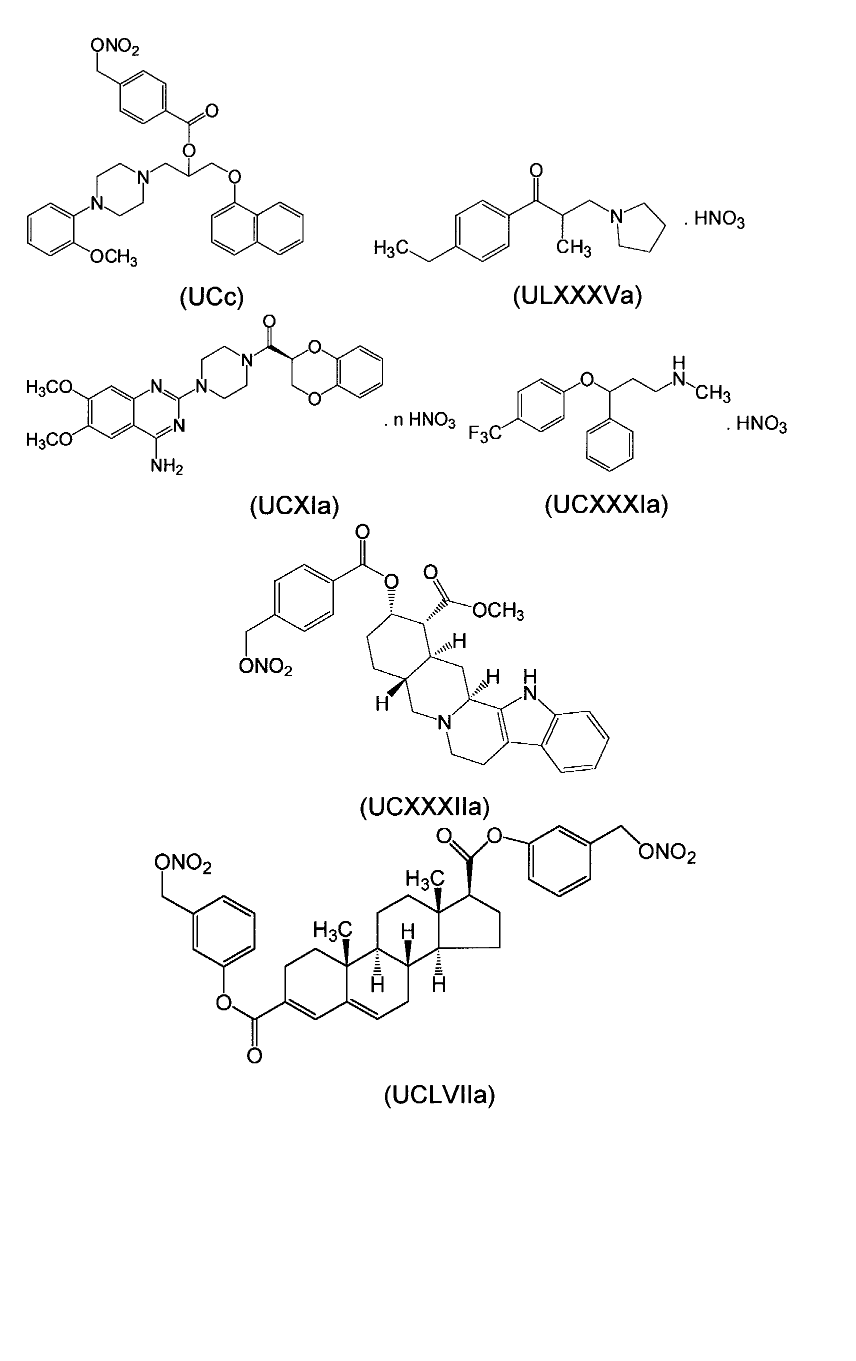

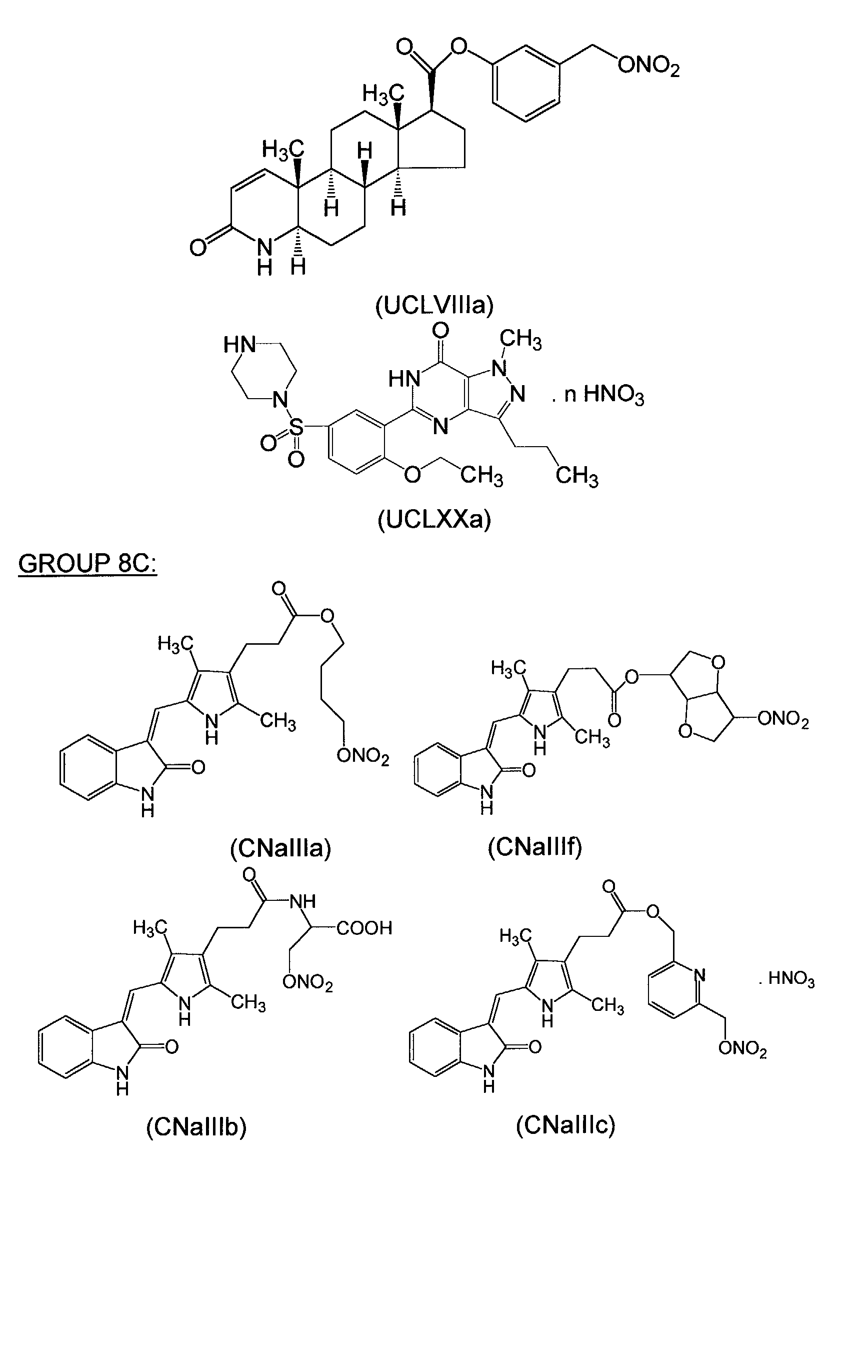

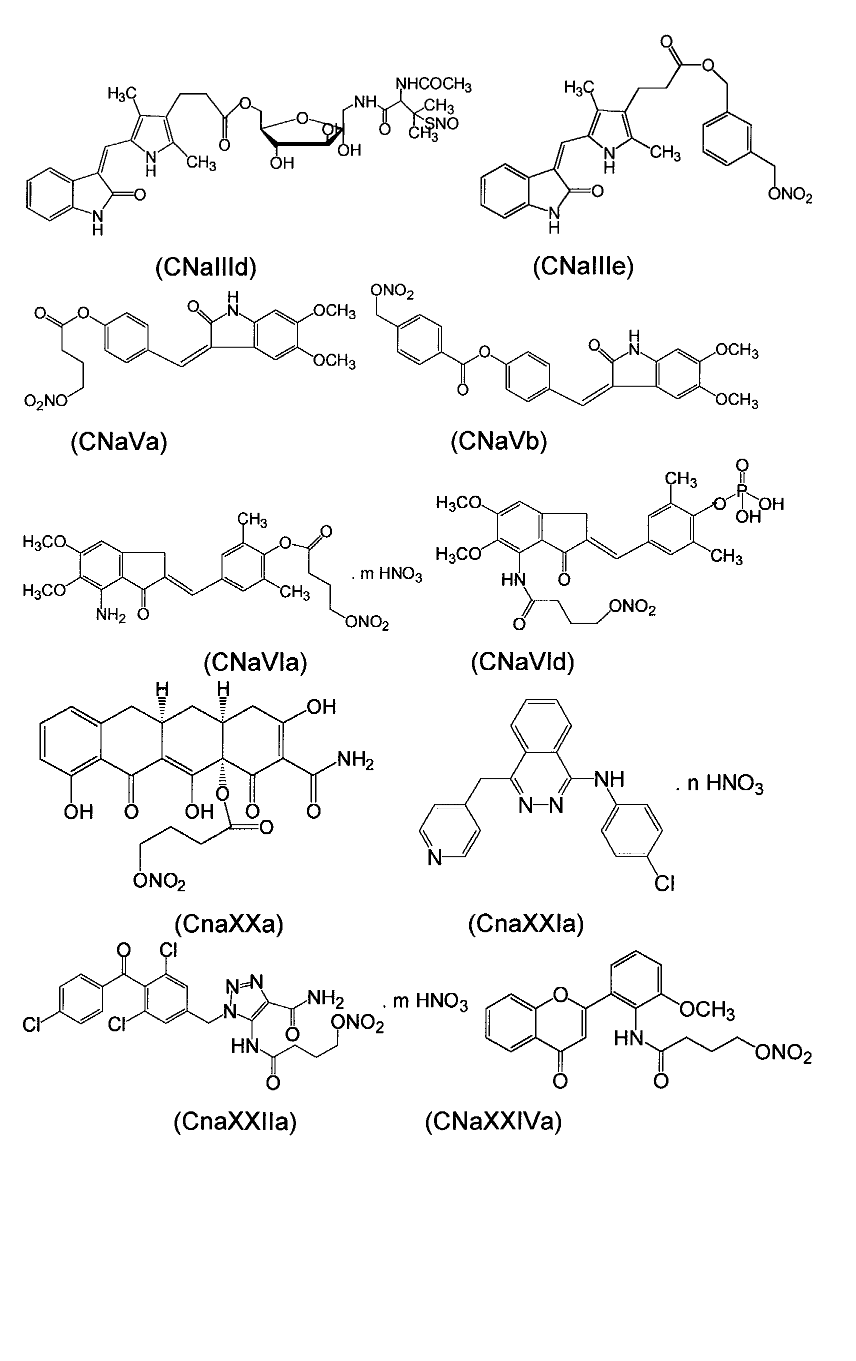

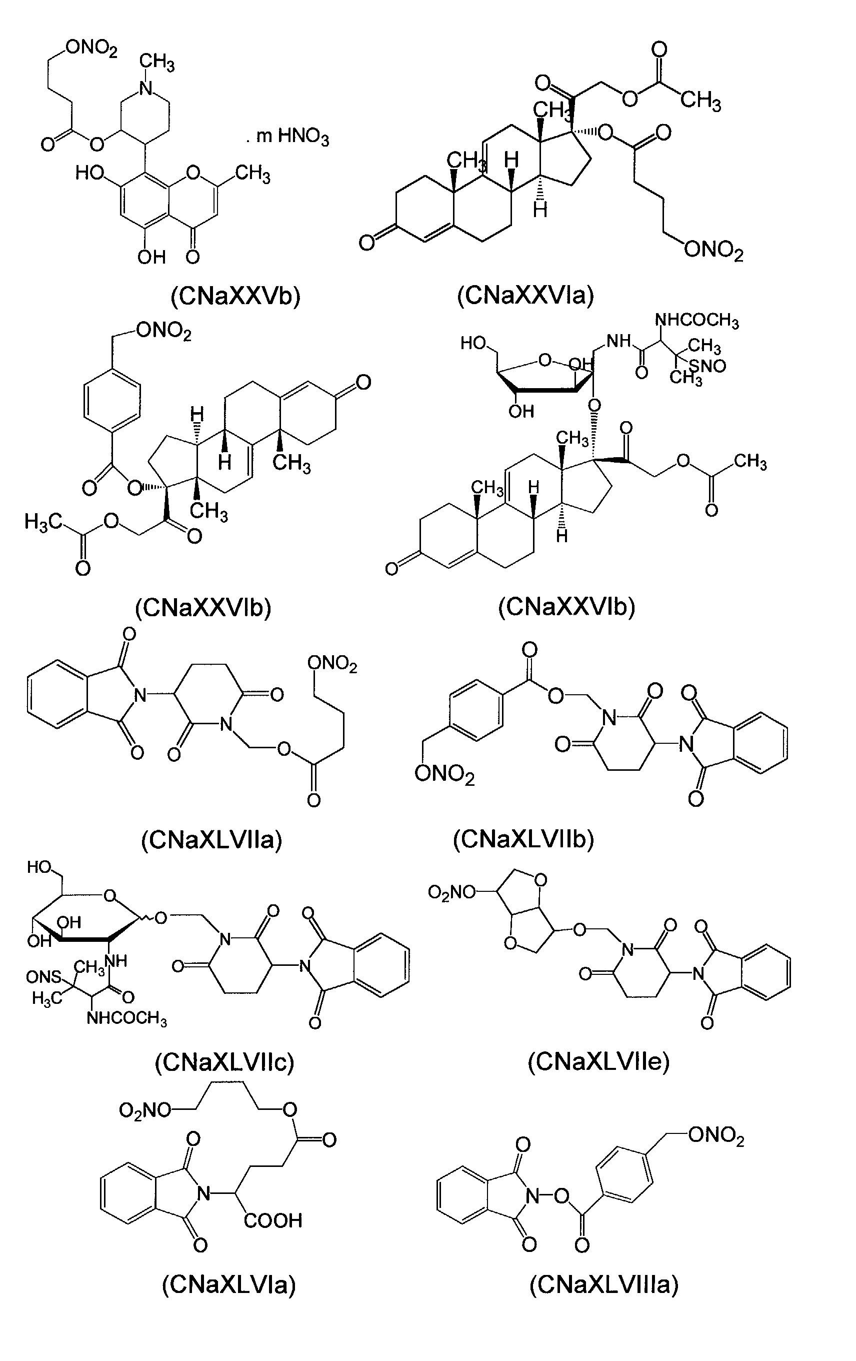

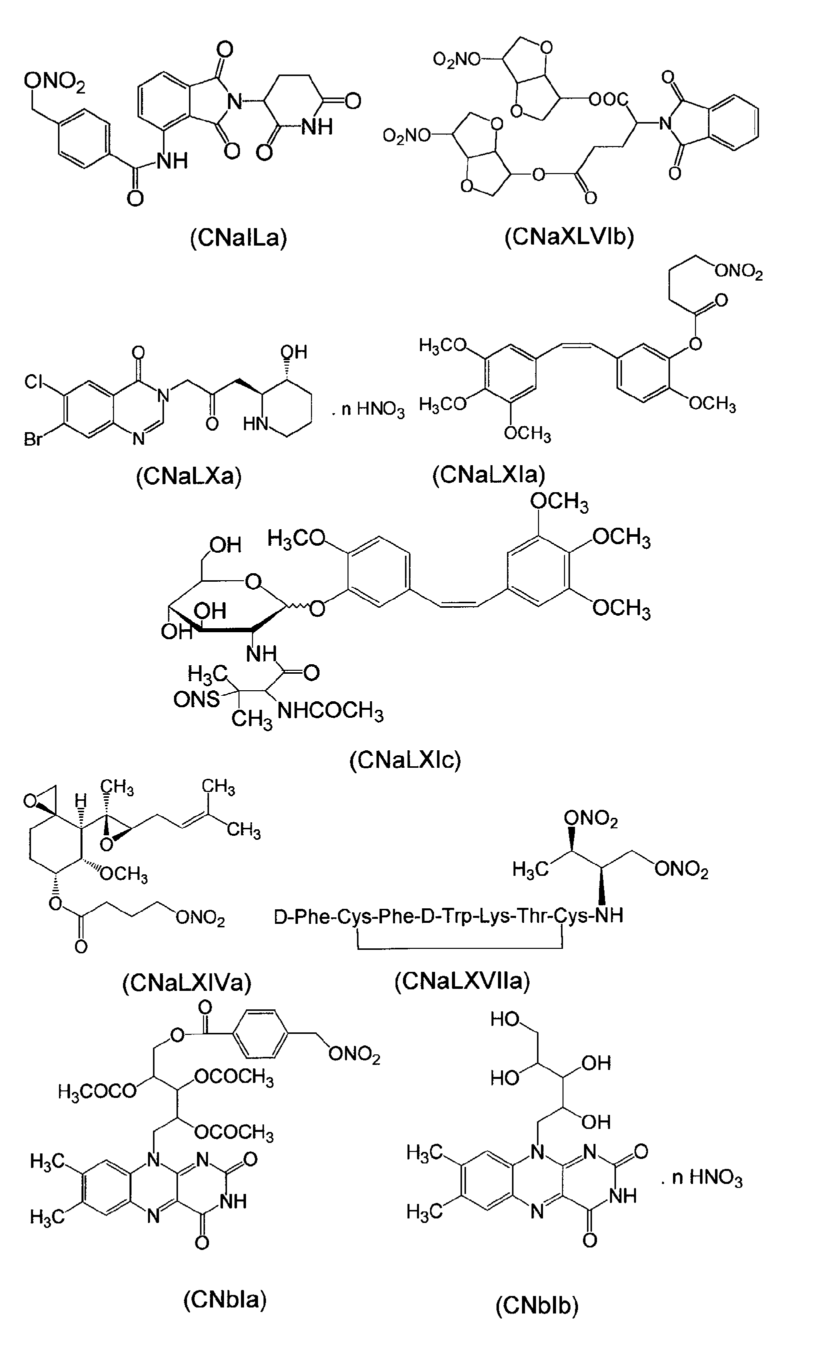

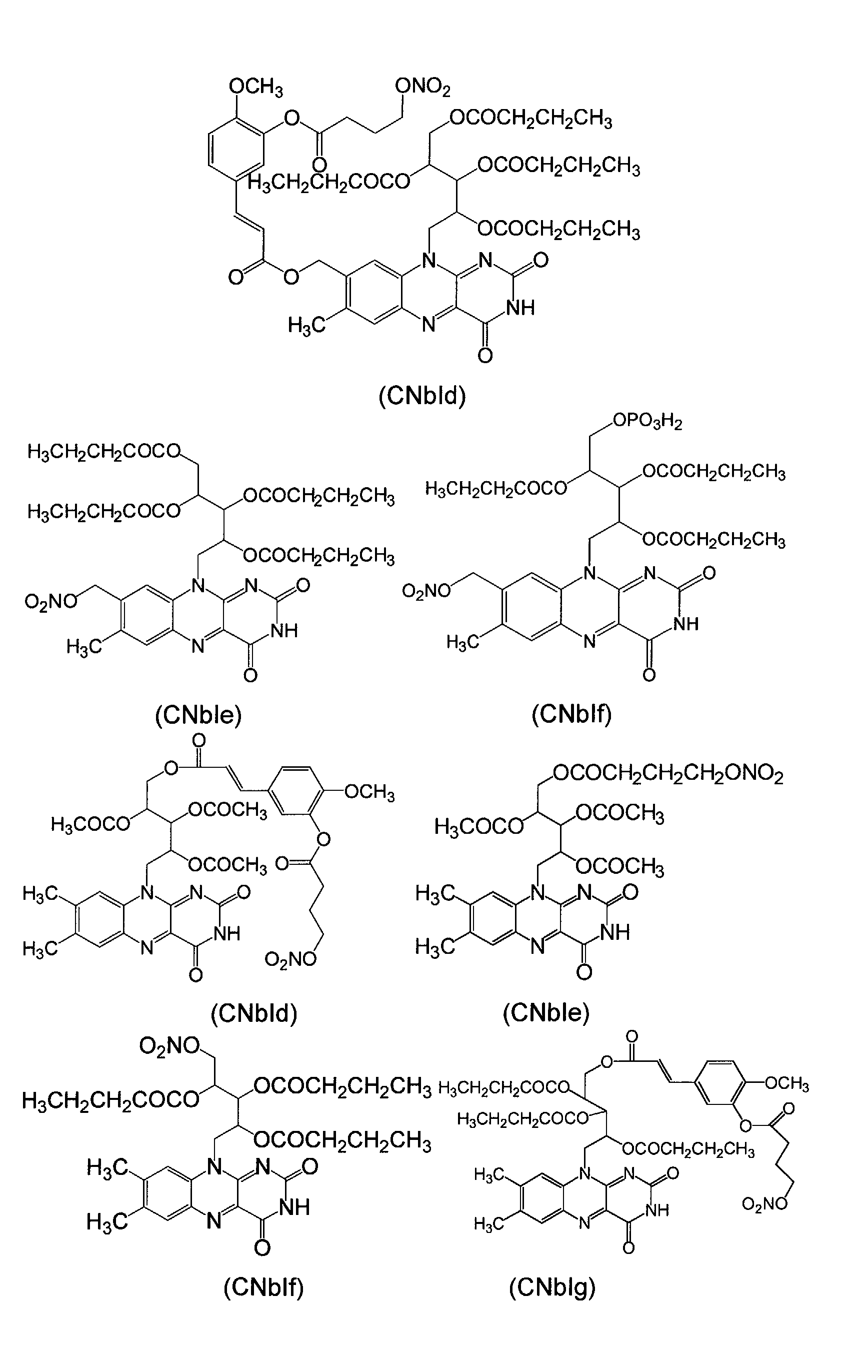

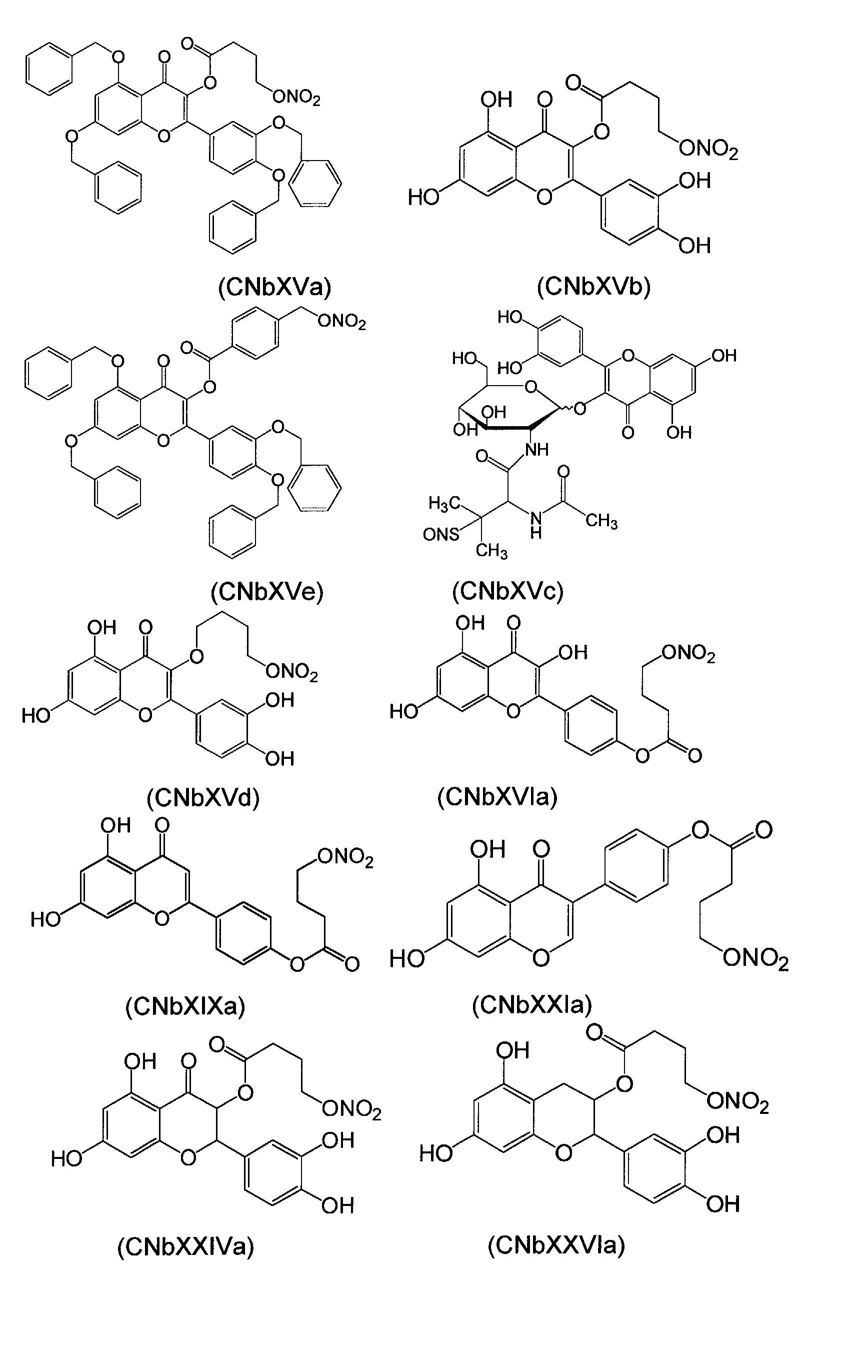

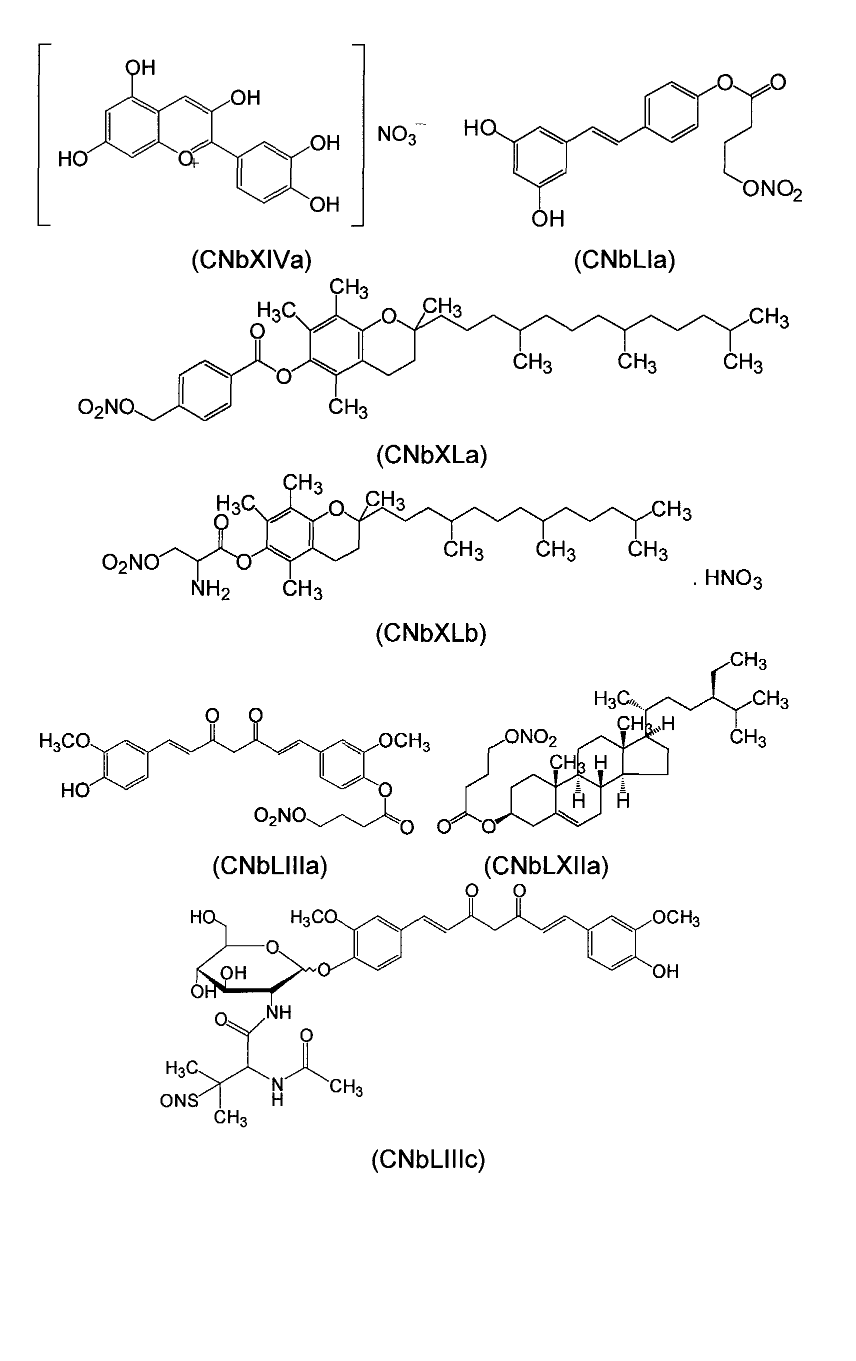

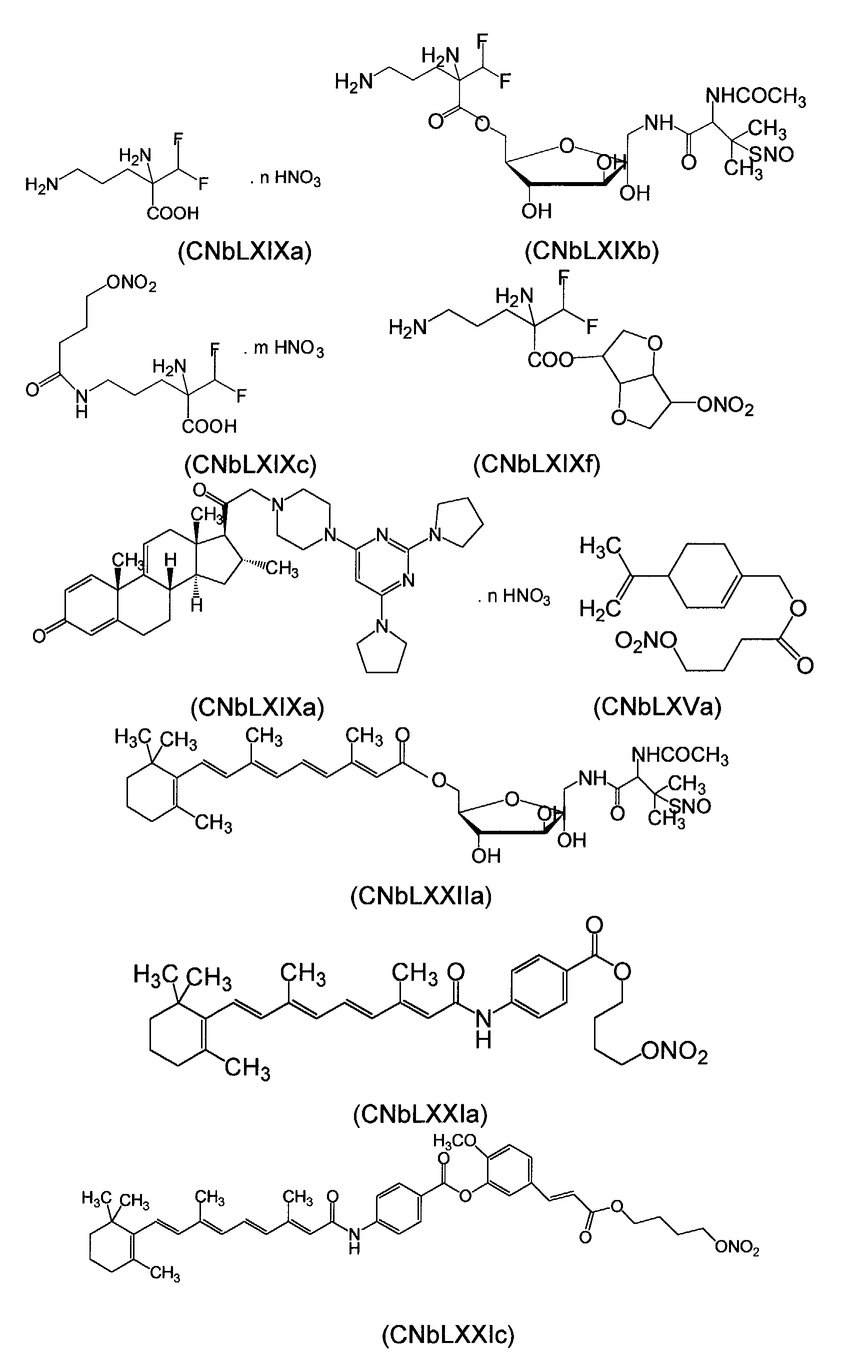

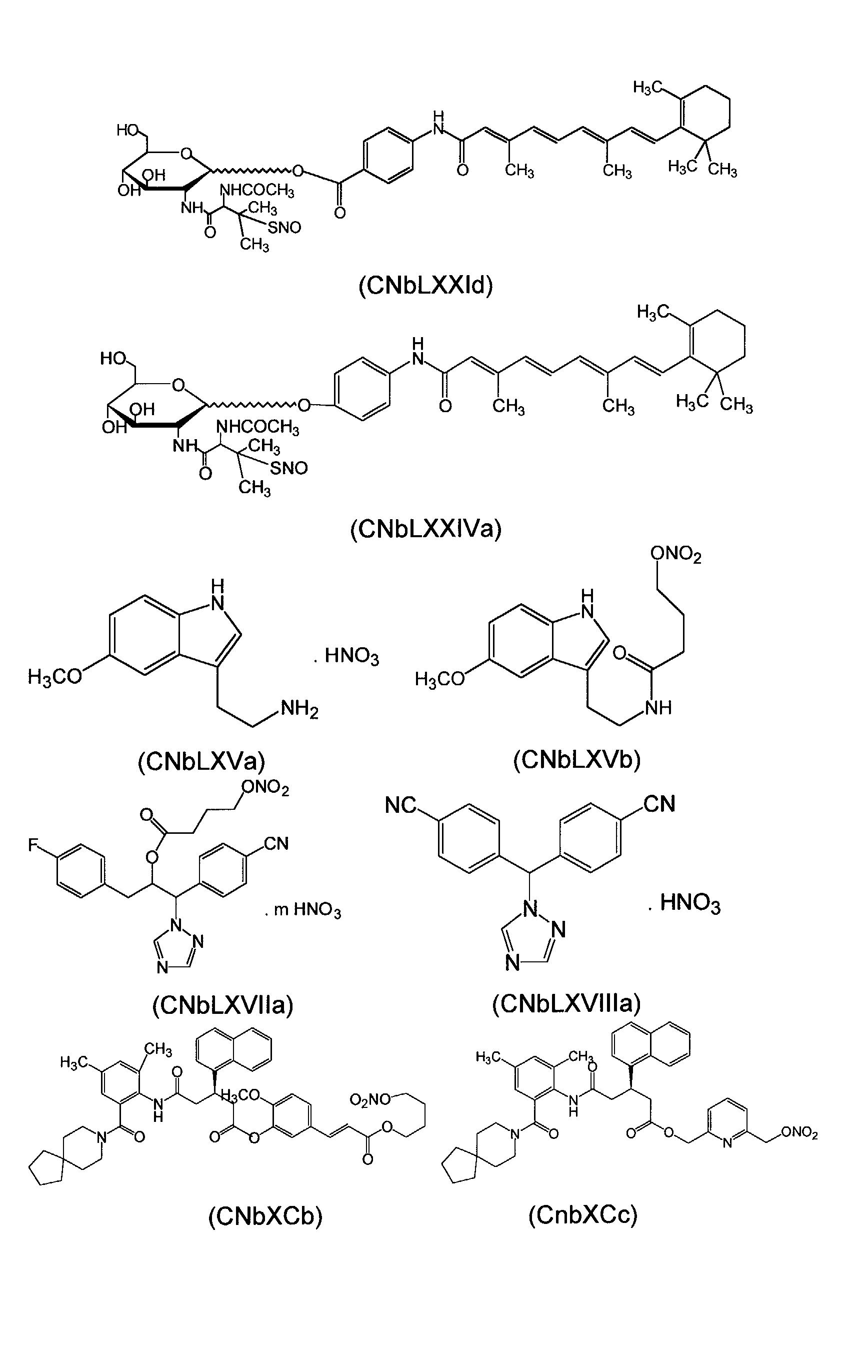

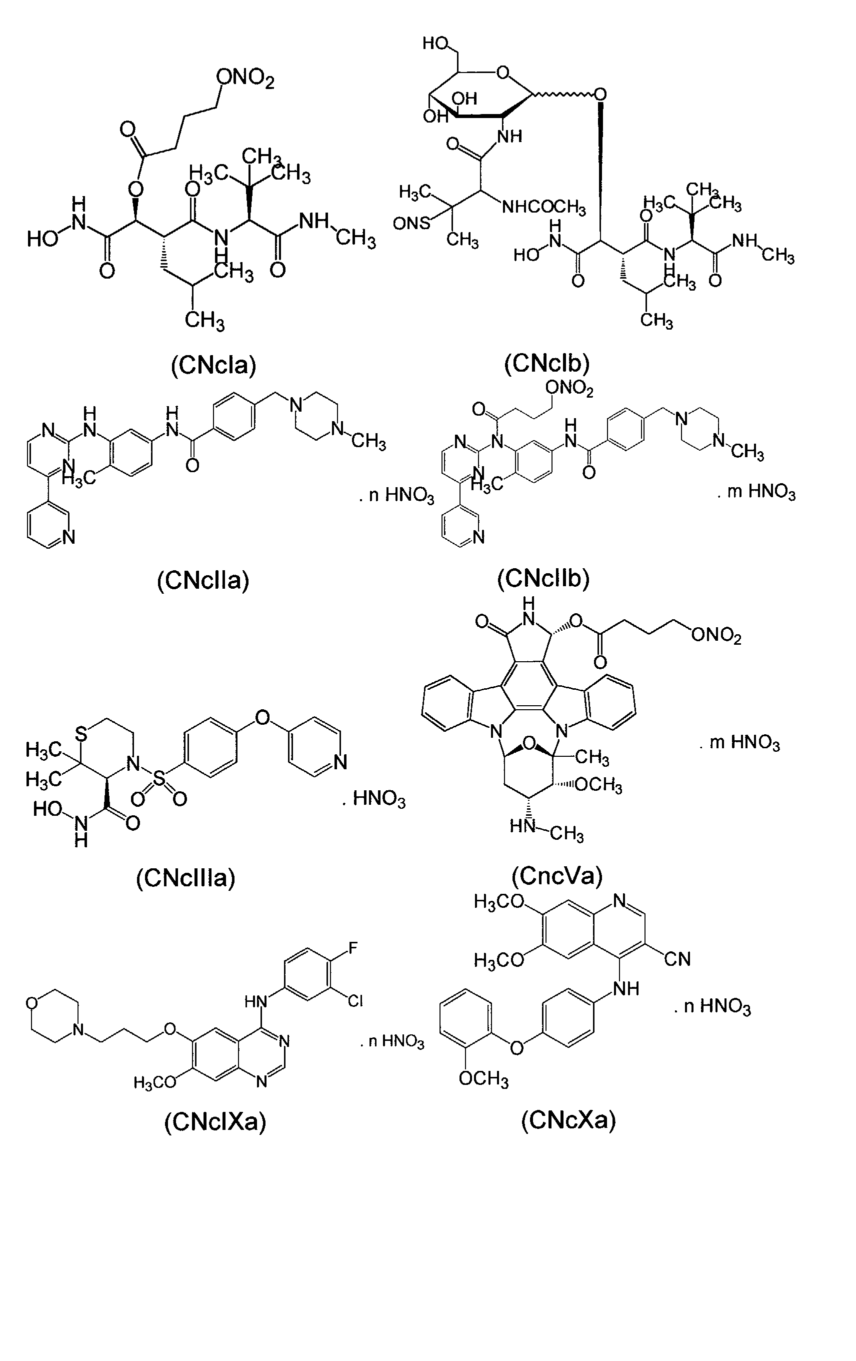

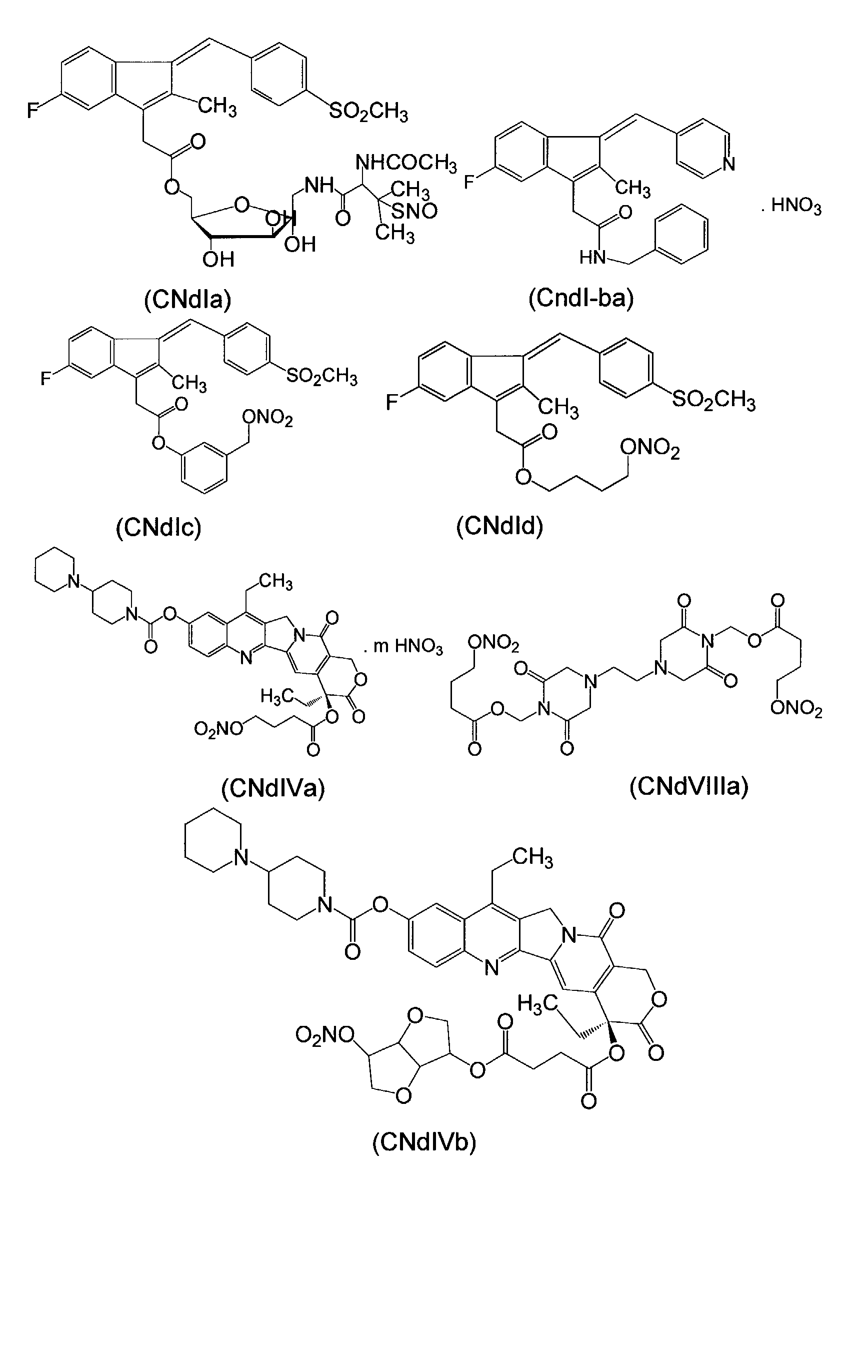

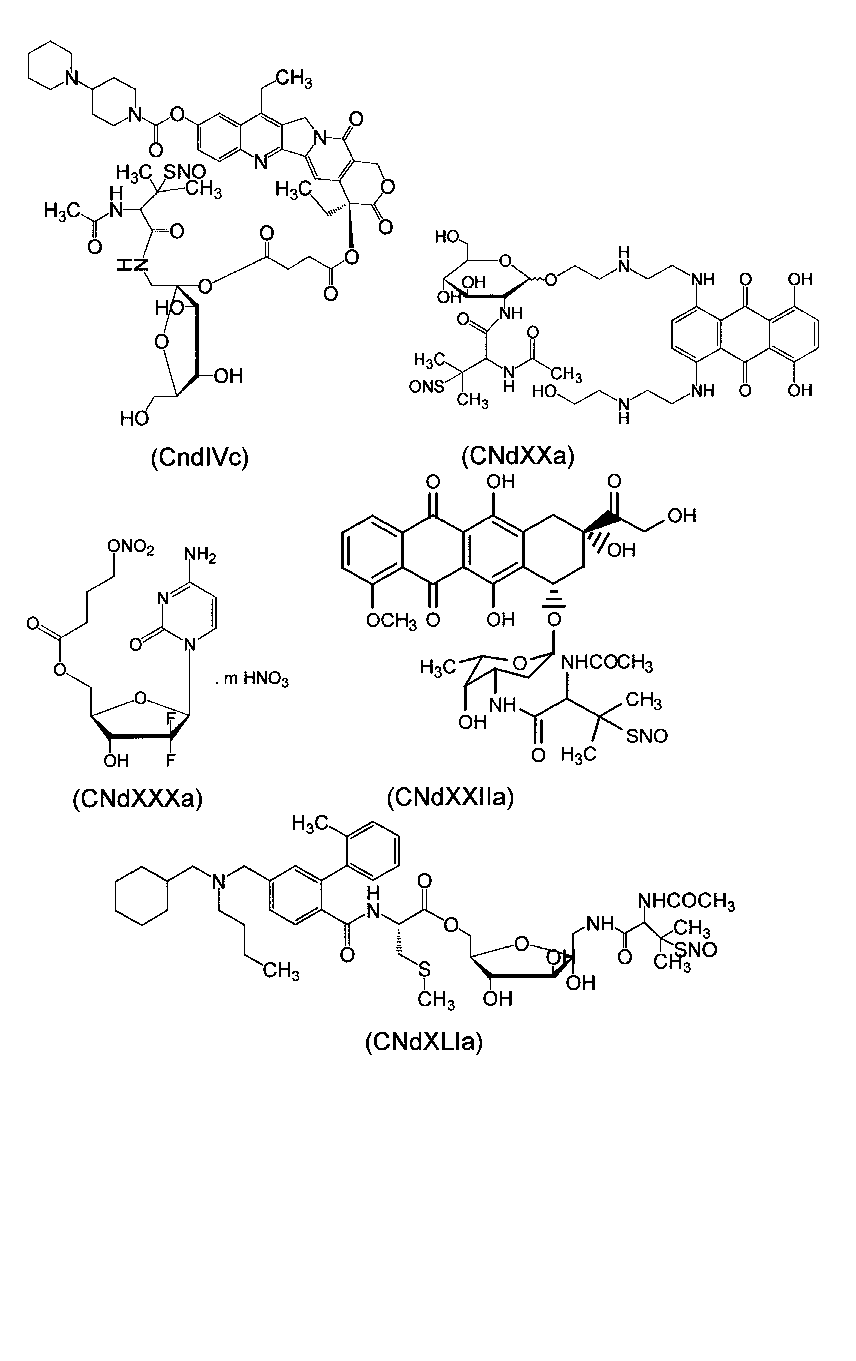

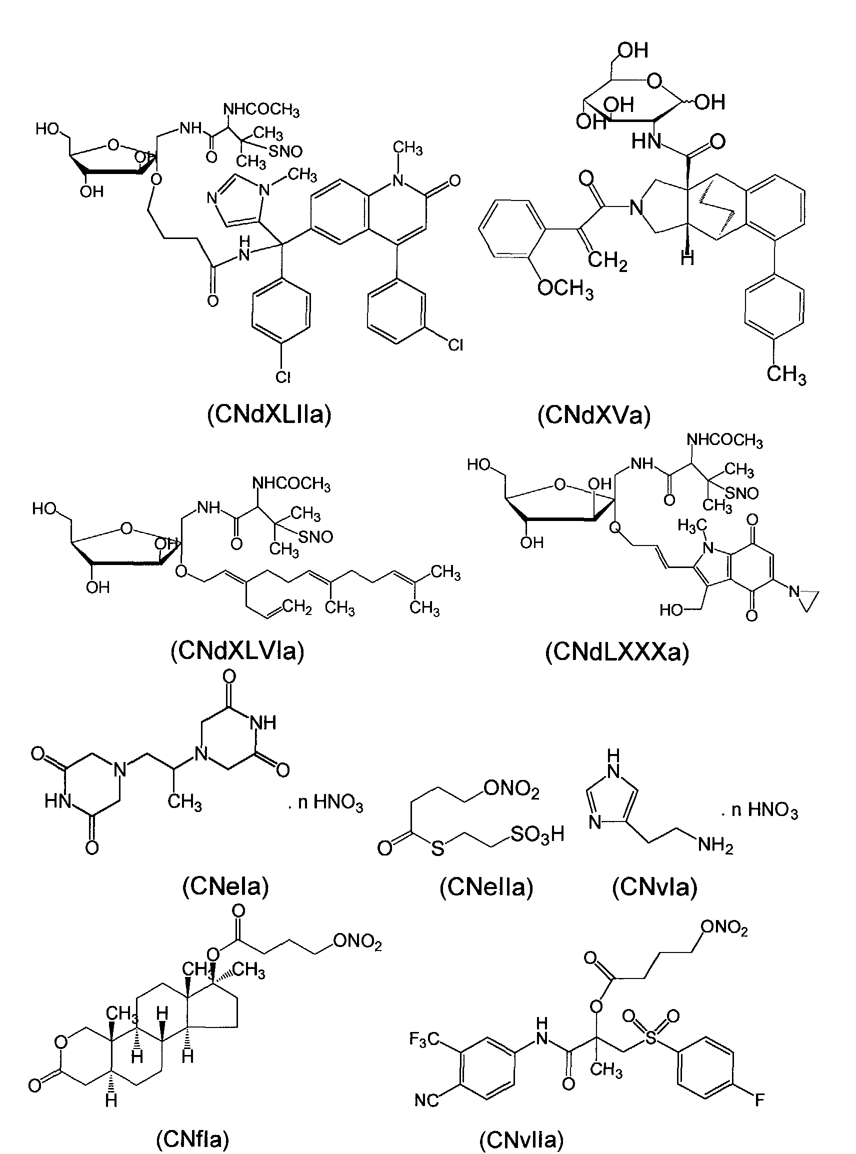

9. Compounds according to claim 8 chosen among the following 6 groups of compounds
-GROUP (Cal, Call)

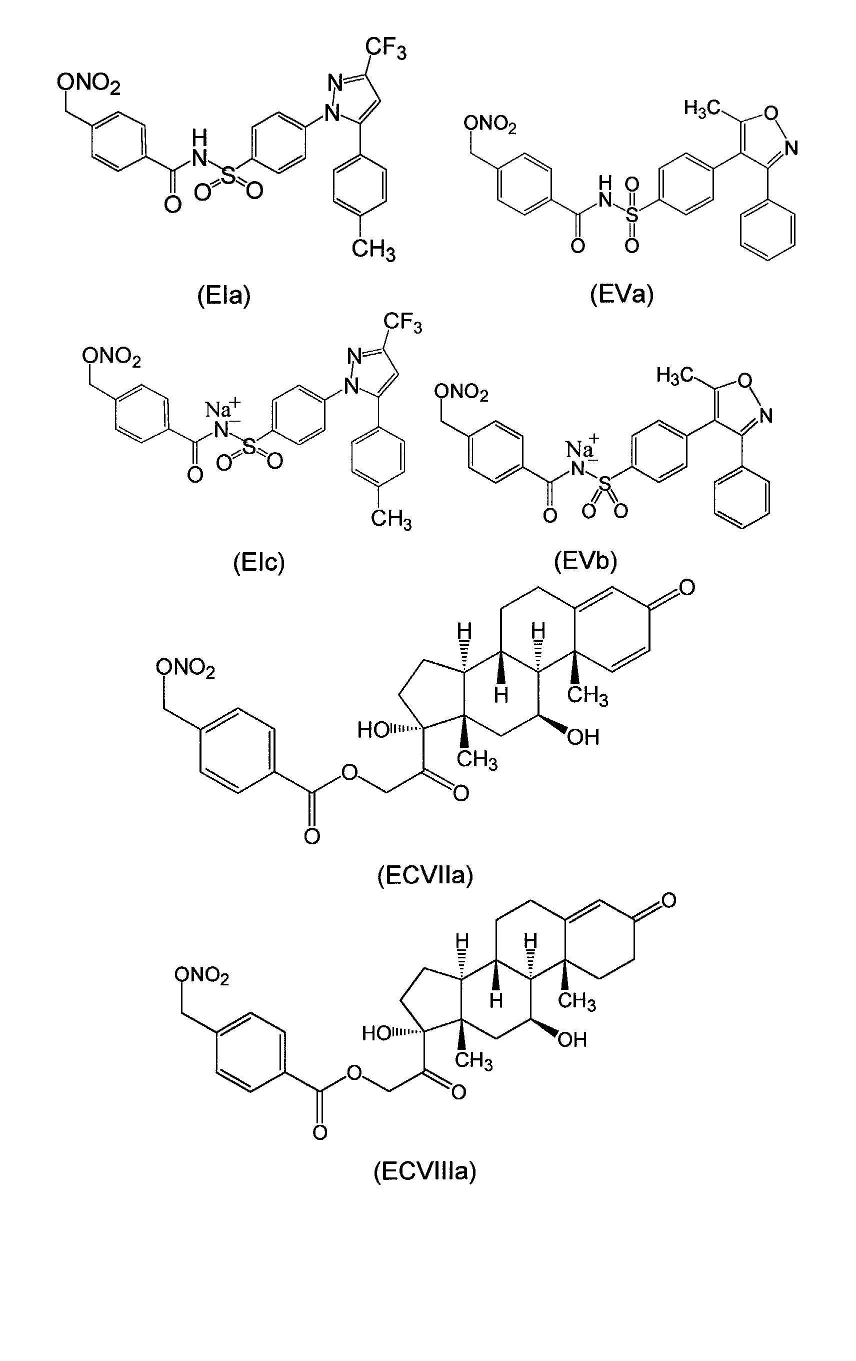

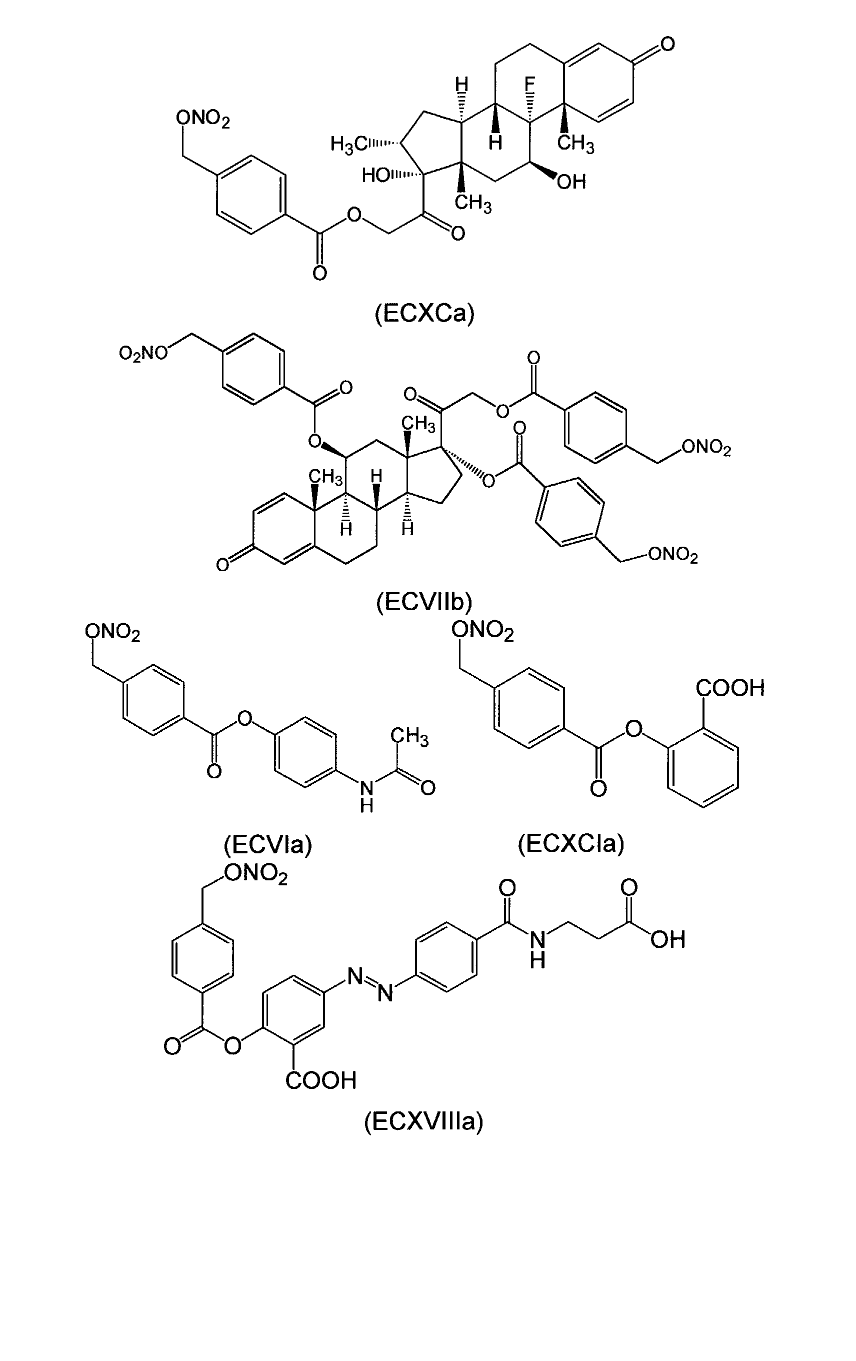

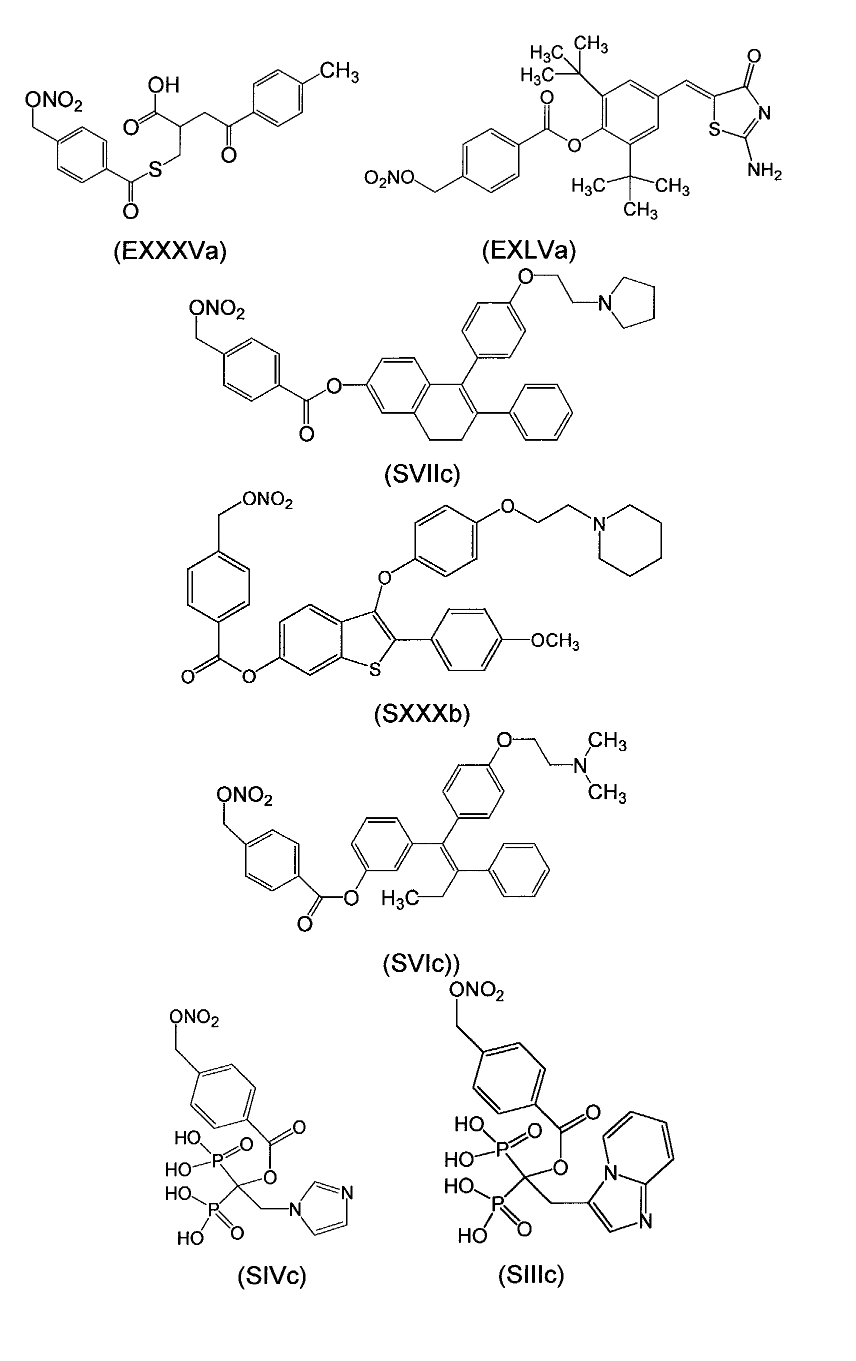

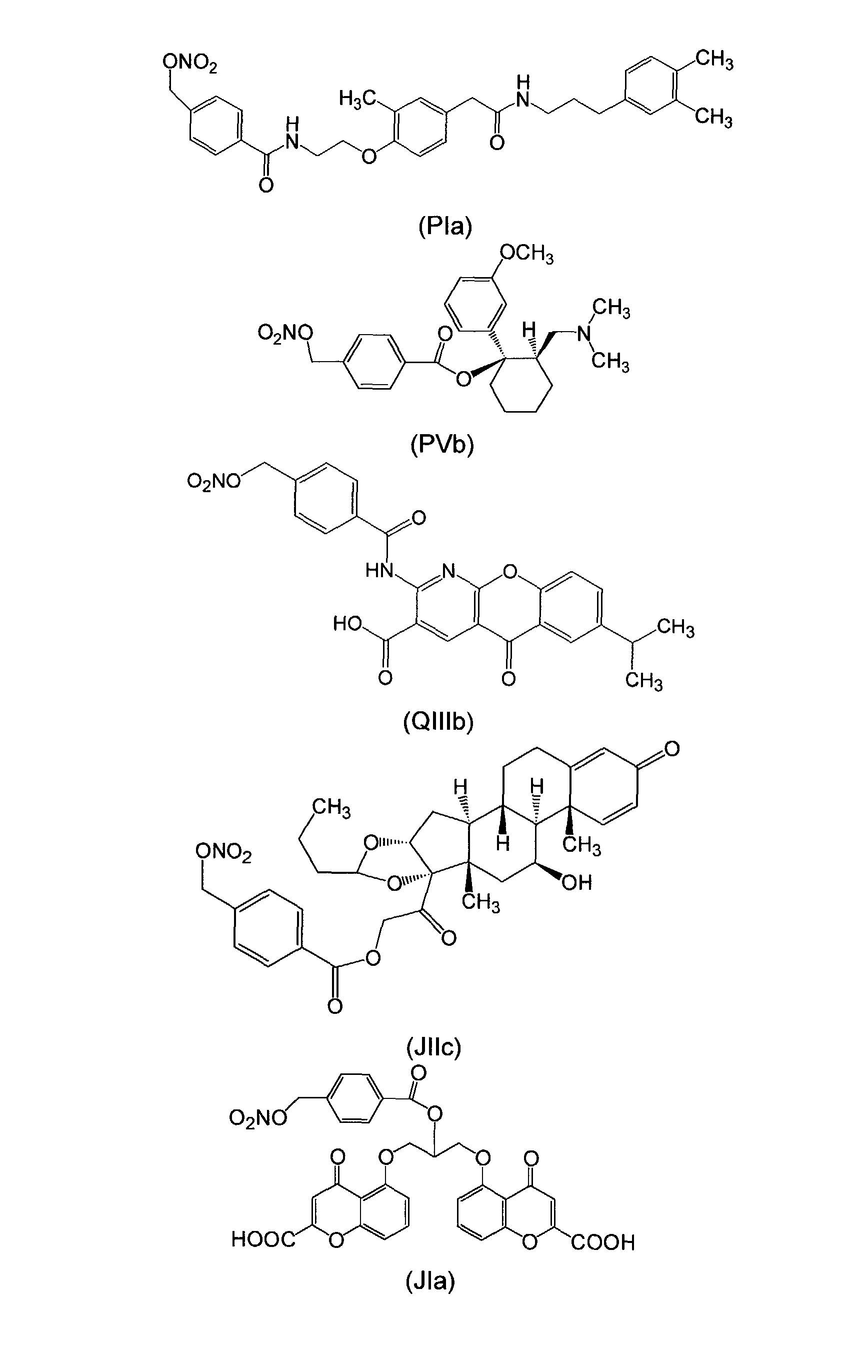

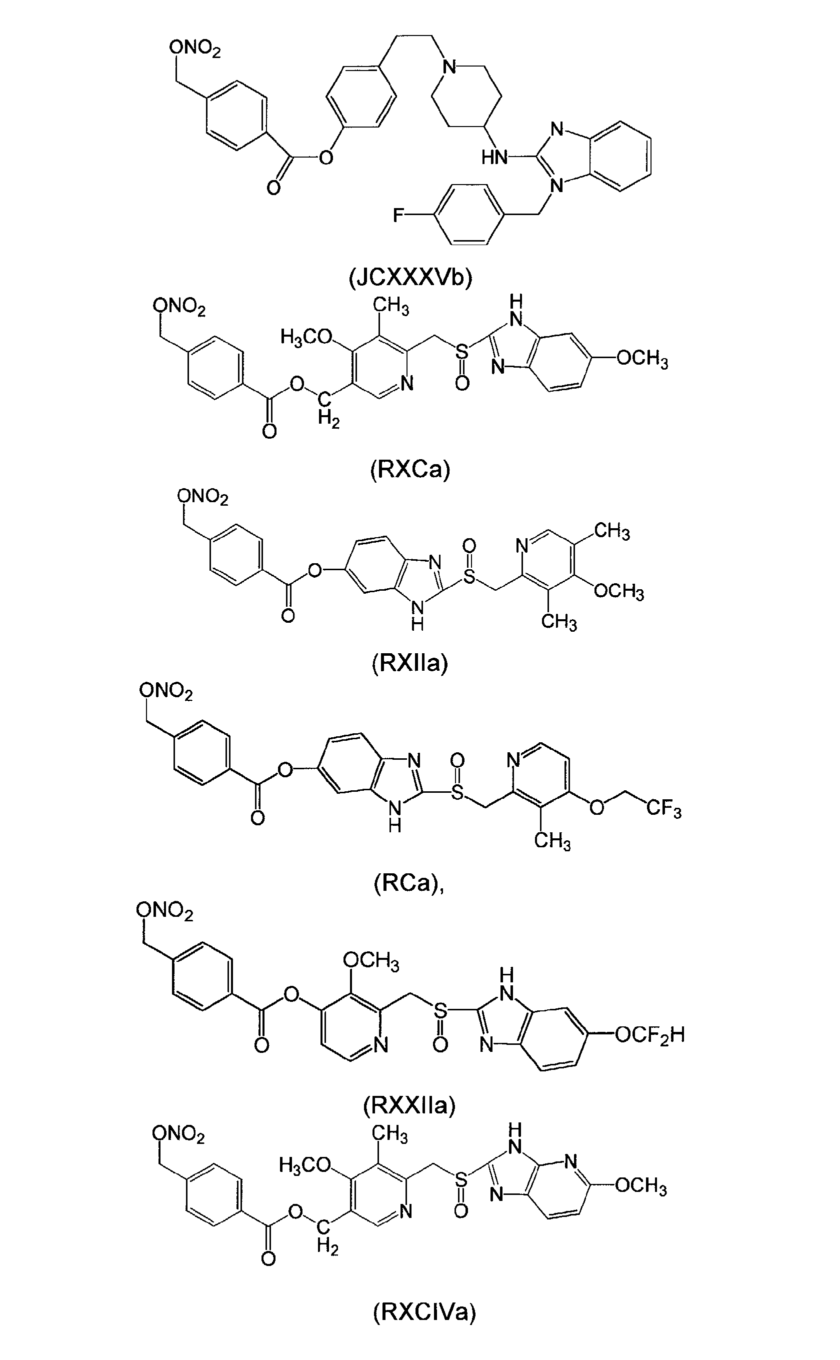

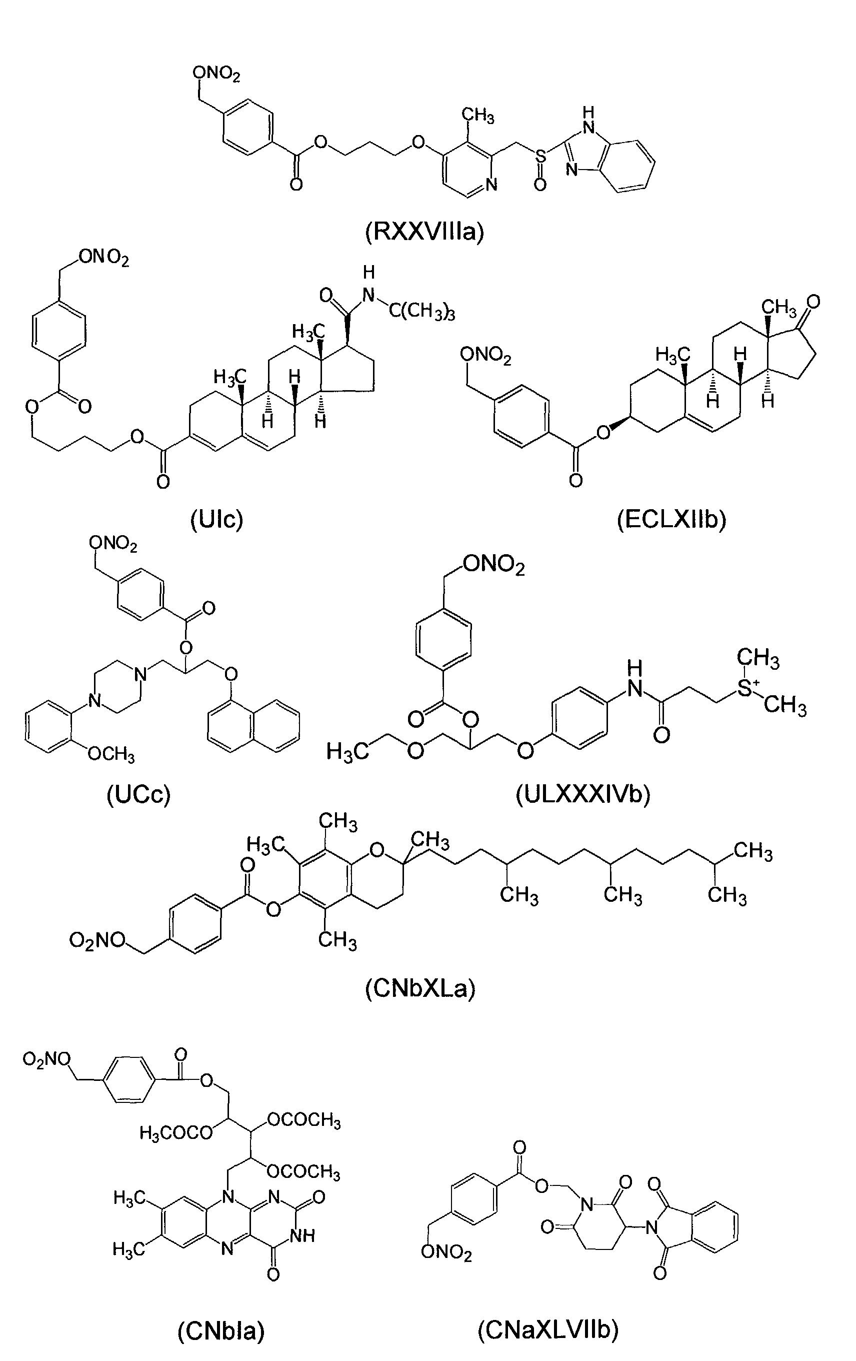

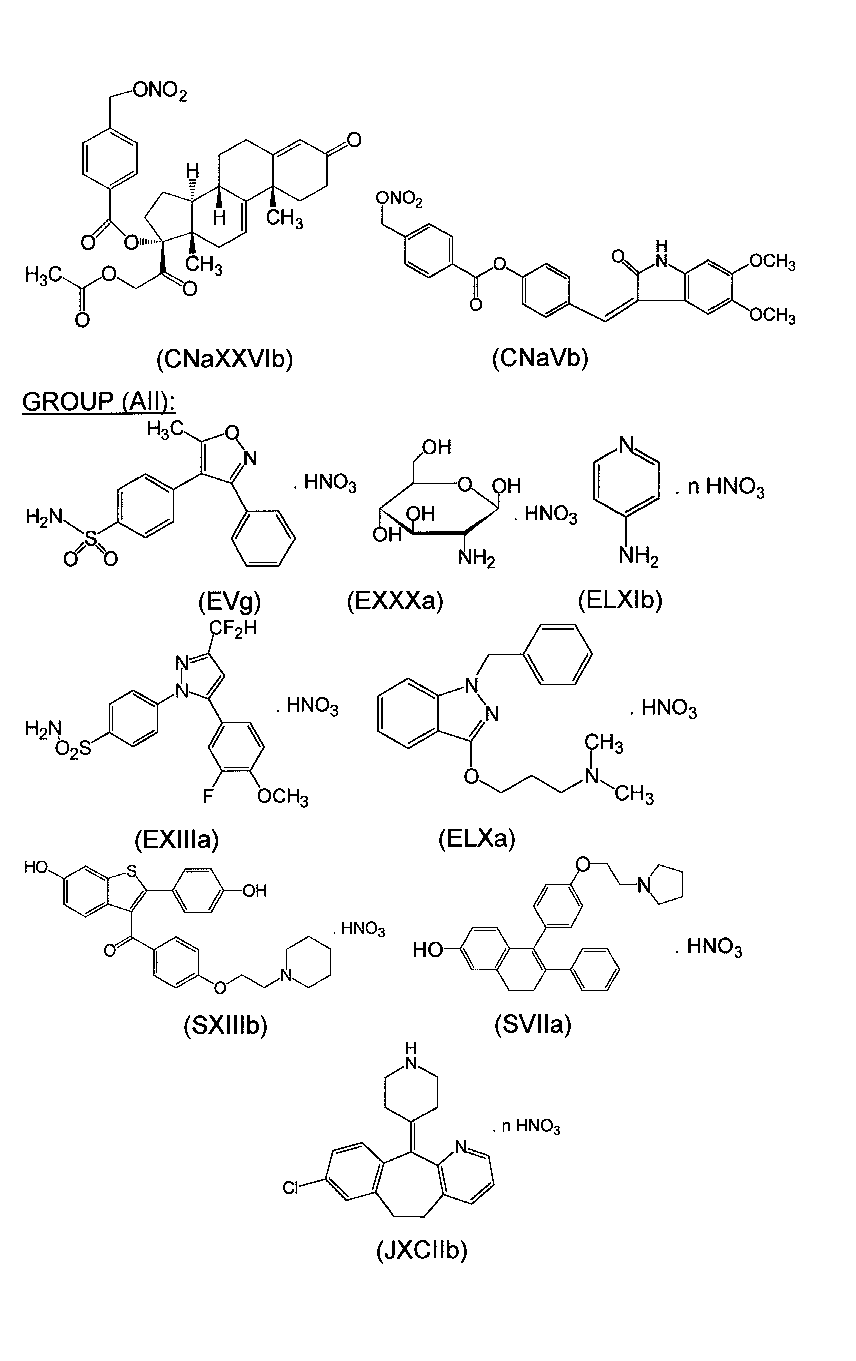

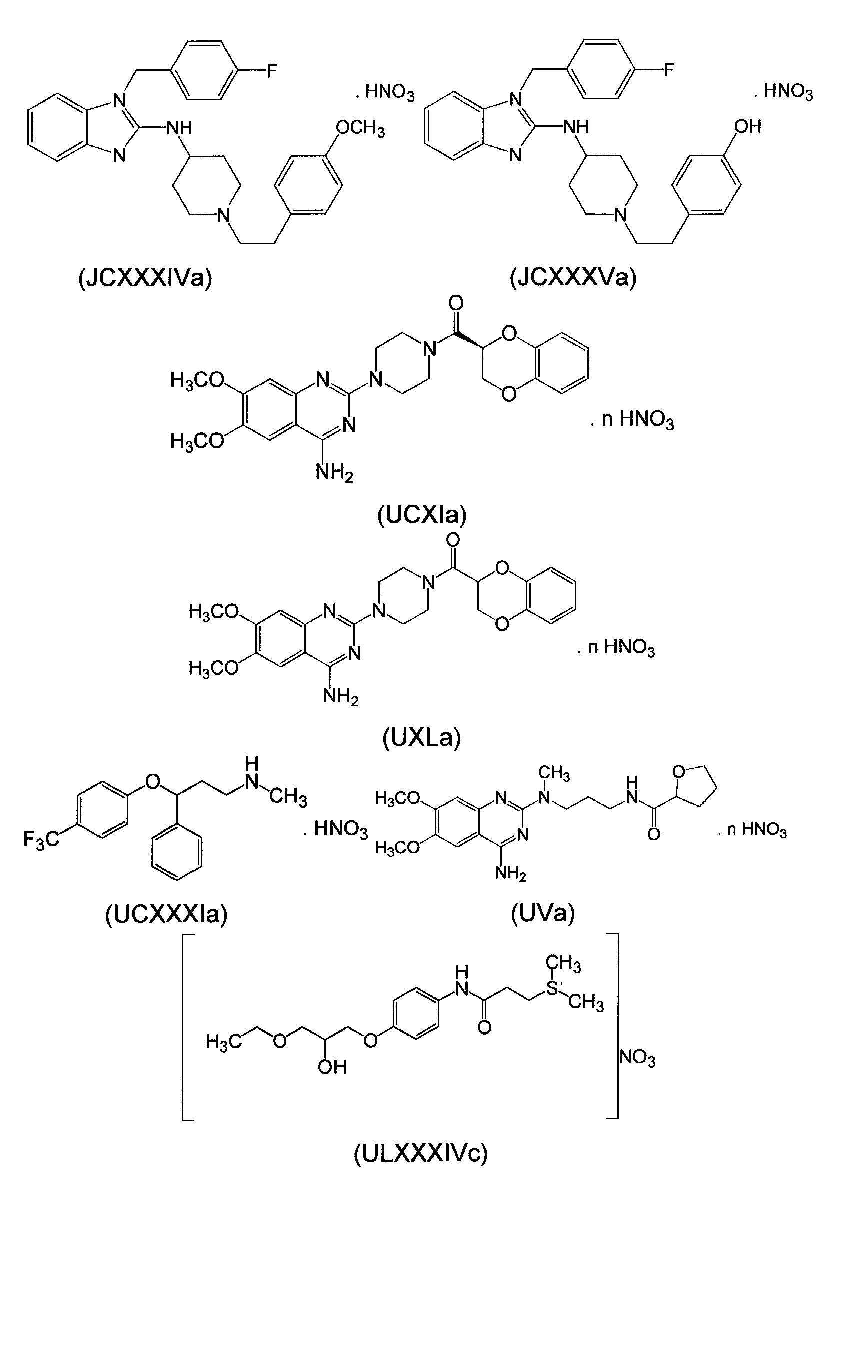

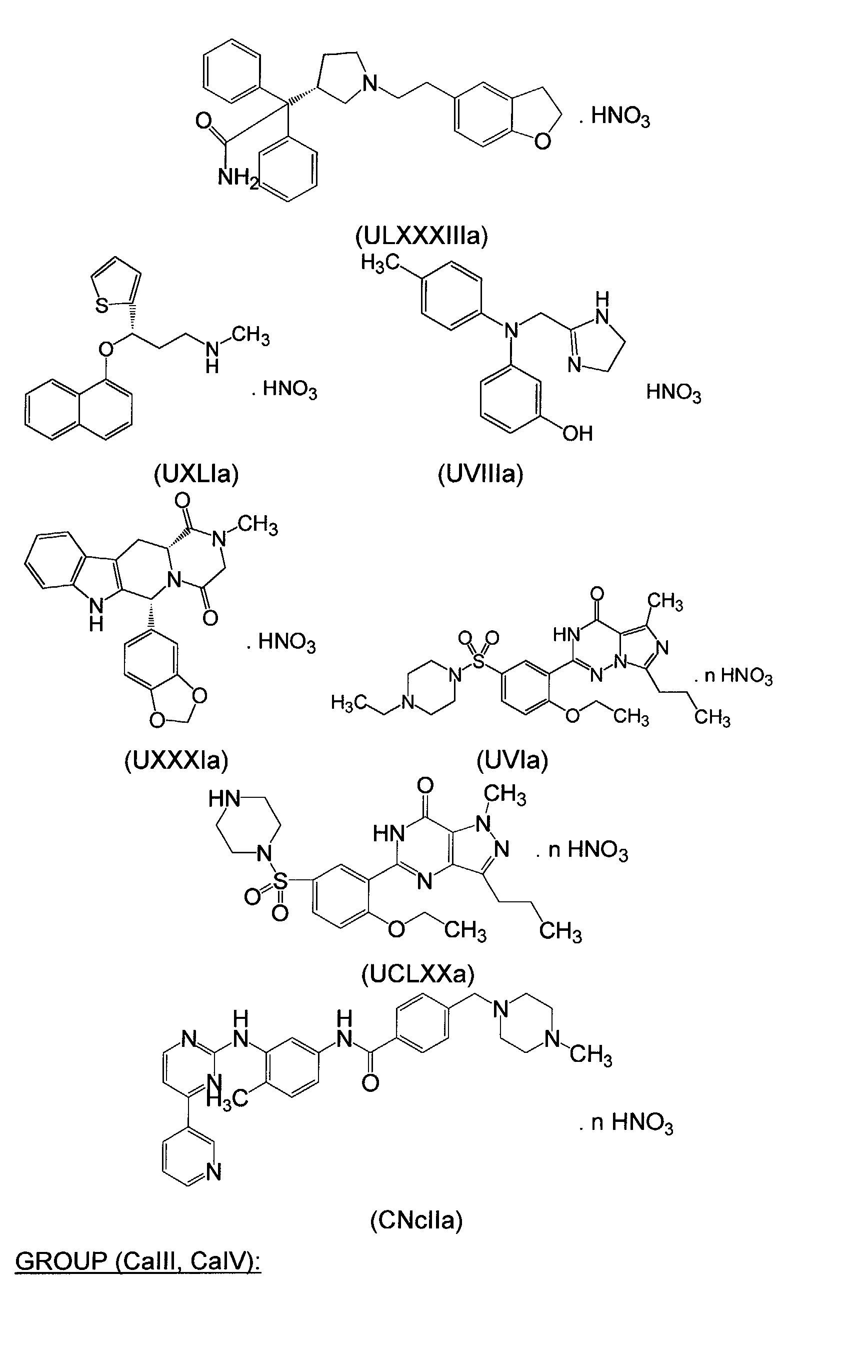

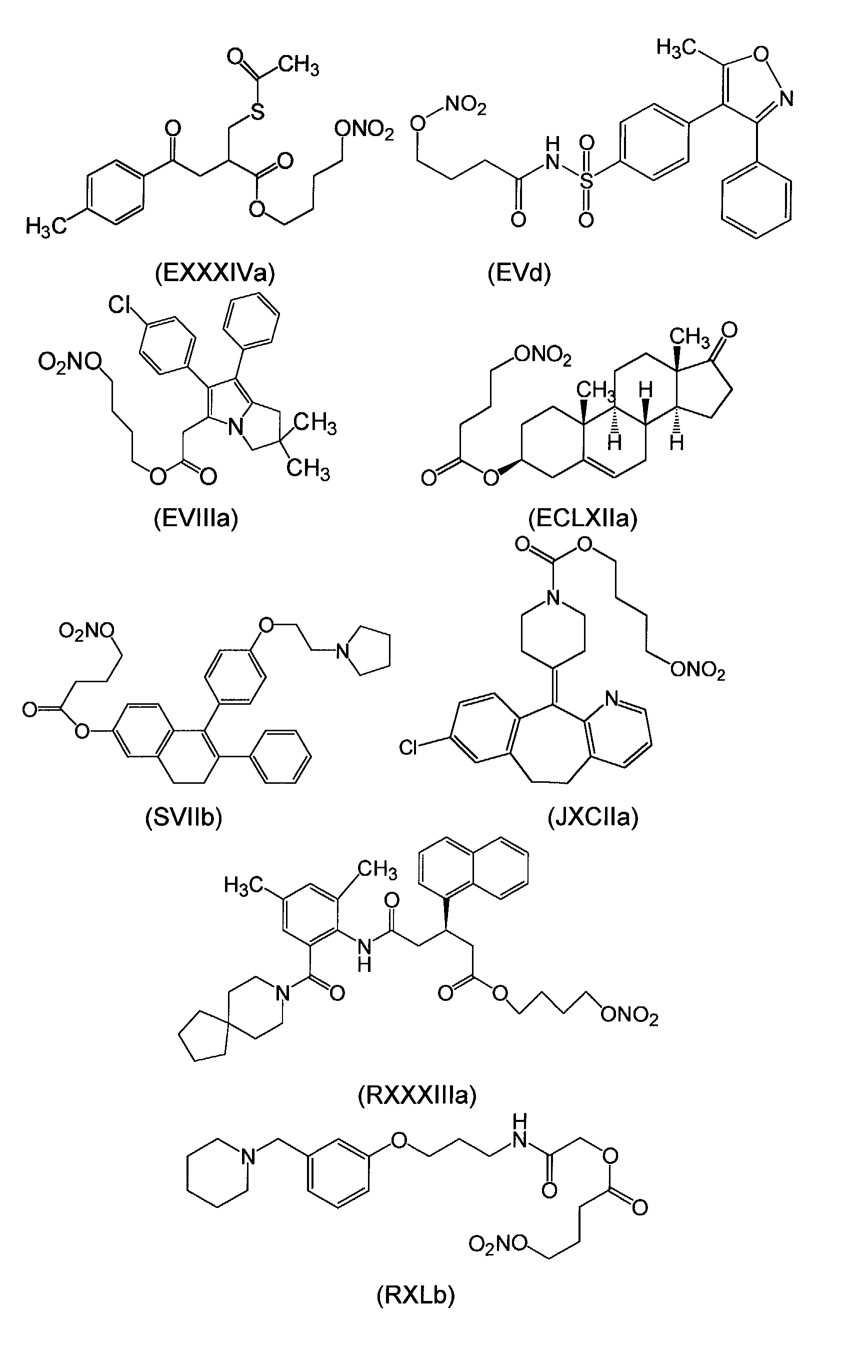

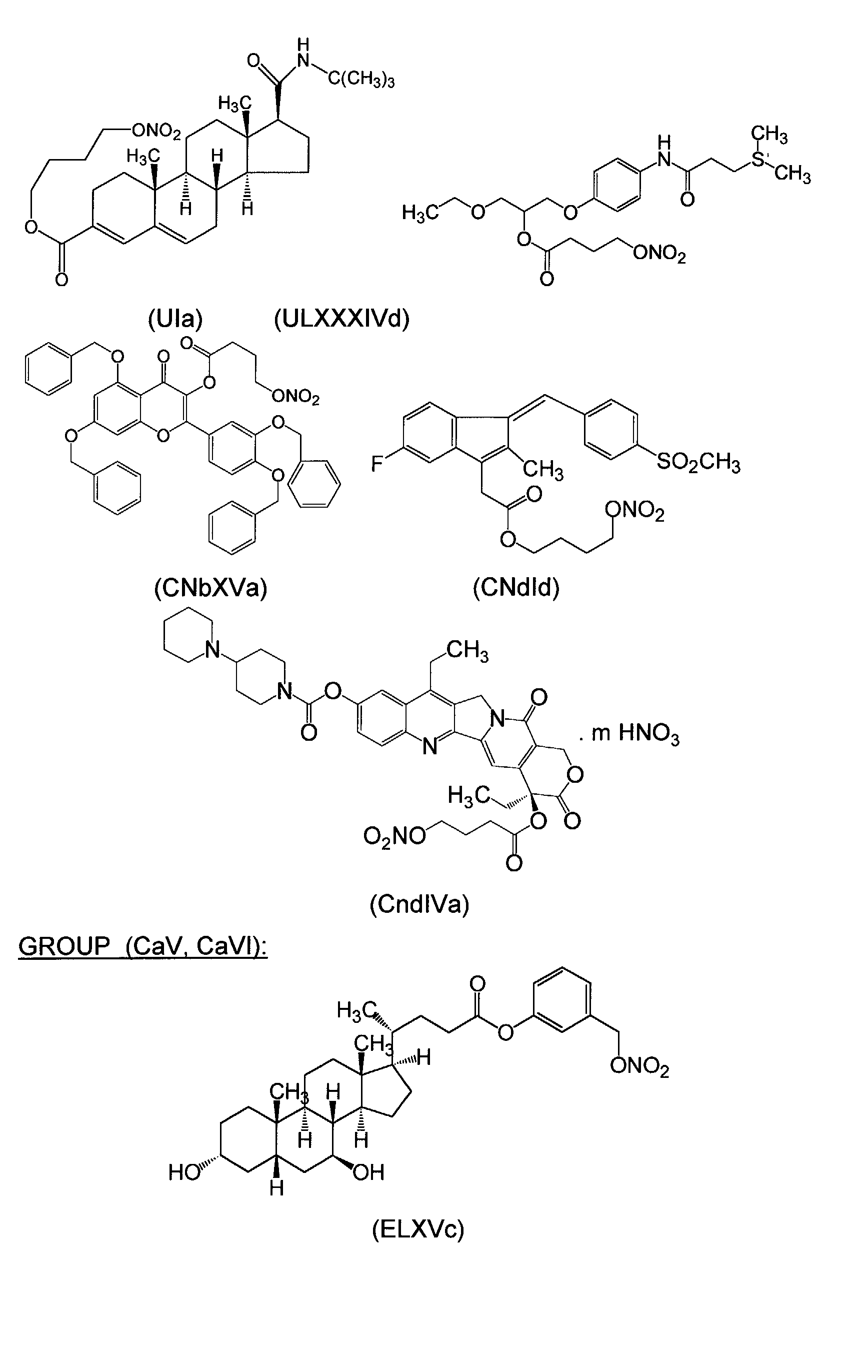

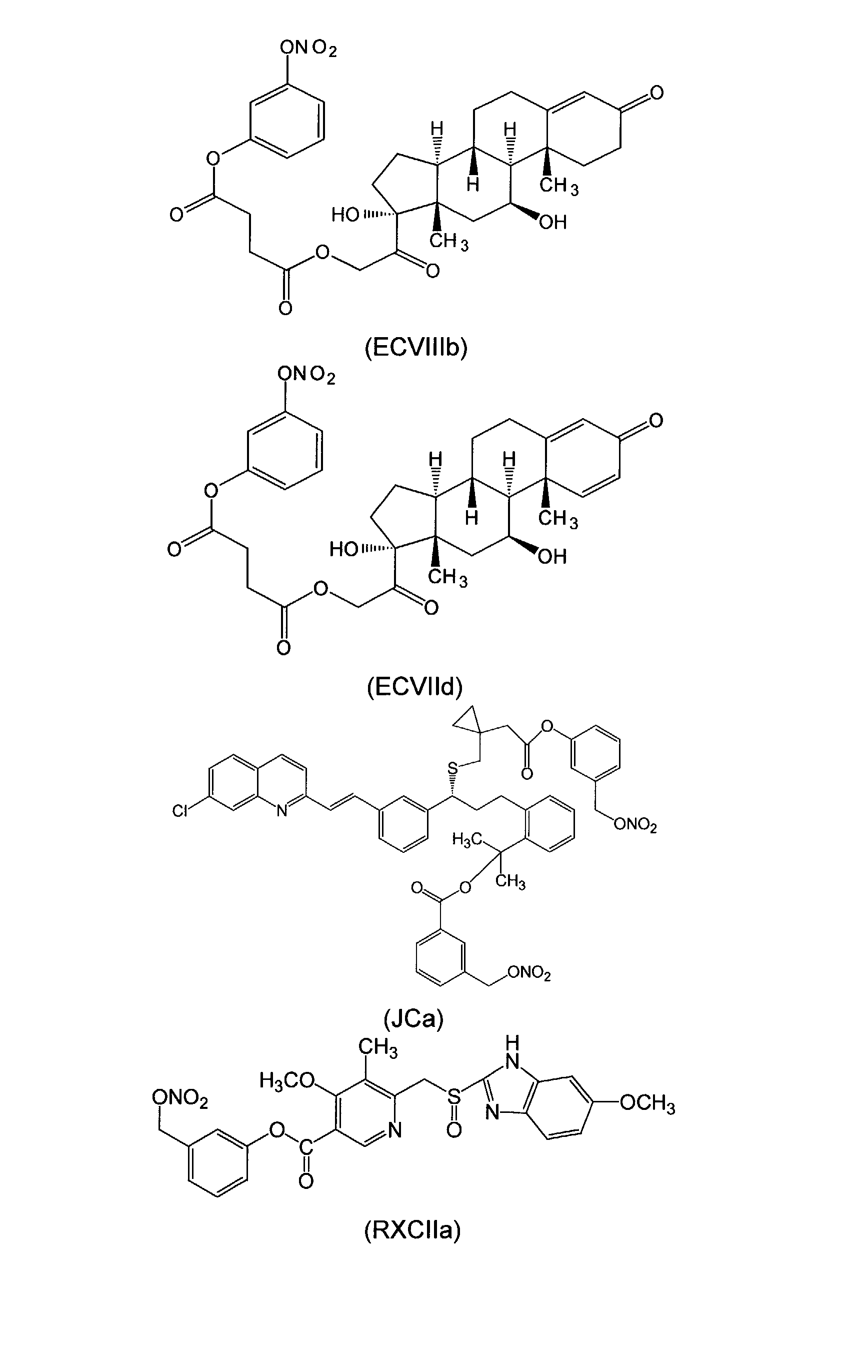

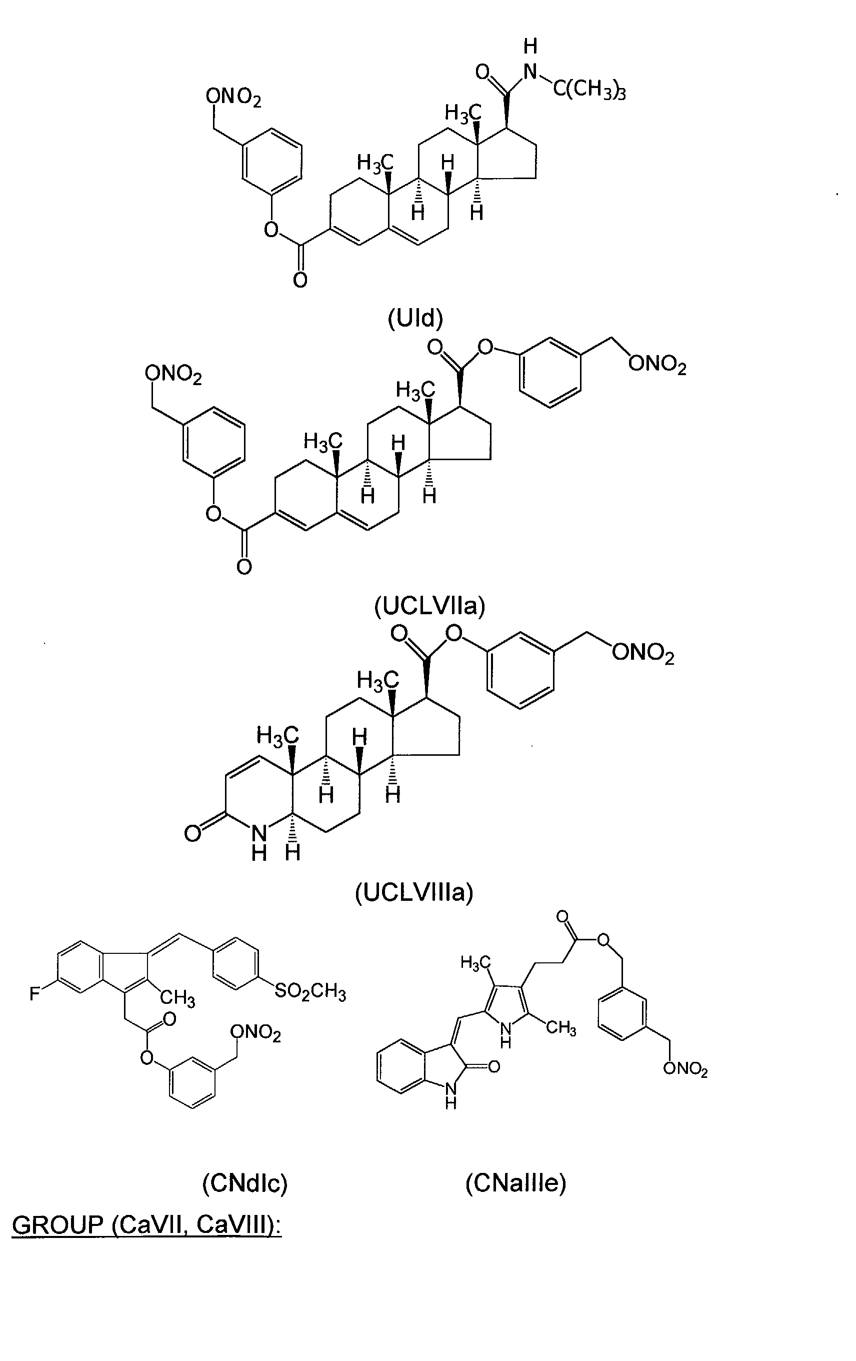

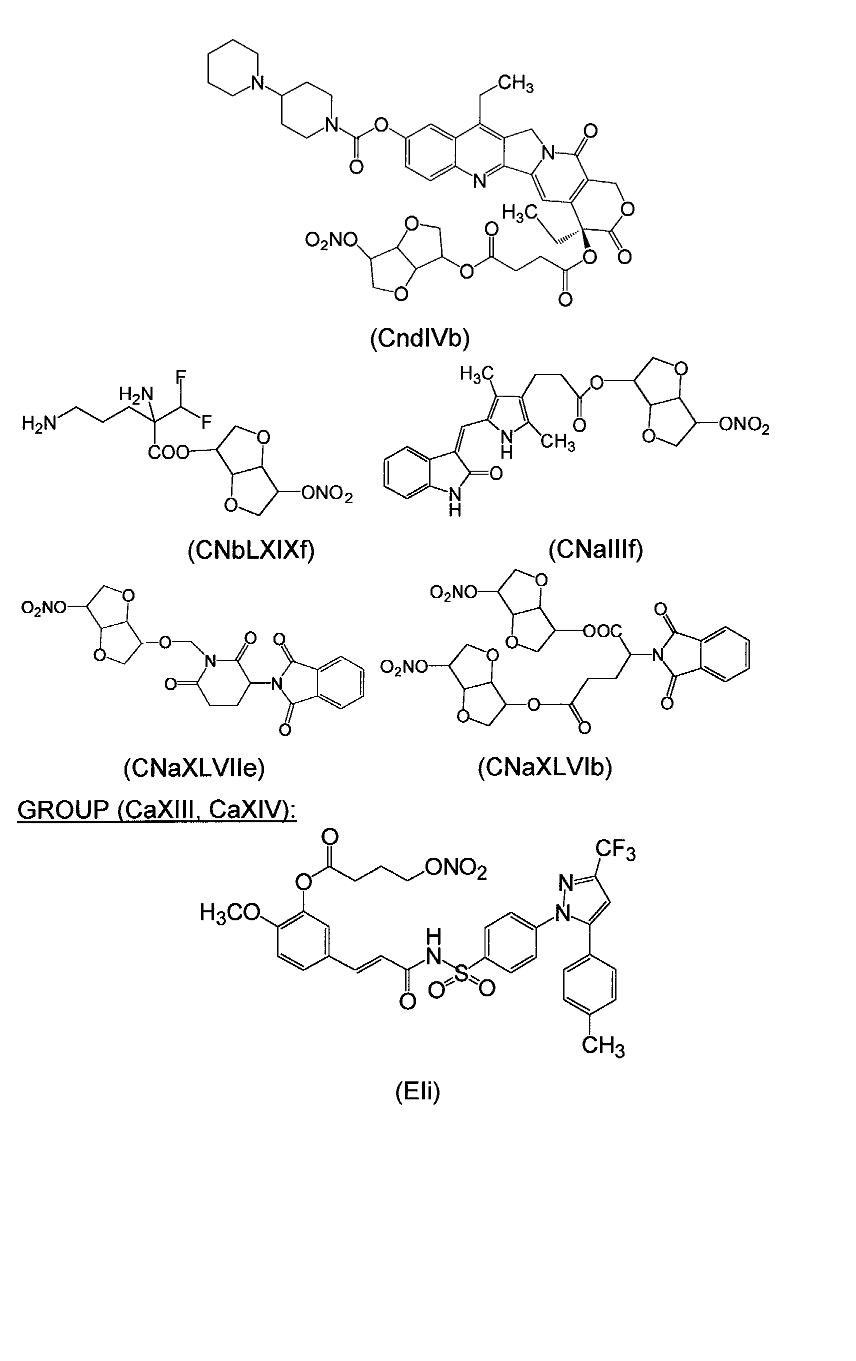

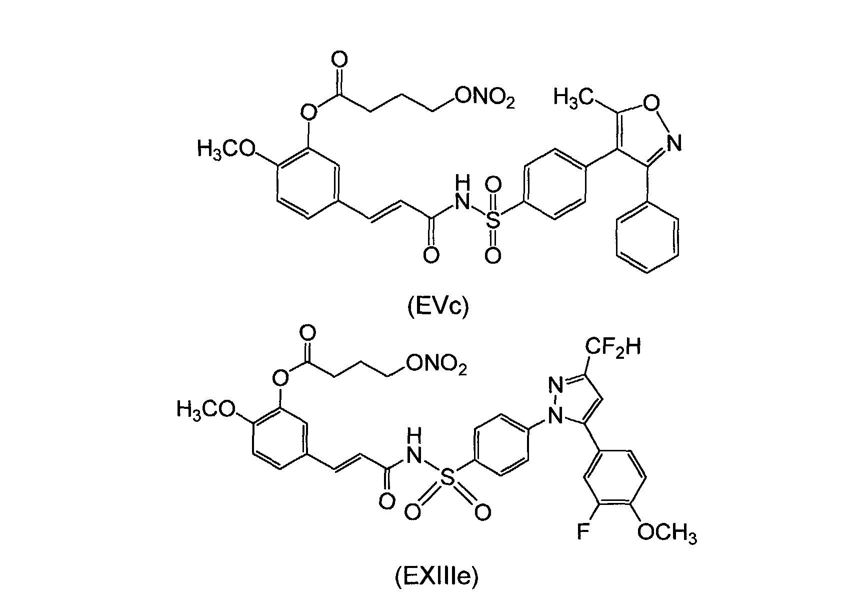

10. Process to synthetize paranitroxymethylbenzoyl derivatives (-Y₃) in which compounds with at least one alcoholic or aminic or thiolic group react in two steps initially with p-chloromethyl-benzoyl chloride or p-bromomethylbenzoyl chloride and then with silver nitrate, or in a single step with previously synthetized paranitroxymethylbenzoic acid through a dehydrating agent.

11. Pharmaceutical composition containing a therapeutic effective quantity of a compound according to claims 1-9 mixed with excipients for pharmaceutical use.

12. Use of the compounds or compositions according to claim 11 for the preparation of medicaments for prevention and treatment of diseases for which a chronic, controlled and selective administration of nitric oxide is useful.

13. Use of the compounds or compositions according to claim 12 for the preparation of medicaments for prevention and treatment of inflammatory, ischemic, degenerative and proliferative diseases of muscoloskeletal system (e.g. rheumatoid arthritis, osteoarthritis, lupus, osteoporosis, Paget disease), of tegumental system (e.g. ulcers, aphtha, psoriasis, dermatitis, skin tumors) of respiratory system (e.g. asthma, chronic obstructive pulmonary disease, allergy, emphysema, bronchitis, ARDS, rhinitis, cystic fibrosis, lung tumors), of gastrointestinal system (e.g. peptic ulcer, duodenal ulcer, ulcerative colitis, Crohn disease, hepatic cirrhosis, pancreatitis, esophagitis, gastritis, colon familial polyposis, colon-rectum tumors, liver tumors, oesophasus tumors, pancreas tumors, stomach tumors), of genital system (e.g. benign prostatic hypertrophy, prostatitis, sexual disorders, prostate tumors, breast tumors, uterus tumors, ovary tumors), of urinary system (urinary incontinence, bladder tumors) and of central nervous system (e.g, multiple sclerosis, Alzheimer disease, brain tumours).
